# EUROPEAN PATENT APPLICATION

(11) **EP 4 295 852 A2**
(43) Date of publication of application: **27.12.2023**
(21) Application number: 23177402.7
(22) Date of filing: 13.07.2017
(51) Int. Cl.: A61K 31/506

(54) **PIPERIDINE DERIVATIVES AS INHIBITORS OF CYCLIN DEPENDENT KINASE 7 (CDK7)**

(30) Priority: 13.07.2016 US 201662361852 P
(62) Divisional of application: 17746235.5
(71) Applicant: Syros Pharmaceuticals, Inc., Cambridge, MA 02140 (US)
(72) Inventor: MARINEAU, Jason J., Franklin, MA 02038 (US); ZAHLER, Robert, Pennington, NJ 08534 (US); CIBLAT, Stephane, Montreal, Quebec H2J 0A2 (CA); WINTER, Dana, Rigaud, Quebec J0P 1P0 (CA); KABRO, Anzhelika, Lachine, Quebec H8T 2T3 (CA); ROY, Stephanie, Lachine, Quebec H8T 2T3 (CA); SCHMIDT, Darby, Arlington, MA 02474 (US); CHUAQUI, Claudio, Arlington, MA 02476 (US); MALOJCIC, Goran, Cambridge, MA 02140 (US); PIRAS, Henri, 34990 Juvignac (FR); WHITMORE, Kenneth Matthew, Boucherville, Quebec J4B 1C6 (CA); LUND, Kate-lyn, Oro-medonte, Ontario L3V 0R8 (CA); SINKO, William, Roslindale, MA 02131 (US); SPROTT, Kevin, Needham, MA 02492 (US)
(74) Representative: Snaith, James Michael

(57) **Abstract**

The present invention provides novel compounds of Formula (I) and pharmaceutically acceptable salts, solvates, hydrates, tautomers, stereoisomers, isotopically labeled derivatives, and compositions thereof. Also provided are methods and kits involving the compounds or compositions for treating or preventing proliferative diseases (e.g., cancers (e.g., leukemia, melanoma, multiple myeloma), benign neoplasms, angiogenesis, inflammatory diseases, autoinflammatory diseases, and autoimmune diseases) in a subject. Treatment of a subject with a proliferative disease using a compound or composition of the invention may inhibit the aberrant activity of cyclin-dependent kinase 7 (CDK7), and therefore, induce cellular apoptosis and/or inhibit transcription in the subject.

## Description

### CLAIM OF PRIORITY

The present application claims priority to U.S. Provisional Application No. 62/361,852, filed July 13, 2016, which is incorporated herein by reference in its entirely.

### BACKGROUND OF THE INVENTION

The members of the cyclin-dependent kinase (CDK) family play critical regulatory roles in proliferation. Unique among the mammalian CDKs, CDK7 has consolidated kinase activities, regulating both the cell cycle and transcription. In the cytosol, CDK7 exists as a heterotrimeric complex and is believed to function as a CDK1/2-activating kinase (CAK), whereby phosphorylation of conserved residues in CDK1/2 by CDK7 is required for full catalytic CDK activity and cell cycle progression. In the nucleus, CDK7 forms the kinase core of the RNA polymerase (RNAP) II general transcription factor complex and is charged with phosphorylating the C-terminal domain (CTD) of RNAP II, a requisite step in gene transcriptional initiation. Together, the two functions of CDK7, *i.e*., CAK and CTD phosphorylation, support critical facets of cellular proliferation, cell cycling, and transcription.

Disruption of RNAP II CTD phosphorylation has been shown to preferentially affect proteins with short half-lives, including those of the anti-apoptotic BCL-2 family. Cancer cells have demonstrated ability to circumvent pro-cell death signaling through upregulation of BCL-2 family members. Therefore, inhibition of human CDK7 kinase activity is likely to result in anti-proliferative activity.

The discovery of selective inhibitors of CDK7 has been hampered by the high sequence and structural similarities of the kinase domain of CDK family members. Therefore, there is a need for the discovery and development of selective CDK7 inhibitors. Such CKD7 inhibitors hold promise as a therapeutic agent for the treatment of CLL and other cancers.

### BRIEF DESCRIPTION OF THE DRAWINGS

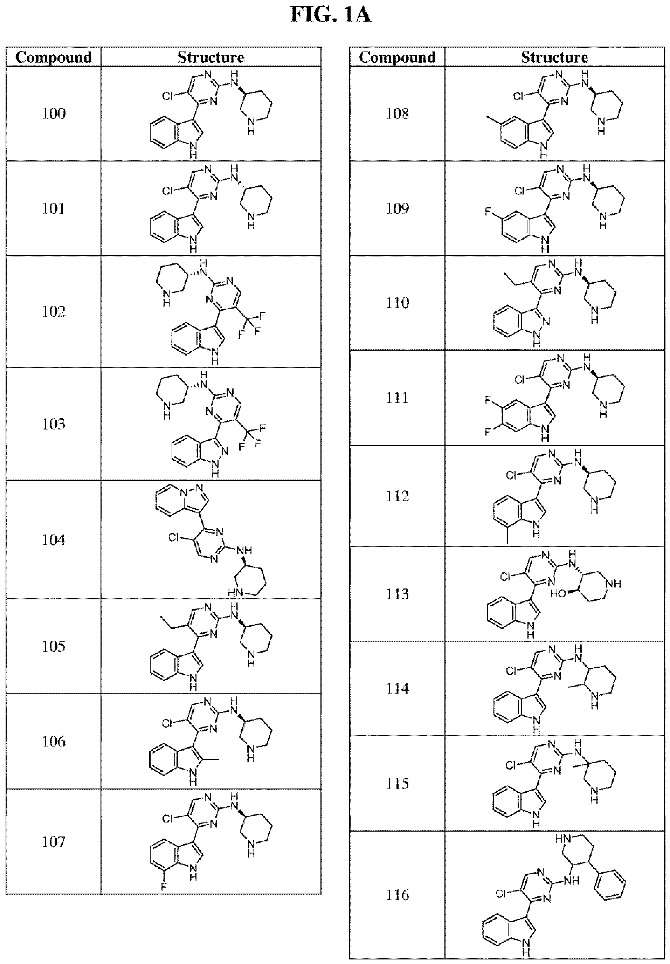

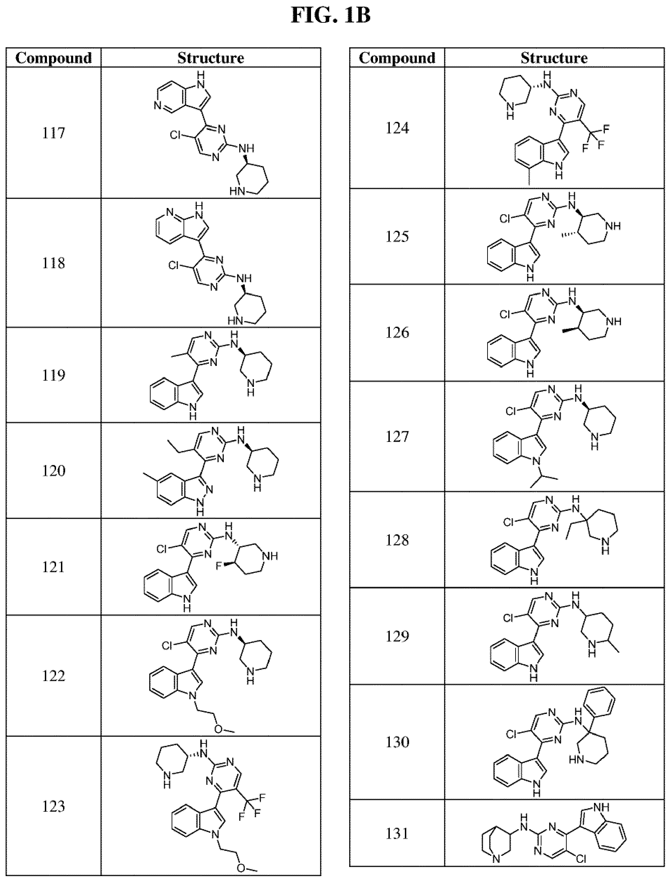

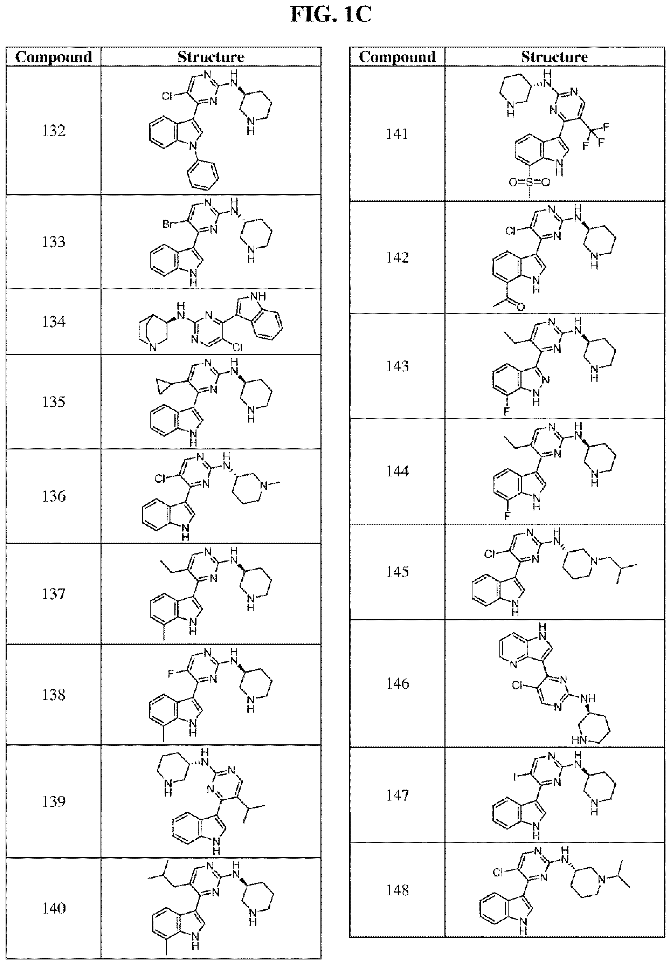

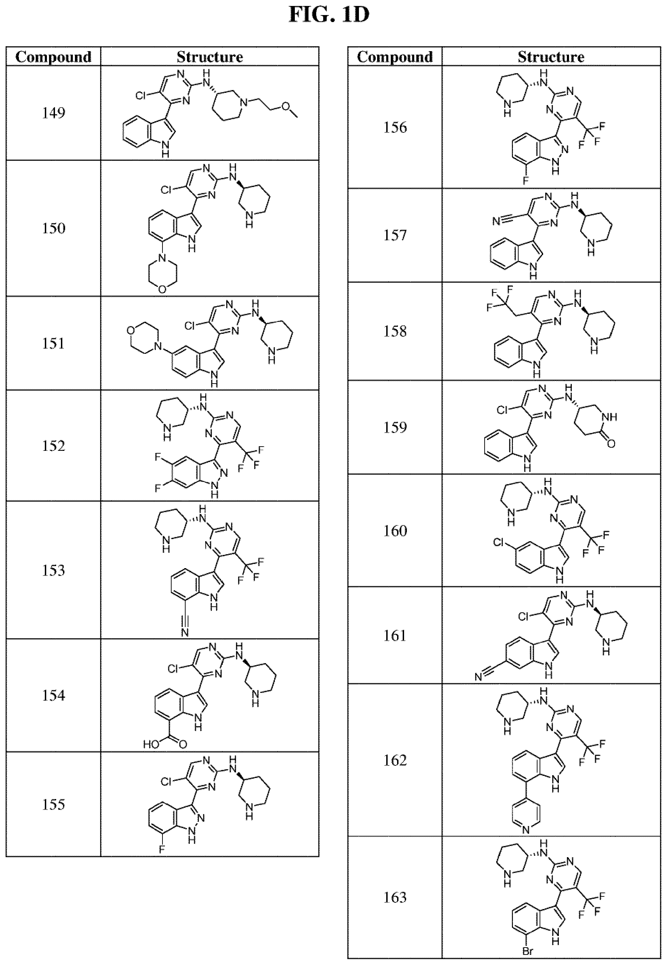

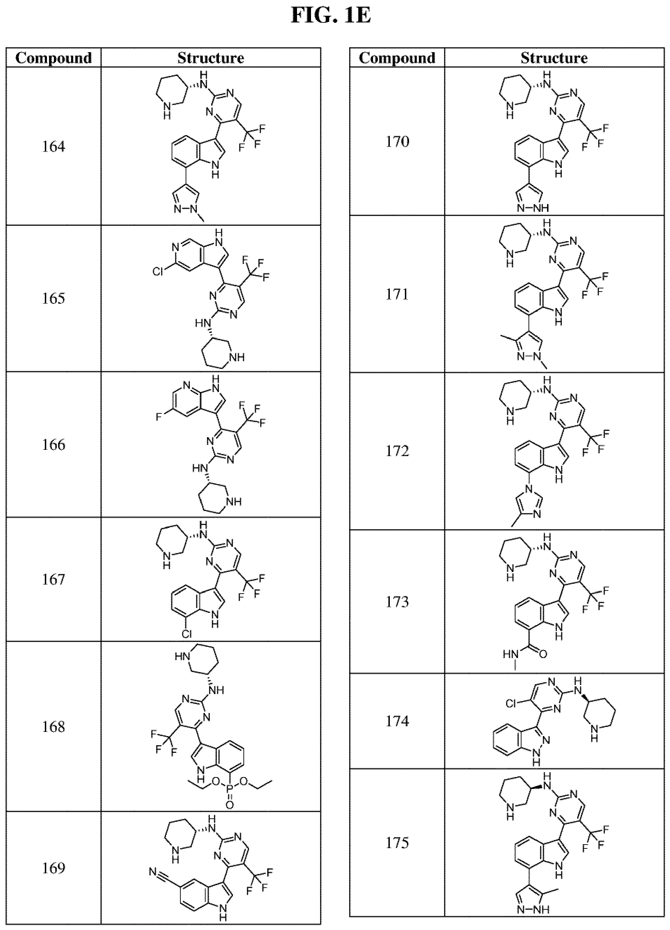

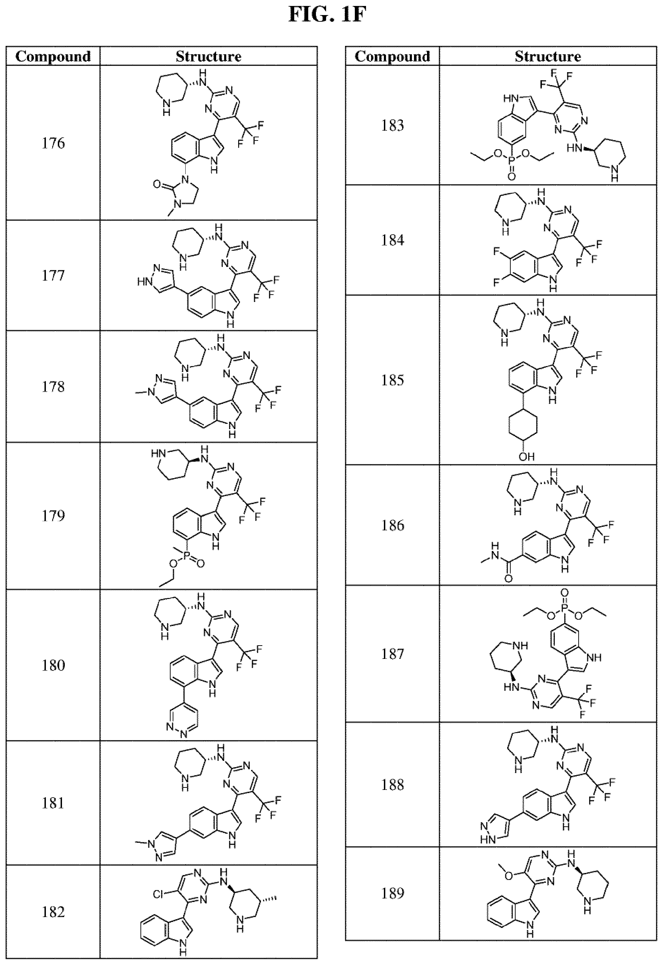

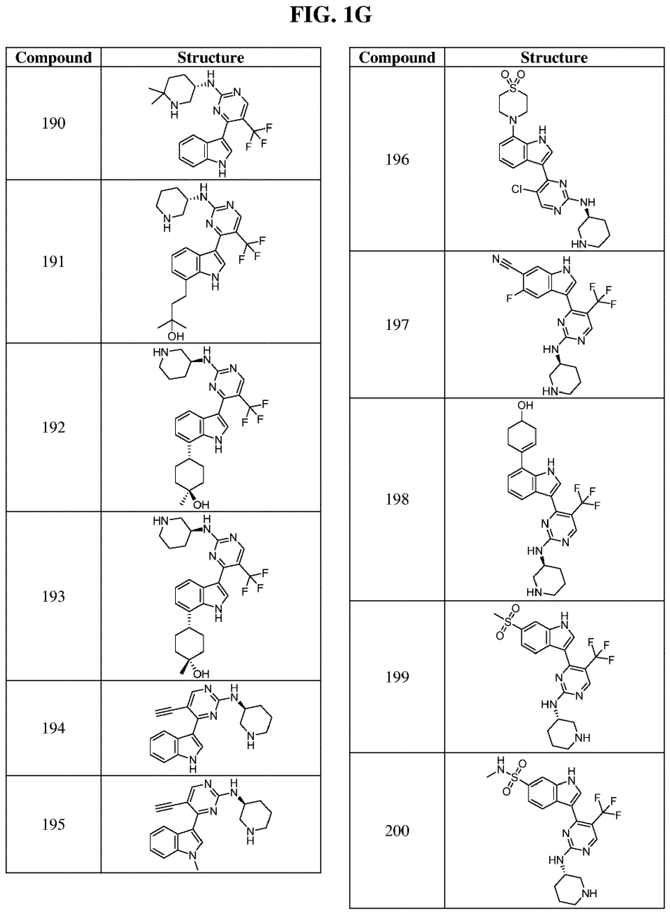

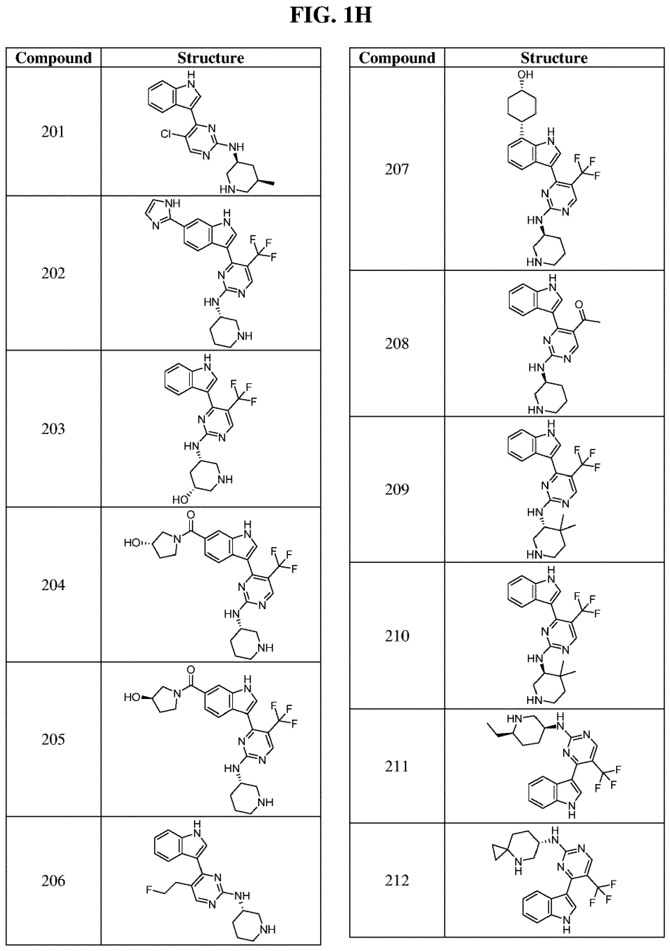

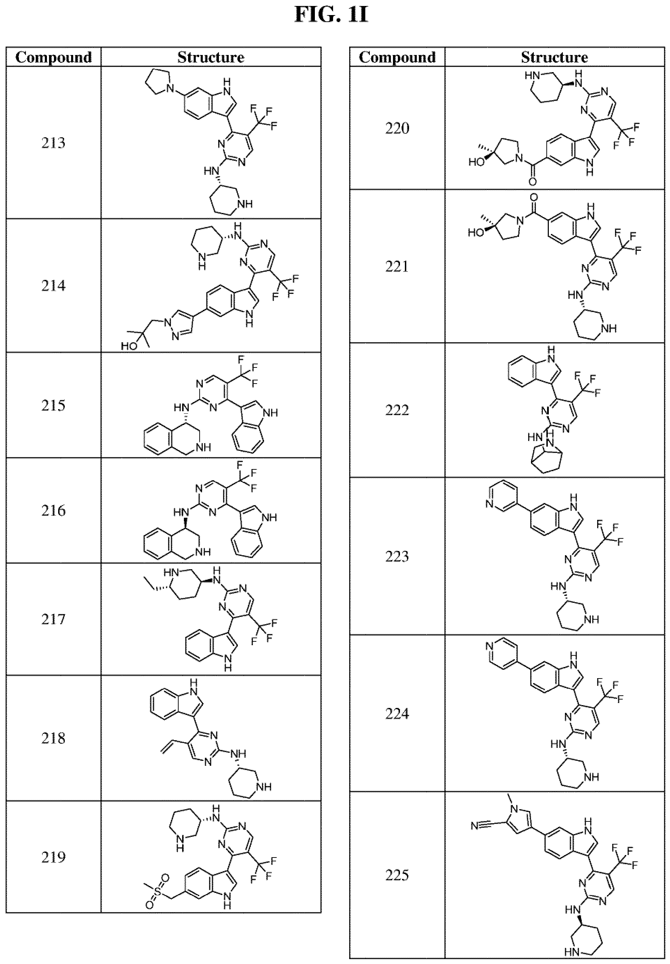

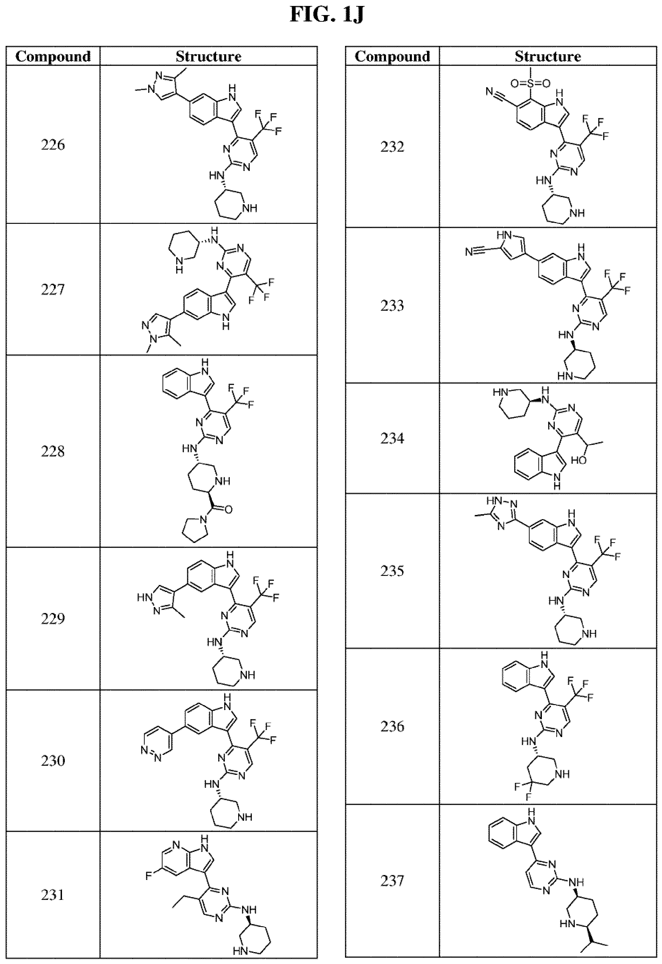

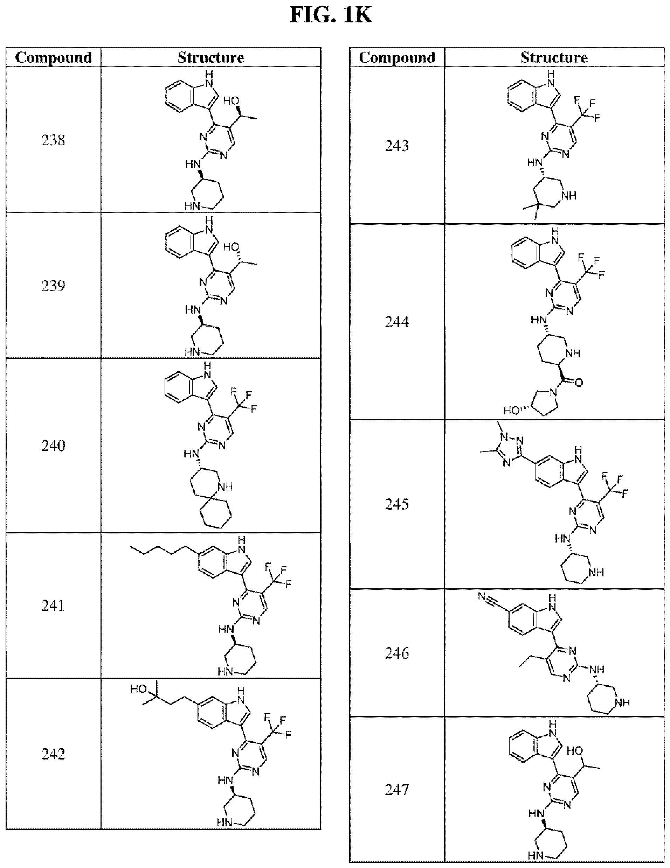

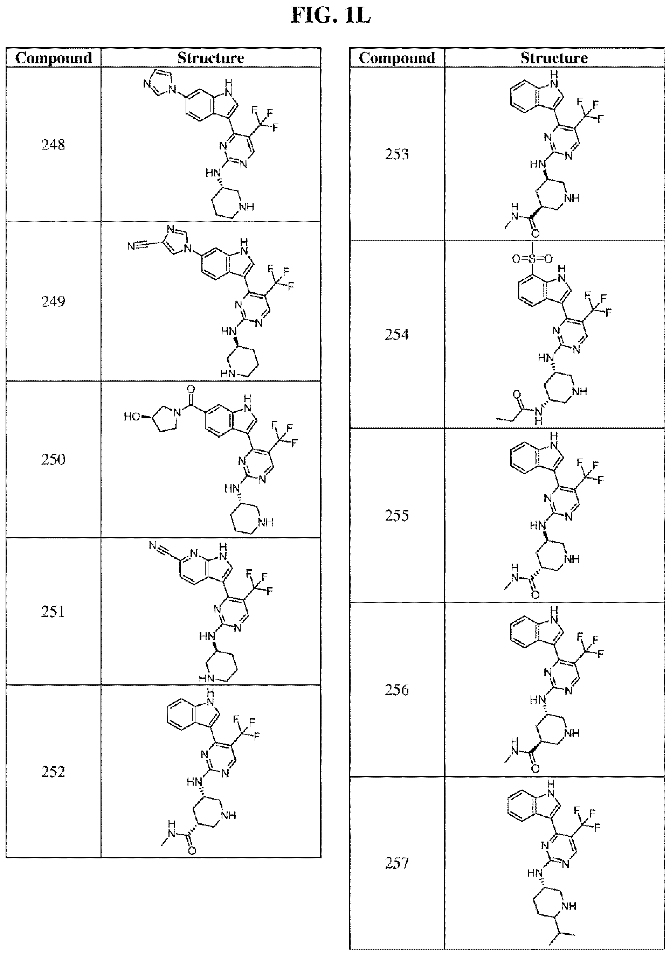

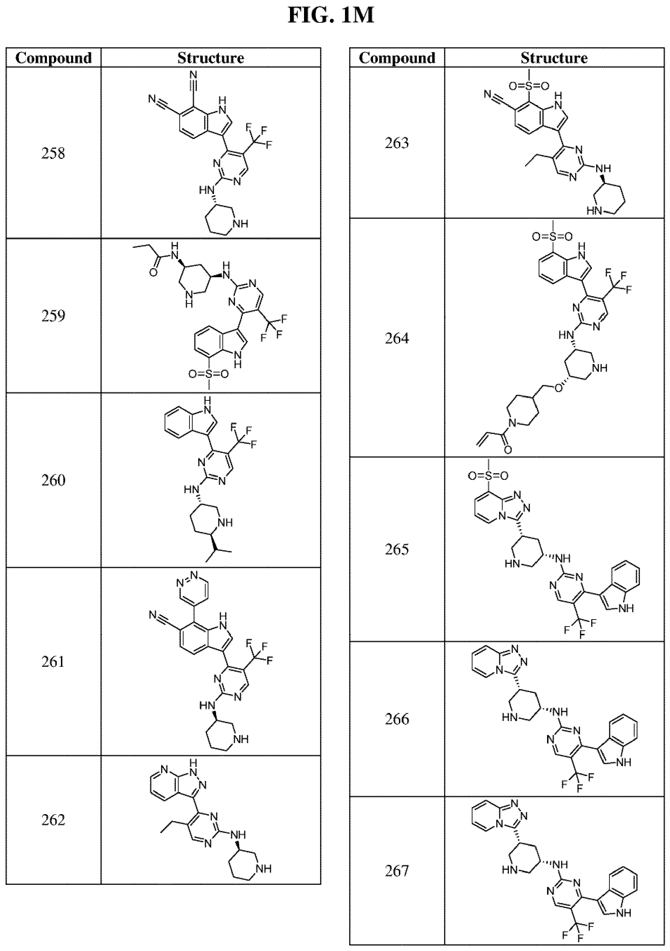

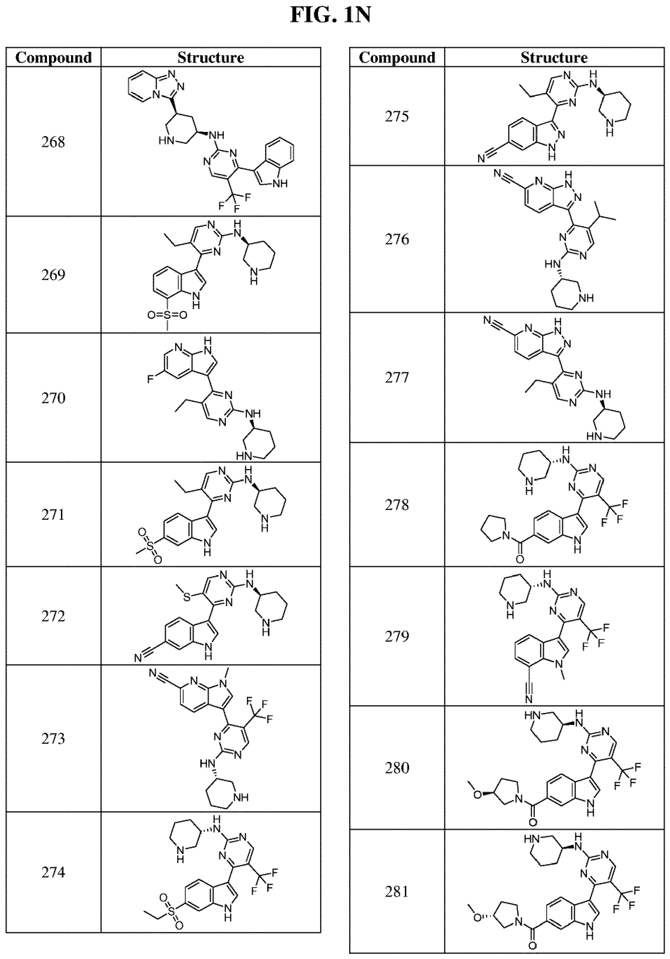

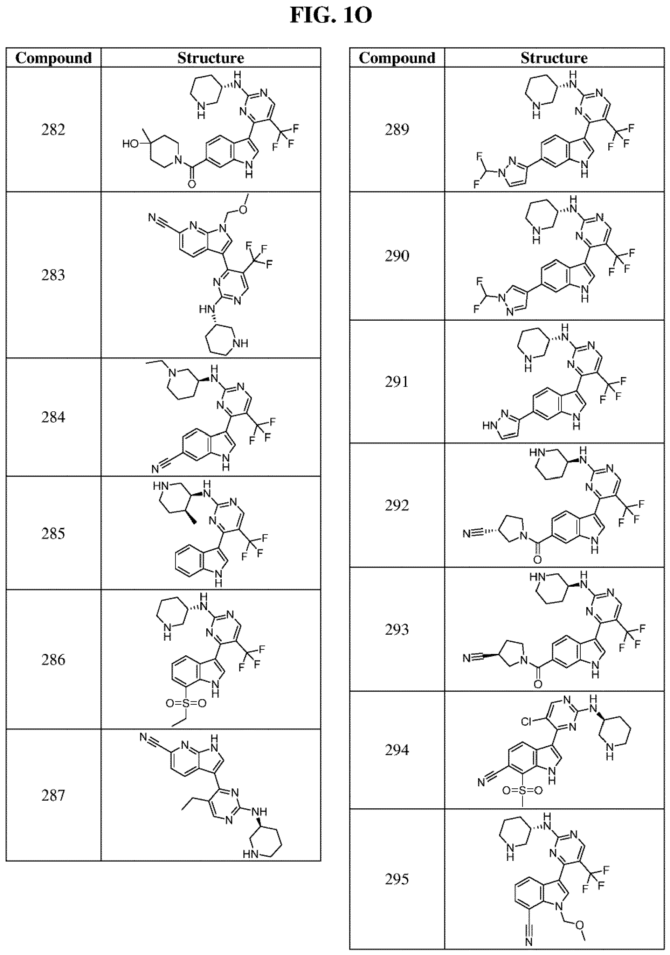

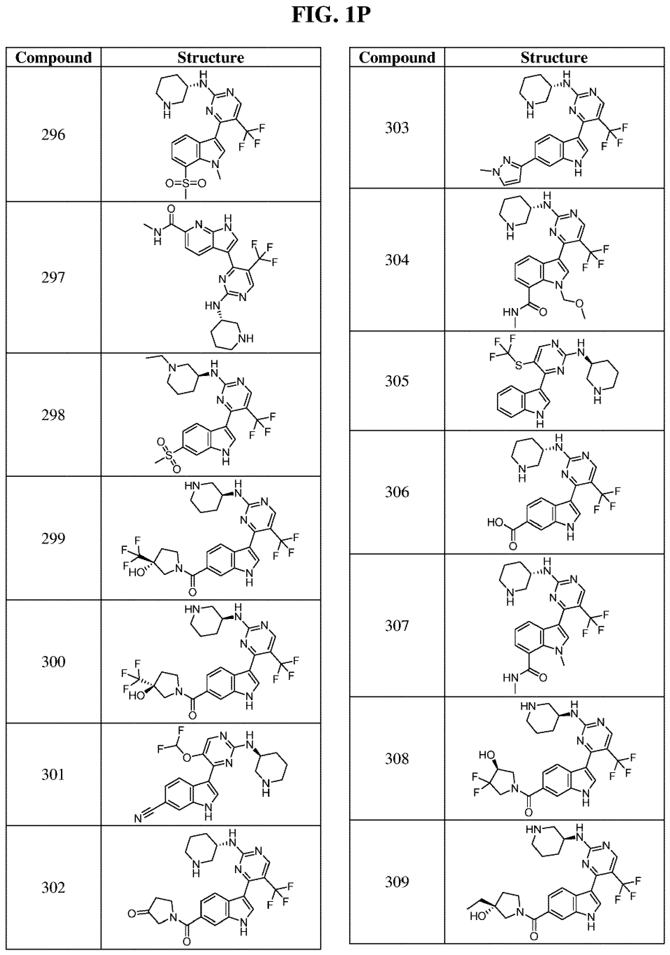

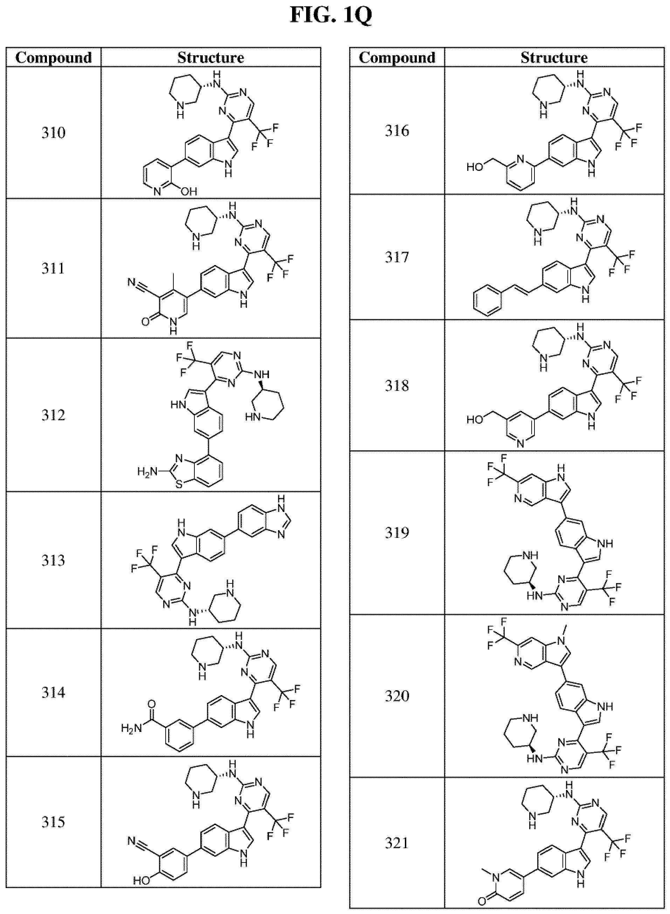

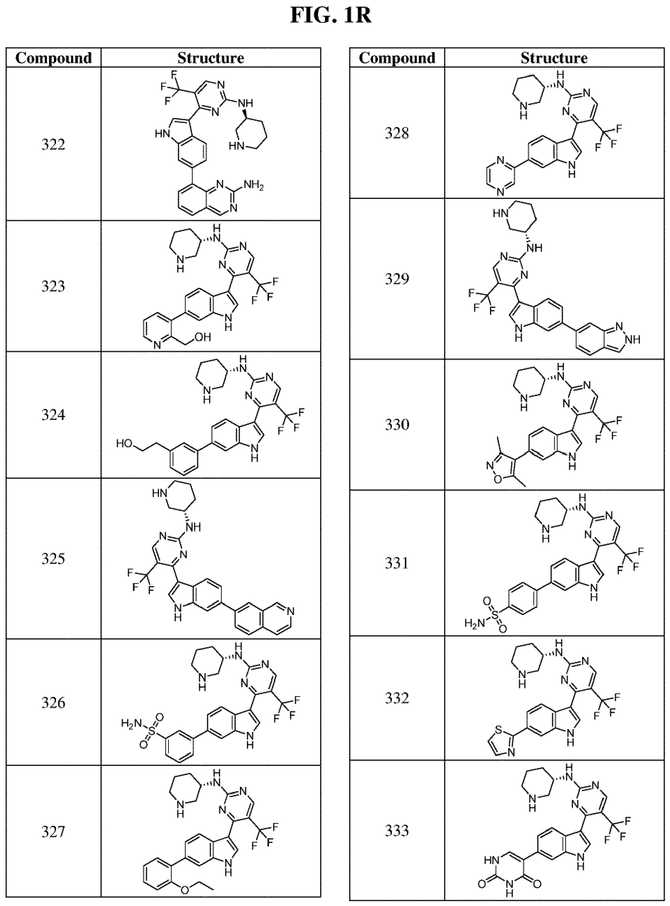

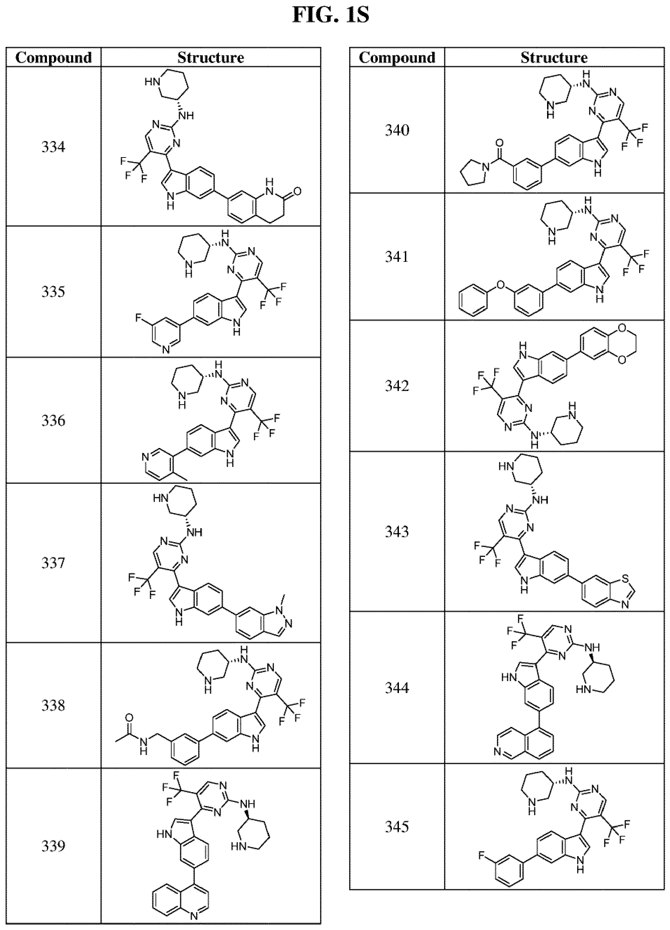

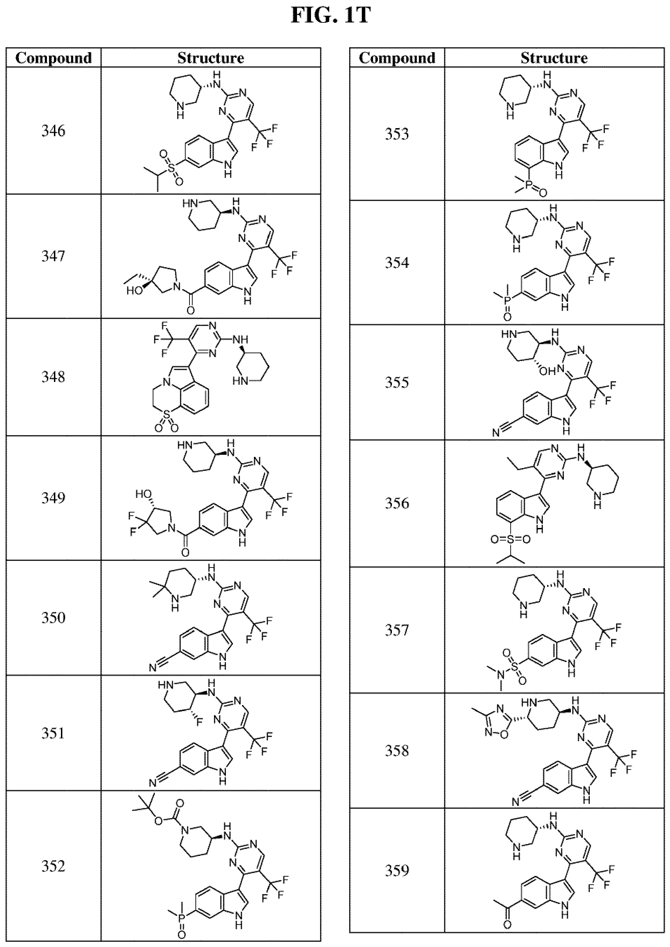

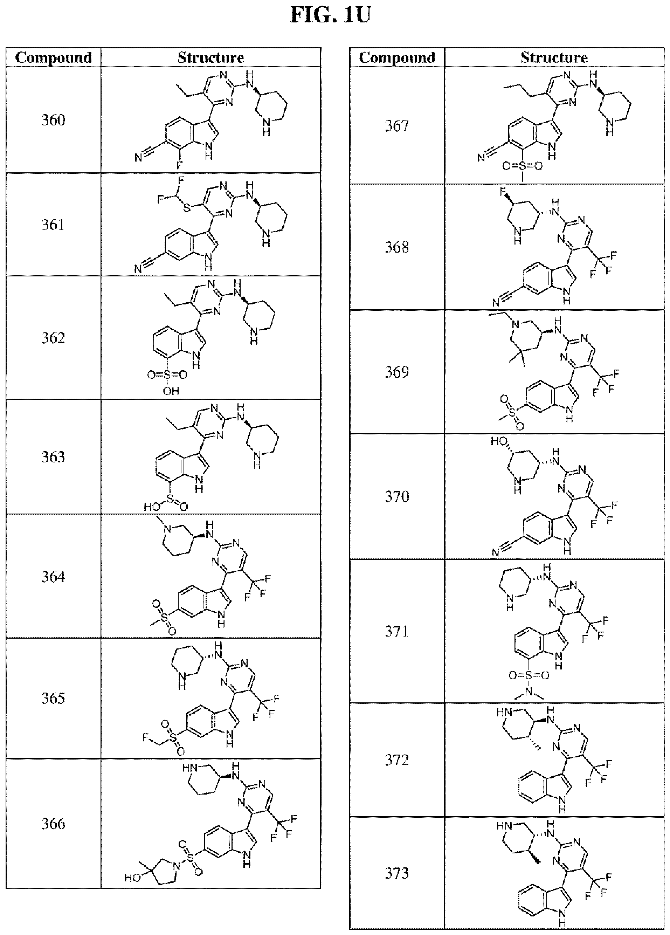

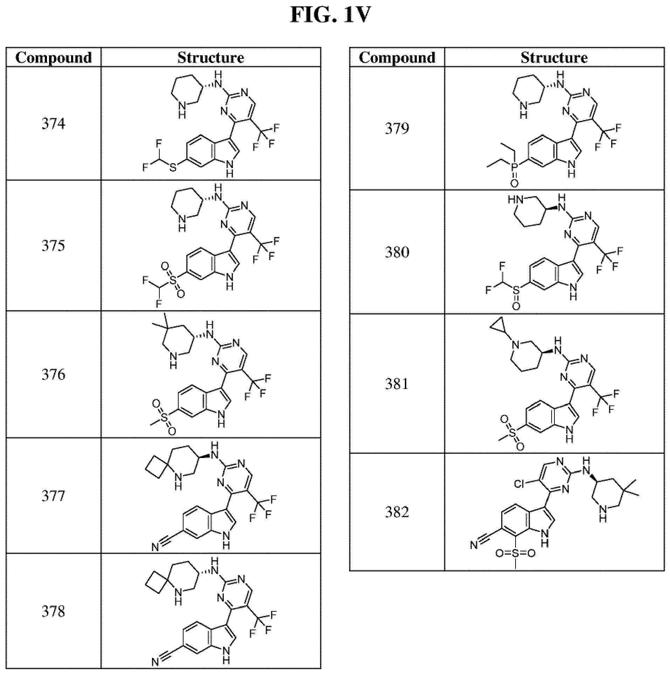

Fig. 1 is a table of exemplary compounds of formula (I).

### SUMMARY OF THE INVENTION

The present invention provides inhibitors of one or more families of kinases, e.g., serine/threonine kinases, such as one or more of the family of CDK proteins. The present invention further provides CDK7 inhibitors, in particular selective CDK7 inhibitors of Formula (I) and pharmaceutically acceptable salts, solvates, hydrates, tautomers, stereoisomers, isotopically labeled derivatives, and compositions thereof. The present invention additionally provides methods of using the compounds of the invention, and pharmaceutically acceptable salts, solvates, hydrates, tautomers, stereoisomers, and compositions thereof as therapeutics for the prevention and/or treatment of diseases associated with overexpression and/or aberrant activity of one or more of the kinases such as serine/threonine kinase family members, e.g., one or more CDK family members, e.g., CDK7 and/or CDK12 and/or CDK13. In certain embodiments, the inventive compounds are used for the prevention and/or treatment of proliferative diseases (*e.g.,* cancers (*e.g.,* leukemia, melanoma, multiple myeloma), benign neoplasms, angiogenesis, inflammatory diseases, autoinflammatory diseases, and autoimmune diseases) in a subject.

In one aspect, the present invention provides compounds of formula (I) and pharmaceutically acceptable salts, solvates, hydrates, tautomers, stereoisomers, and isotopically labeled derivatives thereof, wherein Ring A, L, R¹, R², R³, R⁴, R⁵, R⁶, m, n, and subvariables thereof are as defined herein.

In another aspect, the present invention provides pharmaceutical compositions comprising a compound of Formula (I) or a pharmaceutically acceptable salt, solvate, hydrate, tautomer, stereoisomer, or isotopically labeled derivative thereof, and optionally a pharmaceutically acceptable excipient. In certain embodiments, the pharmaceutical compositions described herein include a therapeutically effective amount of a compound of Formula (I) or a pharmaceutically acceptable salt, solvate, hydrate, tautomer, stereoisomer, or isotopically labeled derivative thereof. The pharmaceutical composition may be useful for treating and/or preventing a proliferative or infectious disease.

In another aspect, the present invention provides methods for treating and/or preventing proliferative diseases. Exemplary proliferative diseases include cancer (*e*.*g*., leukemia, melanoma, multiple myeloma), benign neoplasm, angiogenesis, inflammatory diseases, autoinflammatory diseases, and autoimmune diseases. In other embodiments, the present invention provides methods for treating and/or preventing an infectious disease (*e*.*g*., a viral infection).

In still another aspect, the present invention provides methods of down-regulating the expression of certain serine/threonine kinases, e.g., certain CDK family members, e.g., CDK7 in a biological sample or subject.

Another aspect of the invention relates to methods of inhibiting the activity of certain serine/threonine kinases, e.g., certain CDK family members, e.g., CDK7 in a biological sample or subject.

The present invention also provides methods of inhibiting cell growth in a biological sample or subject.

In still another aspect, the present invention provides methods of inducing apoptosis of a cell in a biological sample or a subject.

In yet another aspect, the present invention provides compounds of formula (I) and pharmaceutically acceptable salts, tautomers, stereoisomers, isotopically labeled derivatives, and compositions thereof, for use in the treatment of a proliferative disease in a subject.

In yet another aspect, the present invention provides compounds of formula (1) and pharmaceutically acceptable salts, solvates, hydrates, tautomers, stereoisomers, isotopically labeled derivatives, and compositions thereof, for use in the treatment or prevention of an infectious disease in a subject. In certain embodiments, the infectious disease is a viral infection.

Another aspect of the present invention relates to kits comprising a container with a compound of formula (I) or a pharmaceutically acceptable salt, solvate, hydrate, tautomer, stereoisomer, or isotopically labeled derivative thereof, or a pharmaceutical composition thereof. In certain embodiments, the kits described herein further include instructions for administering the compound of formula (I) or the pharmaceutically acceptable salt, solvate, hydrate, tautomer, stereoisomer, or isotopically labeled derivative thereof, or the pharmaceutical composition thereof.

The details of one or more embodiments of the invention are set forth herein. Other features, objects, and advantages of the invention will be apparent from the Detailed Description, the Figures, the Examples, and the Claims.

### DEFINITIONS

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Unless otherwise stated, structures depicted herein are also meant to include all isomeric (e.g., enantiomeric, diastereomeric, and geometric (or conformational)) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. Such compounds are useful, for example, as analytical tools, as probes in biological assays, or as therapeutic agents in accordance with the present invention.

Where a particular enantiomer is preferred, it may, in some embodiments be provided substantially free of the corresponding enantiomer, and may also be referred to as "optically enriched." "Optically-enriched," as used herein, means that the compound is made up of a significantly greater proportion of one enantiomer. In certain embodiments the compound is made up of at least about 90% by weight of a preferred enantiomer. In other embodiments the compound is made up of at least about 95%, 98%, or 99% by weight of a preferred enantiomer. Preferred enantiomers may be isolated from racemic mixtures by any method known to those skilled in the art, including chiral high pressure liquid chromatography (HPLC) and the formation and crystallization of chiral salts or prepared by asymmetric syntheses. See, for example, Jacques et al., Enantiomers, Racemates and Resolutions (Wiley Interscience, New York, 1981); Wilen, et al., Tetrahedron 33:2725 (1977); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972).

The term "aliphatic" or "aliphatic group", as used herein, denotes a hydrocarbon moiety that may be straight-chain (i.e., unbranched), branched, or cyclic (including fused, bridging, and spiro-fused polycyclic) and may be completely saturated or may contain one or more units of unsaturation, but which is not aromatic. Unless otherwise specified, aliphatic groups contain 1-6 carbon atoms. In some embodiments, aliphatic groups contain 1-4 carbon atoms, and in yet other embodiments aliphatic groups contain 1-3 carbon atoms. Suitable aliphatic groups include, but are not limited to, linear or branched, alkyl, alkenyl, and alkynyl groups, and hybrids thereof such as (cycloalkyl)alkyl, (cycloalkenyl)alkyl or (cycloalkyl)alkenyl.

The term "alkyl," as used herein, refers to a monovalent saturated, straight- or branched-chain hydrocarbon such as a straight or branched group of 1-12, 1-10, or 1-6 carbon atoms, referred to herein as C₁-C₁₂ alkyl, C₁-C₁₀ alkyl, and C₁-C₆ alkyl, respectively. Examples of alkyl groups include, but are not limited to, methyl, ethyl, n-propyl, isopropyl, n-butyl, iso-butyl, sec-butyl, sec-pentyl, iso-pentyl, tert-butyl, n-pentyl, neopentyl, n-hexyl, see-hexyl, and the like.

The terms "alkenyl" and "alkynyl" are art-recognized and refer to unsaturated aliphatic groups analogous in length and possible substitution to the alkyls described above, but that contain at least one double or triple bond, respectively. Exemplary alkenyl groups include, but are not limited to, -CH=CH₂ and -CH₂CH=CH₂.

The term "alkylene" refers to the diradical of an alkyl group.

The terms "alkenylene" and "alkynylene" refer to the diradicals of an alkenyl and an alkynyl group, respectively.

The term "carbocyclic ring system", as used herein, means a monocyclic, or fused, spiro-fused, and/or bridged bicyclic or polycyclic hydrocarbon ring system, wherein each ring is either completely saturated or contains one or more units of unsaturation, but where no ring is aromatic.

The term "carbocyclyl" refers to a radical of a carbocyclic ring system. Representative carbocyclyl groups include cycloalkyl groups (e.g., cyclopentyl, cyclobutyl, cyclopentyl, cyclohexyl and the like), and cycloalkenyl groups (e.g., cyclopentenyl, cyclohexenyl, cyclopentadienyl, and the like).

The term "aromatic ring system" is art-recognized and refers to a monocyclic, bicyclic or polycyclic hydrocarbon ring system, wherein at least one ring is aromatic.

The term "aryl" refers to a radical of an aromatic ring system. Representative aryl groups include fully aromatic ring systems, such as phenyl, naphthyl, and anthracenyl, and ring systems where an aromatic carbon ring is fused to one or more non-aromatic carbon rings, such as indanyl, phthalimidyl, naphthimidyl, or tetrahydronaphthyl, and the like.

The term "heteroaromatic ring system" is art-recognized and refers to monocyclic, bicyclic or polycyclic ring system wherein at least one ring is both aromatic and comprises a heteroatom; and wherein no other rings are heterocyclyl (as defined below). In certain instances, a ring which is aromatic and comprises a heteroatom contains 1, 2, 3, or 4 independently selected ring heteroatoms in such ring.

The term "heteroaryl" refers to a radical of a heteroaromatic ring system. Representative heteroaryl groups include ring systems where (i) each ring comprises a heteroatom and is aromatic, e.g., imidazolyl, oxazolyl, thiazolyl, triazolyl, pyrrolyl, furanyl, thiophenyl, pyrazolyl, pyridinyl, pyrazinyl, pyridazinyl, pyrimidinyl, indolizinyl, purinyl, naphthyridinyl, and pteridinyl; (ii) one ring is aromatic and comprises a heteroatom, and each additional ring is either aromatic or carbocyclyl, e.g., indolyl, isoindolyl, benzothienyl, benzofuranyl, dibenzofuranyl, indazolyl, benzimidazolyl, benzthiazolyl, quinolyl, isoquinolyl, cinnolinyl, phthalazinyl, quinazolinyl, quinoxalinyl, carbazolyl, acridinyl, phenazinyl, phenothiazinyl, phenoxazinyl, pyrido[2,3-b]-1,4-oxazin-3(4H)-one, 5,6,7,8-tetrahydroquinolinyl and 5,6,7,8-tetrahydroisoquinolinyl; and (iii) one ring is aromatic and is fused, spirofused or bridged to a carbocyclyl, and the aromatic ring shares a bridgehead heteroatom with the carbocyclic ring, e.g., 5,6,7,8-tetrahydroindolizinyl. In certain embodiments, the heteroaryl is a monocyclic or bicyclic ring, wherein each of said rings contains 5 or 6 ring atoms where 1, 2, 3, or 4 of said ring atoms are a heteroatom independently selected from N, O, and S.

The term "heterocyclic ring system" refers to monocyclic, or fused, spiro-fused, and/or bridged bicyclic and polycyclic ring systems where at least one ring is saturated or partially unsaturated (but not aromatic) and comprises a heteroatom. A heterocyclic ring system can be attached to its pendant group at any heteroatom or carbon atom that results in a stable structure and any of the ring atoms can be optionally substituted.

The term "heterocyclyl" refers to a radical of a heterocyclic ring system. Representative heterocyclyls include ring systems in which (i) every ring is non-aromatic and at least one ring comprises a heteroatom, e.g., tetrahydrofuranyl, tetrahydrothienyl, pyrrolidinyl, pyrrolidonyl, piperidinyl, pyrrolinyl, decahydroquinolinyl, oxazolidinyl, piperazinyl, dioxanyl, dioxolanyl, diazepinyl, oxazepinyl, thiazepinyl, morpholinyl, and quinuclidinyl; (ii) at least one ring is non-aromatic and comprises a heteroatom and at least one other ring is an aromatic carbon ring, e.g., 1,2,3,4-tetrahydroquinolinyl, 1,2,3,4-tetrahydroisoquinolinyl; and (iii) at least one ring is non-aromatic and comprises a heteroatom and at least one other ring is aromatic and comprises a heteroatom, e.g., 3,4-dihydro-1H-pyrano[4,3-c]pyridine, and 1,2,3,4-tetrahydro-2,6-naphthyridine. In certain embodiments, the heterocyclyl is a monocyclic or bicyclic ring, wherein each of said rings contains 3-7 ring atoms where 1, 2, 3, or 4 of said ring atoms are a heteroatom independently selected from N, O, and S.

The term "saturated heterocyclyl" refers to a radical of heterocyclic ring system wherein every ring is saturated, e.g., tetrahydrofuran, tetrahydro-2H-pyran, pyrrolidine, piperidine and piperazine.

"Partially unsaturated" refers to a group that includes at least one double or triple bond. A "partially unsaturated" ring system is further intended to encompass rings having multiple sites of unsaturation, but is not intended to include aromatic groups (*e.g.,* aryl or heteroaryl groups) as herein defined. Likewise, "saturated" refers to a group that does not contain a double or triple bond, *i*.*e*., contains all single bonds.

As described herein, compounds of the invention may contain "optionally substituted" moieties. In general, the term "substituted", whether preceded by the term "optionally" or not, means that one or more hydrogens of the designated moiety are replaced with a suitable substituent. Unless otherwise indicated, an "optionally substituted" group may have a suitable substituent at each substitutable position of the group, and when more than one position in any given structure may be substituted with more than one substituent selected from a specified group, the substituent may be either the same or different at each position. Combinations of substituents envisioned under this invention are preferably those that result in the formation of stable or chemically feasible compounds. The term "stable", as used herein, refers to compounds that are not substantially altered when subjected to conditions to allow for their production, detection, and, in certain embodiments, their recovery, purification, and use for one or more of the purposes disclosed herein.

Suitable monovalent substituents on a substitutable carbon atom of an "optionally substituted" group (such as an alkyl, alkenyl, alkynyl, alkylene, alkenylene, alkynylene or the carbon atom of a carbocyclyl, aryl, heterocyclyl or heteroaryl) are independently selected from deuterium; halo; -NO₂; -CN; -N_{3;} -(CH₂)₀₋₄R°; -(CH₂)₀₋₄OR°; -O-(CH₂)₀₋₄C(O)OR°; -(CH₂)₀₋₄SR°; -(CH₂)₀₋₄N(R°)₂; -(CH₂)₀₋₄ C(O)N(R°)₂; -(CH₂)₀₋₄N(R°)C(O)R°; -(CH₂)₀₋₄N(R°)C(O)NR°₂; -(CH₂)₀₋₄N(R)C(O)OR°; -N(R°)N(R°)C(O) R°; - N(R°)N(R°)C(O)NR°₂; -(CH₂)₀₋₄C(O)R°; -(CH₂)₀₋₄C(O)OR°; -(CH₂)₀₋₄-C(O)-N(R°)-S(O)₂-R°; -(CH₂)₀₋₄OC(O)R; -(CH₂)₀₋₄C(O)NR°₂; -(CH₂)₀₋₄OC(O)NR°₂; -C(O)N(OR°)R°; -C(NOR°)R°; -C(NOR°)NR°₂; - C(NCN)NR°₂; -(CH₂)₀₋₄(O))₂R°; -S(O)₂NR°₂; -(CH₂)₀₋₄S(O)R°; -N(R°)S(O)₂NR°₂; -N(R°)S(O)R°; -C(NR°)NR°₂; -P(O)(OR°)₂; -P(O)(OR°)R°; -P(O)R°₂; -OP(O)(OR°)₂; -QP(O)(OR°)R°; -OP(O)R°₂; -SiR°₃; wherein each R° is optionally substituted as defined below and is independently hydrogen, deuterium, C₁₋₆ aliphatic, or a 5-6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), may form a 3-7-membered heterocyclic ring having 1-3 heteroatoms independently selected from nitrogen, oxygen, sulfur or phosphorous.

Suitable monovalent substituents on R° (or the ring formed by taking two independent occurrences of R° together with their intervening atoms), are independently selected from halo, - CN, -NO₂, -N₃, -R^{•}, -(CH₂)₀₋₂OH, -(CH₂)₀₋₂OR^{•}, -(CH₂)₀₋₂C(O)R^{•}, -(CH₂)₀₋₂C(O)OH, -(CH₂)₀₋₂C(O)OR^{•}, -(CH₂)_{0-2N}H₂, -(CH₂)₀₋₂NHR^{•}, and -(CH2)₀₋₂NR^{•}₂, wherein each R^{•} is independently selected from C₁₋₄ aliphatic or a 5-6-membered saturated, partially unsaturated, or aryl ring having 0-4 heteroatoms independently selected from nitrogen, oxygen, or sulfur, and wherein each R^{•} is optionally substituted with one or more independently selected halogens.

Suitable divalent substituents on a saturated carbon atom of an "optionally substituted" group include the following: =O, =NN(R*)₂, =NNR*S(O)₂R*, =NR*, =NOR*, and =NCN; wherein each independent occurrence of R* is selected from hydrogen, deuterium, C₁₋₆ aliphatic or a 5-6-membered saturated, partially unsaturated, or aryl ring having 0-4 heteroatoms independently selected from nitrogen, oxygen or sulfur, and, notwithstanding the above, two independent occurrences of R*, taken together with their intervening atom(s), may form a 3-7-membered heterocyclic ring having 1-2 heteroatoms independently selected from nitrogen or sulfur, wherein each R* is optionally substituted with one or more substituents selected from deuterium, halo, -R^{•}, -OH, -OR^{•}, rCN, -C(O)OH, -C(O)OR^{•}, -NH_{2,} -NHR^{•}, -NR^{•}₂, and NO₂.

Suitable substituents on a substitutable nitrogen of an "optionally substituted" group include -R^{†}, -N(R^{†})₂, -C(O)R^{†}, -C(O)OR^{†}, -C(Q)N(R^{†})₂ -S(O)₂R^{†}, -S(O)₂N(R^{†})₂ -N(R)S(O)2R^{†}, - C(NR^{†})N(R^{†})₂,-C(NOR^{†})N(R^{†})₂, or -C(NCN)N)R^{†})₂; wherein each R^{†} is independently hydrogen, a C₁₋₆ aliphatic group, or a 5-7-membered saturated, partially unsaturated, or aryl ring having 0-4 heteroatoms independently selected from nitrogen, oxygen, or sulfur; or notwithstanding the definition above, two independent occurrences of R^{†}, taken together with their intervening atom(s), may form a 3-7-membered saturated, partially unsaturated, or aryl heterocyclyl having 1-3 heteroams independently selected from nitrogen, oxygen or sulfur, wherein each R^{†} is optionally and independently substituted with one or more substituents independently selected from deuterium, halo, -R^{•}, -OH, -OR^{•}, -CN, -NH₂, -NHR^{•}, -NR^{•}₂, and - NO₂.

"Halo" or "halogen" refers to fluorine (fluoro, -F), chlorine (chloro, -Cl), bromine (bromo, -Br), or iodine (iodo, -I).

These and other exemplary substituents are described in more detail in the Detailed Description, Figures, Examples, and Claims. The invention is not intended to be limited in any manner by the above exemplary listing of substituents.

### Other definitions

The following definitions are more general terms used throughout the present application:

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid or by using other methods known in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N+(C₁₋₄alkyl)₄⁻ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

The term "solvate" refers to forms of the compound that are associated with a solvent, usually by a solvolysis reaction. This physical association may include hydrogen bonding. Conventional solvents include water, methanol, ethanol, acetic acid, DMSO, THF, diethyl ether, and the like. The compounds of formula (I) may be prepared, e.g., in crystalline form, and may be solvated. Suitable solvates include pharmaceutically acceptable solvates and further include both stoichiometric solvates and non-stoichiometric solvates. In certain instances, the solvate will be capable of isolation, for example, when one or more solvent molecules are incorporated in the crystal lattice of a crystalline solid. "Solvate" encompasses both solution-phase and isolable solvates. Representative solvates include hydrates, ethanolates, and methanolates.

The term "hydrate" refers to a compound which is associated with water. Typically, the number of the water molecules contained in a hydrate of a compound is in a definite ratio to the number of the compound molecules in the hydrate. Therefore, a hydrate of a compound may be represented, for example, by the general formula R-x H₂O, wherein R is the compound and wherein x is a number greater than 0. A given compound may form more than one type of hydrates, including, e.g., monohydrates (x is 1), lower hydrates (x is a number greater than 0 and smaller than 1, e.g., hemihydrates (R·0.5 H₂O)), and polyhydrates (x is a number greater than 1, e.g., dihydrates (R·2 H₂O) and hexahydrates (R·6 H₂O)).

The term "tautomers" refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci- and nitro- forms of phenylnitromethane that are likewise formed by treatment with acid or base.

Tautomeric forms may be relevant to the attainment of the optimal chemical reactivity and biological activity of a compound of interest.

It is also to be understood that compounds that have the same molecular formula but differ in the nature or sequence of bonding of their atoms or the arrangement of their atoms in space are termed "isomers". Isomers that differ in the arrangement of their atoms in space are termed "stereoisomers".

Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) of (-)-isomers respectively). A chiral compound can exist as either individual enantiomer or as a mixture thereof. A mixture containing equal proportions of the enantiomers is called a "racemic mixture".

A "subject" to which administration is contemplated includes, but is not limited to, humans (*i.e*., a male or female of any age group, *e.g.,* a pediatric subject (*e.g.,* infant, child, adolescent) or adult subject (*e*.*g*., young adult, middle-aged adult, or senior adult)) and/or other non-human animals, for example, mammals (*e*.*g*., primates (*e*.*g*., cynomolgus monkeys, rhesus monkeys); commercially relevant mammals such as cattle, pigs, horses, sheep, goats, cats, and/or dogs) and birds (*e*.*g*., commercially relevant birds such as chickens, ducks, geese, and/or turkeys). In certain embodiments, the animal is a mammal. The animal may be a male or female and at any stage of development A non-human animal may be a transgenic animal.

The terms "administer," "administering," or "administration," as used herein refers to implanting, absorbing, ingesting, injecting, inhaling, or otherwise introducing an inventive compound, or a pharmaceutical composition thereof.

As used herein, the terms "treatment," "treat," and "treating" refer to reversing, alleviating, delaying the onset of, or inhibiting the progress of a "pathological condition" (*e*.*g*., a disease, disorder, or condition, or one or more signs or symptoms thereof) described herein. In some embodiments, "treatment," "treat," and "treating" require that signs or symptoms of the disease disorder or condition have developed or have been observed. In other embodiments, treatment may be administered in the absence of signs or symptoms of the disease or condition. For example, treatment may be administered to a susceptible individual prior to the onset of symptoms (*e.g.,* in light of a history of symptoms and/or in light of genetic or other susceptibility factors). Treatment may also be continued after symptoms have resolved, for example, to delay or prevent recurrence.

As used herein, the terms "condition," "disease," and "disorder" are used interchangeably.

An "effective amount" of a compound of formula (I) refers to an amount sufficient to elicit the desired biological response, *i.e*., treating the condition. As will be appreciated by those of ordinary skill in this art, the effective amount of a compound of formula (I) may vary depending on such factors as the desired biological endpoint, the pharmacokinetics of the compound, the condition being treated, the mode of administration, and the age and health of the subject. An effective amount encompasses therapeutic and prophylactic treatment For example, in treating cancer, an effective amount of an inventive compound may reduce the tumor burden or stop the growth or spread of a tumor.

A "therapeutically effective amount" of a compound of formula (I) is an amount sufficient to provide a therapeutic benefit in the treatment of a condition or to delay or minimize one or more symptoms associated with the condition. In some embodiments, a therapeutically effective amount is an amount sufficient to provide a therapeutic benefit in the treatment of a condition or to minimize one or more symptoms associated with the condition. A therapeutically effective amount of a compound means an amount of therapeutic agent, alone or in combination with other therapies, which provides a therapeutic benefit in the treatment of the condition. The term "therapeutically effective amount" can encompass an amount that improves overall therapy, reduces or avoids symptoms or causes of the condition, or enhances the therapeutic efficacy of another therapeutic agent.

A "prophylactically effective amount" of a compound of formula (I) is an amount sufficient to prevent a condition, or one or more symptoms associated with the condition or prevent its recurrence. A prophylactically effective amount of a compound means an amount of a therapeutic agent, alone or in combination with other agents, which provides a prophylactic benefit in the prevention of the condition. The term "prophylactically effective amount" can encompass an amount that improves overall prophylaxis or enhances the prophylactic efficacy of another prophylactic agent.

A "proliferative disease" refers to a disease that occurs due to abnormal growth or extension by the multiplication of cells (Walker, Cambridge Dictionary of Biology; Cambridge University Press: Cambridge, UK, 1990). A proliferative disease may be associated with: 1) the pathological proliferation of normally quiescent cells; 2) the pathological migration of cells from their normal location (*e.g.,* metastasis of neoplastic cells); 3) the pathological expression of proteolytic enzymes such as the matrix metalloproteinases (*e.g.,* collagenases, gelatinases, and elastases); or 4) the pathological angiogenesis as in proliferative retinopathy and tumor metastasis. Exemplary proliferative diseases include cancers (*i.e*., "malignant neoplasms"), benign neoplasms, angiogenesis, inflammatory diseases, autoinflammatory diseases, and autoimmune diseases.

The terms "neoplasm" and "tumor" are used herein interchangeably and refer to an abnormal mass of tissue wherein the growth of the mass surpasses and is not coordinated with the growth of a normal tissue. A neoplasm or tumor may be "benign" or "malignant," depending on the following characteristics: degree of cellular differentiation (including morphology and functionality), rate of growth, local invasion, and metastasis. A "benign neoplasm" is generally well differentiated, has characteristically slower growth than a malignant neoplasm, and remains localized to the site of origin. In addition, a benign neoplasm does not have the capacity to infiltrate, invade, or metastasize to distant sites. Exemplary benign neoplasms include, but are not limited to, lipoma, chondroma, adenomas, acrochordon, senile angiomas, seborrheic keratoses, lentigos, and sebaceous hyperplasias. In some cases, certain "benign" tumors may later give rise to malignant neoplasms, which may result from additional genetic changes in a subpopulation of the tumor's neoplastic cells, and these tumors are referred to as "pre-malignant neoplasms." An exemplary pre-malignant neoplasm is a teratoma. In contrast, a "malignant neoplasm" is generally poorly differentiated (anaplasia) and has characteristically rapid growth accompanied by progressive infiltration, invasion, and destruction of the surrounding tissue. Furthermore, a malignant neoplasm generally has the capacity to metastasize to distant sites.

As used herein, the term "cancer" refers to a malignant neoplasm (Stedman's Medical Dictionary, 25th ed.; Hensyl ed.; Williams & Wilkins: Philadelphia, 1990). Exemplary cancers include, but are not limited to, acoustic neuroma; adenocarcinoma; adrenal gland cancer, anal cancer; angiosarcoma (*e*.*g*., lymphangiosarcoma, lymphangioendotheliosarcoma, hemangiosarcoma); appendix cancer, benign monoclonal gammopathy; biliary cancer (*e*.*g*., cholangiocarcinoma); bladder cancer, breast cancer (*e*.*g*., adenocarcinoma of the breast, papillary carcinoma of the breast, mammary cancer, medullary carcinoma of the breast); brain cancer (*e*.*g*., meningioma, glioblastomas, glioma (*e*.*g*., astrocytoma, oligodendroglioma), medulloblastoma); bronchus cancer, carcinoid tumor; cervical cancer (*e*.*g*., cervical adenocarcinoma); choriocarcinoma; chordoma; craniopharyngioma; colorectal cancer (*e*.*g*., colon cancer, rectal cancer, colorectal adenocarcinoma); connective tissue cancer; epithelial carcinoma; ependymoma; endotheliosarcoma (*e*.*g*., Kaposi's sarcoma, multiple idiopathic hemorrhagic sarcoma); endometrial cancer (*e.g.,* uterine cancer, uterine sarcoma); esophageal cancer (*e.g.,* adenocarcinoma of the esophagus, Barrett's adenocarcinoma); Ewing's sarcoma; eye cancer (*e*.*g*., intraocular melanoma, retinoblastoma); familiar hypereosinophilia; gall bladder cancer, gastric cancer (*e*.*g*., stomach adenocarcinoma); gastrointestinal stromal tumor (GIST); germ cell cancer, head and neck cancer (*e.g.,* head and neck squamous cell carcinoma, oral cancer (*e.g.,* oral squamous cell carcinoma), throat cancer (*e*.*g*., laryngeal cancer, pharyngeal cancer, nasopharyngeal cancer, oropharyngeal cancer)); hematopoietic cancers (*e.g.,* leukemia such as acute lymphocytic leukemia (ALL) (*e.g.,* B-cell ALL, T-cell ALL), acute myelocytic leukemia (AML) (*e.g.,* B-cell AML, T-cell AML), chronic myelocytic leukemia (CML) (*e.g.,* B-cell CML, T-cell CML), and chronic lymphocytic leukemia (CLL) (*e*.*g*., B-cell CLL, T-cell CLL)); lymphoma such as Hodgkin lymphoma (HL) (*e.g.,* B-cell HL, T-cell HL) and non-Hodgkin lymphoma (NHL) (*e.g.,* B-cell NHL such as diffuse large cell lymphoma (DLCL) (*e*.*g*., diffuse large B-cell lymphoma), follicular lymphoma, chronic lymphocytic leukemia/small lymphocytic lymphoma (CLL/SLL), mantle cell lymphoma (MCL), marginal zone B-cell lymphomas (*e*.*g*., mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (*i.e*., Waldenstrom's macroglobulinemia), hairy cell leukemia (HCL), immunoblastic large cell lymphoma, precursor B-lymphoblastic lymphoma and primary central nervous system (CNS) lymphoma; and T-cell NHL such as precursor T-lymphoblastic lymphoma/leukemia, peripheral T-cell lymphoma (PTCL) (*e.g.,* cutaneous T-cell lymphoma (CTCL) (*e*.*g*., mycosis fungoides, Sezary syndrome), angioimmunoblastic T-cell lymphoma, extranodal natural killer T-cell lymphoma, enteropathy type T-cell lymphoma, subcutaneous panniculitis-like T-cell lymphoma, and anaplastic large cell lymphoma); a mixture of one or more leukemia/lymphoma as described above; and multiple myeloma (MM)), heavy chain disease (*e*.*g*., alpha chain disease, gamma chain disease, mu chain disease); hemangioblastoma; hypopharynx cancer; inflammatory myofibroblastic tumors; immunocytic amyloidosis; kidney cancer (*e*.*g*., nephroblastoma *a.k.a.* Wilms' tumor, renal cell carcinoma); liver cancer (*e*.*g*., hepatocellular cancer (HCC), malignant hepatoma); lung cancer (*e.g.,* bronchogenic carcinoma, small cell lung cancer (SCLC), non-small cell lung cancer (NSCLC), adenocarcinoma of the lung); leiomyosarcoma (LMS); mastocytosis (*e*.*g*., systemic mastocytosis); muscle cancer, myelodysplastic syndrome (MDS); mesothelioma; myeloproliferative disorder (MPD) (*e*.*g*., polycythemia vera (PV), essential thrombocytosis (ET), agnogenic myeloid metaplasia (AMM) *a.k.a*. myelofibrosis (MF), chronic idiopathic myelofibrosis, chronic myelocytic leukemia (CML), chronic neutrophilic leukemia (CNL), hypereosinophilic syndrome (HES)); neuroblastoma; neurofibroma (*e.g.,* neurofibromatosis (NF) type 1 or type 2, schwannomatosis); neuroendocrine cancer (*e*.*g*., gastroenteropancreatic neuroendocrine tumor (GEP-NET), carcinoid tumor); osteosarcoma (*e*.*g*., bone cancer); ovarian cancer (*e*.*g*., cystadenocarcinoma, ovarian embryonal carcinoma, ovarian adenocarcinoma); papillary adenocarcinoma; pancreatic cancer (*e*.*g*., pancreatic adenocarcinoma, intraductal papillary mucinous neoplasm (IPMN), Islet cell tumors); penile cancer (*e.g.,* Paget's disease of the penis and scrotum); pinealoma; primitive neuroectodermal tumor (PNT); plasma cell neoplasia; paraneoplastic syndromes; intraepithelial neoplasms; prostate cancer (*e.g.,* prostate adenocarcinoma); rectal cancer; rhabdomyosarcoma; salivary gland cancer; skin cancer (*e*.*g*., squamous cell carcinoma (SCC), keratoacanthoma (KA), melanoma, basal cell carcinoma (BCC)); small bowel cancer (*e.g.,* appendix cancer); soft tissue sarcoma (*e.g.,* malignant fibrous histiocytoma (MFH), liposarcoma, malignant peripheral nerve sheath tumor (MPNST), chondrosarcoma, fibrosarcoma, myxosarcoma); sebaceous gland carcinoma; small intestine cancer; sweat gland carcinoma; synovioma; testicular cancer (*e.g.,* seminoma, testicular embryonal carcinoma); thyroid cancer (*e*.*g*., papillary carcinoma of the thyroid, papillary thyroid carcinoma (PTC), medullary thyroid cancer); urethral cancer; vaginal cancer; and vulvar cancer (*e.g.,* Paget's disease of the vulva).

The term "angiogenesis" refers to the formation and the growth of new blood vessels. Normal angiogenesis occurs in the healthy body of a subject for healing wounds and for restoring blood flow to tissues after injury. The healthy body controls angiogenesis through a number of means, *e*.*g*., angiogenesis-stimulating growth factors and angiogenesis inhibitors. Many disease states, such as cancer, diabetic blindness, age-related macular degeneration, rheumatoid arthritis, and psoriasis, are characterized by abnormal (*i*.*e*., increased or excessive) angiogenesis. Abnormal angiogenesis refers to angiogenesis greater than that in a normal body, especially angiogenesis in an adult not related to normal angiogenesis (*e.g.,* menstruation or wound healing). Abnormal angiogenesis can provide new blood vessels that feed diseased tissues and/or destroy normal tissues, and in the case of cancer, the new vessels can allow tumor cells to escape into the circulation and lodge in other organs (tumor metastases).

As used herein, an "inflammatory disease" refers to a disease caused by, resulting from, or resulting in inflammation. The term "inflammatory disease" may also refer to a dysregulated inflammatory reaction that causes an exaggerated response by macrophages, granulocytes, and/or T-lymphocytes leading to abnormal tissue damage and/or cell death. An inflammatory disease can be either an acute or chronic inflammatory condition and can result from infections or non-infectious causes. Inflammatory diseases include, without limitation, atherosclerosis, arteriosclerosis, autoimmune disorders, multiple sclerosis, systemic lupus erythematosus, polymyalgia rheumatica (PMR), gouty arthritis, degenerative arthritis, tendonitis, bursitis, psoriasis, cystic fibrosis, arthrosteitis, rheumatoid arthritis, inflammatory arthritis, Sjogren's syndrome, giant cell arteritis, progressive systemic sclerosis (scleroderma), ankylosing spondylitis, polymyositis, dermatomyositis, pemphigus, pemphigoid, diabetes (*e*.*g*., Type I), myasthenia gravis, Hashimoto's thyroiditis, Graves' disease, Goodpasture's disease, mixed connective tissue disease, sclerosing cholangitis, inflammatory bowel disease, Crohn's disease, ulcerative colitis, pernicious anemia, inflammatory dermatoses, usual interstitial pneumonitis (UIP), asbestosis, silicosis, bronchiectasis, berylliosis, talcosis, pneumoconiosis, sarcoidosis, desquamative interstitial pneumonia, lymphoid interstitial pneumonia, giant cell interstitial pneumonia, cellular interstitial pneumonia, extrinsic allergic alveolitis, Wegener's granulomatosis and related forms of angiitis (temporal arteritis and polyarteritis nodosa), inflammatory dermatoses, hepatitis, delayed-type hypersensitivity reactions (*e*.*g*., poison ivy dermatitis), pneumonia, respiratory tract inflammation, Adult Respiratory Distress Syndrome (ARDS), encephalitis, immediate hypersensitivity reactions, asthma, hayfever, allergies, acute anaphylaxis, rheumatic fever, glomerulonephritis, pyelonephritis, cellulitis, cystitis, chronic cholecystitis, ischemia (ischemic injury), reperfusion injury, allograft rejection, host-versus-graft rejection, appendicitis, arteritis, blepharitis, bronchiolitis, bronchitis, cervicitis, cholangitis, chorioamnionitis, conjunctivitis, dacryoadenitis, dermatomyositis, endocarditis, endometritis, enteritis, enterocolitis, epicondylitis, epididymitis, fasciitis, fibrositis, gastritis, gastroenteritis, gingivitis, ileitis, iritis, laryngitis, myelitis, myocarditis, nephritis, omphalitis, oophoritis, orchitis, osteitis, otitis, pancreatitis, parotitis, pericarditis, pharyngitis, pleuritis, phlebitis, pneumonitis, proctitis, prostatitis, rhinitis, salpingitis, sinusitis, stomatitis, synovitis, testitis, tonsillitis, urethritis, urocystitis, uveitis, vaginitis, vasculitis, vulvitis, vulvovaginitis, angitis, chronic bronchitis, osteomyelitis, optic neuritis, temporal arteritis, transverse myelitis, necrotizing fasciitis, and necrotizing enterocolitis.

As used herein, an "autoimmune disease" refers to a disease arising from an inappropriate immune response of the body of a subject against substances and tissues normally present in the body. In other words, the immune system mistakes some part of the body as a pathogen and attacks its own cells. This may be restricted to certain organs (*e.g.,* in autoimmune thyroiditis) or involve a particular tissue in different places (*e*.*g*., Goodpasture's disease which may affect the basement membrane in both the lung and kidney). The treatment of autoimmune diseases is typically with immunosuppression, *e*.*g*., medications which decrease the immune response. Exemplary autoimmune diseases include, but are not limited to, glomerulonephritis, Goodpasture's syndrome, necrotizing vasculitis, lymphadenitis, peri-arteritis nodosa, systemic lupus erythematosis, rheumatoid, arthritis, psoriatic arthritis, systemic lupus erythematosis, psoriasis, ulcerative colitis, systemic sclerosis, dermatomyositis/polymyositis, anti-phospholipid antibody syndrome, scleroderma, pemphigus vulgaris, ANCA-associated vasculitis (*e*.*g*., Wegener's granulomatosis, microscopic polyangiitis), uveitis, Sjogren's syndrome, Crohn's disease, Reiter's syndrome, ankylosing spondylitis, Lyme arthritis, Guillain-Barré syndrome, Hashimoto's thyroiditis, and cardiomyopathy.

The term "autoinflammatory disease" refers to a category of diseases that are similar but different from autoimmune diseases. Autoinflammatory and autoimmune diseases share common characteristics in that both groups of disorders result from the immune system attacking a subject's own tissues and result in increased inflammation. In autoinflammatory diseases, a subject's innate immune system causes inflammation for unknown reasons. The innate immune system reacts even though it has never encountered autoantibodies or antigens in the subject. Autoinflammatory disorders are characterized by intense episodes of inflammation that result in such symptoms as fever, rash, or joint swelling. These diseases also carry the risk of amyloidosis, a potentially fatal buildup of a blood protein in vital organs. Autoinflammatory diseases include, but are not limited to, familial Mediterranean fever (FMF), neonatal onset multisystem inflammatory disease (NOMID), tumor necrosis factor (TNF) receptor-associated periodic syndrome (TRAPS), deficiency of the interleukin-1 receptor antagonist (DIRA), and Behçet's disease.

The term "biological sample" refers to any sample including tissue samples (such as tissue sections and needle biopsies of a tissue); cell samples (*e*.*g*., cytological smears (such as Pap or blood smears) or samples of cells obtained by microdissection); samples of whole organisms (such as samples of yeasts or bacteria); or cell fractions, fragments or organelles (such as obtained by lysing cells and separating the components thereof by centrifugation or otherwise). Other examples of biological samples include blood, serum, urine, semen, fecal matter, cerebrospinal fluid, interstitial fluid, mucus, tears, sweat, pus, biopsied tissue (*e*.*g*., obtained by a surgical biopsy or needle biopsy), nipple aspirates, milk, vaginal fluid, saliva, swabs (such as buccal swabs), or any material containing biomolecules that is derived from a first biological sample. Biological samples also include those biological samples that are transgenic, such as transgenic oocyte, sperm cell, blastocyst, embryo, fetus, donor cell, or cell nucleus.

### DETAILED DESCRIPTION OF CERTAIN EMBODIMENTS OF THE INVENTION

### Compounds

In one aspect of the present invention, provided are compounds of formula (I): 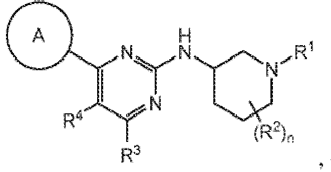 or a pharmaceutically acceptable salt thereof, wherein
ring A is a bicyclic 6,5-ring system selected from: 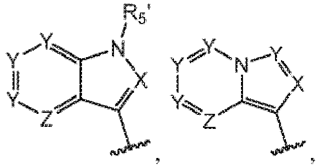 and 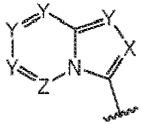 and comprises no more than four ring nitrogen atoms;
X is selected from N, and C(R⁶), wherein R⁶ is selected from hydrogen, -CN, -CH₃, - CH₂F, -CHF₂ and -CF₃;
each Y is independently selected from N and C(R⁷), wherein R⁷ is selected from hydrogen and R⁵;
Z is selected from N and C(R⁸), wherein R⁸ is selected from hydrogen and fluoro;
R¹ is selected from hydrogen, -C₁-C₆ alkyl, -O-(C₁-C₆-alkylene)-O-(C₁-C₄-alkyl), -(C₀-C₆ alkylene)-(C₃-C₈cycloalkyl), -(C₁-C₆ alkylene)-heterocyclyl, -(C₁-C₆ alkylene)-heteroaryl, -(C₁-C₆ alkylene)-N(R^{1'})₂, -(C₁-C₆ alkylene)-NR^{1'}-S(O)₂-(C₁-C₄ alkyl), -(C₁-C₆ alkylene)-NR^{1'}-SO₂. N(R^{1'})2, -(C₁-C₆ alkylene)-S(O)₂-(C₁-C₄ alkyl), and -(C₁-C₆ allcylene-S(O)2-N(R^{1'})₂, wherein any alkyl, alkylene, cycloalkyl, heterocyclyl or heteroaryl portion of R¹ is optionally substituted:
each R¹ is independently selected from hydrogen, and optionally substituted C₁-C₆ alkyl, or
two R^{1'} are optionally taken together with the nitrogen atom to which they are bound to form a 4-6 membered, optionally substituted heterocyclyl or heteroaryl ring comprising up to 2 additional heteroatoms selected from N, O, and S, wherein:
each R², if present, is independently selected from =O, halo, -OH, -CN, -C₁-C₆ alkyl, - (C₀-C₆ alkylene)-(C₃-C₈ cycloalkyl), -(C₀-C₆ alkylene)-heterocyclyl, -(C₀-C₆ alkylene)-heteroaryl, -(C₀-C₆ alkylene)-aryl, -(C₀-C₆ alkylene-C(O)-heterocyclyl, -(C₀-C₆ alkylene)-C(O)-heteroaryl, -O-(C₁-C₆-alkyl); -O)-(C₁-C₆-alkylene)-O-(C₁-C₄-alkyl); -O-(C₁-C₄-alkylene)-(C₃-C₈ cycloalkyl), -O-(C₁-C₆-alkylene)-heterocyclyl, -O-(C₁-C₆-alkylene)-heteroaryl, and -O-(C₁-C₆-alkylene)-aryl, or
R¹ and any R² are taken together with the atoms to which they are bound to form an optionally substituted heterocyclyl or heteroaryl ring fused, spirofused or bridged to the piperidine ring, or
two R² are taken together with the atom or atoms to which they are bound and any intervening ring atoms to form an optionally substituted aryl, cycloalkyl, heterocyclyl or heteroaryl ring fused, spirofused or bridged to the piperidine ring,
wherein any alkyl, alkylene, cycloalkyl, heterocyclyl or heteroaryl portion of R², any ring formed by taking R¹ together with R², or any ring formed by taking two R² together is optionally substituted:
R³ is selected from hydrogen, halo, -CN, optionally substituted -C₁-C₆ alkyl, or optionally substituted C₃-C₈ cycloalkyl;
R⁴ is selected from halo, -CN, -C₁-C₆ alkyl, -C₂-C₆ alkenyl, C₂-C₆ alkynyl, -O-C₁-C₆ alkyl, -S-C₁-C₆ alkyl, and a C₃-C₈ cycloalkyl, wherin any alkyl, alkenyl, or alkynyl portion of R⁴ is optionally substituted;
each R⁵ is independently selected from halo, -OH, -C₁-C₆ alkyl, -CN, -(C₀-C₆ alkylene)-C(O)OH, -(C₀-C₆ alkylene)-C(O)-(C₁-C₄ alkyl), -(C₀-C₆ alkylene)-C(O)-N(R^{1'})₂, -(C₀-C₆ alkylene)-S(O)₂-(C₁-C₄ alkyl), -(C₀-C₆ alkylene)-S(O)₂₋N(R^{1'})₂, -(C₀-C₆ alkylene)-P(O)-O-(C₁-C₄ alkyl)₂, -(C₀-C₆ alkylene)-P(O)-(C₁-C₄ alkyl)(O-C₁-C₄ alkyl), -(C₀-C₆ alkylene)-P(OXC₁-C₄ alkyl)₂, -(C₀-C₆ alkylene)-(C₃-C₈ cycloalkyl), -(C₀-C₆ alkylene)-heterocyclyl, -(C₀-C₆ alkylene)-heteroaryl, -(C₀-C₆ alkylene)-C(O)-heterocyclyl, -(C₀-C₆ alkylene)-C(O)-heteroaryl, -O-(C₁-C₆-alkyl), -O-(C₁-C₆-alkylene)-O-(C₁-C₄-alkyl), -O-(C₀-C₆-alkylene)-(C₃-C₈ cycloalkyl), -O-(C₁-C₆-alkylene)-heterocyclyl, and -O-(C₁-C₆-alkylene)-heteroaryl, wherein any alkyl, alkylene, cycloalkyl, heterocyclyl and heteroaryl portion of R⁵ is optionally substituted; or
two vicinal R⁵ are taken together with the ring atoms to which they are bound to form an optionally substituted cycloalkyl or optionally substituted heterocyclyl, wherein each cycloalkyl or heterocyclyl is fused to ring A;
R^{5'} is selected from hydrogen, -CN, -C₁-C₆ alkyl, -(C₀-C₆ alkylene)-S(O)₂₋N(R^{1'})₂, -(C₀-C₆ alkylene)-(C₃-C₈ cycloalkyl), -(C₀-C₆alkylene)-C(O)-N(R^{1'})₂, -(C₀-C₆ alkylene)-aryl, -(C₀-C₆ alkylene)-heterocyclyl, -(C₀-C₆ alkylene)-heteroaryl, -(C₀-C₆ alkylene)-S(O)₂-(C₁-C₄ alkyl), -(C₁-C₆ alkylene)-O-(C₁-C₃ alkylene)-C(O)-N(R^{1'})₂, -(C₁-C₆ alkylene)-O-(C₁-C₄ alkylene)-P(O)(C₁-C₄ alkyl)₂, -(C₁-C₆ alkylene)-O-(C₁-C₄ alkylene)-P(O)(C₁-C₄ alkyl)-O-(C₁-C₄ alkyl), -(C₁-C₆ alkylene)-O-(C₁-C₄ alkylene)-P(O)-(O-C₁-C₄ alkyl)₂, -(C₁-C₆ alkylene)-O-(C₁-C₄ alkylene)-S(O)₂-(C₁-C₄ alkyl), -(Ci-Cs alkylene)-O-(C₁-C₄ alkylene)-S(O)₂₋N(R^{1'})₂, -(C₁-C₆ alkylene)-O-(C₁-C₄ alkyl), -(C₁-C₆ alkylene)-Q-(C₃-C₈ cycloalkyl), -(C₁-C₆ alkylene)-O-heteroaryl, -(C₁-C₆ alkylene)-O-heterocyclyl, -(C 1-4 alkylene)-P(O)(C₁-C₄ alkyl)₂, -(Ci-Cs alkylene)-P(O)(C₁-C₄ alkyl)-O-(C₁-C₄alkyl), -(C₁-C₆ alkylene)-P(O)-(O-C₁-C₄ alkyl)₂, -(C₁-C₆ alkylene)-C(O)-(C₁-C₄ alkyl), and -(C₁-C₆alkylene)-C(O)OH, wherein any alkyl, alkylene, cycloalkyl, heterocyclyl and heteroaryl portion of R^{5'} is optionally substituted; and
n is 0, 1, 2, 3, or 4.

In some embodiments, R¹ is additionally selected from -C(O)-O-(C₁-C₆ alkyl).

In some embodiments, R¹ is additionally selected from -(C₀-C₆ alkylene)-carbocyclyl, wherein carbocyclyl is optionally substituted.

In some embodiments, each R², if present, is additionally selected from -NH-C(O)-C₁-C₄ alkyl, -C(O)-NH-(unsubstituted C₁-C₄ alkyl).

In some embodiments, each R², if present, is additionally selected from -(C₀-C₆ alkylene)-carbocyclyl or -O-(C₁-C₄-alkylene)-carbocyclyl, wherein each alkylene or carbocyclyl is optionally substituted.

In some embodiments, R³ is additionally selected from optionally substituted carbocyclyl.

In some embodiments, R⁴ is additionally selected from optionally substituted carbocyclyl.

In some embodiments, each R⁵ is additionally selected from -(C₀-C₆ alkylene)-carbocyclyl, -O-(C₀-C₅-alkylene)-carbocyclyl, phenyl, -(C₂-C₄ alkenylene)-phenyl, -S(O)-(C₁-C₄ alkyl), -S-(C₁-C₄ alkyl), -S(O)-OH, and -S(O)₂-OH. wherein any alkyl, alkylene, alkenylene, carbocyclyl, or phenyl is optionally substituted.

In some embodiments, R^{5'} and any R⁵ are taken together with the ring atoms to which they are bound to form an optionally substituted heterocyclyl, wherein each heterocyclyl is fused to ring A.

In some embodiments, the compound of formula (I) is not: 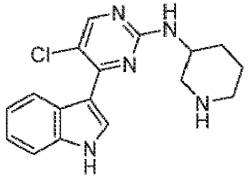 or a pharmaceutical salt of the foregoing.

In some embodiments, the compound of formula (I) is not: 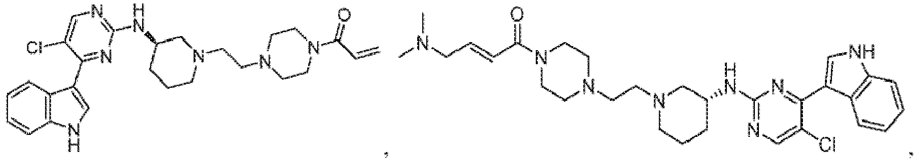 or 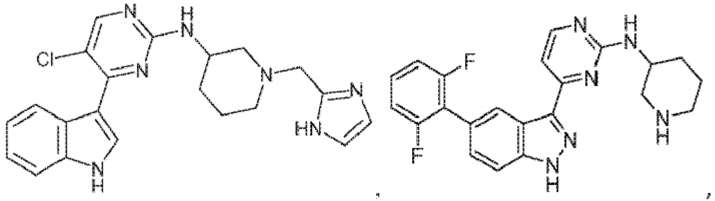 or a stereoisomer or a pharmaceutical salt of any of the foregoing.

In some embodiments, ring A is selected from: 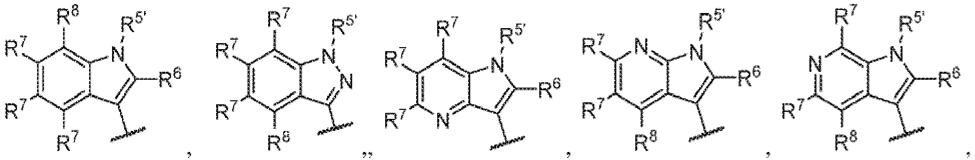 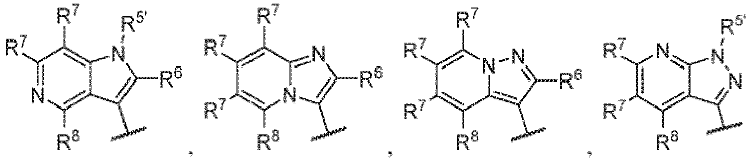 and

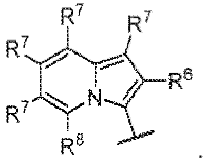

In some embodiments, ring A is selected from indol-3-yl, indazol-3-yl. In some embodiments, ring A is indol-3-yl. In some embodiments, ring A is indazol-3-yl.

In some embodiments, any alkyl or alkylene portion of R¹ is optionally substituted with one or more independently selected monovalent substituents (e.g., such substituents do not include =O).

In some embodiments, any heterocyclyl or heteroaryl portion of R¹ is optionally and independently substituted with one or more substituents independently selected from halo, C₁-C₆ alkyl, C₃-C₆ cycloalkyl, -OH, =O, -CN, -C(O)N(R^{1'})₂, -S(O)₂-(C₁-C₄-alkyl), and -S(O)₂₋N(R^{1'})₂; and any alkyl, alkylene, or cycloalkyl portion of R¹ or a substituent thereon is optionally substituted with one or more substituents independently selected from fluorine, OH and CN.

In some embodiments, R¹ is selected from hydrogen, -C₁-C₆ alkyl, -O-(C₁-C₆-alkylene)-O-(C₁-C₄-alkyl), -(C₁-C₆ alkylene)-N(R^{1'})₂, -(C₁-C₆ alkylene)-NR^{1'}-S(O)₂-(C₁-C₄ alkyl), -(C₁-C₆ alkylene)-NR^{1'}-SO₂₋N(R^{1'})₂, -(C₁-C₆ alkylene)-S(O)₂-(C₁-C₄ alkyl), -(C₁-C₆ alkylene-S(O)₂-N(R^{1'})₂, and -(C₀-C₆ alkylene)-(C₃-C₈ cycloalkyl), wherein any alkyl, or alkylene portion of R¹ is optionally substituted with one or more independently selected monovalent substitutents, any cycloalkyl portion of R¹ is optionally substituted with one or more independently selected substitutents; and wherein each R^{1'} is independently selected from hydrogen, and optionally substituted C₁-C₆ alkyl (i.e., two R^{1'} cannot be taken together to form a ring).

In some embodiments, R¹ is selected from hydrogen, cyclopropyl, -CH₃, - CH₂CH₃, -CH₂CH₂OCH₃, -CH(CH₃)₂, or -CH₂CH(CH₃)₂, or wherein R¹ is taken together with one R² and the ring atoms to which each are bound to form a ring which, taken together with the ring to which R¹ and R² are bound, is 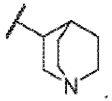 In some embodiments, R¹ is selected from hydrogen, -CH₃, or -CH₂CH₂OCH₃. In some embodiments, R¹ is hydrogen.

In some embodiments, each alkyl in any R^{1'} is optionally substituted with one or more substituents independently selected from fluorine, OH and CN.

In some embodiments, any heterocyclyl and heteroaryl rings formed from two R^{1'} are optionally substituted with one or more substituents independently selected from halo; C₁-C₄ alkyl; C₃-C₆ cycloalkyl optionally substituted with one or more substituents independently selected from fluorine, OH and CN; -OH; =O; -CN; -C(O)NH₂; -C(O)NH(C₁-C₄ alkyl); -C(O)N(C₁-C₄ alkyl)₂; -S(O)₂-C₁-C₄-alkyl; -S(O)₂-NH₂; -S(O)2-NH(C₁-C₄ alkyl); and -S(O)₂-N(C₁-C₄ alkyl)₂, wherein any alkyl portion of a substituent on any heterocyclyl and heteroaryl ring formed from two R^{1'} is optionally substituted with one or more further substituents independently selected from fluorine, OH and CN.

In some embodiments, any alkyl, alkylene, or aryl portion of R² is optionally substituted with one or more independently selected monovalent substituents. In one aspect of these embodiments, any alkyl, alkylene, aryl, cycloalkyl, heterocyclyl or heteroaryl portion of R², any ring formed by taking R¹ together with R², or any ring formed by taking two R² together is optionally substituted with one or more independently selected monovalent substituents.

In some embodiments, any heterocyclyl and heteroaryl portion of R² is optionally substituted with one or more substituents independently selected from halo, -C₁-C₄ alkyl, -OH, =O, -CN, -C(O)N(R^{1'})₂, -C(O)OR^{1'}, -C(O)OH, -S(O)₂-(C₁-C₄-alkyl), -S(O)₂₋N(R^{1'})₂; and any alkyl, alkylene and cycloalkyl portion of R² or a substituent thereon is optionally substituted with one or more substituents independently selected from fluorine, OH and CN.

In some embodiments, when two R² are taken together to form a ring, or R¹ and R² are taken together to form a ring, the resulting ring is optionally substituted with one or more substituents independently selected from halo, C₁-C₄ alkyl, -OH, =O, CN, -C(O)NR^{1'}₂, -S(O)₂-C₁-C₄-alkyl, -S(O)₂₋N(R¹⁻ )₂; and any alkyl portion of a substituent on a ring formed when two R² are taken together to form a ring, or R¹ and R² are taken together is optionallys substituted with one or more substituents independently selected from fluorine, OH and CN.In some embodiments, each R², if present, is independently selected from =O, halo, -OH, -C₁-C₆ alkyl, -NHC(O)-(C₁-C₄ alkyl), -C(O)NH-C₁-C₄ alkyl, -C(O)-(optionally substituted heterocyclyl), optionally substituted aryl, and optionally substituted heteroaryl; or
R¹ and any R² are taken together with the atoms to which they are bound to form an optionally substituted heterocyclyl or heteroaryl ring fused, spirofused or bridged to the piperidine ring, or
two R² are taken together with the atom or atoms to which they are bound and any intervening ring atoms to form an optionally substituted aryl, cycloalkyl, heterocyclyl or heteroaryl ring fused, spirofused or bridged to the piperidine ring,
wherein any alkyl or alkylene portion of R², any ring formed by taking R¹ together with R², or any ring formed by taking two R² together is optionally substituted with one or more independently selected monovalent substituents.

In some embodiments, each R², if present, is independently selected from halo, =O, -OH, optionally substituted -C₁-C₄ alkyl, optionally substituted phenyl and an optionally substituted heteroaryl. In some embodiments, each R² that is -C₁-C₄ alkyl or phenyl is optionally substituted with one or more independently selected monovalent substituents. In some embodiments, each R², if present, is independently selected from halo or optionally substituted -C₁-C₄ alkyl. In some embodiments, each R², if present, is independently selected from halo or -C₁-C₄ alkyl optionally substituted with one or more independently selected monovalent substituents. In some embodiments, each R², if present, is halo. In some embodiments, each R², if present, is optionally substituted -C₁-C₄ alkyl. In some embodiments, each R², if present, is -C₁-C₄ alkyl optionally substituted with one or more independently selected monovalent substituents.

In some embodiments, n is 0, 1, 2 or 3. In some embodiments, n is 0, 1 or 2. In some embodiments, n is 0 or 1. In some embodiments, n is 0. In some embodiments, n is 1. In some embodiments, n is 2. In some embodiments, n is 3.

In some embodiments, n is 0, 1, 2 or 3, and each R², if present, is independently selected from fluoro, =O, -CH₃, -CH₂CH₃, -OH, and unsubstituted phenyl.

In some embodiments, n is 0, 1, 2 or 3, and each R², if present, is independently selected from -CH(CH₃)₂, -C(O)NHCH₃, -NHC(O)CH₂CH₃, 3-methyl-1,2,4-oxadiazol-5-yl, 1,2,4-triazolo[4,3-a]pyridin-3-yl, 8-(methylsulfonyl)-1,2,4-triazolo[4,3-a]pyridin-3-yl, pyrrolidin-1-ylcarbonyl, and 3-hydroxypyrrolidin-1-ylcarbonyl; or two RZ on different atoms are taken together with the atoms to which they are bound and any intervening ring atoms to form a ring which, taken together with the piperdine ring to which both R² are bound, is 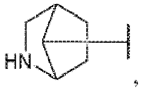 or 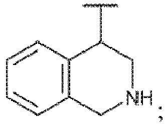 or two R² bound to the same ring atom are taken together with the atom to which they are bound to form a ring which, taken together with the piperdine ring to which both R² are bound, is:

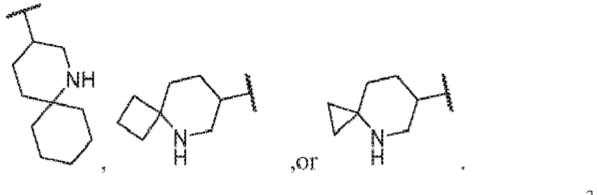

In some embodiments, each alkyl or cycloalkyl portion of R³ is optionally and independently substituted with one or more fluorine.

In some embodiments, R³ is hydrogen.

In some embodiments, any alkyl, alkenyl, alkynyl, or cycloalkyl portion of R⁴ is optionally and independently substituted with one or more substituents independently selected from -OH and fluorine.

In some embodiments, R⁴ is selected from halo, -CN, optionally substituted C₁-C₄ alkyl, optionally substituted C₂-C₄ alkynyl, optionally substituted -O-C₁-C₄ alkyl, and optionally substituted C₃-C₆ cycloalkyl. In some embodiments, R⁴ is selected from halo, -CN, optionally substituted C₁-C₄ alkyl, and optionally substituted C₁-C₄ haloalkyl. In some embodiments, R⁴ is selected from halo, C₁-C₄ alkyl, and C₁-C₄ haloalkyl. In some embodiments, R⁴ is C₁-C₄ alkyl. In some embodiments, R⁴ is C₁-C₄ haloalkyl. In some embodiments, R⁴ is halo.

In some embodiments, R⁴ is hydrogen or -C(O)-(optionally substituted C₁-C₄ alkyl).

In some embodiments, R⁴ is selected from chloro, fluoro, bromo, iodo, cyclopropyl, -CN, -CF₃, -CH₂CF₃, -CH₃, -CH₂CH₃, -CH(CH₃)₂ -CH₂CH(CH₃)₂, -OCH₃, and -C≡CH. In some embodiments, R⁴ is selected from chloro, fluoro, -CF₃, -CH₂CF₃, -CH₃, -CH₂CH₃, and -C≡CH. In some embodiments, R⁴ is selected from chloro, -CF₃, -CH₃, and -CH₂CH₃. In some embodiments, R⁴ is selected from chloro, and -CF₃. In some embodiments, R⁴ is chloro. In some embodiments, R⁴ is -CF₃.

In some embodiments, R⁴ is selected from -CH₂CH₂F, -CH₂CH₂CH₃, -CH(OH)CH₃, - CH=CH₂, -C(O)CH₃, -OCHF₂, -S-CH₃, -S-CHF₂, and -S-CF₃.

In some embodiments, any heterocyclyl or heteroaryl portion of each R⁵ or a ring formed when two vicinal R⁵ are taken together is optionally and independently substituted with one or two substituents independently selected from halo, -CN, C₁-C₆ alkyl, -OH, =O, CN, -C(O)NR^{1'}₂, or -SO₂₋NR^{1'}₂; and any alkyl, alkylene and cycloalkyl portions of R⁵, a substituent on R⁵, or a substituent on a ring formed by taking together two R⁵ is optionally substituted with one or more substituents independently selected from fluorine, OH and CN.

In some embodiments, one R⁵ is an optionally substituted heteroaryl or an optionally substituted heterocyclyl. In one aspect of these embodiments, the heteroaryl or heterocyclyl is selected from pyrazol-4-yl, imidazol-1-yl, morpholin-4-yl, pyridin-4-yl, pyridazin-4-yl, 1H-pyrrol-3-yl, pyridazin-4-yl, 1,2,4-triazol-3-yl, and 1,2,4-oxadiazol-3-yl; and is optionally substituted with one or two substituents selected from halo, -CN, C₁-C₆ alkyl, -OH, CN, - C(O)N(R^{1'})₂, or -SON(R^{1'})₂.

In some embodiments, each R⁷ is independently selected from hydrogen, halo, -C₁-C₆ alkyl, -CN, -C(O)OH, -C(O)-(C₁-C₄ alkyl), -C(O)-N(R^{1'})₂, -S(O)₂-(C₁-C₄ alkyl), -P(O)(C₁-C₄ alkyl)-O-C₁-C₄ alkyl, -P(O)(O-(C₁-C₄ alkyl))₂, heterocyclyl, and heteroaryl, wherein any alkyl, heterocyclyl or heteroaryl is optionally substituted.

In some embodiments, each R⁷ is independently selected from -C(O)-heterocyclyl, - S(O)₂N(R^{1'})₂, -(C₁-C₄ alkylene)-S(O)₂-(C₁-C₄ alkyl), carbocyclyl, -O-(C₀-C₆-alkylene)-carbocyclyl, phenyl, -(C₂-C₄ alkenylene)-phenyl, -S(O)-(C₁-C₄ alkyl), -S-(C₁-C₄ alkyl), -S(O)-OH, and -S(O)₂-OH, wherein any alkyl, alkylene, alkenylene, carbocyclyl, phenyl, or heterocyclyl is optionally substituted.

In some embodiments, each R⁷ is independently selected from hydrogen, fluoro, chloro, bromo, -CN, -CH₃, -CH₂CH₂C(CH₃)₂OH, -C(O)-CH₃, -C(O)OH, -C(O)-NH-CH₃, - P(=O)(OCH₂CH₃)₂, -P(=O)(OCH₂CH₃)CH₃, -S(O)₂CH₃, 1H-pyrazol-4-yl, 1-methylpyrazol-4-yl, 1,3-dimethyl-pyrazol-4-yl, 5-methyl-1H-pyrazol-4-yl, 1-methyl-2-oxoimidazolidin-3-yl, 4-methylimidazol-1-yl, morpholin-4-yl, pyridin-4-yl, 4-hydroxycyclohexyl, 4-hydroxy-4-methylcyclohexyl, 5-methyl-1,2,4-triazol-3-yl, 5-methyl-1,2,4-oxadiazol-3-yl, 1,3-dimethylpyridazin-4-yl, 1,5-dimethylpyridazin-4-yl, 3-methyl-1H-pyridazin-4-yl, 1-(2-methyl-2-hydroxypropyl)pyridazin-4-yl, imidazol-1-yl, 1-methyl-5-cyanopyrrol-3-yl, 5-cyano-1H-pyrrol-3-yl, and pyridazin-4-yl.

In some embodiments, each R⁷ is independently selected from -P(O)-(CH₃)₂, -P(O)-(CH₂CH₃)₂, -S(O)₂N(CH₃)₂, -S(O)₂CH(CH₃)₂, -S(O)₂CH₂F, -S(O)₂CHF₂, -SCHF₂, -S(O)CHF₂, - S(O)OH, -S(O)₂OH, -S(O)₂NHCH₃, -(CH₂)₄CH3, -CH₂S(O)₂CH₃, -S(O)₂-CH₂CH₃, 1H-pyrazol-3-yl, 1-difluoromethyl-pyrazol-3-yl, 1-difluoromethyl-pyrazol-4-yl, 1-methylpyrazol-3-yl, 3-methyl-1H-pyrazol-4-yl, 3-methyl-3-hydroxypyrrolidin-1-ylcarbonyl, 3-hydroxypyrrolidin-1-ylcarbonyl, 4-hydroxycyclohexyl, 4-hydroxycyclohex-1-enyl, 1,1-dioxothiomorpholin-4-yl, 4-cyano-1H-imidazol-1-yl, 2,3-dimethyl-1,2,4-triazol-5-yl, 1,5-dimethyl-pyrazol-4-yl, pyridin-3-yl, 1-(2-methyl-2-hydroxypropan-1-yl)pyrazol-4-yl, pyrrolidin-1-yl, pyrrolidin-1-ylcarbonyl, 1H-pyrazol-2-yl, 3-hydroxy-3-trifluoromethylpyrrolidin-1-ylcarbonyl, 3-methoxypyrrolidin-1-ylcarbonyl, 3-cyanopyrrolidin-1-ylcarbonyl, 4-hydroxy-4-methylpiperindin-1-ylcarbonyl 3-oxopyrrolidin-1-ylcarbonyl, 3-(pyrrolidin-1-ylcarbonyl)phenyl, 3-phenoxyphenyl, thiazol-2-yl, pyrazin-2-yl, 2,4-dioxo-1H,3H-pyrimidin-5-yl, 3-methyl-3-hydroxypyrrolidin-1-ylsulfonyt, 5-flluoropyridin-3-yl, 2-hydroxpyridin-3-yl, 3,3-difluoro-4-hydroxy, 3,5-dimethyloxazol-4-yl, 3-fluorophenyl, 4-methylpyridin-3-yl, 2-hydroxymethylpyridin-3-yl, 6-hydroxymethylpyridin-2-yl, 5-hydroxymethylpyridin-3-yl, 1-methyl-6-oxopyridin-3-yl, 4-aminosulfonylphenyl, 3-aminosulfonylphenyl, 3-hydroxy-3-ethylpyrrolidin-1-ylcarbonyl, 3-cyano-4-hydroxyphenyl, benzo[d]thiazol-6-yl, 2H-indazol-6-yl, 1H-benzoimidazol-5-yl, 2-oxo-3-cyano-4-methylpyridin-5-yl, 2-aminobenzo[d]thiazol-2-yl, 3-aminocarbonylphenyl, 6-trifluoromethyl-1H-pynolo[3,2-c]pyridin-3-yl, 2-aminoquinazolin-8-yl, styryl, 1-methyl-1H-indazol-6-yl, 2,3-dihydrobenzo[b][1,4]dioxin-7-yl, 2-ethoxyphenyl, 3-(2-hydroxyethyl)phenyl, 3-(methylcarbonylaminomethyl)phenyl, 1-methyl-6-trifluoromethyl-1H-pyrrolo[3,2-c]pyridin-3-yl, quinolin-4-yl, isoquinolin-5-yl, isoquinolin-7-yk and 2-oxo-3,4-dihydroquinolin-7-yl.

In some embodiments, each heterocyclyl and heteroaryl portion of R^{5'} is optionally substituted with one or more substituents independently selected from halo, C₁-C₄ alkyl, -OH, =O, CN, -C(O)NR^{1'}₂, or -SO₂₋NR^{1'}₂, and each alkyl, alkylene and cycloalkyl portion of R^{5'} or a substituent of R^{5'} is optionally substituted with one or more substituents independently selected from fluorine, OH and CN.

In some embodiments, R^{5'} is selected from hydrogen, C₁-C₄ alkyl, -(C₀-C₃ alkylene)-aryl and -(C₁-C₃ alkylene)-O-(C₁-C₄ alkyl). In one aspect of these embodiments, R^{5'} is selected from hydrogen, methyl, isopropyl, -CH₂-O-CH₃, -(CH₂)₂-O-CH₃, and phenyl.

In some embodiments, R⁶ is selected from hydrogen and methyl. In one aspect of these embodiments, R⁶ is hydrogen. In another aspect of these embodiments, R⁶ is methyl.

In some embodiments, the compound of formula (I) is a compound of formula (I-a): 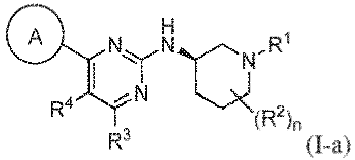 or a pharmaceutically acceptable salt thereof, wherein each of ring A, R¹, R², R³, R⁴, and n is defined as for formula (I).

In some embodiments, the compound of formula (I) is a compound of formula (I-b): 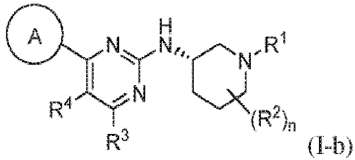 or a pharmaceutically acceptable salt thereof, wherein each of ring A, R¹, R², R³, R⁴, and n is defined as for formula (I).

In some embodiments, the compound of formula (I) is a compound of formula (I-c): 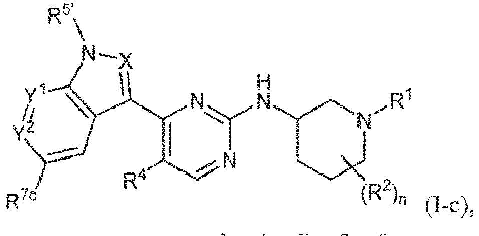 or a pharmaceutically acceptable salt thereof, wherein each of X, R², R⁴, R⁵ , R⁷ , R⁸, and n is defined as for formula (I); Y¹ is selected from N and C(R^{7a}); Y² is selected from N and C(R^{7b}); and no more than one of X, Y¹ or Y² is N, wherein each of R^{7a}, R^{7b} and R^{7c} is independently selected from R⁷as defined as for formula (I).

In some embodiments, the compound of formula (I-c) is a compound of formula (I-cl): 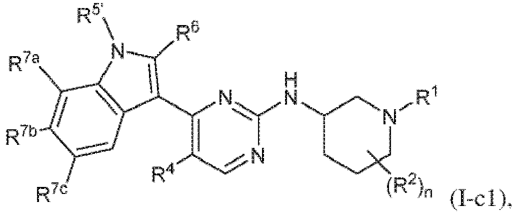 or a pharmaceutically acceptable salt thereof, wherein R⁶ is also as defined as for formula (I).

In some embodiments, the compound of formula (I-c) is a compound of formula (I-c2): 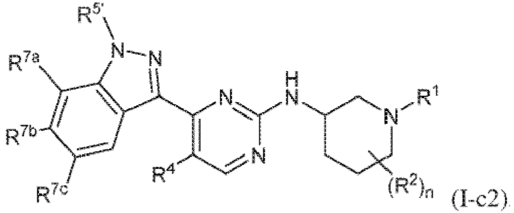 or a pharmaceutically acceptable salt thereof.

In some embodiments, the compound of formula (I) is a compound of formula (II): 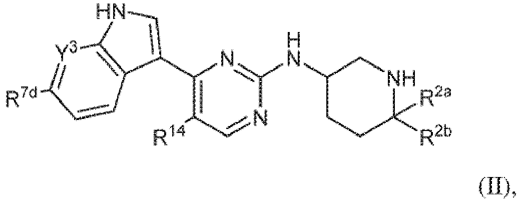 or a pharmaceutically acceptable salt thereof, wherein:
Y³ is selected from N and C(R^{7e});
each of R^{2a} and R^{2b} is independently selected from hydrogen and C₁-C₃ alkyl; or
R^{2a} and R^{2b} are taken together to form a cycloalkyl or a heterocycle spirofused to the piperidine ring, wherein said cycloalkyl or heterocycle is optionally substituted with one or more independently selected C₁-C₄ alkyl or C₁-C₄ haloalkyl;
R^{7d} is selected from hydrogen, -C(O)-(C₁-C₄ alkyl), -CN, and heteroaryl optionally substituted with one or more independently selected C₁-C₄ alkyl or C₁-C₄ haloalkyl;
R^{7e}, if present, is selected from hydrogen, halo, -S(O)₂-(C₁-C₄ alkyl), -P(O)(C₁-C₄ alkyl)₂, -C(O)NH-(C₁-C₄ alkyl), -C(O)N(C₁-C₄ alkyl)₂, -S(O)₂NH-(C₁-C₄ alkyl), -S(O)₂N-(C₁-C₄ alkyl)₂, and heteroaryl optionally substituted with one or more independently selected C₁-C₄ alkyl or C₁-C₄ haloalkyl; and
R¹⁴ is selected from C₁-C₃ alkyl and C₁-C₃ haloalkyl.

In some embodiments, the compound of formula (II) is a compound of formula (IIa): 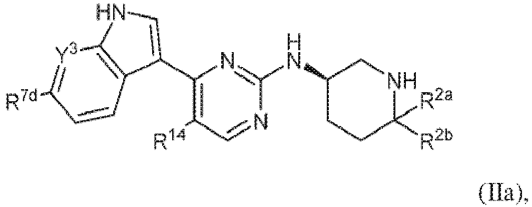 or a pharmaceutically acceptable salt thereof, wherein Y³, R^{2a}, R^{2b}, R^{7d}, R^{7e}, and R¹⁴ are as defined in Formula II.

In some embodiments, the compound of formula (II) is a compound of formula (IIb): 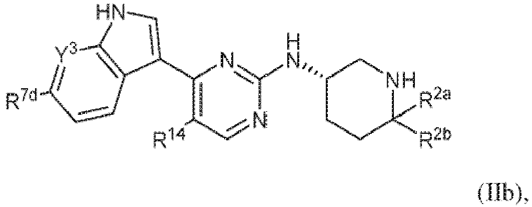 or a pharmaceutically acceptable salt thereof, wherein Y³, R^{2a}, R^{2b}, R^{7d}, R^{7e}, and R¹⁴ are as defined in Formula II.

In some embodiments, the compound of formula (I) is a compound of formula (III): 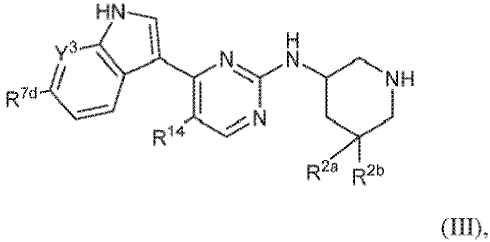 or a pharmaceutically acceptable salt thereof, wherein Y³, R^{2a}, R^{2b}, R^{7d}, R^{7e}, and R¹⁴ are as defined in Formula II.

In some embodiments, the compound of formula (III) is a compound of formula (IIIa): 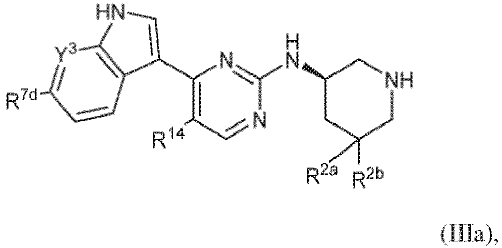 or a pharmaceutically acceptable salt thereof, wherein Y³, R^{2a}, R^{2b}, R^{7d}, R^{7e}, and R¹⁴ are as defined in Formula II

In some embodiments, the compound of formula (III) is a compound of formula (IIIb): 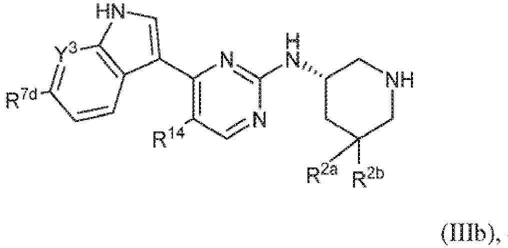 or a pharmaceutically acceptable salt thereof, wherein Y³, R^{2a}, R^{2b}, R^{7d}, R^{7e}, and R¹⁴ are as defined in Formula II.

In some embodiments, in a compound of any one of Formulae II, IIa, IIb, III, IIIa, or IIIb:
R^{2a} is selected from hydrogen and -CH₃;
R^{2b} is selected from hydrogen, -CH₃, -CH₂CH₃, and -CH(CH₃)₂; or
R^{2a} and R^{2b} are taken together to from oxetan-3-yl;
R^{7d} is selected from hydrogen, -C(O)CH₃, -CN, pyridin-3-yl, pyridin-4-yl, 1-methyl-5-cyanopyrrol-3-yl, 1-methylpyrazol-4-yl, 1-methylpyrazol-3-yl, 1H-pyrazol-4-yl, 1H-pyrazol-3-yl, 1H-imidazol-2-yl, 1,3-dimethylpyrazol-4-yl, 1,5-dimethylpyrazol-4-yl, 1,5-dimethyl-1,2,4-triazol-3-yl, imidazol-1-yl, 1-difluoromethylpyrazol-3-yl, 1-difluoromethylpyrazol-4-yl, and thiazol-2-yl;
R^{7e}, if present, is selected from hydrogen, fluoro, chloro, bromo, -CN, -P(O)(CH₃)₂, - S(O)₂CH(CH₃)₂, -S(O)₂CH₂CH₃, -S(O)₂N(CH₃)₂, -C(O)NHCH₃, pyridin-4-yl, pyridazin-4-yl, 5-methyl-1H-pyrazol-4-yl, 1-methylpyrazol-4-yl, 4-methyl-1H-imidazol-1-yl, 1H-benzo[d]imidazol-5-yl, 6-(trifluoromethyl)-1H-pyrrolo[3,2-c]pyridin-3-yl, 1-methyl-6-(trifluoromethyl)-1H-pyrrolo[3,2-c]pyridin-3-yl, isoquinolin-7-yl, isoquinolin-5-yl, pyrazin-2-yl, 2H-indazol-6-yl, 3,5-dimethylisoxazol-4-yl, thiazol-2-yl, 4-methylpyridin-3-yl, 1-methylindazol-6-yl, quinolin-4-yl, benzo[d]thiazol-6-yl, and 1,3-dimethylpyrazol-4-yl; and
R¹⁴ is selected from -CH₃, -CF₃, -CH₂CH₃, -CH₂CF₃, -CH₂CH₂F, and -CH(CH₃)₂.

In some embodiments, in a compound of formula (II)
R^{2a} is selected from hydrogen and -CH₃;
R^{2b} is selected from hydrogen, and -CH₃;
R^{7d} is selected from hydrogen, -CN, pyrazin-2-yl, thiazol-2-yl, and 3,5-dimethylisoxazol-4-yl;
R^{7e}, if present, is selected from hydrogen, fluoro, -C(O)NHCH₃, -P(O)(CH₃)₂, -S(O)₂CH₃, -S(O)₂N(CH₃)₂, 1,3-dimethylpyrazol-4-yl, and pyridazin-4-yl; and
R¹⁴ is selected from -CH₂CH₃, and -CF₃.

In some embodiments, the compound of formula (I) is selected from the group consisting of any one of the compounds in the table in Figure 1 and pharmaceutically acceptable salts, tautomers, stereoisomers, and isotopically labeled derivatives thereof.

### Pharmaceutical Compositions, Kits, and Administration

The present invention provides pharmaceutical compositions comprising a compound of formula (I), *e.g.,* a compound of formula (I) or a pharmaceutically acceptable salt, solvate, hydrate, tautomer, stereoisomer, or isotopically labeled derivative thereof, as described herein, and optionally a pharmaceutically acceptable excipient. In certain embodiments, the pharmaceutical composition of the invention comprises a compound of formula (I) or a pharmaceutically acceptable salt thereof, and optionally a pharmaceutically acceptable excipient. In certain embodiments, the compound of formula (I) or a pharmaceutically acceptable salt, solvate, hydrate, tautomer, stereoisomer, or isotopically labeled derivative thereof, is provided in an effective amount in the pharmaceutical composition. In certain embodiments, the effective amount is a therapeutically effective amount. In certain embodiments, the effective amount is a prophylactically effective amount.

Pharmaceutical compositions described herein can be prepared by any method known in the art of pharmacology. In general, such preparatory methods include the steps of bringing the compound of formula (I) (the "active ingredient") into association with a carrier and/or one or more other accessory ingredients, and then, if necessary and/or desirable, shaping and/or packaging the product into a desired single- or multi-dose unit

Pharmaceutical compositions can be prepared, packaged, and/or sold in bulk, as a single unit dose, and/or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered. By way of example, the composition may comprise between 0.1% and 100% (w/w) active ingredient.

The term "pharmaceutically acceptable excipient" refers to a non-toxic carrier, adjuvant, diluent, or vehicle that does not destroy the pharmacological activity of the compound with which it is formulated. Pharmaceutically acceptable excipients useful in the manufacture of the pharmaceutical compositions of the invention are any of those that are well known in the art of pharmaceutical formulation and include inert diluents, dispersing and/or granulating agents, surface active agents and/or emulsifiers, disintegrating agents, binding agents, preservatives, buffering agents, lubricating agents, and/or oils. Pharmaceutically acceptable excipients useful in the manufacture of the pharmaceutical compositions of the invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Compositions of the present invention may be administered orally, parenterally (including subcutaneous, intramuscular, intravenous and intradermal), by inhalation spray, topically, rectally, nasally, buccally, vaginally or via an implanted reservoir. In some embodiments, provided compounds or compositions are administrable intravenously and/or orally.

The term "parenteral" as used herein includes subcutaneous, intravenous, intramuscular, intraocular, intravitreal, intra-articular, intra-synovial, intrastemal, intrathecal, intrahepatic, intraperitoneal intralesional and intracranial injection or infusion techniques. Preferably, the compositions are administered orally, subcutaneously, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

Pharmaceutically acceptable compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added. In some embodiments, a provided oral formulation is formulated for immediate release or sustained/delayed release. In some embodiments, the composition is suitable for buccal or sublingual administration, including tablets, lozenges and pastilles. A provided compound can also be in micro-encapsulated form.

Alternatively, pharmaceutically acceptable compositions of this invention may be administered in the form of suppositories for rectal administration. Pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For ophthalmic use, provided pharmaceutically acceptable compositions may be formulated as micronized suspensions or in an ointment such as petrolatum.

Pharmaceutically acceptable compositions of this invention may also be administered by nasal aerosol or inhalation.

In order to prolong the effect of a drug, it is often desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation.

Compounds provided herein are typically formulated in dosage unit form, e.g., single unit dosage form, for ease of administration and uniformity of dosage. It will be understood, however, that the total daily usage of the compositions of the present invention will be decided by the attending physician within the scope of sound medical judgment. The specific therapeutically effective dose level for any particular subject or organism will depend upon a variety of factors including the disease being treated and the severity of the disorder; the activity of the specific active ingredient employed; the specific composition employed; the age, body weight, general health, sex and diet of the subject; the time of administration, route of administration, and rate of excretion of the specific active ingredient employed; the duration of the treatment; drugs used in combination or coincidental with the specific active ingredient employed; and like factors well known in the medical arts.

The exact amount of a compound required to achieve an effective amount will vary from subject to subject, depending, for example, on species, age, and general condition of a subject, severity of the side effects or disorder, identity of the particular compound(s), mode of administration, and the like. The desired dosage can be delivered three times a day, two times a day, once a day, every other day, every third day, every week, every two weeks, every three weeks, or every four weeks. In certain embodiments, the desired dosage can be delivered using multiple administrations (e.g., two, three, four, five, six, seven, eight, nine, ten, eleven, twelve, thirteen, fourteen, or more administrations).

In certain embodiments, an effective amount of a compound for administration one or more times a day to a 70 kg adult human may comprise about 0.0001 mg to about 3000 mg, about 0.0001 mg to about 2000 mg, about 0.0001 mg to about 1000 mg, about 0.001 mg to about 1000 mg, about 0.01 mg to about 1000 mg, about 0.1 mg to about 1000 mg, about 1 mg to about 1000 mg, about 1 mg to about 100 mg, about 10 mg to about 1000 mg, or about 100 mg to about 1000 mg, of a compound per unit dosage form.

In certain embodiments, the compounds of formula (I) may be at dosage levels sufficient to deliver from about 0.001 mg/kg to about 100 mg/kg, from about 0.01 mg/kg to about 50 mg/kg, preferably from about 0.1 mg/kg to about 40 mg/kg, preferably from about 0.5 mg/kg to about 30 mg/kg, from about 0.01 mg/kg to about 10 mg/kg, from about 0.1 mg/kg to about 10 mg/kg, and more preferably from about 1 mg/kg to about 25 mg/kg, of subject body weight per day, one or more times a day, to obtain the desired therapeutic effect.

It will be appreciated that dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult.

It will be also appreciated that a compound or composition, as described herein, can be administered in combination with one or more additional pharmaceutical agents. The compounds or compositions can be administered in combination with additional pharmaceutical agents that improve their bioavailability, reduce and/or modify their metabolism, inhibit their excretion, and/or modify their distribution within the body. It will also be appreciated that the therapy employed may achieve a desired effect for the same disorder, and/or it may achieve different effects.

The compound or composition can be administered concurrently with, prior to, or subsequent to, one or more additional pharmaceutical agents, which may be useful as, *e.g.,* combination therapies. Pharmaceutical agents include therapeutically active agents. Pharmaceutical agents also include prophylactically active agents. Each additional pharmaceutical agent may be administered at a dose and/or on a time schedule determined for that pharmaceutical agent. The additional pharmaceutical agents may also be administered together with each other and/or with the compound or composition described herein in a single dose or administered separately in different doses. The particular combination to employ in a regimen will take into account compatibility of the inventive compound with the additional pharmaceutical agents and/or the desired therapeutic and/or prophylactic effect to be achieved. In general, it is expected that the additional pharmaceutical agents utilized in combination be utilized at levels that do not exceed the levels at which they are utilized individually. In some embodiments, the levels utilized in combination will be lower than those utilized individually.

Exemplary additional pharmaceutical agents include, but are not limited to, anti-proliferative agents, anti-cancer agents, anti-diabetic agents, anti-inflammatory agents, immunosuppressant agents, and a pain-relieving agent. Pharmaceutical agents include small organic molecules such as drug compounds (*e*.*g*., compounds approved by the U.S. Food and Drug Administration as provided in the Code of Federal Regulations (CPR)), peptides, proteins, carbohydrates, monosaccharides, oligosaccharides, polysaccharides, nucleoproteins, mucoproteins, lipoproteins, synthetic polypeptides or proteins, small molecules linked to proteins, glycoproteins, steroids, nucleic acids, DNAs, RNAs, nucleotides, nucleosides, oligonucleotides, antisense oligonucleotides, lipids, hormones, vitamins, and cells.

Also encompassed by the invention are kits (*e*.*g*., pharmaceutical packs). The inventive kits may be useful for preventing and/or treating a proliferative disease (*e*.*g*., cancer (*e.g.,* leukemia, melanoma, multiple myeloma), benign neoplasm, angiogenesis, inflammatory disease, autoinflammatory disease, or autoimmune disease). The kits provided may comprise an inventive pharmaceutical composition or compound and a container (*e*.*g*., a vial, ampule, bottle, syringe, and/or dispenser package, or other suitable container). In some embodiments, provided kits may optionally further include a second container comprising a pharmaceutical excipient for dilution or suspension of an inventive pharmaceutical composition or compound. In some embodiments, the inventive pharmaceutical composition or compound provided in the container and the second container are combined to form one unit dosage form.

Thus, in one aspect, provided are kits including a first container comprising a compound described herein, or a pharmaceutically acceptable salt, solvate, hydrate, tautomer, stereoisomer, and isotopically labeled derivative, or a pharmaceutical composition thereof. In certain embodiments, the kit of the invention includes a first container comprising a compound described herein, or a pharmaceutically acceptable salt thereof, or a pharmaceutical composition thereof. In certain embodiments, the kits are useful in preventing and/or treating a proliferative disease in a subject. In certain embodiments, the kits further include instructions for administering the compound, or a pharmaceutically acceptable salt, solvate, hydrate, tautomer, stereoisomer, isotopically and labeled derivative thereof, or a pharmaceutical composition thereof, to a subject to prevent and/or treat a proliferative disease.

### Methods of Treatment and Uses

The present invention also provides methods for the treatment or prevention of a proliferative disease (e.g., cancer, benign neoplasm, angiogenesis, inflammatory disease, autoinflammatory disease, or autoimmune disease) or an infectious disease (*e*.*g*., a viral disease) in a subject. Such methods comprise the step of administering to the subject in need thereof an effective amount of a compound of formula (I) or a pharmaceutically acceptable salt, solvate, hydrate, tautomer, stereoisomer, or isotopically labeled derivative thereof, or a pharmaceutical composition thereof. In certain embodiments, the methods described herein include administering to a subject an effective amount of a compound of formula (1) or a pharmaceutically acceptable salt thereof, or a pharmaceutical composition thereof.

In certain embodiments, the subject being treated is a mammal. In certain embodiments, the subject is a human. In certain embodiments, the subject is a domesticated animal, such as a dog, cat, cow, pig, horse, sheep, or goat. In certain embodiments, the subject is a companion animal such as a dog or cat. In certain embodiments, the subject is a livestock animal such as a cow, pig, horse, sheep, or goat. In certain embodiments, the subject is a zoo animal. In another embodiment, the subject is a research animal such as a rodent, dog, or non-human primate. In certain embodiments, the subject is a non-human transgenic animal such as a transgenic mouse or transgenic pig.

The proliferative disease to be treated or prevented using the compounds of formula (I) will typically be associated with aberrant activity of CDK7. Aberrant activity of CDK7 may be an elevated and/or an inappropriate (e.g., abnormal) activity of CDK7. In certain embodiments, CDK7 is not overexpressed, and the activity of CDK7 is elevated and/or inappropriate. In certain other embodiments, CDK7 is overexpressed, and the activity of CDK7 is elevated and/or inappropriate. The compounds of formula (I) and pharmaceutically acceptable salts, solvates, hydrates, tautomers, stereoisomers, isotopically labeled derivatives, and compositions thereof, may inhibit the activity of CDK7 and be useful in treating and/or preventing proliferative diseases.

In other embodiments, the proliferative disease to be treated or prevented using the compounds of formula (I) will typically be associated with aberrant activity of CDK12 and/or CDK13. Aberrant activity of CDK12 and/or CDK13 may be an elevated and/or an inappropriate (e.g., abnormal) activity of CDK12 and/or CDK13. In certain embodiments, CDK12 and/or CDK13 is not overexpressed, and the activity of CDK12 and/or CDK13 is elevated and/or inappropriate. In certain other embodiments, CDK12 and/or CDK13 is overexpressed, and the activity of CDK12 and/or CDK13 is elevated and/or inappropriate. The compounds of formula (1) and pharmaceutically acceptable salts, solvates, hydrates, tautomers, stereoisomers, isotopically labeled derivatives, and compositions thereof, may inhibit the activity of CDK12 and/or CDK13 and be useful in treating and/or preventing proliferative diseases.

In embodiments, the disease to be treated or prevented using the compounds of formula (I) is associated with aberrant kinase activity. Exemplary kinases include BRAF, CDK1/cyclin A2, CDK1/cyclin B, CDK14 (PFTK1)/cyclin Y, CDK16 (PCTK1)/cyclin Y, CDK17/cyclin Y, CDK18/cyclin Y, CDK2/cyclin A, CDK2/cyclin El, CDK3/cyclin El, CDK5/p35, CDK7/cyclin H/MNAT1, CDK9/cyclin T1, CDKL5, CLK1, CLK2, CLK3, CLK4, DYRK2, DYRK3, ERN1, GAK, GSG2 (Haspin), GSK3A (GSK3 alpha), GSK3B (GSK3 beta), HIPK4, MAP2K1 (MEK1), MAP2K2 (MEK2), MAP2K6 (MKK6), MAP3K8 (COT), MAPK1 (ERK2), MAPK10 (JNK3), MAPK12 (p38 gamma), MAPK13 (p38 delta), MAPK15 (ERK7), MAPK3 (ERK1), MAPK8 (JNK1), MAPK9 (JNK2), PRKCA (PKC alpha), PRKCB2 (PKC beta II), PRKD1 (PKC mu), PRKD2 (PKD2), RAF1 (cRAF) Y340D Y341D, TAOK1, TLK1, and TLK2.

A proliferative disease may also be associated with inhibition of apoptosis of a cell in a biological sample or subject. All types of biological samples described herein or known in the art are contemplated as being within the scope of the invention. Inhibition of the activity of CDK7 is expected to cause cytotoxicity via induction of apoptosis. The compounds of formula (I) and pharmaceutically acceptable salts, solvates, hydrates, tautomers, stereoisomers, isotopically labeled derivatives, and compositions thereof, may induce apoptosis, and therefore, be useful in treating and/or preventing proliferative diseases.

In certain embodiments, the proliferative disease to be treated or prevented using the compounds of formula (I) is cancer. All types of cancers disclosed herein or known in the art are contemplated as being within the scope of the invention. In certain embodiments, the proliferative disease is a cancer associated with dependence on BCL-2 anti-apoptotic proteins (*e.g.,* MCL-1 and/or XIAP). In certain embodiments, the proliferative disease is a cancer associated with overexpression of MYC (a gene that codes for a transcription factor). In certain embodiments, the proliferative disease is a hematological malignancy. In certain embodiments, the proliferative disease is a blood cancer. In certain embodiments, the proliferative disease is leukemia. In certain embodiments, the proliferative disease is chronic lymphocytic leukemia (CLL). In certain embodiments, the proliferative disease is acute lymphoblastic leukemia (ALL). In certain embodiments, the proliferative disease is T-cell acute lymphoblastic leukemia (T-ALL). In certain embodiments, the proliferative disease is chronic myelogenous leukemia (CML). In certain embodiments, the proliferative disease is acute myelogenous leukemia (AML). In certain embodiments, the proliferative disease is lymphoma. In certain embodiments, the proliferative disease is melanoma. In certain embodiments, the proliferative disease is multiple myeloma. In certain embodiments, the proliferative disease is a bone cancer. In certain embodiments, the proliferative disease is osteosarcoma. In some embodiments, the proliferative disease is Ewing's sarcoma. In some embodiments, the proliferative disease is triple-negative breast cancer (TNBC). In some embodiments, the proliferative disease is a brain cancer. In some embodiments, the proliferative disease is neuroblastoma. In some embodiments, the proliferative disease is a lung cancer. In some embodiments, the proliferative disease is small cell lung cancer (SCLC). In some embodiments, the proliferative disease is large cell lung cancer. In some embodiments, the proliferative disease is a benign neoplasm. All types of benign neoplasms disclosed herein or known in the art are contemplated as being within the scope of the invention.

In some embodiments, the proliferative disease is associated with angiogenesis. All types of angiogenesis disclosed herein or known in the art are contemplated as being within the scope of the invention.

In certain embodiments, the proliferative disease is an inflammatory disease. All types of inflammatory diseases disclosed herein or known in the art are contemplated as being within the scope of the invention. In certain embodiments, the inflammatory disease is rheumatoid arthritis. In some embodiments, the proliferative disease is an autoinflammatory disease. All types of autoinflammatory diseases disclosed herein or known in the art are contemplated as being within the scope of the invention. In some embodiments, the proliferative disease is an autoimmune disease. All types of autoimmune diseases disclosed herein or known in the art are contemplated as being within the scope of the invention.

The cell described herein may be an abnormal cell. The cell may be *in vitro* or *in vivo.* In certain embodiments, the cell is a proliferative cell. In certain embodiments, the cell is a blood cell. In certain embodiments, the cell is a lymphocyte. In certain embodiments, the cell is a cancer cell. In certain embodiments, the cell is a leukemia cell. In certain embodiments, the cell is a CLL cell. In certain embodiments, the cell is a melanoma cell. In certain embodiments, the cell is a multiple myeloma cell. In certain embodiments, the cell is a benign neoplastic cell. In certain embodiments, the cell is an endothelial cell. In certain embodiments, the cell is an immune cell.

In another aspect, the present invention provides methods of down-regulating the expression of CDK7 in a biological sample or subject.

In certain embodiments, the methods described herein comprise the additional step of administering one or more additional pharmaceutical agents in combination with the compound of formula (I) a pharmaceutically acceptable salt thereof, or compositions comprising such compound or pharmaceutically acceptable salt thereof. Such additional pharmaceutical agents include, but are not limited to, anti-proliferative agents, anti-cancer agents, anti-diabetic agents, anti-inflammatory agents, immunosuppressant agents, and a pain-relieving agent The additional pharmaceutical agent(s) may synergistically augment inhibition of CDK7 or CDK12 and/or CDK13 induced by the inventive compounds or compositions of this invention in the biological sample or subject. Thus, the combination of the inventive compounds or compositions and the additional pharmaceutical agent(s) may be useful in treating proliferative diseases resistant to a treatment using the additional pharmaceutical agent(s) without the inventive compounds or compositions.

In yet another aspect, the present invention provides the compounds of formula (I) and pharmaceutically acceptable salts, solvates, hydrates, tautomers, stereoisomers, isotopically labeled derivatives, and compositions thereof, for use in the treatment of a proliferative disease in a subject. In certain embodiments, provided by the invention are the compounds described herein, and pharmaceutically acceptable salts and compositions thereof, for use in the treatment of a proliferative disease in a subject In certain embodiments, provided by the invention are the compounds described herein, and pharmaceutically acceptable salts and compositions thereof, for use in inhibiting cell growth. In certain embodiments, provided by the invention are the compounds described herein, and pharmaceutically acceptable salts and compositions thereof, for use in inducing apoptosis in a cell. In certain embodiments, provided by the invention are the compounds described herein, and pharmaceutically acceptable salts and compositions thereof, for use in inhibiting transcription.

### ExAMPLEs

In order that the invention described herein may be more fully understood, the following examples are set forth. The synthetic and biological examples described in this application are offered to illustrate the compounds, pharmaceutical compositions, and methods provided herein and are not to be construed in any way as limiting their scope.

**The** compounds provided herein can be prepared from readily available starting materials using modifications to the specific synthesis protocols set forth below that would be well known to those of skill in the art. It will be appreciated that where typical or preferred process conditions (i.e., reaction temperatures, times, mole ratios of reactants, solvents, pressures, *etc*.) are given, other process conditions can also be used unless otherwise stated. Optimum reaction conditions may vary with the particular reactants or solvents used, but such conditions can be determined by those skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in Greene et al., Protecting Groups in Organic Synthesis, Second Edition, Wiley, New York, 1991, and references cited therein.

### ABBREVIATIONS

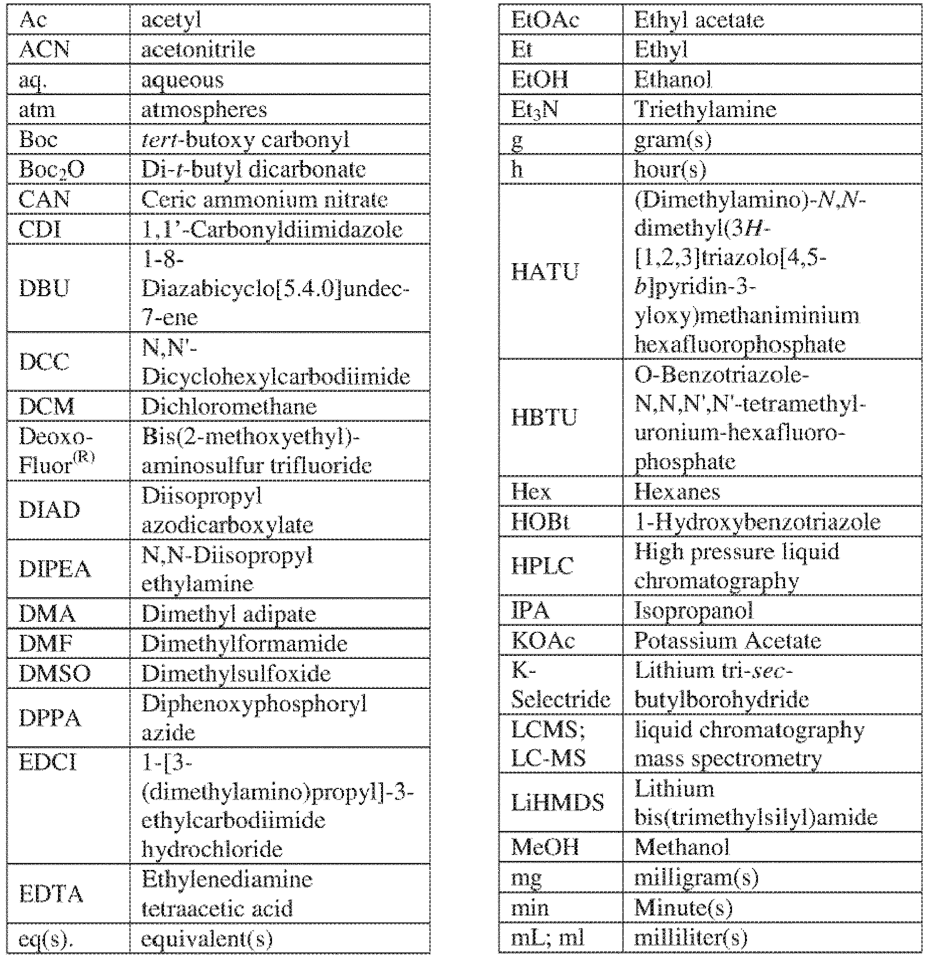

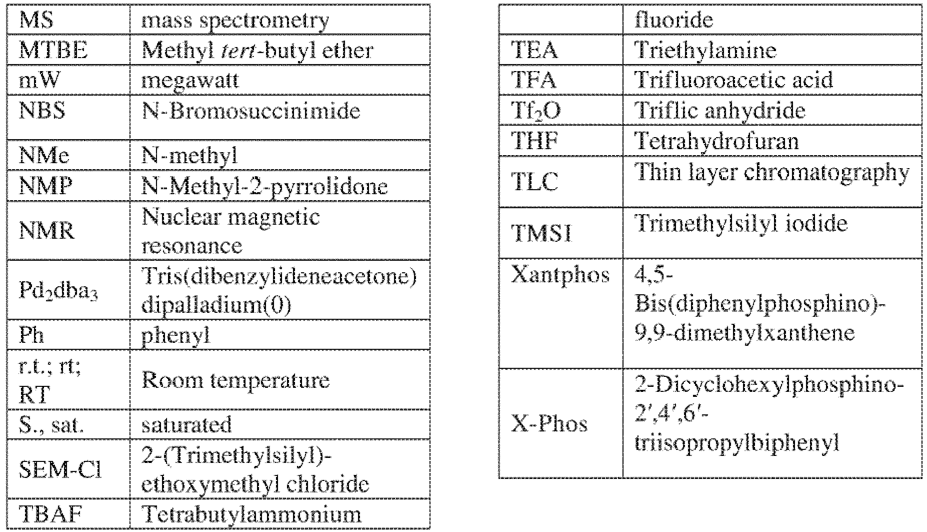

### Example 1. Synthesis of (S)5-chloro-4-(5,6-difluoro-1H-indol-3-yl)-N-(piperidin-3-yl)pyrimidin-2-amine.

### Step 1: 3-(2,5-dichloropyrimidin-4-yl)-5,6-difluoro-1H-indole

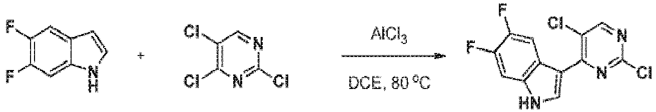

To a solution of 2,4,5-trichloropyrimidine (700 µL, 6.11 mmol) in dichloroethane (40 mL) was added aluminum chloride (940 mg, 7.05 mmol). The resulting suspension was stirred at 80 °C for 30 min. The reaction mixture was then allowed to cool to room temperature and 5,6-difluoroindole (1.00 g, 6.54 mmol) was added and the resulting solution was stirred at 80 °C for 18h. The reaction mixture was allowed to cool to room temperature and crushed ice (15 mL) was added. The obtained slurry was vigorously stirred for 30 min and then further diluted with EtOAc (200 mL) and water (60 mL). The mixture was warmed to dissolve the suspended solid, layers were separated and the aqueous layer was extracted with warm EtOAc (150 mL). The combined organic layers were dried over Na₂SO₄, filtered, and concentrated under reduced pressure. The resulting crude product was triturated in Et₂O to afford the title compound (1.49 g, 4.97 mmol, 81% yield) as a beige solid.

### Step 2: (S)-tert-butyl 3-((5-chloro-4-(5,6-difluoro-1H-mdol-3-yl)pyrimidin-2-yl)amino)piperidine-1-carboxylate

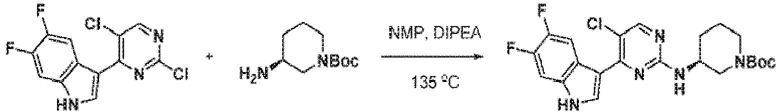

A solution of 3-(2,5-dichloropyrimidin-4-yl)-5,6-difluoro-1H-indole (136 mg, 0.45 mmol), *(S)-tert-butyl 3-aminopiperidine-1-carboxylate* (130 mg, 0.65 mmol), and diisopropylethylamine (0.24 mL, 1.38 mmol) in NMP (3.0 mL) was heated for 4 h at 135 °C in a microwave (MW) reactor. The mixture was diluted with EtOAc (150 mL), washed with water (50 mL) and brine (50 mL), dried over Na₂SO₄, filtered, and concentrated to dryness. The crude residue was purified by SiO₂ chromatography (EtOAc in DCM, 0 to 100% gradient) to afford the title compound (100 mg, 0.216 mmol, 48% yield) as a light yellow solid.

### Step 3: (S)-5-chloro-4-(5,6-difluoro-1H-indol-3-yl)-N-(piperidin-3-yl)pyrimidin-2-amine

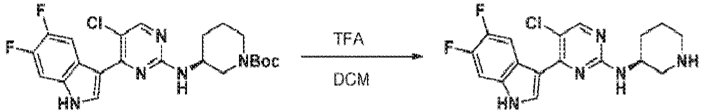

A solution of (S)-tert-butyl 3-((5-chloro-4-(5,6-difluoro-1H-indol-3-yl)pyrimidin-2-yl)amino)piperidine-1-carboxylate (100 mg, 0.216 mmol) DCM (2.0 mL) was treated with trifluoroacetic acid (150 µL, 1.96 mmol) and stirred overnight at r.t. The mixture was concentrated under reduced pressure and co-evaporated 3 times with DCM (20 mL). The obtained residue was neutralized with DIPEA (100 uL), purified by reverse phase chromatography (C18, H₂O/ACN +0.1% HCO₂H, 0 to 90% gradient) and afforded the title compound (60 mg, 0.165 mmol, 77% yield) as a off white solid solid after lyophilisation.

### Example 2. Synthesis of (S)-N-(5-chloro-4-(1H-indol-3-yl)pyrimidin-2-yl)quinuclidin-3-amine (Compound 131).

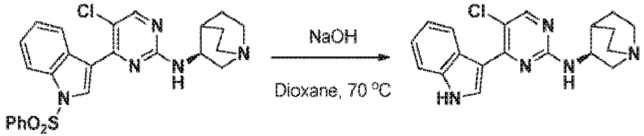

A solution of (S)-N-(5-chloro-4-(1-(phenylsulfonyl)-1H-indol-3-yl)pyrimidin-2-yl)quinuclidin-3-amine (50 mg, 0.101 mmol) and NaOH 5M (1 mL, 5.00 mmol) in dioxane (1.0 mL) was heated 6h at 70°C. The cooled mixture was then concentrated under reduced pressure. The crude residue was purified by reverse phase chromatography (C18, H₂O/ACN +0.1.% HCO₂H, 0 to 100% gradient) and afforded the title compound (24 mg, 0.067 mmol, 67% yield) as a white solid after lyophilisation.

### Example 3. Synthesis of 3-(5-bromo-2-chloropyrimidin-4-yl)-1-(phenylsulfonyl)-1H-indole.

### Step 1: 3-(2,5-dichloropyrimidin-4-yl)-5,6-difluoro-1H-indole

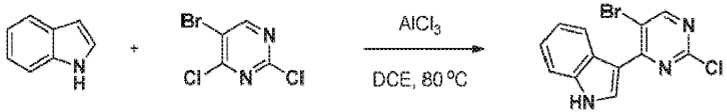

To a solution of 5-bromo-2,4-dichloropyrimidine (500 mg, 2.194 mmol) in dichloroethane (10 mL) was added aluminum chloride (328 mg, 2.46 mmol). The resulting suspension was stirred at 80 °C for 30 min. The reaction mixture was then allowed to cool to room temperature and indole (231 mg, 1.975 mmol) was added and the resulting solution was stirred at 80 °C for 18h. The reaction mixture was cooled to room temperature and crushed ice (15 mL) was added. The resulting slurry was vigorously stirred for 30 min and then further diluted with MeTHF (200 mL) and water (60 mL). The mixture was warmed to dissolve the suspended solid, layers were separated and the aqueous layer was extracted once more with MeTHF (150 mL). The combined organic layers were dried over Na₂SO₄, filtered, and concentrated under reduced pressure to afford the title compound (666 mg, 2.16 mmol, 98% yield) as an orange solid.

### Step 2: 3-(5-bromo-2-chloropyrimidin-4-yl)-1-(phenylsulfonyl)-1H-indole

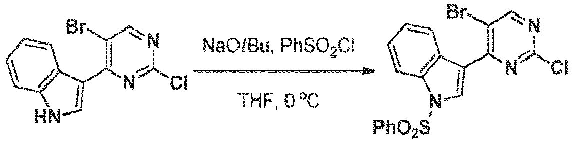

NaOtBu (249 mg, 2.59 mmol) was added to a stirring solution of 3-(2,5-dichloropyrimidin-4-yl)-5,6-difluoro-1H-indole (666 mg, 2.16 mmol) in THF (10 mL) 0°C. The resulting solution was stirred at 0°C for 30 min and then benzene sulfonyl chloride (330 µL, 2.59 mmol) was added dropwise. The resulting solution was stirred at 0°C for 30 min and then added dropwise to 50 mL of water and stirred for 30 min. A brown solid precipitated from the reaction mixture and was collected via filtration. The obtained solid was washed with hexanes (30 mL), minimal ether (5 mL) and dried under vaccum to afford the tile compound (666 mg, 1.48 mmol, δ9 % yield) as brownish solid.

### Example 4. Synthesis of (S)-tert-butyl 3-((5-cyclopropyl-4-(1-(phenylsulfonyl)-1H-indol-3-yl)pyrimidin-2-yl)amino)piperidine-1-carboxylate.

### Step 1: (S)-tert-butyl 3-((5-chloro-4-(1-(phenylsulfonyl)-1H-indol-3-yl)pyrimidin-2-yl)amino)piperidine-1-carboxylate

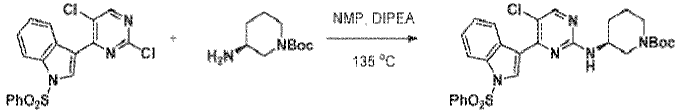

A solution of 3-(2,5-dichloropyrimidin-4-yl)-1-(phenylsulfonyl)-1H-indole (700 mg, 1.73 mmol), *(S)-tert-butyl 3-aminopiperidine-1-carboxylate* (382 mg, 1.91 mmol), and diisopropylethylamine (0.60 mL, 3.46 mmol) in NMP (9.0 mL) was heated for 45 min at 135 °C in a microwave (MW) reactor. The mixture was diluted with EtOAc (200 mL), washed with water (50 mL) and brine (50 mL), dried over Na₂SO₄, filtered, and concentrated under reduced pressure to a yellow oil. The residue was purified by SiO₂ chromatography (EtOAc in DCM, 0 to 100% gradient) to afford the title compound (694 mg, 1.22 mmol, 64% yield) as a light pink solid.

### Step 2: (S)-tert-butyl 3-((5-cyclopropyl-4-(1-(phenylsulfonyl)-1H-indol-3-yl)pyrimidin-2-yl)amino)piperidine-1-carboxylate

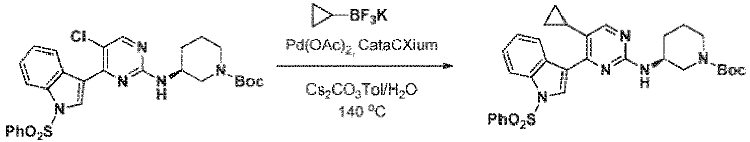

A degassed solution of (S)-tert-butyl 3-((5-chloro-4-(1-(phenylsulfonyl)-1H-indol-3-yl)pyrimidin-2-yl)amino)piperidine-1-carboxylate (142 mg, 0.25 mmol), Cs₂CO₃ (244 mg, 0.75 mmol) and potassium cyclopropyltrifluoroborate (111 mg, 0.75 mmol) in 211 toluene/H₂O (6 mL) was treated with a premixed solution of Pd(OAc)₂ (2.8 mg, 0.013 mmol) and butyldi-1-adamantylphosphine (9.0 mg, 0.025 mmol) in degassed toluene (2 mL). The resulting solution was heated at 140°C for 2h. The cooled mixture was then diluted with EtOAc (50 mL) and saturated NaHCO₃ (50 mL). The layers were separated and the aqueous layer was extracted with EtOAc (2 × 50 mL). The combined organic layers were dried over Na₂SO₄, filtered and evaporated to dryness. The residue was purified by SiO₂ chromatography (EtOAc in DCM 0 to 50% gradient) and afforded the title compound (105 mg, 0.183 mmol, 73% yield) as a yellow foam.

### Example 5. Synthesis of (S)-5-cbloro-N-(1-methylpiperidin-3-yl)4-(1-(phenylsulfonyl)-1H-indol-3-yl)pyrimidin-2-amine.

### Step 1: (S)-5-chloro-4-(1-(phenylsulfonyl)-1H-indol-3-yl)-N-(piperidin-3-yl)pyrimidin-2-amine

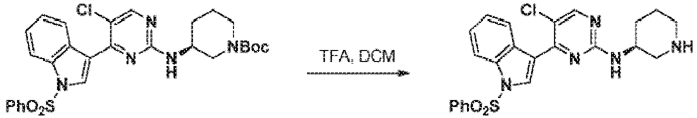

A solution of (S)-tert-butyl 3-((5-chloro-4-(1-(phenylsulfonyl)-1H-indol-3-yl)pyrimidin-2-yl)amino)piperidine-1-carboxylate (272 mg, 0.479 mmol) in DCM (4.8 mL) was treated with trifluoroacetic acid (1.83 mL, 24.0 mmol) and stirred overnight at r.t. The mixture was concentrated under reduced pressure and co-evaporated 3 times with DCM (20 mL). The obtained residue was dissolved in MeTHF (20 mL), washed with NaHCO₃ (2X20 mL), the phases were separated, dried over Na₂SO₄, filtered and concentrated under reduced pressure to afford the title compound (224 mg, 0.479 mmol, 100%) as a yellow foam, used without further purification.

### Step 2: (S)-5-chloro-N-(1-methylpiperidin-3-yl)-4-(1-(phenylsulfonyl)-1H-indol-3-yl)pyrimidin-2-amine

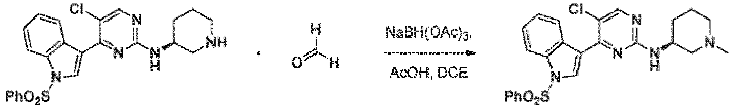

A solution of (S)-5-chloro-4-(1-(phenylsulfonyl)-1H-indol-3-yl)-N-(piperidin-3-yl)pyrimidin-2-amine (112 mg, 0.24 mmol) in DCE (3.0 mL) was added acetic acid (7 uL, 0.12 mmol) followed by the addition of the paraformaldehyde (9 mg, 0.31 mmol). The reaction mixture was stirred at r.t. for 10 minutes followed by the addition of NaBH(OAc)₃ (91 mg, 0.43 mmol) in one portion and the resulting mixture was stirred at room temperature for 24 hours. The mixture was diluted with DCM (20 mL), washed with water (10 mL) and brine (10 mL), dried over Na₂SO₄, filtered, and concentrated under reduced pressure to afford the title compound (111 mg, 0.23 mmol, 96% yield) as an yellowish foam.

### Example 6. Synthesis of (S)-tert-butyl 3-((5-ethyl-4-(7-fluoro-1H-indol-3-yl)pyrimidin-2-yl)amino)piperidine-1-carboxylate.

### Step 1: (S)-tert-butyl 3-((4-(7-fluoro-1H-indol-3-yl)-5-vinylpyrimidin-2-yl)amino)piperidine-1-carboxylate

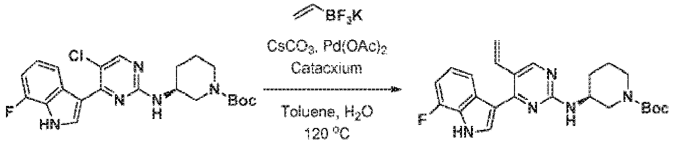

To a mixture of (S)-tert-butyl 3-((5-chloro-4-(7-fluoro-1H-indol-3-yl)pyrimidin-2-yl)amino)piperidine-1-carboxylate (99 mg, 0.22 mmol), potassium vinyltrifluoroborate (91 mg, 0.67 mmol) and Cs₂CO₃ (217 mg, 0.67 mmol) in degassed toluene (4 mL) and H₂O (3 mL) was added Catacxium (8.0 mg, 0.022 mmol) and Pd(OAc)₂ (2.5 mg, 0.011 mmol) under Nitrogen. The resulting solution was heated at 120 °C for 4h. The cooled mixture was diluted with EtOAc (50 mL) and water (50 mL). The layers were separated and the aqueous layer was extracted with EtOAc (2 x 50 mL). The combined organic layers were dried over Na₂SO₄, filtered and evaporated to dryness. The residue was purified by SiO₂ chromatography (THF in DCM 0 to 60% gradient) and afforded the title compound (59 mg, 0.13 mmol, 61% yield) as a yellow foam.

### Step 2: (S)-tert-butyl 3-((5-ethyl-4-(7-fluoro-1H-indol-3-yl)pyrimidin-2-yl)amino)piperidine-1-carboxylate

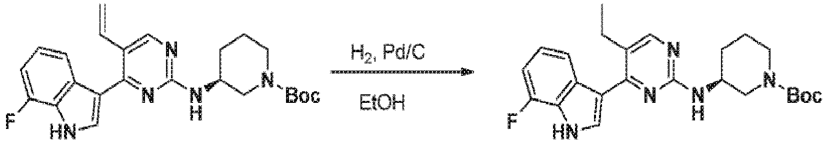

To a solution of (S)-tert-butyl 3-((4-(7-fluoro-1H-indol-3-yl)-5-vinylpyrimidin-2-yl)amino)piperidine-1-carboxylate (59 mg, 0.133 mmol) in EtOH (13 mL) was added Pd/C (10% wlw on activated carbon). The reaction mixture was allowed to stir under a positive pressure of hydrogen (1 atm) at room temperature for 16h. The resulting solution was degassed with N₂, then filtered through celite and concentrated under reduced pressure to afford the title compound (59 mg, 0.13 mmol, 100 % yield) as a light yellow oil.

### Example 7. Synthesis of (S)-5-chloro-N-(1-isopropylpiperidin-3-yl)-4-(1-(phenylsulfonyl)-1H-indol-3-yl)pyrimidin-2-amine.

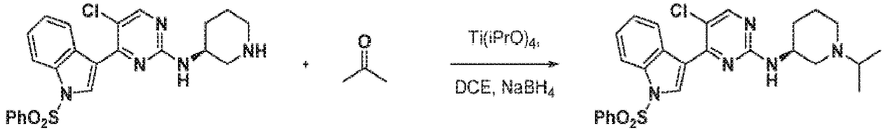

To a solution of (S)-5-chloro-4-(1-(phenylsulfonyl)-1H-indol-3-yl)-N-(piperidin-3-yl)pyrimidin-2-amine (100 mg, 0.21 mmol) and anhydrous acetone (24 µL, 0.32 mmol) in DCE (3.0 mL) was added titanium isopropoxyde (0.32 mL, 1.07 mmol). The reaction mixture was allowed to stir overnight at room temperature. NaBH₄ (24 mg, 0.64 mmol) was then added and the mixture was stirred for 6h. The reaction mixture was then diluted with DCM (150 mL), a saturated aqueous solution of NaHCO₃ (30 mL) and filtered through! celite. The phases were then separated, dried over Na₂SO₄, filtered and concentrated under reduced pressure to afford the title compound (109 mg, 0.21 mmol, 100% yield) as a pale yellow oil.

### Example 8. Synthesis of (S)-5-chloro-N-(1-(2-methoxyethyl)piperidin-3-yl)-4-(1-(phenylsulfonyl)-1H-indol-3-yl)pyrimidin-2-amine.

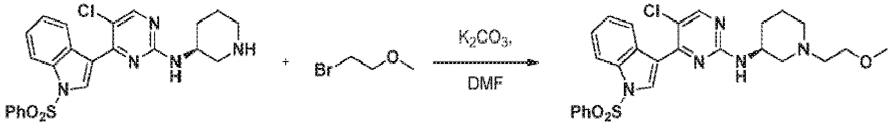

To a solution of (S)-5-chloro-4-(1-(phenylsulfonyl)-1H-indol-3-yl)-*N*-(piperidin-3-yl)pyrimidin-2-amine (100 mg, 0.21 mmol) and potassium carbonate (37 mg, 0.27 mmol) in DMF (2.1 mL) at 0 °C was added 2-bromoethylmethylether (25 uL, 0.27 mmol). The reaction mixture was allowed to stir overnight at room temperature. It was then quenched with saturated aqueous solution of NaHCO₃ (20 mL) and extracted with EtOAc (3X30 mL). The organic layers were combined, washed with water (2X20 mL) dried over Na₂SO₄, filtered and concentrated under reduced pressure to afford the title compound (92 mg, 0.18 mmol, 82% yield) as a yellow oil.

### Example 9. Synthesis of (S)-tert-butyl-3-((5-cyano-4-(1-(phenylsulfonyl)-1H-indol-3-yl)pyrimidin-2-yl)amino)piperidine-1-carboxylate.

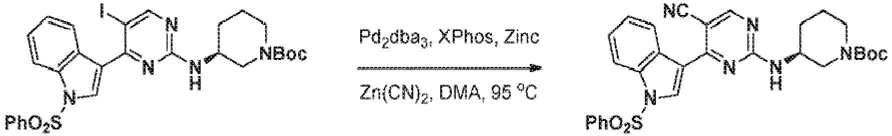

A premixed solution of Zn (2.5 mg, 0.04 mmol), Pd₂dba₃ (35 mg, 0.04 mmol), X-Phos (36mg, 0.08 mmol) and Zn(CN)₂ (27 mg, 0.23 mmol) in degassed DMA (3.6 mL), heated at 95 °C for 10 min was added to a degassed solution of (S)-tert-butyl 3-((5-iodo-4-(1-(phenylsulfonyl)-1H-indol-3-yl)pyrimidin-2-yl)amino)piperidine-1-carboxylate (250 mg, 0.379 mmol) in DMA (4 mL). The reaction mixture was stirred at 95 °C for 18h. The cooled solution was diluted with EtOAc (20 mL) and washed with H₂O (3x5 mL), brine (5 mL), dried over Na₂SO₄, filtered, and concentrated to dryness. The residue was purified by SiO₂ chromatography (EtOAc in DCM, 0 to 50% gradient) to afford the title compound (212 mg, 0.38 mmol, 100% yield) as a light yellow solid.

### Example 10. Synthesis of (S)-tert-butyl 3-((4-(1-(phenylsulfonyl)-1H-indol-3-yl)-5-(2,2,2-trifluoroethyl)pyrimidin-2-yl)amino)piperidine-1-carboxylate.

### Step 1: (S)-tert-butyl-3-((4-(1-(phenylsulfonyl)-1H-indol-3-yl)-5-(4,4,5,5-tetramethyl-1,3,2-dioxaborolan-2-yl)pyrimidin-2-yl)amino)piperidine-1-carboxylate

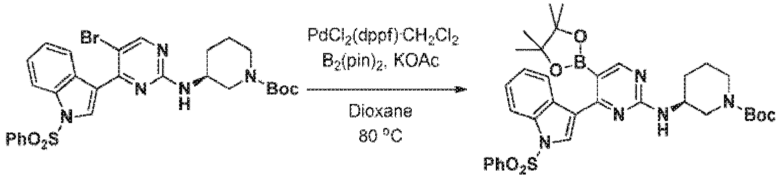

A stirred solution of (S)-tert-butyl 3-({5-bromo-4-(1-(phenylsulfonyl)-1H-indol-3-yl)pyrimidia-2-yl)amino)piperidine-1-carboxylate (900 mg, 1.47 mmol), bis(pinacolato)diboron (933 mg, 3.67 mmol) and potassium acetate (721 mg, 7.35 mmol) in dioxane (11 mL) was degassed with nitrogen for 10 minutes. 1, 1'- Bis(diphenylphosphino)ferrocene palladium(II) dichloride dichloromethane complex (120 mg, 0.147 mmol) was then added and the reaction was heated at 80°C for 4 hours. The cooled mixture was then diluted with EtOAc (100 mL) and water (50 mL). The layers were separated and the aqueous layer was extracted with EtOAc (2 × 100 mL). The combined organic layers were dried over Na₂SO₄, filtered and evaporated to dryness to afford the title compound (969 mg, 1.47 mmol, 100% yield) as a brown solid.

### Step 2: (S)-tert-butyl 3-((4-(1-(phenylsulfonyl)-1H-indol-3-yl)-5-(2,2,2-trifluoroethyl)pyrimidin-2-yl)amino)piperidine-1-carboxylate

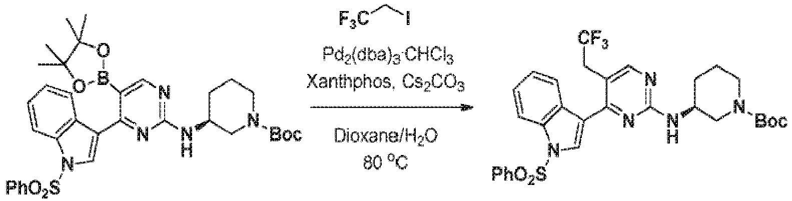

A solution of Pd₂(dba)₃CHCl₃ (76 mg, 0.074 mmol), XantPhos (145 mg, 0.250 mmol) and Cs₂CO₃ (1.92 g, 5.88 mmol) in dioxane (7 mL) were added to a stirring solution of (S)-tert-butyl-3-((4-(1-(phenylsulfonyl)-1H-indol-3-yl)-5-(4,4,5,5-tetramethyl-1,3,2-dioxaborolan-2-yl)pyrimidin-2-yl)amino)piperidine-1-carboxylate (969 mg, 1.47 mmol) and ICH₂CF₃ (350 µL, 2.938 mmol) in degassed dioxane (8 mL) and water (0.98 mL, 54.4 mmol) under nitrogen. The reaction was heated at 80°C for 18h. The cooled mixture was then diluted with EtOAc (100 mL) and water (50 mL). The layers were separated and the aqueous layer was extracted with EtOAc (2 × 50 mL). The combined organic layers were dried over Na₂SO₄, filtered and evaporated to dryness. The residue was purified by SiO₂ chromatography (EtOAc in DCM 0 to 100% gradient) and afforded the title compound (304 mg, 0.49 mmol, 34% yield) as a light brown foam.

### Example 11. Synthesis of (S)-tert-butyl 3-((4-(7-(pyridin-4-yl)-1H-indol-3-yl)-5-(trifluoromethyl)pyrimidin-2-yl)amino)piperidine-1-carboxylate.

### Step 1: tert-butyl 7-bromo-1H-indole-1-carboxylate

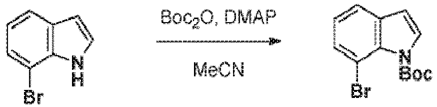

A solution of 7-bromoindole (10.0 g, 51.0 mmol), di-tert-butyl dicarbonate (12.2 g, 56.1 mmol) and DMAP (623 mg, 5.1 mmol) in acetonitrile (100 mL) was stirred at room temperature for 72 h. The reaction mixture was concentrated under reduced pressure, diluted with EtOAc (500 mL), washed with water (300 mL) and brine (2X300 mL). The phases were separated and dried over Na₂SO₄, concentrated under reduced pressure to afford the title compound (15.1 g, 51.0 mmol, 100% yield) as a yellowish oil.

### Step 2: tert-butyl 7-bromo-3-(4,4,5,5-tetramethyl-1,3,2-dioxaborolan-2-yl)-1H-indole-1-carboxylate

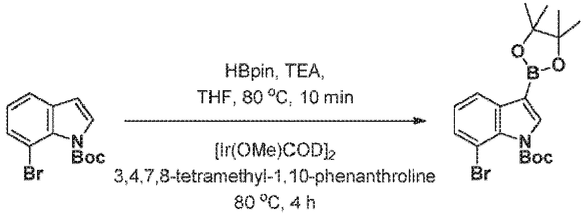

Under an inert atmosphere, to a solution of tert-butyl 7-bromo-1H-indole-1-carboxylate (5.0 g, 16.9 mmol) in previously degassed THF (17 mL) were added HBpin (4.9 mL, 33.8 mmol) and Et₃N (2.4 mL, 16.9 mmol). The reaction vessel was sealed and heated at 80 °C for 10 minutes. [Ir(OMe)COD]₂ (2.8 mg, 0.25 mol%) and 3,4,7,8-tetramethyl-1,10-phenanthroline (4.0 mg, 1.0 mol%) were added and the reaction vessel was sealed and heated at 80 °C overnight The reaction mixture was allowed to return to room temperature, diluted with EtOAc (300 mL) and washed with brine (100 mL). The organic layer was dried over Na₂SO₄ and concentrated under reduced pressure to afford the title compound (5.6 g, 16.9 mmol, 100% yield) as a brown semi-solid.

### Step 3: (S)-tert-butyl 3-((4-(7 bmmo-1H-indol-3-yl)-5-(trifluoromethyl)pyrimidin-2-yl)amino)piperidine-1-carboxylate

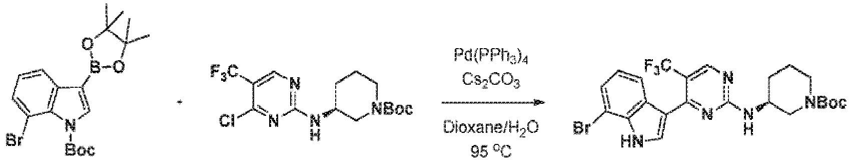

Pd(PPh₃)₄ (376 mg, 0.195 mmol) and Cs₂CO₅ (3.82 g, 11.7 mmol) were added to a stirring solution of (S)-tert-butyl 3-((4-chloro-5-(trifluoromethyl)pyrimidin-2-yl)amino)piperidine-1-carboxylate (PCT Int. Appl., 2014124230, 14 Aug 2014) (1.56 g, 4.10 mmol) and *tert*-butyl 7-bromo-3-(4,4,5,5-tetramethyl-1,3,2-dioxaborolan-2-yl)-1H-indole-1-carboxylate (1.65 g, 3.91 mmol) in Dioxane/H₂O previously degassed (65 mL, 2:1). The resulting mixture was heated at 95 °C under N₂ for 2h. The reaction mixture was allowed to cool to room temperature, water (100 mL) and EtOAc (100 mL) were added. The phases were separated and the aqueous extracted with EtOAc (2X150 mL). The combined organic layers were dried over Na₂SO₄, filtered and evaporated to dryness to a orange oil. The residue was purified by reverse phase chromatography (C18, H₂O/ACN +0.1% HCO₂H, 0 to 80% gradient) and afforded the title compound (343 mg, 0.635 mmol, 16% yield) as as a light yellow foam.

### Step 4: (S)-tert-butyl 3-((4-(7-(pyridin-4-yl)-1H-indol-3-yl)-5-(trifluoromethyl)pyrimidin-2-yl)amino)piperidine-1-carboxylate

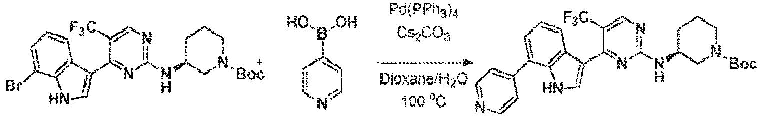

Pd(PPh₃)₄ (16 mg, 0.014 mmol) and Cs₂CO₃ (91 mg, 0.28 mmol) were added to a stirring solution of (S)-tert-butyl 3-((4-(7-bromo-1H-indol-3-yl)-5-(trifluoromethyl)pyrimidin-2-yl)amino)piperidine-1-carboxylate (80 mg, 0.14 mmol) and pyridin-4-ylboronic acid (20 mg, 0.15 mmol) in Dioxane/H₂O previously degassed (1.5 mL, 2:1). The resulting mixture was then heated at 100 °C under N₂ for 18h. The reaction mixture was allowed to cool to room temperature, water (500 mL) and EtOAc (500 mL) were added. The phases were separated and the aqueous extracted twice more with EtOAc (50 mL). The combined organic layers were dried over Na₂SO₄, filtered and evaporated to dryness to a orange oil. The residue was purified by SiO₂ chromatography (MeOH in DCM 0 to 20% gradient) and afforded the title compound (40 mg, 0.070 mmol, 50% yield) as a yellow solid.

### Example 12. Synthesis of (S)-tert-butyl 3-((4-(7-(1-methyl-1H-pyrazol-4-yl)-1H-indol-3-yl)-5-(trifluoromethyl)pyrimidin-2-yl)amino)piperidine-1-carboxylate.

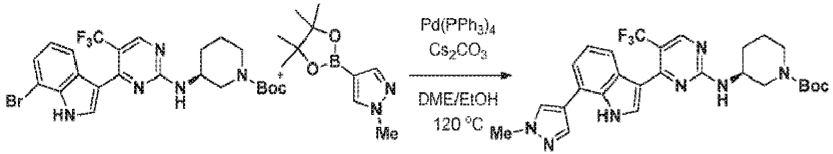

Pd(PPh₃)₄ (5.0 mg, 0.003 mmol) and Cs₂CO₃ (51 mg, 0.16 mmol) were added to a stirring solution of (S)-tert-butyl 3-((4-(7-bromo-1H-indol-3-yl)-5-(trifluoromethyl)pyrimidin-2-yl)amino)piperidine-1-carboxylate (28 mg, 0.052 mmol) and 1-methyl-4-(4,4,5,5-tetramethyl-1,3,2-dioxaborolan-2-yl)-1H-pyrazole (28 mg, 0.052 mmol) in DME/EtOH previously degassed (1.8 mL; 2:1). The resulting mixture was then heated was heated for 30 min at 100 °C in a microwave (MW) reactor. The reaction mixture was allowed to cool to room filtered through celite and evaporated to dryness. The residue was purified by SiO₂ chromatography (EtOAc in DCM 0 to 85% gradient) and afforded the title compound (24.0 mg, 0.044 mmol, 85% yield) as a yellow oil.

### Example 13. Synthesis of (S)-tert-butyl 3-((4-(7-(diethoxyphosphoryl)-1H-indol-3-yl)-5-(trifluoromethyl)pyrimidin-2-yl)amino)piperidine-1-carboxylate.

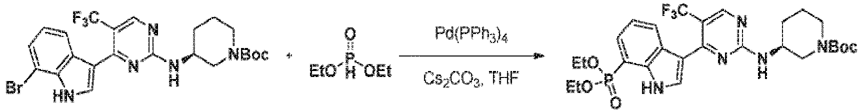

To a mixture of (S)-tert-butyl 3-((4-(7-bromo-1H-indol-3-yl)-5-(trifluoromethyl)pyrimidin-2-yl)amino)piperidine-1-carboxylate (81 mg, 0.15 mmol), Cs₂CO₃ (58 mg, 0.18 mmol) and tetrakistriphenylphosphine palladium (16 mg, 0.014 mmol) in a microwave vial under an inert atmosphere was added THF (1.2 mL) and diethylphosphonate (36 uL, 0.28 mmol). The reaction vessel was sealed and heated to 120 °C under microwave irradiation for 10 minutes. The reaction mixture was cooled to room temperature, the solids were filtered off and washed with DCM (10 mL). The combined filtrates and washings were concentrated evaporated to dryness. The residue was purified by SiO₂ chromatography (MeOH in DCM, 0 to 20% gradient) to afford the title compound (54 mg, 0.090 mmol, 60% yield) as a yellowish solid.

### Example 14. Synthesis of (S)-tert-butyl 3-((4-(7-(4-methyl-1H-imidazol-1-yl)-1H-indol-3-yl)-5-(trifluoromethyl)pyrimidin-2-yl)amino)piperidine-1-carboxylate.

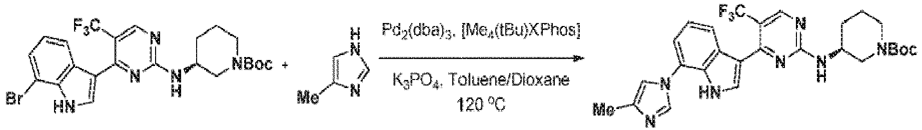

A solution of Pd₂(dba)₃ (7.2 mg, 0.008 mmol) and Me₄(t-Bu)₂XPhos (7.6 mg, 0.016 mmol) in degassed toluene (0.8 mL) and dioxane (0.2 mL) was stirred for 5 min at 120 °C. To this solution was added (S)-*tert*-butyl 3-((4-(7-bromo-1H-indol-3-yl)-5-(trifluoromethyl)pyrimidin-2-yl)amino)piperidine-1-carboxylate (126 mg, 0.20 mmol), 4-methyl-1H-imidazole (25 mg, 0.30 mmol) and K₃PO₄ (84 mg, 0.39 mmol). The reaction vessel was sealed and heated at 120 °C overnight. The reaction mixture was filtered and concentrated to dryness. The reaction mixture was purified by SiO₂ chromatography (MeOH in DCM, 0 to 20% gradient) to afford the title compound (35 mg, 0.065 mmol, 33% yield) as a beige solid.

### Example 15. Synthesis of (S)-tert-butyl 3-((4-(7-(3-methyl-2-oxoimidazolidin-1-yl)-1H-indol-3-yl)-5-(trifluoromethyl)pyrimidin-2-yl)amino)piperidine-1-carboxylate.

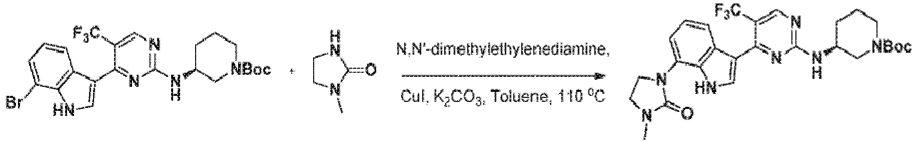

To a mixture of (S)-tert-butyl 3-((4-(7-bromo-1H-indol-3-yl)-5-(trifluoromethyl)pyrimidin-2-yl)amino)piperidine-1-carboxylate (150 mg, 0.28 mmol), 1-methylimidazolidin-2-one (56 mg, 0.56 mmol) in degassed toluene (1.3 mL) was added K₂CO₃ (77 mg, 0.56 mmol), CuI (5.3 mg, 0.03 mmol) and N,N'-dimethylethane-1,2-diamine (6 uL, 0.06 mmol). The reaction vessel was sealed and heated at 110 °C overnight. The reaction mixture was cooled to room temperature, diluted with EtOAc (20 mL), washed with water (30 mL) and brine (30 mL). The phases were separated and the organic layer was dried over Na₂SO₄ and concentrated to dryness. The reaction mixture was purified by SiO₂ chromatography (EtOAc in DCM, 0 to 100% gradient) to afford the title compound (52 mg, 0.093 mmol, 33% yield) as a yellow solid.

### Example 16. Synthesis of (S)-tert-butyl 3-((4-(7-(ethoxy(methyl)phosphoryl)-1H-indol-3-yl)-5-(trifluoromethyl)pyrimidin-2-yl)amino)piperidine-1-carboxylate.

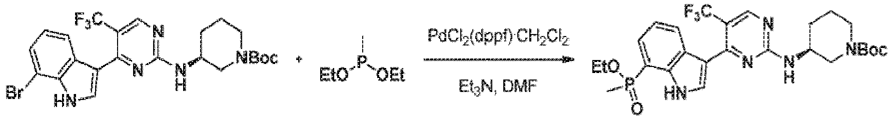

To a degassed solution of (S)-fert-butyl 3-((4-(7-bromo-1H-indol-3-yl)-5-(trifluoromethyl)pyrimidin-2-yl)amino)piperidine-1-carboxylate (51 mg, 0.09 mmol), Et₃N (21 uL, 0.15 mmol) and 1,1'- Bis(diphenylphosphino)ferrocene palladium(II) dichloride dichloromethane complex (7.7 mg, 0.009 mmol) in DMF (0.4 mL), in a microwave vial was added diethyl methylphosphonite (18 uL, 0.12 mmol). The vial was sealed and immediately heated to 130 °C under microwave irradiation for 5 minutes. The reaction mixture was cooled to room temperature and diluted with EtOAc (10 mL), washed with a saturated aqueous solution of NaHCO₃ (15 mL) and brine (15 mL). The phases were separated, dried over Na₂SO₄, filtered and concentrated under reduced pressure. The reaction mixture was purified by SiO₂ chromatography (MeOH in DCM, 0 to 20% gradient) to afford the title compound (53 mg, 0.093 mmol, 98% yield) as a brown oil.

### Example 17. Synthesis of (S)-tert-butyl 3-((4-(7-(pyridazin-4-yl)-1H-indol-3-yl)-5-(trifluoromethyl)pyrimidin-2-yl)amino)piperidine-1-carboxylate.

### Step 1: (S)-tert-butyl 3-((4-(7-(4,4,5,5-tetramethyl-1,3,2-dioxaborolan-2-yl)-1H-indol-3-yl)-5-(trifluoromethyl)pyrimidin-2-yl)amino)piperidine-1-carboxylate

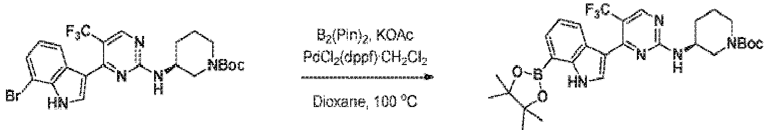

To a degassed solution of (S)-tert-butyl 3-((4-(7-bromo-1H-indol-3-yl)-5-(trifluoromethyl)pyrimidin-2-yl)amino)piperidine-1-carboxylate (100 mg, 0.19 mmol), bis(pinacolato)diboron (117 mg, 0.46 mmol) and potassium acetate (91 mg, 0.93 mmol) in degassed dioxane (1.5 mL) was added 1, 1'- Bis(diphenylphosphino)ferrocene palladium(II) dichloride dichloromethane complex (1.5 mg, 0.019 mmol). The reaction vessel was sealed and heated at 100 °C overnight. The reaction mixture was cooled to room temperature, diluted with ethyl acetate (30 mL) washed with water (20 mL) and brine (20 mL). The combined organic layers were dried over Na₂SO₄, filtered and concentrated under reduced pressure to afford the title compound (109 mg, 0.19 mmol) as a brown foam.

### Step 2: (S)-tert-butyl 3-((4-(7-(pyridazin-4-yl)-1H-indol-3-yl)-5-(trifluoromethyl)pyrimidin-2-yl)amino)piperidine-1-carboxylate

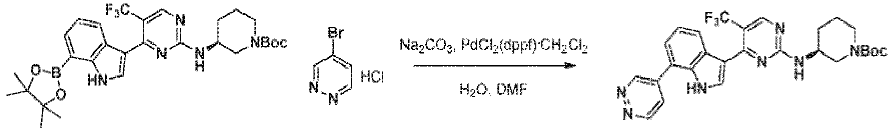

To a solution of (S)-tert-butyl 3-((4-(7-(4,4,5,5-tetramethyl-1,3,2-dioxaborolan-2-yl)-1H-indol-3-yl)-5-(trifluoromethyl)pyrimidin-2-yl)amino)piperidine-1-carboxylate (109 mg, 0.19 mmol) in DMF (3.0 mL) was added 4-bromopyridazine hydrochloride (43 mg, 0.22 mmol), Na₂CO₃ (53 mg, 0.50 mmol) and water (250 µl). Nitrogen was bubbled through the mixture for 15 minutes, [1,1'-bis(diphenyl-phosphino)ferrocene] palladium(II) chloride DCM complex (15 mg, 0.019 mmol) was added and the reaction mixture was heated at 80°C for 2h. To the reaction mixture, 4-bromopyridazine hydrochloride (50 mg, 0.26 mmol), Na₂CO₃ (50 mg, 0.47 mmol) and [1,1'-bis(diphenyl-phosphino)ferrocene] palladium(II) chloride DCM complex (8 mg, 0.009 mmol) were added and the reaction mixture was stirred at the same temperature for 3h. The reaction mixture was cooled to room temperature, diluted with EtOAc (20 mL), washed with saturated NaHCOa (20 mL) and brine (20 mL). The phases were separated and dried over Na₂SO₄, filtered, and concentrated under reduced pressure. The reaction mixture was purified by SiO₂ chromatography (EtOAc in DCM, 0 to 100% gradient) to afford the title compound (62 mg, 0.12 mmol, 57% yield) as a grey-beige solid.

### Example 18. Synthesis of (S)-tert-butyl 3-(2-((1-(tert-butoxycarbonyl)piperidin-3-yl)amino)-5-(trifluoromethyl)pyrimidin-4-yl)-5,6-difluoro-1H-indole-1-carboxylate.

### Step 1: tert-butyl 5,6-difluoro-3-(2-(methylsulfonyl)-5-(trifluoromethyl)pyrimidin-4-yl)-1H-indole-1-carboxylate

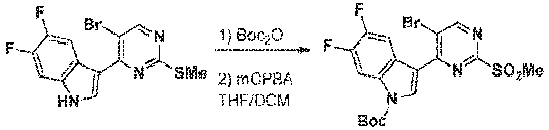

Di-tert-butyl dicarbonate (45 mg, 0.20 mmol) and DMAP (3.3 mg, 0.027 mmol) were added to a stirring solution of 5,6-difluoro-3-(2-(methyltbio)-5-(trifluoromethyl)pyrimidin-4-yl)-1H-indole (47 mg, 0.14 mmol) in THF (1 mL). The reaction mixture was stirred for 1h and was diluted with EtOAc (50 mL), washed with a saturated aqueous solution NaHCO₃ and the phases were separated. The organic layer was dried over Na₂SO₄, filtered and evaporated to dryness to a orange oil. The oil was dissolved with dry THF/DCM (2 mL, 1:1), mCPBA (92 mg, 0.41 mmol) was added and to the reaction mixture was stirred at room temperature for 16h. The reaction mixture was evaporated to dryness and was purified by SiO₂ chromatography (EtOAc in Hexanes, 0 to 100% gradient) to afford the title compound (30 mg, 0.063 mmol, 46% yield) as a yellowish solid.

### Step 2: (S)-tert-butyl 3-(2-((1-(tert-butoxycarbonyl)piperidin-3-yl)amino)-5-(trifluoromethyl)pyrimidin-4-yl)-5,6-difluoro-1H-indole-1-carboxylate

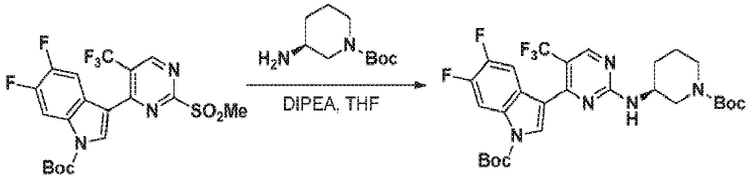

(S)-tert-butyl 3-aminopiperidine-1-carboxylate (14 mg, 0.069 mmol), and diisopropylethylamine (12 µL, 0.069 mmol) were added to a stirring solution of tert-butyl 5,6-difluoro-3-(2-(methylsulfonyl)-5-(trifluoromethyl)pyrimidin-4-yl)-1H-indole-1-carboxylate (30 mg, 0.063 mmol) in THF (1 mL). The reaction mixture was stirred for 1h at room temperature. The mixture was diluted with EtOAc (50 mL), washed with a saturated solution of NaHCO₃ (50 mL) and brine (50 mL). The organic layer was dried over Na₂SO₄, filtered, and concentrated under reduced pressure to a afford the title compound (38 mg, 0.063 mmol, 100% yield) as a yellow solid.

### Example 19. Synthesis of (S)-tert-butyl 3-((4-(7-((1r,4S)-4-hydroxycyclohexyl)-1H-indol-3-yl)-5-(trifluoromethyl)pyrimidin-2-yl)amino)piperidine-1-carboxylate.

### Step 1: (S)-tert-butyl 3-((4-(7-(1,4-dioxaspiro[4.5]decan-8-yl)-1H-indol-3-yl)-5-(trifluoromethyl)pyrimidin-2-yl)amino)piperidine-1-carboxylate

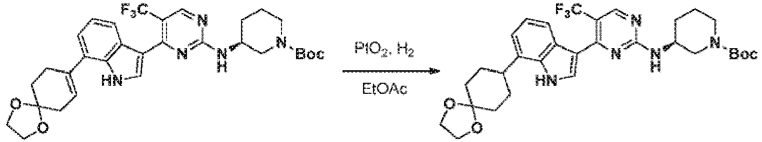

PtO₂ (100 mg, 75 m²/g) was added to a stirring solution of (S)-tert-butyl 3-((4-(7-(1,4-dioxaspiro[4.5]dec-7-en-8-yl)-1H-indol-3-yl)-5-(trifluoromethyl)pyrimidin-2-yl)amino)piperidine-1-carboxylate (260 mg, 0.434 mmol) in EtOAc (15 mL). The resulting solution was stirred under hydrogen (1 atm) for 72h. The mixture was filtered thru celite which was washed with EtOAc (50 mL) and MeOH (20 mL). The combined filtrates were evaporated to dryness to afford the title compound (162 mg, 0.269 mmol, 62% yield) as a brown foam.

### Step 2: (S)-tert-butyl 3-((4-(7-(4-oxocyclohexyl)-1H-indol-3-yl)-5-(trifluoromethyl)pyrimidin-2-yl)amino)piperidine-1-carboxylate

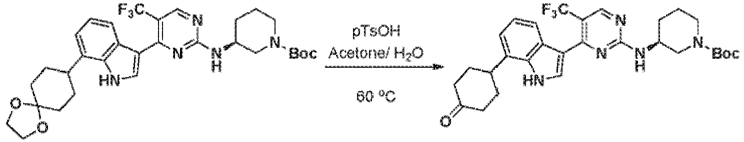

To a solution of (S)-tert-butyl 3-((4-(7-(1,4-dioxaspiro[4.5]decan-8-yl)-1H-indol-3-yl)-5-(trifluoromethyl)pyrimidin-2-yl)amino)piperidine-1-carboxylate (162 mg, 0.27 mmol) in acetone (5 mL) were added water (0.5 mL) and p-toluenesulfonic acid monohydrate (26 mg, 0.135 mmol). The reaction mixture was stirred at 60 °C for 3h. The reaction mixture was cooled to room temperature; a saturated aqueous solution of NaHCO₃ (50 mL) and EtOAc (100 mL) were added. The phases were separated and the aqueous layer was extracted with EtOAc (2X50 mL). The combined organic layers were dried over Na₂SO₄, filtered and evaporated to dryness. The residue was purified by SiO₂ chromatography (EtOAc in DCM 0 to 100% gradient) and afforded the title compound (62 mg, 0.11 mmol, 41% yield) as a white foam.

### Step 3: (S)-tert-butyl 3-((4-(7-((1r,4S)-4-hydroxycyclohexyl)-1H-indol-3-yl)-5-(trifluoromethyl)pyrimidin-1-yl)amino)piperidine-1-carboxylate

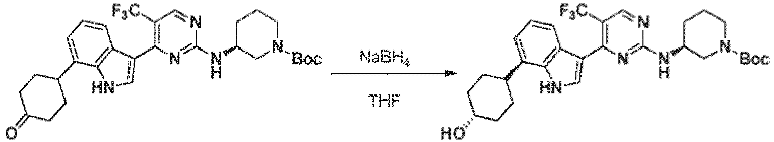

To a solution of (S)-tert-butyl 3-((4-(7-(4-oxocyclohexyl)-1H-indol-3-yl)-5-(trifluoromethyl)pyrimidin-2-yl)amino)piperidine-1-carboxylate (50 mg, 0.09 mmol) in dry THF (2 mL) was added sodium borohydride (5.0 mg, 0.14 mmol). The reaction mixture was stirred at room temperature for 1 h. It was then quenched with methanol (1 mL), water (1 mL) and concentrated to remove organic volatiles. A saturated aqueous solution of NaHCO₃ (50 mL) and EtOAc (100 mL) were added. The phases were separated and the aqueous layer was extracted with EtOAc (2X50 mL). The combined organic layers were dried over Na₂SO₄, filtered and evaporated to dryness to afford the title compound (50.0 mg, 0.09 mmol, 100% yield) as a off-white solid.

### Example 20. Synthesis of (S)-tert-butyl 3-((5-methoxy-4-(1-(phenylsulfonyl)-1H-indol-3-yl)pyrimidin-2-yl)amino)piperidine-1-carboxylate.

### Step 1: 3-(2-chloro-5-methoxypyrimidin-4-yl)-1-(phenylsulfonyl)-1H-indole

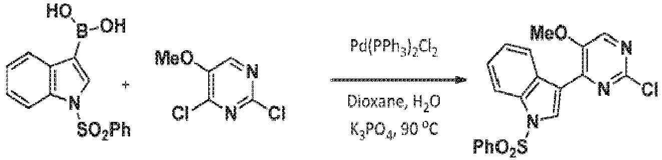

PdCl₂(PPh₃)₂ (97 mg, 0.083 mmol) was added to a stirring solution of (1-(phenylsulfonyl)-1H-indol-3-yl)boronic acid (500 mg, 1.66 mmol) and 2,4-dichloro-5-methoxypyrimidine (267 mg, 1.49 mmol) in Dioxane/H₂O (6.6 mL, 10:1) previously degassed. The resulting reaction mixture was heated at 110 °C for 5h. It was then allowed to cool at room temperature and was evaporated to dryness to yield a yellow brown oil. The reaction mixture was purified by SiO₂ chromatography (EtOAc in DCM, 0 to 30% gradient) to afford the title compound (440 mg, 1.10 mmol, 66% yield) as a light yellow solid.

### [2U9] Step 2: (S)-tert-butyl 3-((5-methoxy-4-(1-(phenylsulfonyl)-1H-indol-3-yl)pyrimidin-2-yl)amino)piperidine-1-carboxylate

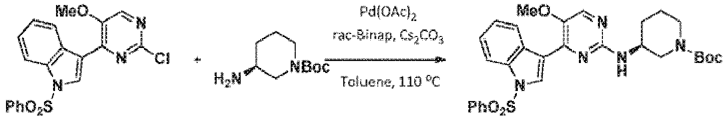

Cs₂CO₃ (367 mg, 1.13 mmol), Pd (OAc)₂ (14.0 mg, 0.038 mmol) and rac-Binap (46.7 mg, 0.075 mmol) were added to a stirring solution of 3-(2-chloro-5-methoxypyrimidin-4-yl)-1-(phenylsulfonyl)-1H-indole (150 mg, 0.375 mmol) and *(S)-tert-butyl 3-aminopiperidine-1-carboxylate* (113 mg, 0.56 mmol) in previously degassed toluene (4.2 mL). The resulting solution was heated at 100 °C overnight. The resulting mixture was cooled to room temperature and concentrated to dryness to a brownish oil. The reaction mixture was purified by SiO₂ chromatography (EtOAc in DCM, 0 to 50% gradient) to afford the title compound (10 mg, 0.018 mmol, 5% yield) as a light yellow solid.

### Example 21. Synthesis of (S)-N-(6,6-dimethylpiperidin-3-yl)-4-(1-(phenylsulfonyl)-1H-indol-3-yl)-5-(trifluoremethyl)pyrimidin-2-amine.

### Step 1: (S)-tert-butyl (1-benzyl-6-oxopiperidin-3-yl)carbamate

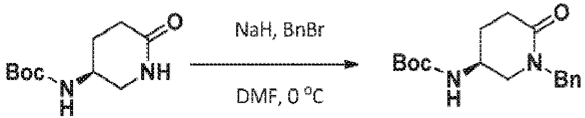

NaH (221 mg, 5.52 mmol) was added to a stirring solution of (S)-5-aminopiperidin-2-one (Tetrahedron Letters, 1995, 36, 82U5-8) (789 mg, 3.68 mmol) in DMF (18 mL) at 0°C. The resulting suspension was stirred for 30 min at which point benzyl bromide (701 µL, 5.89 mmol) was added dropwise and stirred for 30 min. The reaction was then diluted with water (50 mL) and EtOAc (100 mL) and the resulting solution acidified to pH7 with 1M HCl. The phases were separated and the aqueous layer was extracted twice more with EtOAc (100 mL). The combined organic layers were dried over Na₂SO₄, filtered and evaporated to dryness. The residue was purified by SiO₂ chromatography (EtOAc in Hexanes 0 to 100% gradient) and afforded the title compound (690 mg, 2.27 mmol, 62% yield) as a white foam.

### Step 2: (S)-tert-butyl (1-benzyl-6,6-dimethylpiperidin-3-yl)carbamate

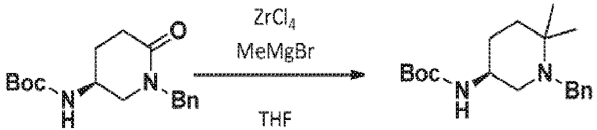

(S)-tert-butyl (1-benzyl-6-oxopiperidin-3-yl)carbamate (43 mg, 0.14 mmol) was dissolved in THF (1.4 mL) and cooled to -10 °C. ZrCl₄ (39 mg, 0.17 mmol) was added and the reaction stirred for 30 min. Then MeMgBr (0.30 mL, 3M, 0.91 mmol) was added to the reaction mixture, the solution allowed to slowly warm up and stirred for 16h. The solution was quenched with 30% NaOH (30 mL) and extracted with DCM (3X50 mL). The combined organic layers were dried over Na₂SO₄, filtered, evaporated to dryness and afforded the title compound (37 mg, 0.12 mmol, 83% yield) as a orange oil.

### Step 3: (S)-1-benzyl-6,6-dimethylpiperidin-3-amine

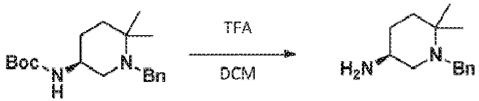

TFA (2.10 mL, 27.3 mmol) was added to a stirring solution of (S)-tert-butyl (1-benzyl-6,6-dimethylpiperidin-3-yl)carbamate (87 mg, 0.27 mmol) at r.t. The resulting solution was stirred for 2h, concentrated under reduced pressure, azeotroped with DCM (3X 25 mL) and afforded the title compound (60 mg, 0.27 mmol, 100 % yield) as an orange oil.

### Step 4: 3-(2-(methylthio)-5-(trifluoromethyl)pyrimidin-4-yl)-1-(phenylsulfonyl)-1H-indole

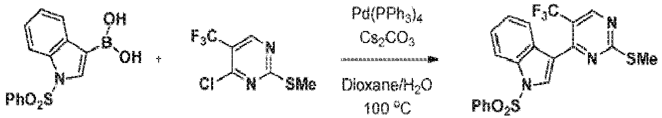

Pd(PPh₃)₄ (1.01 g, 0.87 mmol) and Cs₂CO₃ (5.70 g, 17.5 mmol) were added to a stirring solution of 4-chloro-2-(methylthio)-5-(trifluoromethyl)pyrimidine 4-chloro-2-(methylthio)-5-(trifluoromethyl)pyrimidine (U.S. Pat. Appl. Publ., 20130017194, 17 Jan 2013) (2.00 g, 8.75 mmol) and (1-(phenylsulfonyl)-1H-indol-3-yl)boronic acid (2.77 g, 9.19 mmol) in Dioxane/H₂O previously degassed (180 mL, 2:1). The resulting mixture was then heated at 100 °C under N₂ for 1.5h. The reaction mixture was allowed to cool to room temperature, a saturated solution of NaHCO₃ (100 mL) and EtOAc (200 mL) were added. The phases were separated and the aqueous extracted with EtOAc (2X200 mL). The combined organic layers were dried over Na₂SO₄, filtered and evaporated to dryness. The residue was purified by SiO₂ chromatography (EtOAc in Hexanes 0 to 100% gradient) and afforded the title compound (2.13 g, 4.73 mmol, 54% yield) as a light green foam.

### Step 5: 3-(2-(methylsulfonyl)-5-(trifluoromethyl)pyrimidin-4-yl)-1-(phenylsulfonyl)-1H-indole

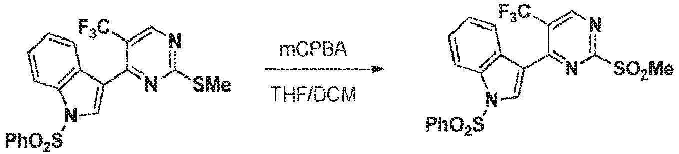

3-(2-(methylthio)-5-(trifluoromethyl)pyrimidin-4-yl)-1-(phenylsulfonyl)-1H-indole (2.13 g, 4.73 mmol) was dissolved with dry THFIDCM (50 mL, 1:1), mCPBA (2.45 g, 14.2 mmol)) was added and to the reaction mixture was stirred at room temperature for 16h. The reaction mixture was evaporated to dryness and was purified by SiO₂ chromatography (EtOAc in Hexanes, 0 to 70% gradient) to afford the title compound (2.15 g, 4.47 mmol, 95% yield) as a light yellow foam.

### Step 6: (S)-N-(1-benzyl-6,6-dimethylpiperidin-3-yl)-4-(1-(phenylsulfonyl)-1H-indol-3-yl)-5-(trifluoromethyl)pyrimidin-2-amine

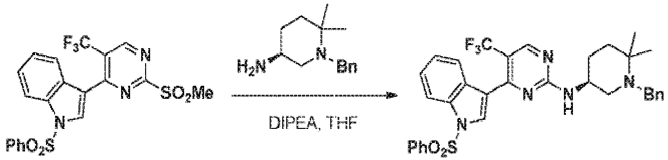

(S)-1-benzyl-6,6-dimethylpiperidin-3-amine (61 mg, 0.28 mmol), and diisopropylethylamine (146 µL, 0.84 mmol) were added to a stirring solution of 3-(2-(methylsulfonyl)-5-(trifluoromethyl)pyrimidin-4-yl)-1-(phenylsulfonyl)-1H-indole (135 mg, 0.28 mmol) in THF (4.6 mL). The reaction mixture was evaporated to dryness and was purified by SiO₂ chromatography (EtOAc in DCM, 0 to 50% gradient) to afford the title compound (46 mg, 0.074 mmol, 27% yield) as a light yellow oil.

### Step 7: (S)-N-(6,6-dimethylpiperidin-3-yl)-4-(1-(phenylsulfonyl)-1H-indol-3-yl)-5-(trifluoromethyl)pyrimidin-2-amine

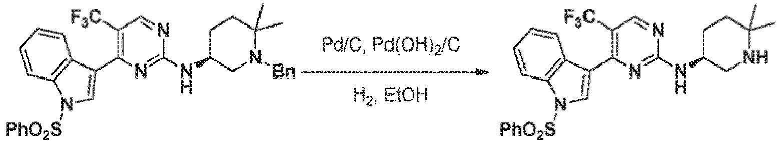

To a degassed solution of (S)-N-(1-benzyl-6,6-dimethylpiperidin-3-yl)-4-(1-(phenylsulfonyl)-1H-indol-3-yl)-5-(triffuoromethyl)pyrimidin-2-amine (46 mg, 0.074 mmol) in EtOH (7 mL) was added a Pd/C (10% wlw on activated carbon)/Pd(OH)₂/C mix. The reaction mixture was then allowed to stir under a positive pressure of hydrogen (1 atm) at room temperature for 16h. The reaction mixture was degassed with N₂, filtered through celite and was concentrated under reduced pressure to afford the title compound (27 mg, 0.052 mmol, 70% yield) as a light yellow solid.

### Example 22. Syntheses of (S)-tert-butyl 3-((4-(7-((1r,4S)-4-hydroxy-4-methylcyclohexyl)-1H-indol-3-yl)-5-(trifluoromethyl)pyrimidon-2-yl)amino)piperidine-1-carboxylate (A) and (S)-tert-butyl 3-((4-(7-((1s,4R)-4-hydroxy-4-methylcyclohexyl)-1H-indol-3-yl)-5-(trifluoromethyl)pyrimidin-2-yl)amino)piperidine-1-carboxylate (B).

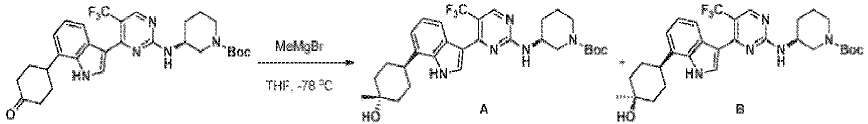

To a solution of (S)-tert-butyl 3-((4-(7-(4-oxocyclohexyl)-1H-indol-3-yl)-5-(trifluoromethyl)pyrimidin-2-yl)amino)piperidine-1-carboxylate (57 mg, 0.10 mmol) in THF (0.5 mL) at -78 °C was added MeMgBr (68 µL, 3M, 0.20 mmol). The reaction mixture was stirred at -78 °C for 1h and allowed to slowly go up to room temperature over 3h. The reaction mixture was quenched a saturated aqueous solution of NH₄Cl (10 mL) and extracted with DCM (3X30 mL). The combined organic layers were dried over Na₂SO₄, filtered and evaporated to dryness to afford the title compounds (59 mg, 0.10 mmol, 100% yield) as a yellow oil. The crude oil was used as is in the next step.

### Example 23. Synthesis of (S)-tert-butyl 3-((4-(7-(3-hydroxy-3-methylbutyl)-1H-indol-3-yl)-5-(trifluoromethyl)pyrimidin-2-yl)amino)piperidine-1-carboxylate.

### Step 1: (S)-tert-butyl 3-((4-(7-bromo-1-((2-(trimethylsilyl)ethoxy)methyl)-1H-indol-3-yl)-5-(trifluoromethyl)pyrimidin-1-yl)amino)piperidine-1-carboxylate

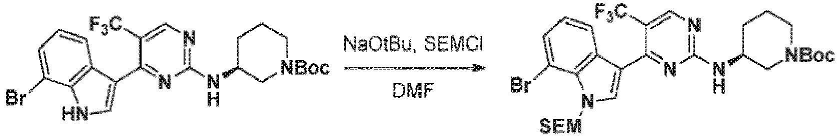

To a solution of (S)-tert-butyl 3-((4-(7-bromo-1H-indol-3-yl)-5-(trifluoromethyl)pyrimidin-2-yl)amino)piperidine-1-carboxylate (200 mg, 0.37 mmol) in DMF (2.0 mL) was added NaOtBu (71 mg, 0.44 mmol). The solution was stirred for 1 h at room temperature then cooled to 0 °C and (2-(chloromethoxy)ethyl)trimethylsilane (74 mg, 0.44 mmol) was added and the reaction mixture was stirred at room temperature overnight. The reaction was diluted with water (25 mL), extracted with EtOAc (3X50 mL), dried over Na₂SO₄ and concentrated to dryness. The reaction mixture was purified by SiO₂ chromatography (EtOAc in Hexanes, 0 to 50% gradient) to afford the title compound (237 mg, 0.35 mmol, 95% yield) as an off-white oil.

### Step 2: (S)-tert-butyl 3-((4-(7-(3-methoxy-3-oxoprop-1-en-1-yl)-1-((2-(trimethylsilyl)ethoxy)methyl)-1H-indol-3-yl)-5-(trifluoromethyl)pyrimidin-2-yl)amino)piperidine-1-carboxylate

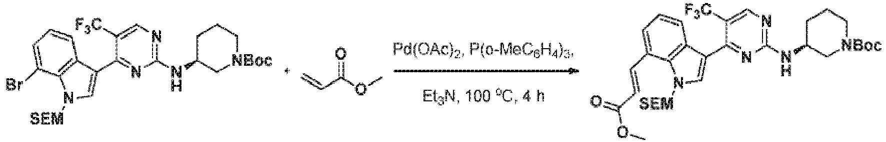

To a solution of (S)-tert-butyl 3-((4-(7-bromo-1-((2-(trimethylsilyl)ethoxymethyl)-1H-indol-3-yl)-5-(trifluoromethyl)pyrimidin-2-yl)amino)piperidine-1-carboxylate (237 mg, 0.35 mmol) and methyl acrylate (95 µL, 1.06 mmol) in Et₃N (1.01 mL), Pd(OAc)₂ (7.9 mg, 0.035 mmol) and P(o-MeC₆H₄)₃ (32 mg, 0.11 mmol) were added. The reaction mixture was sealed and heated to 100 °C and stirred for 4 h. The reaction mixture was diluted with EtOAc (50 mL) and extracted with H₂O (3X60 mL). The organic layer was washed with brine (60 mL), dried over Na₂SO₄ and concentrated to dryness. The reaction mixture was purified by SiO₂ chromatography (EtOAc in Hexanes, 0 to 100% gradient) to afford the title compound (148 mg, 0.219 mmol, 62% yield) as a yellow oil.

### Step 3: (S)-tert-butyl 3-((4-(7-(3-methoxy-3-oxopropyl)-1-((2-(trimethylsilyl)ethoxy)methyl)-1H-indol-3-yl)-5-(trifluoromethyl) pyrimidin-2-yl)amino)piperidine-1-carboxylate

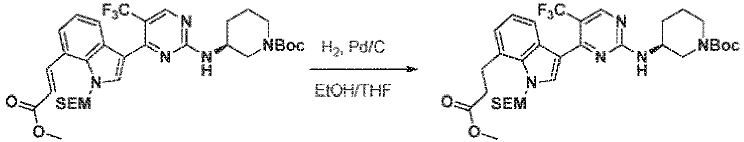

To a degassed solution of (S)-tert-butyl 3-((4-(7-(3-methoxy-3-oxoprop-1-en-1-yl)-1-((2-(trimethylsilyl)ethoxy)methyl)-1H-indol-3-yl)-5-(trifluoromethyl)pyrimidin-2-yl)amino)piperidine-1-carboxylate (83 mg, 0.15 mmol) in EtOH (10 mL) and THF (10 mL), Pd/C ( 10% w/w on activated carbon) was added (3 mg, 0.023 mmol) and the reaction mixture was stirred at room temperature overnight under H₂ (1 atm). The reaction mixture was degassed with N₂, filtered through celite and was concentrated under reduced pressure to afford the title compound (148 mg, 0.219 mmol, 100% yield) as a yellow oil.

### Step 4: (S)-tert-butyl 3-((4-(7-(3-hydroxy-3-methylbutyl)-1-((2-(trimethylsilyl)ethoxy)methyl)-1H-indol-3-yl)-5-(trifluoromethyl)pyrimidin-1-yl)amino)piperidine-1-carboxylate

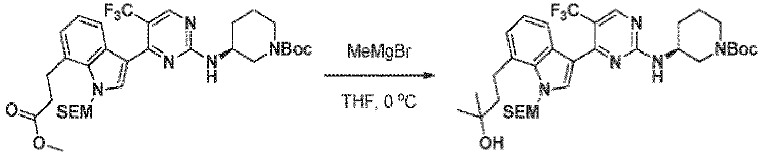

To a solution of (S)-tert-butyl 3-ζ(4-(7-(3-methoxy-3-oxopropyl)-1-((2-(trimethylsilyl)ethoxy)methyl)-1H-indol-3-yl)-5-(trifluoromethyl)pyrimidin-2-yl)amino)piperidine-1-carboxylate (148 mg, 0.218 mmol) in THF (0.5 mL) at 0 °C was added MeMgBr (0.44 mL, 3M, 1.31 mmol). The reaction mixture was warmed to room temperature and stirred overnight. The reaction was quenched with saturated aqueous solution of NH₄Cl (10 mL) and extracted with EtOAc (30 mL). The phases were separated and the organic layer was washed with brine (20 mL), dried over Na₂SO₄ and concentrated to dryness. The reaction mixture was purified by SiO₂ chromatography (EtOAc in Hexanes, 0 to 100% gradient) to afford the title compound (60 mg, 0.089 mmol, 41% yield) as an off-white solid.

### Step 5: (S)-tert-butyl 3-((4-(7-(3-hydroxy-3-methylbutyl)-1H-indol-3-yl)-5-(trifluoromethyl)pyrimidin-2-yl)amino)piperidine-1-carboxylate

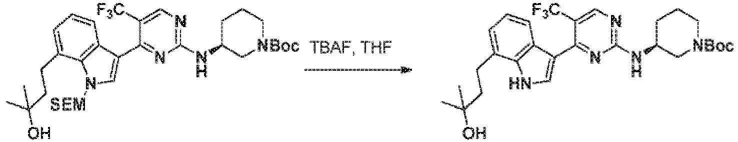

To a solution of (S)-tert-butyl 3-((4-(7-(3-hydroxy-3-methylbutyl)-1-((2-(trimethylsilyl)ethoxy)methyl)-1H-indol-3-yl)-5-(trifluoromethyl)pyrimidin-2-yl)amino)piperidine-1-carboxylate (30 mg, 0.044 mmol) in THF (1.5 mL) at 0 °C was added tetrabutylammonium fluoride 1M in THF (0.18 mL, 0.18 mmol). The reaction mixture was stirred for 1 h at room temperature and then heated to 70 °C and stirred overnight. The reaction mixture was quenched with a saturated aqueous solution of NH₄Cl (10 mL), extracted with EtOAc (3X30 mL). The combined organic layers were dried over Na₂SO₄ and concentrated to dryness. The reaction mixture was purified by SiO₂ chromatography (EtOAc in Hexanes, 0 to 100% gradient) to afford the title compound (20 mg, 0.037 mmol, 83% yield) as a yellowish oil.

### Example 24. Syntheses of (S)-tert-butyl 3-((5-ethynyl-4-(1H-indol-3-yl)pyrimidin-2-yl)amino)piperidine-1-carboxylate (A) and (S)-tert-butyl 3-((5-ethynyl-4-(1-methyl-1H-indol-3-yl)pyrimidin-2-yl)ainino)piperidine-1-carboxylate (B).

### Step 1: (S)-tert-butyl 3-((4-(1-(phenylsulfonyl)-1H-indol-3-yl)-5-((trimethylsilyl)ethynyl)pyrimidin-2-yl)amino)piperidine-1-carboxylate

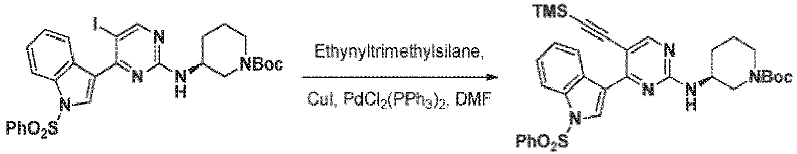

A degassed solution of (S)-tert-butyl 3-((5-iodo-4-(1-(phenylsulfonyl)-1H-indol-3-yl)pyrimidin-2-yl)amino)piperidine-1-carboxylate (150 mg, 0.23 mmol), ethynyltrimethylsilane (64 uL, 0.45 mmol), Cul (3.4 mg, 0.018 mmol), PdCI₂(PPh₃)2 (13 mg, 0.018 mmol) and Et₃N (0.316 mL, 2.27 mmol) in DMF (2.7 mL) was stirred at room temperature overnight. The reaction mixture was concentrated under reduced pressure and dissolved in EtOAc (50 mL), washed with NH₄OH (50 mL), and brine (50 mL). The phases were separated and the organic layer was dried over Na₂SO₄, filtered and concentrated to dryness. The reaction mixture was purified by SiO₂ chromatography (EtOAc in Hexanes:DCM (1:1), 0 to 100% gradient) to afford the title compound (143 mg, 0.23 mmol, 100% yield) as a beige foam.

### Step 2: (S)-tert-butyl 3-((5-ethynyl-4-(1H-indol-3-yl)pyrimidin-2-yl)amino)piperidine-1-carboxylate (A) and (S)-tert-butyl 3-((5-ethynyl-4-(1-methyl-1H-indol-3-yl)pyrimidin-2-yl)amino)piperidine-1-carboxylate (B)

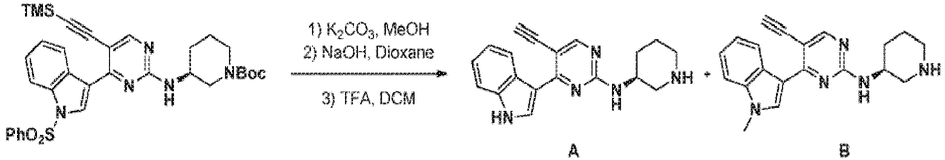

To a solution of (S)-tert-butyl 3-((4-(1-(phenylsulfonyl)-1H-indol-3-yl)-5-((trimethylsilyl)ethynyl)pyrimidin-2-yl)amino)piperidine-1-carbaxylate (143 mg, 0.23 mmol) in degassed MeOH (2.3 mL) was added K₂CO₃ (31 mg, 0.23 mmol). The reaction mixture was stirred at room temperature for 2 h. It was then concentrated to dryness, dissolved in dioxane (2.3 mL) and 5M NaOH (1.4 mL, 6.8 mmol) was added and the reaction mixture was heated overnight at 75 °C. The reaction mixture was concentrated under reduced pressure. and extracted with MeTHF (3X50 mL). The combined organic layers were dried over Na₂SO₄, filtered and concentrated to dryness. The reaction mixture was purified by SiO₂ chromatography (MeOH in DCM, 0-20% gradient) to yield a brown oil. The oil was dissolved in DCM (2.0 mL), TFA (0.87 mL, 11.4 mmol) was added and the reaction mixture was stirred at room temperature for 30 min. The reaction mixture was concentrated under reduced pressure, was dissolved in MeTHF (10 mL) and washed twice with saturated NaHCO₃ (20 mL). The phases were separated, the organic layer was dried over Na₂SO₄, filtered and concentrated to dryness. The obtained residue was purified by reverse phase chromatography (C18, H₂O/ACN [10mM ammonium formate in H₂O], 0 to 100% gradient) and afforded the title compound A (18 mg, 0.057 mmol, 25% yield) as a yellowish solid after lyophilisation and title compound B (10 mg, 0.030 mmol, 13% yield) as a yellowish solid after lyophilisation.

### Example 25. Synthesis of (S)-5-ethyl-4-(7-fluoro-1H-indazol-3-yl)-N-(piperidin-3-yl)pyrimidin-2-amine (Compound 143).

### Step 1: 2-[[3-(2-chloro-5-ethyl-pyrimidin-4-yl)-7-fluoro-indazol-1-yl]methoxy]ethyl-trimethylsilane

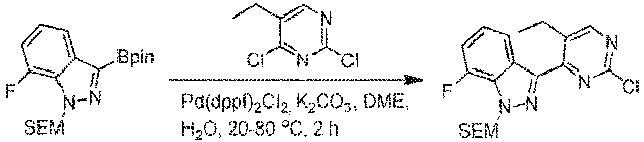

To a mixture of 2-[[7-fluoro-3-(4,4,5,5-tetramethyl-1,3,2-dioxaborolan-2-yl)indazol-1-yl] methoxy]ethyl-trimethyl-silane (2 g, 3.57 mmol) and 2,4-dichloro-5-ethyl-pyrimidine (947.53 mg, 5.36 mmol) in DME (25 mL) and H₂O (5 mL) was added Pd(dppf)Cl₂ (261.09 mg, 357.00 umol) and K₂CO₃ (986.33 mg, 7.14 mmol) in one portion at 20 °C under N₂. The mixture was stirred at 80 °C for 2 h. The mixture was poured into water (100 mL) and extracted with EA (50 mL*2). The combined organic phase was washed with brine (100 mL*2), dried with anhydrous Na₂SO₄, filtered and concentrated in vacuum. The residue was purified by silica gel chromatography (PE/EA=100/1, 40/1) to give 2-[[3-(2-chloro-5-ethyl-pyrimidin-4-yl)-7-fluoro-indazol-1-yl]methoxy]ethyl-trimethyl-silane (600 mg, crude) as colorless oil. LCMS: M+H⁺: 407.1 @ 1.087 min (5-95% ACN in H₂O, 1.5 min).

### Step 2: tert-butyl (3S)-3-[[5-ethyl-4-[7-fluoro-1-(2-trimethylsilytethoxymethyl)indazol-3-yl]pyrimidin-2-yl]amino]piperidine-1-carboxylate

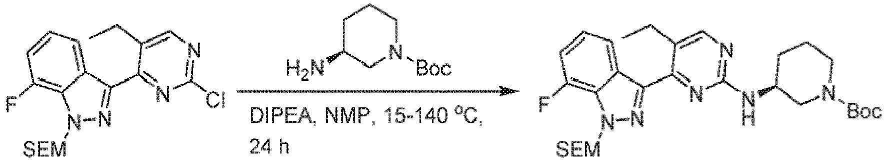

To a mixture of 2-[[3-(2-chloro-5-ethyl-pyrimidin-4-yl)-7-fluoro-indazol-1-yl]methoxy] ethyl-trimethyl-silane (500 mg, 1.23 mmol) and tert-butyl (3S)-3-aminopiperidine-1- carboxylate (369.52 mg, 1.84 mmol) in NMP (5 mL) was added DIPEA (794.83 mg, 6.15 mmol) in one portion at 15 °C under N₂. The mixture was stirred at 140 °C for 24 h, then poured into water (20 mL) and extracted with EA (10 mL*2). The combined organic phase was washed with brine (10 mL*2), dried with anhydrous Na₂SO₄, filtered and concentrated in vacuum. The residue was purified by silica gel chromatography (PE/EA=50/1, 20/1) to afford tert-butyl(3S)-3-[[5-ethyl-4-[7-fluoro-1-(2-trimethylsilylethoxymethyl) indazol-3-yl] pyrimidin-2-yl]amino]piperidine-1-carboxylate (350 mg, 49.85%) as yellow oil. TLC: R_{f}=0.11 (PE:EA=3:1)

### Step 3: 5-exhyl-4-(7-fluoro-1H-indazol-3-yl)-N-[(3S)-3-piperidyl]pyrimidin-2-amine

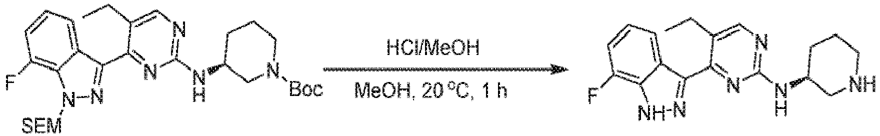

To a mixture of tert-butyl (3S)-3-[[5-ethyl-4-[7-fluoro-1-(2-trimethylsilylethoxymethyl) indazol-3-yl]pyrimidin-2-yl]amino]piperidine-1-carboxylate (200 mg, 350.40 umol) in MeOH (3 mL) was added HCl/MeOH (50 mL) in one portion at 20 °C under N₂. The mixture was stirred at 20 °C for 1 h. The mixture was concentrated under reduced pressure. The residue was purified by prep-HPLC(FA) to afford 5-ethyl-4-(7-fluoro-1H-indazol-3-yl)-N- [(3S)-3-piperidyl]pyrimidin-2-amine (22 mg, 16.25%, FA) as a white solid. LCMS: M+H⁺: 341.3@ 2.038 min (10-80% ACN in H₂O, 4.5 min). **¹HNMR:** (MeOD, 400 MHz) δ 8.53 (s, 1 H), 8.32 (s, 1 H), 8.21 (d, *J* = 7.78 Hz, 1 H), 7.25-7.13 (m, 2 H), 4.34-4.25 (m, 1 H), 3.57 (dd, *J* = 12.42, 3.39 Hz, 1 H), 3.13-2.98 (m, 4 H), 2.27-2.18 (m, 1 H), 2.16-2.06 (m, 1 H), 1.96-1.73 (m, 2 H), 1.20 (t, *J* = 7.40 Hz, 3 H).

### Example 26. Synthesis of 3-(2-chloro-5-(2-fluoroethyl)pyrimidin-4-yl)-1-(phenylsulfonyl)-1H-indole intermediate useful in synthesizing (Compound 206)

### Step 1: 2-(2-Chloro-4-(1-(phenylsulfonyl)-1H-indol-3-yl)pyrimidin-5-yl)acetaldehyde

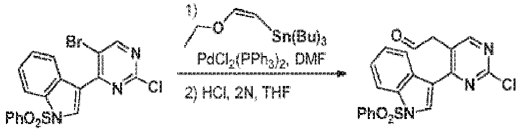

PdCl₂(PPh₃)₂ (78.2 mg, 0.111 mmol) was added to a solution of 3-(5-bromo-2-chloropyrimidin-4-yl)-1-(phenylsulfonyl)-1H-indole (500 mg, 1.114 mmol) and (Z)-tributyl(2-ethoxyvinyl)stannane (409 uL, 1.226 mmol) in dry DMF (2.3 mL). The vial was evacuated and backfilled with nitrogen twice and then being sealed was heated at 80 °C for 3 hours. The reaction mixture was quenched with 2M KF (10 mL) and extracted with EtOAc. The combined organic layers were washed with brine, dried over Na₂SO₄, and concentrated to providing crude intermediate (1.0 g) as brown semi-solid. Obtained compound was diluted with THF (9 mL) and 2N HCl aq. (1 mL) was added. The resulting brown solution was stirred at 70 °C for overnight, and then the organic solvent was removed under reduced pressure. The residue was diluted with water, the pH neutralized with 5N NaOH, and product was extracted with MeTHF. The organic phase was washed with brine, dried over Na₂SO₄, and concentrated. The residue was purified by SiO₂ chromatography (EtOAc in DCM/Hexane (1:1), 0 to 100% gradient) and afforded the title compound (300 mg, 0.728 mmol, 65% yield over 2 steps) as a yellowish semi-solid.

### Step 2: 2-(2-Chloro-4-(1-(phenylsulfonyl)-1H-indol-3-yl)pyrimidin-5-yl)ethanol

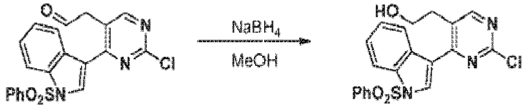

To methanol (15 mL) solution of 2-(2-chloro-4-(1-(phenylsulfonyl)-1H-indol-3-yl)pyrimidin-5-yl)acetaldehyde (300 mg, 0.728 mmol) at 0 °C, sodium borohydride (41 mg, 1.093 mmol) was added. The reaction mixture was stirred at 0 °C for 1 h and then at room temperature for overnight. Water (2 mL) was added and the mixture was concentrated. Crude was re-dissolved in MeTHF (15 mL), washed with brine (15 mL), organic layer was then separated, dried over Na₂SO₄, filtered, and concentrated. The residue was purified by SiO₂ chromatography (EtOAc in DCM, 0 to 100% gradient) and afforded the title compound (125 mg, 0.302 mmol, 42% yield) as a yellowish solid.

### Step 3: 3-(2-Chloro-5-(2-fluoroethyl)pyrimidin-4-yl)-1-(phenylsulfonyl)-1H-indole

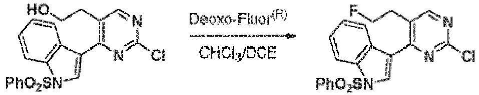

To a suspension of 2-(2-chloro-4-(1-(phenylsulfonyl)-1H-indol-3-yl)pyrimidin-5-yl)ethanol (125 mg, 0.302 mmol) in 2 mL of chloroform/DCE (1:1) under inert atmosphere at 25°C, Deoxo-Fluor^{®} (170 uL, 0.906 mmol) was added. Reaction mixture instantly became homogeneous. Reaction was then quenched with a saturated aqueous solution of NaHCO₃ (5 mL) and crude product was extracted with chloroform (3x5 mL). Combined organic phase was dried over Na₂SO₄, filtered, and concentrated providing the crude title compound (148 mg) as a yellow gum that was used without further purification.

### Example 27. Synthesis of (S)-tert-butyl 3-((4-(7-((1s,4S)-4-hydroxycyclohexyl)-1H-indol-3-yl)-5-(trifluoromethyl)pyrimidin-2-yl)amino)piperidine-1-carboxylate intermediate useful in synthesizing (Compound 207).

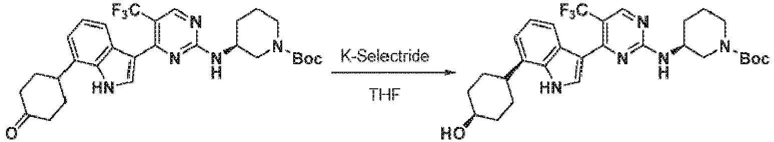

To a solution of (S)-tert-butyl 3-((4-(7-(4-oxocyclohexyl)-1H-indol-3-yl)-5-(trifluaromethyl)pyrimidin-2-yl)amino)piperidine-1-carboxylate (62 mg, 0.111 mmol) in dry THF (1 mL) at -20 °C was added K-Selectride (1M in THF, 167 uL, 0.167 mmol). The reaction mixture was stirred at room temperature for 15 min at this temperature. It was then quenched with water (1 mL) and concentrated to remove organic volatiles. A saturated aqueous solution of NaHCOa (5 mL) and MeTHF (10 mL) were added. The phases were separated and the aqueous layer was extracted with MeTHF (2X10 mL). The combined organic layers were dried over Na₂SO₄, filtered and evaporated to dryness to afford the title compound (62.0 mg, 0.11 mmol, quantitative yield) as a yellowish solid that was used without further purification for final deprotection.

### Example 28. Synthesis of (S)-N-(4,4-dimethylpiperidin-3-yl)-4-(1H-indol-3-yl)-5-(trifluoromethyl)pyrimidin-2-amine (Compund 210) and (R)-N-(4,4-dimethylpiperidin-3-yl)-4-(1H-indol-3-yl)-5-(trifluoromethyl)pyrimidin-2-amine (Compound 209)

### Step 1: 1-Benzyl-4,4-dimethylpiperidine-2,6-dione

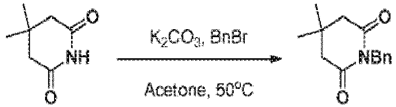

To a solution of 4,4-dimethylpiperidine-2,6-dione (5.0 g, 35.42 mmol) in acetone (177 mL) at 25 °C was added K₂CO₃ (16.3 g, 70.84 mmol) and the resulting suspension was stirred while adding BnBr (4.63 mL, 38.96 mmol) dropwise. The suspension was then placed in an oil bath set to 50 °C and stirred for 6 h. The reaction was cooled to 25°C and stirred for a further 48 h. The fine white suspension was filtered under vacuum on a small-pore frit, and the colorless filtrate was concentrated under reduced pressure. The residue was purified by flash chromatography on SiO₂ (EtOAc in Hexanes, 10 to 40% gradient) to afford the title compound as a white solid (7.19 g, 31.09 mmol, 88%).

### Step 2: (Z)-1-benzyl-3-(hydroxyimino)-4,4-dimethylpiperidine-2,6-dione

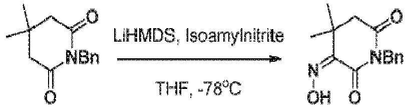

To a flame-dried flask under nitrogen was added a solution of 1-benzyl-4,4-dimethylpiperidine-2,6-dione (3.00 g, 12.97 mmol) in THF (60 mL) at 25 °C. This solution was cooled to -78°C (internal temperature), then LiHMDS, 1M in hexanes (13 mL, 12.97 mmol) was added dropwise into this solution forming a milky white suspension. Stirred for 20 minutes at -78°C, then slowly added isoamylnitrite (2.08 mL, 15.66 mmol) over 15 minutes forming a mango-yellow suspension. Reaction mixture was stirred for an additional 30 minutes before warming up to 25 °C over 1 h, then quenched with saturated solution of NH₄Cl and stirred for 16 h. Added water and extracted with MeTHF (3x60 mL) Combined organic phase was washed with water (2x40 mL), brine (2x40 mL), then dried over Na₂SO₄, filtered, and concentrated under reduced pressure. To the obtained oil, added 10% EtOAc in hexanes (10 mL), and a white solid slowly precipitated from the solution. Cooled in an ice bath, then filtered and washed with hexanes to obtain the title compound as a white solid (526.1 mg, 2.02 mmol, 16%).

### Step 3: 1-Benzyl-4,4-dimethylpiperidin-3-amine

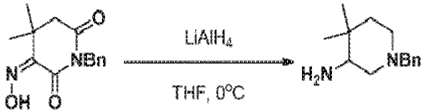

A solution of (Z)-1-benzyl-3-(hydroxyimino)-4,4-dimethylpiperidine-2,6-dione (526 mg, 2.02 mmol) in dry THF (15 mL) was added a suspension of LiA1H₄ (383 mg, 10.10 mmol) in dry THF (5 mL) under nitrogen at 0 °C. The suspension was gradually warmed up to 25 °C and allowed to stir for 1 h at this temperature before being placed in an oil bath at 60°C for 48 h. Cooled this mixture to 0 °C in an ice bath, then added Na₂SO₄ (wetted with water) until no more gas evolved and finally added EtOAc (30 mL) and stirred for 1h at 25 °C. Filtered the solid material off via a Buchner funnel, washed with EtOAc and MeOH and dried the filtrate with Na₂SO₄. Filtered the solids and concentrated the filtrate to obtain the crude title compound (1 g) as yellow oil that was used without further purification.

### Step 4: (S)-N-(1-benzyl-4,4-dimethylpiperidin-3-yl)-4-(1-(phenylsulfonyl)-1H-indol-3-yl)-5-(trifluoromeihyl)pyrimidin-2-amine (A), (R)-N-(1-benzyl-4,4-dimethylpiperidin-3-yl)-4-(1-(phenylsulfonyl)-1H-indol-3-yl)-5-(trifluoromethyl)pyrimidin-2-amine (B)

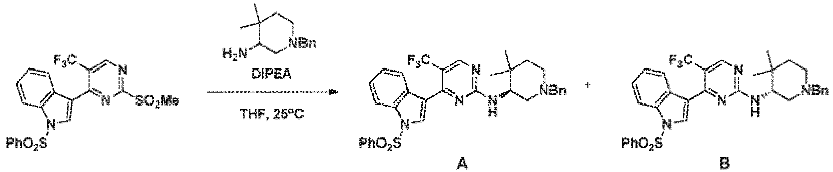

Crude 1-benzyl-4,4-dimethylpiperidin-3-amine (2.02 mmol) from the previous step, 3-(2-(methylsulfonyl)-5-(trifluoromethyl)pyrimidin-4-yl)-1-(phenylsulfonyl)-1H-indole (650 mg, 1.35 mmol) and *N,N*-diisopropylethylamine (0.96 mL, 6.06 mmol) (dried over Na₂SO₄) were dissolved in dry THF (34 mL) (dried over Na₂SO₄). The mixture was stirred at 25 °C for 3 h, and then concentrated under reduced pressure. Residue was purified by flash chromatography on SiO₂ (EtOAc in DCM 0 to 100% gradient) to obtain the title compound as a red foam (260 mg, 0.42 mmol, 21% yield over 2 steps). This material was separated into the corresponding (S)- and (R)-enantiomers using a ChiralPak IA column with 5.000 ul injections and 0.5% EtOH, 0.1% DCM in hexane for elution. Peak 1 (**A**, tentatively assigned as (S)-enantiomer): 109.1 mg (>96% purity, 99.796 *ee)* as a pale yellow solid, and Peak 2 (**B**, tentatively assigned as (R)-enantiomer): 98.0 mg (>93% purity, 90.0% *ee)* as a pale yellow solid.

### Step 5: (S)-methyl 4,4-dimethyl-3-((4-(1-(phenylsulfonyl)-1H-indol-3-yl)-5-(trifluoromethyl)pyrimidin-2-yl)amino)piperidine-1-carboxylate

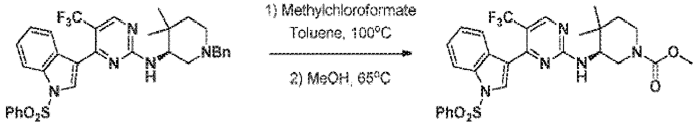

To a solution of (S)-N-(1-benzyl-4,4-dimethylpiperidin-3-yl)-4-(1-(phenylsulfonyl)-1H-indol-3-yl)-5-(trifluoromethyl)pyrimidin-2-amine (A, stereochemistry tentatively assigned) (103.2 mg, 0.167 mmol) in toluene (1.4 mL) at 25 °C was added methylchloroformate (0.90 mL, 8.53 mmol) and *N,N*-diisopropylamine (1.15 ml, 8.15 mmol) and resulting mixture was heated to 100 °C under nitrogen for 16 h. The solution concentrated under reduced pressure, then added MeOH (5 mL) and the resulting mixture was heated at 65 °C for 2 h, then stined for 16 h at 25 °C, and finally concentrated under reduced pressure. The residue was then purified by reverse-phase chromatography on C18 (MeCN in aq. ammonium formate, 10 mM, pH 3.8, 0 to 100% gradient). Title compound was obtained as a yellow solid (50 mg, 0.085 mmol, 51% yield).

### Step 6: (S)-N-(4,4-dimethylpiperidin-3-yl)-4-(1H-indol-3-yl)-5-(trifluoromethyl)pyrimidin-2-amine

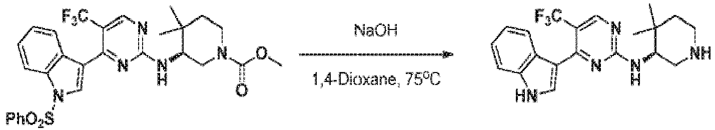

To a solution of (S)-methyl 4,4-dimethyl-3-((4-(1-(phenylsulfonyl)-1H-indol-3-yl)-5-(trifluoromethyl)pyrimidin-2-yl)amino)piperidine-1-carboxylate (50 mg, 0.085 mmol) in 1,4-dioxane (0.3 mL) was added 5M NaOH (2.65 mL), and reaction mixture was then stirred at 75°C for 16 h. Reaction was cooled down to 25 °C and crude product was then extracted with MeTHF (3x10 mL). Combined organic extract was washed with brine (5 mL), dried over Na₂SO₄, filtered, and concentrated. The residue was purified by reverse-phase chromatography on C18 (MeCN in aq. ammonium formate, 10 mM, pH 3.8, 0 to 100% gradient). The title compound was obtained as a white fluffy solid (6.79 mg, 0.017 mmol, 10% yield).

### Example 29. Synthesis of N-((3S,6R)-6-ethylpiperidin-3-yl)-4-(1-(phenylsulfonyl)-1H-indol-3-yl)-5-(trifluoromethyl)pyrimidin-2-amine (A) and N-((3S,6S)-6-ethylpiperidin-3-yl)-4-(1-(phenylsulfonyl)-1H-indol-3-yl)-5-(trifluoromethyl)pyrimidin-2-amine (B) Intermediates useful in the synthesis of Compound 211 and Compund 217

### Step 1: tert-Butyl ((3S,6R)-1-benzyl-6-ethylpiperidin-3-yl)carbamate (2R,5R)-1-(tert-butoxycarbonyl)-5-((tert-butyldimethylsilyl)oxy)piperidine-2-carboxylic acid (A) and tert-butyl ((3S,6S)-1-benzyl-6-ethylpiperidin-3-yl)carbamate (B)

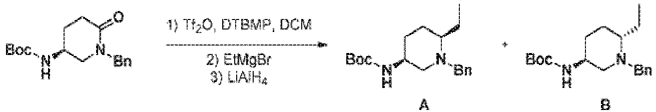

To a solution of (S)-tert-butyl (1-benzyl-6-oxopiperidin-3-yl)carbamate (106 mg, 0.348 mmol) and 2,6-di-tert-butyl-4-methylpyridine (DTBMP) (85.8 mg, 0.418 mmol) in DCM (3.5 mL) was added Tf₂O (71 uL, 0.418 mmol) dropwise at -78 °C. The reaction mixture was stirred at -78 °C for 45 minutes. A solution of EtMgBr (120 uL, 3.0 M in ether, 0.418 mmol) was added dropwise to the to the reaction mixture and the mixture was allowed to warm slowly to room temperature and stirred for 1 h at room temperature. Then LiA1H₄ (39.6 mg, 1.045 mmol) was added in one portion. After stirring for 1 h, the reaction mixture was diluted with ether and quenched by careful addition of 0.11 mL H₂O, 0.06 mL of 30% NaOH and 0.33 mL of H₂O. The resulting mixture was stirred for 20 minutes and MgSO₄ was added and stirred overnight. The reaction mixture was filtered through celite and washed with DCM and ether. The filtrate was concentrated under reduced pressure and purified by SiO₂ chromatography (EtOAc in DCM:Hexanes (1:1), 0 to 100% gradient) to afford the title compound (A) as a clear oil (35.9 mg, 0.113 mmol, 32% yield) and (**B**) as a white semi-solid (27.3 mg, 0.086 mmol, 25% yield).

### Step 2: (3S.6R)-1-benzyl-6-ethylpiperidin-3-amine

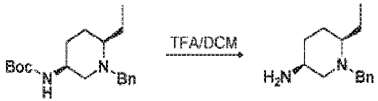

To a solution of tert-butyl ((3S,6R)-1-benzyl-6-ethylpiperidin-3-yl)carbamate (69 mg, 0.218 mmol) in DCM (4.4 mL) at 0 °C was added TFA (1.67 mL, 21.762 mmol) and the reaction mixture was allowed to warm to room temperature with stirring for 16 h. The reaction mixture was concentrated under reduced pressure and azeotroped three times with DCM to afford the title compound as a yellow oil (47 mg, 0.218 mmol, 100%) that was used without further purification.

### Step 3: N-((3S,6R)-1-benzyl-6-ethylpiperidin-3-yl)4-(1-(phenylsulfonyl)-1H-indol-3-yl)-5-(trifluoromethyl)pyrimidin-2-amine

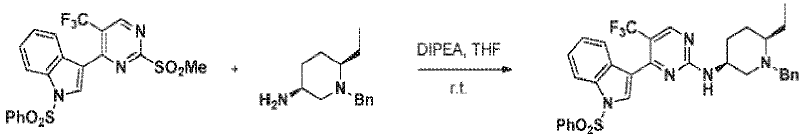

(3S,6R)-1-benzyl-6-ethylpiperidin-3-amine (47 mg, 0.218 mmol) and diisopropylethylamine (0.19 mL, 1.088 mmol) were added to a stirring solution of 3-(2-(methylsulfonyl)-5-(trifluoromethyl)pyrimidin-4-yl)-1-(phenylsulfonyl)-1H-indole (121 mg, 0.250 mmol) in THF (3.6 mL). The reaction mixture was stirred at room temperature for overnight The reaction mixture was diluted with EtOAc, washed with brine, dried over Na₂SO₄, filtered, and concentrated under reduced pressure. The residue was purified by flash column chromatography on SiO₂ (EtOAc in DCM, 0 to 50% gradient) to obtain the title compound as a light yellow oil (106 mg, 0.171 mmol, 79% yield).

### Step 4: N-((3S,6R)-6-ethylpiperidin-3-yl)-4-(1-(phenylsulfonyl)-1H-indol-3-yl)-5-(trifluoromethyl)pyrimidin-2-amine

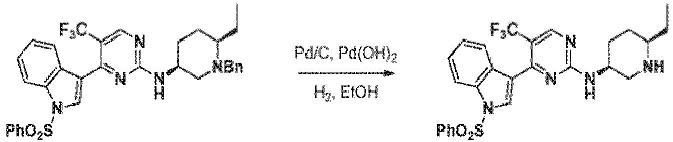

To a degassed solution of N-((3S,6R)-1-benzyl-6-ethylpiperidin-3-yl)-4-(1-(phenylsulfonyl)-1H-indol-3-yl)-5-(trifluoromethyl)pyrimidin-2-amine (106 mg, 0.171 mmol) in EtOH (13 mL), was added Pd/C (10% wlw on activated carbon)/Pd(OH)₂/C (20% dispersion) mix and the reaction mixture was stirred at room temperature overnight under H₂ (1 atm). The reaction mixture was degassed with N₂, filtered through pad of Celite and then concentrated under reduced pressure to afford the title compound as an off-white solid (34 mg, 0.063 mmol, 37% yield) that was used without further purification.

### Example 30. Synthesis of (S)-4-azaspiro[2.5]octan-6-amine intermediate useful in synthesizing (Compund 212)

### Step 1: (S)-tert-butyl benzyl(1-benzyl-6-oxopiperidin-3-yl)carbamate

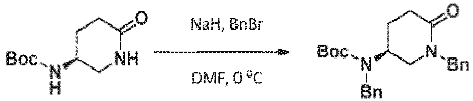

NaH (221 mg, 5.52 mmol) was added to a stirring solution of (S)-5-aminopiperidin-2-one (Tetrahedron Letters, 1995, 36, 8205-8) (789 mg, 3.68 mmol) in DMF (18 mL) at 0°C. The resulting suspension was stirred for 30 min at which point benzyl bromide (701 uL, 5.89 mmol) was added dropwise and stirred for 30 min. The reaction was then diluted with water (50 mL) and EtOAc (100 mL) and the resulting solution acidified to pH 7 with 1M HCl. The phases were separated and the aqueous layer was extracted twice more with EtOAc (100 mL). The combined organic layers were dried over Na₂SO₄, filtered, and evaporated to dryness. The residue was purified by SiO₂ chromatography (EtOAc in Hexanes, 0 to 100% gradient) and afforded the title compound (384 mg, 0.973 mmol, 26% yield) as a yellow oil.

### Step 2: (S)-tert-butyl benzyl(4-benzyl-4-azaspiro[2.5]octan-6-yl)carbamate

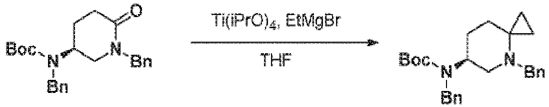

To a solution of ethylmagnesium bromide (831 uL, 2.5 mmol; 3.0 M in Et₂O) in THF (10 mL) was added titanium isopropoxide (325 uL, 1.097 mmol) at -78 °C and the solution was stirred for 10 min. Then, a solution of (S)-tert-butyl benzyl(1-benzyl-6-oxopiperidin-3-yl)carbamate (328 mg, 0.831 mmol) in THF (1 mL) was added dropwise at the same temperature, the mixture was allowed to warm up to room temperature over 2 h and was subsequently heated to 70 °C for overnight. The reaction mixture was cooled to room temperature and was quenched with 10% aq. NaOH (0.5 mL) and diluted with MeTHF (20 mL), washed with brine (30 mL), dried over Na₂SO₄, filtered, and concentrated under reduced pressure. The crude product was purified by SiO₂ chromatography (EtOAc in Hexanes, 0 to 100% gradient) to afford the title compound as a colorless oil (170 mg, 0.418 mmol, 50% yield).

### Step 3: (S)-4-azaspiro[2.5]octan-6-amine

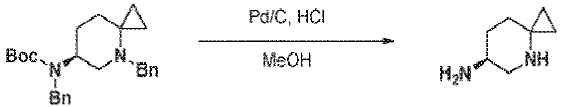

To a degassed solution of (S)-tert-butyl benzyl(4-benzyl-4-azaspiro[2.5]octan-6-yl)carbamate (144 mg, 0.352 mmol) in 5% HCl in MeOH (3.5 mL) was added Pd/C (10% wlw on activated carbon). The reaction mixture was then allowed to stir under a positive pressure of hydrogen (1 atm) at room temperature for 16 h. The reaction was monitored by LCMS. The reaction mixture was degassed with N₂, then filtered through a pad of Celite and concentrated under reduced pressure to afford the title compound as a light yellow solid (44 mg, 0.352 mmol, quantitative yield).

### Example 31. Synthesis of 2-benzyl-N-(4-(1-(phenylsulfonyl)-1H-indol-3-yl)-5-(trifluoromethyl)pyrimidin-2-yl)-1,2,3,4-tetrahydroisoquinolin-4-amine intermediate useful in the synthesis of Compound 215 and Compound 216

### Step 1: N-(isoquinolin-4-yl)benzamide

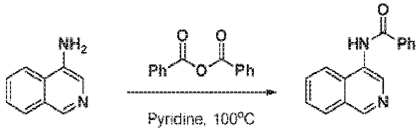

To a solution of isoquinolin-4-amine (1.00 g, 6.94 mmol) in pyridine (7 mL) at 25 °C under nitrogen, was added benzoic anhydride (1.57 g, 6.94 mmol) in pyridine (1.5 mL). The resulting solution was heated at 100 °C for 4 h, then cooled to 25°C and quenched using saturated solution of NaHCO₃ (5 mL). Crude product was extracted with CHCl₃ (5x10 mL). Combined organic phase was washed with water (2x15 mL) and brine (15 mL), then dried over Na₂SO₄, filtered, and concentrated under reduced pressure to yield the title compound as a purple-red solid (1.66 g, 6.70 mmol, 97% yield) that was used without further purification.

### Step 2: N-(1,2,3,4-tetrahydroisoquinolin-4-yl)benzamide

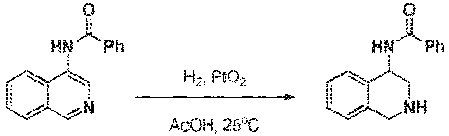

To a degassed solution of *N*-(isoquinolin-4-yl)benzamide (1.66 g, 6.70 mmol) in AcOH (134 mL) at 25 °C, was added PtO₂ (240 mg, 1.074 mmol) and then reaction mixture was stirred under hydrogen (1 atm) for 24 h. Mixture was filtered through a pad of Celite and concentrated to 30 mL of residue remaining, then diluted with CHCl₃ (50 mL) and basified to pH 9 using wetted NaHCO₃. Water (100 mL) was added and product was extracted with CHCl₃ (3x100 mL). Combined organic phase was then washed with water (2x50 mL), brine (2x50 mL), dried over Na₂SO₄, filtered, and concentrated under reduced pressure to afford a dark yellow foam of the title compound (1.36 g, 5.38 mmol, 80% yield) that was used without further purification.

### Step 3: N-(2-benzyl-1,2,3,4-tetrahydroisoquinolin-4-yl)benzamide

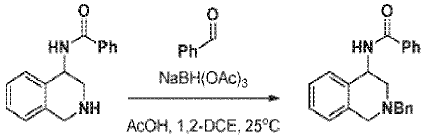

To a solution of N-(1,2,3,4-tetrahydroisoquinohn-4-yl)benzamide (434.3 mg, 1.72 mmol) in 1,2-dichloroethane (11.5 mL), was added AcOH (0.05 mL, 0.86 mmol) followed by the addition of benzaldehyde (237 mg, 2.24 mmol). The milky mixture was stirred at 25°C for 10 minutes under nitrogen, then NaBH(OAc)₃ (657 mg, 3.10 mmol) was added in one portion and the resulting mixture was stirred at room temperature for 16 h. Reaction mixture was diluted with DCM (50 mL) and water was slowly added at 0°C following by basification to pH 8 with saturated NaHCO₃. Crude product was extracted with DCM (2x30 mL). Combined organic phase was then washed with water (2x30 mL), brine (2x30 mL), dried over Na₂SO₄, filtered, and concentrated to afford the title compound as a white powder (565 mg, 1.65 mmol, 96% yield) that was used without further purification.

### Step 4: 2-benzyl-1,2,3,4-tetrahydroisoquinolin-4-amine

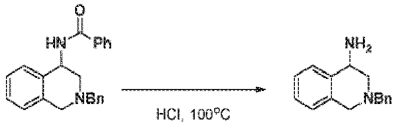

*N*-(2-benzyl-1,2,3,4-tetrahydroisoquinolin-4-yl)benzamide (430 mg, 1.256 mmol) in 6 M HCl (30 mL) was heated at 100 °C under nitrogen for 40 h. The resulting solution was then concentrated under reduced pressure. Residue was dissolved in DCM (10 mL) and MeOH (2 mL) and dried over Na₂SO₄, filtered, and concentrated to afford the title compound as a sticky brown-orange solid (299 mg, 1.26 mmol, quantitative yield) which was used without further purification.

### Step 5: 2-benzyl-N-(4-(1-(phenylsulfonyl)-1H-indol-3-yl)-5-(trifluoromethyl)pyrimidin-2-yl)-1,2,3,4-tetrahydroisoquinolin-4-amine

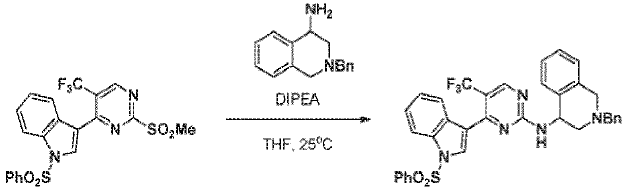

To a solution of 2-benzyl-1,3,4-tetrahydroisoquinolin-4-amine (299 mg, 1.26 mmol) in dry THF (12.5 mL) with *N,N-*diisopropylethylamine (2.75 ml, 6.27 mmol, dried over Na₂SO₄) under nitrogen, was added 3-(2-(methylsulfonyl)-5-(trifluaromethyl)pyrimidin-4-yl)-1-ζphenylsulfonyl)-1H-indole (404 mg, 0.84 mmol) at 25 °C. Reaction mixture was stirred at room temperature for 16 h, concentrated under reduced pressure, and then crude was purified by column chromatography on SiO₂ (EtOAc in DCM, 0 to 100% gradient). The title compound was obtained as a grey-green solid (484 mg, 0.757 mmol, 60% yield). 440 mg of this material was separated into the corresponding (S)- and (R)-enantiomers using a ChiralPak IA column with 5.000 ul injections and 10% MeOH, 10% DCM in hexane for elution. Peak 1 (A, tentatively assigned as (S)-enantiomer): 161.8 mg (>99% purity, 99.396 *ee)* as a pale yellow solid, and Peak 2 (B, tentatively assigned as (R)-enantiomer): 148.3 mg (>99% purity, 99.2% *ee)* as a pale yellow solid.

### Example 32. Synthesis of (2R,5S)-tert-butyl 5-amino-2-(pyrrolidine-1-carbonyl)piperidine-1-carboxylate intermediate useful to synthesize Compound 228.

### Step 1: (2R,5R)-1-(tert-butoxycarbonyl)-5-((tert-butyldimethylsilyl)oxy)piperidine-2-carboxylic acid

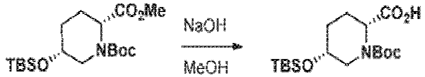

To a solution of (2R,SR)-1-*tert-*butyl 2-methyl 5-((tert-butyldimethylsilyl)oxy)piperidine-1,2-dicarboxylate (270 mg, 0.72 mmol) in MeOH (0.50 mL) was added NaOH (0.54 mL, 2M) at 0 °C. The reaction mixture was stirred at room temperature for 20 h. The reaction mixture was concentrated under reduced pressure to remove the MeOH and 1M HCl was carefully added until the reaction mixture had a pH of 2. The reaction mixture was extracted with MeTHF (3×10 mL). The organic phases were combined, dried over Na₂SO₄, and concentrated under reduced pressure to afford the title compound as a white solid (219 mg, 0.61 mmol, 84% yield) which was used without further purification.

### Step 2: (2R,5R)-tert-butyl 5-((tert-butyldimethylsilyl)oxy)-2-(pyrrolidine-1-carbonyl)piperidine-1-carboxylate

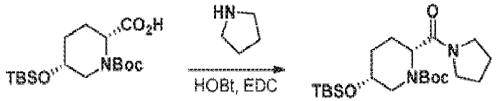

To a solution of (2R,5R)-1-(*tert*-butoxycarbonyl)-5-((tert-butyldimethylsilyl)oxy)piperidine-2-carboxylic acid (119 mg, 0.33 mmol) in DMF (1.0 mL) was added HOBt (54 mg, 0.40 mmol) and EDC (95 mg, 0.50 mmol). After stirring at room temperature for 1 h, pyrrolidine (31 mg, 0.43 mmol) was added. The reaction was allowed to stir at room temperature for overnight. The reaction mixture was then diluted with MeTHF (10 mL) and washed with saturated solution of NaHCO₃ (50 mL). The phases were separated and the organic was washed twice more with H₂O (50 mL) and brine (50 mL). The organic phase was then dried over Na₂SO₄ and concentrated under reduced pressure. The residue was purified by SiO₂ chromatography (EtOAc in Hexanes, 0 to 100% gradient) to afford the title compound as an off-white solid (112 mg, 0.27 mmol, 82% yield).

### Step 3: (2R,5R)-tert-butyl 5-hydroxy-2-(pyrrolidine-1-carbonyl)piperidine-1-carboxylale

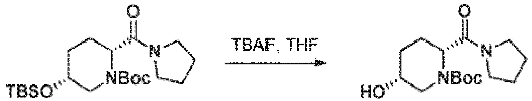

To a solution of (2R,5R)-tert-butyl 5-((*tert*-butyldimethylsilyl)oxy)-2-(pyrrolidine-1-carbonyl)piperidine-1-carboxylate (205 mg, 0.50 mmol) in THF (16.6 mL) was added TBAF (1.99 mL, 1M solution in THF) at 0 °C. The reaction mixture was warmed to room temperature and stirred for overnight. The reaction mixture was quenched with saturated solution NH₄Cl (50 mL, added slowly at first) and extracted with EtOAc (3x100 mL). The phases were separated, dried over Na₂SO₄ and concentrated under reduced pressure. The residue was purified by reverse phase chromatography (C18, MeCN in aq. 10mM ammonium formate pH 3.8, 0 to 100% gradient) to afford the title compound as a white solid (112 mg, 0.375 mmol, 76% yield).

### Step 4: (2R,5R)-tert-butyl 5-((methylsulfonyl)oxy)-2-(pyrrolidine-1-carbonyl)piperidine-1-carboxylate

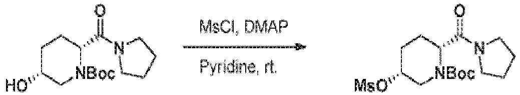

To a solution of (2R,5R)-tert-butyl 5-hydroxy-2-(pyrrolidine-1-carbonyl)piperidine-1-carboxylate (72 mg, 0.241 mmol) and 4-dimethylaminopyridine (2.9 mg, 0.024 mmol) in pyridine (1.6 mL) at 0 °C, was added methanesulfonyl chloride (16 uL, 0.253 mmol) dropwise. The reaction mixture was stirred at room temperature for 16 h and then concentrated under reduced pressure to remove most of the solvent. Brine (50 mL) was added and the reaction mixture was extracted with ethyl acetate (3x50mL). The organic layers were combined, dried over Na₂SO₄ and concentrated under reduced pressure. The residue was purified by SiO₂ chromatography (EtOAc in Hexanes, 0 to 100% gradient) to afford the title compound as clear oil (55 mg, 0.146 mmol, 61% yield).

### Step 5: (2R,5S)-tert-butyl 5-azido-2-(pyrrolidine-1-carbonyl)piperidine-1-carboxylate

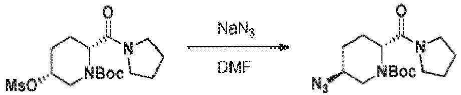

To a solution of (2R,5R)-tert-butyl 5-((methylsulfonyl)oxy)-2-(pyrrolidine-1-carbonyl)piperidine-1-carboxylate (55 mg, 0.146 mmol) in DMF (1.5 mL) was added NaN₃ (28.5 mg, 0.438 mmol) at room temperature. The reaction mixture was stirred at 60 °C for 2 days, monitoring by LCMS. The reaction mixture was diluted with water (20 mL) and then crude product was extracted with EtOAc (2x50 mL), dried over Na₂SO₄ and concentrated under reduced pressure. The residue was purified by SiO₂ chromatography (EtOAc in Hexanes, 0 to 100% gradient) to afford the title compound as pale oil (13 mg, 0.040 mmol, 28% yield).

### Step 6: (2R,5S)-tert-butyl 5-amino-2-(pyrrolidine-1-carbonyl)piperidine-1-carboxylate

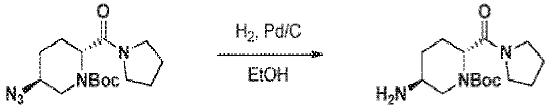

To a degassed solution of (2R,5S)-tert-butyl 5-azido-2-(pyrrolidine-1-carbonyl)piperidine-1-carboxylate (13 mg, 0.040 mmol) in EtOH (1.3 mL), Pd/C (10% wlw on activated carbon; 1 mg, 0.004 mmol) was added and the reaction mixture was stirred under H₂ (1 atm) at room temperature for overnight. The reaction mixture was degassed with N₂, filtered through a pad of Celite and then concentrated under reduced pressure to afford the title compound (12 mg, 0.040 mmol, quantitative yield) as an off-white solid which was used without further purification.

### Example 33. Synthesis of (3S)-tert-butyl 3-((5-(1-hydroxyethyl)-4-(1-(phenylsulfonyl)-1H-indol-3-yl)pyrimidin-2-yl)amino)piperidine-1-carboxylate intermediate for use in the synthesis of Compound 234 and its enantiomers Compound 238 and Compound 239.

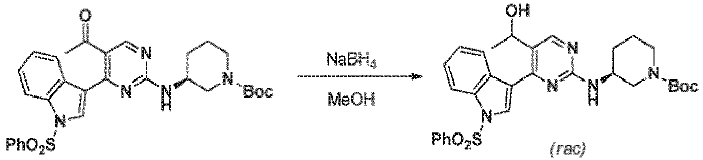

To a solution of (S)-tert-butyl 3-((5-acetyl-4-(1-(phenylsulfonyl)-1H-indol-3-yl)pyrimidin-2-yl)amino)piperidine-1-carboxylate (328 mg, 0.570 mmol) in MeOH (6 mL), was added sodium borohydride (32.0 mg, 0.855 mmol). The reaction mixture was stirred at room temperature for 1 h, after which another portion of sodium borohydride (32.0 mg, 0.855 mmol) was added. Reaction mixture was stirred another 1 h at room temperature and was following by LCMS. Reaction mixture was then concentrated, the residue was re-dissolved in MeTHF (20 mL), washed with brine (20 mL), dried over Na₂SO₄, filtered and concentrated to dryness. The crude product was purified by SiO₂ chromatography (EtOAc in DCM, 0 to 100% gradient) to afford the *racemic* title compound (230 mg, 0.398 mmol, 70% yield) as light yellow solid. 166 mg of *racemic* compound were separated by preparative chiral HPLC (Chiralpak IA, Sum, 20x250 mm, 6:6:88 = MeOH/DCM/hexane+0.1%DEA, 16-22 mg/inj) providing Peak 1 (79 mg) as a white solid, 99.9% *de,* and Peak 2 (75 mg) as a white solid, 98.0% *de.*

### Example 34. Synthesis of N-((3S)-1-benzyl-6-pentylpiperidin-3-yl)-4-(1-(phenylsulfonyl)-1H-indol-3-yl)-5-(trifluoromethyl)pyrimidin-2-amine and (S)-1-benzyl-N-(4-(1-(phenylsulfonyl)-1H-indol-3-yl)-5-(trifluoromethyl)pyrimidin-2-yl)-1-azaspiro[5.5]undecan-3-amine intermediates for use in synthesis of Compound 240 and Compound 241

### Step 1: tert-butyl ((3S)-1-benzyl-6-pentylpiperidin-3-yl)carbamate (A) and (S)-tert-butyl (1-benzyl-1-azaspiro[5.5]undecan-3-yl)carbamate (B)

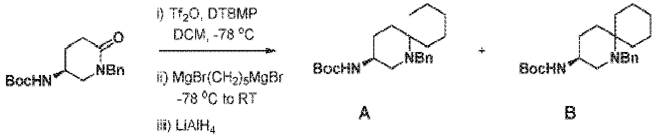

To a solution of (S)-tert-butyl (1-benzyl-6-oxopiperidin-3-yl)carbamate (300 mg, 0.986 mmol) in DCM (10 mL), was added di-tert-butyl methylpyridine (243 mg, 1.183 mmol) and cooled to -78 °C. Tf₂O (0.20 mL, 1.183 mmol) was added dropwise to the mixture and the resulting orange solution was stirred at -78 °C for 1 h, at which point the pentamethylene bis-(magnesium bromide) (2.07 mL, 1.035 mmol) was added dropwise. The resulting solution stirred for 7 h at - 78 °C, and then slowly warmed to 25 °C over 16 h. Reaction mixture was then cooled down again to -78 °C and additional Tf₂O (0.20 mL, 1.183 mmol) was added forming a bright orange suspension, which was stirred at this temperature for 2 h before adding additional pentamethylene bis-(magnesium bromide) (2.07 mL, 1.04 mmol). Reaction mixture was slowly warmed to 25 °C over 16 h, and then quenched with water (2 mL), and concentrated under reduced pressure. Water (10 mL) and EtOAc (10 mL) were added, and the aqueous layer was extracted with EtOAc (2x15 mL). Collected organics was washed with water (2x15 mL), (1:1) water/brine (2x10 mL), brine (10 mL), then dried over Na₂SO₄, filtered and concentrated under reduced pressure. The residue was re-dissolved in DCM (3 mL, dried over Na₂SO₄), added di-tert-butyl methylpyridine (243 mg, 1.183 mmol) and cooled to -78 °C. Tf₂O (0.20 mL, 1.183 mmol) was added dropwise and allowed mixture to stir at -78 °C for 1 h before adding pentamethylene bis-(magnesium bromide) (4.14 mL, 2.07 mmol) dropwise and stirring for 3 h warming up to 25 °C, then stirred for 16 h. Reaction mixture was then cooled down to 0 °C and LiAlH₄ (135 mg, 3.55 mmol) was added followed by stirring for 1 h and warming up to room temperature. Wetted Na₂SO₄ (150 mg) was added, and reaction mixture was stirred for 1 h followed by filtration under vacuum. After being concentrated under reduced pressure, a green-brown oily residue was taken up in MeTHF (10 mL), washed with water (5 mL), and extracted from aqueous layer with MeTHF (2×15 mL). Collected organics was washed with water (2×10 mL), brine (10 mL), dried over Na₂SO₄, filtered, and concentrated to afford 780 mg of a mixture of the title compounds (A) and (B).

### Step 2: (3S)-1-benzyl-6-pentylpiperidin-3-amine (C) and (S)-1-benzyl-1-azaspiro[5.5]undecan-3-amine (D)

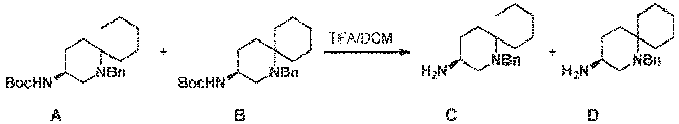

To a mixture of (**A**) and (**B**) from the previous experiment (473 mg, 0.985 mmol) in DCM (10 mL), TFA (3.35 mL, 49.23 mmol) was added, and the resulting solution was stirred at 25 °C for 48 h. Then, reaction mixture was concentrated under reduced pressure, azeotroped few times with DCM to provide a mixture of the title compounds (C) and (D) as an orange oil (85 mg, 0.325 mmol, 33%) which was used without further purification.

### Step 3: N-((3S)-1-benzyl-6-pentylpiperidin-3-yl)-4-(1-(phenylsulfonyl)-1H-indol-3-yl)-5-(trifluoromethyl)pyrimidin-2-amine (E) and (S)-1-benzyl-N-(4-(1-(phenylsulfonyd)-1H-indol-3-yl)-5-(trifluoromethyl)pyrimidin-2-yl)-1-azaspiro[5.5]undecan-3-amine (F)

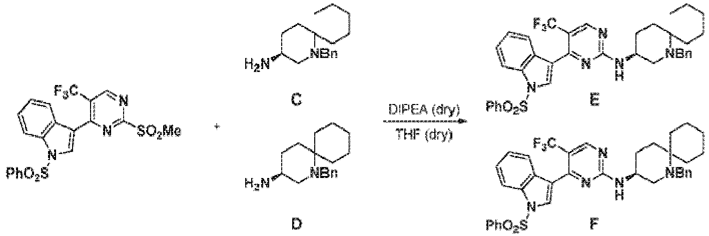

To a mixture of amines (C) and (D) (85 mg, 0.325 mmol) in dry THF (4 mL), 3-(2-(methylsulfonyl)-5-(trifluoromethyl)pyrimidin-4-yl)-1-(phenylsulfonyl)-1H-indole (314 mg, 0.650 mmol) and dry *N,N-*diisoprapylethylamine (1.42 mL, 3.25 mmol) were added. Resulting reaction mixture was stirred at 25 °C for 16 h, then concentrated under reduced pressure. Residue was re-dissolved in MeTHF (5 mL) and water (10 mL), aqueous layer was extracted with MeTHF (2x5 mL). Combined organics was washed with water (3x10 mL) and brine (2x10 mL), dried over Na₂SO₄, filtered and concentrated. The crude was purified by reverse-phase column chromatography (C18, MeCN in aq. ammonium formate pH 3.8, 10 mM, 0 to 100% gradient) to afford the title compound (E) (not pure, enriched in (E), 76.0 mg, 0.115 mmol, 35% yield) as a yellow solid, and pure (F) (24.1 mg, 0.037 mmol, 11% yield) as an orange paste.

### Example 35. Synthesis of (2R,5S)-tert-butyl 5-amino-2-((S)-3-hydroxypyrrelidine-1-carbonyl)piperidine-1-carboxylate intermediate useful in the Synthesis of Compound 244 and Compound 250.

### Step 1: (2R,5R)-1-tert-butyl 2-methyl 5-((methylsulfonyl)oxy)piperidine-1,2-dicarboxylale

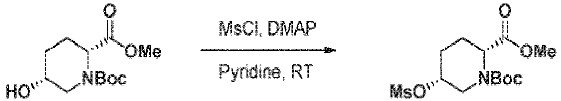

To a solution of (2R,5R)-1-*tert*-butyl 2-methyl 5-hydroxypiperidine-1,2-dicarboxylate (329 mg, 1.269 mmol) (WO 2006125974) and 4-dimethylaminopyridine (140 mg, 1.14 mmol) in pyridine (12.7 mL) at 0 °C, was added methanesulfonyl chloride (89 uL, 1.396 mmol) dropwise. The reaction mixture was stirred at room temperature for 12 h and then concentrated under reduced pressure. Brine (50 mL) was added and the reaction mixture was extracted with ethyl acetate (3x50 mL). The organic layers were combined, dried over Na₂SO₄, and concentrated under reduced pressure. The residue was purified by SiO₂ chromatography (EtOAc in DCM:Hexanes (1:1), 0 to 60% gradient) to afford the title compound as a clear oil (182 mg, 0.539 mmol, 43% yield).

### Step 2: (2R,5S)-1-tert-butyl 2-methyl 5-azidopiperidine-1,2-dicarboxylate

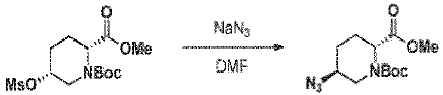

To a solution of (2R,5R)-1-tert-butyl 2-methyl 5-((methylsulfonyl)oxy)piperidine-1,2-dicarboxylate (182 mg, 0.539 mmol) in DMF (5.4 mL), was added NaN₃ (105 mg, 1.62 mmol) at room temperature. The reaction mixture was stirred at 60 °C for 16 h, monitoring by LCMS. The reaction mixture was diluted with water (20 mL) and extracted with EtOAc (2x50 mL), dried over Na₂SO₄ and concentrated under reduced pressure. The residue was purified by SiO₂ chromatography (EtOAc in HexaneszDCM (1:1), 0 to 40% gradient) to afford the title compound as a pale oil (95 mg, 0.330 mmol, 62% yield).

### Step 3: (2R,5S)-5-azido-1-(tert-butoxycarbonyl)piperidine-2-carboxylic acid

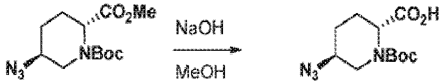

To a solution of (2R,5S)-1-tert-butyl 2-methyl 5-azidopiperidine-1.2-dicarboxylate (95 mg, 0.330 mmol) in MeOH (2.2 mL), was added NaOH (0.10 mL, 5M) at 0 °C. The reaction mixture was stirred at room temperature for 20 h. The reaction mixture was concentrated under reduced pressure to remove the MeOH and 1M HCl was carefully added until the reaction mixture had a pH of 2. The reaction mixture was extracted with MeTHF (3x10 mL). The organic phases were combined, dried over Na₂SO₄. and concentrated under reduced pressure to afford the title compound (90 mg, 0.330 mmol, quantitative yield) as a white solid which was used without further purification.

### Step 4: (2R,5S)-tert-butyl 5-azido-2-((S)-3-hydroxypyrrolidine-1-carbonyl)piperidine-1-carboxylate

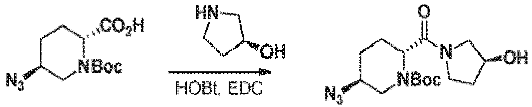

To a solution of (2R,5S)-5-azido-1-(*tert*-butoxycarbonyl)piperidine-2-carboxylic acid (90 mg, 0.330 mmol) and (S)-pyrrolidin-3-ol (31 uL, 0.383 mmoL) in DMF (6.7 mL), was added HBTU (189 mg, 0.499 mmoL) and DIPEA (290 uL, 1.66 mmol). The reaction was allowed to stir at room temperature for overnight The reaction mixture was then diluted with MeTHF (10 mL) and washed with saturated solution of NaHCO₃ (50 mL). The phases were separated and the organic was washed with H₂O (2x50 mL) and brine (50 mL). The organic phase was then dried over Na₂SO₄, and concentrated under reduced pressure. The residue was purified by SiO₂ chromatography (THF in DCM, 0 to 50% gradient) to afford the title compound as clear oil (92 mg, 0.271 mmol, 81% yeld).

### Step 5: (2R,5S)-tert-butyl 5-amino-2-((S)-3-hydroxypyrrolidine-1-carbonyl)piperidine-1-carboxylate

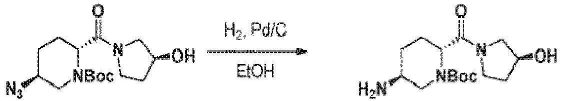

To a degassed solution of (2R,5S)-*tert*-butyl 5-azido-2-((S)-3-hydroxypyrrolidine-1-carbonyl)piperidine-1-carboxylate (92 mg, 0.271 mmol) in EtOH (5.4 mL), Pd/C (10% w/w on activated carbon; 4.3 mg, 0.041 mmol) was added and the reaction mixture was stirred at room temperature for overnight under hydrogen (1 atm). The reaction mixture was degassed with N₂, filtered through a pad of Celite and then concentrated under reduced pressure to afford the title compound (66 mg, 0.211 mmol, 78% yield) as clear oil which was used without further purification.

### Example 36. Synthesis of 3-(4,4,5,5-tetramethyl-1,3,2-dioxaborolan-2-yl)-1-((2-(trimethylsilyl)ethoxy)methyl)-1H-indole-6-carbonitrile intermediate useful in the synthesis of Compound 246.

### Step 1: 3-iodo-1H-indole-6-carbonitrile

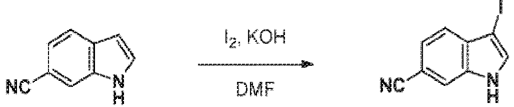

To a suspendion of 6-cyanoindole (2.00 g, 14.1 mmol) in DMF (40 mL) at 0 °C, was added KOH (1.58 g, 28.1 mmol) and iodine (3.61 g, 14.2 mmol) and the reaction mixture was stirred for 30 min. The reaction mixture was quenched with water (100 mL) and extracted with EtOAc (100 mL). The organic layer was washed with a saturated solution of Na₂S₂O₃ (100 mL), water (100 mL) and brine (100 mL), dried over Na₂SO₄ and concentrated under reduced pressure to afford the title compound (3.73 g, 13.9 mmol, 99% yield) as a light brown-orange solid.

### Step 2: 3-iodo-1-((2-(trimethylsilyl)ethoxy)methyl)-1H-indole-6-carbonitrile

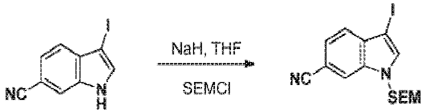

To a suspension of 3-iodo-1H-indole-6-carbonitrile (3.73 g, 13.9 mmol) in DMF (70 mL) at 0 °C, was added NaH, 60% suspension in mineral oil (668 mg, 16.7 mmol). The reaction was stirred for 1 h at this temperature followed by addition of SEM-Cl (2.96 mL, 16.7 mmol). The reaction mixture was then allowed to stir for another 1 h. The reaction mixture was quenched with water (100 mL), extracted with EtOAc (3x150 mL). The organics were combined, dried over Na₂SO₄, filtered, and concentrated under reduced pressure. The residue was purified by flash chromatography on SiO₂ (EtOAc in Hexanes, 0 to 30% gradient) to afford the title compound (4.05 g, 10.17 mmol, 73% yield) as a light yellow oil.

### Step 3: 3-iodo-1H-indole-6-carbonitrile

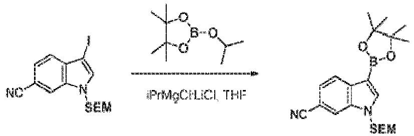

To a solution of 3-iodo-1-((2-(trimethylsilyl)ethoxy)methyl)-1H-indole-6-carbonitrile (500 mg, 1.26 mmol) in THF (8.4 mL) at -10 °C, was added isopropyl magnesium chloride lithium chloride complex (Turbo Grignard) (1.06 mL, 1.3 M, 1.38 mmol) dropwise. The solution was stirred for 10 min and then 2-isopropoxy-4,4,5,5-tetramethyl-1,3,2-dioxaborolane (0.44 mL, 2.13 mmol) was added dropwise. The reaction mixture was stirred for 1 h. The reaction mixture was then quenched with saturated ammonium chloride solution (100 mL) and extracted with EtOAc (3x150 mL). The organics were then combined, dried over Na₂SO₄, filtered, and concentrated under reduced pressure to afford the title compound (500 mg, 1.26 mmol, quantitative yield) as a light green oil which was used without further purification.

### Example 37. Synthesis of 5-(1-methoxyethyl)-4-(1-(phenylsulfonyl)-1H-indol-3-yl)-N-((S)-piperidin-3-yl)pyrimidin-2-amine intermediate useful in the synthesis of Comopund 247.

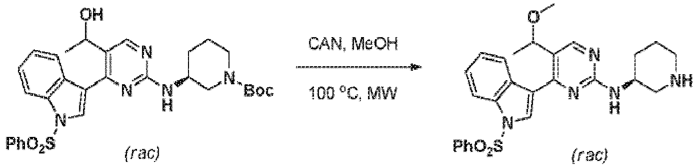

To methanol (1 mL) solution of (3S)-*tert*-butyl 3-((5-(1-hydroxyethyl)-4-(1-(phenylsulfanyl)-1H-indol-3-yl)pyrimidin-2-yl)amino)piperidine-1-carboxylate (32 mg, 0.055 mmol) in a 0.5-2.5 mL MW tube, CAN (12 mg, 0.022 mmol) was added, and the reaction mixture was stirred at 100 °C under MW irradiation twice for 15 min. Reaction mixture was then concentrated, re-dissolved in MeTHF (5 mL), washed with saturated solution of NaHCO₃ (5 mL). Organic phase was dried over Na₂SO₄, filtered, and concentrated providing the title compound (25 mg, 0.051 mmol, 93% yield) which was used without further purification.

### Example 38. Synthesis of (3R,5S)-tert-butyl 3-(methylcarbamoyl)-5-((4-(1-(phenylsulfonyl)-1H-indol-3-yl)-5-(trifluoromethyl)pyrimidin-2-yl)amino)piperidine-1-carboxylate (A), (3S,5R)-tert-butyl 3-(methylcarbamoyl)-5-((4-(1-(phenylsulfonyl)-1H-indol-3-yl)-5-(trifluoromethyl)pyrimidin-2-yl)amino)piperidine-1-carboxylate (B), (3R,5R)-tert-butyl 3-(methylcarbamoyl)-5-((4-(1-(phenylsulfonyl)-1H-indol-3-yl)-5-(trifluoromethyl)pyrimidin-2-yl)amino)piperidine-1-carboxylate (C), and (3S,5S)-tert-butyl 3-(methylcarbamoyl)-S-((4-(1-(phenylsulfonyl)-1H-indol-3-yl)-5-(trifluoromethyl)pyrimidin-2-yl)amino)piperidine-1-carboxylate (D), Intermediates useful in the syntheses of Compound 252, Compound 253, Compound 255, and Compound 256.

### Step1: 1-tert-butyl 3-methyl 5-(((benzyloxy)carbonyl)amino)piperidine-1,3-dicarboxylate

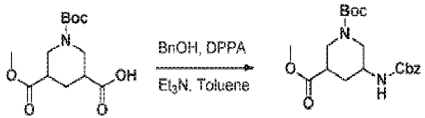

To a solution of 1-(*tert*-butoxycarbonyl)-5-(methoxycarbonyl)piperidine-3-carboxylic acid (1.32 g, 4.59 mmol) (synthesized as in US 5817678A) in toluene (18.4 mL), was added triethylamine (2.26 mL, 16.08 mmol) and diphenylphosphoryl azide (DPPA) (1.19 mL, 5.51 mmol). The resulting solution was stirred at 110 °C for 1 h. The mixture was then cooled down to 80 °C, benzyl alcohol (2.38 mL, 22.97 mmol) and triethylamine (2.26 mL, 16.08 mmol) were added, and the resulting mixture was stirred at 80 °C for 18 h. The reaction mixture was then allowed to cool down to room temperature, then diluted with water (30 mL) and extracted with EtOAc (3x50 mL). The organic phase was combined and washed with brine (2x50 mL), dried over Na₂SO₄, and concentrated under reduced pressure. The residue was purified by SiO₂ chromatography (EtOAc in DCM, 0 to 100% gradient) to afford the title compound (556 mg, 1.42 mmol, 31% yield) as a pale yellow oil.

### Step 2: 5-(((benzyloxy)carbonyl)amino)-1-(tert-butoxycarbonyl)piperidine-3-carboxylic acid

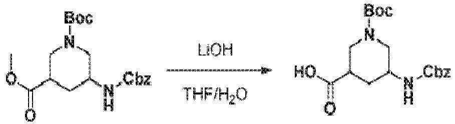

To a solution of 1-tert-butyl 3-methyl 5-(((benzyloxy)carbonyl)amino)piperidine-1,3-dicarboxylate (556 mg, 1.42 mmol) in THF (14.2 mL), was added a solution of LiOH (170 mg, 7.08 mmol) in distilled water (14.2 mL), and the resulting suspension was stirred at 23 °C for 1 h. Mixture was then concentrated under reduced pressure, diluted with water (20 mL), carefully acidified to pH 4 using 2N HCl and extracted with EtOAc (3x20 mL). The combined organic phase was washed with water at pH 4 (3×10mL), brine (10 mL), and then dried over Na₂SO₄, filtered, concentrated under reduced pressure to afford the title compound (536 mg, 1.42 mmol, quantitative yield) as a white foam which was used without further purification.

### Step 3: tert-butyl 3-(((benzyloxy)carbonyl)amino)-5-(methylcarbamoyl)piperidine-1-carboxylate

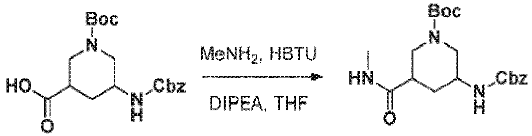

To a solution of 5-(((benzyloxy)carbonyl)amino)-1-(tert-butoxycarbonyl)piperidine-3-carboxylic acid (536 mg, 1.42 mmol) in DMF (7.3 mL) in a scalable tube, was added DIPEA (1.27 mL, 7.31 mmol), HBTU (832 mg, 2.19 mmol) and MeNH₂ (2M in THF, 14.6 mL, 29.2 mmol). The tube was sealed, and the reaction mixture was stirred at 23 °C for 1 h. Mixture was then concentrated, diluted with water (20 mL) and carefully acidified with 1N HCl to pH 5. Water phase was extracted with EtOAc (3x20 mL), and the combined organics was washed with brine (5×10mL), then dried over Na₂SO₄, filtered, and concentrated. The residue was purified by flash chromatography on SiO₂ (EtOAc in DCM, 0 to 100% gradient) to afford the title compound (514 mg, 1.31 mmol, 92% yield) as a white foam.

### Step 4: tert-butyl 3-amino-5-(methylcarbamoyl)piperidine-1-carboxylate

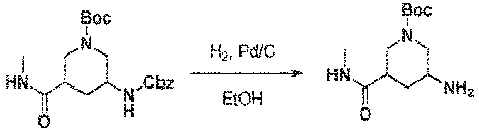

To a solution of tert-butyl 3-(((benzyloxy)carbonyl)amino)-5-(methylcarbamoyl)piperidine-1-carboxylate (514 mg, 1.31 mmol) in EtOH (13 mL), was added Pd/C (10% w/w; 70 mg, 0.066 mmol). The reaction mixture was stirred under hydrogen atmosphere (1 atm) for 15 h. The suspension was filtered through a pad of Celite, rinsed with EtOH and MeTHF, then concentrated under reduced pressure to afford the title compound (338 mg, 1.31 mmol, quantitative yield) as a yellow oil which was used without further purification.

### Step 5: (3R,5S)-tert-butyl 3-(methylcarbamoyl)-5-((4-(1-(phenylsulfonyl)-1H-indol-3-yl)-5-(trifluoromethyl)pyrimidin-2-yl)amino)piperidine-1-carboxylate (A), (3S,5R)-tert-butyl 3-(methylcarbamoyl)-5-((4-(1-(phenylsulfonyl)-1H-indol-3-yl)-5-(trifluoromethyl)pyrimidin-2-yl)amino)piperidine-1-carboxylate (B), (3R,5R)-tert-butyl 3-(methylcarbamoyl)-5-((4-(1-(phenylsulfonyl)-1H-indol-3-yl)-5-(trifluoromethyl)pyrimidin-2-yl)amino)piperidine-1-carboxylate (C), (3S,5S)-tert-butyl 3-(methylcarbamoyl)-5-(4-(1-(phenylsulfonyl)-1H-indol-3-yl)-5-(trifluoromethyl)pyrimidin-2-yl)amino)piperidine-1-carboxylate (D).

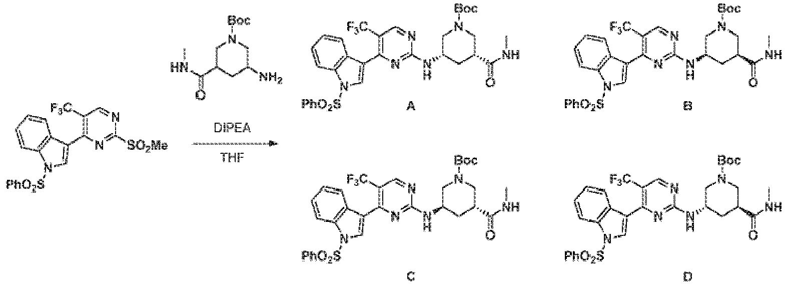

To a solution of *racemic tert*-butyl 3-amino-5-(methylcarbamoyl)piperidine-1-carboxylate (338 mg, 1.31 mmol) in dry THF (13 mL), was added DIPEA (2.88 mL, 6.57 mmol) and 3-(2-(methylsulfonyl)-5-(trifluoromethyl)pyrimidin-4-yl)-1-(phenylsulfonyl)-1H-indole (632 mg, 1.31 mmol) and stirred at 23 °C for 48 h. The reaction mixture was then concentrated under reduced pressure, water (20 mL) was added, and crude product was extracted with MeTHF (3x20 mL). The combined organics was washed with water (2×20mL), brine (10 mL), then dried over Na₂SO₄, filtered, and concentrated under reduced pressure. The residue was purified by flash chromatography on SiO₂ (EtOAc in DCM, 0 to 100% gradient). Obtained *racemic* material (566 mg, 0.859 mmol, 65% yield) was separated by preparative chiral HPLC (ChiralPak IA column, 10% MeOH, 10% DCM in hexanes with 0.1% MeNH₂ for elution, 5.000ul inj.) providing four stereoisomers **A-D** as white foams. Peak 1 (**C**, 70 mg, 0.107 mmol, 8% yield, 98.3% *de*)*,* Peak 2 (**A**, 147 mg, 0.224 mmol, 17% yield, 98.1% *de*), Peak 3 (**B**, 151 mg, 0.229 mmol, 17% yield, 98.2% *de*), Peak 4 (**D**, 64 mg, 0.096 mmol, 7% yield, 99.4% *de*); stereochemistry is tentatively assigned.

### Example 39. Synthesis of cis-1-tert-butyl 3-methyl 5-((4-(1-(phenylsulfonyl)-1H-indol-3-yl)-5-(trifluoromethyl)pyrimidin-2-yl)amino)piperidine-1,3-dicarboxylate intermediate useful in the synthesis of Compound 266.

### Step1: cis-1-tert-butyl 3-methyl 5-aminopiperidine-1,3-dicarboxylate

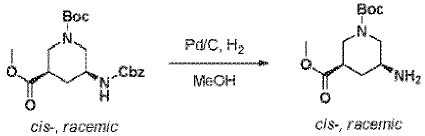

To a solution of *cis-*1*-tert-*butyl 3-methyl 5-(((benzyloxy)carbonyl)amino)piperidine-1,3-dicarboxylate (860 mg, 2.191 mmol) in MeOH (22 mL) at 23 °C under inert atmosphere, was added palladium on carbon (10% wlw; 233 mg, 0.219 mmol). The reaction mixture was stirred under hydrogen atmosphere (1 atm) for 1 h. The suspension was then filtered through a pad of Celite and concentrated under reduced pressure providing a title compound (566 mg, 2.191 mmol, quantitative yield) as a beige solid which was used without further purification.

### Step 2: cis-1-tert-butyl 3-methyl 5-((4-(1-(phenylsulfonyl)-1H-indol-3-yl)-5-(trifluoromethyl)pyrimidin-2-yl)amino)piperidine-1,3-dicarboxylate

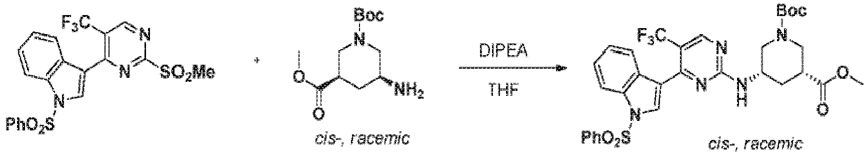

*Cis-*1*-tert-*butyl 3-methyl 5-aminopiperidine-1,3-dicarboxylate (566 mg, 2.191 mmol) and DIPEA (1.15 mL, 6.57 mmol) were added to a stirring solution of 3-(2-(methylsulfonyl)-5-(trifluoromethyl)pyrimidin-4-yl)-1-(phenylsulfonyl)-1H-indole (1.05 g, 2.191 mmol) in dry THF (37 mL). The reaction mixture was stirred at room temperature for overnight The mixture was then diluted with EtOAc (30 mL), washed with brine (30 mL), dried over Na₂SO₄, filtered, and concentrated under reduced pressure. The residue was purified by SiO₂ chromatography (EtOAc in DCM, 0 to 100% gradient) to afford the title compound (430 mg, 0.652 mmol, 30% yield) as a light yellow oil.

### Step 3: cis-1-(tert-butoxycarbonyl)-5-((4-(1-(phenylsulfonyl)-1H-indol-3-yl)-5-(trifluoromethyl)pyrimidin-2-yl)amino)piperidine-3-carboxylic acid

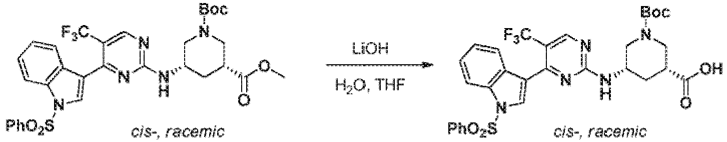

To a solution of *cis-1-tert-butyl* 3-methyl 5-((4-(1-(phenylsulfonyl)-1H-indol-3-yl)-5-(trifluoromethyl)pyrimidin-2-yl)amino)piperidine-1,3-dicarboxylate (414 mg, 0.627 mmol) in THF (5 mL), was added a solution of LiOH·H₂O (26.3 mg, 0.627 mmol) in H₂O (5 mL). The reaction mixture was stirred at room temperature for 56 h. Another portion of LiOH·H₂O (79 mg, 1.881 mmol) was added, and the mixture was stirred for another 18 h. The reaction mixture was quenched by the addition of 1M HCl until a pH 3, then concentrated from THF under reduced pressure. Aqueous layer was extracted with EtOAc (2x30 mL), the organic phase was then washed with water (30 mL) and brine (30 mL), dried over Na₂SO₄, filtered, and concentrated under reduced pressure to afford the title compound (394 mg, 0.610 mmol, 97% yield) as a white solid which was used without further purification.

### Step 4: cis-1-tert-butyl 3-methyl 5-((4-(1-(phenylsulfonyl)-1H-indol-3-yl)-5-(trifluoromethyl)pyrimidin-2-yl)amino)piperidine-1,3-dicarbaxylate

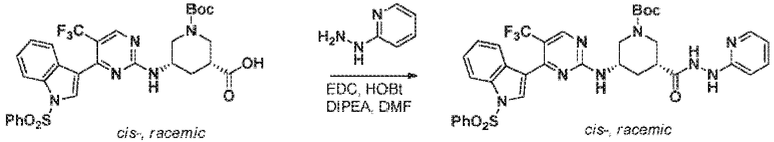

To a solution of *cis*-1-(*tert-*butoxycarbonyl)-5-((4-(1-(phenylsulfonyl)-1H-indol-3-yl)-5-(trifluoromethyl)pyrimidin-2-yl)amino)piperidine-3-carboxylic acid (394 mg, 0.610 mmol) in DMF (6 mL), was added HOBt (99 mg, 0.740 mmol), EDC (176 mg, 0.920 mmol) and DIPEA (214 uL, 1.230 mmol). After stirring at room temperature for 1 h, 2-hydrazinylpyridine (134 mg, 1.230 mmol) was added. The reaction was allowed to stir at room temperature for overnight. The reaction mixture was then diluted with EtOAc (50 mL), washed with saturated solution of NaHCO₃ (50 mL), water (2x50 mL), and brine (50 mL), then dried over Na₂SO₄, filtered, and concentrated under reduced pressure. The residue was purified by SiO₂ chromatography (THF in DCM, 0 to 100% gradient) to afford the title compound (375 mg, 0.509 mmol, 83% yield) as a beige semi-solid.

### Step 5: cis-1-tert-butyl 3-methyl 5-((4-(1-(phenylsulfonyl)-1H-indol-3-yl)-5-(trifluoromethyl)pyrimidin-2-yl)amino)piperidine-1,3-dicarboxylate

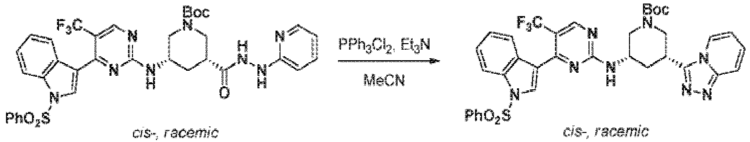

To a solution of *cis-*1*-tert-*butyl 3-methyl 5-((4-(1-(phenylsulfonyl)-1H-indol-3-yl)-5-(trifluoromethyl)pyrimidin-2-yl)amino)piperidine-1,3-dicarboxylate (375 mg, 0.509 mmol) in acetonitrile (11 mL), was added triethylamine (0.20 mL, 1.43 mmol) and PPh₃Cl₂ (339 mg, 1.02 mmol) portion wise. The reaction mixture was stirred under heating at 80 °C for 1 hour. The mixture was the cooled down to room temperature, diluted with EtOAc (30 mL) and washed with saturated aq. solution of NaHCO₃ (50 mL). The aqueous phase was extracted with EtOAc (30 mL), and the combined organic phase was washed with brine (30 mL), dried over Na₂SO₄, filtered, and concentrated under reduced pressure. The residue was purified by reverse phase chromatography (C18, MeCN in aq.10 mM ammonium format, pH 3.8, 0 to 100% gradient) to afford the title *racemic cis*-compound (300 mg, 0.417 mmol, 82% yield) as a yellow solid.

### Example 40. 5-chloro-4-[7-(1, 1-dioxo-1, 4-thiazinan-4-yl) -1H-indol-3-yl]-N-[(3S)-3-piperidyl]pyrimidin-2-amine (Compound 196).

### Step 1: 4-(1H-indol-7-yl) thiomorpholine

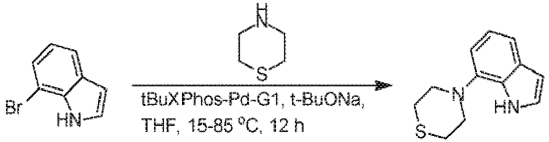

To a mixture of 7-bromo-1H-indole (2.00 g, 10.20 mmol, 1.00 eq) and thiomorpholine (1.05 g, 10.20 mmol, 965.82 uL, 1.00 eq) in THF (50.00 mL) was added NaOt-Bu (1.96 g, 20.40 mmol, 2.00 eq) and [2-(2-aminoethyl) phenyl]-chloro-palladium; ditert-butyl-[2-(2,4,6-triisopropylphenyl)phenyl]phosphine (700.56 mg, 1.02 mmol, 0.10 eq) in one portion at 15 °C under N₂. The mixture was stirred at 85 °C for 12 h. The mixture (combined with another batch) was poured into water (100 mL) and extracted with EtOAc (30 mLx2). The combined organic phase was washed with brine (50 mLx2), dried with anhydrous Na₂SO₄, filtered and concentrated in vacuum. The residue was purified by silica gel chromatography (PE/EtOAc=20/1, 5/1) to afford the title compound (2.20 g, 10.08 mmol, 98.79% yield) as kelly solid

### Step 2: 4-[3-(2,5-dichloropyrimidin -4-yl)-1H-indol-7-yl]thiomorpholine

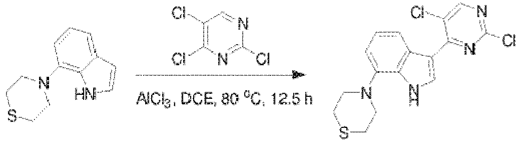

A mixture of AlCl₃ (122.15 mg, 916.09 umol, 50.06 uL, 1.00 eq) and 2,4,5-trichloropyrimidine (202 mg, 10.99 mmol, 1.20 eq) in DCE (50.00 mL) was stirred at 80 °C for 30 min, then 4-(1H-indol-7-yl)thiomorpholine (200.00 mg, 916.09 umol, 1.00 eq) was stirred at 80 °C and stirred for 12 h. The mixture (combined with another batch) was poured into water (200 mL) and extracted with EtOAc (100 mLx2). The combined organic phase was washed with brine (100 mLx2), dried with anhydrous Na₂SO₄, filtered and concentrated in vacuum. The residue was purified by silica gel chromatography (PE/EtOAc=10/1, 1/1) to afford the title compound (2.00 g, 5.48 mmol, 59.83% yield) as yellow solid.

### Step 3: 4-[1-(benzenesulfonyl)-3-(2,5-dichloro pyrimidin-4-yl)indol-7-yl]thiomorpholine

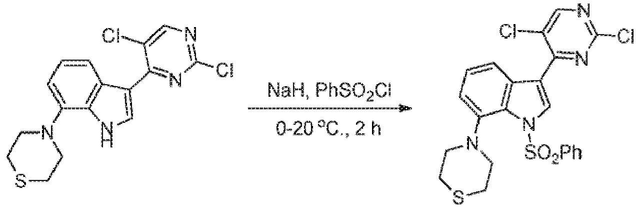

To a solution of 4-[3-(2, 5-dichloropyrimidin-4-yl)-1H-indol-7-yl] thiomorpholine (500.00 mg, 1.37 mmol, 1.00 eq) in DMF (10.00 mL) was added NaH (82.20 mg, 2.06 mmol, 60% purity, 1.50 eq). The mixture was stirred at 0 °C for 0.5 h, then benzenesulfonyl chloride (362.95 mg, 2.06 mmol, 263.01 uL, 1.50 eq) was added and the mixture was stirred at 20 °C for 1 h. The residue was poured into water (100 mL). The aqueous phase was extracted with EtOAc (50 mLx3). The combined organic phase was washed with brine (100 mL), dried with anhydrous Na₂SO₄, filtered and concentrated in vacuum to afford the title compound (500.00 mg, crude) as a yellow solid.

### Step 4: 4-[1-(benzenesulfonyl)-3-(2,5- dichloropyrimidin-4-yl)indol-7-yl]-1,4-thiazinane 1,1-dioxide

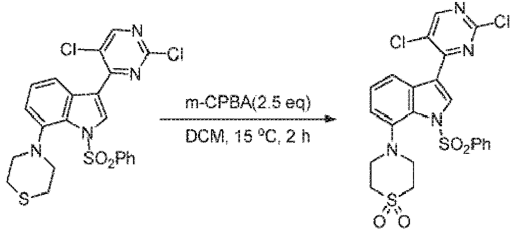

To a mixture of 4-[1-(benzenesulfonyl)-3-(2,5-dichloropyrimidin-4-yl)indol-7-yl] thiomorpholine (200.00 mg, 395.69 umol, 1.00 eq) in DCM (3.00 mL) was added m-CPBA (243.88 mg, 989.22 umol, 70% purity, 2.50 eq) in one portion at 15 °C under N2. The mixture was stirred at 15 °C for 2 h. The mixture (combined with another batch) was poured into water (10 mL) and extracted with DCM (5 mLx2). The combined organic phase was washed with aqueous NaHCO₃ (10 mLx2), dried with anhydrous Na₂SO₄, filtered and concentrated in vacuum. The residue was purified by silica gel chromatography (PE/EtOAc=5/1, 0/1) to afford the title compound (250.00 mg, crude) as yellow solid.

### Step 5: tert-butyl (3S)-3-[[4-[1-(benzenesulfonyl) -7-(1,1-dioxo-1,4-thiazinan-4-yl)indol-3-yl]-5-chloro-pyrimidin-2-yl]amino]piperidine-1-carboxylate

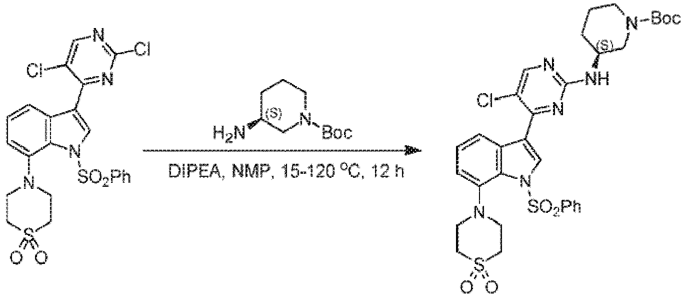

To a mixture of 4-[1-(benzenesulfonyl)-3-(2,5-dichloropyrimidin-4-yl)indol -7-yl]-1,4-thiazinane 1,1-dioxide (250.00 mg, 465.17 umol, 1.00 eq) and tert-butyl (3S)-3-aminopiperidine-1-carboxylate (139.75 mg, 697.76 umol, 1.50 eq) in NMP (5.00 mL) was added DIPEA (300.59 mg, 2.33 mmol, 406.20 uL, 5.00 eq) in one portion at 15 °C under N₂. The mixture was stirred at 120 °C for 12 h. The mixture was poured into water (10 mL) and extracted with EtOAc (10 mLx2). The combined organic phase was washed with brine (20 mLx2), dried with anhydrous Na₂SO₄, filtered and concentrated in vacuum. The residue was purified by silica gel chromatography (DCM/MeOH=100/1, 20/1) to afford the title compound (300.00 mg, crude) as yellow solid.

### Step 6: tert-butyl (3S)-3-[[5-chloro-4-[7-(1, 1-dioxo-1,4-thiazinan-4-yl)-1H-indol-3-yl]pyrimidm-2-yl]amino]piperidine-1-carboxylate

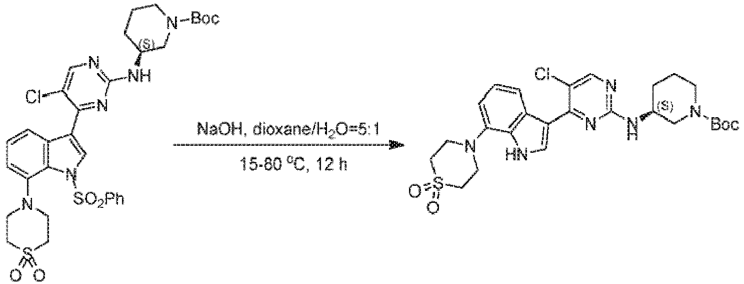

To a mixture of tert-butyl (3S)-3-[[4-[1-(benzenesulfonyl)-7-(1,1-dioxo- 1,4-thiazinan-4-yl)indol-3-yl]-5-chloro-pyrimidin-2-yl]amino]piperidine-1-carboxylate (300.00 mg, 427.81 umol, 1.00 eq) in dioxane (5.00 mL) and H₂O (1.00 mL) was added NaOH (34.22 mg, 855.62 umol, 2.00 eq) in one portion at 15 °C under N₂. The mixture was stirred at 80 °C for 12 h. The residue was poured into water (10 mL) and extracted with EtOAc (10 mLx2). The combined organic phase was washed with brine (10 mLx2), dried with anhydrous Na₂SO₄, filtered and concentrated in vacuum to afford the title compound (200.00 mg, crude) as yellow solid.

### Step 7: 5-chloro-4-[7-(1,1-dioxo-1,4-thiazinan-4-yl) -1H-indol-3-yl]-N-[(3S)-3-piperidyl]pyrimidin-2-amine

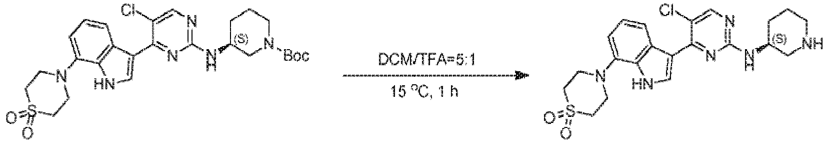

To a mixture of tert-butyl(3S)-3-[[5-chloro-4-[7-(1,1-dioxo-1,4-thiazinan-4-yl)-1H-indol-3-yl]pyrimidin-2-yl]amino]piperidine-1-carboxylate (150.00 mg, 267.34 umol, 1.00 eq) in DCM (5.00 mL) was added TFA (1.00 mL) in portions at 15 °C under N₂. The mixture was stirred at 15 °C for 1 h. The mixture (combined with another batch) was concentrated under reduced pressure. The residue was purified by prep-HPLC(FA) to afford the title compound (50.00 mg, 98.62 umol, 36.89% yield, FA) as white solid.

### Example 41. 5-fluoro-3-[2-[[(3S)-3-piperidyl] amino]-5-(trifluoromethyl)pyrimidin-4-yl]-1H-indole-6-carbonitrile (Compound 197).

### Step 1: (E)-2-(4-bromo-5-fluoro-2-nitro-phenyl)-N, N-dimethyl-ethenamine

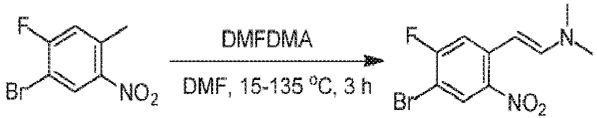

To a mixture of 1-bromo-2-fluoro-4-methyl-5-nitro-benzene (6.00 g, 25.64 mmol, 1.00 eq) in DMF (60.00 mL) was added DMFDMA (3.36 g, 28.20 mmol, 3.73 mL, 1.10 eq) in one portion at 15 °C under N₂. The mixture was stirred at 135 °C for 3 hours. The mixture was poured into water (200 mL) and extracted with EtOAc (100 mLx2).The combined organic phase was washed with brine (100 mLx2), dried with anhydrous Na₂SO₄, filtered and concentrated in vacuum. The residue was purified by silica gel chromatography (PE/EtOAc=50/1, 10/1) to afford the title compound (6.00 g, crude) as brown solid.

### Step 2:6-bromo-5-fluoro-1H-indole

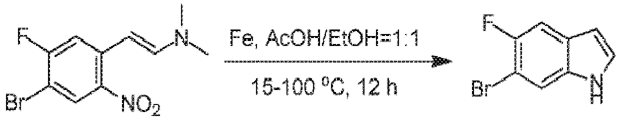

To a mixture of (E)-2-(4-bromo-5-fluoro-2-nitro-phenyl)-N,N-dimethyl-ethenamine (2.00 g, 6.92 mmol, 1.00 eq) in EtOH (30.00 mL) and AcOH (30.00 mL) was added Fe (1.93 g, 34.60 mmol, 5.00 eq) in one portion at 15 °C under N₂. The mixture was stirred at 100 °C for 12 h. The reaction mixture was filtered and the filter was concentrated. The residue was purified by silica gel chromatography (PE/EtOAc=20/1, 5/1) to afford the tide compound (700.00 mg, 3.27 mmol, 47.26% yield) as yellow solid.

### Step 3: 5-fluoro-1H-indole-6-carbonitrile

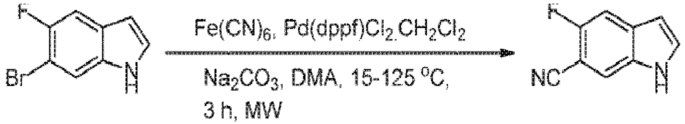

To a mixture of 6-bromo-5-fluoro-1H-indole (350.00 mg, 1.64 mmol, 1.00 eq) and tetrapotassium;hexacyanoiron (906.12 mg, 2.46 mmol, 1.50 eq) in DMA (10.00 mL) was added Na₂CO₃ (347.65 mg, 3.28 mmol, 2.00 eq) and Pd(dppf)Cl₂.CH₂Cl₂ (133.93 mg, 164.00 umol, 0.10 eq) in one portion at 15 °C under N₂. The mixture was stirred at 125 °C under MW for 3 h. The mixture was poured into water (30 mL) and extracted with EA (20 mLx2). The combined organic phase was washed with brine (30 mLx2), dried with anhydrous Na₂SO₄, filtered and concentrated in vacuum. The residue was purified by column (PE/EtOAc=20/1, 511) to afford the title compound (600.00 mg, crude) as white solid.

### Step 4: 5-fluoro-1-(2-trimethylsilylethoxymethyl)indole-6-carbonitrile

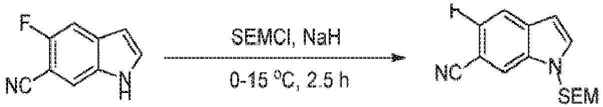

To a mixture of 5-fluoro-1H-indole-6-carbonitrile (350.00 mg, 2.19 mmol, 1.00 eq) in DMF (10.00 mL) was added NaH (175.20 mg, 4.38 mmol, 60% purity, 2.00 eq) in portions at 0 °C under N₂. The mixture was stirred at 15 °C for 30 min, then SEM-Cl (438.14 mg, 2.63 mmol, 466.11 uL, 1.20 eq) was added and stirred at 15 °C for 2 h. The mixture was poured into water (50 mL) and extracted with EtOAc (20 mLx2). The combined organic phase was washed with brine (30 mLx2), dried with anhydrous Na₂SO₄, filtered and concentrated in vacuum. The residue was purified by column (PE/EtOAc=20/1, 5/1) to afford the title compound (600.00 mg, crude) as white solid.

### Step 5: 3-bromo-5-fluoro-1-(2-trimethylsilylethoxymethyl) indole-6-carbonitrile

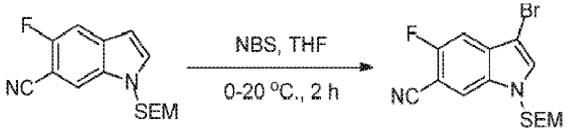

To a mixture of 5-fluoro-1-(2-trimethylsilylethoxymethyl)indole-6-carbonitrile (500.00 mg, 1.72 mmol, 1.00 eq) in THF (3.00 mL) was added NBS (337.07 mg, 1.89 mmol, 1.10 eq) in one portion at 0 °C under N₂. The mixture was stirred at 20 °C for 2 h. The mixture was concentrated in reduced pressure. The residue was purified by silica gel chromatography (PE/EtOAc=20/1, 5/1) to afford the title compound (450.00 mg, used directly) as white solid.

### Step 6: 5-fluoro-3-(4,4,5,5-tetramethyl-1,3,2-dioxaborolan-2-yl)-1-(2-trimethylsilylethoxymethyl)indole-6-carbonitrile

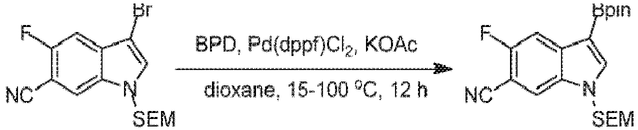

To a mixture of 3-bromo-5-fluoro-1-(2-trimethylsilylethoxymethyl)indole-6 -carbonitrile (350.00 mg, 947.74 umol, 1.00 eq) and BPD (361.00 mg, 1.42 mmol, 1.50 eq) in dioxane (10.00 mL) was added KOAc (186.02 mg, 1.90 mmol, 2.00 eq) and Pd(dppf)Cl₂ (69.35 mg, 94.77 umol, 0.10 eq) in one portions at 15 °C under N₂. The mixture was stirred at 100 °C for 12 h. The mixture was poured into water (30 mL) and extracted with EtOAc (15 mLx2). The combined organic phase was washed with brine (20 mLx2), dried with anhydrous Na₂SO₄, filtered and concentrated in vacuum. The residue was purified by silica gel chromatography (PE/EtOAc=20/1, 511) to afford the title compound (250.00 mg, crude) as yellow oil.

### Step7: tert-butyl(3S)-3-[[4-[6-cyano-5-fluoro-1-(2-trimethylsilylethoxymethyl)indol -3-yl]-5-(trifluoromethyl)pyrimidin-2-yl]amino]piperidine-1-carboxylate

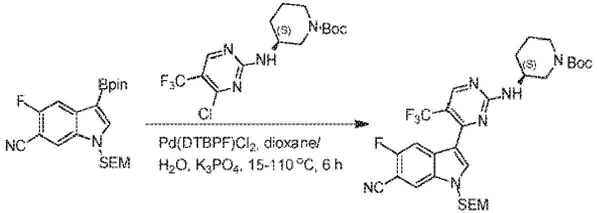

To a mixture of 5-fluoro-3-(4,4,5,5-tetramethyl-1,3,2-dioxaborolan-2-yl)-1-(2-trimethylsilylethoxymethyl)indole-6-carbonitrile (250.00 mg, 600.43 umol, 1.00 eq) and tert-butyl(3S)-3-[[4-chloro-5-(trifluoromethyl)pyrimidin-2-yl]amino] piperidine-1-carboxylate (342.96 mg, 900.65 umol, 1.50 eq) in dioxane (10.00 mL) and H₂O (2.00 mL) was added K₃PO₄ (254.91 mg, 1.20 mmol, 2.00 eq) and ditert-butyl(cyclopentyl)phosphane;dichloropalladium;iron (39.13 mg, 60.04 umol, 0.10 eq) in one portion at 15 °C under N₂. The mixture was stirred at 110 °C for 6 h. The mixture was poured into water (20 mL) and extracted with EtOAc (10 mLx2). The combined organic phase was washed with brine (10 mLx2), dried with anhydrous Na₂SO₄, filtered and concentrated in vacuum. The residue was purified by silica gel chromatography (PE/EtOAc=30/1, 511) to afford the title compound (200.00 mg, 315.09 umol, 52.48% yield) as yellow oil.

### Step 8: 5-fluoro-3-[2-[[(3S)-3-piperidyl]amino]-5-(triflluoromethyl)pyrimidin-4-yl]-1H-indole-6-carbonitrile

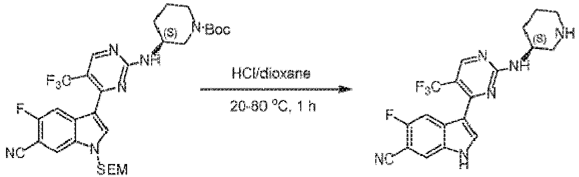

To a mixture of tert-butyl (3S)-3-[[4-[6-cyano-5-fluoro-1-(2-trimethylsilylethoxy methyl) indol-3-yl]-5-(trifluoromethyl) pyrimidin-2-yl] amino] piperidine-1-carboxylate (150.00 mg, 236.32 umol, 1.00 eq) in dioxane (5.00 mL) was added HCl (500.00 uL) in portions at 20 °C under N₂. The mixture was stirred at 80 °C for 1 h. The mixture was concentrated under reduced pressure. The residue (combined with another batch) was purified by prep-HPLC (FA) to afford the title compound (20.00 mg, 44.41 umol, 18.79% yield, FA) as white solid.

### Example 42. 4-(6-methylsulfonyl-1H-indol-3-yl)-N-[(3S)-3-piperidyl]-5-(trifluoromethyl) pyrimidin- 2- amine (Compound 199).

### Step 1: 6-methylsulfonyl-1H-indole

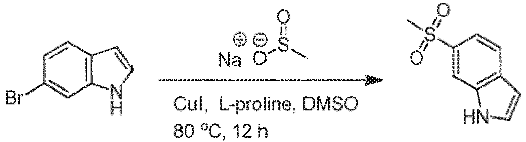

A mixture of 6-bromo-1H-indole (2.00 g, 10.20 mmol, 1.00 eq), BLAHsodium (1.35 g, 13.26 mmol, 1.30 eq), CuI (388.59 mg, 2.04 mmol, 0.20 eq) and L-PROLINE (469.82 mg, 4.08 mmol, 0.40 eq) in DMSO (20.00 mL) was degassed and purged with N₂ for 3 times, and then the mixture was stirred at 80 °C for 12 h under N₂ atmosphere. The reaction mixture was quenched by addition saturated aqueous NH₄Cl 200 mL at 20 °C, and then extracted with EtOAc (100 mLx3). The combined organic layers were washed with brine (300 mLx2), dried over Na₂SO₄, filtered and concentrated under reduced pressure to give a residue. The residue was purified by column chromatography (SiO₂, PE/EtOAc=10:1 to 1:1) to afford the title compound (700 mg) as an off-white solid.

### Step 2: 3-[2-chloro-5 -(trifluoromethyl)pyrimidin-4-yl] -6-methylsulfonyl- 1H-indole

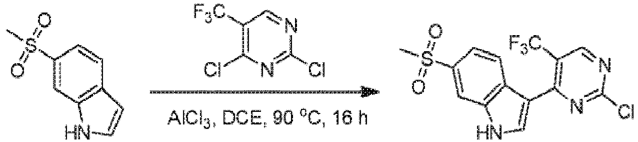

To a solution of 2, 4- dichloro-5-(tiifluoromethyl) pyrimidine (1.17 g, 5.39 mmol, 1.50 eq) in DCE (20.00 mL) was added AlCl₃ (765.90 mg, 5.74 mmol, 313.90 uL, 1.60 eq). After addition, the mixture was stirred at 90 °C for 30 min, and then 6-methylsulfonyl-1H-indole (700.00 mg, 3.59 mmol, 1.00 eq) was added at 90 °C. The resulting mixture was stirred at 90 °C for 15.5 h. The reaction was stopped. The reaction mixture was diluted with water 30 mL and extracted with DCM (20 mLx3). The combined organic layers were washed with brine (100 mLx2), dried over Na₂SO₄, filtered and concentrated under reduced pressure to give a residue (500 mg). The residue was purified by prep-HPLC (TFA condition) to give desired compound. The pH of the eluent solution was adjusted to 8 with saturated aqueous NaHCO₃, and the solution was extracted with EtOAc (20 mLx3). The combined organic layers were washed with brine (50 mLx2), dried over Na₂SO₄, filtered and concentrated under reduced pressure to afford the title compound (300.00 mg, 758.48 umol, 21.13% yield, 95% purity) as a yellow solid.

### Step 3: tert-butyl (3S)-3-[[4-(6-methylsulfonyl-1H-indol-3-yl)-5-(trifluoromethyl) pyrimidin-2-yl] amino] piperidine-1- carboxylate

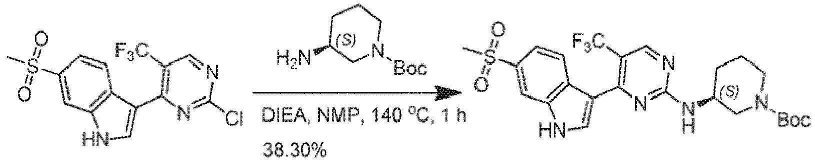

A mixture of 3-[2-chloro-5-(trifluoromethyl)pyrimidin-4-yl]-6-methylsulfonyl-1H-indole (320.00 mg, 851.63 umol, 1.00 eq), tert-butyl (3S)-3-aminopiperidine-1-carboxylate (255.85 mg, 1.28 mmol, 1.50 eq) and DIEA (330.19 mg, 2.55 mmol, 446.21 uL, 3.00 eq) in NMP (5.00 mL) was stirred at 140 °C for 1h. The reaction was stopped. The reaction mixture was diluted with water 50 mL and extracted with EtOAc (20 mLx3). The combined organic layers were washed with brine (100 mLx2), dried over Na₂SO₄, filtered and concentrated under reduced pressure to give a residue. The residue was purified by column chromatography (SiO₂, PE/EtOAc =10:1 to 2:1) to afford the tide compound (200.00 mg, 326.19 umol, 38.30% yield, 88% purity) as a yellow oil.

### Step 4: 4-(6-methylsulfonyl-1H-indol-3-yl)-N-[(3S)-3-piperidyl]-5-(trifluoromethyl) pyrimidin- 2- amine

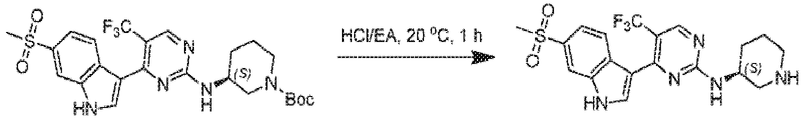

A mixture of tert-butyl (3S)-3-[[4-(6-methylsulfonyl-1H- indol-3-yl)-5-(trifluoromethyl) pyrimidin-2-yl] amino] piperidine-1-carboxylate (200.00 mg, 370.67 umol, 1.00 eq) and HCl/EtOAc (4 M, 2.00 mL) was stirred at 20 °C for 1h. The reaction was stopped. The reaction mixture was concentrated under reduced pressure to give desired compound as a yellow solid, the residue was adjusted pH to 8 with saturated aqueous NaHCO₃, and extracted with EtOAc (20 mLx3). The combined organic layers were washed with brine (100 mL x2), dried over Na₂SO₄, filtered and concentrated under reduced pressure to give a residue (120 mg). The residue was purified by prep-HPLC (FA condition) to afford the title compound (52.70 mg, 107.47 umol, 28.99% yield, 99% purity, FA) as a white solid.

### Example 43. N-methyl-3-[2-[[(3S)-3- piperidyl] amino] - 5- (trifluoromethyl) pyrimidin-4-yl]-1H-indole-6-sulfonamide (Compound 200).

### Step 1: 1H-indole-6-sulfonyl chloride

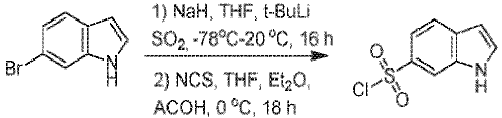

To a solution of Crbromo-1H-indole (2.00 g, 10.20 mmol, 1.00 eq) in THF (20 mL) was added batchwise NaH (408.00 mg, 10.20 mmol, 60% purity, 1.00 eq) at 0 °C. After addition, the mixture was stirred at this temperature for 30 min, the mixture was cooled to -78 °C and then t-BuLi (1.3 M, 15.69 mL, 2.00 eq) was added dropwise. The resulting mixture was stirred at -78 °C for 30 min, then SO₂ (3.1 M in THF, 6.58 mL, 19% purity, 2.00 eq) was added dropwise, the mixture was stirred at 20 °C for 16 h. (To the resulting solid was added 30 mL of Et₂O and 0.76 mL of glacial acetic acid. The mixture was stirred for 30 min at 0 °C, and filtered). The solids were then suspended in 30 mL of Et₂O, chilled to 0 ⁹C, and 1.36 g of NCS was carefully added. The resulting suspension was stirred rapidly for 30 min. The reaction was stopped. The reaction mixture was filtered and the filtrate was concentrated to afford the title compound (1.22 g) as a black brown solid. It was used in the next step directly.

### Step 2: N-methyl-1H-indole-6-sulfonamide

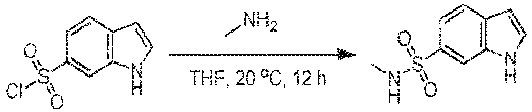

A mixture of 1H-indole-6-sulfonyl chloride (1.22 g, 5.66 mmol, 1.00 eq), methanamine (2 M in THF, 14.15 mL, 5.00 eq) in THF (5.00 mL) was stirred at 20 °C for 12 h. The reaction mixture was diluted with water 30 mL and extracted with EtOAc (15 mLx3). The combined organic layers were washed with brine (50 mLx2), dried over Na₂SO₄, filtered and concentrated under reduced pressure to give a residue. The residue was purified by column chromatography (SiO₂, PE/EtOAc =5:1 to 2:1) to afford the title compound (520 mg) as a black brown oil.

### Step 3: 3-[2-chloro-5- (trifluoromethyl) pyrimidin-4-yl] -N-methyl-1H-indole-6-sulfonamide

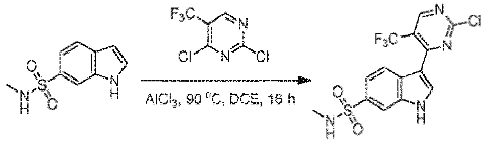

To a solution of 2, 4-dichloro-5 - (trifluoromethyl) pyrimidine (866.89 mg, 4.00 mmol, 2.00 eq) in DCE (6.00 mL) was added AlCl₃ (559.36 mg, 4.20 mmol, 229.25 uL, 2.10 eq). The mixture was stirred at 90 °C for 30 min, and then N-methyl-1H-indole-6-sulfonamide (420.00 mg, 2.00 mmol, 1.00 eq) was added. The resulting mixture was stirred at 90 °C for 15.5 h. The reaction was stopped. The reaction mixture was filtered. The filtrate was diluted with water (50 mL) and extracted with EtOAc (30 mLx3). The combined organic layers were washed with brine (100 mLx2), dried over Na₂SO₄, filtered and concentrated under reduced pressure to give a residue.

The residue was purified by column chromatography (SiO2, PE/EtOAc =10:1 to 1:1) to get desired product as a red solid (330 mg) which was then purified by prep-HPLC (TFA condition). The pH of the solution after prep-HPLC was adjusted to 8 by saturated aqueous NaHCO₃ and extracted with EtOAc (20 mLx3). The combined organic layers were washed with brine (100 mLx2), dried over Na₂SO₄, filtered and concentrated under reduced pressure to afford the title compound (140 mg) as a yellow solid. (Note: combined purification with other two batches in 50 mg scale in the same condition).

### Step 4: Tert-butyl (3S)-3-[[4-[6-(methylsulfamoyl) - 1H-indol-3-yl]-5-(trifluoromethyl) pyrimidin -2 -yl] amino] piperidine-1-carboxylate

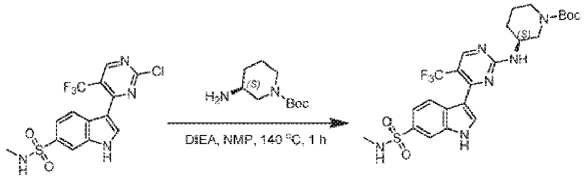

A mixture of 3-[2-chloro-5- (trifluoromethyl)pyrimidin-4-yl]- N-methyl-1H- indole-6-sulfonamide (120.00 mg, 307.09 umol, 1.00 eq), tert-butyl (3S)-3- aminopiperidine-1-carboxylate (92.26 mg, 460.63 umol, 1.50 eq) and DIEA (119.06 mg, 921.27 umol, 160.89 uL, 3.00 eq) in NMP (2.00 mL) was stirred at 140 °C for 1 h. The reaction was stopped. The reaction mixture was diluted with water 20 mL and extracted with EtOAc (10 mLx3). The combined organic layers were washed with brine (30 mLx2), dried over Na₂SO₄, filtered and concentrated under reduced pressure to afford the title compound (540.00 mg, crude) as a black brown oil. It was used in the next step directly.

### Step 6: N-methyl-3-[2-[[(3S)-3- piperidyl] amino]- 5- (trifluoromethyl)pyrimidin-4-yl]-1H-indole-6-sulfonamide

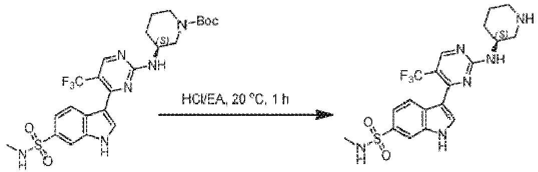

A mixture of tert-butyl (3S)-3-[[4-[6-(methylsulfamoyl) - 1H-indol-3-yl]-5- (trifluoromethyl) pyrimidin-2-yl] amino] piperidine-1-carboxylate (540.00 mg, 973.69 umol, 1.00 eq) and HCl/EtOAc (4 M, 5.00 mL) was stirred at 20 °C for 1 h. The reaction was stopped. The reaction mixture was concentrated under reduced pressure to give desired compound as a yellow solid, the residue was adjusted pH to 8 by saturated aqueous NaHCO₃, and extracted with EtOAc (10 mLx3). The combined organic layers were washed with brine (50 mLx2), dried over Na₂SO₄, filtered and concentrated under reduced pressure to give a residue The residue was purified by prep-HPLC (FA condition) to afford the title compound (20.30 mg, 40.56 umol, 4.17% yield, 100% purity, FA) as a yellow solid.

### Example 44. 5-chloro-4-(1H-indol-3-yl)-N-[(3S, 5R)-5-methyl-3-piperidyl] pyrimidin-2-amine (Compound 201).

### Step 1: Methyl (2S)-5-oxopyrrolidine-2-carboxylate

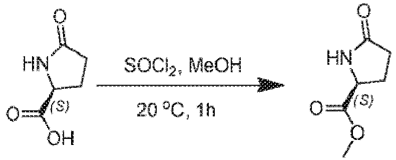

To a solution of (2S)-5-oxopyrrolidine-2-carboxylic acid (20.00 g, 154.91 mmol, 1.00 eq) in MeOH (100.00 mL) was added SOCl₂ (36.86 g, 309.82 mmol, 22.48 mL, 2.00 eq). The mixture was stirred at 20 °C for 1 h. The reaction mixture was concentrated. The residue was diluted with EtOAc (250 mL) and TEA (20 mL), the solid formed and filtered. The filtrate was concentrated to afford the title compound (27.90 g, crude) as a yellow oil. The crude product was used to next step without further purification.

### Step 2: O1-tert-butyl O2-methyl (2S)-5-oxopyrrolidine-1, 2-dicarboxylate

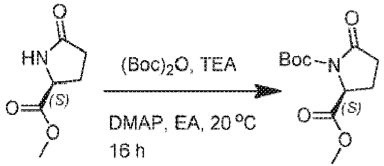

To a solution of methyl (2S)-5-oxopyrrolidine-2-carboxylate (27.90 g, 194.91 mmol, 1.00 eq) and DMAP (2.86 g, 23.39 mmol, 0.12 eq) in EtOAc (150.00 mL) was added dropwise tert-butoxycarbonyl tert-butyl carbonate (55.30 g, 253.39 mmol, 58.21 mL, 1.30 eq). The mixture was stirred at 20 °C for 16 h. The reaction mixture was washed with HCl (1M, 50 mL), Sat. NaHCO₃ (150 mL), brine (500 mL), dried over Na2SO4, filtered and concentrated under reduced pressure to give a residue. The residue was purified by re-crystallization from MTBE (250 mL) to afford the title compound (21.75 g, 88.52 mmol, 45,4196 yield, 99% purity) as a yellow solid.

### Step 3: O₁-tert-butyl O₂-methyl (2S)-4-methyl-5-oxo-pyrrolidine-1, 2-dicarboxylate

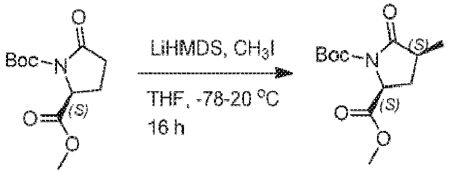

To a solution of O1-tert-butyl O2-methyl (2S)-5-oxopyrrolidine-1, 2-dicarboxylate (21.75 g, 89.41 mmol, 1.00 eq) in THF (400.00 mL) was added dropwise LiHMDS (1 M, 98.35 mL, 1.10 eq) at -78 °C under N₂ atmosphere. After addition, the mixture was stirred at this temperature for 0.5 h, and then iodomethane (31.73 g, 223.53 mmol, 13.92 mL, 2.50 eq) was added dropwise at - 78 °C under N₂ atmosphere. The resulting mixture was stirred at 20 °C for 15.5 h. Glacial HOAc (10 mL) in THF (50 mL) was used to quench the reaction. The solvent was removed on the rotary evaporator and treated with water (500 mL) and EtOAc (150 mL) and stirred for 10 min. The aqueous layer was removed and extracted with EtOAc (200 mLx3), and the organic phase was dried over Na₂SO₄, and evaporated under reduced pressure. The residue was purified by column chromatography (SiO₂, PE/EtOAc =10:1 to 5:1) to afford the title compound (17.57 g, used directly) as a yellow oil.

### Step 4: Tert-butyl N-[(1S)-4-hydroxy-1-(hydroxymethyl)-3-methyl-butyl] carbamate

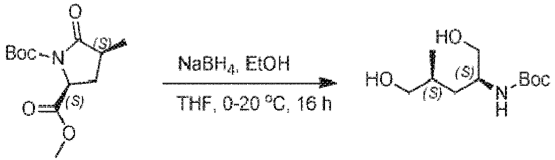

To a solution of O1-tert-butyl O2-methyl (2S)-4-methyl-5-oxo-pyrrolidine-1,2-dicarboxylate (17.57 g, 68.29 mmol, 1.00 eq) in THF (200.00 mL) was added batchwise NaBH₄ (7.75 g, 204.87 mmol, 3.00 eq) at 0 °C under N₂ atmosphere. After addition, EtOH (34.70 g, 753.24 mmol, 43.93 mL, 11.03 eq) was added dropwise at 0 °C. The resulting mixture was stirred at 20 °C for 16 h.

The reaction mixture was adjust pH to 5 by AcOH, and then H₂O (200 mL) was added and extracted with EtOAc (100 mLx3). The combined organic layers were washed with Sat. NaHCO₃, dried over Na₂SO₄, filtered and concentrated under reduced pressure to afford the title compound (11.00 g, crude) as a yellow oil.

### Step 5: [(2R,4S)-4-(tert-butoxycarbonylamino)-2-methyl-5-methylsulfonyloxy-pentyl] methanesulfonate and [(2S,4S)-4-(tert-butoxycarbonylamino) -2-methyl-5-methylsulfonyloxy-pentyl] methanesulfonate

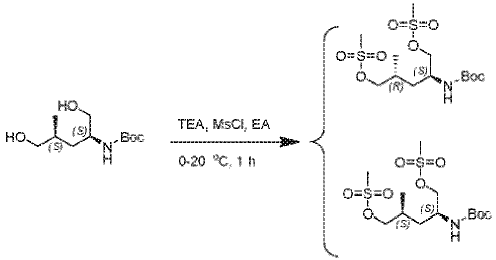

To a solution of tert-butyl N-[(1S)-4-hydroxy-1-(hydroxymethyl)-3-methyl-butyl]carbamate (11.00 g, 47.15 mmol, 1.00 eq) and TEA (19.08 g, 188.60 mmol, 26.14 mL, 4.00 eq) in EA (110.00 mL) was added dropwise MsCl (16.20 g, 141.45 mmol, 10.95 mL, 3.00 eq) at 0 °C, the resulting mixture was stirred at 20 °C for 1 h. The reaction mixture was poured into water 200 mL, and then extracted with EtOAc (50 mLx3). The combined organic layers were washed with brine (200 mL), dried over Na₂SO₄, filtered and concentrated under reduced pressure to give a residue to provide [(2R,4S)-4-(tert-butoxycarbonylamino)-2-methyl-5-methylsulfonyloxy-pentyl] methanesulfonate and [(2S,4S)-4-(tert-butoxycarbonylamino) -2-methyl-5-methylsulfonyloxy-pentyl] methanesulfonate (17.70 g, crude) as a yellow solid. (Note: isomer (2S, 4S) was detected at this step. It is minor and should be from step3)

### Step 6: Tert - butyl N-[(3S, 5R)-1-benzyl-5-methyl-3-piperidyl] carbamate

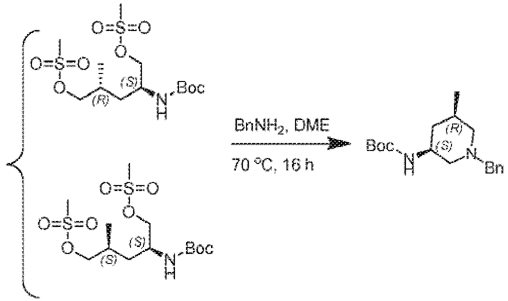

A mixture of [(2S,4S)-4-(tert-butoxycarbonylamino)-2-methyl-5-methylsulfonyloxy-pentyl] methanesulfonate; [(2R,4S)-4-(tert-butoxycarbonylamino) -2-methyl-5-methylsulfonyloxy-pentyl] methanesulfonate (13.70 g, 35.18 mmol, 1.00 eq), phenylmethanamine (12.06 g, 112.56 mmol, 12.31 mL, 3.20 eq) in DME (30.00 mL) was stirred at 70 °C for 16 h. The reaction mixture was diluted with water 300 mL and extracted with EtOAc (150 mLx3). The combined organic layers were washed with brine (500 mLx2), dried over Na₂SO₄, filtered and concentrated under reduced pressure to give a residue. The residue was purified by column chromatography (SiO₂, PE/EtOAc=40:1 to 20:1) to afford the title compound (850.00 mg, 1.95 mmol, 5.54% yield, 70% purity) as a white solid.

### Step 7: (3S, 5R)-1-benzyl-5-methyl-piperidin-3-amine

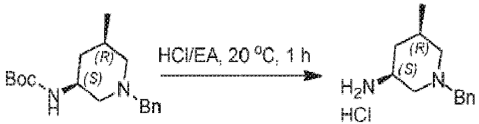

A mixture of tert-butyl N-[(3S, 5R)-1-benzyl-5-methyl-3-piperidyl] carbamate (1.15 g, 3.78 mmol, 1.00 eq) and HCl/EtOAc (4 M, 10.00 mL) was stirred at 20 °C for 1 h. The reaction mixture was concentrated under reduced pressure to give a residue to afford the title compound (860.00 mg, crude, HCl) as a white solid. It was used in the next step directly.

### Step 8: 4-[1-(benzenesulfonyl) indol-3-yl]-N- [(3S, SR) -1-benzyl-5-methyl-3- piperidyl]-5- chloro -pyrimidin-2-amine

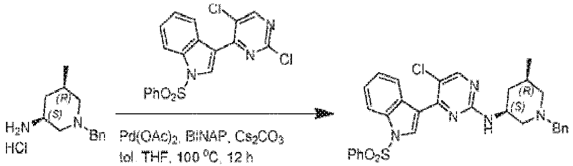

A mixture of (3S,5R)-1-benzyl-5-methyl-piperidin-3-amine (595.00 mg, 2.47 mmol, 1.20 eq, HCl), 1-(benzenesulfonyl)-3-(2,5-dichloropyrimidin-4-yl) indole (832.54 mg, 2.06 mmol, 1.00 eq), Cs₂CO₃ (1.34 g, 4.12 mmol, 2.00 eq), Pd(OAc)₂ (46.23 mg, 205.83 umol, 0.10 eq) and 2,2'-Bis(diphenylphosphino)-1,1'-Binaphthalene (128.23 mg, 205.83 umol, 0.10 eq) in toluene (6.00 mL) and THF (3.00 mL) was degassed and purged with N2 for 3 times, and then the mixture was stirred at 100 °C for 12 h under N₂ atmosphere. The reaction mixture was diluted with water 60 mL and extracted with EtOAc (30 mLx3). The combined organic layers were washed with brine (100 mLx2), dried over Na₂SO₄, filtered and concentrated under reduced pressure to give a residue. The residue was purified by column chromatography (SiO₂, PE/EtOAc =10:1 to 5:1) to afford the title compound (310 mg) as a yellow solid.

### Step 9: N-[(3S, 5R)-1-benzyl- 5-methyl-3- piperidyl]-5- chloro-4-(1H- indol-3- yl) pyrimidin- 2- amine

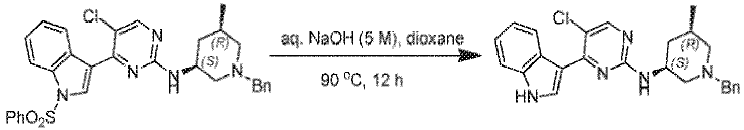

A mixture of 4-[1-(benzenesulfonyl)indol-3-yl]-N-[(3S, 5R)-1-benzyl-5-methyl- 3-piperidyl]-5-chloro-pyrimidin-2-amine (310.00 mg, 541.84 umol, 1.00 eq) and NaOH (5 M, 1.08 mL, 10.00 eq) in dioxane (5.00 mL) was stirred at 90 °C for 12 h. The reaction mixture was diluted with water 10 mL and extracted with EtOAc (5 mLx3). The combined organic layers were washed with brine (15 mLx2), dried over Na₂SO₄, filtered and concentrated under reduced pressure to give a residue to afford the title compound (260.00 mg, crude) as a yellow solid.

### Step 10: 5-chloro-4-(1H-indol-3-yl)-N-[(3S, 5R)-5-methyl-3-piperidyl] pyrimidin-2-amine

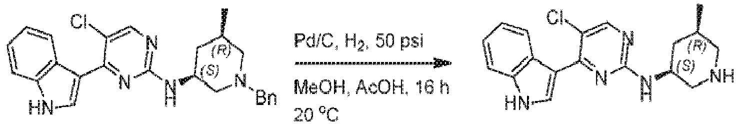

To a solution of N-[(3S,SR)-1-benzyl-5- methyl-3-piperidyl]-5-chloro- 4-(1H-indol-3-yl) pyrimidin-2-amine (160.00 mg, 370.40 umol, 1.00 eq) and AcOH (66.73 mg, 1.11 mmol, 63.55 uL, 3.00 eq) in MeOH (2.00 mL) was added Pd/C (10%, wet, 10 mg) under N₂. The suspension was degassed under vacuum and purged with H₂ three times. The mixture was stirred under H₂ (50 psi) at 20 °C for 16 h. The reaction mixture was filtered and the filtrate was concentrated to give desired compound (100 mg). The residue was purified by prep-HPLC (FA condition) to afford the title compound (10.30 mg, 25.76 umol, 6.95% yield, 97% purity, FA) as a yellow solid.

### Example 45. 4-[6-(1H-imidazol-2-yl)-1H-indol-3-yl]-N-[(3S)-3-piperidyl]-5-(trifluoromethyl) pyrimidin-2-amine (Compound 202).

### Step 1: Tert- butyl (3S)-3-[[4-[1-(benzenesulfonyl)-6-(4, 4, 5, 5-tetramethyl-1, 3, 2-dioxaborolan-2-yl) indol-3-yl]-5-(trifluoromethyl) pyrimidin-2-yl] amino] piperidine-1-carboxylate

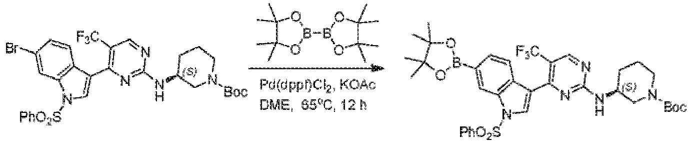

A mixture of tert-butyl (3S)-3-[[4-[1-(benzenesulfonyl)-6-bromo-indol-3-yl]-5-(trifluoromethyl)pyrimidin-2-yl]amino]piperidine-1-carboxylate (1.23 g, 1.81 mmol, 1.00 eq), 4,4,5,5-tetramethyl-2-(4,4,5,5-tetramethyl-1,3,2-dioxaborolan-2-yl)-1, 3, 2-dioxaborolane (689.45 mg, 2.72 mmol, 1.50 eq), Pd(dppf)Cl₂.CH₂Cl₂ (147.81 mg, 181.00 umol, 0.10 eq) and AcOK (355.27 mg, 3.62 mmol, 2.00 eq) in DME (10.00 mL) was degassed and purged with N₂ for 3 times, and then the mixture was stirred at 65°C for 12 h under N₂ atmosphere. The reaction mixture was diluted with water 100 mL and extracted with EtOAc (50 mLx3). The combined organic layers were washed with brine (100 mLx2), dried over Na₂SO₄, filtered and concentrated under reduced pressure to give a residue. The residue was purified by column chromatography (SiO₂, PE/EtOAc =10/1 to 3:1) to afford the title compound (700 mg) as a yellow solid (Note: combined purification with another batch. Scale: 100 mg).

### Step 2: 2-[(2-bromoimidazol-1-yl) methoxy] ethyl-trimethyl-silane

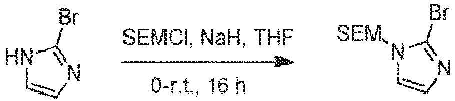

To a solution of 2-bromo-1H-imidazole (300.00 mg, 2.04 mmol, 1.00 eq) in THF (5.00 mL) was added batchwise NaH (106.00 mg, 2.65 mmol, 60% purity, 1.30 eq) at 0°C. After addition, the mixture was stirred at this temperature 0.5 h, and then 2- (chloromethoxy) ethyl-trimethyl-silane (442.14 mg, 2.65 mmol, 470.36 uL, 1.30 eq) was added dropwise at 0°C. The resulting mixture was stirred at 20°C for 15.5 h. It was quenched by addition water 50 mL, and extracted with EtOAc (20 mLx3), dried over Na₂SO₄, filtered and concentrated under reduced pressure to give a residue. The residue was purified by column chromatography (SiO₂, PE/EtOAc =20/1 to 5:1) to afford the title compound (500.00 mg, 1.80 mmol, 88.41% yield) as a colorless oil.

### Step 3: Tert- butyl (3S)-3-[[5-(trifluoromethyl)-4-[6-[1-(2-trimethylsilylethoxymethyl) imidazol-2-yl]-1H-indol-3-yl] pyrimidin-2-yl] amino] piperidine-1-carboxylate

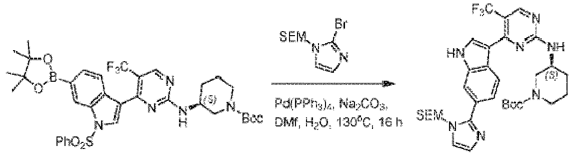

A mixture of tert-butyl (3S)-3-[[4-[1-(benzenesulfonyl)-6-(4, 4, 5, 5-tetramethyl-1, 3, 2-dioxaborolan-2-yl) indol-3-yl]-5- (trifluoromethyl) pyrimidin-2-yl]amino]piperidine-1-carboxylate (300.00 mg, 412.31 umol, 1.00 eq), 2-[(2-bromoimidazol-1-yl)methoxy]ethyl-trimethyl-silane (137.17 mg, 494.77 umol, 1.20 eq), Pd(PPh₃)₄ (47.65 mg, 41.23 umol, 0.10 eq), Na₂CO₃ (5 M, 164.92 uL, 2.00 eq) in DMF (3.00 mL) was degassed and purged with N₂ three times, and then the mixture was stirred at 130°C for 3 h under N₂ atmosphere. The reaction mixture was poured into water 50 mL, and then extracted with EtOAc (20 mLx3), dried over Na₂SO₄, filtered and concentrated under reduced pressure to give a residue. The residue was purified by column chromatography (SiO₂, PFJEtOAc =10/1 to 1:2) to afford the title compound (150 mg) as a yellow oil. (Note: combined purification with another batch. Scale: 100 mg).

### Step 4: 4-[6-(1H-imidazol-2-yl)-1H-indol-3-yl]-N-[(3S)-3-piperidyl]-5-(trifluoromethyl) pyrimidin-2-amine

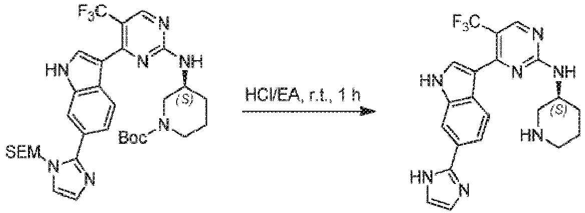

A mixture of tert-butyl (3S)-3-[[5-(trifluoromethyl)-4-[6-[1-(2-trimethylsilylethoxymethyl) imidazol-2-yl]-1H-indol-3-yl] pyrimidin-2-yl] amino] piperidine-1-carboxylate (200.00 mg, 304.04 umol, 1.00 eq) in HCl/EtOAc (4 M, 2.28 mL, 30.00 eq) was stirred at 20°C for 1 h. The reaction mixture was concentrated and adjusted pH to 8 by Sat. NaHCO₃, and then extracted with EtOAc (50 mLx3), dried over Na₂SO₄, filtered and concentrated under reduced pressure to give a residue The residue was purified by prep-HPLC (FA condition) to afford the title compound (5.30 mg, 10.52 umol, 3.46% yield, 94% purity, FA) as a white solid.

### Example 46. 5-choloro-4-[7-(1, 1-dioxo-1, 4-thiazinan-4-yl) -1H-indol-3-yl]-N-[(3S)-3-piperidyl] pyrimidin-2-amine (Compound 203).

### Step 1: (2R,4R)-4-hydroxypyrrolidine-2-carboxylic acid

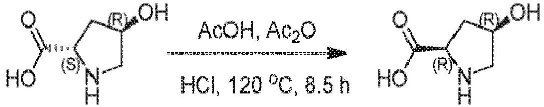

A mixture of AcOH (628.70 g, 10.47 mol) and Ac₂O (203.45 g, 1.99 mol) was heated to 50 °C, then (2S, 4R)-4-hydroxypyrrolidine-2-carboxylic acid (47.00 g, 358.42 mmol) was added in one portion. The mixture was heated to 120 °C and stirred for 5.5 h. After cooling to r.t., the solvent was removed. The residue was dissolved in HCl (650 mL), and then the mixture was heated to 120 °C and stirred for 3 h. Then activated charcoal (2.5 g) was added, the hot mixture was filtered immediately through a Celite layer and the cake was washed with hot water. The colorless solution was neutralized with triethylamine and evaporated to dryness. The crude product was refluxed in ethanol (2500 mL) and water was added carefully to the boiling mixture until the solid disappeared (but the solution remained still a little turbid). The solution was then left to stand overnight at -20 °C to give white crystals, which were filtered off and washed with cold ethanol afford the title compound (22.00 g, 44.5%) as a white solid.

### Step 2: methyl (2R, 4R)-4-hydroxypyrrolidine-2-carboxylate

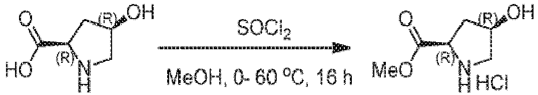

To a solution of (2R,4R)-4-hydroxypyrrolidine-2-carboxylic acid (22.00 g, 167.77 mmol) in MeOH (500 mL) was added SOCl₂ (23.95 g, 201.32 mmol) at 0 °C. The mixture was stirred at 60 °C for 16 h under N_{2.} The mixture was concentrated to afford the title compound (16.00 g, crude).

### Step 3: methyl (2R, 4R)-1-benzyl-4-hydroxy-pyrrolidine-2-carboxylate

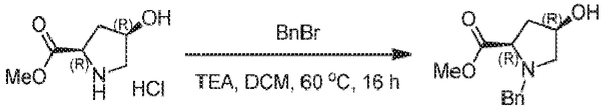

The mixture of methyl (2R,4R)-4-hydroxypyrrolidine-2-carboxylate (16.00 g, 88.10 mmol), BnBr (18.08 g, 105.72 mmol), TEA (26.74 g, 264.29 mmol) in DCM (200 mL) was degassed and purged with N2 for 3 times, and then it was stirred at 60 °C for 16 h under N₂ atmosphere. The residue was poured into water (500 mL). The aqueous phase was extracted with EtOAc (300 mLx3). The combined organic phase was washed with brine (500 mL), dried with anhydrous Na₂SO₄, filtered and concentrated in vacuum. The residue was purified by silica gel chromatography (SiO₂, PE/EtOAc=20/1, 10/1) to afford the title compound (18.00 g, 78.2%).

### Step 4: Methyl (2R, 4R)-1-benzyl-4-[tert-buryl(dimethyl)silyl]oxy-pyrrolidine-2-carboxylate.

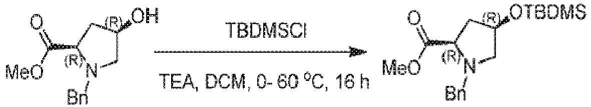

To a solution of methyl (2R,4R)-1-benzyl-4-hydroxy-pyrrolidine-2-carboxylate (18.00 g, 76.50 mmol) andimidazole (15.63 g, 229.50 mmol) in DCM (300 mL) was added TBDMSC1 (17.30 g, 114.75 mmol) at 0°C . The mixture was stirred at 60 °C for 16 h. The residue was poured into water (1000 mL). The aqueous phase was extracted with EtOAc (500 mLx3). The combined organic phase was washed with brine (1000 mL), dried with anhydrous Na₂SO₄, filtered and concentrated in vacuum. The residue was purified by silica gel chromatography (SiO₂, PE/EtOAc=10/1, 511) to afford the title compound (25.00 g, 79.46%).

### Step 5: [(2R,4R)-1-benzyl-4-[tert-butyl(dimethyl)silyl]oxy-pyrrolidin-2-yl]methanol

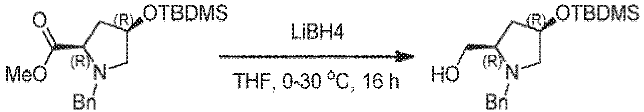

To a solution of methyl (2R, 4R)-1-benzyl-4-[tert-butyl(dimethyl)silyl]oxy- pyrrolidine-2-carboxylate (25.00 g, 71.52 mmol) in THF (300 mL) was added LiBH₄ (3.12 g, 143.04 mmol) at 0 °C under N₂. The mixture was stirred at 30 °C for 16 h. The residue was poured into water (1000 mL). The aqueous phase was extracted with EtOAc (500 mLx3). The combined organic phase was washed with brine (1000 mL), dried with anhydrous Na₂SO₄, filtered and concentrated. The residue was purified by column chromatography (SiO₂, PE /EtOAc=5/1 to 3:1) to afford the title compound (22.00 g, 86.1%).

### Step 6: [(3R,5S)-5-azido-1-benzyl-3-piperidyl]oxy-tert-butyl-dimethyl-silane

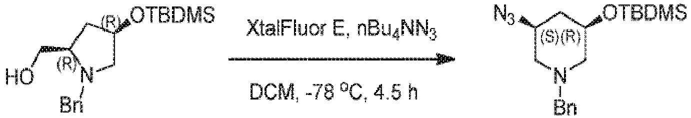

To a solution of [(2R, 4R)-1-benzyl-4-[tert-butyl (dimethyl)silyl]oxy-pyrrolidin-2-yl] methanol (5.00 g, 15.55 mmol) in CH₂Cl₂ (20 mL) was added drop-wise (difluoro-sulfanylidene)-diethylammonium;tetrafluoroborate (3.92 g, 17.11 mmol) and tetrabutylammonium;azide (4.42 g, 15.55 mmol) at -78 °C by10 min/hr. After addition, the mixture was stirred at this temperature for 4.5 h, and then concentrated. The residue was purified by column chromatography (SiO₂, PE/EtOAc=20/1) to afford the title compound (6 g, crude).

### Step 7: (3S,5R)-1-benzyl-5-[tert-butyl(dimethyl)silyl]oxy-piperidin-3-amine

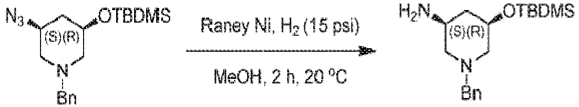

To a solution of [(3R, 5S)-5-azido-l-benzyl-3-piperidyl]oxy-tert-butyl-dimethyl-silane (6.00 g, 17.31 mmol) in MeOH (20 mL) was added Ra-Ni (1 g) under N₂. The suspension was degassed under vacuum and purged with H₂ several times. The mixture was stirred under H₂ (40 psi) at 20 °C for 2 h. The mixture was filtered and concentrated. The mixture was purified by prep-HPLC (TFA) to afford the title compound (1.50 g, 33.9%).

### Step 8: N-[(3S,5R)-1-benzyl-5-[tert-butyl(dimethyl)silyl]oxy-3-piperidyl]-4-(1H-indol-3-yl)-5-(trifluoromethyl)pyrimidin-2-amine

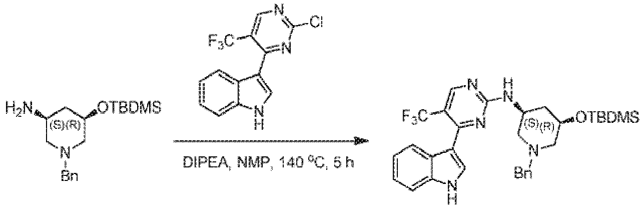

To a solution of (3S,5R)-1-benzyl-5-[tert-butyl(dimethyl)silyl]oxy-piperidin-3-amine (500 mg, 1.56 mmol) in NMP (5 mL) was added DIPEA (403 mg, 3.12 mmol) and 3-[2-chloro-5-(trifluoromethyl)pyrimidin-4-yl]-1H-indole (511 mg, 1.72 mmol). The mixture was stirred at 140 °C for 5 h. The residue was poured into water (100 mL). The aqueous phase was extracted with EtOAC (50 mLX3). The combined organic phase was washed with brine (100 mL), dried with anhydrous Na₂SO₄, filtered and concentrated. The residue was purified by column chromatography (SiO₂, PE/EtOAc =10/1 to 511) to afford the title compound (600 mg, 60.69%).

### Step 9: N-[(3S,5R)-5-[tert-butyl(dimethyl)silyl]oxy-3-piperidyl]-4-(1H-indol-3-yl)-5-(trifluoromethyl)pyrimidin-2-amine

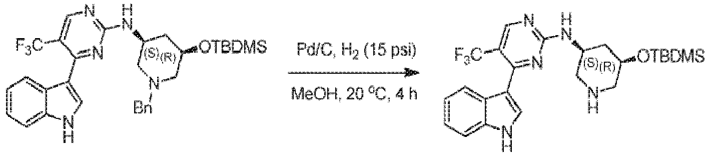

To a mixture of N-[(3S,5R)-1-benzyl-5-[tert-butyl(dimethyl)silyl]oxy-3-piperidyl]-4- (1H-indol-3-yl)-5-(trifluoromethyl)pyrimidin-2-amine (450 mg, 773.53 umol) and NH₃.H₂O (81 mg, 2.32 mmol) in MeOH (5 mL) was added Pd-C (10%, 0.05 g) under N₂. The suspension was degassed under vacuum and purged with H₂ several times. The mixture was stirred under H₂ (15 psi) at 20 °C for 4 h. The mixture was concentrated to afford the title compound (200 mg, crude).

### Step 10: (3R,5S)-5-[[4-(1H-indol-3-yl)-5-(trifluoromethyl)pyrimidin-2-yl]amino]piperidin-3-ol

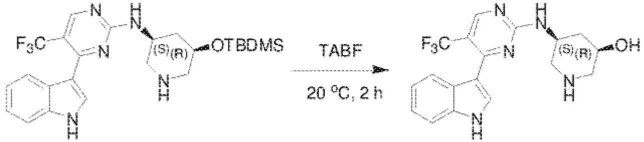

To a solution of N-[(3S, 5R)-5-[tert-butyl (dimethyl) silyl] oxy-3-piperidyl]-4- (1H-indol-3-yl)-5-(trifluoromethyl) pyrimidin-2-amine (200 mg, 406.82 umol) in THF (5 mL) was added TBAF (0.5 mL, 1 M). The mixture was stirred at 20 °C for 2 h. The mixture was concentrated. The residue was purified by prep-HPLC (FA) to afford the title compound (87 mg, 56.10%).

### Example 47. [(3S)-3-hydroxypyrrolidin-1-yl]-[3-[2-[[(3S)-3-piperidyl]amino]-5-(trifluoromethyl)pyrimidin-4-yl]-1H-indol-6-yl]methanone (Compound 204).

### Step 1: Methyl 3-[2-chloro-5-(trifluoromethyl) pyrimidin-4-yl]-1H-indole-6-carboxylate

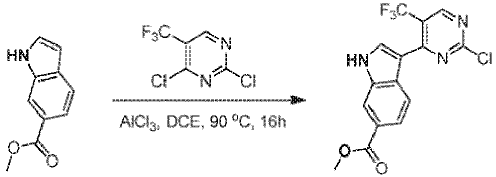

To a solution of 2, 4-dichloro -5- (trifluoromethyl) pyrimidine (24.77 g, 114.16 mmol, 2.00 eq) in DCE (200.00 mL) was added AlCl₃ (15.98 g, 119.87 mmol, 6.55 mL, 2.10 eq). The mixture was stirred at 90 °C for 30 min, and then methyl 1H-indole- 6-carboxylate (10.00 g, 57.08 mmol, 1.00 eq) was added at 90 °C. The resulting mixture was stirred at 90 °C for 15.5 h. The reaction mixture was filtered. The filtrate was diluted with water (200 mL) and extracted with DCM (80 mLx3). The combined organic layers were washed with brine (200 mLx2), dried over Na₂SO₄, filtered and concentrated under reduced pressure to give a residue. The residue was washed with MeOH (50 mL) and filtered to afford the title compound (4.00 g, 10.57 mmol, 18.52% yield, 94% purity) as a yellow solid.

### Step 2: Methyl 3-[2-[[(3S)-1-tert-butoxycarbonyl-3-piperidyl] amino]-5-(trifluoromethyl) pyrimidin-4-yl]-1H-indole-6-carboxylate

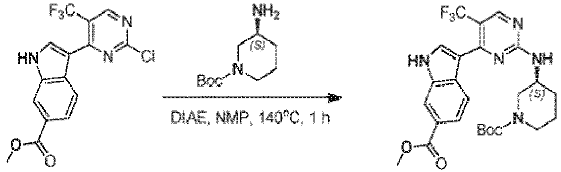

A mixture of methyl 3-[2-chloro-5-(trifluoromethyl)pyrimidin-4-yl]-1H-indole-6-carboxylate (2.50 g, 7.03 mmol, 1.00 eq), tert-butyl (3S)-3-aminopiperidine-1-carboxylate (1.83 g, 9.14 mmol, 1.30 eq), DIEA (4.54 g, 35.15 mmol, 6.14 mL, 5.00 eq) in NMP (10.00 mL) was stirred at 140 °C for 1 h. The reaction mixture was poured into water 200 mL, and then extracted with EtOAc (70 mLx3). The combined organic layers were washed with brine (300 mL), dried over Na₂SO₄, filtered and concentrated under reduced pressure to give a residue. The residue was purified by column chromatography (SiO₂, PE/EtOAc =5:1 to 1:1) to afford the title compound (2.20 g, 3.39 mmol, 48.19% yield, 80% purity) as a yellow solid.

### Step 3: 3-[2-[[(3S)-1-tert-butoxycarbonyl-3-piperidyl] amino]-5-(trifluoromethyl) pyrimidin-4yl]-1H-indole-6-carboxylic acid

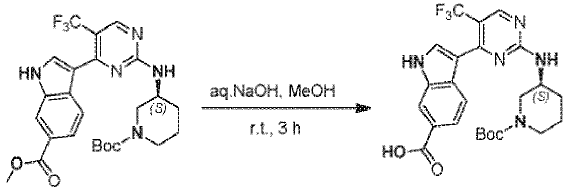

A mixture of methyl 3-[2-[[(3S)-1-tert-butoxycarbonyl-3-piperidyl] amino]-5-(trifluoromethyl) pyrimidin-4-yl]-1H-indole-6-carboxylate (2.20 g, 4.23 mmol, 1.00 eq), LiOH (5 M, 1.69 mL, 2.00 eq) in MeOH (20.00 mL) was stirred at 25 °C for 3 h. The reaction mixture was concentrated, and then diluted with water 100 mL and extracted with EtOAc (50 mLx3). The organic layers was removed, and the aqueous phase was adjusted pH to 1 by HCl (1M), extracted with EtOAc (50 mLx3). The combined organic layers were washed with brine (300 mL), dried over Na₂SO₄, filtered and concentrated under reduced pressure to afford the title compound (1.80 g, 3.20 mmol, 75.57% yield, 90% purity) as a yellow solid.

### Step 4: Tert- butyl (3S)-3-[[4-[6-[(3S)-3-hydroxypyrrolidine-1-carbonyl]-1H-indol-3-yl]-5-(trifluoromethyl) pyrimidin-2-yl] amino] piperidine-1-carboxylate

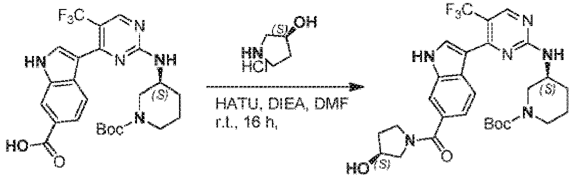

A mixture of 3-[2-[[(3S)-1-tert-butoxycarbonyl-3-piperidyl]amino]-5-(trifluoromethyl)pyrimidin-4-yl]-1H-indole-6-carboxylic acid (100.00 mg, 197.83 umol, 1.00 eq), (3S)-pyrrolidin-3-ol (24.45 mg, 197.83 umol, 22.64 uL, 1.00 eq, HCl), HATU (75.22 mg, 197.83 umol, 1.00 eq), DIEA (25.57 mg, 197.83 umol, 34.55 uL, 1.00 eq) in DMF (3.00 mL) was stirred at 20 °C for 16 h. The reaction mixture was concentrated. The residue was purified by prep-HPLC (TFA condition) and concentrated. The residue was treated with water (50 mL) and adjusted to pH =8 by NaHCO₃ and extracted with EtOAc (30 mLx3), dried over Na₂SO₄, concentrated to afford the title compound (80.00 mg, 125.31 umol, 63.34% yield, 90% purity) as a white solid

### Step 5: [(3S)-3-hydroxypyrrolidin-1-yl]-[3-[2-[[(3S)-3-piperidyl]amino]-5-(trifluoromethyl)pyrimidin-4-yl]-1H-indol-6-yl]methanone

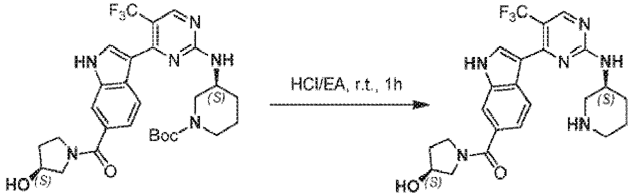

A mixture of tert-butyl (3S)-3-[[4-[6-[(3S)-3-hydroxypyrrolidine-1-carbonyl]-1H-indol-3-yl]-5-(trifluoromethyl)pyrimidin-2-yl] amino]piperidine-1-carboxylate (90.00 mg, 156.63 umol, 1.00 eq) in HCl/EtOAc (4 M, 1.17 mL, 30.00 eq) was stirred at 20 °C for 1 h. The reaction mixture was concentrated. The residue was purified by prep-HPLC (FA condition) to afford the title compound (14.10 mg, 26.55 umol, 16.95% yield, 98% purity, FA) as a white solid.

### Example 48. [(3R)-3-hydroxypyrrolidin-1-yl]-[3-[2-[[(3S)-3-piperidyl] amino]-5-(trifluoromethyl)pyrimidin-4-yl]-1H-indol-6-yl]methanone (Compound 205).

### Step 1: Tert- butyl (3S)-3-[[4-[6-[(3R)-3-hydroxypyrrolidine-1-carbonyl]-1H-indol-3-yl]-5-(trifluoromethyl)pyrimidin-2-yl] amino] piperidine-1-carboxylate

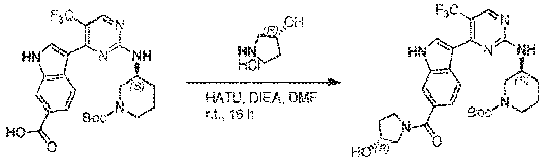

A mixture of 3-[2-[[(3S)-1-tert-butoxycarbonyl-3-piperidyl]amino]-5-(trifluoromethyl)pyrimidin-4-yl]-1H-indole-6-carboxylic acid (100.00 mg, 197.83 umol, 1.00 eq), (3R)-pyrrolidin-3-ol (24.45 mg, 197.83 umol, 22.64 uL, 1.00 eq, HCl), HATU (75.22 mg, 197.83 umol, 1.00 eq), DIEA (25.57 mg, 197.83 umol, 34.55 uL, 1.00 eq) in DMF (3.00 mL) was stirred at 20 °C for 16 h. The reaction mixture was poured into water 50 mL, and extracted with EtOAc (20 mLx3). The combined organic layers were washed with brine 100 mL, dried over Na₂SO₄, filtered and concentrated under reduced pressure to afford the title compound (200.00 mg, crude) as a yellow oil.

### Step 2: [(3R)-3-hydroxypyrrolidin-1-yl]-[3-[2-[[(3S)-3-piperidyl] amino]-5-(trifluoromethyl) pyrimidin-4-yl]-1H-indol-6-yl] methanone

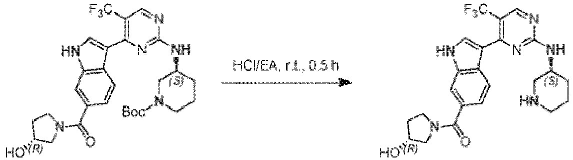

A mixture of tert-butyl (3S)-3-[[4-[6-[(3R)-3-hydroxypyrrolidine-1-carbonyl]-1H-indol-3-yl]-5-(trifluoromethyl) pyrimidin-2-yl] amino] piperidine-1-carboxylate (200.00 mg, 348.07 umol, 1.00 eq) in HCl/EtOAc (4 M, 4.35 mL, 50.00 eq) was stirred at 20 °C for 0.5 h. The reaction mixture was concentrated. The residue was purified by prep-HPLC (FA condition) to afford the title compound (10.50 mg, 19.77 umol, 5.68% yield, 98% purity, FA) as a yellow solid.

### Example 49. N-[(3S)-3-piperidyl]-4-(6-pyrrolldin-1-yl-1H-indol-3-yl)-5-(trifluoromethyl) pyrimidin-2-amine (Compound 213).

### Step 1: tert-butyl (3S)-3-[[4-(6-pyrrolidin-1-yl-1H-indol-3-yl)-5-(trifluoromethyl) pyrimidin-2-yl] amino] piperidine-1-carboxylate

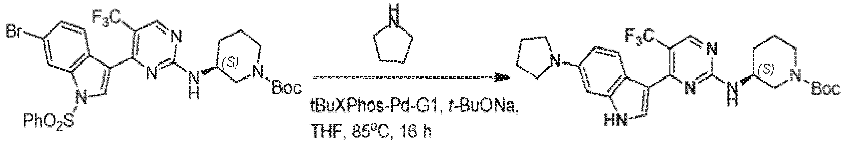

A mixture of tert-butyl (3S)-3-[[4-[1-(benzenesulfonyl)-6-bromo-indol-3-yl]-5-(trifluoromethyl) pyrimidin-2-yl] amino] piperidine-1-carboxylate (200.00 mg, 293.89 umol, 1.00 eq), pyrrolidine (313.52 mg, 4.41 mmol, 368.85 uL, 15.00 eq), t-BuONa (56.49 mg, 587.78 umol, 2.00 eq), [2-(2-aminoethyl)phenyl]-chloro-palladium;ditert-butyl-[2-(2,4,6-triisopropylphenyl) phenyl] phosphane (20.18 mg, 29.39 umol, 0.10 eq) in THF (5.00 mL) was degassed and purged with N₂ for 3 times, and then the mixture was stirred at 80°C for 16 h under N₂ atmosphere. The reaction mixture was poured into water 50 mL, and then extracted with EtOAc (30 mLx3). The combined organic layers were washed with brine (150 mL), dried over Na₂SO₄, filtered and concentrated under reduced pressure to give a residue. The residue was purified by column chromatography (SiO₂, PE/EtOAc =10:1 to 2:1) to afford the title compound (160 mg) as a yellow solid. (Note: combined purification with another batch. SM scale: 100 mg,)

### Step 2: N-[(3S)-3-piperidyl]-4-(6-pyrrolidin-1-yl-1H-indol-3-yl)-5-(trifluoromethyl) pyrimidin-2-amine

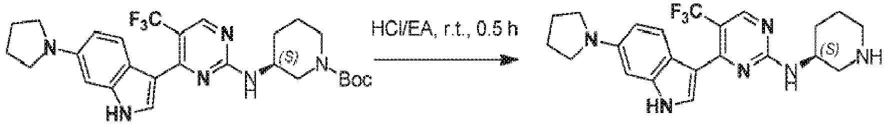

A mixture of tert-butyl (3S)-3-[[4-(6-pyrrolidin-1-yl-1H-indol-3-yl)-5-(trifluoromethyl) pyrimidin-2-yl] amino] piperidine-1-carboxylate (160.00 mg, 301.55 umol, 1.00 eq) in HCl/EtOAc (4 M, 2.26 mL, 30.00 eq) was stirred at 20°C for 0.5 h. It was concentrated, and the residue was purified by prep-HPLC (FA condition) to afford the title compound (51.50 mg, 106.89 umol, 35.45% yield, 98.9% purity, FA) as a yellow solid.

### Example 50. 2-methyl-1-[4-[3-[2-[[(3S)-3-piperidyl] amino]-5-(trifluoromethyl) pyrimidin-4-yl]-1H-indol-yl] pyrazol-1-yl] propan-2-ol (Compound 214).

### Step 1: 2-methyl-1-[4-(4,4,5,5-tetramethyl-1,3,2-dioxaborolan-2-yl)pyrazol-1-yl]propan-2-ol

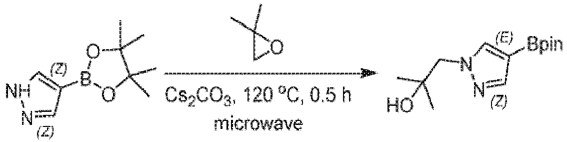

To a solution of 4-(4, 4, 5, 5-tetramethyl-1, 3, 2-dioxaborolan-2-yl)-1H-pyrazole (800 mg, 4.12 mmol) in 2,2-dimethyloxirane (3.86 g, 53.60 mmol) was added Cs₂CO₃ (2.01 g, 6.18 mmol) at 20 °C. The mixture was heated at 120° C for 30 min using microwave irradiation under N₂. The reaction mixture was filtered and washed by DCM. The filtrate was concentrated under reduced pressure to afford the title compound (970 mg, 79.62%) as a white solid.

### Step 2: tert-butyl (3S)-3-[[4-[1-(benzenesulfonyl)-6-[1-(2-hydroxy-2-methylpropyl)pyrazol -4-yl]indol-3-yl]-5-(trifluoromethyl)pyrimidin-2-yl]amino]piperidine-1-carboxylate

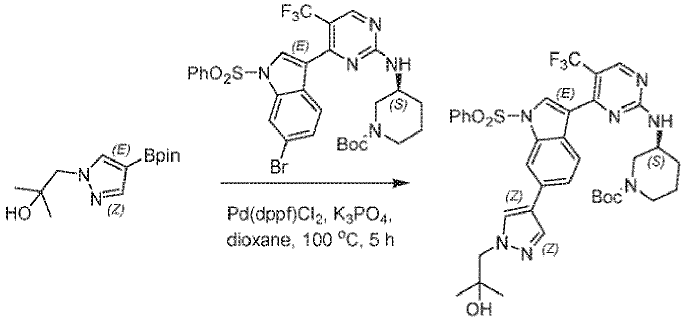

2-methyl-1-[4-(4,4,5,5-tetramethyl-1,3,2-dioxaborolan-2-yl)pyrazol-1-yl]propan-2-ol (117 mg, 440.82 umol) and tert-butyl (3S)-3-[[4-[1-(benzenesulfonyl)-6-bromo- indol-3-yl]-5-(trifluoromethyl)pyrimidin-2-yl]amino]piperidine-1-carboxylate (200 mg, 293.88 umol) were dissolved in dioxane (6 mL), then Pd(dppf)Cl₂ (21 mg, 29.39 umol) and K₃PO₄ (124 mg, 587.76 umol) were added into the mixture at 20 °C. The suspension was degassed under vacuum and purged with N₂ three times. The mixture was stirred under N₂ at 100°C for 5 h. The reaction mixture was filtered and concentrated under reduced pressure to give a residue. The residue was purified by column chromatography (SiO₂, PE/EtOAc =10/1-5/1) to afford the title compound (200 mg, 73.59%) as a yellow solid.

### Step 3: tert-butyl (3S)-3-[[4-[6-[1-(2-hydroxy-2-methyl-propyl)pyrazol-4-yl]-1H-indol-3-yl] -5-(trifluoromethyl)pyrimidin-2-yl]amino]piperidine-1-carboxylate

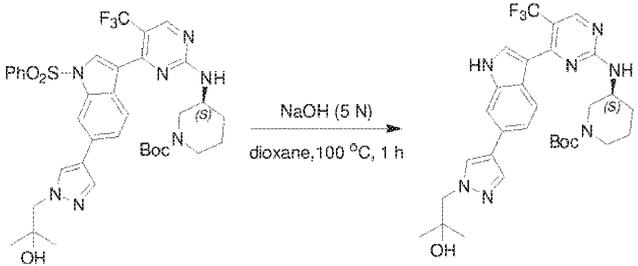

To a solution of tert-butyl (3S)-3-[[4-[1-(benzenesulfonyl)-6-[1-(2-hydroxy-2- methylpropyl)pyrazol-4-yl]indol-3-yl]-5-(trifluoromethyl)pyrimidin-2-yl] amino]piperidine-1-carboxylate (150 mg, 202.75 umol) in dioxane (5 mL) was added a solution of NaOH (40 mg, 1.01 mmol) in H₂O (1 mL) at 20 °C. The mixture was heated to 100 °C and stirred for 1 h. The reaction mixture was poured into water (50 mL) and extracted with EtOAc (50 mLx3). The combined organic layers were dried over Na₂SO₄, filtered and concentrated under reduced pressure to afford the title compound (120 mg, crude) as a yellow oil.

### Step 4: 2-methyl-1-[4-[3-[2-[[(3S)-3-piperidyl]amino]-5-(trifluoromethyl) pyrimidin-4-yl]-1H-indol-6-yl]pyrazol-1-yl]propan-2-ol

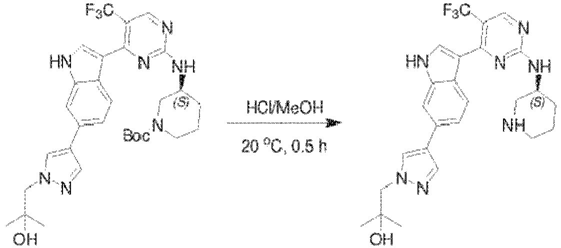

To a solution of tert-butyl (3S)-3-[[4-[6-[1-(2-hydroxy-2-methyl-propyl)pyrazol-4-yl] - 1H-indol-3-yl]-5-(trifluoromethyl)pyrimidin-2-yl]amino]piperidine-1-carboxylate (100 mg, 166.76 umol) in MeOH (2 mL) was added HCl/MeOH (4 M, 20 mL). The mixture was stirred at 20 °C for 0.5 h. Then the mixture was concentrated under reduced pressure to give a residue. The residue was purified by prep-HPLC (FA) to afford the title compound (51.20 mg, 55.15%) as a light yellow solid.

### Example 51. 4-[6-(methylsulfonylmethyl)-1H-indol-3-yl] -N-[(3S)-3-piperidyl] -5-(trifluoromethyl) pyrimidin-2- amine (Compound 219).

### Step 1: Methyl 3-[2-chloro-5-(trifluoromethyl)pyrimidin-4-yl] -1-(2-trimethylsilylethoxymethyl) indole-6-carboxylate

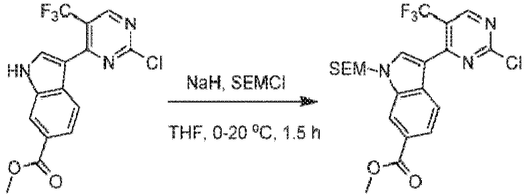

To a solution of methyl 3-[2-chloro-5- (trifluoromethyl)pyrimidin-4-yl] -1H-indole-6-carboxylate (1.00 g, 2.81 mmol, 1.00 eq) in THF (10.00 mL) was added NaH (168.68 mg, 4.22 mmol, 60% purity, 1.50 eq) at 0 °C. After addition, the mixture was stirred at this temperature for 30 min, and then 2-(chloromethoxy) ethyl-trimethyl-silane (703.06 mg, 4.22 mmol, 747.94 uL, 1.50 eq) was added dropwise at 0 °C. The resulting mixture was stirred at 20°C for 1 h. The reaction mixture was quenched with water 20 mL and extracted with EtOAc (10 mLx3). The combined organic layers were washed with brine (30 mLx2), dried over Na₂SO₄, filtered and concentrated under reduced pressure to give a residue. The residue was purified by column chromatography (SiO2, PE/EtOAc=20:1 to 10:1) to afford the title compound (860.00 mg, 1.63 mmol, 57.94% yield, 92% purity) as a white solid.

### Step 2: Methyl 3-[2-[[(3S)-1-tert-butoxycarbonyl-3-piperidyl] amino]-5-(trifluoromethyl) pyrimidin-4-yl]-1-(2-trimethylsilylethoxymethyl) indole-6-carboxylate

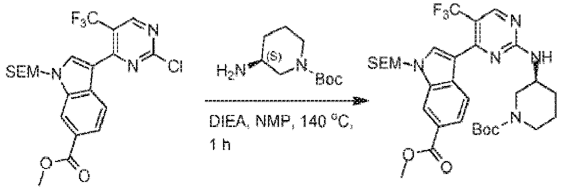

A mixture of methyl 3-[2-chloro-5-(trifluoromethyl)pyrimidin-4- yl]- 1-(2-trimethylsilylethoxymethyl) indole- 6-carboxylate (860.00 mg, 1.77 mmol, 1.00 eq), tert-butyl (3S)-3-aminopiperidine-1-carboxylate (531.65 mg, 2.66 mmol, 1.50 eq) and DIEA (686.15 mg, 5.31 mmol, 927.23 uL, 3.00 eq) in NMP (8.00 mL) was stirred at 140 °C for 1 h. The reaction mixture was diluted with water 50 mL and extracted with EtOAc (30 mLx3). The combined organic layers were washed with brine (100 mLx2), dried over Na2SO4, filtered and concentrated under reduced pressure to give a residue. The residue was purified by column chromatography (SiO₂, PE/EtOAc =10:1 to 3:1) to afford the title compound (1.07 g, 1.43 mmol, 80.94% yield) as a yellow oil.

### Step 3: Tert-butyl (3S)-3-[[4-[6-(hydroxymethyl)-1 - (2-trimethylsilylethoxymethyl)indol-3-yl] -5-(trifluoromethyl)pyrimidin-2-yl]amino]piperidine-1-carboxylate

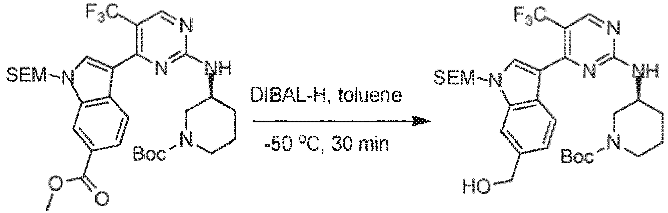

To a solution of methyl 3-[2-[[(3S)-1-tert-butoxycarbonyl- 3-piperidyllaminol- 5-(trifluoromethyl)pyrimidin-4-yl]-1-(2-trimethylsilylethoxymethyl)indole-6-carboxylate (1.07 g, 1.65 mmol, 1.00 eq) in toluene (10.00 mL) was added dropwise DIBAL-H (1 M, 3.96 mL, 2.40 eq) at -50 °C. The mixture was stirred at -50 °C for 30 min. The reaction mixture was quenched by addition MeOH (2 mL) and H₂O (2 mL) at 0 °C and filtered, the filtrate was concentrated under reduced pressure to give a residue. The residue was purified by column chromatography (SiO₂, PE/EtOAc =5:1 to 1:1) to afford the title compound (600.00 mg, 878.15 umol, 53.22% yield, 91% purity) as a yellow oil.

### Step 4: Tert-butyl (3S)-3-f [4-[6-(chloromethyl)-1 -(2-trimethylsilylethoxymethyl)indol-3-yl] -5-(trifluoromethyl)pyrimidin-2-yl]amino]piperidine-1-carboxylate and tert-butyl (3S)-3 -[[4-[6-(methylsulfonyloxymethyl)- 1-(2-trimethylsilylethoxymethyl) indol -3-yl]- 5-(trifluoromethyl) pyrimidin-2-yl]amino]piperidine-1-carboxylate

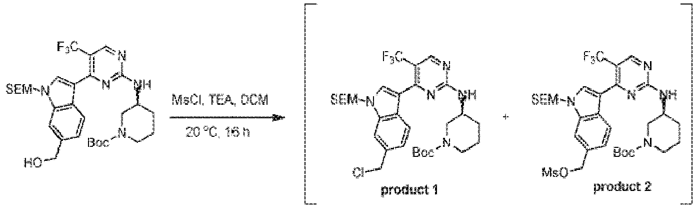

To a solution of tert-butyl (3S)-3-[[4-[6-(hydroxymethyl)-1- (2-trimethylsilylethoxymethyl)indol-3 -yl]-5 -(trifluoromethyl)pyrimidin-2-yl]amino] piperidine - 1-carboxylate (600.00 mg, 965.00 umol, 1.00 eq) in DCM (6.00 mL) was added TEA (146.47 mg, 1.45 mmol, 200.64 uL, 1.50 eq) and methanesulfonyl chloride (331.62 mg, 2.90 mmol, 224.07 uL, 3.00 eq). The mixture was stirred at 20 °C for 16 h. The reaction mixture was diluted with water 20 mL and extracted with DCM (10 mLx3). The combined organic layers were washed with brine (30 mLx2), dried over Na₂SO₄, filtered and concentrated under reduced pressure to afford 740 mg of the title compounds as a crude mixture. It was used in the next step directly without further purification.

### Step 5: Tert-butyl (3S)-3-[[4-[6-(methylsulfonylmethyl) - 1-(2-trimethylsilylethoxymethyl) indol-3-yl]-5- (trifluoromethyl)pyrimidin-2-yl] amino] piperidine-1-carboxylate

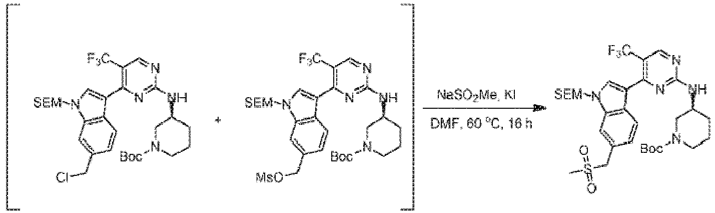

To a solution of tert-butyl (3S)-3-[[4-[6-(chloromethyl)-1- (2-trimethylsilylethoxymethyl)indol-3-yl]-5-(trifluoromethyl)pyrimidin-2-yl]amino] piperidine-1-carboxylate and tert-butyl (3S)-3-[[4- [6-(methylsulfonyloxymethyl) -1-(2-trimethylsilylethoxymethyl)indol-3-yl] -5 - (trifluoromethyl)pyrimidin-2-yl] amino] piperidine-1- carboxylate (340.00 mg, 531.08 umol, 1.00 eq) in DMF (5.00 mL) was added methylsulfonylsodium (108.44 mg, 1.06 mmol, 2.00 eq) and KI (105.79 mg, 637.29 umol, 1.20 eq). The mixture was stirred at 60 °C for 16 h. The reaction mixture was diluted with water 50 mL and extracted with ethyl acetate (30 mLx3). The combined organic layers were washed with brine (100 mLx2), dried over Na₂SO₄, filtered and concentrated under reduced pressure to give a residue. The residue was purified by column chromatography (SiO₂, PE/EtOAc =10:1 to 1:1) to afford the title compound (210.00 mg, 251.81 umol, 47.41% yield, 82% purity) as a yellow solid.

### Step 6: 4-[6-(methylsulfonylmethyl)-1H-indol-3-yl]-N-[(3S)-3-piperidyl]-5-(trifluoromethyl) pyrimidin-2-amine

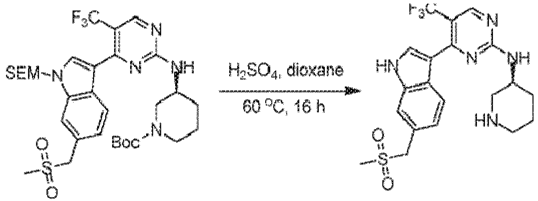

To a solution of tert-butyl (3S)-3- [[4-[6-(methylsulfonylmethyl)-1- (2-trimethylsilylethoxymethyl) indol-3-yl] - 5-(trifluoromethyl) pyrimidin-2-yl] amino] piperidine-1-carboxylate (210.00 mg, 307.08 umol, 1.00 eq) in dioxane (5.00 mL) was added H₂SO₄ (301.18 mg, 3.07 mmol, 163.69 uL, 10.00 eq). The mixture was stirred at 60 °C for 16 h. The reaction mixture was poured into water (20 mL) at 0 °C, then the mixture was adjusted pH to 8 by aqueous NaOH, and extracted with dichloromethane (10 mLx3), The combined organic layers were washed with brine (30 mLx2), dried over Na₂SO₄, filtered and concentrated under reduced pressure to give a residue (200 mg). The residue was purified by prep-HPLC (FA condition) to afford the title compound (8.80 mg, 16.91 umol, 5.51% yield, 96% purity, FA) as a white solid (Note: combined purification with another batch. SM scale: 50 mg).

### Example 52. [(3S)-3-hydroxy-3-methyl-pyrrolidin-1-yl]-[3-[2-[[(3S)-3-piperidyl]amino]-5-(trifluoromethyl)pyrimidin-4-yl]-1Hindol-6-yl]methanone (Compound 220) and [(3R)-3-hydroxy-3-methyl-pyrrolidin-1-yl]-[3-[2-[[(3S)-3-piperidyl]amino]-5-(trifluoromethyl)pyrimidin-4-yl]-1Hindol-6-yl]methanone (Compound 221).

### Step 1: Tert-butyl (3S)-3-[[4-[6-(3-hydroxy-3-methyl-pyrrolidine-1-carbonyl)-1H-indol-3-yl]-5-(trifluoromethyl) pyrimidin-2-yl] amino] piperidine-1-carboxylate

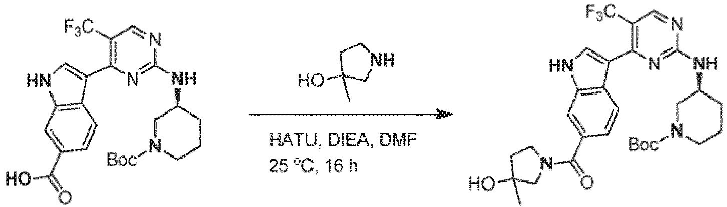

A mixture of 3-[2-[[(3S)-1-tert-butoxycarbonyl-3-piperidyl]amino]-5-(trifluoromethyl)pyrimidin-4-yl]-1H-indole-6-carboxylic acid (300.00 mg, 593.48 umol, 1.00 eq), 3-methylpyrrolidin-3-ol (60.03 mg, 593.48 umol, 1.00 eq), HATU (225.66 mg, 593.48 umol, 1.00 eq), DIEA (230.10 mg, 1.78 mmol, 310.95 uL, 3.00 eq) in DMF (5.00 mL) was degassed and purged with N₂ for three times, and then the mixture was stirred at 25 °C for 16 h under N₂ atmosphere. The reaction mixture was poured into water 50 mL, and extracted with EtOAc (20 mLx3). The combined organic layers were washed with brine 100 mL, dried over Na₂SO₄, filtered and concentrated under reduced pressure to afford the title compound (500.00 mg, crude) as a yellow oil. It was used for next step directly.

### Step 2: (3-hydroxy-3-methyl-pyrrolidin-1-yl)- [3-[2-[[(3S)-3-piperidyl] amino]-5-(trifluoromethyl) pyrimidin-4-yl]-1H-indol-6-yl] methanone

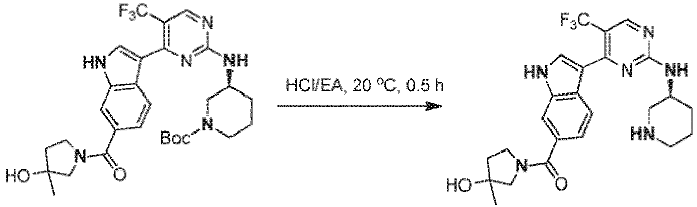

A mixture of tert-butyl-(3S)-3 A mixture of tert-butyl (3S)-3-[[4-[6-(3-hydroxy-3-methyl-pyrrolidine-1-carbonyl)-1H-indol-3-yl]-5-(trifluoromethyl) pyrimidin-2-yl] amino] piperidine-1-carboxylate (500.00 mg, 849.44 umol, 1.00 eq) in HCl/EtOAc (4 M, 6.37 mL, 30.00 eq) was stirred at 20 °C for 0.5 h. It was concentrated and the residue was purified by prep-HPLC (TFA condition) to afford the title compound (80.00 mg, 163.77 umol, 19.28% yield) as a yellow solid.

### Step 3: [(3S)-3-hydroxy-3-methyl-pyrrolidin-1-yl]-[3-[2-[[(3S)-3-piperidyl]amino]-5-(trifluoromethyl)pyrimidin-4-yl]-1Hindol-6-yl]methanone&[(3R)-3-hydroxy-3-methylpyrrolidin-1-yl]-[3-[2-[[(3S)-3-piperidyl]amino]-5-(trifluoromethyl)pyrimidin-4-yl]-1Hindol-6-yl]methanone

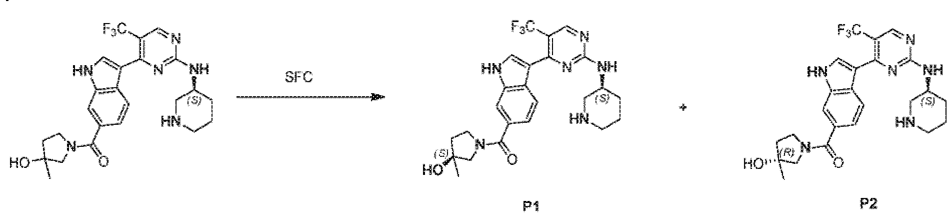

(3-hydroxy-3-methyl-pyrrolidin-1-yl)-[3-[2-[[(3S)-3-piperidyl] amino]-5-(trifluoromethyl) pyrimidin-4-yl]-1H-indol-6-yl]methanone (80 mg) was separated by SFC. P1 (20 mg) was further purified by prep-HPLC (TFA condition) and concentrated. The residue was treated with Sat. NaHCO₃ and extracted with DCM (2x10 mL), dried over Na₂SO₄ and concentrated. It was then repurified by prep-HPLC (FA condition) and concentrated to give [(3S)-3-hydroxy-3-methyl-pyrrolidin-1-yl]-[3-[2-[[(3S)-3-piperidyl] amino]-5-(trifluoromethyl) pyrimidin-4-yl]-1Hindol-6-yl] methanone (3.00 mg, 6.02 umol, 3.68% yield, 98% purity) as a white solid (P1, tentatively assigned) and [(3R)-3-hydroxy-3-methyl-pyrrolidin-1-yl]- [3-[2-[[(3S)-3-piperidyl] amino]-5-(trifluoromethyl) pyrimidin-4-yl]-1Hindol-6-yl] methanone (18.00 mg, 35.37 umol, 21.60% yield, 96% purity) (P2, tentatively assigned).

### Example 53. N-[4-(1H-indol-3-yl)-5-(trifluoromethyl)pyrimidin-2-yl]-3-azabicyclo [2.2.1]heptan-7-amine (Compound 222).

### Step 1: 5-[(1S)-1-phenylethyl]-5-azabicyclo [2.2.1]hept-2-ene

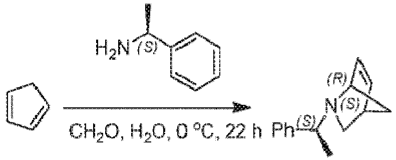

To a solution of (1S)-1-phenylethanamine (5.00 g, 41.26 mmol) in H₂O (56 mL) was added AcOH (2.48 g, 41.26 mmol) in H₂O (29 mL) at 0 °C followed by the addition of cyclopenta-1,3-diene (5.45 g, 82.52 mmol) and formaldehyde (5.02 g, 61.89 mmol) at the sample temperature. The mixture was stirred at 0 °C for 22 h. The mixture was poured into water (300 mL). The aqueous phase was extracted with EA (150 mLx3). The combined organic phase was washed with brine (150 mL), dried with anhydrous Na₂SO₄, filtered and concentrated to afford the title compound (5.20 g, 37.94%) as a yellow oil.

### Step 2: (12S)-14-BLAH-12-bromo-14-[(1S)-1-phenylethyl]-14azatricycloheptane

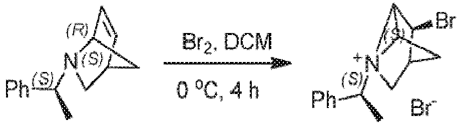

To a solution of 5-[(1S)-1-phenylethyl]-5-azabicyclo[2.2.1]hept-2-ene (1.00 g, 5.02 mmol) in DCM (20 mL) was added a solution of Br₂ (882.47 mg, 5.52 mmol) at 0 °C. The mixture was stirred at 0 °C for 4 h. The mixture was concentrated. The residue was washed with DCM/PE=1/50 to afford the title compound (1.60 g, crude) as a light yellow solid.

### Step 3: [3-[(1S)-1-phenylethyl]-3-azabicyclo [2.2.1]heptan-7-yl]isoindoline-1,3-dione

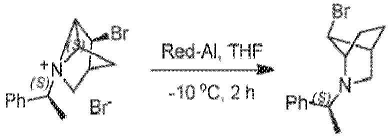

To a solution of (12S)-14-BLAH-12-bromo-14-[(1S)-1-phenylethyl]- 14azatricycloheptane (800.00 mg, 2.23 mmol) in THF (20.00 mL) was added sodium;bis(2-methoxyethoxy)alumanuide (1.28 g, 4.46 mmol) at -10°C . The mixture was stirred at -10 °C for 2 hour under N₂. The mixture was poured into water (100 mL). The aqueous phase was extracted with EtOAc (50 mLx3). The combined organic phase was washed with brine (100 mL), dried over anhydrous Na₂SO₄, filtered and concentrated. The product was purified by prep-TLC (PE/EtOAc= 311) to afford the title compound (300 mg, 20.40%).

### Step 4: 2-[3-[(1S)-1-phenylethyl]-3-azabicyclo[2.2.1] heptan-7-yl]isoindoline-1,3-dione

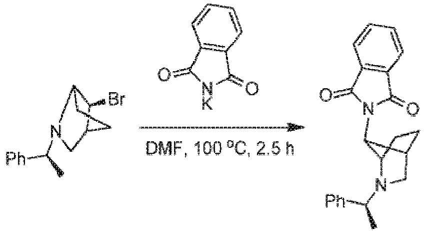

A mixture of (7S)-7-bromo-3-[(1S)-1-phenylethyl]-3-azabicyclo[2.2.1]heptane (250 mg, 892.22 umol) and (1,3-dioxoisoindolin-2-yl)potassium (181.78 mg, 981.44 umol) in DMF (5.00 mL) was degassed and purged with N₂ for 3 times, and then the mixture was stirred at 100 °C for 2.5 h under N₂ atmosphere. The mixture was poured into water (100 mL). The mixture was extracted with EtOAc (50 mLx3), and then the combined organic phase was washed with brine (100 mL), dried with anhydrous Na₂SO₄, filtered and concentrated in vacuum to afford the title compound (350.00 mg, crude).

### Step 5: 3-[(1S)-1-phenylethyl]-3-azabicyclo[2.2.1] heptan-7-amine

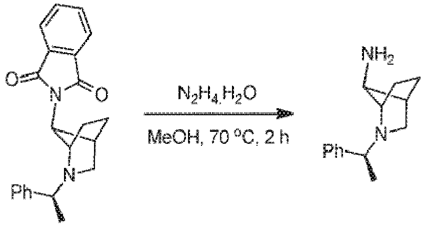

To a solution of 2-[3-[(1S)-1-phenylethyl]-3-azabicyclo[2.2.1]heptan-7-yl]isoindoline -1,3-dione (1.40 g, 4.04 mmol) in MeOH (15.00 mL) was added N₂H₄.H₂O (412.74 mg, 8.08 mmol). The mixture was stirred at 70 °C for 2 h. The mixture was filtered and concentrated to afford the title compound (600.00 mg, 65.22%)

### Step 6: N-[4-(1H-indol-3-yl)-5-(trifluoromethyl) pyrimidin-2-yl]-3-[(1S)-1-phenylethyl]-3-azabicyclo[2.2.1]heptan-7-amine

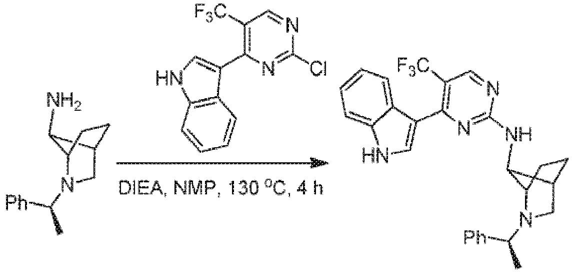

To a solution of 3-[(1S)-1-phenylethyl]-3-azabicyclo[22.1]heptan-7-amine (200.00 mg, 924.56 umol) in NMP (5.00 mL) was added DIEA (358.47 mg, 2.77 mmol). The mixture was stirred at 130 °C for 4 h. The mixture was poured into water (100 mL). The mixture was extracted with EtOAc (50 mLx3), and then the combined organic phase was washed with brine (100 mL), dried with anhydrous Na₂SO₄, filtered and concentrated in vacuum to afford the title compound (300.00 mg, crude).

### Step 7: N-[4-(1H-indol-3-yl)-5-(trifluoromethyl)pyrimidin-2-yl]-3-azabicyclo[2.2.1] heptan-7-amine

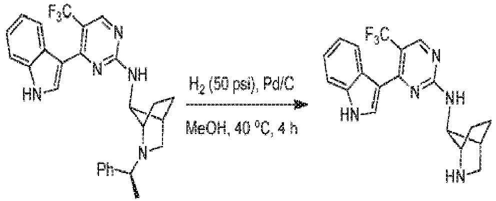

To a solution of N-[4-(1H-indol-3-yl)-5-(trifluoromethyl)pyrimidin-2-yl]-3-[(1S)-1-phenylethyl]-3-azabicyclo[2.2.1]heptan-7-amine (150.00 mg, 314.12 umol) and in MeOH (5.00 mL) was added Pd-C (10%, 50 mg) under N₂. The suspension was degassed under vacuum and purged with H₂ several times. The mixture was stirred under H₂ (50 psi) at 40 °C for 4 h. The mixture was concentrated. The residue was purified by prep-HPLC (FA) to afford the title compound (50.00 mg, 18.43%).

### Example 54. N-[(3S)-3-piperidyl]-4-[6-(3-pyridyl)-1H-indol-3-yl]-5-(trifluoromethyl) pyrimidin-2-amine (Compound 223).

### Step 1: tert-butyl (3S)-3-[[4-[1-(benzenesulfonyl)-6 -(3-pyridyl)indol-3-yl]-5-(trifluoromethyl)pyrimidin-2-yl]amino]piperidine-1-carboxylate

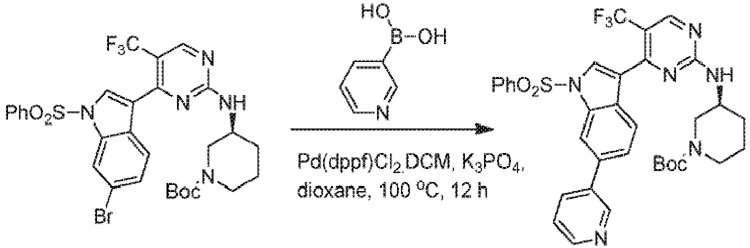

Tert-butyl (3S)-3-[[4-[1-(benzenesulfonyl)-6-bromo-indol-3-yl]-5-(trifluoromethyl) pyrimidin-2-yl]amino]piperidine-1-carboxylate (100 mg, 146.94 umol) and 3-pyridylboronic acid (18 mg, 146.94 umol) were dissolved in dioxane (5 mL) and H₂O (1 mL). Pd(dppf)Cl₂.CH₂Cl₂ (12 mg, 14.69 umol) and K₃PO₄ (93 mg, 440.83 umol) were added into the mixture. The mixture was heated to 100 °C and stirred for 12 h under N₂. The reaction mixture was filtered and concentrated under reduced pressure to give a residue. The residue was purified by column chromatography (SiO₂, PE/EtOAc=10/1-5/1) to afford the title compound (80 mg, 72.19%) as a yellow solid

### Step 2: tert-butyl (3S)-3-[[4-[6-(3-pyridyl)-1H-indol-3-yl]-5-(trifluoromethyl)pyrimidin-2-yl]amino]piperidine-1-carboxylate

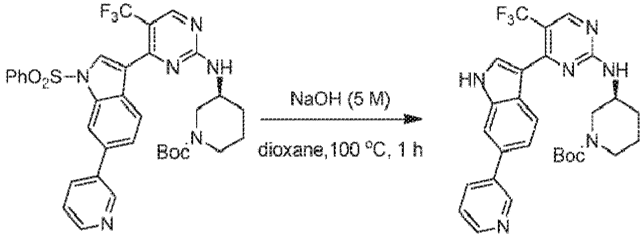

To a solution of tert-butyl (3S)-3-[[4-[1-(benzenesulfonyl)-b-(3pyridyl)indol-3-yl]-5-(trifluoromethyl)pyrimidin-2-yl]amino]piperidine-1-carboxylate (80 mg, 117.87 umol) in dioxane (2 mL) was added NaOH (23 mg, 589.34 umol) and H₂O (500 uL) at 20 °C. The mixture was heated to 100 °C and stirred for 1 h under N₂. The reaction mixture was poured into water (20 mL) and extracted with EtOAc (20 mLx3). The combined organic layers were dried over Na₂SO₄, filtered and concentrated under reduced pressure to afford the title compound (60 mg, crude) as a yellow oil.

### Step 3: N-[(3S)-3-piperidyl]-4-[6-(3-pyridyl)-1H-indol-3-yl]-5-(trifluoromethyl) pyrimidin-2-amine

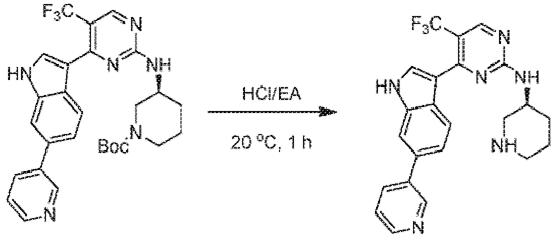

A solution of tert-butyl (3*S*)-3-[[4-[6-(3-pyridyl)-1H-indol-3-yl]-5- (trifluoromethyl)pyrimidin-2-yl]amino]piperidine-1-carboxylate (60 mg, 111.41 umol) in HCl/EtOAc (4 M, 10 mL) was stirred for 1 h at 20 °C. Then the mixture was concentrated under reduced pressure to give a residue. The residue was purified by prep-HPLC (FA) to afford the title compound (11.10 mg, 20.06%) as a white solid.

### Example 55. N-[(3S)-3-piperidyl]-4-[6-(4-pyridyl)-1H-indol-3-yl]-5-(trifluoromethyl) pyrimidin-2-amine (Compound 224).

### Step 1: 6-bromo-3-[2-chloro-5-(trifluoromethyl)pyrimidin-4-yl]-1H-indole

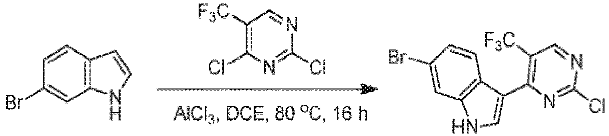

To a solution of 2, 4-dichloro-5-(trifluoromethyl) pyrimidine (16.60 g, 76.52 mmol, 1.50 *eq)* in DCE (50 mL) was added AlCl₃ (7.48 g, 56.11 mmol, 3.07 mL, 1.10 *eq).* After addition, the mixture was stirred at 80°C for 0.5 h, and then 6-bromo-1H-indole (10.00 g, 51.01 mmol, 1.00 eq) in DCE (50 mL) was added. The resulting mixture was stirred at 80 °C for 15.5 h. The reaction mixture was quenched by addition Sat. NaHCO₃, and then extracted with EtOAc (150 mLx3), dried over Na₂SO₄, filtered and concentrated under reduced pressure to give a residue. The residue was purified by column chromatography (SiO₂, PE/EtOAc=10:1 to 1:1) to give a crude product. The crude product was washed with MeOH (100 mL), and filtered to collect the cake to afford the title compound (6.00 g, 15.62 mmol, 30.61% yield, 98 % purity) as a yellow solid.

### Step2: 1-(benzenesulfonyl)-6-bromo-3-[2 -chloro-5-(trifluoromethyl) pyrimidin-4-yl] indole

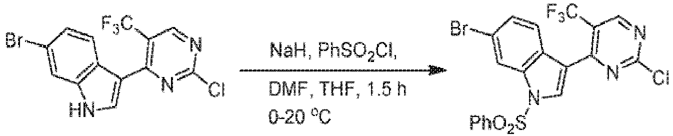

To a solution of 6-bromo-3-[2-chloro-5-(trifluoromethyl)pyrimidin-4-yl]-1H-indole (6.00 g, 15.93 mmol, 1.00 *eq)* in THF (54.00 mL) and DMF (6.00 mL) was added bathwise NaH (956.02 mg, 23.90 mmol, 60% purity, 1.50 *eq)* at 0 °C. After addition, the mixture was stirred at this temperature for 30 min, and then benzenesulfonyl chloride (4.22 g, 23.90 mmol, 3.06 mL, 1.50 eq) was added dropwise at 0 °C. The resulting mixture was stirred at 20 °C for 1 h. The reaction mixture was quenched by addition water 200 mL at 0 °C and extracted with EtOAc (100 mLx2). The combined organic layers were washed with brine (200 mLx2), dried over Na₂SO₄, filtered and concentrated under reduced pressure to give a residue. The residue was purified by column chromatography (SiO2, PE/EtOAc=10:1 to 5:1) to afford the title product (5.3 g) as a yellow solid.

### Step 3: tert-butyl (3S)-3-[[4-[1-(benzenesulfonyl)-6-(4-pyridyl)indol-3-yl]-3-(trifluoromethyl)pyrimidin-2-yl]amino]piperidine-1-carboxylate

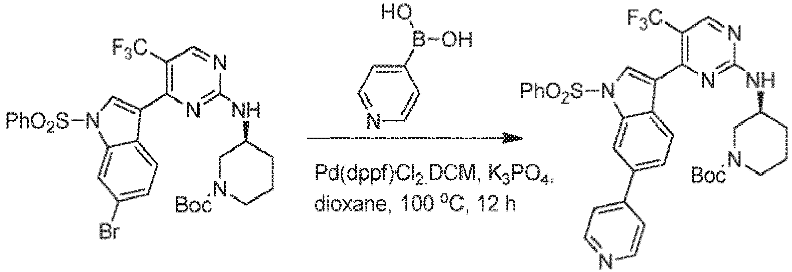

tert-butyl (3S)-3-[[4-[1-(benzenesulfonyl)-6-bromo-indol-3-yl]-5-(trifluoromethyl) pyrimidin-2-yl]amino]piperidine-1-carboxylate (200 mg, 293.89 umol) and 4-pyridylboronic acid (72 mg, 587.78 umol) were dissolved in dioxane (5 mL) and H₂O (1 mL). Pd(dppf)Cl₂.CH₂Cl₂ (24 mg, 29.39 umol) and K₃PO₄ (187 mg, 881.67 umol) were added into the mixture. The mixture was heated to 100 °C and stirred for 12 h under N₂. The reaction mixture was filtered and concentrated under reduced pressure to give a residue. The residue was purified by column chromatography (SiO₂, PE/EtOAc=10/1-5/1) to afford the title compound (200 mg, 90.24%) as a yellow solid.

### Step 4: tert-butyl (3S)-3-[[4-[6-(4-pyridyl)-1H-indol-3-yl]-5-(trifluoromethyl)pyrimidin-2-yl]amino]piperidine-1-carboxylate

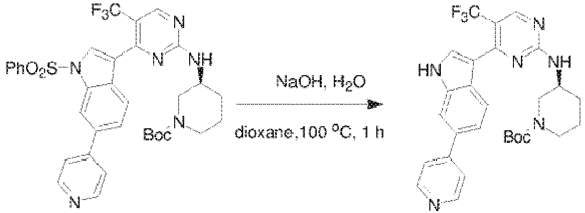

To a solution of tert-butyl (3S)-3-[[4-[1-(benzenesulfonyl)-6-(4-pyridyl)indol- 3-yl]-5-(trifluoromethyl)pyrimidin-2-yl]amino]piperidine-1-carboxylate (150 mg, 221.00 umol) in dioxane (2 mL) was added NaOH (44.20 mg, 1.11 mmol) and H₂O (500 uL) at 20 °C. The mixture was heated to 100 °C and stirred for 1 h under N₂. The reaction mixture was poured into water (20 mL) and extracted with EtOAc (20 mL * 3). The combined organic layers were dried over Na₂SO₄, filtered and concentrated under reduced pressure to afford the title compound (120 mg, crude) as a white solid.

### Step 5: N-[(3S)-3-piperidyl]-4-[6-(4-pyridyl)-1H-indol-3-yl]-5-(trifluoromethyl)pyrimidin-2-amine

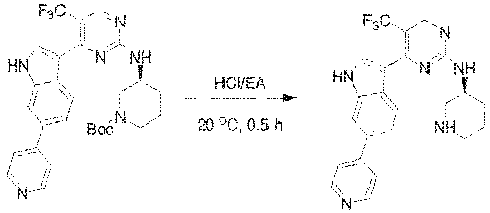

To a solution of tert-butyl (3S)-3-[[4-[6-(4-pyridyl)-1H-indol-3-yl]-5- (trifluoromethyl) pyrimidin-2-yl]amino]piperidine-1-carboxylate (120 mg, 222.82 umol) in HCl/EtOAc (4 M, 10 mL) was stirred for 0.5 h at 20 °C. Then the mixture was concentrated under reduced pressure to give a residue. The residue was purified by prep-HPLC (FA) to afford the title compound (67.70 mg, 60.76%) as a yellow solid.

### Example 56. 1-methyl-4-[3-[2-[[(3S)-3-piperidyl] amino]-5-(trifluoromethyl) pyrimidin-4-yl]-1H-indol-6-yl] pyrrole-2-carbonitrile (Compound 225).

### Step 1: 1-methylpyrrole-2-carbonitrile

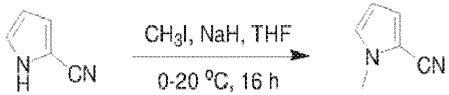

To a solution of 1H-pyrrole-2-carbonitrile (300.00 mg, 3.26 mmol, 1.00 eq) in THF (5.00 mL) was added batchwise NaH (156.40 mg, 3.91 mmol, 60% purity, 1.20 eq) at 0 °C. After addition, the mixture was stirred at this temperature for 0.5 h, and then iodomethane (601.54 mg, 4.24 mmol, 263.83 uL, 1.30 eq) was added dropwise at 0°C. The resulting mixture was stirred at 20 °C for 15.5 h. The reaction mixture was quenched by addition water 50 mL, and then extracted with EtOAc (20 mL * 3). The combined organic layers were washed with brine 100 mL, dried over Na₂SO₄, filtered and concentrated under reduced pressure to give a residue. The residue was purified by column chromatography (SiO₂, PE/EtOAc=50/1 to 20:1) to afford the title compound (200.00 mg, 1.88 mmol, 57.81% yield) as a yellw oil.

### Step 2: 4-bromo-1-methyl-pyrrole-2-carbonitrile

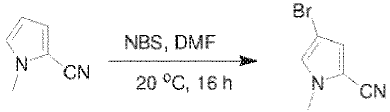

To a solution of 1-methylpyrrole-2-carbonitrile (200.00 mg, 1.88 mmol, 1.00 eq) in DMF (5.00 mL) was added NBS (335.40 mg, 1.88 mmol, 1.00 eq). The mixture was stirred at 20 °C for 16 h. The reaction mixture was poured into water 50 mL, and then extracted with EtOAc (20 mL * 3). The combined organic layers were washed with brine (100 mL), dried over Na₂SO₄, filtered and concentrated under reduced pressure to give a residue. The residue was purified by column chromatography (SiO₂, PEr/EtOAc=50:1 to 20:1) to afford the title compound (190.00 mg, 1.03 mmol, 54.62% yield) as a brown solid.

### Step 3: Tert-butyl (3S)-3-[[4-[6-(5-cyano-1-methyl-pyrrol-3-yl)-1H-indol-3-yl]-5-(trifluoromethyl) pyrimidin-2-yl] amino] piperidine-1-carboxylate

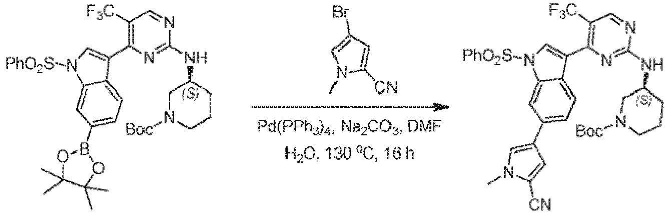

A mixture of 4-[1-(benzenesulfonyl)-6-(4,4,5,5-tetramethyl-1,3,2-dioxaborolan-2-yl)indol-3-yl]-N-[(3S)-3-piperidyl]-5-(trifluoromethyl)pyrimidin-2-amine (300.00 mg, 478.10 umol, 1.00 eq), 4-bromo-1-methyl-pyrrole-2-carbonitrile (132.69 mg, 717.15 umol, 1.50 eq), Pd(PPh₃)₄ (55.25 mg, 47.81 umol, 0.10 eq), Na₂CO₃ (5 M, 191.24 uL, 2.00 eq) in DMF (3.00 mL) was degassed and purged with N2 for 3 times, and then the mixture was stirred at 130°C for 16 h under N₂ atmosphere. The reaction mixture was poured into water 100ml, and then extracted with EtOAc (50 mL * 3). The combined organic layer were washed with brine (200 mL), dried over Na₂SO₄, filtered and concentrated under reduced pressure to afford the title compound (500.00 mg, crude) as a brown oil (Combined treatment with another batch, the SM scale: 100 mg). It was used for next step directly

### Step 4: Tert-butyl (3S)-3-[[4-[6-(5-cyano-1-methyl-pyrrol-3yl)-1H-indol-3-yl]-5-(trifluoromethyl) pyrimidin-2-yl] amino] piperidine-1-carboxylate

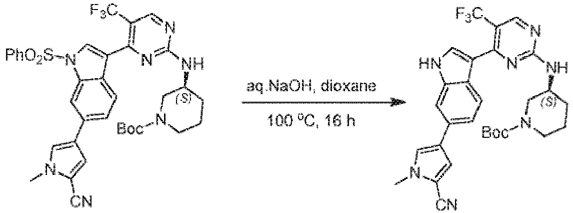

To a solution of tert-butyl (3S)-3-[[4-[1-(benzenesulfonyl)-6-(5-cyano-1-methyl-pyrrol-3-yl) indol-3-yl]-5-(trifluoromethyl)pyrimidin-2-yl]amino]piperidine-1-carboxylate (500.00 mg, 708.47 umol, 1.00 eq) in dioxane (10.00 mL) was added NaOH (5 M, 708.47 uL, 5.00 eq). The mixture was stirred at 100°C for 16 h. The reaction mixture was poured into water 50 mL, and then extracted with EtOAc (25 mL * 3). The combined organic layers were washed with brine (100 mL), dried over Na₂SO₄, filtered and concentrated under reduced pressure to give a residue.

The residue was purified by column chromatography (SiO₂, PE/EtoAc=5:1 to 1:1) to afford the title compound (160 mg) as a yellow solid.

### Step 5: 1-methyl-4-[3-[2-[[(3S)-3-piperidyl] amino]-5-(trifluoromethyl) pyrimidin-4-yl]-1H-indol-6-yl] pyrrole-2-carbonitrile

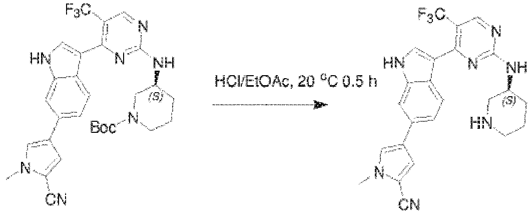

A mixture of tert-butyl (3S)-3-[[4-[6-(5-cyano-1-methyl-pyrrol-3-yl)-1H-indol-3-yl]-5-(trifluoromethyl)pyrimidin-2- yl]amino]piperidine-1-carboxylate (160.00 mg, 282.89 umol, 1.00 eq) in HCl/EtOAc (4 M, 2.12 mL, 30.00 eq) was stirred at 20 °C for 0.5 h. It was concentrated. The residue was purified by prep-HPLC (FA condition) to afford the title compound (24.90 mg, 48.68 umol, 17.2196 yield, 100% purity, FA) as a yellow oil.

### Example 57. 4-[6-(1, 3-dimethylpyrazol-4-yl)-1H-indol-3-yl]-N-[(3S)-3-piperidyl]-5-(trifluoromethyl) pyrimidin-2-amine (Compound 226).

### Step 1: tert-butyl (3S)-3-[[4-[1-(benzenesulfonyl)-6-(1,3-dimethy]pyrazol-4-yl)indol-3-yl] -5-(trifluoromethyl)pyrimidin-2-yl]amino]piperidine-1-carboxylate

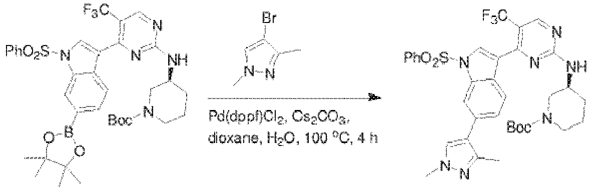

To a solution of tert-butyl (3S)-3-[[4-[1-(benzenesulfonyl)-6-(4,4,5,5-tetramethyl-1,3,2-dioxaborolan-2-yl)indol-3-yl]-5-(trifluoromethyl)pyrimidin-2-yl]amino]piperidine-1-carboxylate (150.00 mg, 206.16 umol) and 4-bromo-1,3-dimethyl-pyrazole (54.13 mg, 309.24 umol) in dioxane (5.00 mL) and H₂O (1.00 mL) was added Pd(PPh₃)₄ (23.82 mg, 20.62 umol) and Cs₂CO₃ (134.34 mg, 412.32 umol). The mixture was stirred at 100 °C for 4 h under N₂. The mixture was poured into water (100 mL). The aqueous phase was extracted with EtOAc (50 mL*3). The combined organic phase was washed with brine (100 mL), dried with anhydrous Na₂SO₄, filtered and concentrated. The residue was purified by column chromatography (SiO₂, Petroleum ether/Ethyl acetate=5/1 to 2/1) to afford the title compound (150 mg, crude).

### Step 2: The product of Step 1 was then converted to the title compound following Steps 4 and 5 of Example 56.

### Example 58. 4-[6-(1, 5-dimethylpyrazol-4-yl)-1H-indol-3-yl]-N-[(3S)-3-piperidyl]-5-(trifluoromethyl) pyrimidin-2-amine (Compound 227).

### Step 1: Tert-butyl (3S)-3-[[4-[1-(benzenesulfonyl)-6-(1,5-dimethylpyrazol-4-yl)indol-3-yl] -5-(trifluoromethyl)pyrimidin-2-yl]amino]piperidine-1-carboxylate

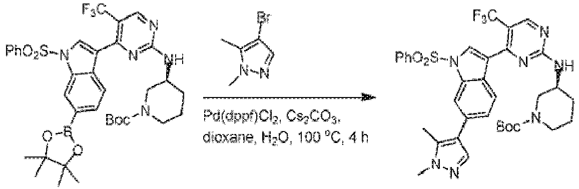

To a solution of tert-butyl (3S)-3-[[4-[1-(benzenesulfonyl)-6-(4,4,5,5-tetramethyl-1,3,2-dioxaborolan-2-yl)indol-3-yl]-5-(trifluoromethyl)pyrimidin-2-yl]amino]piperidine-1-carboxylate (120.00 mg, 164.93 umol) and 4-bromo-1,5-dimethyl-pyrazole (31.75 mg, 181.42 umol) in dioxane (5 mL) and H₂O (500 uL) was added Pd(PPh₃)₄ (19.06 mg, 16.49 umol) and Cs₂CO₃ (107.47 mg, 329.85 umol). The mixture was stirred at 100 °C for 4 h under N₂. The mixture was poured into water (100 mL). The aqueous phase was extracted with EA (50 mL*3). The combined organic phase was washed with brine (100 mL), dried with anhydrous Na₂SO₄, filtered and concentrated. The residue was purified by column chromatography (SiO₂, Petroleum ether/Ethyl acetate=5/1 to 2:1) to give to afford the title compound (150 mg, crude).

### Step 2: The product of Step 1 was then converted to the title compound following Steps 4 and 5 of Example 56.

### Example 59. Synthesis of 4-[6-(3-methyl-1H-pyrazol-4-yl)-1H-indol-3-yl]-N-[(3S)-3-piperidyl]-5-(trifluoromethyl) pyrimidin-2-amine (Compound 229).

### Step 1: 2-[(4-bromo-3-methyl-pyrazol-1-yl)methoxy]ethyl-trimethyl-silane

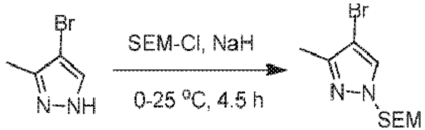

To a solution of 4-bromo-3-methyl-IH-pyrazole (50.00 mg, 310.56 umol, 1.00 eq) in THF (5.00 mL) was added NaH (13.66 mg, 341.61 umol, 1.10 eq). The mixture was stirred at 0°C for 0.5 h. 2-(chloromethoxy)ethyl-trimethyl-silane (54.37 mg, 326.09 umol, 57.84 uL, 1.05 eq) was added, and then the mixture was stirred at 25°C for 4 h. The residue was poured into water (100 mL). The aqueous phase was extracted with EtOAc (50 mL*3). The combined organic phase was washed with brine (100 mL), dried with anhydrous Na₂SO₄, filtered and concentrated in vacuum to afford the title compound (50.00 mg, 44.22%).

### Step 2: Starting with tert-butyl (3S)-3-[[4-[1-(benzenesulfonyl)-6-(4,4,5,5-tetramethyl-1,3,2-dioxaborolan-2-yl)indol-3-yl]-5-(trifluoromethyl)pyrimidin-2-yl]amino]piperidine-1-carboxylate and the product from Step 1, the title compound was produced following steps 3,4 and 5 of Example 56.

### Example 60. N-[(3S)-3-piperidyl]-4-(6-pyridazin-4-yl-1H-indol-3-yl)-5-(trifluoromethyl) pyrimidin-2-amine (Compound 230).

### Step 1: Tert-butyl (3S)-3-[[4-(6-pyridazin-4-yl-1H-indol-3-yl)-5-(trifluoromethyl) pyrimidin-2-yl] amino] piperidine-1-carboxylate

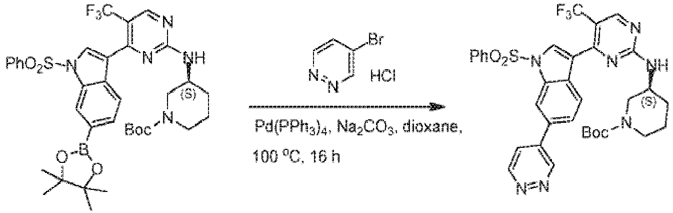

A mixture of 4-[1-(benzenesulfonyl)-6-(4,4,5,5-tetramethyl-1,3,2-dioxaborolan-2-yl)indol-3-yl]-N-[(3S)-3-piperidyl]-5-(trifluoromethyl)pyrimidin-2-amine (400.00 mg, 637.47 umol, 1.00 eq), 4-bromopyridazine (124.59 mg, 637.47 umol, 1.00 eq, HCl), Pd(PPh₃)₄ (73.66 mg, 63.75 umol, 0.10 eq), Na₂CO₃ (5 M, 254.99 uL, 2.00 eq) in dioxane (10.00 mL) was degassed and purged with N₂ for 3 times, and then the mixture was stirred at 100°C for 16 h under N₂ atmosphere. The reaction mixture was poured into water 50 mL, and then extracted with EtOAc (25 mL * 3), dried over Na₂SO₄, filtered and concentrated under reduced pressure to give a residue. The residue was purified by column chromatography (SiO₂, PE/EtOAc=5/1 to 1:3) to afford the title compound (100 mg) as a yellow solid which was used directly in the next step.

### Step 2: The product of Step 1 was then converted to the title compound following Steps 4 and 5 of Example 56.

### Example 61. 5-ethyl-2-(5-fluoro-1H- pyrrolo[2,3-b]pyridin-3-yl)-N-[(3S)-3-piperidyl] pyrimidin-4-amine (Compound 231).

### Step 1: 5-fluoro-3-(2-trimethylsilylethynyl) pyridine -2-amine

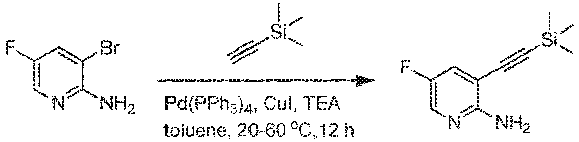

To a solution of 3-bromo-5-fluoro-pyridin-2-amine (8.00 g, 41.88 mmol, 1.00 eq), Pd(PPh₃)₄ (484.00 mg, 418.80 umol, 0.01 eq), CuI (79.77 mg, 418.80 umol, 0.01 eq) in toluene (40.00 mL) was added dropwise TEA (5.93 g, 58.63 mmol, 8.12 mL, 1.40 eq) at 20 °C under N₂ atmosphere. After addition, the mixture was stirred at this temperature for 30 min, and then ethynyl (trimethyl) silane (4.94 g, 50.26 mmol, 6.96 mL, 1.20 eq) was added at 20 °C. The resulting mixture was stirred at 60 °C for 11.5 h. The reaction mixture was filtered. The filtrate was diluted with water 250 mL and extracted with EtOAc (150 mL*3). The combined organic layers were washed with brine (500 mL*2), dried over Na₂SO₄, filtered and concentrated under reduced pressure to give a residue. The residue was purified by column chromatography (SiO₂, Petroleum ether/Ethyl acetate=50:1 to 5:1) to afford the title compound (6.40 g, 27.65 mmol, 66.02% yield, 90% purity) as a yellow solid.

### Step 2: 5-fluoro-1H- pyrrolo [2, 3-b] pyridine

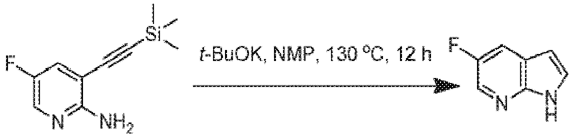

A mixture of 5-fluoro-3-(2-trimethylsilylethynyl) pyridin-2-amine (6.40 g, 30.72 mmol, 1.00 eq) and t-BuOK (5.72 g, 51.00 mmol, 1.66 eq) in NMP (60.00 mL) was stirred at 130 °C for 12 h. The reaction mixture was diluted with water 200 mL and extracted with ethyl acetate (100 mL*3). The combined organic layers were washed with brine (300 mL*2), dried over Na₂SO₄, filtered and concentrated under reduced pressure to give a residue. The residue was purified by column chromatography (SiO₂, Petroleum ether/Ethyl acetate=10:1 to 5:1) to afford the title compound (3.20 g, 23.04 mmol, 74.99% yield, 98% purity) as a yellow solid.

### Step 3: 1-(benzenesulfonyl)-5-fluoro- pyrrolo [2, 3-b] pyridine

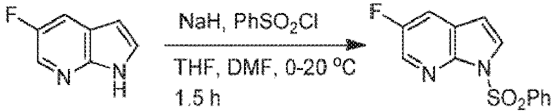

To a solution of 5-fluoro-1H-pyrrolo[2,3-b]pyridine (3.20 g, 23.51 mmol, 1.00 eq) in THF (36.00 mL) and DMF (4.00 mL) was added bathwise NaH (1.41 g, 35.26 mmol, 60% purity, 1.50 eq) at 0 °C. After addition, the mixture was stirred at this temperature for 30 min, and then benzenesulfonyl chloride (6.23 g, 35.26 mmol, 4.51 mL, 1.50 eq) was added dropwise at 0 °C. The resulting mixture was stirred at 20 °C for 1 h. The reaction mixture was quenched by addition H₂O 20 mL. The solid was formed and filtered to afford the title compound (6.20 g, 21.99 mmol, 93.55% yield, 98% purity) as a white solid.

### Step 4: 1-(benzenesulfonyl)-3-bromo-5-fluoro- pyrrolo [2, 3-b] pyridine

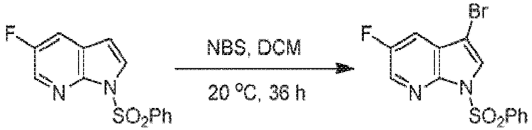

To a solution of 1-bromopyrrolidine-2,5-dione (4.43 g, 49.78 mmol, 1.11 eq) in DCM (20 mL) was added dropwise 1-(benzenesulfonyl)-5-fluoro-pyrrolo[2,3-b]pyridine (6.20 g, 22.44 mmol, 1.00 eq) in DCM (40 mL) at 20 °C. After addition, the mixture was stirred at this temperature for 12 h. After 12 h, 1-bromopyrrolidine-2, 5-dione (4.43 g, 49.78 mmol, 1.11 eq) was added to the mixture again, and stirred at 20°C for 24 h. The reaction mixture was diluted with water 100 mL and extracted with DCM (50 mL*2). The combined organic layers were washed with brine (200 mL*2), dried over Na₂SO₄, filtered and concentrated under reduced pressure to give a residue. The residue was purified by column chromatography (SiO₂, PE/EtOAc=20:1 to 10:1) to afford the title compound (6.24 g, 14.23 mmol, 63.4196 yield, 81% purity) as a yellow solid.

### Step 5: 1-(benzenesulfonyl)-5-fluoro-3-(4, 4, 5, 5-tetramethyl-1, 3, 2-dioxaborolan-2-yl) pyrrolo [2, 3-b] pyridine

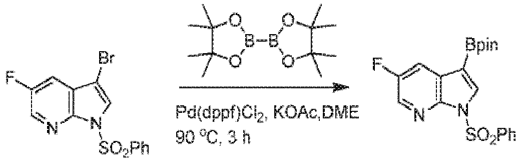

A mixture of 1-(benzenesulfonyl)-3-bromo-5-fluoro-pyrrolo[2,3-b]pyridine (2.00 g, 5.63 mmol, 1.00 eq), 4, 4, 5, 5-tetramethyl-2-(4, 4, 5, 5- tetramethyl-1, 3, 2- dioxaborolan-2-yl)-1, 3, 2-dioxaborolane (1.86 g, 7.32 mmol, 1.30 eq), Pd(dppf)Cl₂ (412.02 mg, 563.09 umol, 0.10 eq), AcOK (1.11 g, 11.26 mmol, 2.00 eq) in DME (10.00 mL) was degassed and purged with N₂ for 3 times, and then the mixture was stirred at 90 °C for 3 h under N₂ atmosphere. The reaction mixture was diluted with water 100 mL and extracted with EtOAc (50 mL*3). The combined organic layers were washed with brine (200 mL*2), dried over Na₂SO₄, filtered and concentrated under reduced pressure to give a residue. The residue was purified by column chromatography (SiO₂, PE/EtOAc=30:1 to 3:1) to afford the title compound (1.04 g) as a yellow solid.

### Step 6: Tert-butyl (3S)-3-[[2-[1-(benzenesulfonyl)-5- fluoro- pyrrolo [2, 3-b] pyridin-3-yl] - 5-chloro-pyrimidin-4-yl] amino] piperidine-1-carboxylate

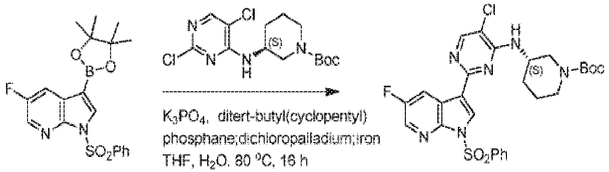

A mixture of 1-(benzenesulfonyl)-5-fluoro-3-(4,4,5,5-tetramethyl-1,3,2-dioxaborolan-2-yl) pyrrolo[2,3-b]pyridine (1.04 g, 2.59 mmol, 1.00 eq), tert-butyl (3S)-3-[(2,5-dichloropyrimidin - 4-yl)amino] piperidine- 1-carboxylate (899.35 mg, 2.59 mmol, 1.00 eq), K₃PO₄ (1.10 g, 5.18 mmol, 2.00 eq), and ditert butyl(cyclopentyl)phosphane;dichloropalladium;iron (168.80 mg, 259.00 umol, 0.10 eq) in THF (10.00 mL) and H₂O (2.00 mL) was degassed and purged with N₂ for 3 times, and then the mixture was stirred at 80 °C for 16 h under N₂ atmosphere. The reaction mixture was diluted with water 50 mL and extracted with EtOAc (30 mL*3). The combined organic layers were washed with brine (100 mL*2), dried over Na₂SO₄, filtered and concentrated under reduced pressure to give a residue. The residue was purified by column chromatography (SiO₂, PE/EtOAc=10:1 to 5:1) to give desired product as a yellow oil (970 mg), then the residue was purified by prep-HPLC (TFA condition) to give desired product as a solution. The pH of the eluent solution was adjusted to 8 by saturated aqueous NaHCOa and extracted with EtOAc (20 mL*3). The combined organic layers were washed with brine (100 mL*2), dried over Na₂SO₄, filtered and concentrated under reduced pressure to afford the title compound (400.00 mg, 681.35 umol, 26.3 1% yield, 100% purity) as a yellow solid.

### Step 7: Tert-butyl (3S)-3-[[2-[1-(benzenesulfonyl) - 5-fluoro-pyrrolo [2, 3-b] pyridin-3-yl] - 5-vinyl-pyrimidin-4-yl] amino] piperidine-1-carboxylate

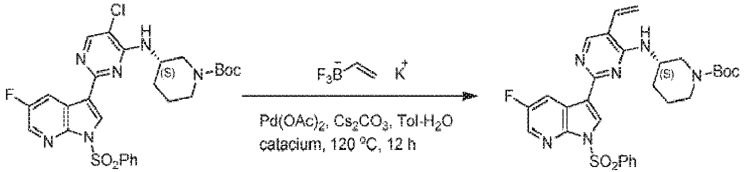

A mixture of trifluoro-potassio-vinyl-boron(1-) (342.26 mg, 2.56 mmol, 5.00 eq), tert-butyl (3S)-3-[[2-[1-(benzenesulfonyl)-5-fluoro-pyrrolo[2,3-b]pyridin-3-yl]-5-chloro-pyrimidin-4-yl]amino]piperidine-1-carboxylate (300.00 mg, 511.01 umol, 1.00 eq), bis(1-adamantyl)-butyl-phosphane (36.64 mg, 102.20 umol, 0.20 eq), Cs₂CO₃ (333.00 mg, 1.02 mmol, 2.00 eq) and Pd(OAc)₂ (11.47 mg, 51.10 umol, 0.10 eq) in toluene (5.00 mL), H₂O (1.00 mL) was degassed and purged with N2 for 3 times, and then the mixture was stirred at 120 °C for 12 h under N₂ atmosphere. The reaction mixture was diluted with water 20 mL and extracted with EtOAc (10 mL*3). The combined organic layers were washed with brine (50 mL*2), dried over Na₂SO₄, filtered and concentrated under reduced pressure to give a residue. The residue was purified by column chromatography (SiO₂, PE/EtOAc=10:1 to 3:1) to afford the title compound (190 mg) as a yellow solid.

### Step 8: Tert-butyl (3S)-3-[[2-[1-(benzenesulfonyl)- 5-fluoro-pyrrolo[2,3-b]pyridin-3-yl]-5-ethyl-pyrimidin-4-yl]amino] piperidine-1-carboxylate

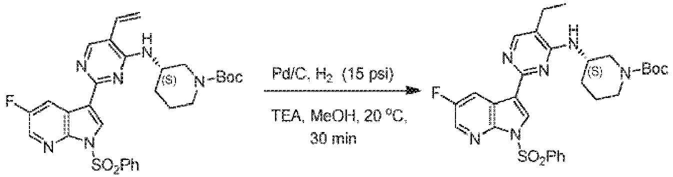

To a solution of tert-butyl (3S)-3-[[2-[1-(benzenesulfonyl)-5- fluoro-pyrrolo[2,3-b] pyridin-3-yl]-5-vinyl-pyrimidin-4-yl]amino]piperidine-1-carboxylate (190.00 mg, 328.34 umol, 1.00 eq) and TEA (99.68 mg, 985.03 umol, 136.54 uL, 3.00 eq) in MeOH (2.00 mL) was added Pd-C (10%, wet, 10 mg) under N₂. The suspension was degassed under vacuum and purged with H₂ 3 times. The mixture was stirred under H₂ (15 psi) at 20 °C for 30 min. The reaction mixture was diluted with water 20 mL and extracted with EtOAc (10 mL*3). The combined organic layers were washed with brine (30 mL*2), dried over Na₂SO₄, filtered and concentrated under reduced pressure to give a residue. The residue was purified by column chromatography (SiO₂, PE/EtOAc=5:1 to 2:1) to afford the title compound (150.00 mg, 255.74 umol, 77.89% yield, 99% purity) as a yelow solid.

### Step 9: Tert-butyl (3S)-3-[[5-ethyl-2-(5-fluoro-1H-pyrrolo [2, 3-b] pyridin-3-yl) pyrimidin-4-yl] amino] piperidine-1-carboxylate

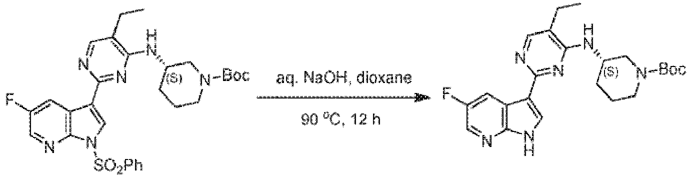

A mixture of tert-butyl (3S)-3-[[2-[1-(benzenesulfonyl)-5-fluoro-pyrrolo[2,3-b] pyridin-3-yl]-5-ethyl-pyrimidin-4-yl]amino]piperidine-1-carboxylate (150.00 mg, 258.32 umol, 1.00 eq) and NaOH (5 M, 516.64 uL, 10.00 eq) in dioxane (2.00 mL) was stirred at 90 °C for 12 h. The reaction mixture was diluted with water 20 mL and extracted with EtOAc (10 mL*3). The combined organic layers were washed with brine (30 mL*2), dried over Na₂SO₄, filtered and concentrated under reduced pressure to afford the title compound (110.00 mg, crude) as a yellow solid.

### Step 10: 5-ethyl-2-(5-fluoro-1H-pyrrolo [2, 3-b] pyridin-3-yl)-N- [(3S)-3-piperidyl] pyrimidin-4-amine

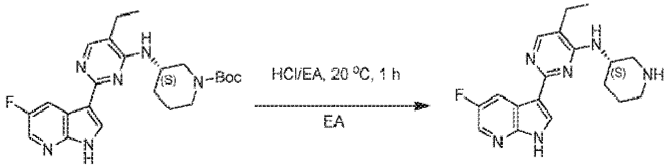

A mixture of tert-butyl (3S)-3-[[5-ethyl-2-(5-fluoro-1H- pyrrolo [2, 3-b] pyridin-3-yl) pyrimidin-4-yl] amino] *piperidine-1-carboxylate* (110.00 mg, 249.71 umol, 1.00 eq) and HCl/EtOAc (4 M, 2.00 mL) in EA (2.00 mL) was stirred at 20 °C for 1 h. The reaction mixture was concentrated under reduced pressure to give a residue (80 mg HCl salt). The residue was purified by prep-HPLC (FA condition) to afford the title compound (9.40 mg, 24.08 umol, 9.64% yield, 99% purity, FA) as a yellow solid. (Combined purification with another batch. Scale: 30 mg).

### Example 62. 7-methylsulfonyl-3-[2-[[(3S)-3-piperidyl]amino]-5-(trifluoromethyl) pyrimidin-4-yl]-1H-indole-6-carbonitrile (Compound 232).

### Step 1: 1-bromo-2-methylsulfanyl-3-nitro-benzene

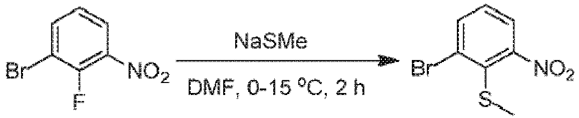

An aqueous solution of NaSMe (10.35 g, 29.55 mmol, 20% purity) was added to a solution of 1-bromo-2-fluoro-3-nitro-benzene (5.00 g, 22.73 mmol) in DMF (50 mL) at 0 °C within 10 min. The mixture was stirred for 1 h 50 min at 15 °C. The reaction mixture was dropwisely added into water (200 mL) and stirred for 30 min, filtered and the solid was dried under reduced pressure to afford the title compound (5.00 g, 79.81%) as a white solid.

### Step 2: 1-bromo-2-methylsulfonyl-3-nitro-benzene

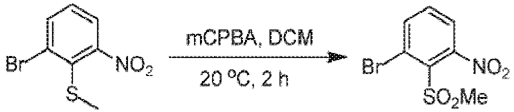

To a mixture of 1-bromo-2-methylsulfanyl-3-nitro-benzene (6.00 g, 24.18 mmol, 1.00 eq) in DCM (100.00 mL) was added m-CPBA (14.90 g, 60.45 mmol, 70% purity, 2.50 eq) in one portion at 20 °C under N₂. The mixture was stirred at 20 °C for 2 h. The mixture was poured into water (300 mL) and extracted with EtOAc (150 mL*2). The combined organic phase was washed with aqueous Na₂SO₃ (200 mL*3), brine (200 mL*2), dried with anhydrous Na₂SO₄, filtered and concentrated in vacuum. The residue was purified by silica gel chromatography (PE/DCM=20/1, 0/1) to afford the title compound (4.40 g, crude) as a yellow solid.

### Step 3: 6-bromo-7-methylsulfonyl-1H-indole

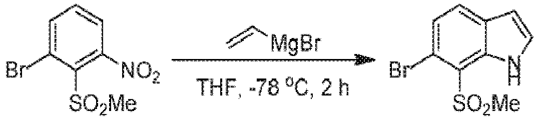

To a mixture of 1-bromo-2-methylsulfonyl-3-nitro-benzene (4.40 g, 15.71 mmol, 1.00 eq) in THF (100.00 mL) was added bromo(vinyl)magnesium (1 M, 78.55 mL, 5.00 eq) in portions at - 78 °C under N₂. The mixture was stirred at -78 °C for 2 h. The mixture was poured into water (300 mL) and extracted with EtOAc (150 mL*2). The combined organic phase was washed with brine (200 mL*2), dried with anhydrous Na₂SO₄, filtered and concentrated in vacuum. The residue was purified by silica gel chromatography (PE/EtOAc=20/1, 0/1) to afford the title compound (1.80 g, 6.57 mmol, 41.80% yield) as yellow solid.

### Step 4: 7-methylsulfonyl-1H-indole-6-carbonitrile

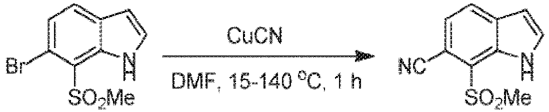

To a mixture of 6-bromo-7-methylsulfonyl-1H-indole (700.00 mg, 2.55 mmol, 1.00 eq) in DMF (20.00 mL) was added CuCN (685.13 mg, 7.65 mmol, 1.67 mL, 3.00 eq) in one portion at 15 °C under N₂. The mixture was stirred at 140 °C for 1 h. The residue was poured into water (100 mL) and extracted with EtOAc (50 mL*2). The combined organic phase was washed with brine (100 mL*2), dried with anhydrous Na₂SO₄, filtered and concentrated in vacuum. The residue was purified by silica gel chromatography (PE/EtOAc=10/1, 3/1) to afford the title compound (450.00 mg, crude) as yellow solid.

### Step 5: 3-bromo-7-methylsulfonyl-1H-indole-6-carbonitrile

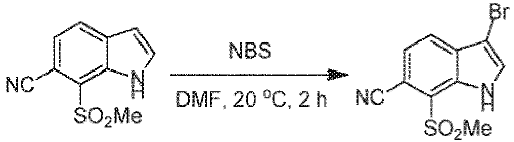

To a mixture of 7-methylsulfonyl-1H-indole-6-carbonitrile (450.00 mg, 2.04 mmol, 1.00 eq) in DMF (20.00 mL) was added NBS (399.39 mg, 2.24 mmol, 1.10 eq) in one portion at 20 °C under N₂. The mixture was stirred at 20 °C for 2 h. The mixture was poured into water (100 mL) and extracted with EtOAc (50 mL*2). The combined organic phase was washed with brine (50 mL*2), dried with anhydrous Na₂SO₄, filtered and concentrated in vacuum. The residue was purified by silica gel chromatography (PE/EtOAc=10/1, 211) to afford the title compound (400.00 mg, 1.34 mmol, 65.55% yield) as white solid.

### Step 6:Tert-butyl 3-bromo-6-cyano-7-methylsulfonyl-indole-1-carboxylate

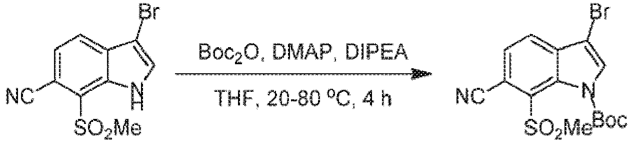

To a mixture of 3-bromo-7-methylsulfonyl-1H-indole-6-carbonitrile (400.00 mg, 1.34 mmol, 1.00 eq) and Boc₂O (437.75 mg, 2.01 mmol, 460.79 uL, 1.50 eq) in THF (20.00 mL) was added DMAP (32.67 mg, 267.43 umol, 0.20 eq) and DIPEA (345.63 mg, 2.67 mmol, 467.07 uL, 2.00 eq) in one portion at 20 °C under N₂. The mixture was stirred at 80 °C for 4 h. The residue was poured into water (50 mL) and extracted with EtOAc (30 mL*2). The combined organic phase was washed with brine (50 mL*2), dried with anhydrous Na₂SO₄, filtered and concentrated in vacuum. The residue was purified by silica gel chromatography (PFJEtOAe=10/1, 2/1) to afford the title compound (400.00 mg, crude) as white solid.

### Step 7: Tert-butyl 6-cyano-7-methylsulfonyl-3-(4,4,5,5-tetramethyl-1,3,2-dioxaborolan-2-yl) indole-1-carboxylate

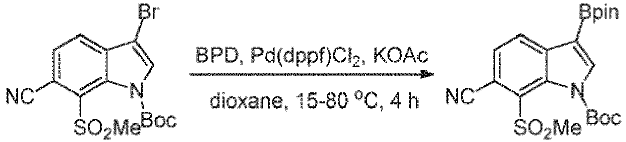

To a mixture of tert-butyl 3-bromo-6-cyano-7-methylsulfonyl-indole-1-carboxylate (350.00 mg, 876.62 umol, 1.00 eq) and BPD (267.13 mg, 1.05 mmol, 1.20 eq) in dioxane (10.00 mL) was added Pd(dppf)Cl₂ (64.14 mg, 87.66 umol, 0.10 eq) and KOAc (172.06 mg, 1.75 mmol, 2.00 eq) in one portion at 15 °C under N₂. The mixture was stirred at 80 °C for 4 h. The residue was poured into water (20 mL) and extracted with EtOAc (10 mL*2). The combined organic phase was washed with brine (10 mL*2), dried with anhydrous Na₂SO₄, filtered and concentrated in vacuum. The residue was purified by prep-HPLC (TFA) to afford the title compound (100.00 mg, 178.46 umol, 20.36% yield, TFA salt) as a yellow oil.

### Step 8: Tert-butyl (3S)-3-[[4-(6-cyano-7-methylsulfonyl-1H-indol-3-yl)-5-(trifluoromethyl) pyrimidin-2-yl]amino]piperidine-1-carboxylate

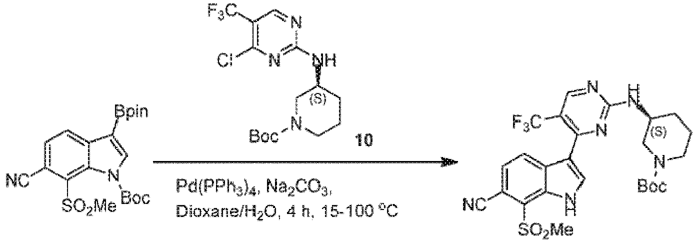

To a mixture of tert-butyl 6-cyano-7-methylsulfonyl-3-(4,4,5,5-tetramethyl-1,3,2- dioxaborolan-2-yl)indole-1-carboxylate (95.00 mg, 212.85 umol, 1.00 eq) and tert-butyl (3S)-3-[[4-chloro-5-(trifluoromethyl)pyrimidin-2-yl]amino]piperidine-1-carboxylate (121.58 mg, 319.27 umol, 1.50 eq) in dioxane (10.00 mL) and H₂O (2.00 mL) was added Pd(PPh₃)₄ (24.60 mg, 21.29 umol, 0.10 eq) and Na₂CO₃ (45.12 mg, 425.70 umol, 2.00 eq) in one portion at 15 °C under N₂. The mixture was stirred at 100 °C for 4 h. The residue was poured into water (30 mL) and extracted with EtOAc (20 mL*2). The combined organic phase was washed with brine (20 mL*2), dried with anhydrous Na₂SO₄, filtered and concentrated in vacuum. The residue was purified by silica gel chromatography ( PE/EtOAc=10/1, 211) to afford the title compound (80.00 mg, crude) as yellow solid.

### Step 9: 7-methylsulfonyl-3-[2-[[(3S)-3-piperidyl]amino]-5-(trlfluoromethyl)pyrimidin-4-yl] -1H-indole-6-carbonitrile

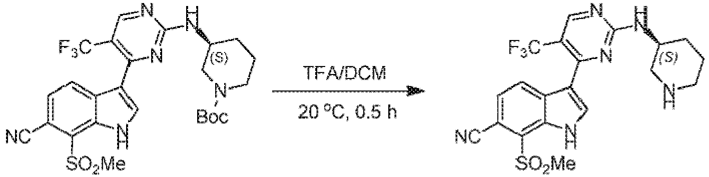

To a mixture of tert-butyl(3S)-3-[[4-(6-cyano-7-methylsulfonyl-1H-indol-3-yl)-5-(trifluoromethyl)pyrimidin-2-yl]amino]piperidine-1-carboxylate (60.00 mg, 106.27 umol, 1.00 eq) in DCM (2.00 mL) was added TFA (400.00 uL) in portions at 20 °C under N₂. The mixture was stirred at 20 °C for 30 min. The mixture was concentrated under reduced pressure. The residue was purified by prep-HPLC(FA) to afford the title compound (14.00 mg, 27.42 umol, 25.80% yield, FA) as white solid (Note: Combined purification with another batch. Scale: 60 mg)

### Example 63. 4-[3-[2-[[(3S)-3-piperidyl] amino]-5-(trifluoromethyl) pyrimidin-4-yl]-1H-indol- 6-yl]-1H- pyrrole-2-carbonitrile (Compound 233).

### Step 1: 1-(2-trimethylsilylethoxymethyl) pyrrole-2-carbonitrile

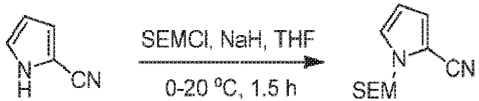

To a solution of 1H-pyrrale-2-carbonitrile (300.00 mg, 3.26 mmol, 1.00 eq) in THF (2.00 mL) was added batchwise NaH (195.44 mg, 4.89 mmol, 60% purity, 1.50 eq) at 0 °C. After addition, the mixture was stirred at this temperature for 30 min, and then 2-(chloromethoxy) ethyl-trimethyl-silane (814.59 mg, 4.89 mmol, 866.59 uL, 1.50 eq) was added dropwise at 0 °C. The resulting mixture was stirred at 20 °C for 1 h. The reaction mixture was quenched with H₂O (20 mL), and extracted with EtOAc (10 mL*3). The combined organic layers were washed with brine (50 mL*2), dried over anhydrous Na₂SO₄, filtered and concentrated under reduced pressure to give a residue. The residue was purified by column chromatography (SiO₂, PE/EtOAc=20:1 to 10:1) to afford the title compound (550 mg) as a yellow oil.

### Step 2: 4-bromo-1-(2-trimethylsilylethoxymethyl)pyrrole-2-carbonitrile

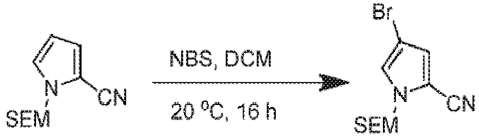

To a solution of 1-(2-trimethylsilylethoxymethyl) pyrrole-2-carbonitrile (550.00 mg, 2.47 mmol, 1.00 eq) in DCM (8.00 mL) was added1-bromopyrrolidine-2, 5-dione (483.57 mg, 2.72 mmol, 1.10 eq). The mixture was stirred at 20 °C for 16 h. The reaction mixture was concentrated under reduced pressure to give a residue. The residue was purified by column chromatography (SiO₂, PE/EtOAc=90:1 to 80:1) to afford the title compound (200.00 mg, 597.51 umol, 24.19% yield, 90% purity) as a yellow oil.

### Step 3: Tert-butyl (3S)-3-[[4-[1-(benzenesulfonyl)-6- [5-cyano-1-(2-trimethylsilylethoxymethyl) pyrrol-3-yl] indol-3-yl]- 5-(trifluoromethyl) pyrimidin-2-yl] amino] piperidine- 1-carboxylate

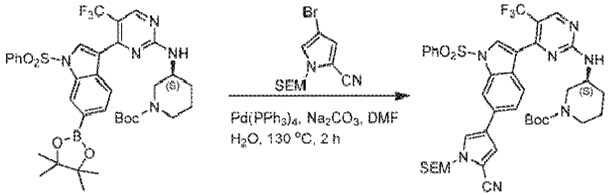

A mixture of tert-butyl (3S)-3-[[4-[1-(benzenesulfonyl)-6- (4,4.5,5-tetramethyl-1,3,2-dioxaborolan-2-yl)indol-3-yl]-5-(trifluoromethyl)pyrimidin-2-yl]amino]piperidine-1-camoxylate (399.22 mg, 548.68 umol, 1.00 eq), 4-bromo-1-(2-trimethylsilylethoxymethyl)pyrrole-2-carbonitrile (200.00 mg, 663.90 umol, 1.21 eq), Pd(PPh₃)₄ (63.40 mg, 54.87 umol, 0.10 eq) and Na₂CO₃ (5 M, 219.47 uL, 2.00 eq) in DMF (10.00 mL) was degassed and purged with N₂ for 3 times, and then the mixture was stirred at 130 °C for 2 h under N₂ atmosphere. The reaction mixture was diluted with water 50 mL and extracted with EtOAc (30 mL*3). The combined organic layers were washed with brine (100 mL*2), dried over Na₂SO₄, filtered and concentrated under reduced pressure to afford the title compound (500.00 mg, crude) as a black brown oil. It was used in the next step directly and without further purification.

### Step 4: Tert-butyl (3S)-3-[[4-[6-[5-cyano-1-(2-trimethylsilylethoxymethyl)pyrrol-3-yl] - 1H-indol-3-yl]-5-(trifluoromethyl)pyrimidin-2-yl]amino]piperidine-1-carboxylate

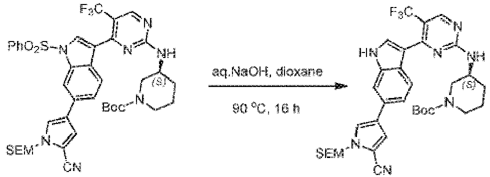

A mixture of tert-butyl (3S)-3-[[4-[1-(benzenesulfonyl)-6-[5-cyano-1-(2-trimethylsilylethoxymethyl) pyrrol-3-yl]indol-3-yl]-5-(trifluoromethyl) pyrimidin-2-yl]amino] piperidine-1-carboxylate (500.00 mg, 608.29 umol, 1.00 eq) and NaOH (5 M, 1.22 mL, 10.00 eq) in dioxane (5.00 mL) was stirred at 90 °C for 16 h. The reaction mixture was diluted with water 50 mL and extracted with EtOAc (30 mL*3). The combined organic layers were washed with brine (100 mL*2), dried over Na₂SO₄, filtered and concentrated under reduced pressure to give a residue. The residue was purified by column chromatography (SiO₂, PE/EtOAc=10:1 to 3:1) to afford the title compound (200.00 mg, 222.93 umol, 36.65% yield, 76% purity) as a yellow solid.

### Step 5: 4-[3-[2-[[(3S)-3-piperidyl] amino]-5-(trifluoromethyl) pyrimidin-4-yl]- IH-indol-6-yl]-lH- pyrrole-2-carbonitrile

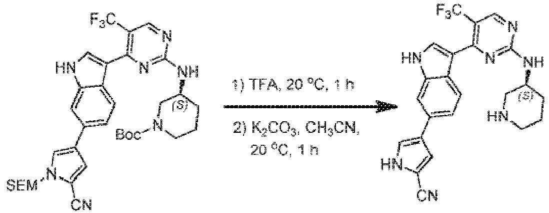

A mixture of tert-butyl (3S)-3-[[4-[6-[5-cyano-1-(2-trimethylsilylethoxymethyl) pyrrol-3-yl]-1H-indol-3-yl]-5-(trifluoromethyl) pyrimidin-2-yl]amino] piperidine- 1-carboxylate (170.00 mg, 249.33 umol, 1.00 eq) in TFA (3.00 mL) was stirred at 20 °C for 1 h, the mixture was concentrated under reduced pressure to give a residue. To a solution of the residue in CH₃CN (3.00 mL) was added K₂CO₃ (103.38 mg, 747.99 umol, 3.00 eq). The mixture was stirred at 20 °C for 1 h. The reaction was stopped. The reaction mixture was filtered. The filtrate was concentrated under reduced pressure to give a residue as a yellow oil. The residue was purified by prep-HPLC (FA condition) to afford the title compound (10.12 mg, 19.33 umol, 7.75% yield, 95% purity, FA) as a yellow solid.

### Example 64. N-[(3S)-3-piperidyl]-4-[6-(1H-1, 2, 4-triazol-3-yl)-1H-indol-3-yl]-5-(trifluoremethyl) pyrimidin-2-amine (Compound 236).

### Step 1: 2-[(3-bromo-5-methyl-1,2,4-triazol-1-yl)methoxy]ethyl-trimethyl-silane

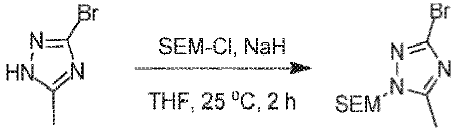

To a mixture of 3-hromo-5-methyl-1H-1,2,4-triazole (100.00 mg, 617.32 umol) and Et₃N (484.74 mg, 4.79 mmol) in THF (5.00 mL) was added SEM-CL (123.50 mg, 740.79 umol). The mixture was stirred at 25 °C for 2 h N₂ atmosphere. The residue was poured into water (100 mL). The aqueous phase was extracted with EtOAc (50 mL*3). The combined organic phase was washed with brine (100 mL), dried with anhydrous Na₂SO₄, filtered and concentrated in vacuum to afford the title compound (150.00 mg, crude).

### Step 2: tert-butyl (3S)-3-[[4-[1-(benzenesulfonyl)-6-[5-methyl-1-(2-trimethylsilylethoxymethyl)-1,2,4-triazol-3-yl]indol-3-yl]-5-(trifluoromethyl)pyrimidin-2-yl]amino]piperidine-1-carboxylate

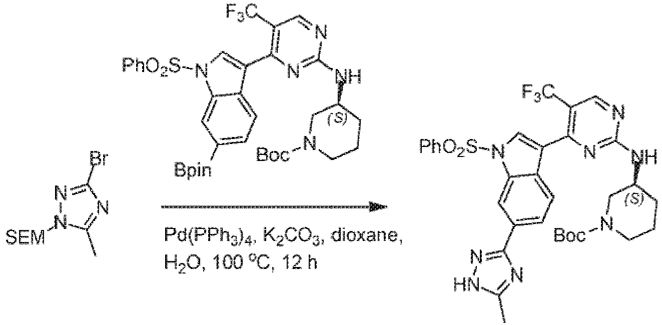

To a solution of tert-butyl (3S)-3-[[4-[1-(benzenεsulfonyl)-6-(4,4,5,5- tetramethyl-1,3,2-dioxaborolan-2-yl)indol-3-yl]-5-(trifluoromethyl)pyrimidin-2-yl]aminojpiperidine- 1-carboxylate (210.00 mg, 288.62 umol) and 2-[(3-bromo-5-methyl- 1,2,4-triazol-1-yl)methoxy]ethyl-trimethyl-silane (95.00 mg, 325.06 umol) in dioxane (5.00 mL) and H₂O (500.00 uL) was added K₂CO₃ (80.00 mg, 578.83 umol) and Pd(PPh₃)₄ (167.00 mg, 144.52 umol). The mixture was stirred at 100 °C for 12 h under N₂. The residue was purified by prep-HPLC (TFA condition), and then the product was adjust PH=8 with NaHCO₃ (aq). The aqueous phase was extracted with EtOAc (30 mL*3). The combined organic phase was washed with brine (80 mL), dried with anhydrous Na₂SO₄, filtered and concentrated in vacuum to afford the title compound (70.00 mg, 28.34%).

### Step 3: tert-butyl (3S)-3-[[5-(trifluoromethyl)-4-[6-[1-(2-trimethylsilylethoxymethyl)-1,2,4-triazol-3-yl]-1H-indol-3-yl]pyrimidin-2-yl]amino]piperidine-1-carboxylate

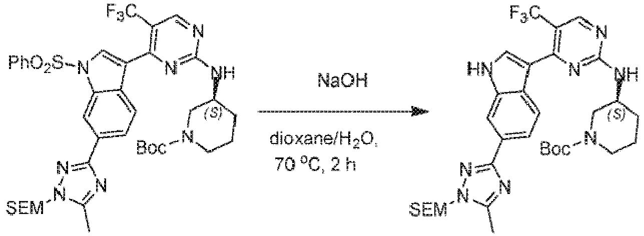

To a solution of tert-butyl (3S)-3-[[4-[1-(benzenesulfonyl)-6-[5-methyl-1-(2-trimethylsilylethoxymethyl)-1,2,4-triazol-3-yl]indol-3-yl]-5-(trifluoromethyl)pyrimidin-2-yl]amino]piperidine-1-carboxylate (70.00 mg, 86.10 umol) in dioxane (8.00 mL) was added NaOH (5 M, 500.07 uL). The mixture was stirred at 70 °C for 2 h. The residue was poured into water (100 mL). The aqueous phase was extracted with EtOAc (50 mL*3). The combined organic phase was washed with brine (100 mL), dried with anhydrous Na₂SO₄, filtered and concentrated to afford the title compound (50.00 mg, crude).

### Step 4: N-[(3S)-3-piperidyl]-4-[6-(1H-1,2,4-triazol-3-yl)-1H-indol-3-yl]-5-(trifluoromethyl)pyrimidin-2-amine

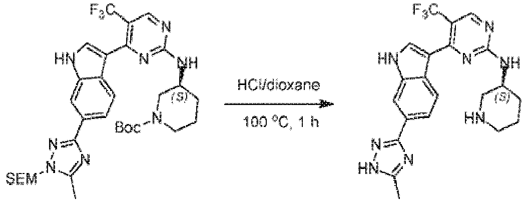

A mixture of tert-butyl (3S)3-[[5-(trifluoromethyl)-4-[6-[1-(2-trimethylsilylethoxymethyl)-1,2,4-triazol-3-yl]-1H-indol-3-yl]pyrimidin-2-yl]amino]piperidine-1-carboxylate (50.00 mg, 75.90 umol) in HCl (600.00 uL) and dioxane (3.00 mL) was degassed and purged with N₂ for 3 times, and then the mixture was stirred at 100 °C for 1 h under N₂ atmosphere. The residue was purified by prep-HPLC (FA acid) to afford the title compound (15.00 mg, 41.07%).

### Example 65. N-[(3S)-5, 5-difluoro-3-piperidyl]-4-(1H-indol-3-yl)-5-(trifluoromethyl) pyrimidin-2-amine (Compound 236).

### Step 1: tert-butyl N-[(3S,5R)-1-benzyl-5-[tert-butyl(dimethyl)silyl]oxy-3-piperidyl] carbamate

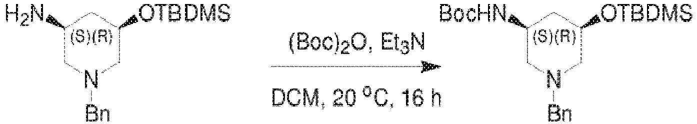

To a solution of (3S,5R)-1-benzyl-5-[tert-butyl(dimethyl)silyl]oxy-piperidin-3- amine;[(2R,4R)-1-benzyl-4-[tert-butyl(dimethyl)silyl]oxy-pyrrolidin-2-yl]methanamine (15.00 g, crude) in DCM (60.00 mL) was added Et₃N (4.74 g, 46.80 mmol, 6.49 mL, 2.00 eq) and tert-butoxycarbonyl tert-butyl carbonate (7.66 g, 35.10 mmol, 8.06 mL, 1.50 eq). The mixture was stirred at 20 °C for 16 h. The residue was poured into water (300 mL). The aqueous phase was extracted with EtOAc (150 mLx3). The combined organic phase was washed with brine (300 mL), dried with anhydrous Na₂SO₄, filtered and concentrated in vacuum. The residue was purified by column chromatography (SiO₂, PE/EtOAc=100/1 to 80/1) to afford the title compound (17.00 g, used directly).

### Step 2: tert-butyl N-[(3S,5R)-5-[tert-butyl(dimethyl)silyl]oxy-3-piperidy]carbamate

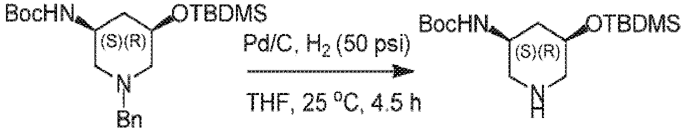

To a solution of tert-butyl N-[(3S, SR)-1-benzyl-5-[tert-butyl (dimethyl) silyl] oxy- 3-piperidyl]carbamate (5.00 g, 11.89 mmol, 1.00 eq) in THF (30.00 mL) was added Pd/C (wet: 10 %, 4 g) under N₂. The suspension was degassed under vacuum and purged with H₂ several times. The mixture was stirred under H₂ (50 psi) at 25 °C for 4.5 h. The mixture was filtered and concentrated to afford the title compound (3.80 g, crude).

### Step 3: benzyl (3S,5R)-3-(tert-butoxycarbonylamino)-5-[tert butyl(dimethyl)silyl] oxy-piperidine-1-carboxylate

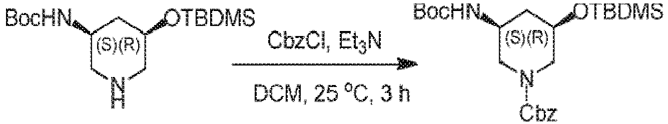

To a mixture of tert-butyl N-[(3S, SR)-5-[tert-butyl(dimethyl)silyl]oxy-3-piperidyl] carbamate (4.00 g, 12.10 mmol, 1.00 eq) and Et₃N (3.67 g, 36.30 mmol, 5.03 mL, 3.00 eq) in DCM (10.00 mL) was added CbzCl (2.48 g, 14.52 mmol, 2.06 mL, 1.20 eq). The mixture was stirred at 25 °C for 3 h. The residue was poured into water (500 mL). The aqueous phase was extracted with EtOAc (200 mLx3). The combined organic phase was washed with brine (500 mL), dried with anhydrous Na₂SO₄, filtered and concentrated in vacuum. The residue was purified by column chromatography (SiO2, PE/EtOAc=10/1) to afford the title compound (4.00 g, 67.59%).

### Step 4: benzyl (3S)-3-(tert-butoxycarbonylamino)-5-oxo-piperidine-1-carboxylate

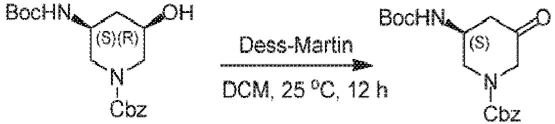

To a solution of benzyl (3S,5R)-3-(tert-butoxycarbonylamino)-5-hydroxy-piperidine -1-carboxylate (200.00 mg, 570.76 umol, 1.00 eq) in DCM (5.00 mL) was added Dess-Martin reagent (484.16 mg, 1.14 mmol, 353.40 uL, 2.00 eq). The mixture was stirred at 25 °C for 12 h. The reaction mixture was filtered and concentrated. The residue was purified by column chromatography (SiO₂, PE/EtOAc=10/1 to 3/1) to afford the title compound (150.00 mg, 60.35%).

### Step 5: Benzyl (5S)-5-(tert-butoxycarbonylamino)-3,3-difluoro-piperidine-1-carboxylate

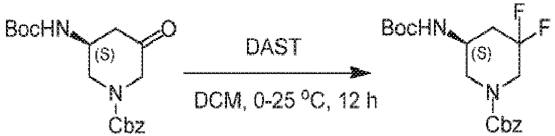

To a solution of benzyl (3S)-3-(tert-butoxycarbonylamino)-5-oxo-piperidine-1-carboxylate (130.00 mg, 373.15 umol, 1.00 eq) in DCM (5.00 mL) was added DAST (483.58 mg, 3.00 mmol, 396.38 uL, 8.04 eq) at 0°C., and then the mixture was warmed to 25 °C and stirred at this temperature for 12 h under N₂. The residue was poured into water (100 mL). The aqueous phase was extracted with EtOAc (50 mLx3). The combined organic phase was washed with brine (100 mL), dried with anhydrous Na₂SO₄, filtered and concentrated in vacuum. The residue was purified by column chromatography (SiO₂, PE/EtOAc=10/1 to 3/1) to afford the title compound (100.00 mg, crude).

### Step 6: tert-butyl (3S)-3-[[4-[6-[3-methyl-1-(2-trimethylsilylethoxymethyl)pyrazol-4-yl]-1H-indol-3-yl]-5-(trifluoromethyl)pyrimidin-2-yl]amino]piperidine-1-carboxylate

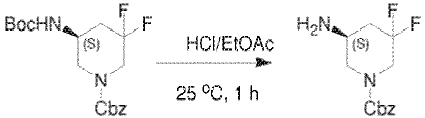

To a solution of benzyl (5S)-5-(tert-butoxycarbonylamino)-3,3-difluoro-piperidine-1-carboxylate (100.00 mg, 269.99 umol, 1.00 eq) in EtOAc (2.00 mL) was added HCl/EtOAc (2.00 mL). The mixture was stirred at 25 °C for 1 h. The mixture was concentrated, the residue was adjusted pH=8 with AMBERLYST(R) A21 to afford the title compound (80.00 mg, crude).

### Step 7: Benzyl (5S)-3,3-difluoro-5-[[4-(1H-indol-3-yl)-5-(trifluoromethyl)pyrimidin-2-yl] amino]piperidine-1-carboxylate

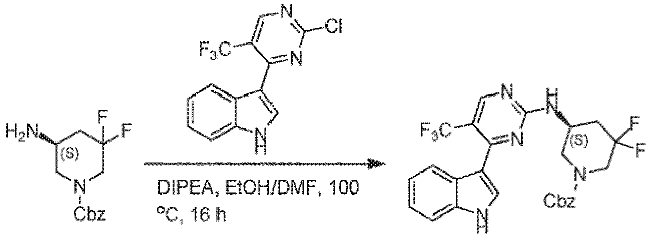

A mixture of benzyl (5S)-5-amino-3,3-difluoro-piperidine-1-carboxylate (70.00 mg, 259.00 umol, 1.00 eq), 3-[2-chloro-5-(trifluoromethyl)pyrimidin-4-yl]-1H-indole (92.51 mg, 310.80 umol, 1.20 eq), DIEA (66.95 mg, 518.00 umol, 90.47 uL, 2.00 eq) in EtOH (3.00 mL) and DMF (1.00 mL) was degassed and purged with N₂ for 3 times, and then the mixture was stirred at 100 °C for 16 hour under N₂ atmosphere. The residue was poured into water (100 mL). The aqueous phase was extracted with EtOAc (50 mL*3). The combined organic phase was washed with brine (100 mL), dried with anhydrous Na₂SO₄, filtered and concentrated in vacuum. The residue was purified by column chromatography (SiO₂, PEIEtOAc--511 to 2:1) to afford the title compound (150.00 mg, 78.46%).

### Step 8: N-[(3S)-5,5-difluoro-3-piperidyl]-4-(1H-indol-3-yl)-5-(trifluoromethyl)pyrimidin-2-amine

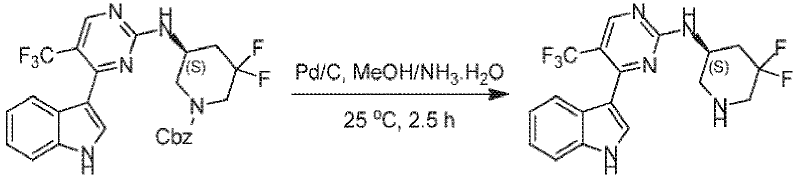

To a solution of benzyl (5S)-3,3-difluoro-5-[[4-(1H-indol-3-yl)-5-(trifluoromethyl) pyrimidin-2-yl]amino]piperidine-1-carboxylate (100.00 mg, 188.15 umol, 1.00 eq) and NH₃.H₂O (13.19 mg, 376.31 umol, 14.49 uL, 2.00 eq) in MeOH (5.00 mL) was added Pd-C (10%, 0.1 g) under N₂. The suspension was degassed under vacuum and purged with H₂ several times. The mixture was stirred under H₂ (15 psi) at 25°C for 2.5 h. The mixture was filtered and concentrated. The residue was purified by prep-HPLC (FA condition) to afford the title compound (9.00 mg, 10.59%).

### Example 66.2-methyl-4-[3-[2-[[(3S)-3-piperidyl] amino]-5-(trifluoromethyl) pyrimidin-4-yl]-1H-indol-6-yl] butan-2-ol (Compound 242).

### Step 1: tert-butyl (3S)-3-[[4-[1-(benzenesulfonyl)-6-[(E)-3-hydroxy-3-methyl-but-1-enyl] indol-3-yl]-5-(trifluoromethyl)pyrimidin-2-yl]amino]piperidine-1-carboxylate

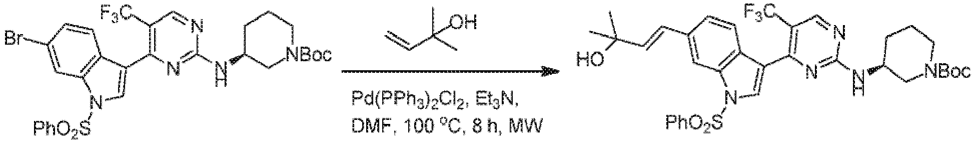

To a solution of tert-butyl (3S)-3-[[4-[1-(benzenesulfonyl)-6-bromo-indol-3-yl]-5-(trifluoromethyl)pyrimidin-2-yl]amino]piperidine-1-carboxylate (500.00 mg, 734.72 umol) and 2-methylbut-3-en-2-ol (316.41 mg, 3.67 mmol) in DMF (10 mL) was added Et₃N (148.69 mg, 1.47 mmol) and Pd(PPh₃)₂Cl₂ (51.57 mg, 73.47 umol) at 20 °C. The suspension was degassed under vacuum and purged with N₂ several times. The mixture was heated to 100 °C and stirred for 8 h under N₂ under microwave. The reaction mixture was quenched by water (50 mL) and extracted with EtOAc (50 mL * 3). The combined organic layers were dried over Na₂SO₄, filtered and concentrated under reduced pressure to give a residue. The residue was purified by column chromatography (SiO₂, PE/EtOAc=10/1 to 3:1) to afford the title compound (320.00 mg, 57.16%) as a yellow solid.

### Step 2: tert-butyl (3S)-3-{[4-[1-(benzenesulfonyl)-6-(3-hydroxy-3-methyl-butyl)indol- 3-ylj- 5-(trifluoromethyl)pyrimidin-2-yl]amino]piperidine-1-carboxylate

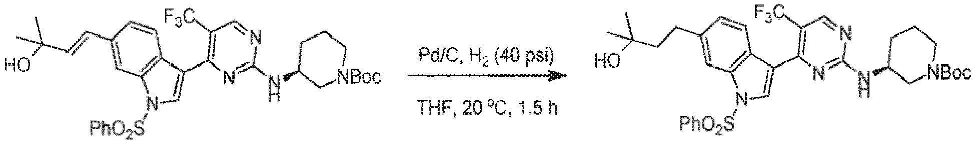

To a solution of tert-butyl (3S)-3-[[4-[1-(benzenesulfonyl)-6-[(E)-3-hydroxy- 3-methyl-but-1-enyl]indol-3-yl]-5-(trifluoromethyl)pyrimidin-2-yl]amino]piperidine-1-carboxylate (250.00 mg, 364.56 umol) in THF (10 mL) was added wet Pd-C (10 %, 0.25 g) under N₂. The suspension was degassed under vacuum and purged with H₂ several times. The mixture was stirred under H₂ (40 psi) at 20 °C for 1.5 h. The mixture was filtered and concentrated under reduced pressure to give the residue. The residue was purified by column chromatography (SiO₂, PE/EtOAc=12/1 to 4:1) to afford the title compound (250.00 mg, crude) as a white solid.

### Step 3: tert-butyl(3S)-3-[[4-[6-(3-hydroxy-3-methyl-butyl)-1H-indol-3-yl]-5-(trifluoromethyl) pyrimidin-2-yl]amino]piperidine-1-carboxylate

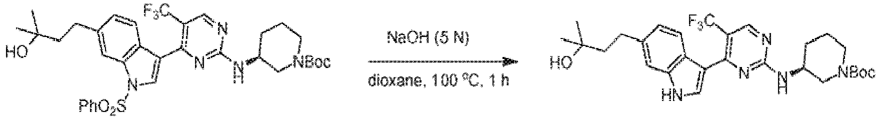

Tert-butyl (3S)-3-[[4-[1-(benzenesulfonyl)-6-(3-hydroxy-3-methyl-butyl)indol-3-yl]- 5-(trifluoromethyl)pyrimidin-2-yl]amino]piperidine-1-carboxylate (250.00 mg, 363.49 umol) was dissolved in dioxane (5 mL) and a solution of NaOH (72.70 mg, 1.82 mmol) in H₂O (1 mL) was added into the mixture at 20 °C. The mixture was heated to 100 °C and stirred for 1 h. The reaction mixture was quenched by water (50 mL) and extracted with EtOAc (40 mL * 3). The combined organic layers were dried over Na₂SO₄, filtered and concentrated under reduced pressure to afford the title compound (170.00 mg, 76.01%) as a yellow solid.

### Step 4: 2-methyl-4-[3-[2-[[(3S)-3-piperidyl]amino]-5-(trifluoromethyl)pyrimidin-4-yl]-1H-indol-6yl]butan-2-ol

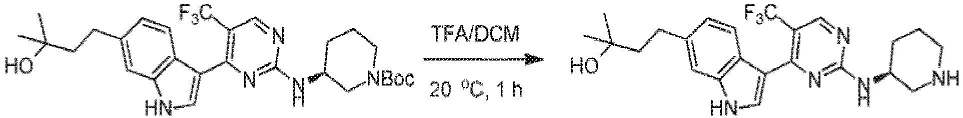

To a solution of tert-butyl (3S)-3-[[4-[6-(3-hydroxy-3-methyl-butyl)-1H-indol-3-yl]- 5-(trifluoromethyl)pyrimidin-2-yl]amino]piperidine-1-carboxylate (150.00 mg, 273.92 umol) in DCM (10 mL) was drop added TFA (1 mL) at 20 °C. The mixture was stirred for 1 h. The mixture was adjusted to pH-7 with saturated NaHCO₃. The organic phase was separated and dried by Na₂SO₄, filtered and concentrated under reduced pressure to give a residue. The residue was purified by prep-HPLC (FA) to afford the title compound (38.90 mg, 28.60%) as a white solid.

### Example 67. Benzyl (5S)-S-[[4-(1H-indol-3-yl)-5-(trifluoromethyl) pyrimidin-2-yl] amino]-3, 3-dimethyl-pipertdine-1-carboxylate (Compound 243)

### Step 1: methyl (2S)-5-oxopyrrolidine-2-carboxylate

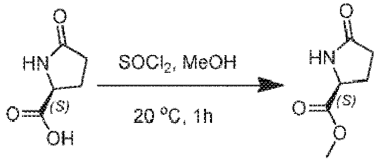

To a solution of (2S)-5-oxopyrrolidine-2-carboxylic acid (10.00 g, 77.45 mmol, 1.00 eq) in MeOH (100.00 mL) was added SOCl₂ (18.43 g, 154.91 mmol, 11.24 mL, 2.00 eq). The mixture was stirred at 20 °C for 1 h. The reaction mixture was concentrated. The residue was diluted with EtOAc (250 mL) and TEA (20 mL). The solid formed and filtered. The filtrate was concentrated to afford the title compound (13.00 g, crude) as a yellow oil. The crude product was used to next step without further purification.

### Step 2: O1-tert-butyl O2-methyl (2S)-5-oxopyrrolidine-1, 2-dicarboxylate

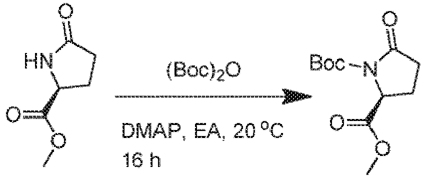

To a solution of methyl (2S)-5-oxopyrrolidine-2-carboxylate (13.00 g, 90.82 mmol, 1.00 eq) and DMAP (1.33 g, 10.90 mmol, 0.12 eq) in EtOAc (150.00 mL) was added dropwise tert-butoxycarbonyl tert-butyl carbonate (25.77 g, 118.07 mmol, 27.12 mL, 1.30 eq). The mixture was stirred at 20 °C for 16 h. The reaction mixture was washed with HCl (0.5 M, 50 mL), Sat. NaHCO₃ (150 mL), brine (500 mL), dried over Na₂SO₄, filtered and concentrated under reduced pressure to give a residue. The residue was purified by re-crystallization from MTBE (250 mL) to afford the title compound (11.00 g, 45.22 mmol, 49.79% yield, 100% purity) as a red solid

### Step 3: O1-tert-butyl d2-methyl (2S)-4,4-dimethyl-5-oxo-pyrrolidine-1,2-dicarboxylate

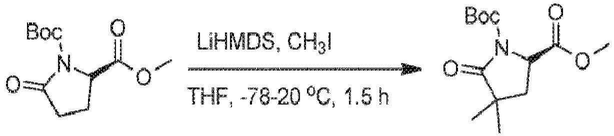

To a solution of O1-tert-butyl O2-methyl (2S)-5-oxopyrrolidine-1, 2-dicarboxylate (14.00 g, 57.55 mmol, 1.00 eq) in THF (400.00 mL) was added dropwise LiHMDS (1 M, 120.86 mL, 2.10 eq) at -78 °C under N₂ atmosphere. After addition, the mixture was stirred at this temperature for 0.5 h, and then iodomethane (24.51 g, 172.65 mmol, 10.75 mL, 3.00 eq) was added dropwise at - 78 °C under N₂ atmosphere. The resulting mixture was stirred at 20 °C for 1 h. The reaction mixture was diluted with saturated aqueous NH₄Cl (400 mL) and extracted with EtOAc (200 mLx3). The combined organic layers were washed with brine (600 mL*2), dried over Na₂SO₄, filtered and concentrated under reduced pressure to give a residue. The residue was purified by column chromatography (SiO₂, PE/EtOAc=20:1 to 10:1) to afford the title compound (6.00 g, 16.37 mmol, 28.44% yield, 74% purity) as a yellow solid.

### Step 4: tert-butyl N-[(1S)-4-hydroxy-1-(hydroxymethyl)-3,3-dimethyl-butyl]carbamate

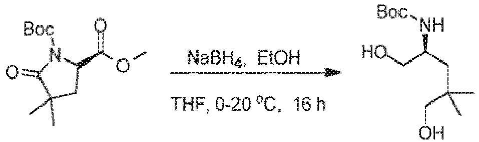

To a solution of O1-tert-butyl O2-methyl (2S)-4, 4-dimethyl-5-oxo-pyrrolidine-1, 2-dicarboxylate (5.24 g, 19.31 mmol, 1.00 eq) in THF (10.00 mL) was added batchwise NaBH₄ (2.19 g, 57.94 mmol, 3.00 eq) at 0 °C under N₂ atmosphere. After addition, EtOH (9.81 g, 213.03 mmol, 12.42 mL, 11.03 eq) was added dropwise at 0 °C. The resulting mixture was stirred at 20 °C for 16 h. The reaction mixture was diluted with saturated aqueous NH₄Cl (150 mL) and extracted with EtOAc (80 mL*3). The combined organic layers were washed with brine (250 mLx2), dried over Na₂SO₄, filtered and concentrated under reduced pressure to afford the title compound (6.46 g, crude) as a yellow oil.

### Step 5: [(2S)-2-(tert-butoxycarbonylamino)-4, 4-dimethyl-5-methylsulfonyloxy-pentyl] methanesulfonate

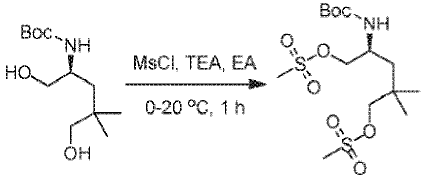

To a solution of tert-butyl N-[(1S)-4-hydroxy-1-(hγdrnaymethyl)-3,3 -dimethyl-butyl]carbamate (6.46 g, 26.12 mmol, 1.00 eq) and TEA (10.57 g, 104.48 mmol, 14.48 mL, 4.00 eq) in EtOAc (60.00 mL) was added dropwise MsCl (8.98 g, 78.36 mmol, 6.07 mL, 3.00 eq) at 0 °C. The resulting mixture was stirred at 20 °C for 1 h. The reaction mixture was poured into water 150 mL, and then extracted with EtOAc (20 mL*3). The combined organic layers were washed with brine (200 mL), dried over Na₂SO₄, filtered and concentrated under reduced pressure to afford the title compound (10.41 g, crude) as yellow oil.

### Step 6: tert-butyl N-[(3S)-1-benzyl-5, 5-dimethyl-3-piperidyl] carbamate

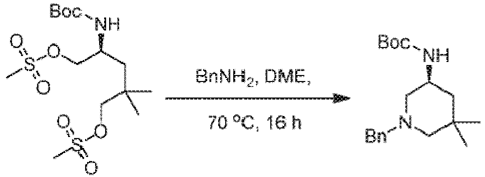

A mixture of [(2S)-2-(tert-butoxycarbonylamino)-4, 4-dimethyl-5-methylsulfonyloxy-pentyl] methanesulfonate (10.41 g, 25.80 mmol, 1.00 eq), phenylmethanamine (8.85 g, 82.56 mmol, 9.03 mL, 3.20 eq) in DME (100.00 mL) was stirred at 70 °C for 16 h. The reaction mixture was diluted with water 500 mL and extracted with EtOAc (200 mLx3). The combined organic layers were washed with brine (500 mL*2), dried over Na₂SO₄, filtered and concentrated under reduced pressure to give a residue. The residue was purified by column chromatography (SiO₂, PE/EtOAc=40:1 to 30:1) to afford the title compound (1.00 g, 2.67 mmol, 10.35% yield, 85% purity) as a yellow oil.

### Step 7: tert-butyl N-[(3S)-5, 5-dimethyl-3-piperidyl] carbamate

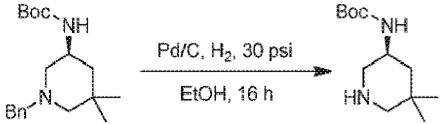

To a solution of tert-butyl N-[(3S)-1-benzyl-5, 5-dimethyl-3-piperidyl] carbamate (1.00 g, 3.14 mmol, 1.00 eq) in EtOH (10.00 mL) was added Pd-C (10%, 1 g) under N₂. The suspension was degassed under vacuum and purged with H₂ 3 times. The mixture was stirred under H₂ (30 psi) at 20 °C for 16 h. The reaction mixture was filtered and the filtrate was concentrated to afford the title compound (500.00 mg, crude) as yellow oil.

### Step 8: benzyl (5S)-5-(tert-butoxycarbonylamino)-3, 3-dimethyl-piperidine-1-carboxylate

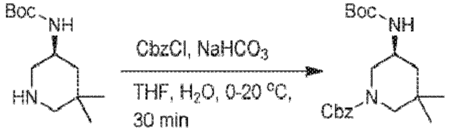

To a solution of tert-butyl N-[(3S)-5, 5-dimethyl-3-piperidyl] carbamate (500.00 mg, 2.19 mmol, 1.00 eq) and NaHCO₃ (1.29 g, 15.33 mmol, 596.24 uL, 7.00 eq) in THF (5.00 mL) and H₂O (5.00 mL) was added dropwise and benzyl carbonochloridate (560.34 mg, 3.29 mmol, 466.95 uL, 1.50 eq). The mixture was stirred at 20 °C for 30 min. The reaction mixture was diluted with water 100 mL and extracted with EtOAc (50 mL*3). The combined organic layers were washed with brine (200 mL*2), dried over Na₂SO₄, filtered and concentrated under reduced pressure to give a residue. The residue was purified by column chromatography (SiO₂, PE/EtOAc=20:1 to 5:1) to afford the title compound (740.00 mg, 1.37 mmol, 62.56% yield, 67% purity) as a yellow oil. (Note: Combined purification with another bacth, scale: 60 mg).

### Step 9: benzyl (5S)-5-amino-3, 3-dimethyl-piperidine-1-carboxylate

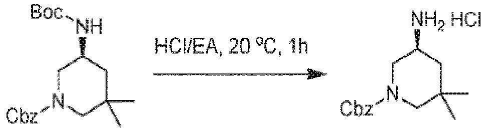

A mixture of benzyl (5S)-5-(tert-butoxycarbonylamino)-3, *3-dimethyl-piperidine-1-carboxylate* (740.00 mg, 2.04 mmol, 1.00 eq) and HCl/EtOAc (4 M, 5.00 mL) was stirred at 20 °C for 1 h. The reaction mixture was concentrated directly to afford the title compound (525 mg) as a yellow solid.

### Step 10: benzyl (5S)-5-[[4-(1H-indol-3-yl)-5-(trifluoromethyl) pyrimidin-2-yl] amino]-3, 3-dimethyl-piperidine-1-carboxylate

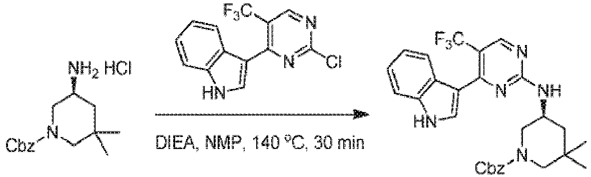

A mixture of 3-[2-chloro-5-(trifluoromethyl)pyrimidin-4-yl]-1H-indole (497.09 mg, 1.67 mmol, 1.00 eq), benzyl (5S)-5-amino-3,3-dimethyl-piperidine-1-carboxylate (500.00 mg, 1.67 mmol, 1.00 eq, HCl) and DIEA (647.49 mg, 5.01 mmol, 874.99 uL, 3.00 eq) in NMP (5.00 mL) was stirred at 140 °C for 30 min. The reaction mixture was diluted with water 100 mL and extracted with EtOAc (50 mLx3). The combined organic layers were washed with brine (200 mL*2), dried over Na₂SO₄, filtered and concentrated under reduced pressure to give a residue. The residue was purified by column chromatography (SiO₂, PE/EtOAc=10:1 to 5:1) to afford the title compound (300.00 mg, 561.55 umol, 33.63% yield, 98% purity) as a yellow solid. (Note; Combined purification with another batch. Scale: 20 mg).

### Step 11: N-[(3S)-5, 5-dimethyl-3-piperidyl]-4-(1H-indol-3-yl)-5-(trifluoromethyl) pyrimidin-2-amine

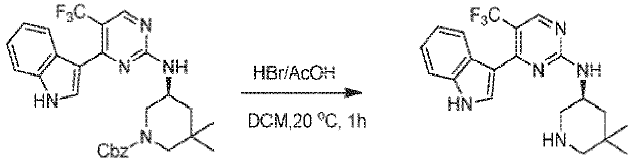

To a solution of benzyl (5S)-5-[[4-(1H-indol-3-yl)-5- (trifluoromethyl) pyrimidin-2-yl]amino]-3, 3-dimethyl- piperidine-1-carboxylate (200.00 mg, 382.01 umol, 1.00 eq) in DCM (3.00 mL) was added hydrogen bromide (883.09 mg, 3.82 mmol, 592.68 uL, 35% purity, 10.00 eq). The mixture was stirred at 20 °C for 1 h. The reaction mixture was poured into MTBE (10 mL), the solid formed and filtered to collect cake as a yellow solid (200 mg). The residue was purified by prep-HPLC (FA condition) to afford the title compound (110.43 mg, 253.61 umol, 66.39% yield, FA) as a white solid. (Note: Combined purification with another batch. Scale: 100 mg).

### Example 68. 4-[6-(1, 5-dimethyl-1, 2, 4-triazol-3-yl)-1H-indol-3-yl]-N-[(3S)-3-piperidyl]-5-(trifluoromethyl) pyrimidin-2-amine (Compound 245).

### Step 1: tert-butyl (3S)-3-[[4-[1-(benzenesulfonyl)-6-(1,5-dimethyl-1,2,4-triazol-3-yl)indol-3-yl]-5-(trifluoromethyl)pyrimidin-2-yl]amino]piperidine-1-carboxylate

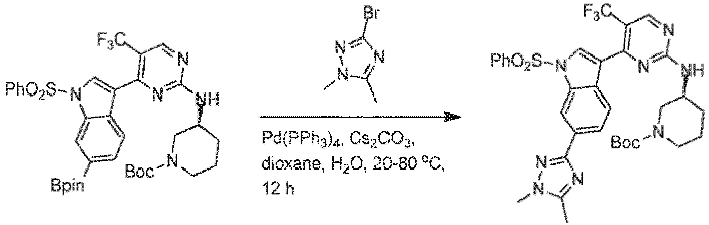

To a mixture of tert-butyl (3S)-3-[[4-[1-(benzenesulfonyl)-6-(4,4,5,5-tetramethyl- 1,3,2-dioxaborolan-2-yl)indol-3-yl]-5-(trifluoromethyl)pyrimidin-2-yl]amino]piperidine-1-carboxylate (200.00 mg, 274.88 umol, 1.00 eq) and 3-bromo-1,5- dimethyl-1,2,4-triazole (53.22 mg, 302.36 umol, 1.10 eq) in dioxane (5.00 mL) and H₂O (1.00 mL) was added Pd(PPh₃)₄ (63.53 mg, 54.98 umol, 0.20 eq) and Cs₂CO₃ (179.12 mg, 549.75 umol, 2.00 eq) in one portion at 20 °C under N₂. The mixture was stirred at 80 °C for 12 h. The residue was poured into water (10 mL) and extracted with EtOAc (10 mLx2). The combined organic phase was washed with brine (10 mLx2), dried with anhydrous Na₂SO₄, filtered and concentrated in vacuum. The residue was purified by prep-HPLC (TFA) to afford the title compound (130.00 mg, 58.33%, TFA) as a yellow solid.

### Step 2: tert-butyl (3S)-3-[[4-[6-(1,5-dimethyl-1,2,4- triazol-3-yl)-1H-indol-3-yl]-5-(trifluoromethyl)pyrimidin-2-yl]amino]piperidine-1-carboxylate

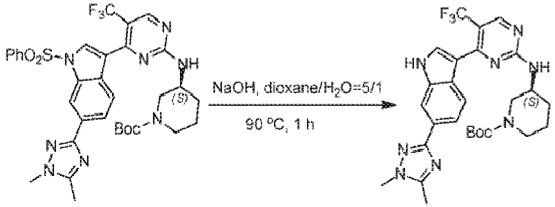

To a solution of tert-butyl (3S)-3-[[4-[1-(benzenesulfonyl)-6-(1,5-dimethyl-1,2,4-triazol-3-yl)indol-3-yl]-5-(trifluoromethyl)pyrimidin-2-yl]amino]piperidine-1-carboxylate (150.00 mg, 215.29 umol, 1.00 eq) in dioxane (5.00 mL) was added a solution of NaOH (43.06 mg, 1.08 mmol, 5.00 eq) in H₂O (1.00 mL) at 20 °C. The mixture was heated to 90 °C and stirred for 1 h. The reaction mixture was quenched by water (20 mL) and extracted with EtOAc (40 mL x3). The combined organic layers were dried over Na₂SO₄, filtered and concentrated under reduced pressure to give a residue. The residue was purified by column chromatography (SiO₂, DCM/MeOH=100% to 60:1) to afford the title compound (110.00 mg, 73.44%) as a yellow solid.

### Step 3: 4-[6-(1, 5-dimethyl-1,2,4-triazol-3-yl)-1H-indol-3-yl]-N-[(3S)-3-piperidyl]-5-(trifluoromethyl)pyrimidin-2-amine

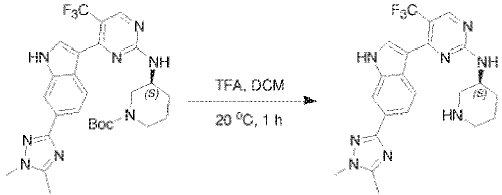

To a solution of tert-butyl (3S)-3-[[4-[6-(1,5-dimethyl-1,2,4-triazol-3-yl)-1H- indol-3-yl]-5-(trifluoromethyl)pyrimidin-2-yl]amino]piperidine-1-carboxylate (90.00 mg, 129.36 umol, 1.00 eq) in DCM (8 mL) was drop added TFA (1 mL) for 1 h at 20 °C. The mixture was concentrated under reduced pressure to give a residue. The residue was purified by prep-HPLC (FA) to afford the title compound (31.40 mg, 47.42%, FA) as a white solid.

### Example 69. 4-(6-imidazol-1-yl-1H-indol-3-yl)-N-[(3S)-3-piperidyl]-5-(trifluoromethyl) pyrimidin-2-amine (Compound 248).

### Step 1: tert-butyl 6-bromoindole-1-carboxylate

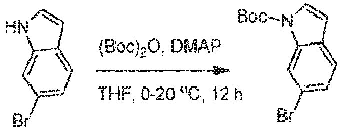

To a solution of 6-bromo-1H-indole (5.00 g, 25.50 mmol, 1.00 eq), DMAP (373.84 mg, 3.06 mmol, 0.12 eq) in THF (50.00 mL) was added dropwise tert-butoxycarbonyl tert-butyl carbonate (7.23 g, 33.15 mmol, 7.61 mL, 1.30 eq) at 0°C. The resulting mixture was stirred at 20°C for 12 h. The reaction mixture was poured into aq. HCl (200 mL, 0.5 M), and extracted with EtOAc (75 mL x3). The combined organic layers were washed with Sat. NaHCO₃ (200 mL) and brine (300 mLx3), dried over Na₂SO₄, filtered and concentrated under reduced pressure to give a residue. The residue was purified by column chromatography (SiO₂, PE/EtOAc=100:1 to 10:1) to afford the title compound (6.50 g, 21.95 mmol, 86.07% yield) as a white solid.

### Step 2: 6-imidazol-1-yl-1H-indole

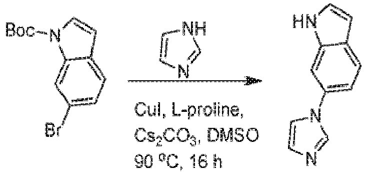

A mixture of tert-butyl 6-bromoindole-1-carboxylate (6.50 g, 21.95 mmol, 1.00 eq), imidazole (2.99 g, 43.90 mmol, 2.00 eq), Cs₂CO₃ (14.30 g, 43.90 mmol, 2.00 eq), CuI (417.99 mg, 2.20 mmol, 0.10 eq) L-PROLINE (3.79 g, 32.93 mmol, 1.50 eq) in DMSO (50.00 mL) was degassed and purged with N₂ for 3 times, and then the mixture was stirred at 90 °C for 16 h under N₂ atmosphere. The reaction mixture was poured into water 500 mL, and then extracted with EtOAc (150 mL x3). The combined organic layers were washed with brine (700 mL), dried over Na₂SO₄, filtered and concentrated under reduced pressure to give a residue. The residue was purified by column chromatography (SiO₂, PF/EtOAc=5:1 to 1:3) to afford the title compound (700.00 mg, 3.82 mmol, 17.40% yield) as an offwhite solid.

### Step 3: 3-[2-chloro-5-(trifluoromethyl)pyrimidin-4-yl]-6-imidazol-1-yl-1H-indole

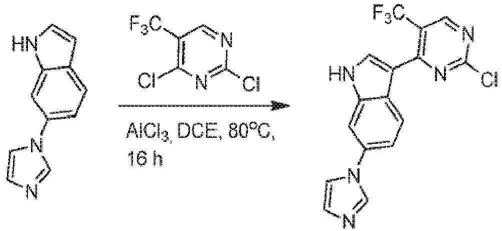

To a solution of 2, 4-dichloro-5-(trifluoromethyl) pyrimidine (1.42 g, 6.55 mmol, 2.00 eq) in DCE (5.00 mL) was added AlCl₃ (873.36 mg, 6.55 mmol, 357.93 uL, 2.00 eq) at 80 °C. After addition, the mixture was stirred at this temperature for 0.5 h, and then frimidazol-1-yl-1H-indole (600.00 mg, 3.27 mmol, 1.00 eq) was added. The resulting mixture was stirred at 80 °C for 15.5 h. The reaction mixture was quenched by addition Sat. NaHCO₃, and then extracted with EtOAc (50 mL x3), dried over Na₂SO₄, filtered and concentrated under reduced pressure to give a residue. The residue was purified by prep-HPLC (TFA condition). It was concentrated, the residue was treated with Sat. NaHCO₃ (50 mL) and extracted with EtOAc (30 mLx3), dried over Na₂SO₄, filtered and concentrated to afford the title compound (100.00 mg, 263.94 umol, 8.07% yield, 96% purity) as a yellow solid.

### Step 4: tert - butyl (3S)-3-[[4-(6-imidazol-1-yl-1H-indol-3-yl)-5-(trifluoromethyl) pyrimidin-2-yl] amino] piperidine-1- carboxylatee

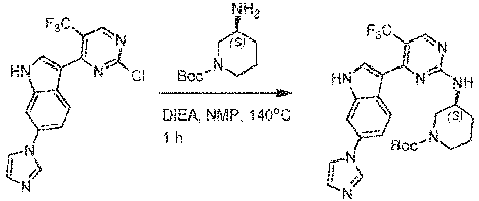

A mixture of 3-[2-chloro-5-(trifluoromethyl) pyrimidin-4-yl]-6-imidazol-1-yl-1H-indole (80.00 mg, 219.95 umol, 1.00 eq), tert butyl (3S)-3-aminopiperidine-1-carboxylate (57.27 mg, 285.93 umol, 1.30 eq), DIEA (142.13 mg, 1.10 mmol, 192.07 uL, 5.00 eq) in NMP (2.00 mL) was stirred at 140 °C for 1 h. The reaction mixture was poured into water 50 mL, and then extracted with EtOAc (25 mL x3). The combined organic layers were washed with brine (100 mL), dried over Na₂SO₄, filtered and concentrated to afford the title compound (200.00 mg, crude) as brown oil. It was used for next step directly.

### Step 5: 4-(6-imidazol-1-yl-1H-indol-3-yl)-N-[(3S)-3-piperidyl]-5-(trifluoromethyl) pyrimidin-2-amine

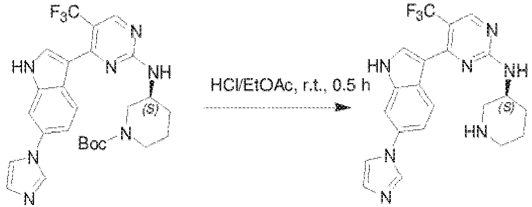

A mixture of tert-butyl (3S)-3-[[4-(6-imidazol-1-yl-1H-indol-3-yl)-5-(trifluoromethyl) pyrimidin-2-yl]amino] piperidine-1- carboxylate (80.00 mg, 151.65 umol, 1.00 eq) in HCl/EtOAc (4 M, 1.90 mL, 50.00 eq) was stirred at 20 °C for 0.5 h. It was concentrated. The residue was purified by prep-HPLC (FA condition) to afford the title compound (8.40 mg, 15.97umol, 10.53% yield, 90% purity, FA) as a yellow solid.

### Example 70. 3-[2-[[(3S)-3-piperidyl] amino]-5-(trifluoromethyl) pyrimidin-4-yl]-1H-pyrrolo [2, 3-b] pyridine-6-carbonitrile (Compound 251).

### Step 1:1H-pyrrolo [2, 3-b] pyridine-6-carbonitrile

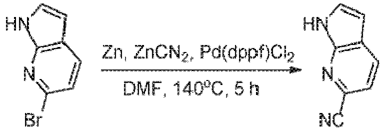

A mixture of 6-bromo-1H-pyrrolo[2,3-b]pyridine (4.00 g, 20.30 mmol, 1.00 eq), Zn (132.75 mg, 2.03 mmol, 0.10 eq), Zn(CN)₂(1.67 g, 14.21 mmol, 902.05 uL, 0.70 eq) and Pd(dppf)Cl₂.CH₂Cl₂ (828.95 mg, 1.02 mmol, 0.05 eq) in DMF (10.00 mL) was degassed and purged with N₂ for 3 times, and then the mixture was stirred at 140°C for 5 h under N₂ atmosphere. The reaction mixture was diluted with EtOAc (50 mL) and washed with aq. saturated bicarbonate solution (100 mL) and brine (100 mL*2), dried over sodium sulfate, filtered and evaporated. The residue was purified by column chromatography (SiO₂, PE/EtOAc=10:1 to 3:1) to afford the title compound (1.50 g, 8.91 mmol, 43.88% yield, 85% purity) as a white solid.

### Step 2: 3-bromo-1H-pyrrolo [2, 3-b] pyridine-6-carbonitrile

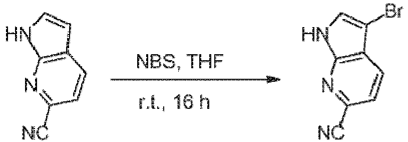

To a solution of 1H-pyrrolo [2, 3-b] pyridine-6-carbonitrile (1.50 g, 10.48 mmol, 1.00 eq) in DCM (3.00 mL) was added NBS (2.24 g, 12.57 mmol, 1.20 eq) at 0°C. The mixture was stirred at 20°C for 16 h. It was concentrated. The residue was washed with PE (50 mL) and filtered to afford the title compound (3.00 g, 6.76 mmol, 64.46% yield, 50% purity) as a yellow solid. It was used for next step directly.

### Step 3: 1-(benzenesulfonyl)-3-bromo-pyrrolo

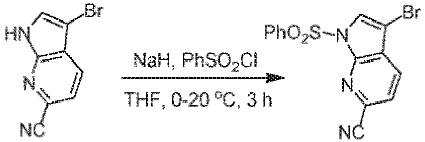

To a solution of 3-bromo-1H-pyrrolo[2,3-b]pyridine-6-carbonitrile (3.50 g, 15.76 mmol, 1.00 eq) in THF (10.00 mL) was added NaH (630.40 mg, 15.76 mmol, 60% purity, 1.00 eq) at 0°C. After addition, the mixture was stirred at this temperature for 0.5 h, and then benzenesulfonyl chloride (3.62 g, 20.49 mmol, 2.62 mL, 1.30 eq) was added dropwise at 0°C. The resulting mixture was stirred at 20°C for 2.5 h. The reaction mixture was quenched by addition water 50 mL, and then extracted with EtOAc (20 mL * 3). The combined organic layers were washed with brine (100 mL), dried over Na₂SO₄, filtered and concentrated under reduced pressure to give a residue. The residue was purified by column chromatography (SiO₂, PE/EtOAc=10:1 to 3:1) to afford the title compound (2.00 g, 5.52 mmol, 35.04% yield) as a yellow solid.

### Step 4: 1-(benzenesulfonyl)-3-(4,4,5,5-tetramethyl-1,3,2-dioxaborolan-2-yl)pyrrolo[2,3-b]pyridine-6-carbonitrile

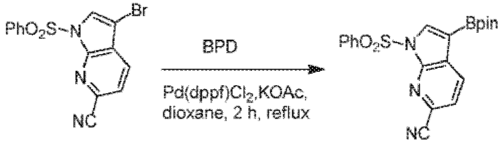

A mixture of 1-(benzenesulfonyl)-3-bromo-pyrrolo[2,3-b]pyridine-6-carbonitrile (200.00 mg, 552.18 umol, 1.00 eq), 4,4,5,5- tetramethyl-2-(4,4,5,5-tetramethyl-1,3,2-dioxaborolan-2-yl)-1,3,2-dioxabomlane (210.33 mg, 828.27 umol, 1.50 eq), Pd(dppf)Cl₂ (40.40 mg, 55.22 umol, 0.10 eq), KOAc (108.38 mg, 1.10 mmol, 2.00 eq) in dioxane (5.00 mL) was degassed and purged with N₂ for 3 times, and then the mixture was stirred at 80°C for 2 h under N₂ atmosphere. The reaction mixture was poured into water 50 mL, and then extracted with EtOAc (20 mL * 3). The combined organic layers were washed with brine (150 mL), dried over Na₂SO₄, filtered and concentrated under reduced pressure to afford the title compound (200 mg, crude). It was used for next step directly.

### Step 5: tert-butyl (3S)-3-[[4-[1-(benzenesulfonyl)-6-cyano-pyrrolo [2, 3-b]pyridin-3-yl]-5-(trifluoromethyl pyrimidin-2-yl] amino] piperidine-1-carboxylate

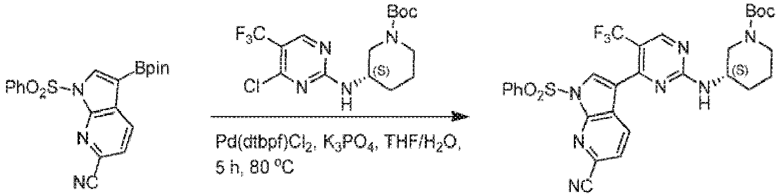

A mixture of 1-(benzenesulfonyl)-3-(4,4,5,5-tetramethyl-1,3,2-dioxaborolan-2-yl)pyrrolo[2,3-b]pyridine-6-carbonitrile (200.00 mg, 488.67 umol, 1.00 eq), tert-butyl (3S)-3-[[4-chloro-5-(trifluoromelhyl)pyrimidin-2-yl]amino]piperidine-1-carboxylate(186.08 mg, 488.67 umol, 1.00 eq), K₃PO₄ (207.46 mg, 977.35 umol, 2.00 eq), ditert- butyl (cyclopentyl) phosphane;dichloropalladium;iron (31.85 mg, 48.87 umol, 0.10 eq) in THF (5.00 mL) and H₂O (1.00 mL) was degassed and purged with N₂ for 3 times,and then the mixture was stirred at 80 °C for 5 h under N₂ atmosphere. The reaction mixture was poured into water 50 mL, and then extracted with EtOAc (20 mL * 3). The combined organic layers were washed with brine (150 mL), dried over Na₂SO₄, filtered and concentrated under reduced pressure to give a residue. The residue was purified by column chromatography (SiO₂, PE/EtOAc=10:1 to 3:1) to afford the title compound (150.00 mg, 215.09 umol, 44.02% yield, 90% purity) as a brown solid.

### Step 6: tert-butyl (3S)-3-[[4-(6-cyano-1H-pyrrolo [2, 3-bpyridin-3-yl)-5-(trifluoromethyl) pyrimidin-2-yl] amino] piperidine-1-carboxylate

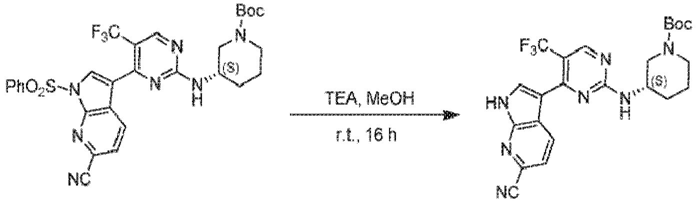

A mixture of tert-butyl (3S)-3-[[4-[1-(benzenesulfonyl)-6-cyano-pyrrolo [2, 3-b] pyridin-3-yl]-5-(trifluoromethyl) pyrimidin-2-yl] amino] piperidine-1-carboxylate (120.00 mg, 191.19 umol, 1.00 eq), TEA (96.73 mg, 955.96 umol, 132.51 uL, 5.00 eq) in MeOH (2.00 mL) was stirred at 20 °C for 16 h. It was concentrated to afford the title compound (150 mg, crude) as a yellow solid (Note: Combined purification with another batch. SM scale: 10 mg). It was used for next step directly.

### Step 7: 3-[2-[[(3S)-3-piperidyl] amino]-5-(trifluoromethyl)pyrimidin-4-yl]-1H-pyrrolo [2, 3-b] pyridine-6-carbonitrile

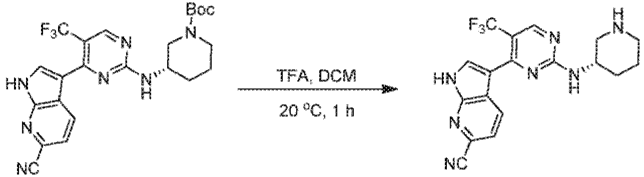

To a solution of tert-butyl (3S)-3-[[4-(6-cyano-1H-pyrrolo [2, 3-b] pyridin-3-yl)-5-*(trifluoromethyl)* pyrimidin-2-yl] amino] *piperidine-1-carboxylate* (140.00 mg, 287.19 umol, 1.00 eq) in DCM (2.00 mL) was added TFA (327.46 mg, 2.87 mmol, 212.64 uL, 10.00 eq). The mixture was stirred at 20 °C for 1 h. It was concentrated. The residue was purified by prep-HPLC (FA condition) to afford the title compound (22.7 mg, FA salt, 99% purity) was obtained as a white solid (Note: Combined purification with another batch. SM scale: 10 mg).

### Example 71. N-[(3R,5S)-5-[[4-(7-methylsulfonyl-1H-indol-3-yl)-5-(trifluoromethyl)pyrimidin-2-yl]amino]-3-piperidyl]propanainide (Comopund 254) and N-[(3S,5R)-5-[[4-(7-methylsulfonyl-1H-indol-3-yl)-5-(trifluoromethyl)pyrimidin-2-yl]amino]-3-piperidyl]propanamide (Compound 259).

### Step 1: 2-[[3-[2-chloro-5-(trifluoromethyl) pyrimidin-4-yl]-7-methylsulfonyl-indol-1-yl]methoxy]ethyl-trimethyl-silane

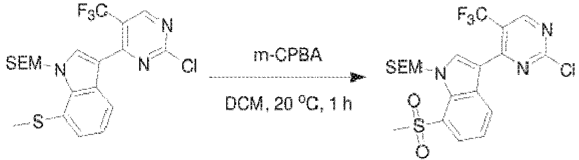

To a stirred solution of 3-(2-Chloro-5-trifluoromethyl-pyrimidin-4-yl)-7-methylsulfanyl-1-(2-trimethylsilanyl-ethoxymethyl)-1H-indole (250.00 mg, 527.42 umol, 1.00 eq) in DCM (8 mL) was added m-CPBA (213.17 mg, 1.05 mmol, 85% purity, 1.99 eq). The mixture was stirred for 1 h at 20 °C. The reaction mixture was washed by NaHCO₃ (20 mL* 2), H₂O and extracted with EtOAc (30 mL * 3). The combined organic layers were dried over Na₂SO₄, filtered and concentrated under reduced pressure to give a residue. The residue was purified by column chromatography (SiO2, PE: EtOAc = 15:1) to afford the title compound (260.00 mg, 87.68% yield) as a light yellow oil.

### Step 2: tert-butyl (3R,5S)-3-amino-5-[[4-[7- methylsulfonyl-1-(2-trimethylsilylethoxymethyl)indol-3-yl]-5-(trifluoromethyl)pyrimidin-2-yl]amino]piperidine-1-carboxylate

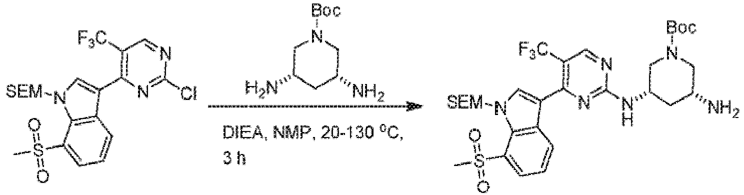

To a stirred solution of 2-[[3-[2-chlrnn-5-(trifluoromethyl)pyrimidin-4-yl]-7- methylsulfonyl-iadol-1-yl]methoxyjethyl-trimethyl-silane (150.00 mg, 296.44 umol, 1.00 eq) and 2-[[3-[2-chloro-5-(trifluoromethyl)pyrimidin-4-yl]-7-methylsulfonyl-indol-1-yl]methoxy]ethyl-trimethyl-silane (150.00 mg, 296.44 umol, 1.00 eq) in NMP (1.50 mL) was added DIPEA (319.26 mg, 1.48 mmol, 431.44 uL, 5.00 eq) at 20 °C. Then the mixture was heated to 130 °C and stirred for 3 h under N₂. The reaction mixture was quenched by water (50 mL) and extracted with EtOAc (40 mL * 3). The combined organic layers were dried over Na₂SO₄, filtered and concentrated under reduced pressure to give a residue. The residue was purified by column chromatography (SiO₂, DCM: MeOH = 100:1-50:1) to afford the title compound (110.00 mg, 43.35% yield) as a white solid.

### Step 3: tert-butyl (3S,5R)-3-[[4-[7-methylsulfonyl-1-(2-trimethylsilylethoxymethyl)indol-3-yl]-5-(trifluoromethyl)pyrimidin-1-yl]amino]-5-(propanoylamino)piperidine-1-carboxylate

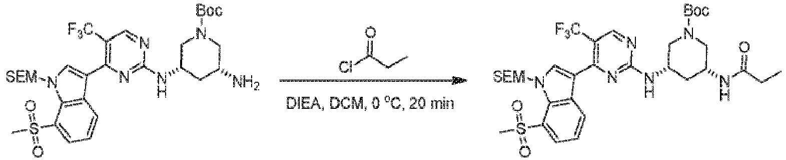

To a solution of tert-butyl (3R,5S)-3-amino-5-[[4-[7-methylsulfonyl-1- (2-trimethylsilylethoxymethyl)indol-3-yl]-5-(trifluoromethyl)pyrimidin-2-yl]amino]piperidine-1-carboxylate (110.00 mg, 128.50 umol, 1.00 eq) and DIPEA (33.21 mg, 257.00 umol, 44.88 uL, 2.00 eq) in DCM (5.00 mL) was slowly drop added propanoyl chloride (10.70 mg, 115.65 umol, 10.70 uL, 0.90 eq) at 0 °C. The mixture was stirred for 20 min. The reaction mixture was quenched by water (10 mL) and extracted with DCM (20 mL * 2). The combined organic layers were dried over Na₂SO₄, filtered and concentrated under reduced pressure to give a residue. The residue was purified by prep-TLC (SiO₂, DCM: MeOH = 10:1) to afford the title compound (90.00 mg, 71.84% yield) as a yellow solid.

### Step 4: N-[(3R,5S)-5-[[4-(7-methylsulfonyl-1H-indol-3-yl)-5-(trifluoromethyl)pyrimidin-2-yl]amino]-3-piperidyl]propanamide & N-[(3S,5R)-5-[[4-(7-methylsulfonyl-1H-indol-3-yl)-5-(trifluoromethyl) pyrimidin-2-yl]amino]-3-piperidyl]propanamide

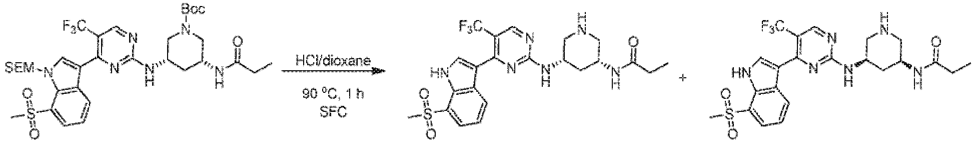

To a solution of tert-butyl (3S, SR)-3-[[4-[7-methylsulfanyl-1- (2-trimethylsilylethoxymethyl) indol-3-yl]-5-(trifluoromethyl) pyrimidin-2-yl] amino]-5-(propanoylamino)piperidine-1-carboxylate (90.00 mg, 88.67 umol, 1.00 eq) in dioxane (3 mL) was added HCl (500 uL). The mixture was heated to 90 °C and stirred for 1 h under N₂. The reaction mixture was filtered and concentrated under reduced pressure to give a residue. The residue was purified by prep-HPLC (FA) to give racemic product (25 mg, FA). The racemic product was separated by SFC [column: AD (250mm*30mm, 5um); mobile phase: [0.1%NH₃H₂O ETOH]; B%: 40%-40%, min] (Combined purification with another batch. Scale: 25 mg).
Product 1: possible configuration N-[(3R, SS)-5-[[4-(7-methylsulfonyl-1H-indol-3-yl)-5-(trifluoromethyl) pyrimidin-2-yl] amino]-3-piperidyl]propanamide (8.40 mg, 18.22% yield) was obtained as a white solid, designated Compound 254.
Product 2: possible configuration N-[(3S, SR)-5-[[4-(7-methylsulfonyl-1H-indol-3-yl)-5-(trifluoromethyl) pyrimidin-2-yl] amino]-3-piperidyl]propanamide (6.00 mg, 12.75% yield) was obtained as a white solid, designated Compound 259.

### Example 72. 3-[2-[[(3S)-3-piperidyl] amino]-5-(trifluoromethyl) pyrimidin-4-yl] -1H-indole-6, 7-dicarbonitrile (Compound 258).

### Step 1: 2-bromo-3-nitro-benzonitrile

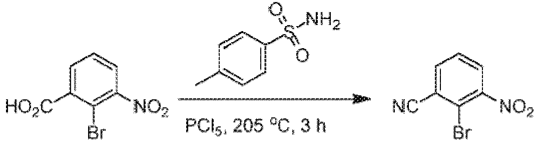

A mixture of 2-bromo-3-nitro-benzoic acid (10.00 g, 40.65 mmol, 1.00 eq) 4-methylbenzenesulfonamide (11.00 g, 64.23 mmol, 1.58 eq), PCl₅ (26.16 g, 125.60 mmol, 3.09 eq) in a 50 mL single-necked round bottom flask was warmed to 205 °C for 0.5 h, and then maintained 205 °C for 2.5 h. Cooling the mixture gave a solid, which was dissolved in warm pyridine (120 ml). Water (500 ml) was added cautiously to the stirred solution, then the resulting suspension was cooled to give a residue. The residue was purified by column chromatography (SiO₂, PE/EtOAc=10/1 to 2:1, plate 1) to afford the title compound (6.00 g, 26.43 mmol) as an off-white solid.

### Step 2: 7-bromo-1H-indole-6-carbonitrile

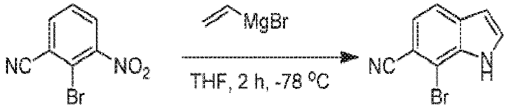

To a solution of 2-bromo-3-nitro-benzonitrile (4.00 g, 17.62 mmol, 1.00 eq) in THF (40.00 mL) was added dropwise bromo(vinyl)magnesium (1 M, 88.10 mL, 5.00 eq) at -78 °C. The resulting mixture was stirred at -78 °C for 2 h. The reaction mixture was quenched by addition water 200 mL, and extracted with EtOAc (75mL*3). The combined organic layers were washed with brine (500 mL), dried over Na₂SO₄, filtered and concentrated under reduced pressure to give a residue. The residue was purified by column chromatography (SiO₂, PE/Ethyl acetate=5:1 to 1:1) to afford the title compound (1.30 g, 5.29 mmol, 30.04% yield, 90% purity) as an yellow solid.

### Step 3: 7-bromo-3-[2-chloro-5-(trifluoromethyl) pyrimidin-4-yl]-1H-indole-6-carbonitrile

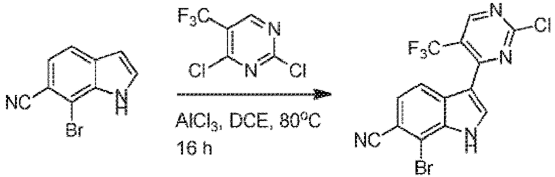

To a solution of 2, 4-dichloro-5-(trifluoromethyl) pyrimidine (2.65 g, 12.21 mmol, 3.00 eq) in DCE (5.00 mL) was added dropwise AlCl₃ (5.43 g, 40.70 mmol, 2.23 mL, 10.00 eq) at 80 °C. After addition, the mixture was stirred at this temperature for 0.5 h, and then 7-bromo-1H-indole-6-carbonitrile (900.00 mg, 4.07 mmol, 1.00 eq) was added dropwise at 80 °C. The resulting mixture was stirred at 80 °C for 15.5 h. The reaction mixture was quenched by addition water 200mL, and extracted with EA (75 mL*3). The combined organic layers were washed with brine (200mL), dried over Na₂SO₄, filtered and concentrated under reduced pressure to give a residue. The residue was purified by column chromatography (SiO₂, PE/EtOAc=10/1 to 3:1). The residue was purified by prep-HPLC (TFA condition) to afford the title compound (600.00 mg, 582.71 umol, 14.32% yield, 39% purity) as a yellow solid.

### Step 4: 7-bromo-3-[2-chloro-5-(trifluoromethyl) pyrimidin-4-yl]-1- (2-trimethylsilylethoxy methyl) indole-6-carbonitrile

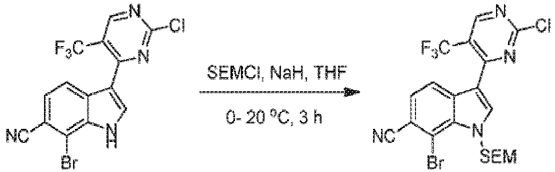

To a solution of 7-bromo-3-[2-chloro-5- (trifluoromethyl)pyrimidin-4-yl]- 1H-indole-6 - carbonitrile (280.00 mg, 697.26 umol, 1.00 eq) in THF (5.00 mL) was added NaH (27.89 mg, 697.26 umol, 60% purity, 1.00 eq) at 0 °C. After addition, the mixture was stirred at this temperature for 0.5 h, and then 2-(chloromethoxy) ethyl-trimethyl-silane (116.25 mg, 697.26 umol, 123.67 uL, 1.00 eq) was added dropwise at 0 °C. The resulting mixture was stirred at 20 °C for 2.5 h. The reaction mixture was quenched by addition water 20 mL, and extracted with EtOAc (10 mL*3), dried over Na₂SO₄, filtered and concentrated under reduced pressure to give a residue. The residue was purified by column chromatography (SiO₂, PE/EtOAc=20:1 to 3:1) to afford the title compound (100 mg) as a yellow oil.

### Step 5:tert - butyl (3S)-3-[[4-[7-bromo-6-cyarro-1-(2-trimethylsilylethoxymethyl)indol-3-yl]-5-(trifluoromethyl)pyrimidin-2-yl]amino]piperidine-1-carboxylate

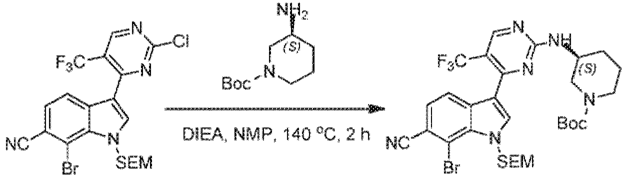

A mixture of 7-bromo-3-[2-chloro-5-(trifluoromethyl) pyrimidin-4-yl]-1- (2-trimethylsilylethoxymethyl)indole-6-carbonitrile (100.00 mg, 188.03 umol, 1.00 eq), tert-butyl (3S)-3-aminopiperidine-1-carboxylate (48.96 mg, 244.44 umol, 1.30 eq), DIEA (121.50 mg, 940.15 umol, 164.20 uL, 5.00 eq) in NMP (2.00 mL) was stirred at 140 °C for 2 h. The reaction mixture was poured into water 50 mL, and extracted with EtOAc (20 mL*3). The combined organic layers were washed with brine (100 mL), dried over Na₂SO₄, filtered and concentrated under reduced pressure to give a residue. The residue was purified by column chromatography (SiO₂, PE/EtOAc=10:1 to 1:1) to afford the title compound (100.00 mg, 143.75 umol, 76.45% yield) as a yellow solid.

### Step 6: tert- butyl (3S)-3-[[4-[6, 7-dicyano-1-(2-trimethylsilylethoxymethyl) indol-3-yl]-5-(trifluoromethyl) pyrimidin-2-yl] amino] piperidine-1-carboxylate

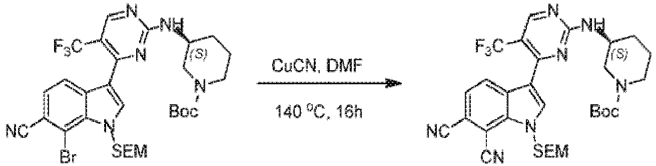

A mixture of tert-butyl (3S)-3-[[4-[7-bromo-6ccyano-1-(2-trimethylsilylethoxymethyl) indol-3-yl]-5-(trifluoromethyl)pyrimidin-2-yl]amino]piperidine-1-carboxylate (100.00 mg, 143.75 umol, 1.00 eq), CuCN (38.62 mg, 431.26 umol, 94.20 uL, 3.00 eq) in DMF (2.00 mL) was degassed and purged with N₂ for 3 times, and then the mixture was stirred at 140 °C for 16 h under N₂ atmosphere. The mixture was filtered and concentrated. The residue was purified by column chromatography (SiO₂, PE/EtOAc=10:1 to 1:1) to afford the title compound (70.00 mg, 109.08 umol, 75.88% yield) as a yellow solid.

### Step 7: 3-[2-[[(3S)-3-piperidyl] amino]-5-(trifluoromethyl) pyrimidin-4-yl] -1H-indole-6, 7-dicarbonitrile

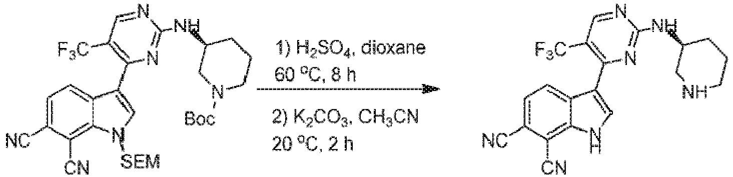

To a solution of tert-butyl (3S)-3-[[4-[6, 7-dicyano-1-(2-trimethylsilylethoxymethyl) indol-3-yl]-5-(trifluoromethyl) pyrimidin-2-yl] amino] piperidine-1-carboxylate (80.00 mg, 124.66 umol, 1.00 eq) in dioxane (2.00 mL) was added H₂SO₄ (122.26 mg, 1.25 mmol, 66.45 uL, 10.00 eq). Then, the mixture was heated to 60 °C for 8 h. The mixture was poured into water (20 mL) and adjusted pH to 8 by aq. NaOH (1M), extracted with EtOAc (10 * 3mL), dried over Na₂SO₄ and concentrated. Then, the residue was diluted with MeCN (1 mL) and K₂CO₃ (20 mg) was added and the mixture was stirred at 20 °C for 2 h. The mixture was poured into water (10 mL), and extracted with DCM (3 mL*3), dried over Na₂SO₄ and concentrated. The residue was purified by prep-HPLC (FA condition) to afford the title compound (7.10 mg, 15.21 umol, 12.20% yield, 98% purity, FA) as a white solid.

### Example 73. 3-[2-[[(3S)-3-piperidyl]amino]-5-(trifluoromethyl)pyrimidin-4-yl]-7-pyridazin-4-yl-1H-indole-6-car bonitrile (Compound 261).

### Step 1: Tert-butyl(3S)-3-[[4-[6-cyano-7-pyridazin-4- yl-1-(2-trimethylsilylethoxymethyl) indol-3-y]-5- (trifluoromethyl)pyrimidin-2-yl]amino]piperidine-1-carboxylate

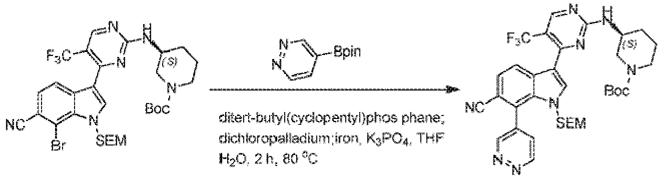

A mixture of 4-(4,4,5,5-tetramethyl-1,3,2-dioxaborolan-2-yl)pyridazine (35.54 mg, 172.50 umol, 1.00 eq), tert-butyl (3S)-3-[[4-[7-bromo-6-cyano-1-(2-trimethylsilylethoxymethyl) indol-3-yl]-5-(trifluorometbyl)pyrimidin-2-yl]amino]piperidine-1-carboxylate (120.00 mg, 172.50 umol, 1.00 eq), K₃PO₄ (73.23 mg, 345.00 umol, 2.00 eq), ditert-butyl(cyclopentyl) phosphane;dichloropalladium;iron (11.24 mg, 17.25 umol, 0.10 eq) in THF (2.00 mL) and H₂O (500.00 uL) was degassed and purged with N₂ for 3 times, and then the mixture was stirred at 80 °C for 2 h under N₂ atmosphere. The reaction mixture was poured into water 50 mL, and then extracted with EtOAc (20 mL*3). The combined organic layers were washed with brine (100 mL), dried over Na₂SO₄, filtered and concentrated under reduced pressure to give a residue. The residue was purified by column chromatography (SiO₂, PE/EtOAc=10:1 to 0:1) to afford the title compound (50 mg) as a yellow solid.

### Step 2: 3-[2-[[(3S)-3-piperidyl] amino]-5-(trifluoromethyl) pyrimidin-4-yl] -7-pyridazin-4 -yl-1H- indole-6-car bonitrile

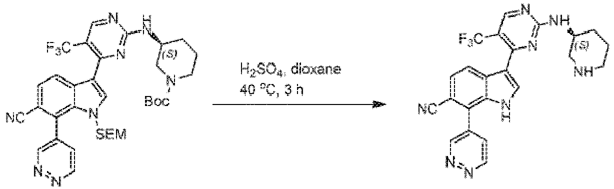

To a solution of tert-butyl (3S)-3-[[4-[6-cyano-7-pyridazin-4-yl -1 -(2-trimethylsilylethoxymethyl)indol-3-yl]-5-(trifluoromethyl)pyrimidin-2-yl]amino]piperidine-1-carboxylate (50.00 mg, 71.96 umol, 1.00 eq) in dioxane (1.00 mL) was added H₂SO₄ (70.58 mg, 719.61 umol, 38.36 uL, 10.00 eq). The mixture was stirred at 40 °C for 3 h. The mixture was treated with NaOH (2 M) and adjusted pH to 9, extracted with EtOAc (5 mL*3), dried over Na₂SO₄ and concentrated to give a residue. To a solution of the crude product in CH₃CN (1 mL) was added K₂CO₃ (15 mg). The mixture was stirred at 20 °C for 1 h. The reaction mixture was poured into water 10 mL, and then extracted with EtOAc (5 mL*3). The combined organic layers were washed with brine (30 mL), dried over Na₂SO₄, filtered and concentrated under reduced pressure to give a residue. The residue was purified by prep-HPLC (FA condition) to afford the title compound (6 mg, FA salt) as a yellow solid. (Combined with ET5361-706, SM scale: 9 mg).

### Example 74. 5-ethyl-N-[(3S)-3-piperidyl]-4-(1H-pyrazolo[3,4-b]pyridin-3-yl)pyrimidin-2-amine (Compound 262).

### Step 1: 1H-pyrazolo[3,4-b]pyridine

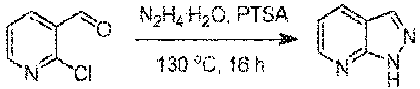

To a solution of 2-chloropyridine-3-carbaldehyde (3.00 g, 21.19 mmol, 1.00 eq) in hydrazine; hydrate (6.36 g, 127.14 mmol, 6.18 mL, 6.00 eq) was added PTSA (1.82 g, 10.60 mmol, 0.50 eq). The mixture was stirred at 130 °C for 16 h. It was concentrated. The residue was purified by column chromatography to afford the title compound (1.50 g, 11.33 mmol, 53.48% yield, 90% purity) as a white solid.

### Step 2: 3-bromo-1H-pyrazolo [3, 4-b] pyridine

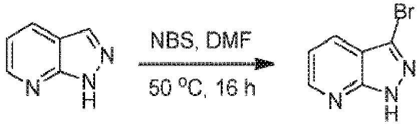

To a solution of 1H-pyrazolo [3,4-b] pyridine (1.10 g, 9.23 mmol, 1.00 eq) in DMF (2.00 mL) was added NBS (2.46 g, 13.85 mmol, 1.50 eq). The mixture was stirred at 50 °C for 16 h. The reaction mixture was poured into water 100 mL, and then extracted with EtOAc (30 mL * 3). The combined organic layers were washed with brine (200 mL), dried over Na₂SO₄, filtered and concentrated under reduced pressure to give a residue. The residue was purified by column chromatography (SiO₂, PE/EtOAc=10:1 to 3:1) to afford the title compound (1.2 g) as a white solid. (Note: Combined purification with another batch: SM scale: 200 mg).

### Step 3: 3-bromo-1-trityl- pyrazolo[3, 4-b] pyridine

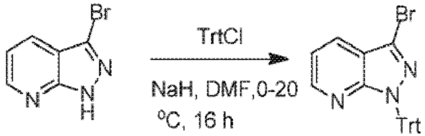

To a solution of 3-bromo-1H-pyrazolo[3,4-b]pyridine (1.20 g, 6.06 mmol, 1.00 eq) in DMF (10.00 mL) was added NaH (290.88 mg, 7.27 mmol, 60% purity, 1.20 eq) at 0 °C. After addition, the mixture was stirred at this temperature for 0.5 h, and then [chloro (diphenyl) methyl] benzene (1.86 g, 6.67 mmol, 1.10 eq) was added dropwise at 0 °C. The resulting mixture was stirred at 20 °C for 15.5 h. The reaction mixture was quenched by addition water 100 mL, and then extracted with EtOAc (50 mL * 3). The combined organic layers were washed with brine (200 mL), dried over Na₂SO₄, filtered and concentrated under reduced pressure to give a residue. The residue was purified by column chromatography (SiO₂, PE/EtOAc= 100:1 to 10:1) to afford the title compound (1.60 g, 3.27 mmol, 53.96% yield, 90% purity) as a white solid.

### Step 4: 3-(4, 4, 5, 5-tetramethyl-1, 3, 2-dioxaborolan-2-yl)-1-trityl-pyrazolo [3, 4-b] pyridine

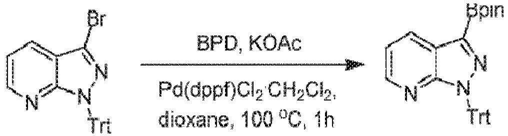

A mixture of 3-bromo-1-trityl-pyrazolo[3,4-b]pyridine (600.00 mg, 1.36 mmol, 1.00 eq), 4,4,5,5-tetramethyl-2-(4,4,5,5-tetramethyl-1,3,2-dioxaborolan-2-yl)-1,3,2-dioxaborolane (690.72 mg, 2.72 mmol, 2.00 eq), Pd(dppf)Cl₂.CH₂Cl₂ (111.06 mg, 136.00 umol, 0.10 eq), KOAc (400.41 mg, 4.08 mmol, 3.00 eq) in dioxane (12.00 mL) was degassed and purged with N₂ for 3 times, and then the mixture was stirred at 100 °C for 1 h under N₂ atmosphere. The reaction mixture was poured into water 100 mL, and then extracted with EtOAc (50 mL * 3). The combined organic layers were washed with brine (200 mL), dried over Na₂SO₄, filtered and concentrated under reduced pressure to afford the title compound (600.00 mg, crude) as brown oil.

### Step 5: 3-(5-ethyl-2-methylsulfanyl-pyrimidin-4-yl)-1-trityl-pyrazolo [3, 4-b] pyridine

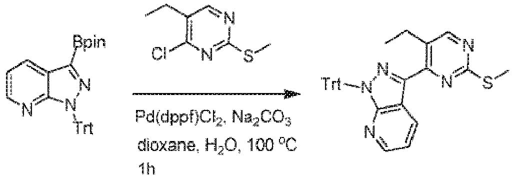

A mixture of 3-(4,4,5,5-tetramethyl-l,3,2-dioxaborolan-2-yl)-1-trityl-pyrazolo[3,4-b]pyridme (600.00 mg, 1.23 mmol, 1.00 eq), 4-chloro-5-ethyl-2-methylsulfanyl-pyrimidine (139.25 mg, 738.00 umol, 0.60 eq), Pd(dppf)Cl₂ (90.08 mg, 123.00 umol, 0.10 eq), Na₂CO₃ (2 M, 1.23 mL, 2.00 eq) in dioxane (10.00 mL) was degassed and purged with N₂ for 3 times, and then the mixture was stirred at 100 °C for 1 h under N₂ atmosphere. The reaction mixture was poured into water 100 mL, and then extracted with EtOAc (50 mL * 3). The combined organic layers were washed with brine (200 mL), dried over Na₂SO₄, filtered and concentrated under reduced pressure to give a residue. The residue was purified by column chromatography (SiO₂, PE/EtOAc=30:1 to 5:1) to afford the title compound (250 mg) as a white solid.

### Step 6: 3-(5-ethyl-2-methylsulfonyl-pyrimidin-4-yl)-1-trityl-pyrazolo [3, 4-b] pyridine

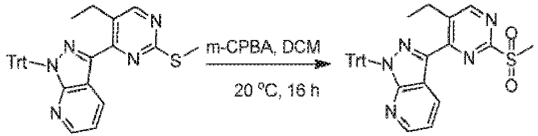

To a solution of 3-(5-ethyl-2-methylsulfanyl-pyrimidin-d-yl)-1-trityl pyrazolo [3, 4-b] pyridine (250.00 mg, 486.70 umol, 1.00 eq) in DCM (5.00 mL) was added m-CPBA (217.39 mg, 1.07 mmol, 85% purity, 2.20 eq). The mixture was stirred at 20 °C for 16 h. To a solution were added 20 mL of Sat Na₂SO₃ and 20 mL of Sat. NaHCOj. The mixture was stirred at 20 °C for 1 h. The mixture was extracted with DCM (20 mL * 3). The combined organic layers were washed with brine (100 mL), dried over Na₂SO₄, filtered and concentrated under reduced pressure to give a residue. The residue was purified by column chromatography (SiO₂, PE/EtOAc=20/1 to 5:1) to afford the title compound (200 mg) was obtained as a white solid.

### Step 7: Tert-butyl (3S)-3-[[5-ethyl-4-(1-tritylpyrazolo [3, 4-b] pyridin-3-yl) pyrimidine-2-yl] amino] piperidine-1-carboxylate

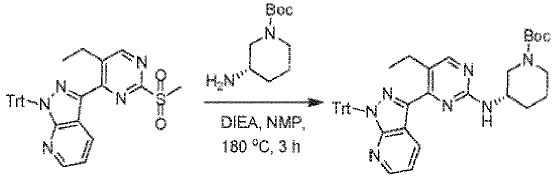

A mixture of 3-(5-ethyl-2-methylsulfonyl-pyrimidin-4-yl)-1-trityl-pyrazolo[3,4-b]pyridine (180.00 mg, 329.88 umol, 1.00 eq), tert-butyl (3S)-3-aminopiperidine-1-carboxylate (132.14 mg, 659.76 umol, 2.00 eq), DIEA (213.17 mg, 1.65 mmol, 288.07 uL, 5.00 eq) in NMP (2.00 mL) was stirred at 180 °C for 3 h. The reaction mixture was poured into water 20 mL, and then extracted with EtOAc (10 mL * 3). The combined organic layers were washed with brine, dried over Na₂SO₄, filtered and concentrated under reduced pressure to give a residue. The residue was purified by column chromatography (SiO₂, PE/EtOAc=10:1 to 1:1) to afford the title compound (100 mg) as a yellow oil.

### Step 8: 5-ethyl-N-[(3S)-3-piperidyl]-4-(1H-pyrazolo [3, 4-b] pyridin-3yl) pyrimidin-2-amine

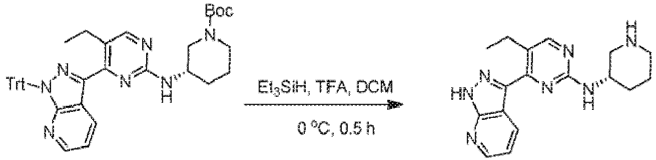

To a solution of tert-butyl (3S)-3-[[5-ethyl-4-(1-tritylpyrazolo[3,4-b]pyridin-3-yl)pyridin-2-yl]amino]piperidine-1-carboxylate (80.00 mg, 120.15 umol, 1.00 eq) in DCM (1.00 mL) was added Et₃SiH (558.85 mg, 4.81 mmol, 765.55 uL, 40.00 eq) and TFA (1.10 g, 9.61 mmol, 711.68 uL, 80.00 eq) at 0 °C. The mixture was stirred at 0 °C for 0.5 h. It was concentrated. The residue was purified by prep-HPLC to afford the title compound (10.55 mg, FA salt) as a yellow solid. (Note: Combined purification with another batch. SM scale: 20 mg).

### Example 75. 3-[5-ethyt-2-[[(3S)-3-piperidyl]amino] pyrimidin-4-yl] -7-methylsulfonyl-1R-indole-6-carbonitrile (Compound 263).

### Step 1:tert-butyl 1-bromo-2-methylsulfanyl-3-nitro-benzene

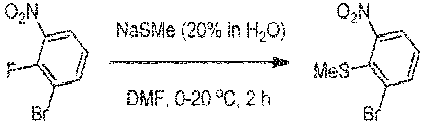

To a mixture of 1-bromo-2-fluoro-3-nitro-benzene (25.00 g, 113.64 mmol, 1.00 eq) in DMF (250.00 mL) was added NaSMe (51.77 g, 147.73 mmol, 47.06 mL, 20% purity, 1.30 eq) in portions at 0 °C under N₂. The mixture was stirred at 20 °C for 2 h. Ice-water (wlw = 1/1) (500 mL) was added and stirred for 30 min. The mixture was filtered and washed with water, dried under reduced pressure to afford the title compound (20.00 g, 80.61 mmol, 70.94% yield) as yellow solid.

### Step 2:tert-butyl 6-bromo-7-methylsulfanyl-1H-indole

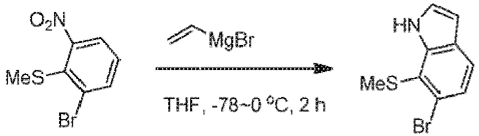

To a mixture of 1-bromo-2-methylsulfanyl-3-nitro-benzene (10.00 g, 40.31 mmol, 1.00 eq) in THF (200.00 mL) was added bromo(vinyl)magnesium (1 M, 201.55 mL, 5.00 eq) in one portion at -78 °C under N₂. The mixture was stirred at 0 °C for 2 h. The mixture was poured into ice-water (w/w = 1/1) (500 mL) and extracted with EtOAc (300 mL*2). The combined organic phase was washed with brine (300 mL*2), dried with anhydrous Na₂SO₄, filtered and concentrated in vacuum. The residue was purified by silica gel chromatography (PE/EtOAc=50/1, 2011) to afford the title compound (6.00 g, 24.78 mmol, 61.47% yield) as yellow oil.

### Step 3:tert-butyl 6-bromo-3-(2,5-dichloropyrimidin-4-yl)-7-methylsulfanyl-1H-indole

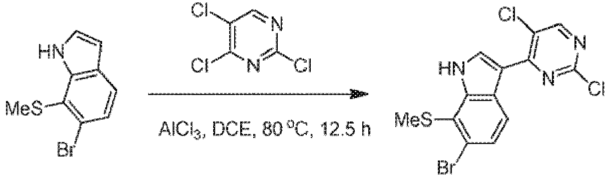

To a mixture of 2,4,5-trichloropyrimidine (2.73 g, 14.87 mmol, 1.20 eq) in DCE (30.00 mL) was AlCl₃ (826.04 mg, 6.20mmol, 338.54 uL, 1.50 eq) in one portion at 80 °C under N₂. The mixture was stirred at 80 °C for 30 min, then 6-bromo-7-methylsulfanyl-1H-indole (3.00 g, 12.39 mmol, 1.00 eq) was added and stirred at 80 °C for 12 h. The mixture was poured into ice-water (w/w = 1/1) (200 mL) and extracted with EtOAc (200 mL*2). The combined organic phase was washed with brine (200 mL*2), dried with anhydrous Na₂SO₄, filtered and concentrated in vacuum. The residue was purified by silica gel chromatography (PE/EtOAc=20/1, 2/1)) to afford the title compound (1.50 g, crude) as brown solid.

### Step 4: 2-[[6-bromo-3-(2,5-dichloropyrimidin-4-yl)-7- methylsulfanyl-indol-1-yl]methoxy]ethyl-trimethyl-silane

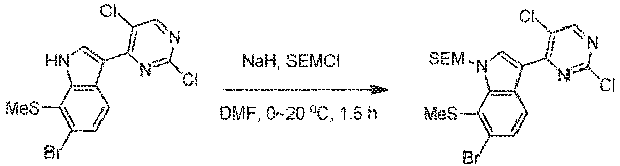

To a mixture of 6-bromo-3-(2,5-dichloropyrimidin-4-yl)-7-methylsulfanyl-1H-indole (2.00 g, 5.14 mmol, 1.00 eq) in DMF(50.00 mL) was added NaH (411.20 mg, 10.28 mmol, 60% purity, 2.00 eq) in portions at 0 °C under N₂. The mixture was stirred at 0 °C for 30 min, then SEM-Cl (1.29 g, 7.71 mmol, 1.37 mL, 1.50 eq) was added into the mixture and stirred at 20 °C for 1 h. The mixture was poured into ice-water (w/w = 1/1) (100 mL) and extracted with EtOAc (50 mL*2). The combined organic phase was washed with brine (50 mL*2), dried with anhydrous Na₂SO₄, filtered and concentrated in vacuum. The residue was purified by silica gel chromatography (PE/EtOAc=30/1, 10/1) to afford the title compound (1.40 g, 2.70 mmol, 52.44% yield) as yellow solid.

### Step 5: 2-[[6-bromo-3-(2,5-dichloropyrimidin-4-yl)-7- methylsulfonyl-indol-1-yt]methoxy]ethyl-trimethyl-silane

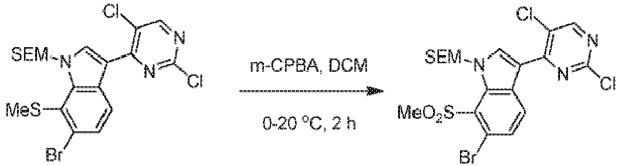

To a mixture of 2-[[6-bromo-3-(2,5-dichloropyrimidin-4-yl)-7-methylsulfanyl-indol-1-yl] methoxy]ethyl-lrimethyl-silane (1.20g, 2.31 mmol, 1.00 eq) in DCM (50.00 mL) was added m-CPBA (1.66 g, 5.78 mmol, 60% purity, 2.50 eq) in one portion at 0 °C under N₂. The mixture was stirred at 20 °C for 2 h. The mixture was poured into ice-water (w/w = 1/1) (200 mL) and extracted with EtOAc (100 mL*2). The combined organic phase was washed with aqueous of K₂CO₃ solution (50 mL*2), brine (100 mL*2), dried with anhydrous Na₂SO₄, filtered and concentrated in vacuum to afford the title compound (1.00 g, crude) as white solid.

### Step 6: 3-(2,5-dichloropyritnidin-4-yl)-7-methylsulfonyl-1-(2-trimethylsilylethoxymethyl)indole-6-carbonitrile

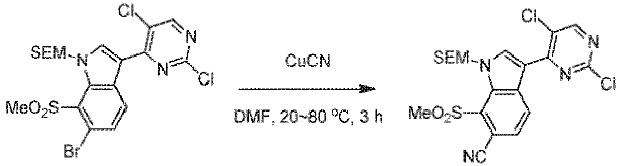

To a mixture of 2-[[6-bromo-3-(2,5-dichloropyrimidin-4-yl)-7-methylsulfonyl-indol-1-yl] methoxy]ethyl-trimethyl-silane(700.00 mg, 1.27 mmol, 1.00 eq) in DMF (20.00 mL) was added CuCN (170.61 mg, 1.91 mmol, 416.12 uL, 1.50 eq) in one portion at 20 °C under N₂. The mixture was stirred at 80 °C for 3 h. The mixture was poured into ice-water (w/w = 1/1) (50 mL) and extracted with EtOAc (50 mL*2). The combined organic phase was washed with brine (100 mL*2), dried with anhydrous Na₂SO₄, filtered and concentrated in vacuum. The residue was purified by silica gel chromatography (PE/FAOAc=5/1, 111) to afford the title compound (400.00 mg, 804.07umol, 63.31% yield) as yellow solid.

### Step 7:tert-butyl (3S)-3-[[5-chloro-4-[6-cyano-7- methylsulfonyl-1-(2-trimethylsilylethoxymethyl)indol-3-yl]pyrimidin-2-yl]amino]piperidine-1-carboxylate

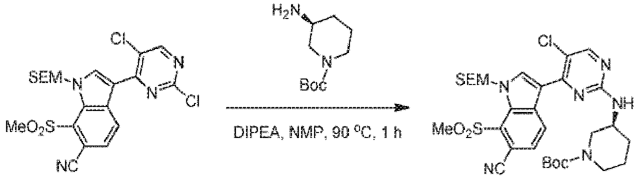

To a solution of 3-(2,5-dichloropyrimidin-4-yl)-7-methylsulfonyl-1-(2-trimethylsilylethoxymethyl)indole-6-carbonitrile (200.00 mg, 402.03 umol, 1.00 eq) in NMP (1.00 mL) was added tert-butyl (3S)-3-aminopiperidine-1-carboxylate (322.08 mg, 1.61 mmol, 4.00 eq) and DIPEA (155.88 mg, 1.21 mmol, 210.64 uL, 3.00 eq). The mixture was stirred at 90 °C for 1 h. The reaction mixture was poured into H₂O (10 mL), while yellow solid formed. The solid was filtered, and the solid was washed with H₂O (2 mL*2) and dried to afford the title compound (130.00 mg, crude). It was used into the next step without further purification.

### Step 8:tert-butyl (3S)-3-[14-[6-cyano-7-methylsulfonyl-1-(2-trimethylsilylethoxymethyl)indol-3-yl]-5-vinyl-pyrimidin-2-yl]amino]piperidine-1-carboxylate

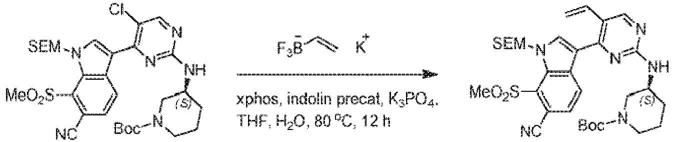

To a solution of tert-butyl (3S)-3-[[5-chloro-4-[6-cyano-7-methylsulfonyl-1-(2-trimethylsilylethoxymethyl)indol-3-yl]pyrimidin-2-yl]amino]piperidine-1-carboxylate (140.00 mg, 211.71 umol, 1.00 eq) in THF (1.00 mL) and H₂O (0.2 mL) was added potassium;trifluoro(vinyl)boranuide (141.79 mg, 1.06 mmol, 5.00 eq), K₃PO₄ (89.88 mg, 423.41 umol, 2.00 eq) and [2-(2-aminoethyl)phenyl]-chloro-palladium;dicyclohexyl -[2-(2,4,6-triisopropylphenyl)phenyl]phosphane (23.46 mg, 31.76 umol, 0.15 eq) under N₂. The mixture was stirred under N₂ at 80 °C for 12 h. The reaction mixture was concentrated under reduced pressure to give a residue. The residue was diluted with H₂O 10 mL and extracted with EtOAc (10 mL * 2). The combined organic layers were washed with brine (5 mL * 2), dried over Na₂SO₄, filtered and concentrated under reduced pressure to give a residue. The residue was purified by column chromatography (SiO₂, PE/EtOAc=10/1 to 3:1) to afford the title compound (100.00 mg, 91.90 umol, 43.41% yield, 60% purity) as yellow solid.

### Step 9: Tert-butyl (3S)-3-[[4-[6-cyano-7-methylsulfonyl-1-(2-trimethylsilylethoxymethyl)indol-3-y]-5-etyl-pyrimidin-2-yl]ammo]piperidine-1-carboxylate

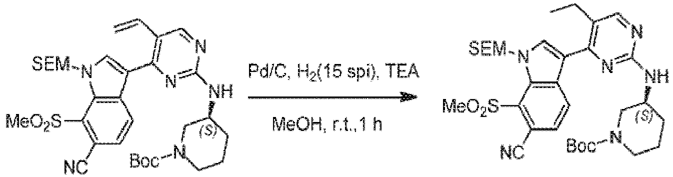

To a solution of tert-butyl (3S)-3-[[4-[6-cyano-7-methylsulfonyl-1-(2-trimethylsilylethoxymethyl)indol-3-yl]-5-vinyl-pyrimidin-2-yl]amino]piperidine-1-carboxylate (100.00 mg, 153.17 umol, 1.00 eq) in MeOH (5.00 mL) was added Pd-C(10%, 0.05 g) and TEA (31.00 mg, 306.34 umol, 42.46 uL, 2.00 eq) under N₂. The suspension was degassed under vacuum and purged with H₂ several times. The mixture was stirred under H₂ (15 psi) at 15 °C for 1 h. The reaction mixture was filtered and the filtrate was concentrated to afford the title compound (60.00 mg, crude). It was used into the next step without further purification.

### Step 10: 3-[5-ethyl-2-[[(3S)-3-piperidyl]amino]pyrimidin-4-yl] -7-methylsulfonyl-1H-indole-6-carbonitrile

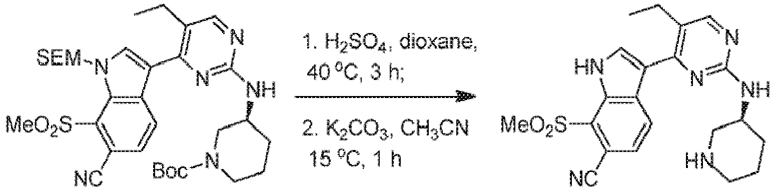

To a solution of tert-butyl (3S)-3-[[4-[6-cyano-7-methylsulfonyl-1-(2-trimethylsilylethoxymethyl)indol-3-yl]-5-ethyl-pyrimidin-2-yl]amino]piperidine-1-carboxylate (60.00 mg, 91.62 umol, 1.00 eq) in dioxane (500.00 uL) was added H₂SO₄ (89.86 mg, 916.18 umol, 48.84 uL, 10.00 eq). The mixture was stirred at 40 °C for 3 h. The reaction mixture was concentrated under reduced pressure to remove dioxane. The residue was diluted with CH₃CN 1 mL. Then K₂CO₃ (0.2 g, powder) was added. The mixture was stirred at 15 °C for another 1 h. Then the mixture was poured into H₂O (10 mL), and extracted with EtOAc (10 mL*2). The combined organic layers were washed with brine (10 mL * 2), dried over Na₂SO₄, filtered and concentrated under reduced pressure to give a residue. The residue was purified by prep-HPLC (FA condition) to afford the title compound (4.20 mg, 8.73 umol, 9.53% yield, 97.83% purity, FA) as a white solid.

### Example 76.1-[4-[[(3R,5S)-5-[[4-(7-methylsulfonyl-1H-indol-3-yl)-5-(trifluoromethylpyrimidin-2-yl]amino]-3-piperidyl]oxymethyl]-1-piperidyl]prop-2-en-1-one (Comopund 264).

### Step 1: Tert-butyl 4-(hydroxymethyl) piperidine-1-carboxylate

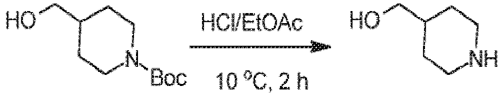

A mixture of tert-butyl 4-(hydroxymethyl) piperidine-1-carboxylate (5.00 g, 23.22 mmol, 1.00 eq) in HCl/EtOAc (40.00mL) was stirred at 10 °C for 2 h. The mixture was concentrated in vacuum to afford the title compound (3.20 g, crude, HCl) as white solid.

### Step 2: allyl 4-(hydroxymethyl)piperidine-1-carboxylate

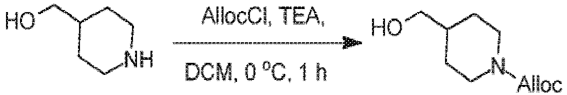

To a solution of 4-piperidylmethanol (2.70 g, 17.81 mmol, 1.00 eq, HCl) in DCM (30.00 mL) was added TEA (5.41 g, 53.43mmol, 7.41 mL, 3.00 eq), followed by addition of allylcarbonochloridate (2.36 g, 19.59 mmol, 2.07 mL, 1.10 eq). The mixture was stirred at 0 °C for 1 h. The residue was poured into water (50 mL) and the aqueous phase was extracted with DCM (50 mL*3). The combined organic phase was dried with anhydrous Na₂SO₄, filtered and concentrated in vacuum. The residue was purified by silica gel chromatography (PE: EtOAc=10:1-5:1-2:1) to afford the title compound (1.48 g, 7.43 mmol, 41.71% yield) as colorless oil.

### Step 3: allyl 4-(trifluoromethylsulfonyloxymethyl) piperidine-1-carboxylate

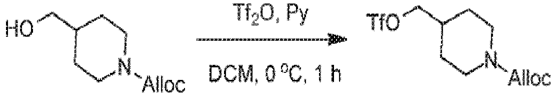

To a solution of allyl 4-(hydroxymethyl)piperidine-1-carboxylate (1.48 g, 7.43 mmol, 1.00 eq) in DCM (10.00 mL) was added Py (881.31 mg, 11.15 mmol, 899.30 uL, 1.50 eq), followed by addition of Tf₂O (2.52 g, 8.92 mmol, 1.47 mL, 1.20 eq). The mixture was stirred at 0 °C for 1 h. The residue was poured into water (20 mL). The aqueous phase was extracted with DCM (20 mL*3). The combined organic phase was dried with anhydrous Na₂SO₄, filtered and concentrated in vacuum. The residue was purified by column (PE: EA=10:1-5:1) to afford the title compound (1.20 g, crude) as brown oil.

### Step 4: benzyl (3R)-3-[(1-allyloxycarbonyl-4-piperidyl)methoxy]-5-(tert-butoxycarbonylamino) piperidine-1-carboxylate

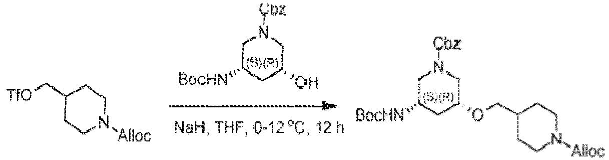

To a solution of benzyl (5R)-3-(tert-butoxycarbonylamino)-5-hydroxy-piperidine-1-carboxylate (520.00 mg, 1.48 mmol, 1.00 eq) in THF (5 mL) was added NaH (89.04 mg, 2.23 mmol, 60% purity, 1.50 eq). The mixture was stirred at 0 °C for 0.5 h. Then allyl 4-(trifluoromethylsulfonyloxymethyl) piperidine-1-carboxylate (983.31 mg, 2.97 mmol, 2.00 eq) was dissolved in THF (5 mL) was added to the above mixture, and the reaction was stirred at 12 °C for 11.5 h. The residue was poured into water (30 mL). The aqueous phase was extracted with EtOAc (30 mL*3). The combined organic phase was washed with brine (50 mL*2), dried with anhydrous Na₂SO₄, filtered and concentrated in vacuum. The residue was purified by silica gel chromatography (PE: EA = 10:1-5:1-3:1-2:1) to afford the title compound (550.00 mg, crude) as colorless oil.

### Step 5: benzyl (3R,5S)-3-[(1-allyloxycarbonyl-4-piperidyl) methoxy]-5-amino-piperidine-1-carboxylate

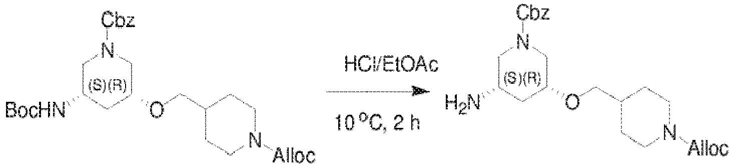

A solution of benzyl (3R,5S)-3-[(1-allyloxycarbonyl-4-piperidyl)methoxy]-5-(tert-Butoxycarbonylamino) piperidine-1-carboxylate (500.00 mg, 940.49 umol, 1.00 eq) in HCl/EtOAc (10.00 mL) was stirred at 10 °C for 2 h. The mixture was concentrated in vacuum to afford the title compound (600.00 mg, crude, HCl) as yellow oil.

### Step 6: Benzyl (3R,5S)-3-[(1-allyloxycarbonyl-4-piperidyl) methoxy]-5-[[4-(7-methylsulfonyl-1H-indol-3-yl)-5-(trifluoromethyl) pyrimidin-2-yl] amino] piperidine-1-carboxylate

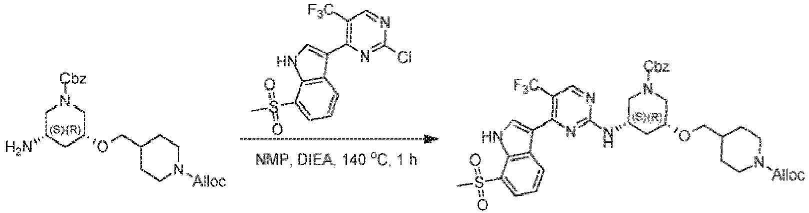

To a solution of 3-[2-chloro-5-(trifluoromethyl)pyrimidin-4-yl]-7-methylsulfonyl-1H-indole (350.00 mg, 931.47 umol, 1.00eq) and benzyl (3R,5S)-3-[(1-allyloxycarbonyl-4-piperidyl)methoxy]-5-amino-piperidine-1-carboxylate (501.30 mg, 1.07 mmol, 1.15 eq, HCl) in NMP (5.00 mL) was added DIEA (601.92 mg, 4.66 mmol, 813.41 uL, 5.00 eq). The mixture was stirred at 140 °C for 1 h. The residue was poured into water (50 mL). The aqueous phase was extracted with EtOAc (50 mL*3). The combined organic phase was washed with brine (50 mL*2), dried with anhydrous Na₂SO₄, filtered and concentrated in vacuum. The residue was purified by silica gel chromatography (DCM: MeOH = 100:1-50:1) to afford the title compound (500.00 mg, 648.66 umol, 69.64% yield) as yellow solid.

### Step 7: Allyl 4-[[3R, 5S)-5-[[4-(7-methylsulfonyl-1H-indol-3-yl)-5-(trifluoromethyl) pyrimidin-2-yl] amino]-3-piperidyl]oxymethyl]piperidine-1-carboxylate

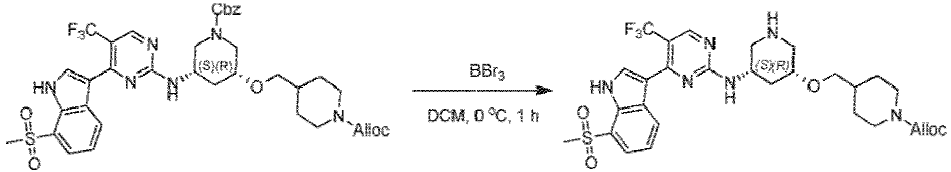

To a solution of benzyl (3R, 5S)-3-[(1-allyloxycarbonyl-4-piperidyl) methoxy]-5-[[4-(7-methylsulfonyl-1H-indol-3-yl)-5-(trifluoromethyl) pyrimidin-2-yl] amino] piperidine-1-carboxylate (390.00 mg, 505.95 umol, 1.00 eq) in DCM (10.00 mL) was added BBr₃ (380.26 mg, 1.52 mmol, 146.25 uL, 3.00 eq) at 0 °C dropwise. The mixture was stirred at 0 °C for 1 h. The mixture was concentrated in vacuum. The residue was washed with PE: EtOAc=1:1, the mixture was filtered and the filter cake was concentrated in vacuum to afford the title compound (0.5 g crude, HBr salt) as yellow solid.

### Step 8: tert-butyl (3R,5S)-3-[(1-allyloxycarbonyl-4-piperidyl) methoxy]-5-[[4-(7-methylsulfonyl-1H-indol-3-yl)-5-(trifluoromethyl) pyrimidin-2-yl] amino] piperidine-1-carboxylate

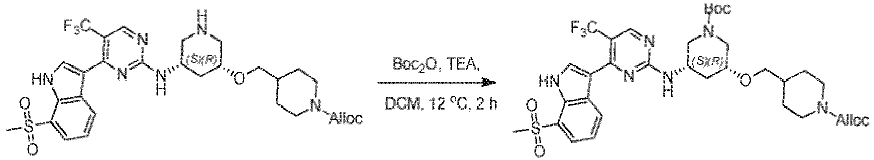

To a solution of allyl 4-[[(3R, 5S)-5-[[4-(7-methylsulfonyl-1H-indol-3-yl)-5-(trifluoromethyl)pyrimidin-2-yl] amino]-3-piperidyl] oxymethyl] *piperidine-1-carboxylate* (400.00 mg, 628.26 umol, 1.00 eq) in DCM (15.00 mL) was added Boc₂O (164.54 mg, 753.91 umol, 173.20 uL, 1.20 eq) and TEA (190.72 mg, 1.88 mmol, 261.26 uL, 3.00 eq). The mixture was stirred at 12 °C for 2 h. The mixture was poured into water (20 mL). The aqueous phase was extracted with DCM (20 mL*3). The combined organic phase was dried with anhydrous Na₂SO₄, filtered and concentrated in vacuum. The residue was purified by silica gel chromatography (DCM: MeOH=100:1-80:1) to afford the title compound (220.00 mg, crude) as yellow oil.

### Step 9: tert-butyl (3S,5R)-3-[[4-(7-methylsulfonyl-1H-indol-3-yl)-5-(trifluoromethyl)pyrimidin-2-yl]amino]-5-(4-piperidylmethoxy)piperidine-1-carboxylate

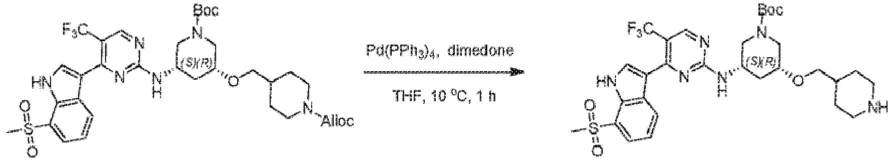

To a solution of tert-butyl (3R, 5S)-3-[(1-allyloxycarbonyl-4-piperidyl)methoxy]-5-[[4-(7-methylsulfonyl-1H-indol-3-yl)-5-(trifluoromethyl) pyrimidin-2-yl] amino] piperidine -1-carboxylate (220.00 mg, 209.01 umol, 1.00 eq) in THF (5.00 mL) was added Pd (PPh₃)₄ (24.15 mg, 20.90 umol, 0.10 eq) and dimedone (222.67 mg, 1.59 mmol, 7.60 eq). The mixture was stirred at 10 °C under N₂ protection for 1 h. The mixture was concentrated in vacuum. The residue was purified by silica gel chromatography (DCM: MeOH=30:1-20:1-10:1-5:1-1:1) to afford the title compound (120.00 mg, crude) as yellow oil.

### Step 10: tert-butyl (3S,5R)-3-[[4-(7-methylsulfonyl-1H-indol-3-yl)-5-(trifluoromethyl) pyrimidin-2-yl] amino]-5-[(1-prop-2-enoyl-4-piperidyl) methoxy]piperidine-1-carboxylate

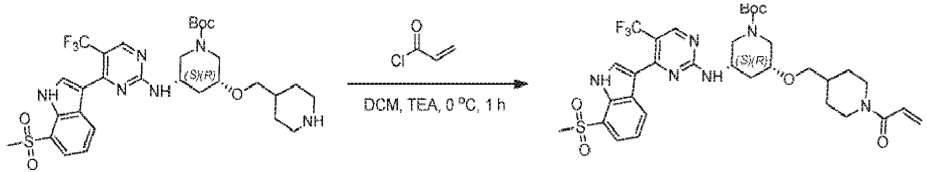

To a solution of tert-butyl (3S, 5R)-3-[[4-(7-methylsulfonyl-1H-indol-3-yl)-5-(trifluoromethyl) pyrimidin-2-yl] amino]-5-(4-piperidylmethoxy) piperidine-1-carboxylate (110.00 mg, 168.52 umol, 1.00 eq) in DCM (3.00 mL) was added TEA (51.16 mg,505.57 umol, 70.08 uL, 3.00 eq), followed by addition of prop-2-enoyl chloride (15.25 mg, 168.52 umol, 13.74 uL, 1.00 eq) at 0 °C. The mixture was stirred at 0 °C for 1 h. The mixture was concentrated in vacuum to afford the title compound (200.00 mg, crude) as yellow oil.

### Step 11:1-[4-[[(3R,5S)-5-[[4-(7-methylsulfonyl-1H-indol-3-yl)-5-(trifluoromethylpyrimidin-2-yl] amino]-3-piperidyl] oxymethyl]-1-piperidyl]prop-2-en-1-one

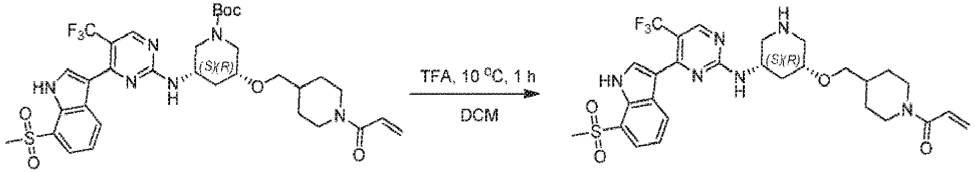

A solution of tert-butyl (3S, 5R)-3-[[4-(7-methylsulfonyl-1H-indol-3-yl)-5-(trifluoromethyl) pyrimidin-2-yl]amino]-5-[(1-prop-2-enoyl-4-piperidyl)methoxy]piperidine-1-carboxylate (200.00 mg, 282.98 umol, 1.00 eq) in DCM (5.00 mL) and TFA (1.00 mL) was stirred at 10 °C for 1 h. The mixture was concentrated in vacuum. The residue was purified by prep-HPLC (FA) to afford the title compound (9.17 mg, 12.84 umol, 4.54% yield, 91.4% purity, FA) as white solid (Note: Combined purification with another batch: SM scale: 20 mg).

### Example 77. 4-(1H-indol-3-yl)-N-[(1R,3S)-3-(8-methylsulfonyl-[1,2,4]triazolo[4, 3-a] pyridin-3-yl) cyclohexyl]-5-(trifluoromethyl)pyrimidin-2-amin (Compound 265).

### Step 1: (3-methylsulfonyl-2-pyridyl)hydrazine

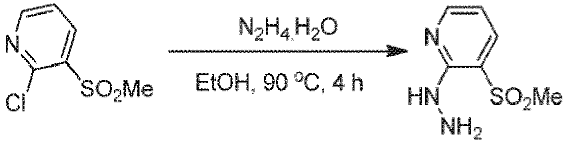

A solution of 2-chloro-3-methylsulfonyl-pyridine (200.00 mg, 1.04 mmol, 1.00 eq) in N₂H₄.H₂O (2.00 mL) and EtOH (2.00mL) was stirred at 90 ° C for 4 h. The mixture was concentrated in vacuum. The residue was purified by silica gel chromatography (DCM: MeOH = 100:1) to afford the title compound (150 mg) as yellow solid.

### Step 2: (1S,3R)-3-[[4-(1H-indol-3-yl)-5-(triftuoromethyl)pyrimidin-2-yl]amino]-N'-(3-methylsulfonyl-2-pyridyl)cyclohexanecarbohydrazide

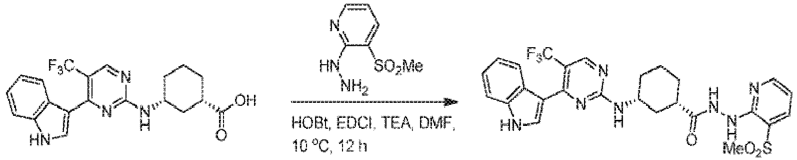

To a solution of (1S,3R)-3-[[4-(1H-indol-3-yl)-5-(trifluoromethyl)pyrimidin-2yl]amino]cyclohexanecarboxylic acid (150.00 mg, 370.93 umol, 1.00 *eq*) and (3-methylsulfonyl-2-pyridyl)hydrazine (83.33 mg, 445.12 umol, 1.20 *eq*) in DMF (3.00 mL) was added HOBt (60.14 mg, 445.12 umol, 1.20 *eq*), EDCI (85.33 mg, 445.12 umol, 1.20 *eq*), followed by addition of TEA (112.60 mg, 1.11 mmol, 154.25 uL, 3.00 *eq*). The mixture was stirred at 10 °C for 12 hours. The mixture was concentrated in vacuum. The residue was purified by silica gel chromatography (DCM: MeOH=100:1-80:1-10:1) to afford the title compound (120 mg) as yellow solid.

### Step 3: 4-(1H-indol-3-yl)-N-[(1R, 3S)-3-(8-methylsulfonyl-[1, 2, 4] triazolo [4, 3-a] pyridin-3-yl) cyclohexyl]-5-(trifluoromethyl) pyrimidin-2-amine

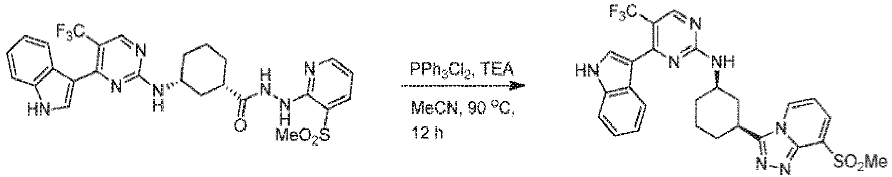

To a solution of (1S, 3R)-3-[[4-(1H-indol-3-yl)-5-(trifluoromethyl) pyrimidin-2-yl] amino]-N'-(3-methylsulfonyl-2-pyridyl) cyclohexanecarbohydrazide (100.00 mg, 174.34 umol, 1.00 eq) in CH₃CN (20.00 mL) was added TEA (705.66 mg, 6.97 mmol, 966.66 uL, 40.00 eq) and dichloro (triphenyl)-phosphane (232.35 mg, 697.36 umol, 4.00 eq). The mixture was stirred at 90 °C for 12 h. The mixture was concentrated in vacuum. The residue was purified by prep-TLC (DCM: MeOH=20:1) and prep-HPLC (FA) to afford the title compound (15.35 mg, 25.52 umol, 14.64% yield, 100% purity, FA) as yellow solid.

### Example 78. Synthesis of (S)-3-(5-ethyl-2-(piperidin-3-ylamino)pyrimidin-4-yl)-1H-pyrrolo[2,3-b]pyridine-6-carbonitrile (Compound 287)

### Step 1: 3-bromo-1H-pyrrolo[2,3-b]pyridine-6-carbonitrile

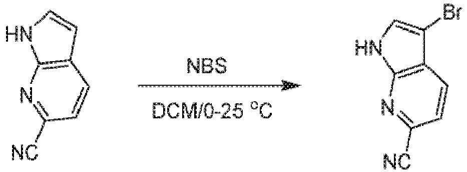

To a mixture of 1H-pyrrolo[2,3-b]pyridine-6-carbonitrile (4.0 g, 28 mmol, 1.0 *eq*) in DCM (20 mL) was added NBS (5.5 g, 31 mmol, 1.1 *eq*) in portions at 0°C under N₂. The mixture was stirred at 25°C for 3 hrs. TLC (Petroleum ether: EtOAc = 3:1, R_{f}= 0.3) showed a new spot. The mixture was concentrated in reduced pressure at 45°C to afford 3-bromo-1H-pyrrolo[2,3-b]pyridine-6-carbonitrile (6.0 g, crude) as a yellow solid.

### Step 2: 3-bromo-1-(phenylsulfonyl)-1H-pyrrolo[2,3-b]pyridine-6-carbonitrile

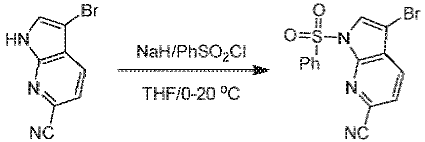

To a mixture of 3-bromo-1H-pyrrolo[2,3-b]pyridine-6-carbonitrile (6.0 g, 27.02 mmol, 1.0 *eq*) in THF (40 mL) was added NaH (4.32 g, 108.09 mmol, 60% purity, 4.0 *eq*) in portions at 0°C under N₂. The mixture was stirred at 0°C for 30 mins. Then PhSO₂Cl (15 g, 84.93 mmol, 3.1 *eq*) was added to the mixture at 0°C. And the mixture was stirred at 25°C for 12 hrs. TLC (Petroleum ether: EtOAc = 3:1, R_{f} = 0.35) shows a new spot. H₂O (20 mL) was added to the mixture. The mixture concentrated in reduced pressure at 45°C. The aqueous phase was extracted with EtOAc (30 mL × 3). The combined organic phase was washed with brine (50 mL), dried with anhydrous Na₂SO₄, filtered and concentrated in vacuum to afford 3bromo-1-(phenylsulfonyl)-1H-pyrrolo[2,3-b]pyridine-6-carbonitrile (4.0 g, crude) as brown oil.

### Step 3: 1-(phenylsulfonyl)-3-(4,4,5,5-tetramethyl-1,3,2-dioxaborolan-2-yl)-1H-pyrrolo[2,3-b]pyridine-6-carbonitrile

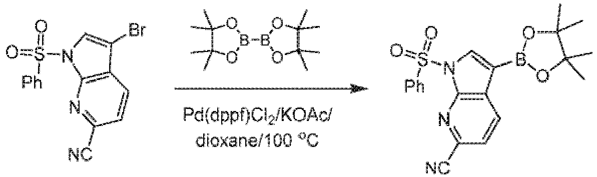

3-bromo-1-(phenylsulfonyl)-1H-pyrrolo[2,3b]pyridine-6-carbonitrile (4.0 g, 11.04 mmol, 1.0 *eq*), bis(pinacolato)diboron (3.37 g, 13.25 mmol, 1.2 *eq*), KOAc (2.17 g, 22.09 mmol, 2.0 *eq*) and Pd(dppf)Cl₂ (808.07 mg, 1.10 mmol, 0.1 *eq*) in dioxane (50 mL) was degassed and then heated to 100°C for 2 hrs under N₂. TLC (Petroleum ether: EtOAc = 3:1, R_{f}= 0.3) showed a new spot. The mixture was cooled to 25°C and concentrated in reduced pressure at 50°C. H₂O (30 mL) was added to the mixture. The aqueous phase was extracted with EtOAc (50 mL × 3). The combined organic phase was washed with brine (100 mL), dried with anhydrous Na₂SO₄, filtered and concentrated in vacuum. It was purified by silica gel chromatography (Petroleum ether: EtOAc = 20:1- 1:1) to afford 1-(phenylsulfonyl)-3-(4,4,5,5-tetramethyl-1,3,2-dioxaborolan-2-yl)-1H-pyrrolo[2,3-b]pyridine-6-carbonitrile (1.8 g, crude) as a yellow solid.

### Step 4: 3-(2-chloro-5-ethylpyrimidin-4-yl)-1-(phenylsulfonyl)-1H-pyrrolo[2,3-b]pyridine-6-carbonitrile

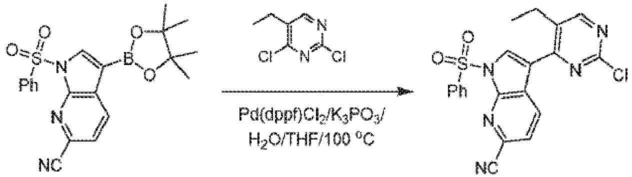

1-(phenylsulfonyl)-3-(4,4,5,5-tetramethyl-1,3,2-dioxaborolan-2-yl)-1H-pyrrolo[2,3-b]pyridine-6-carbonitrile (1.8 g, 4.40 mmol, 1.0 *eq*), 2,4-dichloro-5-ethylpyrimidine (778.60 mg, 4.40 mmol, 1.0 *eq*), K₃PO₄ (1.87 g, 8.80 mmol, 2 *eq*) and Pd(dppf)Cl₂ (321.81 mg, 439.81 umol, 0.10 *eq*) in H₂O (0.5 mL) and THF (5 mL) was degassed and then heated to 100°C for 4 hrs under N₂. TLC (Petroleum ether: EtOAc = 3:1, R_{f}= 0.24) shows a new spot. The mixture was cooled to 25°C and concentrated in reduced pressure at 50°C. H₂O (20 mL) was added to the mixture. The aqueous phase was extracted with EtOAc (20 mL × 3). The combined organic phase was washed with brine (30 mL), dried with anhydrous Na₂SO₄, filtered and concentrated in vacuum. The residue was purified by silica gel chromatography: (Petroleum ether. EtOAc= 20:1-1:1) to afford 3-(2-chloro-5-ethylpyrimidin-4-yl)-1-(phenylsulfonyl)-1H-pyrrolo[2,3-b]pyridine-6-carbonitrile (1.1 g, crude) as yellow solid.

### Step 5: Tert-butyl (S)-3-((4-(6-cyano-1-(phenylsulfonyl)-1H-pyrrolo[2,3-b]pyridin-3-yl)-5-ethylpyrimidin-2-yl)amino)piperidine-1-carboxylate and tert-butyl (S)-3-((4-(6-cyano-1H-pyrrolo[2,3-b]pyridin-3-yl)-5-ethylpyrimidin-2-yl)amino)piperidine-1-carboxylate

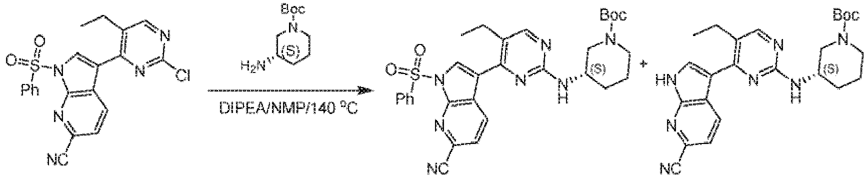

To a mixture of tert-butyl (S)-3-aminopiperidine-1-carboxylate (708 mg, 3.54 mmol, 3.0 *eq*) in NMP (1 mL) was added DIPEA (762 mg, 5.90 mmol, 1.03 mL, 5.0 *eq*) and 3-(2-chloro-5-ethylpyrimidin-4-yl)-1-(phenylsulfonyl)-1H-pyrrolo[2,3-b]pyridine-6-carbonitrile (500 mg, 1.18 mmol, 1.0 *eq*) at 25°C under N₂. The mixture was stirred at 140°C for 12 hrs until complete by LC/MS. The mixture was cooled to 25°C. H₂O (15 mL) was added to the mixture. The aqueous phase was extracted with EtOAc (10 mL × 3). The combined organic phase was washed with brine (30 mL), dried with anhydrous Na₂SO₄, filtered and concentrated in vacuum to afford a mixture of tert-butyl (S)-3-((4-(6-cyano-1-(phenylsulfonyl)-1H-pyirolo[2,3-b]pyridin-3-yl)-5-ethylpyrimidin-2-y1)amino)piperidine-1-carboxylate and tert-butyl (S)-3-((4-(6-cyano-1H-pyrrolo[2,3-b]pyridin-3-yl)-5-ethylpyrimidin-2-yl)amino)piperidine-1-carboxylate (0.7 g, crude) as an orange oil which was used directly in the next step.

### Step 6: (S)-3-(5-ethyl-2-(piperidin-3-ylamino)pyrimidin-4-yl)-1H-pyrrolo[2,3-b]pyridine-6-carbonitrile

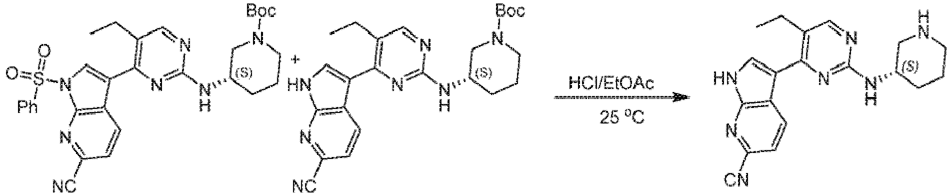

To a mixture of tert-butyl (S)-3-((4-(6-cyano-1-(phenylsulfonyl)-1H-pyrrolo[2,3-b]pyridin-3-yl)-5-ethylpyrimidin-2-yl)amino)piperidine-1-carboxylate and tert-butyl (S)-3-((4-(6-cyano-1H-pyrrolo[2,3-b]pyridin-3-yl)-5-ethylpyrimidin-2-yl)amino)piperidine-1-carboxylate (0.7 g, 1.56 mmol, 1.0 *eq,* crude) in EtOAc (5 mL) was added HCl/ EtOAc (4 M, 7.82 mL, 20 *eq*). The mixture was stirred at 25°C for 3 hrs. It was concentrated in reduced pressure at 45°C and purified by prep-HPLC: (column: Phenomenex luna (2) C18 250*50 10u; mobile phase: [water (0.225%FA)-ACN]; B%: 0%-30%, 20min) and prep-HPLC: (column: Waters Xbridge Prep OBD C18 150*30 5u; mobile phase: [water (0.225%FA)-ACN]; B96:596-2596, 12min) to afford (S)-3-(5-ethyl-2-(piperidin-3-ylamino)pyrimidin-4-yl)-1H-pyrrolo[2,3-b]pyridine-6-carbonitrile (48 mg, 132.64umol, 8.48% yield, 96% purity) as a yellow solid.

### Example 79. Synthesis of (S)-5-ethyI-4-(5-fluoro-1H-pyrrolo[2,3-b]pyridin-3-yl)-N-(piperidin-3-yl)pyrimidin-2-amine (Compound 270)

### Step 1: 3-(2-chloro-5-ethylpyrimidin-4-yl)-5-fluoro-1-tosyl-1H-pyrrolo[2,3-b]pyridine

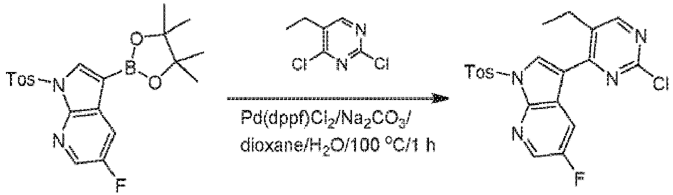

To a mixture of 5-fluoro-3-( 4,4,5,5-tetramethyl-1,3,2-dioxaborolan-2-yl)-1-tosyl-1H-pyrrolo[2,3-b]pyridine (850 mg, 2.04 mmol, 1.00 *eq*) and 2,4-dichloro-5-ethylpyrimidine (361 mg, 2.04 mmol, 1.00 *eq*) in dioxane (15 mL) and H₂O (2 mL) was added Pd(dppf)Cl₂ (149 mg, 204 umol, 0.10 *eq*) and Na₂CO₃ (432 mg, 4.08 mmol, 2.00 *eq*) in one portion at 25°C under N₂. The mixture was stirred at 100°C for 1 h. TLC (Petroleum ether: EtOAc = 1:1, R_{f} = 0.64) showed the reaction was completed. The mixture was cooled to 25°C and concentrated in reduced pressure at 45°C. The residue was poured into water (10 mL) and stirred for 1 min. The aqueous phase was extracted with EtOAc (5 mLx3). The combined organic phase was washed with brine (5 mL×1), dried with anhydrous Na₂SO₄, filtered and concentrated in vacuum. The residue was purified by silica gel chromatography (Petroleum ether: Ethyl acetate = 10:1, 1:1), to afford 3-(2-chloro-5-ethylpyrimidin-4-yl)-5-fluoro-1-tosyl-1H-pyrrolo[2,3-b]pyridine (450 mg, 1.04 mmol, 51.19% yield) as white solid.

### Step 2: tert-butyl (S)-3-((5-ethyl-4-(5-fluoro-1H-pyrrolo[2,3-b]pyridin-3-yl)pyrimidin-2-yl)amino)piperidine-1-carboxylate

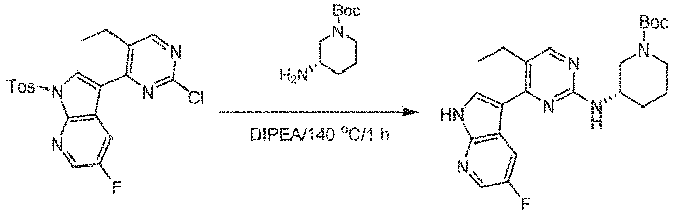

To a mixture of 3-(2-chloro-5-ethylpyrimidin-4-yl)-5-fluoro-1-tosyl-1H-pyrrolo[2,3-b]pyridine (600 mg, 1.39 mmol, 1.00 *eq*) in 2-methoxyethanol (10 mL) was added tert-butyl (S)-3-aminopiperidine-1-carboxylate (835 mg, 4.17 mmol, 3.00 *eq*) in one portion at 25°C under N₂. The mixture was stirred at 130°C for 36 hrs. LCMS showed the reaction was completed. The mixture was cooled to 25°C and added K₂CO₃ (500 mg) at 25°C and stirred for 3 hrs. The mixture was poured into water (30 mL) and stirred for 2 mins. The aqueous phase was extracted with EtOAc (10 mLx3). The combined organic phase was washed with brine (10 mL×1), dried with anhydrous Na₂SO₄, filtered and concentrated in vacuum. The single product concentrated in vacuum to afford tert-butyl (S)-3-((5-ethyl-4-(5-fluoro-1H-pyrrolo[2,3-b]pyridin-3-yl)pyrimidin-2-yl)amino)piperidine-1-carboxylate (600 mg, 585.68 umol, 42.14% yield, 43% purity) as yellow solid.

### Step 3: General procedure for preparation of (S)-5-ethyl-4-(5fluom-1H-pyrrolo[2,3-b]pyridin-3-yl)-N-(piperidin-3-yl)pyrimidin-2-amine

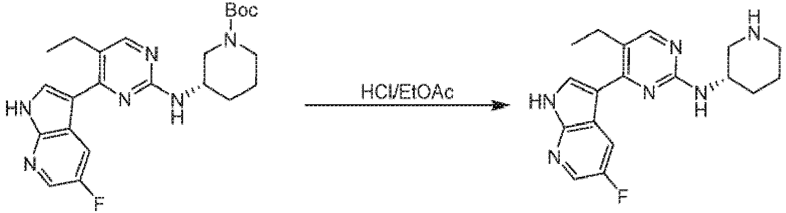

The solution of tert-butyl (S)-3-((5-ethyl-4-(5-fluoro-1H-pyrrolo[2,3-b]pyridin-3-yl)pyrimidin-2-yl)amino)piperidine-1-carboxylate (600 mg, 1.36 mmol, 1.00 *eq*) in HCl/EtOAc (30.00 mL, 4M) at 25°C under N₂ was stirred for 1 h. LCMS and HPLC showed the reaction was completed. The mixture was cooled to 25°C and concentrated in reduced pressure at 40°C. The residue was purified by prep-HPLC (column: Agela Durashell C18 150*25 5u;mobile phase: [water(10mM NH₄HCO₃)-ACN];B%: 5%-35%,12min and column: YMC-Actus Triart C18 100*30mm*5um;mobile phase: [water(10mM NH₄HCO₃)-ACN];B%: 25%-45%,12min) to afford (S)-5-ethyl-4-(5-fluoro-1H-pyrrolo[2,3-b]pyridin-3-yl)-N-(piperidin-3-yl)pyrimidin-2-amine (S)-5-ethyl-4-(5-fluoro-1H-pyrrolo[2,3-b]pyridin-3-yl)-N-(piperidin-3-yl)pyrimidin-2-amine (30 mg, 88.1 umol, 6.5%).

### Example 80. Synthesis of (S)-5-ethyl-4-(7-(methylsulfonyl)-1H-indol-3-yl)-N-(piperidin-3-yl)pyrimidin-2-amine (Compound 269)

### Step 1: 7-(methylthio)-1H-indole

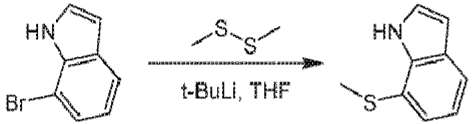

To a solution of 7-bromo-1H-indole (5.00 g, 25.5 mmol, 1.00 *eq*) in THF (100 mL) was added a solution of t-BuLi (6.13 g, 95.6 mmol, 73.5 mL, 3.75 eq) drop-wise at -78°C over a period of 30 mins under N₂ during which the temperature was maintained below -78°C. The reaction mixture was stirred at -78°C for 1 h. Then (methyldisulfanyl)methane (3.60 g, 38.2 mmol, 3.43 mL, 1.50 *eq*) in THF (50 mL) was added to the reaction mixture at -78°C. The reaction mixture was stirred at 25°C for another 4 hrs. TLC (Petroleum ether:EtOAc = 5:1, R_{f}= 0.55) showed the starting material was remained. The reaction was quenched by sat.NH₄Cl (100 mL) slowly and then extracted with EtOAc (50 mL × 3). The combined organic phase was washed with brine (25 mL × 2), dried over anhydrous Na₂SO₄, filtered and concentrated in vacuo. The residue was purified by column chromatography (Petroleum ether:EtOAc = 10:1 to 3:1) to give the pure 7-(methylthio)-1H-indole (3.52 g, 84% yield) as a light-yellow solid.

### Step 2: 7-(methylsulfonyl)-1H-indole

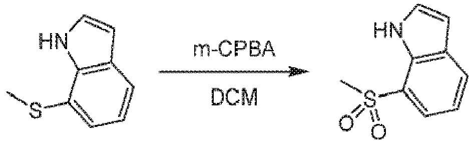

To a mixture of 7-(methylthio)-1H-indole (1.30 g, 7.96 mmol, 1.00 *eq*) in DCM (20 mL) was added m-cPBA (3.93 g, 15.9 mmol, 70% purity, 2.00 *eq*) by portions at 0°C. Then the mixture was stirred at 15°C for 12 hrs. The colour of the soluble mixture turned from white to purple. TLC (Petroleum ether: EtOAc = 1:1, R_{f}= 0.51) showed 7-(methylthio)-1H-indole was consumed and a blue-fluorescence new spot was formed. The mixture was quenched with sat.NaHCO₃ (25 mL), and extracted with EtOAc (25 mL × 3). The combined organic layers were washed with brine (25 mL), dried over Na₂SO₄ and concentrated to dryness to give a purple residue. The residue was purified by column chromatography (SiO₂, Petroleum ether:EtOAc= 50:1 to 3:1) to give 7-(methylsulfonyl)-1H-indole (1.10 g, 5.63 mmol, 71% yield) as a light-purple solid.

### Step 3: 3-(2-chloro-5-ethylpyrimidin-4-yl)-7-(methylsulfonyl)-1H-indole

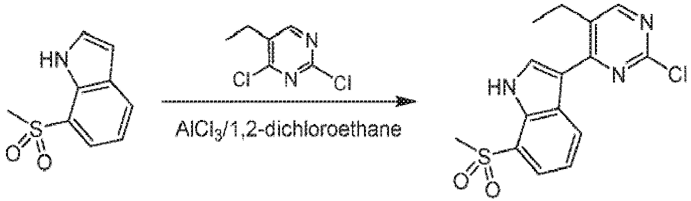

Two reactions were carried out in parallel. To a mixture of 2,4-dichloro-5-ethyl-pyrimidine (1.00 g, 5.65 mmol, 1.00 *eq)* in 1,2-dichloroethane (8.0 mL) was added AlCl₃ (791 mg, 5.93 mmol, 324 uL, 1.05 *eq*) by portions at 10°C under N₂. The mixture was stirred at 80°C for 1.5 hrs. Then 7-(methylsulfonyl)-1H-indole (805 mg, 4.12 mmol, 0.73 *eq*) in 1,2-dichloroethane (8.0 mL) was added drop-wise at 80°C. The reaction mixture was stirred for 15 hrs at 80°C. TLC showed 7-(methylsulfonyl)-IH-indole (Petroleum ether:EtOAc = 1:1, R_{f}= 0.51) remained and a new major spot (Petroleum ether: EtOAc = 111, R_{f} = 0.61) was detected. The two reactions were combined to work up. The mixture was purified by silica gel chromatography (100-200 mesh silica gel, Petroleum ether:EtOAc = 10:1, 1:1) to afford 3-(2-chloro-5-ethylpyrimidin-4-yl)-7-(methylsulfonyl)-1H-indole (600 mg, 14% yield, 92% purity) as a yellow solid

### Step 4: tert-butyl (S)-3-((5-ethyl-4-(7-(methylsulfonyl)-1H-indol-3-yl)pyrimidin-2-yl)amino)piperidine-1-carboxylate

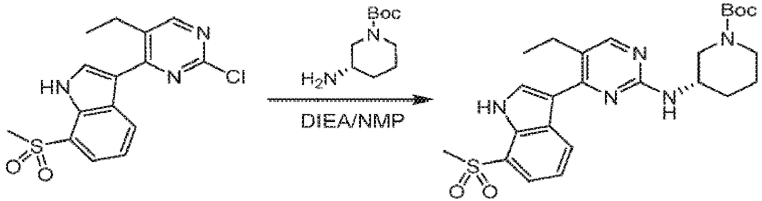

A mixture of 3-(2-chloro-5-ethylpyrimidin-4-yl)-7-(methylsulfonyl)-1H-indole (200 mg, 595 umol, 1.00 *eq*), tert-butyl (S)-3-aminopiperidine-1-carboxylate (119 mg, 595 umol, 1.00 *eq*), DIEA (385 mg, 2.98 mmol, 520 uL, 5.00 *eq*) in NMP (3.00 mL) was stirred at 140°C for 1 h under N₂. TLC showed most of 3-(2-chloro-5-ethylpyrimidin-4-yl)-7-(methylsulfonyl)-1-H-indole remained. Then the reaction was stirred at 140°C for another 10 hrs under M.W. under N₂. TLC showed the reaction was complete and a new major spot (Petroleum ether. EtOAc = 1:1, R_{f} = 0.32) was detected. The reaction was diluted with 50 mL H₂O and extracted with EtOAc (10 mL × 3). The combined organic layers were washed with brine (10 mL × 3), dried over Na₂SO₄, filtered and concentrated to dryness to give tert-butyl (S)-3-((5-ethyl-4-(7-(methylsulfonyl)-1H-indol-3-yl)pyrimidin-2-yl)amino)piperidine-1-carboxylate (150 mg, 35% yield, 70% purity) as a brown oil which was used directly without further purification.

### Step 5: (S)-5-ethyl-4-(7-(methylsulfonyl)-1H-indol-3-yl)-N-(piperidin-3-yl)pyrimidin-2-amine

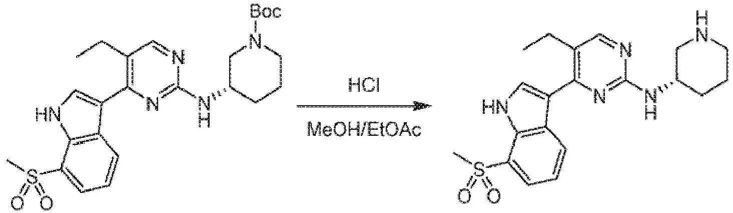

To a mixture of tert-butyl (S)-3-((5-ethyl-4-(7-(methylsulfonyl)-1H-indol-3-yl)pyrimidin-2-yl)amino)piperidine-1-carboxylate (200 mg, 400 umol, 1.00 *eq*) in MeOH (2 mL) and EtOAc (5 mL) was added HCl/EtOAc (5 mL, 4 M) drop-wise. Then the mixture was stirred at 15°C for 0.5 h. TLC (Petroleum ether:EtOAc = 1:1, R_{f}= 0.0) showed the reaction was completed. The reaction was concentrated to dryness. The residue was purified by prep-HPLC (column: Waters Xbridge 150 × 25 5u; mobile phase: [water(10mM NH₄HCO₃)-ACN]; B%: 10%-40%,11min) to give (S)-5-ethyl-4-(7-(methylsulfonyl)-1H-indol-3-yl)-N-(piperidin-3-yl)pyrimidin-2-amine (25.0 mg, 18% yield, 98% purity) as a white solid.

### Example 81. Synthesis of (S)-3-(5-ethyl-2-(piperidin-3-ylamino)pyrimidin-4-yl)-1H-indazole-6-carbonitiile (Compound 275)

### Step 1: 3-iodo-1H-indazole-6-carbonitrile

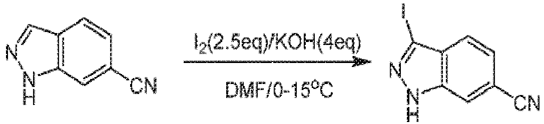

To a solution of 1H-indazole-6-carbonitrile (8.00 g, 55.9 mmol, 1.00 eq) and I₂ (35.5 g, 140 mmol, 28 mL, 2.50 eq) in DMF (56 mL) was added KOH (12.5 g, 224 mmol, 4.00 eq) at 0°C. The mixture was stirred at 15°C for 6 hrs under N₂. TLC (Petroleum ether: EtOAc = 5:1, R_{f}= 0.43) indicated one major new spot with larger polarity was detected. The reaction mixture was quenched with saturated Na₂S₂O₃ solution (80 mL), and filtered to give a residue. The residue was purified by column chromatography (SiO₂, Petroleum ether. EtOAc = 3:1). 3-iodo-1H-indazole-6-carbonitrile (2.70 g, 18% yield) was obtained as a white solid.

### Step 2: 3-iodo-1-(tetrahydro-2H-pyran-2-yl)-1H-indazole-6-carbonitrile

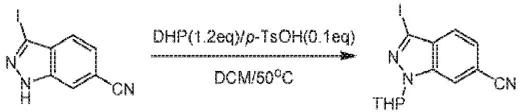

A mixture of 3-iodo-1H-indazole-6-carbonitrile (2.70 g, 10.0 mmol, 1.00 eq), DHP (1.01 g, 12.0 mmol, 1.10 mL, 1.20 eq), p-TsOH (173 mg, 1.00 mmol, 0.10 eq) in DCM (25 mL) was degassed and purged with N₂ for 3 times, and then the mixture was stirred at 50°C for 5 hrs under N₂ atmosphere. TLC (Petroleum ether: EtOAc = 3:1, R_{f}= 0.43) indicated one major new spot with larger polarity was detected. The reaction mixture was quenched by addition of NaHCO₃ (20 mL) at 0°C, and then extracted with EtOAc (25 mL × 2). The combined organic layers were washed with brine (10 mL), dried over Na₂SO₄, filtered and concentrated under reduced pressure to give a residue. The residue was purified by column chromatography (SiO₂, Petroleum ether: EtOAc = 3:1). 3-iodo-1-(tetrahydro-2H-pyran-2-yl)-1H-indazole-6-carbonitrile (2.00 g, 56% yield) was obtained as a white solid.

### Step 3: 3-(2-chloro-5-ethylpyrimidin-4-yl)-1-(tetrahydro-2H-pyran-2-yl)-1H-indazole-6-carbonitrile

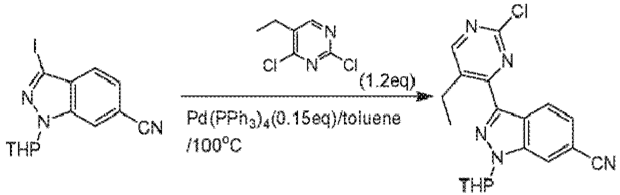

A mixture of 3-iodo-1-(tetrahydro-2H-pyran-2-yl)-1H-indazole-6-carbonitrile (1.00 g, 2.83 mmol, 1.00 eq), 2,4-dichloro-5-ethylpyrimidine (602 mg, 3.40 mmol, 1.2 eq), trimethyl(trimethylstannyl)stannane (928 mg, 2.83 mmol, 587 uL, 1.00 eq), Pd(PPh₃)₄ (491 mg, 425 umol, 0.15 eq) in toluene (10 mL) was degassed and purged with Ar for 3 times, and then the mixture was stirred at 100°C for 12 hrs under Ar atmosphere. TLC (Petroleum ether. EtOAc = 3:1, R_{f}= 0.43) indicated one major new spot with larger polarity was detected. The reaction mixture was filtered and concentrated under reduced pressure to give a residue. The residue was purified by column chromatography (SiO₂, Petroleum ether: EtOAc = 3: 1). 3-(2-chloro-5-ethylpyrimidin-4-yl)-1-(tetrahydro-2H-pyran-2-yl)-1H-indazole-6-carbonitrile (0.5 g, 34% yield) was obtained as a yellow solid.

### Step 4: tert-butyl (3S)-3-((4-(6-cyano-1-(tetrahydro-2H-pyran-2-yl)-1H-indazol-3-yl)-5-ethylpyriniidin-2-yl)amino)piperidine-1-carboxylate

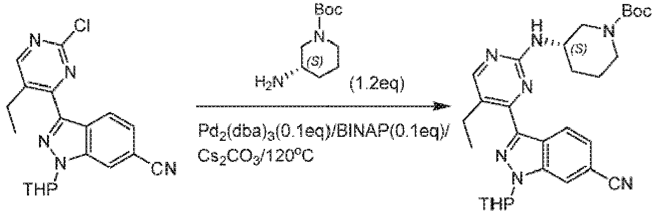

A mixture of 3-(2-chloro-5-ethylpyrimidin-4-yl)-1-(tetrahydro-2H-pyran-2-yl)-1H-indazole-6-carbonitrile (0.26 g, 707 umol, 1.00 eq), tert-butyl (S)-3-aminopiperidine-1-carboxylate (170 mg, 848 umol, 1.20 eq), Pd₂(dba)₃ (64.7 mg, 70.7 umol, 0.10 eq), BINAP (44.0 mg, 70.7 umol, 0.10 eq) and Cs₂CO₃ (461 mg, 1.41 mmol, 2.00 eq) in dioxane (10 mL) was degassed and purged with N₂ for 3 times, and then the mixture was stirred at 120°C for 5 hrs under N₂ atmosphere. TLC (Petroleum ether: EtOAc = 1: 1, R_{f}= 0.24) and HPLC indicated one major new spot with larger polarity was detected. The reaction mixture was added silicone to remove Pd₂(dba)₃ (64.7 mg, 70.7 umol, 0.10 eq), the mixture was stirred 30 mins then was filtered and the solvent was concentrated under reduced pressure to give a residue. The residue was purified by prep-TLC (Petroleum ether: EOAc = 1: 1). Tert-butyl (3S)-3-((4-(6-cyano-1-(tetrahydro-2H-pyran-2-yl)-1H-indazol-3-yl)-5-ethylpyrimidin-2-yl)amino)piperidine-1-carboxylate (0.15 g, 282 umol, 40% yield) was obtained as red oil.

### Step 5: (S)-3-(5-ethyl-2-(piperidin-3-ylamino)pyrimidin-4-yl)-1H-indazole-6-carbonitrile

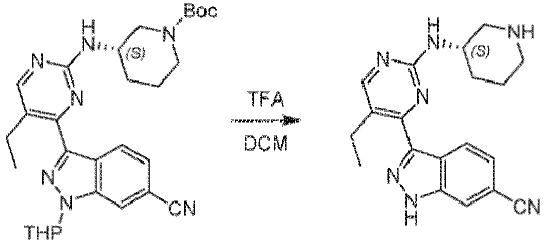

To a solution of tert-butyl (3S)-3-((4-(6-cyano-l-(tetrahydro-2H-pyran-2-yl)-1H-indazol-3-yl)-5-ethylpyrimidin-2-yl)amino)piperidine-1-carboxylate (0.15 g, 282 umol, 1 eq) in DCM (5 mL) was added TFA (3.08 g, 27.0 mmol, 2 mL, 95.7 eq) at 0°C. The mixture was stirred at 10°C for 48 hrs. LCMS showed desired compound was detected. The reaction mixture was quenched by addition of NaHCOa (20 mL) at 0°C, and then extracted with EtOAc (20 mL × 2). The combined organic layers were washed with brine (10 mL) and concentrated under reduced pressure to give a residue. The residue was purified by prep-HPLC (column: YMC-Actus ODS-AQ 100 × 30 5u; mobile phase: [water (0.225%FA)-ACN]; B%: 18%-48%, 12min). (S)-3-(5-ethyl-2-(piperidin-3-ylamino)pyrimidin-4-yl)-1H-indazole-6-carbonitrile (0.025 g, 26% yield, 100% purity) was obtained as a white solid.

### Example 82. Synthesis of (S)-3-(5-isopropyl-2-(piperidin-3-ylamino)pyrimidin-4-yl)-1H-pyrazolo[3,4-b]pyridine-6-carbonitrile (Compound 276)

### Step 1: Methyl 2-formyl-3-methylbutanoate

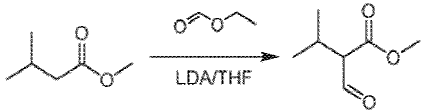

To a solution of methyl 3-methylbutanoate (30.0 g, 258 mmol, 34.1 mL, 1.00 eq) in THF (300 mL) was added LDA (2 M, 129 mL, 1.00 eq) drop-wise at -40°C. The mixture was stirred for 30 mins at -40°C. Then ethyl formate (76.5 g, 1.03 mol, 83.2 mL, 4.00 eq) was added drop-wise at -60°C. The mixture was stirred for 12 hrs at 15°C. The reaction mixture was quenched by addition satNH₄Cl (300 mL) at 0°C, and then extracted with EtOAc (300 mL × 3). The combined organic layers were washed with brine (300 mL), dried over Na₂SO₄, filtered and the filtrate was concentrated under reduced pressure to give methyl 2-formyl-3-methylbutanoate (37.2 g, crude) as a yellow oil.

### Step 2: 5-isopropyl-2-thioxo-2,5-dihydropyrimidin-4(3H)-one

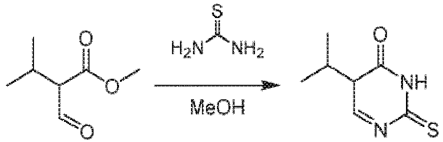

To a mixture of methyl 2-formyl-3-methylbutanoate (37.0 g, 257 mmol, 1.00 eq) and thiourea (19.5 g, 257 mmol, 1.00 eq) in MeOH (150 mL) with the exclusion of moisture was stirred at 65°C for 6 hrs. LCMS showed one main peak with desired MS was detected. The reaction mixture was cooled in an ice/salt bath under vigorous stirring, acidified (drop-wise) with 20% aqueous HCl to pH = 3. The acidified reaction mixture was left in the ice/salt bath for another 0.5-1 h, then a yellow solid which separated, was collected with suction and dried under reduced pressure to give 5-isopropyl-2-thioxo-2,5-dihydropyrimidin-4(3H)-one (30.0 g, 176 mmol, 68% yield) as a yellow solid.

### Step 3: 2,4-dichloro-5-isopropylpyrimidine

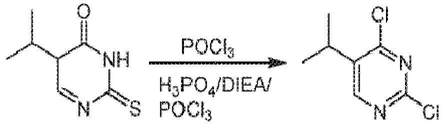

5-isopropyl-2-thioxo-2,5-dihydropyrimidin-4(3H)-one (15.0 g, 88.1 mmol, 1.00 eq) was suspended in POCl₃ (60.0 mL) and H_{3P}O₄ (949.89 mg, 9.69 mmol, 565.41 uL, 0.11 eq) was added, followed by DIEA (12.53 g, 96.93 mmol, 16.93 mL, 1.10 eq) dropwise. The reaction mixture was slowly heated to 100°C and stirred for 12 hrs. TLC (Petroleum ether:EtOAc= 1:1, R_{f} = 0.6) showed one new spot formed. The reaction mixture was cooled to room temperature. Then the mixture was added drop-wise to a mixture of heptane (200 mL) and warm water (200 mL) below 45°C. Then separated and the aqueous phase was extracted with heptane (100 mL × 3). The combined organic layers were washed with brine (100 mL × 1), dried over Na₂SO₄, filtered and concentrated under reduced pressure to give 2,4-dichloro-5-isopropylpyrimidine (15.0 g, 78.5 mmol, 89.1% yield) as a yellow oil.

### Step 4: 6-chloro-1-(tetrahydro-2H-pyran-2-yl)-1H-pyrazolo[3,4-b]pyridine

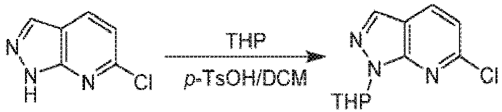

To a solution of 6-chloro-1H-pyrazolo[3,4-b]pyridine (10 g, 65.1 mmol, 1.00 eq) in DCM (200 mL) was added 3,4-dihydro-2H-pyran (6.57 g, 78.1 mmol, 7.14 mL, 1.20 eq) followed by p-toluenesulfonic acid monohydrate (1.24 g, 6.51 mmol, 0.10 eq) and the reaction mixture was then stirred at 30°C for 3 hrs. TLC (Petroleum ether: EtOAc = 1:1, R_{f}= 0.43) showed one new spot formed. The mixture was washed with sat.NaHCO₃ (100 mL x2) and brine (100 mL), dried over Na₂SO₄, filtered and the filtrate was evaporated to give 6-chloro-1-(tetrahydro-2H-pyran-2-yl)-1H-pyrazolo[3,4-b]pyridine (15 g, crude) as a yellow oil.

### Step 5: 1-(tetrahydro-2H-pyran-2-yl)-1H-pyrazolo[3,4-b]pyridine-6-carbonitrile

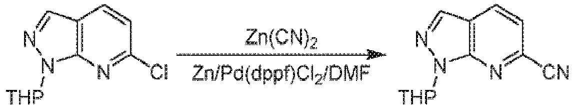

To a mixture of 6-chloro-1-(tehahyd ro-2H-pyran-2-yl)-1H-pyracolo[3,4-b]pyridine (15 g, 63.1 mmol, 1.00 eq), Zn (413 mg, 6.31 mmol, 0.10 eq), Zn(CN)₂ (5.19 g, 44.2 mmol, 2.80 mL, 0.70 eq) and Pd(dppf)Cl₂.CH₂Cl₂ (2.58 g, 3.16 mmol, 0.05 eq) in DMF (105 mL) was degassed under vacuum and purged with N₂ three times. The mixture was stirred at 140°C for 5 hrs. TLC (Petroleum ether:EtOAc = 1:1, R_{f} = 0.61) showed one new spot formed. The reaction mixture was diluted with H₂O (300 mL) and filtered, the filtrate was extracted with EtOAc (200 mL × 3). The combined organic layers were washed with brine (100 mL), dried over Na₂SO₄, filtered and the filtrate was concentrated under reduced pressure to give a residue. The residue was purified by column chromatography (SiO₂, Petroleum ether.EtOAc = 100:1 to 1:1) to give 1-(tetrahydro-2H-pyran-2-yl)-1H-pyrazolo[3,4-b]pyridine-6-carbonitrile (12 g, 52.6 mmol, 83% yield) as a yellow solid.

### Step 6: 1H-pyrazolo[3,4-b]pyridine-6-carbonitrile

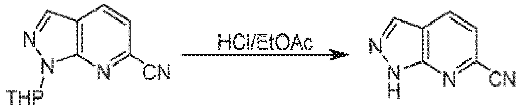

To a solution of 1-(tetrahydro-2H-pyran-2-yl)-1H-pyrazolo[3,4-b]pyridine-6-carbonitrile (12 g, 52.6 mmol, 1.00 eq) in EtOAc (120 mL) was added HCl/ EtOAc (100 mL, 4M) drop-wise at 15°C over a period of 10 mins. The reaction mixture was stirred at 15°C for 2 hrs. TLC (Petroleum ether:EtOAc = 1:1, R_{f} = 0.44) showed one new spot formed. The reaction mixture was filtered and the filtered cake was concentrated under reduced pressure to give 1H-pyrazolo[3,4-b]pyridine-6-carbonitrile (5.1 g, 35.4 mmol, 67% yield) as a white solid.

### Step 7.· 3-iodo-1H-pyrazolo[3,4-b]pyridine-6-carbonitrile

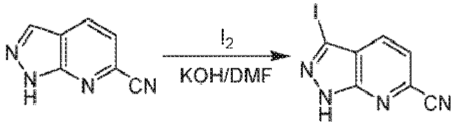

1H-pyrazolo[3,4-b]pyridine-6-carbonitrile (5.1 g, 35.4 mmol, 1.00 eq) was dissolved in DMF (60 mL). I₂ (18.0 g, 70.8 mmol, 14.3 mL, 2 eq) and KOH (7.94 g, 141 mmol, 4 eq) were added. The reaction mixture was stirred at 140°C for 3 hrs. TLC (Petroleum ether: EtOAc = 1:1, R_{f} = 0.53) showed one new spot formed. The reaction mixture was quenched by addition 10% Na₂SO₃ 180 mL at 15°C, then a solid formed. The suspension was filtered and the filtered cake was concentrated under reduced pressure to give 3-iodo-1H-pyrazolo[3,4-b]pyridine-6-carbonitrile (7.1 g, 26.3 mmol, 74% yield) as a brown solid.

### Step 8: 3-iodo-1-(tetrahydro-2H-pyran-2-yl)-1H-pyrazolo[3,4-b]pyridine-6-carbonitrile

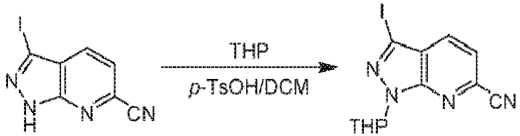

To a solution of 3-iodo-1H-pyrazolo[3,4-b]pyridine-6-carbonitrile (7.1 g, 26.3 mmol, 1.00 eq) in DCM (70 mL) was added 3,4-dihydro-2H-pyran (2.65 g, 31.6 mmol, 2.88 mL, 1.20 eq) followed by p-toluenesulfonic acid monohydrate (500 mg, 2.63 mmol, 0.10 eq) and the reaction mixture was stirred at 25°C for 3 hrs. TLC (Petroleum ether:EtOAc = 2:1, R_{f}= 0.43) showed one new spot formed. The mixture was washed with sat.NaHCO₃ (30 mL × 2) and brine (20 mL), dried over Na₂SO₄, filtered and the filtrate was evaporated to give a residue. The residue was purified by column chromatography (SiO₂, Petroleum ether. EtOAc= 100: I to 1:1) to give 3-iodo-1-(tetrahydro-2H-pyran-2-yl)-1H-pyrazolo[3,4-b]pyridine-6-carbonitrile (5.9 g, 16.7 mmol, 63% yield) as a yellow solid.

### Step 9: 3-(2-chloro-5-isopropylpyrimidin-4-yl)-1-(tetrahydro-2H-pyran-2-yl)-1H-pyrazolo[3,4-b]pyridine-6-carbonitrile

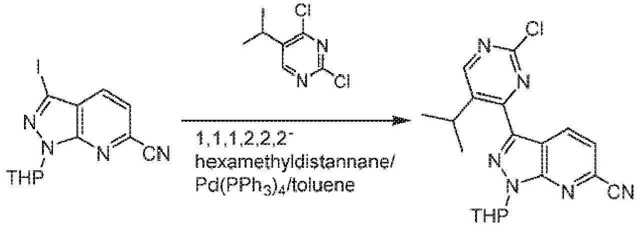

A mixture of 3-iodo-1-(tetrahydro-2H-pyran-2-yl)-1-H-pyrazolo[3,4-b]pyridine-6-carbonitrile (2 g, 5.65 mmol, 1 eq), 2,4-dichloro-5-isopropylpyrimidine (1.29 g, 6.78 mmol, 1.2 eq), 1,1,1,2,2,2-hexamethyldistannane (1.85 g, 5.65 mmol, 1.17 mL, 1 eq), Pd(PPh₃)₄ (653 mg, 565 umol, 0.1 eq) in toluene (20 mL) was degassed and purged with N₂ for 3 times, and then the mixture was stirred at 100°C for 12 hrs under N₂ atmosphere. LCMS showed desired MS was detected. The reaction mixture was concentrated under reduced pressure to remove toluene. The residue was purified by column chromatography (SiO₂, Petroleum ether.EtOAc = 100:1 to 1:1, R_{f} = 0.38) to give 3-(2-chloro-5-isopropylpyrimidin-4-yl)-1-(tetrahydro-2H-pyran-2-yl)-1H-pyrazolo[3,4-b]pyridine-6-carbonitrile (310 mg, 810 umol, 14% yield) as a yellow solid.

### Step 10: Tert-butyl(3S)-3-((4-(6-cyano-1-(tetrahydro-2H-pyran-2-yl)-1H-pyrazolo[3,4-b]pyridin-3-yl)-5-isopropylpyrimidin-2-yl)amino)piperidine-1-carboxylate

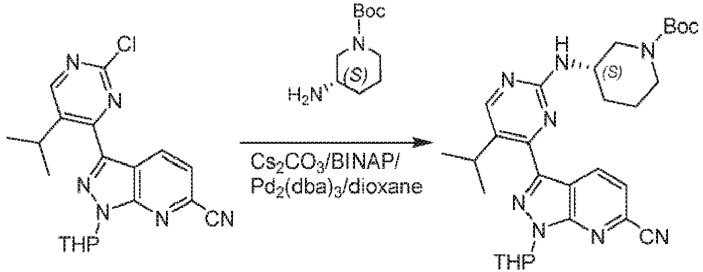

A mixture of 3-(2-chloro-5-isopropylpyrimidin-4-yl)-1-(tetrahydro-2H-pyran-2-yl)-1H-pyrazolo[3,4-b]pyridine-6-carbonitrile (310 mg, 810 umol, 1 eq), tert-butyl (S)-3-aminopiperidine-1-carboxylate (195 mg, 972 umol, 1.2 eq), Pd₂(dba)₃ (74.2 mg, 81.0 umol, 0.1 eq), BINAP (50.4 mg, 81.0 umol, 0.1 eq) and Cs₂CO₃ (528 mg, 1.62 mmol, 2 eq) in dioxane (10 mL) was degassed and purged with N₂ for 3 times at 20°C, and then the mixture was stirred at 100°C for 12 hrs under N₂ atmosphere. TLC (Petroleum ether.EtOAc = 2:1, R_{f}= 0.19) indicated 3-(2-chloro-5-isopropylpyrimidin-4-yl)-1-(tetrahydro-2H-pyran-2-yl)-1H-pyrazolo[3,4-b]pyridine-6-carbonitrile was consumed completely. The reaction mixture was filtered and the filtrate was diluted with H₂O (20 mL) and extracted with EtOAc (20 mL × 3). The combined organic layers were washed with brine (20 mL), dried over Na₂SO₄, filtered and the filtrate was concentrated under reduced pressure to give a residue. The residue was purified by column chromatography (SiO2, Petroleum ether:EtOAc=100:1 to 1:1) to give tert-butyl (3S)-3-((4-(6-cyano-1-(tetrahydro-2H-pyran-2-yl)-1H-pyrazolo[3,4-b]pyridin-3-yl)-5-isopropylpyrimidin-2-yl)amino)piperidine-1-carboxylate (250 mg, 208 umol, 25.6% yield) as a yellow solid.

### Step 11: (S)-3-(5-isopropyl-2-(piperidin-3-ylamino)pyrimidin-4-yl)-1H-pyrazolo[3.4-b]pyridine-6-carbonitrile

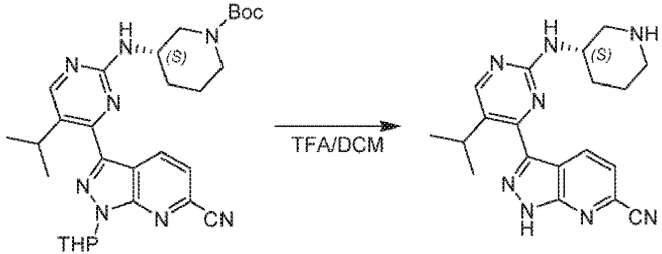

To a solution of tert-butyl (3S)-3-((4-(6-cyano-1-(tetrahydro-2H-pyran-2-yl)-1H-pyrazolo[3,4-b]pyridin-3-yl)-5-isopropylpyrimidin-2-yl)amino)piperidine-1-carboxylate (250 mg, 457 umol, 1 eq) in DCM (9 mL) was added TFA (3 mL) drop-wise at 15°C. The mixture was stirred at 15°C for 12 hrs. LCMS showed desired MS was detected. The reaction mixture was concentrated under reduced pressure to remove DCM. The residue was purified by prep-HPLC (neutral condition, column: YMC-Actus Triart C18 150*30 5u;mobile phase: [water(10mM NH₄HCO₃)-ACNI;B%: 15%-55%,10min) to give (S)-3-(5-isopropyl-2-(piperidin-3-ylamino)pyrimidin-4-yl)-1H-pyrazolo[3,4-b]pyridine-6-carbonitrile (38 mg, 102 umol, 22% yield, 97.024% purity) as a white solid.

### Example 83. Synthesis of (S)-3-(5-ethyl-2-(piperidin-3-ylamino)pyrimidin-4-yl)-1H-pyrazolo[3,4-b]pyridine-6-carbonitrile (Compound 277)

### Step 1: 3-(2-chloro-5-ethylpyrimidin-4-yl)-1-(tetrahydro-2H-pyran-2-yl)-1H-pyrazolo[3,4-b]pyridine-6-carbonitrile

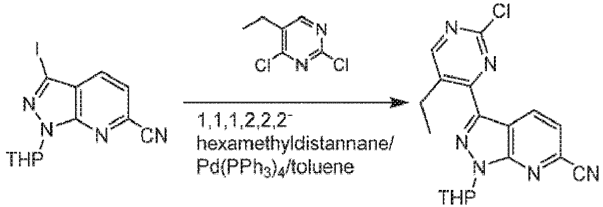

A mixture of 3-iodo-1-(tetrahydro-2H-pyran-2-yl)-1H-pyrazolo[3,4-b]pyridine-6-carbonitrile (1.93 g, 5.45 mmol, 1 *eq*), 2,4-dichloro-5-ethylpyrimidine (1.16 g, 6.54 mmol, 1.2 *eq*), 1,1,1,2,2,2-hexamethyldistannane (1.79 g, 5.45 mmol, 1 *eq*), Pd(PPh₃)₄ (630 mg, 545 umol, 0.1 *eq*) in toluene (20 mL) was degassed and purged with N₂ for 3 times, and then the mixture was stirred at 100°C for 12 hrs under N₂ atmosphere. LCMS showed desired MS was detected. The reaction mixture was concentrated under reduced pressure to remove toluene. The residue was purified by column chromatography (SiO₂, Petroleum ether.EtOAc= 100:1 to 1:1) to give 3-(2-chloro-5-ethylpyrimidin-4-yl)-1-(tetrahydro-2H-pyran-2-yl)-1H-pyrazolo[3,4-b]pyridine-6-carbonitrile (730 mg, 1.98 mmol, 36% yield) as a yellow solid.

### Step 2: Tert-butyl(3S)-3-((4-(6-cyano-1-(tetrahydro-2H-pyran-2-yl)-1H-pyrazolo[3,4-b]pyridin-3-yl)-5-ethylpyrimidin-2-yl)amino)piperidine-1-carboxylate

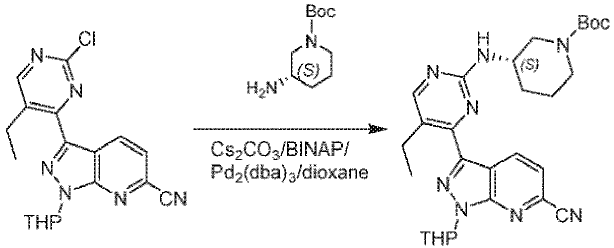

A mixture of 3-(2-chloro-5-ethylpyrimidin-4-yl)-1-(tetrahydro-2H-pyran-2-yl)-1H-pyrazolo[3,4-b]pyridine-6-carbonitrile (730 mg, 1.98 mmol, 1 eq), tert-butyl (S)-3-aminopiperidine-1-carboxylate (476 mg, 2.38 mmol, 1.2 eq), Pd₂(dba)₃ (181 mg, 197 umol, 0.1 eq), BINAP (123 mg, 197 umol, 0.1 eq) and Cs₂CO₃ (1.29 g, 3.96 mmol, 2 eq) in dioxane (15 mL) was degassed and purged with N₂ for 3 times at 20°C, and then the mixture was stirred at 100°C for 12 hrs under N₂ atmosphere. LCMS showed desired MS was detected. The reaction mixture was filtered and the filtrate was diluted with H₂O (20 mL) and extracted with EtOAc (20 mL × 3). The combined organic layers were washed with brine (20 mL), dried over Na₂SO₄, filtered and the filtrate was concentrated under reduced pressure to give a residue. The residue was purified by column chromatography (SiO₂, Petroleum ether:EtOAc = 100:1 to 1:1) to give tert-butyl (3S)-3-((4-(6-cyano-1-(tetrahydro-2H-pyran-2-yl)-1H-pyrazolo[3,4-b]pyridin-3-yl)-5-ethylpyrimidin-2-yl)amino)piperidine-1-carboxylate (700 mg, 665 umol, 3396 yield, 50.6% purity) as a yellow solid.

### Step 3: (S)-3-(5-ethyl-2-(piperidin-3-ylamino)pyrimidin-4-yl)-1H-pyrazolo[3,4-b]pyridine-6-carbonitrile

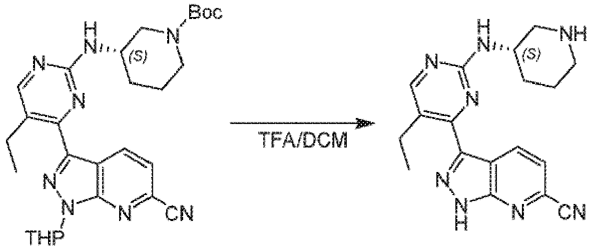

To a solution of tert-butyl (3S)-3-((4-(6-cyano-1-(tetrahydro-2H-pyran-2-yl)-1H-pyrazolo[3,4-b]pyridin-3-yl)-5-ethylpyrimidin-2-yl)amino)piperidine-1-carboxylate (100 mg, 1.31 mmol, 1 eq) in DCM (12 mL) was added TFA (4 mL) dropwise at 0°C. The mixture was stirred at 15°C for 12 hrs. LCMS showed desired MS was detected. The reaction mixture was concentrated under reduced pressure to remove DCM. The residue was purified by prep-HPLC (neutral condition, column: Phenomenex luna(2) C18 250*50 10u; mobile phase: [water(0.1%TFA)-ACN];B%: 15%-45%,20min) to give (S)-3-(5-ethyl-2-(piperidin-3-ylamino)pyrimidin-4-yl)-1H-pyrazolo[3,4-b]pyridine-6-carbonitrile (50 mg, 137.00 umol, 10% yield, 95.4% purity) as a light yellow solid.

### Example 84. Synthesis of (3R,5S)-tert-butyl3-([1,2,4]triazolo[4,3-a]pyridin-3-yl)-5-((4-(1-(phenylsulfonyl)-1H-indol-3-yl)-5-(trifluoromethyl)pyrimidin-2-yl)amino)piperidine-1-carboxylate (A) and (3S,5R)-tert-butyl 3-([1,2,4]triazolo[4,3-a]pyridin-3-yl)-5-((4-(1-(phenylsulfonyl)-1H-indol-3-yl)-5-(trifluoromethyl)pyrimidin-2-yl)amino)piperidine-1-carboxylate (B) Intermediates Useful in the Synthesis of Compounds 267 and 268.

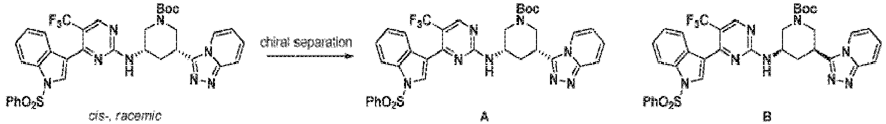

*Cis-tert*-butyl 3-([1,2,4]triazolo[4,3-a]pyridin-3-yl)-5-((4-(1-(phenylsulfonyl)-1H-indol-3-yl)-5-(trifluoromethyl)pyrimidin-2-yl)amino)piperidine-1-carboxylate (250 mg, 0.348 mmol) was separated using a ChiralPak IB with 5.000 ul injections and 15:15:70 MeOH:DCM:Hexane for elution to give Peak 1 (A, tentatively assigned as (3*R*,5*S*)): 85 mg, pale yellow solid, 99.9% *e.e.,* and Peak 2 (**B**, tentatively assigned as (3S,5R)): 56 mg, pale yellow solid, 97.1% *e.e.*

### Example 85. Synthesis of (S)-tert-butyl 3-((5-ethyl-4-(6-(methylsulfonyl)-1H-indol-3-yl)pyrimidln-2-yl)amino)piperidine-1-carboxylate Intermediate Useful in the Synthesis of Compound 271.

### Step 1: (S)-tert-butyl 3-((4-(6-bromo-1H-indol-3-yl)-5-ethylpyrimidin-2-yl)amino)piperidine-1-carboxylate

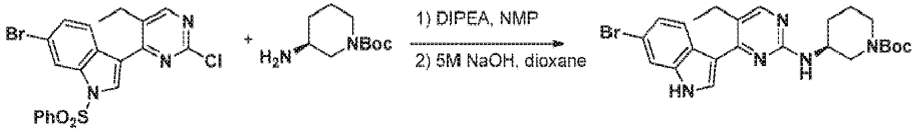

In a sealed vial, a solution of 6-bromo-3-(2-chloro-5-ethylpyrimidin-4-yl)-1-(phenylsulfonyl)-1H-indole (370 mg, 0.776 mmol), (*S*)-*tert*-butyl 3-aminopiperidine-1-carboxylate (223 mg, 1.164 mmol), and DIPEA (0.54 mL, 3.10 mmol) in NMP (0.8 mL, 1 M) was heated for 16 h at 130 °C in an oil bath. The mixture was then diluted with MeTHF (20 mL), washed with water (2x20 mL) and brine (20 mL), dried over Na₂SO₄, filtered, and concentrated under reduced pressure. The residue was dissolved in 1,4-dioxane (2 mL), and 5 M NaOH (3 mL) was added. The reaction mixture was then stirred at 50 °C for 3 h until full conversion. The reaction mixture was cooled down to RT, and crude product was then extracted with MeTHF (3x15 mL). Combined organic extract was washed with brine (10 mL), dried over Na₂SO₄, filtered, and concentrated under reduced pressure. The residue was purified by SiO₂ chromatography (EtOAc in a (1:1) mixture of DCM and Hexanes, 0 to 30% gradient). The title compound (250 mg, 0.50 mmol, 64% yield) was obtained as a pale brown solid.

### Step 2: (S)-tert-butyl3-((5-ethyl-4-(6-(methylsulfonyl)-1H-indol-3yl)pyrimidin-2-yl)amino)piperidine-1-carboxylate

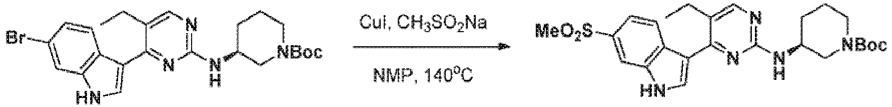

To a solution of (*S*)-*tert*-butyl 3-((4-(6-bromo-1H-indol-3-yl)-5-ethylpyrimidin-2-yl)amino)piperidine-1-carboxylate (160 mg, 0.32 mmol) in NMP (2 mL), CuI (237 mg, 1.247 mmol) and sodium methanesulfinate (131 mg, 1.279 mmol) were added. Nitrogen was bubbled through the reaction mixture for approximately one minute and then the mixture, in a sealed vial, was heated at 140 °C. After 5 h, heating was stopped (longer hearting cause decomposition). The reaction mixture was purified directly by reverse phase chromatography (C18, MeCN in aq.10 mM ammonium formate, pH 3.8, 0 to 100% gradient) to afford the title compound (35 mg, 0.070 mmol, 22% yield) as a brown solid.

### Example 8b. Synthesis of 3-bromo-1-methyl-1H-pyrrolo[2,3-b]pyridine-6-carbonitrile Intermediate Useful in the Synthesis of Compound 273. For compound 231 (SY-4464).

### Step 1: 1-methyl-1H-pyrrolo[2,3-b]pyridine-6-carbonitrile

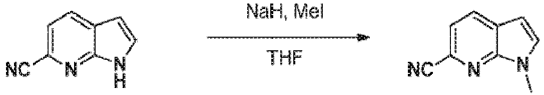

To a solution of 1 H-pyrrolo[2,3-b]pyridine-6-carbonitrile (WO2011128455) (100 mg, 0.70 mmol) in THF (3.5 mL) at 0°C, was added NaH (60% in mineral oil, 31 mg, 0.77 mmol), and the reaction mixture was stirred for 30 minutes, warming to RT. Methyl iodide (46 uL, 0.73 mmol) was added, and the reaction mixture was stirred at RT for 1h, then concentrated under reduced pressure, diluted with MeTHF (10 mL) and water (5 mL) and extracted with MeTHF (2 × 5 mL). The organics were combined and washed with brine (2 × 5 mL), dried over Na₂SO₄, filtered, and concentrated under reduced pressure to give the title compound (114 mg, 0.70 mmol, quantitative yield) as a pale orange solid, which was used without further purification.

### Step 2: 3-bromo-1-methyl-1H-pyrrolo[2,3-b]pyridine-6-carbonitrile

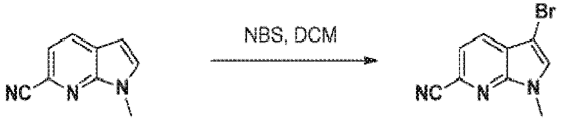

To a solution of 1-methyl-1H-pyrrolo[2,3-b]pyridine-6-carbonitrile (189 mg, 0.93 mmol) in DCM (3.5 mL) at 0 °C, was added NBS (180 mg, 1.01 mmol), and the reaction mixture was stirred for 2h at RT. The reaction was quenched by the addition of saturated Na₂S₂O₃ (5 mL) and diluted with water (5 mL), then extracted with DCM (3 × 10 mL). The organics were combined and washed with Na₂S₂O₃ (1x5 mL), water (2 × 10 mL) and brine (2x5 mL), dried over Na₂SO₄, filtered, and concentrated under reduced pressure to give the title compound (219 mg, 82.8 mmol, 82% yield) as a pale yellow solid.

### Example 87. Synthesis of 1-(methoxymethyl)-1H-pyrrolo[2,3-b]pyridine-6-carbonitrile Intermediate Useful in the Synthesis of Compound 283.

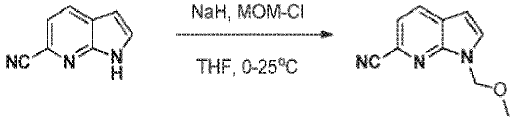

To a solution of 1H-pyrrolo[2,3-b]pyridine-6-carbonitrile (WO2011128455) (150 mg, 1.05 mmol) in THF (5.2 mL) at 0 °C, was added NaH (60% in mineral oil, 46 mg, 1.15 mmol), and the reaction mixture was stirred for 30 minutes, warming to RT. Chloromethyl methyl ether (MOM-Cl) (80 uL, 1.05 mmol) was added, and the reaction mixture was stirred at RT for 1 h, then quenched with sat. NaHCO₃ (0.1 mL) and concentrated under reduced pressure. The residue was diluted with water (10 mL), extracted with DCM (3 × 10 mL), and the collected organics were washed with (1:1) water/NaHCO₃ (2 × 10 mL) and brine (2x5 mL), dried over Na₂SO₄, filtered, and concentrated under reduced pressure to give the title compound (189 mg, 0.93 mmol, 87% yield) as a dark brown solid, which was used without further purification.

### Example 8$. Synthesis of (S)-4-(6-bromo-1-(phenylsulfonyl)-1H-indol-3-yl)-N-(1-ethylpiperidin-3-yl)-5-(trifluoromethyl)pyrimidin-2-amine Intermediate Useful in the Synthesis of Compounds 284 and 285.

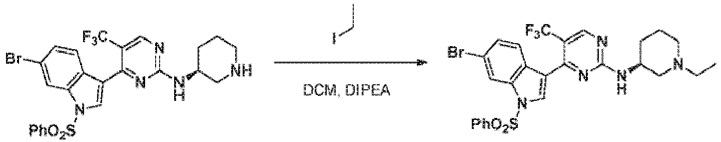

To a solution of (*S*)-4-(6-bromo-1-(phenylsulfonyl)-1H-indol-3-yl)-N-(piperidin-3-yl)-5-(trifluoromethyl)pyrimidin-2-amine (315 mg, 0.54 mmol) in DCM (2.5 mL), were added DIPEA (190 uL, 1.09 mmol) and iodoethane (48 uL, 0.6 mmol). The solution was stirred at RT for 72 h. The solvent was removed in vacuo, and the residue was re-dissolved in EtOAc and washed with brine (twice). Organic phase was separated, dried over Na₂SO₄, filtered, and concentrated. The crude product was purified by reverse phase chromatography (C1$, MeCN in aq.10 mM ammonium formate, pH 3.8, 0 to 100% gradient) to afford the title compound (219 mg, 0.36 mmol, 66% yield) as a white foam.

### Example 89. Synthesis of 3-bromo-N-methyl-1H-pyrrolo[2,3-b]pyridine-6-carboxamide Intermediate Useful in the Synthesis of Compound 297.

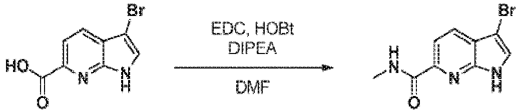

To a solution of 3-bromo-1H-pyrrolo[2,3-b]pyridine-6-carboxylic acid (260 mg, 1.08 mmol), HOBt (262 mg, 1.94 mmol) and methylamine (1.29 mmol, 2 M in THF) in DMF (21 mL) at 0 °C, were added EDCI × HCl (310 mg, 1.62 mmol) and DIPEA (570 uL, 3.24 mmol). The reaction mixture was stirred at RT for 20 h. After full conversion, the reaction mixture was diluted with EtOAc and washed with brine. The organic phase was separated, dried over Na₂SO₄, filtered, and concentrated under reduced pressure. The crude residue was purified by reverse phase chromatography (C18, MeCN in aq.10 mM ammonium formate, pH 3.8, 30 to 60% gradient) to afford the title compound (35 mg, 0.14 mmol, 13% yield).

### Example 90. Synthesis of (S)-tert-butyl 3-((4-(1-(methoxymethyl)-7-(methylcarbamoyl)-1H-indol-3-yl)-5-(trifluoromethyl)pyrimidin-2-yl)amino)piperidine-1-carboxylate Intermediate Useful in the Synthesis of Compound 304.

### Step 1: (S)-3-(2-((1-(tert-butoxycarbonyl)piperidin-3-yl)amino)-5-(trifluoromethyl)pyrimidin-4-yl)-1-(methoxymethyl)-1H-indole-7-carboxylic acid

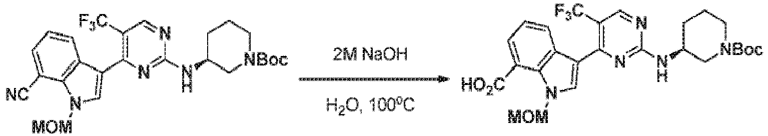

To a solution of (*S*)-*tert*-butyl 3-((4-(7-cyano-1-(methoxymethyl)-1H-indol-3-yl)-5-(trifluoromelhyl)pyrimidin-2-yl)amino)piperidinie-1-carboxylate (138 mg, 0.26 mmol) in dioxane (1.3 mL), was added NaOH (2 M, 2 mL, 4 mmol), and the reaction mixture was heated at 100 °C for 16 h. The reaction mixture was cooled to RT and concentrated under reduced pressure, then diluted with water (10 mL) and MeTHF (10 mL), acidified with 1M HCl to pH 3, and extracted with MeTHF (2 × 10 mL). The organics were combined and washed with water (2 × 10 mL) and brine (2 × 10 mL), dried over Na₂SO₄, filtered, and concentrated under reduced pressure. The residue was purified by reverse phase chromatography (C18, MeCN in aq. 10 mM ammonium formate, pH 3.8, 0 to 100% gradient) to obtain the title compound (54 mg, 0.10 mmol, 37% yield) as a white solid.

### Step 2: (S)-1-(methoxymethyl)-N-methyl-3-(2-(piperidin-3-ylamino)-5-(trifluoromethyl)pyrimidin-4-yl)-1H-indole-7-carboxamide

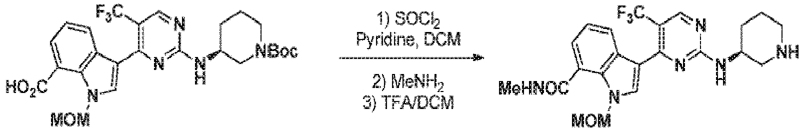

To a solution of (5)-3-(2-((l-(tert-butoxycarbonyl)piperidin-3-yl)amino)-5-(trifluoromethyl)pyrimidin-4-yl)-1-(methoxymethyl)-1H-indole-1-carboxylic acid (54 mg, 0.10 mmol) in DCM (2 mL), were added pyridine (32 uL, 0.39 mmol) and SOCl₂ (22 uL, 0.30 mmol), and the reaction mixture was stirred at RT for 15 minutes. Methylamine (2 M in THF, 0.49 mL, 0.98 mmol) was then added and stirred for another 1.5 h. The reaction mixture was diluted with MeOH and concentrated under reduced pressure, then diluted with water (3 mL) and MeTHF (3 mL), basified to pH 8 with sat. aq. NaHCO₃, extracted with MeTHF (2 × 5 mL), and the collected organics were washed with sat. aq. NaHCO₃ (3 mL), water (3mL), and brine (2 × 3mL), dried over Na₂SO₄, filtered, and concentrated under reduced pressure. The residue was dissolved in DCM (2 mL), treated with TFA (0.15 mL, 1.96 mmol) and stirred for 20 minutes. The mixture was then concentrated under reduced pressure. The residue was purified by reverse phase chromatography (C18, MeCN in aq. 10 mM ammonium formate, pH 3.8, 0 to 100% gradient) to obtain the title compound (10.1 mg, 0.018 mmol, 18% yield) as an off-white solid.

### Example 91, Synthesis of 4-(1H-indol-3-yl)-N-(piperidin-3-yl)-5-((trifluoromethyl)thio)pyrimidln-2-amine Intermediate Useful in the Synthesis of Compound 305.

### Step 1: (3S)-tert-butyl 3-((5-((3-((2-ethylhexyl)oxy)-3-oxopropyl)thio)4-(1-(phenylsulfonyl)-1H-indol-3-yl)pyrimidin-2-yl)amino)piperidine-1-carboxylate

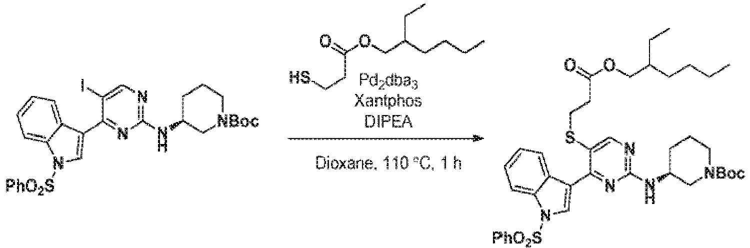

To a solution of (*S*)-*tert*-butyl 3-((5-iodo-4-(1-(phenylsulfonyl)-1H-indol-3-yl)pyrimidin-2-yl)amino)piperidine-1-carboxylate (400 mg, 0.606 mmol) in previously degassed dioxane (1.6 mL), were added Xantphos (36.2 mg, 10 mol %) and Pd₂dba₃ (5 mol %) under nitrogen, followed by 2-ethylhexyl-3-mercaptopropionate (156 uL, 0.667 mmol) and DIPEA (213 uL, 1.21 mmol). The reaction mixture was stirred at 110 °C under MW irradiation for 1 h, cooled to RT, and filtered through Celite. The filtrate was concentrated under reduced pressure, and the resulting crude product was purified by SiO₂ chromatography (EtOAc in Hexanes, 0 to 100% gradient) to afford the title compound (286 mg, 0.38 mmol, 63% yield) as a yellow oil.

### Step 2: (S)-tert-butyl 3-((5-mercapto-4-(1-(phenylsulforryl)-1H-indol-3-yl)pyrimidin-2-yl)amino)piperidine-1-carboxylate

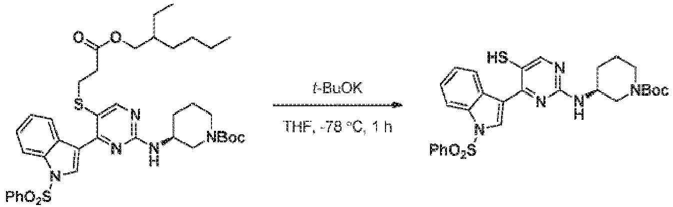

To a solution of (3*S*)-*tert*-butyl 3-((5-((3-((2-ethylhexyl)oxy)-3-oxopmpyl)thio)-4-(1-(phenylsulfonyl)-1H-indol-3-yl)pyrimidin-2-yl)amino)piperidine-1-carboxylate (286 mg, 0.381 mmol) in anhydrous THF (1 mL), was added potassium *tert*-butoxide solution (1 M, 1.14 mL. 1.14 mmol) at -78 °C. The resulting mixture was stirred at -78 °C for 1 h. The reaction mixture was cooled to -78 °C and quenched by the addition of K₂CO₃ (2 M, 0.5 mL). The solution was concentrated under reduced pressure, and the residue was diluted with K₂CO₃ (2 M, 20 mL) and Et₂O (20 mL). The layers were separated, and aqueous layer was back-extracted with Et₂O (10 mL). The aqueous layer was acidified to pH 4-5 with 6 M HCl and extracted with CHCl₃/isopropanol (9:1). The organic layer was separated and dried over Na₂SO₄, filtered, and concentrated under reduced pressure to afford the crude title compound as a yellow solid, which was used in the next step without further purification.

### Step 3: (S)-tert-butyl 3-((4-(1H-indol-3-yl)-5-((trifluoromethyl)thio)pyrimidin-2-yl)amino)piperidine-1-carboxylate

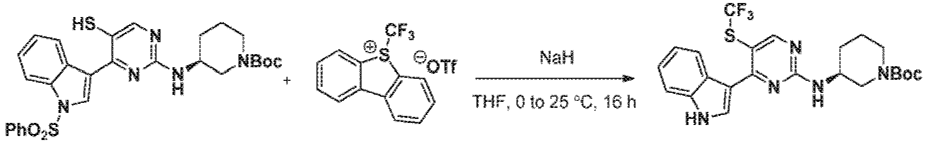

To a solution of crude (*S*)-*tert*-butyl 3-((5-mercapto-4-(1-(phenylsulfonyl)-1H-indol-3-yl)pyrimidin-2-yl)amino)piperidine-1-carboxylate (0.371 mmol) in dry THF (1.5 mL), was added sodium hydride (60% suspension in mineral oil, 38.6 mg, 0.965 mmol) at 0 °C and stirred for 10 min. 5-(Trifluaromethyl)dibenzothiophenium trifluoromethanesulfonate (494 mg, 1.19 mmol) was then added in one drop. The resulting mixture was stirred at RT for 16 h. The reaction was diluted with water and CHCl₃, the organic layer was washed with sat. aq. NaHCO₃ and water, dried over Na₂SO₄, filtered, and concentrated under reduced pressure. The crude residue was purified by SiO₂ chromatography (EtOAc in Hexanes, 0 to 80% gradient) to afford the title compound (64 mg, 0.132 mmol, 35% yield) as a yellowish oil.

### Example 92. Synthesis of (S)-3-(2-((6,6-dimethylpiperidin-3-yl)amino)-5-(trifluoromethyl)pyrimidin-4-yl)-1H-indole-6-carbonitrile Intermediate Useful in the Synthesis of Compound 350.

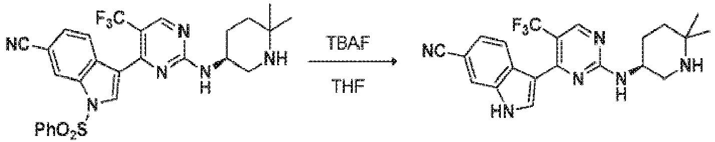

To a solution of (S)-3-(2-((6,6-dimethylpiperidin-3-yl)amino)-5-(trifluoromethyl)pyrimidin-4-yl)-1-(phenylsulfonyl)-1H-indole-6-carbonitrile (9 mg, 0.016 mmol) in THF (0.5 mL) at 0 °C, was added TBAF (1 M in THF, 0.07 mL, 0.07 mmol). The reaction mixture was stirred for 1 h at 70 °C. The reaction mixture was then quenched with saturated aq. NH₄Cl (30 mL) and extracted with MeTHF (3 x 50 mL). The organic extracts were combined and washed twice more with sat. aq. NH₄Cl (30 mL) and water (30 mL), dried over Na₂SO₄, and concentrated under reduced pressure. The residue was purified by reverse phase chromatography (C18, MeCN in aq.10 mM ammonium formate, pH 3.8, 0 to 100% gradient) to afford the title compound (0.51 mg, 0.001 mmol, 7% yield) as a white solid.

### Example 93. Synthesis of (3R,4R)-tert-butyl 3-((4-(6-cyano-1-(phenylsulfonyl)-1H-indol-3-yl)-5-(trifluoromethyl)pyrimidin-2-yl)amino)-4-fluoropiperidine-1-carboxylate Intermediate Useful in the Synthesis of Compound 351.

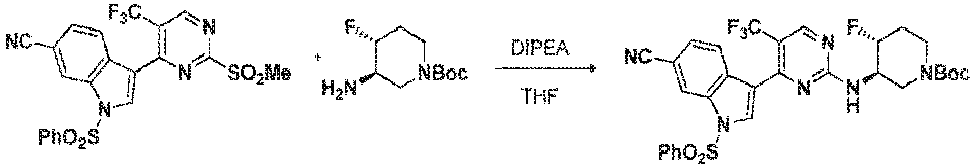

To a solution of 3-(2-(methylsulfonyl)-5-(trifluoromethyl)pyrimidin-4-yl)-1-(phenylsulfonyl)-1H-indole-6-carbonitrile (103 mg, 0.204 mmol) in dry THF (2 mL), were added (3*R*,4*R*)-*tert*-butyl 3-amino-4-fluoropiperidine-1-carboxylate (50 mg, 0.224 mmol) and DIPEA (108 uL, 0.612 mmol). The reaction mixture was stirred at RT for 16 h, diluted with EtOAc, washed with brine, dried over Na₂SO₄, filtered, and concentrated under reduced pressure. The residue was purified by SiO₂ chromatography (EtOAc in Hexanes, 0 to 40% gradient) to afford the title compound (57 mg, 0.088 mmol, 43% yield) as a thick colourless oil.

### Example 94. Synthesis of (S)-dimethyl(3-(2-(piperidin-3-ylamino)-5-(trifluoromethyl)pyrimidin-4-yl)-1H-indol-7-yl)phoaphine oxide (Compound 352).

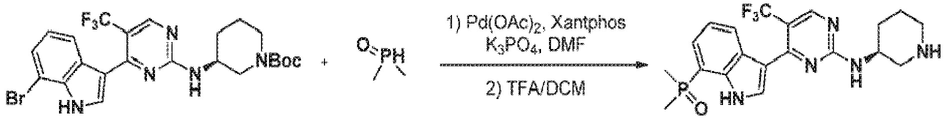

(*S*)-*tert*-Butyl 3-((4-(7-bromo-1H-indol-3-yl)-5-(trifluoromethyl)pyrimidin-2-yl)amino)piperidine-1-carboxylate (50 mg, 0.093 mmol), Xantphos (5.4 mg, 0.0093 mmol), palladium (II) acetate (1.0 mg, 0.0047 mmol) and K₃P0₄ (22 mg, 0.1023 mmol) were combined in a microwave tube under nitrogen. Dimethylphosphine oxide (9 mg, 0.11 mmol) was dissolved in anhydrous DMF (0.3 mL) and the mixture degassed before combining with the other reactants. The reaction mixture was heated at 150 °C in a microwave for 45 min. The mixture was then cooled down to ambient temperature, re-dissolved in MeTHF (10 mL), washed with sat. NaHCO₃ (10 mL) and brine (10 mL). Organic phase was separated, dried over Na₂SO₄, filtered, and concentrated under reduced pressure. The crude was re-dissolved in DCM (1 mL) and TFA (0.214 mL, 2.79 mmol) was added. After 1 h of stirring at room temperature, reaction mixture was concentrated and the residue was purified by reverse phase chromatography (C18, MeCN in aq.10 mM ammonium formate, pH 3.8, 0 to 100% gradient) to afford the title compound (28 mg, 0.064 mmol, 69% yield) as a white solid.

### Example 95. Synthesis of (2R,5S)-tert-butyl 5-((4-(6-cyano-1H-indol-3-yl)-5-(trifluoromethyl)pyrimidin-2-yl)amino)-2-(3-methyl-1,2,4-oxadiazol-5-yl)piperidine-1-carboxylate Intermediate Useful in the Synthesis of Compound 358.

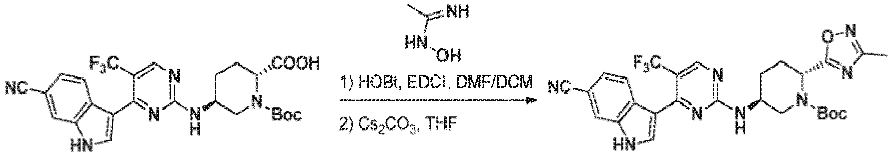

A mixture of (2*R*,5*S*)-1-(*tert*-butoxycarbonyl)-5-((4-(6-cyano-1H-indol-3-yl)-5-(trifluoromethyl)pyrimidin-2-yl)amino)piperidine-2-carboxylic acid (135 mg, 0.254 mmol), HOBt (58 mg, 0.43 mmol) and EDCI (50 mg, 0.26 mmol) in dry DMF/DCM (0.3/1.2 mL) was stirred at room temperature for 5 min. Then, N'-hydroxyacetimidamide (20 mg, 0.25 mmol) was added, and the reaction mixture was stirred at RT for 48 h. The reaction mixture was then diluted with water and extracted with DCM. The organic phase was washed with sat. aq. NaHCO₃ and brine, dried over Na₂SO₄, filtered. Evaporation of the solvent provided yellow solid of crude intermediate which was dissolved in dry THF (2.5 mL), and Cs₂CO₃ (182 mg, 0.56 mmol) was added. The reaction mixture was heated at 50 °C for 3 h until full conversion. The reaction was diluted with brine and extracted with MeTHF. The organic phase was dried over Na₂SO₄, filtered, and concentrated. Residue was purified by reverse phase chromatography (C18, MeCN in aq. 10 mM ammonium formate, pH 3.8, 0 to 100% gradient) to afford the title compound (30 mg, 0.053 mmol, 21% yield) as a yellow oil.

### Example 96. Synthesis of N,N-dimethyl-1H-indole-7-sulfonamide Intermediate Useful in the Synthesis of Compound 371.

### Step 1: N,N-dimethyl-2-nitrobenzenesulfonamide

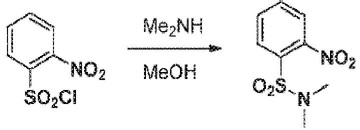

To a solution of nitrobenzenesulfonyl chloride (5.0 g, 22.561 mmol) in MeOH (9.6 mL) at 0 °C, was added dimethyl amine (2 M, 24.8 mL) dropwise *via* dropping funnel. The reaction mixture was stirred at RT for overnight. The reaction mixture was concentrated under reduced pressure and diluted with water (50 mL) and EtOAc (50 mL), then extracted with EtOAc (3 x 50 mL). The organic phase was combined, washed with sat. aq. NaHCO₃ (50 mL) and brine (50 mL), dried over Na₂SO₄, filtered, and concentrated under reduced pressure to give the title compound (4.72 g, 20.50 mmol, 91% yield) as a pale solid, which was used in the next step without further purification.

### Step 2: N,N-dimethyl-1H-indole-7-sulfonamide

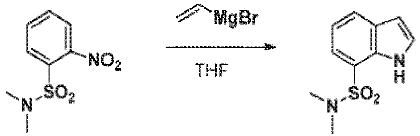

To a solution of *N*,*N*-dimethyl-2-nitrobenzenesulfonamide (2.36 g, 10.25 mmol) in THF (82 mL) at 0 °C, was added vinylmagnesium bromide (30.75 mL, 1 M in THF). The reaction mixture was stirred at RT for overnight. The reaction mixture was quenched with sat. aq. NH₄Cl (100 mL) and extracted with EtOAc (3 x 150 mL). The organics were combined, dried over Na₂SO₄, filtered, and concentrated under reduced pressure to give the title compound (2.30 g, 10.25 mmol, quantitative yield), which was used in the next step without further purification.

### Example 97. Synthesis of (3S,4R)-tert-butyl 4-methyl-3-((4-(1-(phenylsulfonyl)-1H-indol-3-yl)-5-(trifluoromethyl)pyrimidin-2-yl)amino)piperidine-1-carboxylate (A) and (3R,4S)-tert-butyl 4-methyl-3-((4-(1-(phenylsulfonyl)-1H-indol-3-yl)-5-(trifluoromethyl)pyrimidin-2-yl)amino)piperidine-1-carboxylate (B) Intermediates Useful in the Synthesis of Compounds 372 and 373.

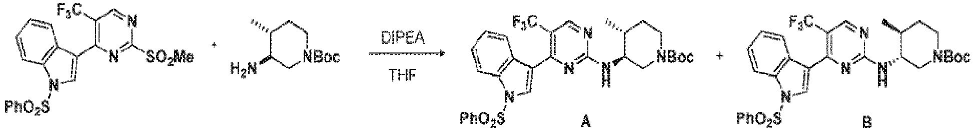

To a solution of 3-(2-(methylsulfonyl)-5-(trifluoromethyl)pyrimidin-4-yl)-1-(phenylsulfonyl)-1H-indole (160 mg, 0.331 mmol) in dry THF (3 mL) under nitrogen, were added *trans*-racemic *tert*-butyl 3-amino-4-methylpiperidine-1-carboxylate (prepared following CN103664743A) (78 mg, 0.364 mmol) and DIPEA (0.175 mL, 1 mmol). The reaction mixture was stirred at RT for 16 h, then diluted with EtOAc, washed with brine, dried over Na₂SO₄, filtered, and concentrated under reduced pressure. The residue was purified by reverse phase chromatography (C18, MeCN in aq.10 mM ammonium formate, pH 3.8, 30 to 100% gradient) to afford the title compounds (124 mg, 0.201 mmol, 61% yield) as a thick colourless oil. The mixture of enantiomers was separated by SFC: ChiralPak IA, 10 x 250 mm 5 um, 15% IPA, 10 mL/min, 150 bar. Peak 1 (**A**) (R*t* = 11.67 min): white solid, 50 mg; Peak 2 (**B**) (R*t* = 14.04 min): white solid, 50 mg; stereochemistry for **A** and **B** tentatively assigned.

### Example 98. Synthesis of (R)-3-(2-(5-azaspiro[3.5]nonan-7-ylamino)-5-(trifluoromethyl)pyrimidin-4-yl)-1H-indole-6-carbonitrile (A; Compound 377) and (S)-3-(2-(5-azaspiro[3.5]nonan-7-ylamino)-5-(trifluoromethyl)pyrimidin-4-yl)-1H-indole-6-carbonitrile (B; Compound 378).

### Step 1: 5-tosyl-5-azaspiro[3.5]nonan-7-one

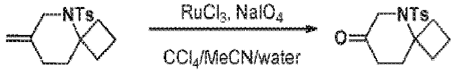

Sodium periodate (735 mg, 3.44 mmol) and ruthenium(III) chloride (10.4 mg, 0.05 mmol) were added to the solution of 7-methylene-5-tosyl-5-azaspiro[3.5]nonane (prepared as in J. Org. Chem. 2003, 68(11), 4286) (247 mg, 0.856 mmol) in CCl₄ (2 mL), acetonitrile (2 mL), and water (3 mL). After being stirred at room temperature for 1.5 h, the reaction mixture was diluted with water (10 mL), extracted with CH₂Cl₂ (20 mL x 4), dried over sodium sulfate, filtered, and concentrated. The crude ketone was purified by SiO₂ column chromatography (hexanes in EtOAc, 0 to 100% gradient) to provide the title compound (175 mg, 0.596 mmol, 70% yield) as a white semi-solid.

### Step 2: (R)-2-methyl-N-((S)-5-tosyl-5-azaspiro[3.5]nonan-7-yl)propane-2-sulfinamide and (R)-2-methyl-N-((R)-5-tosyl-5-azaspiro[3.5]nonan-7-yl)propane-2-sulfinamide

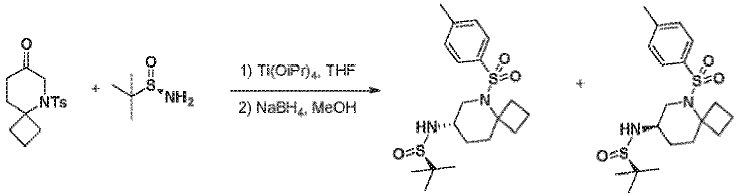

(*R*)-(+)-2-Methylpropane-2-sulfmamide (80 mg, 0.66 mmol) and titanium(IV) isopropoxide (0.353 mL, 1.2 mmol) were added to a stirring solution of 5-tosyl-5-azaspiro[3.5]nonan-7-one (175 mg, 0.596 mmol) in THF (5 mL) at room temperature. The reaction mixture was heated at 70 °C for 3 h. EtOAc (20 mL) and sat. aq. solution of NH₄Cl (5 mL) were added. The mixture was filtered through Celite. Organic layer was separated, washed with sat. NH₄Cl and brine, dried over Na₂SO₄, filtered, and concentrated under reduced pressure. The residue was dissolved in MeOH (5 mL) and NaBH₄ (45 mg, 1.2 mmol) was then added portion wise. Reaction mixture was stirred at RT for 5 h. The reaction was quenched with sat. aq. solution of NaHCO₃, organic phase was separated, washed with brine, dried over Na₂SO₄, filtered, and concentrated under reduced pressure. The residue was purified by SiO₂ column chromatography (EtOAc in DCMlhexanes (1:1), 0 to 100% gradient) to provide the mixture of diastereomers (100 mg, 0.25 mmol, 42% yield) as a white foam.

### Step 3: 5-tosyl-5-azaspiro[3.5]nonan-7-amine hydrochloride

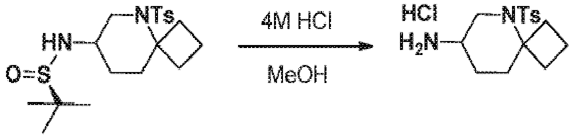

To a solution of (*R*)-2-methyl-*N*-(5-tosyl-5-azaspiro[3.5]nonan-7-yl)propane-2-sulfinamide (100 mg, 0.25 mmol) in anhydrous MeOH (1.5 mL), was added HCl (4 M in dioxane, 1.5 mL, 6 mmol). After stirring at RT for 10 min, reaction mixture was concentrated under reduced pressure. Crude product (white foam) used in the next step without further purification.

### Step 4: 1-(phenylsulfonyl)-3-(2-((5-tosyl-5-azaspiro[3.5]nonan-7-yl)amino)-5-(trifluoromethyl)pyrimidin-4-yl)-1H-indole-6-carbonitrile

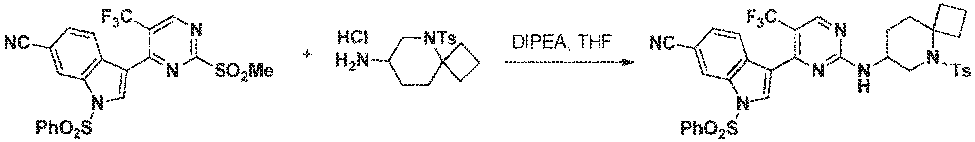

The mixture of 3-(2-(methylsulfonyl)-5-(trifluoromethyl)pyrimidin-4-yl)-1-(phenylsulfonyl)-1H-indole-6-carbonitrile (140 mg, 0.275 mmol), 5-tosyl-5-azaspiro[3.5]nonan-7-amine hydrochloride (0.25 mmol), and DIPEA (0.131 mL, 0.75 mmol) in dry THF (2.5 mL) was stirred at RT for 3 h. Reaction mixture was then diluted with EtOAc, washed with sat. solution of NaHCO₃ and brine. Organic phase was dried over Na₂SO₄, filtered, and concentrated. The residue was purified by reverse phase chromatography (C18, MeCN in aq.10 mM ammonium formate, pH 3.8, 0 to 100% gradient) to afford the title compound (70 mg, 0.097 mmol, 39% yield over 2 steps) as a beige solid.

### Step 5: (R)-3-(2-(5-azaspiro[3.5]nonan-7-ylamino)-5-(trifluoromethyl)pyrimidin-4-yl)-1H-indole-6-carbonitrile (A) and (S)-3-(2-(5-azaspiro[3.5]nonan-7-ylamino)-5-(trifluoromethyl)pyrimidin-4-yl)-1H-indole-6-carbonitrile (B)

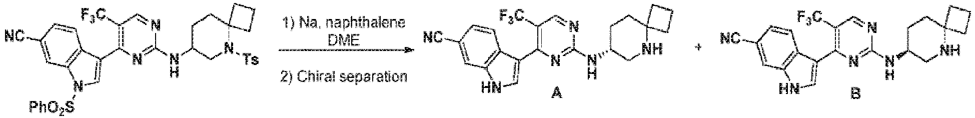

To 1-(phenylsulfonyl)-3-(2-((5-tosyl-5-azaspiro[3.5]nonan-7-yl)amino)-5-(trifluoromethyl)pyrimidin-4-yl)-1H-indole-6-carbonitrile (60 mg, 0.083 mmol) in DME (4 mL) was added Na/naphthalene [which was prepared by stirring fresh sodium (27 mg, 1.162 mmol) and naphthalene (160 mg, 1.245 mmol) in DME (1 mL) for 30 min at room temperature] at -78 °C, and the mixture was stirred for 20 min at this temperature. The reaction was quenched with sat. aq. NH₄Cl and stirred at RT for a while. The aq. layer was extracted with MeTHF (3 x 10 mL). The combined organic phase was washed with brine and dried over Na₂SO_{4,} filtered, and concentrated. The residue was purified by reverse phase chromatography (C18, MeCN in aq.10 mM ammonium formate, pH 3.8, 0 to 100% gradient) to afford the title *racemic* compound (25 mg, 0.059 mmol, 71% yield) as a pale yellow solid. Enantiomers were separated by chiral semi-prep SFC: AS 10 x 250 mm, 5 um, Isocratic 35% ACN+EtOH 1:1,10 mL/min, 100 Bar, 35 °C, 10 min. Peak 1 (**A**) (R*t* = 4.54 min): 7 mg, white solid, *ee* = 99.937%; Peak 2 (**B**) (R*t* = 6.59 min): 8 mg, white solid, *ee* = 99.872%; stereochemistry for **A** and **B** tentatively assigned.

### Example 99. 3-[5-methylsulfanyl-2-[[(3S)-3-piperidyl] amino] pyrimidin-4-yl]-1H-indole-6-carbonitrile (Compound 272).

### Step 1: 3-(2-chloro-5-methylsulfanyl-pyrimidin-4-yl)-1H-indole-6-carbonitrile

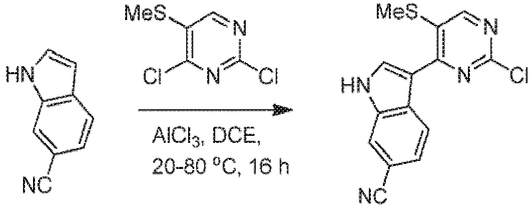

To a solution of 2, 4-dichloro-5-methylsulfanyl-pyrimidine (494.00 mg, 2.53 mmol, 1.2 eq) in DCE (10 mL) was added dropwise AlCl₃ (422.09 mg, 3.17 mmol, 172.99 uL, 1.5 eq) at 20 °C. After addition, the mixture was stirred at 80 °C for 0.5 h, and then 1H-indole-6-carbonitrile (0.3 g, 2.11 mmol, 1 eq) was added. The resulting mixture was stirred at 80 °C for 15.5 h. The mixture was poured into Sat. NaHCO₃ (100 mL) and the yellow solid formed. The solution was filtered and collected the cake to afford the title compound (0.3 g, 947.58 umol, 44.90% yield, 95% purity) as a yellow solid.

### Step 2: Tert-butyl (3S)-3-[[4-(6-cyano-1H-indol-3yl)-5-methylsulfanyl-pyrimidin-2-yl]amino] piperidine-1-carboxylate

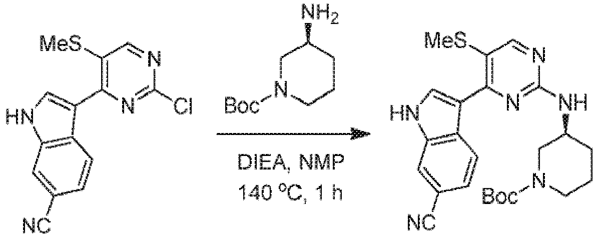

A mixture of 3-(2-chloro-5-methylsulfanyl-pyrimidin-4-yl)-1H-indole-6-carbonitrile (0.2 g, 664.97 umol, 1 eq), tert-butyl (3S)-3-aminopiperidine-1-carboxylate (133.18 mg, 664.97 umol, 1 eq), DIEA (429.70 mg, 3.32 mmol, 579.11 uL, 5 eq) in NMP (2mL) was stirred at 140 °C for 1 h. The reaction mixture was poured into water 20 mL, and then extracted with EtOAc (10mL x 3). The combined organic layers were washed with brine (50 mL), dried over Na₂SO₄, filtered and concentrated under reduced pressure to give a residue. The residue was purified by column chromatography (SiO2, PE/EtOAc=5/1 to 1:1) to afford the title compound (60 mg) as a brown solid. (Note: Combined with another reaction in 20 mg scale)

### Step 3: 3-[5-methylsulfanyl-2-[[(3S)-3-piperidyl] amino] pyrimidin-4-yl]-1H-indole-6-carbonitrile

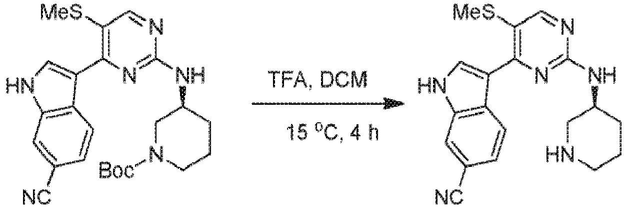

To a solution of tert-butyl (3S)-3-[[4-(6-cyano-1H-indol-3-yl)-5-methylsulfanyl -pyrimidin-2-yl] amino] piperidine-1-carboxylate (60 mg, 129.15 umol, 1 eq) in DCM (2 mL) was added TFA (770.00 mg, 6.75 mmol, 0.5 mL, 52.29 eq). The mixture was stirred at 15 °C for 4 h. The reaction mixture was concentrated under reduced pressure to give a residue. The residue was purified by prep-HPLC (FA condition) to afford the title compound (8 mg, 18.90 umol, 14.64% yield, 97% purity, FA) as a white solid

### Example 100. [3-[2-[[(3S)-3-piperidyl] amino]-5-(trifluoromethyl) pyrimidin-4-yl]-1H-indol-6-yl]-Pyrrolidin-1-yl-methanone (Compound 278).

### Step 1: Tert-butyl (3S)-3-[[4-[6-(pyrrolidine-1-carbonyl)-1H-indol-3-yl]-5-(trifluoromethyl)pyrimidin-2-yl] amino] piperidine-1-carboxylate

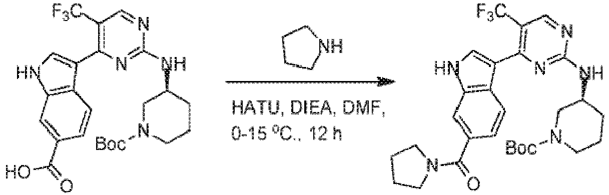

To a solution of carboxylic acid (50 mg, 98.91 umol, 1 eq) in DMF (0.5 mL) was added pyrrolidine (8.44 mg, 118.70 umol, 9.91 uL, 1.2 eq), HATU (45.13 mg, 118.70 umol, 1.2 eq) and DIEA (38.35 mg, 296.74 umol, 51.69 uL, 3 eq) at 0 °C. The mixture was stirred at 15 °C for 12 h. The residue was poured into water (20 mL). The aqueous phase was extracted with EtOAc (20 mL x 3). The combined organic phase was washed with brine (10 mL x 2), dried with anhydrous Na₂SO₄, filtered and concentrated in vacuum. The residue was purified by silica gel chromatography DCM: MeOH = 100:1-80:1-50:1 to afford the title compound (35 mg) as yellow solid (Combined purification with another batch with 20 mg scale).

### Step 2: [3-[2-[[(3S)-3-piperidyl] amino]-5-(trifluoromethyl)pyrimidin-4-yl]-1H-indol-6-yl]-Pyrrolidin-1-yl-methanon

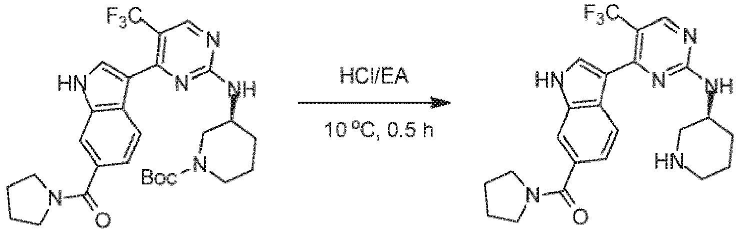

A solution of tert-butyl (3S)-3-[[4-[6-(pyrrolidine-1-carbonyl)-1H-mdol-3-yl]-5-(trifluoromethyl) pyrimidin-2-yl] amino] piperidine-1-carboxylate (30 mg, 53.71 umol, 1 eq) in HCl/EtOAc (3 mL) was stirred at 10 °C for 0.5 h. The mixture was concentrated in vacuum. The residue was purified by prep-HPLC (neutral condition) to afford the title compound (7.03 mg, 15.33umol, 28.55% yield, 100 % purity) as white solid.

### Example 101. (3S)-3-methoxypyrrolidin-1-yl]-[3-[2-[[(3S)-3-piperidyl] amino]-5-(trifluoromethyl) pyrimidin-4-yl]-1H-indol-6-yl]methanone (Compound 280) and [(3R)-3-methoxypyrrolidin-1-yl]-[3-[2-[[(3S)-3-piperidyl] amino]-5-(trifluoromethyl) pyrimidin-4-yl]-1H-indol-6-yl]methanone (Compound 281).

### Step 1: tert-butyl(3S)-3-[[4-[6-(3-methoxypyrrolidine-1-carbonyl)-1H-indol-3-yl]-5-(trifluoromethyl)pyrimidin-2-yl]amino]piperidine-1-carboxylate

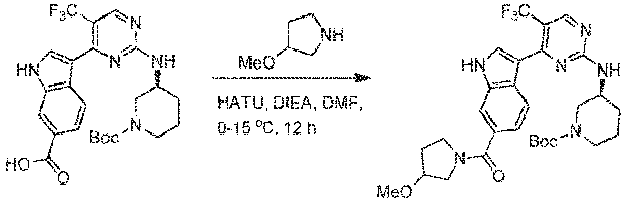

To a solution of 3-[2-[[(3S)-1-tert-butoxycarbonyl-3-piperidyl] amino]-5-(trifluoromethyl)pyrimidin-4-yl]-1H-indole-6-carboxylic acid (0.16 g, 316.53 umol, 1 eq) in DMF (2 mL) was added DIEA (122.73 mg, 949.58 umol, 165.40 uL, 3 eq), HATU (144.42 mg, 379.83 umol, 1.2 eq) and 3-methoxypyrrolidine (56.62 mg, 411.48 umol, 1.3 eq, HCl) at 0 °C. The mixture was stirred at 15 °C for 12 h. The residue was poured into water (20 mL). The aqueous phase was extracted with EtOAc (20 mLx3). The combined organic phase was washed with brine (30 mLx2), dried with anhydrous Na₂SO₄, filtered and concentrated in vacuum. The residue was purified by prep-HPLC (TFA). The residue was adjusted to pH = 9 with sat. NaHCO₃ and extracted with EtOAc (30 mL×3). The combined organic phase was dried with anhydrous Na₂SO₄, filtered and concentrated in vacuum to afford the title compound (30 mg, 95% purity) as yellow solid.

### Step 2: [(3S)-3-methoxypyrrolidin-1-yl]-[3-[2-[[(3S)-3-piperidyl] amino]-5-(trifluoromethyl) pyrimidin-4-yl]-1H-indol-6-yl]methanone & [(3R)-3-methoxypyrrolidin-1-yl]-[3-[2-[[(3S)-3-piperidyl] amino]-5-(trifluoromethyl) pyrimidin-4-yl]-1H-indol-6-yl]methanone

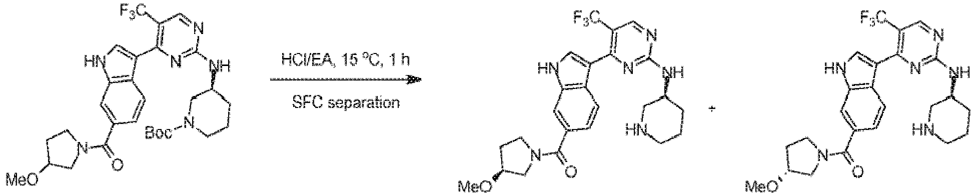

A solution of tertbutyl (3S)-3-[[4-[6-(3-methoxypyrrolidine-1-carbonyl)-1H-indol-3-yl]-5-(trifluoromethyl) pyrimidin-2-yl] amino] piperidine-1-carboxylate (20 mg, 33.98 umol, 1 eq) in HCl/EtOAc (5 mL) was stirred at 15 °C for 1 h. The residue was concentrated in vacuum. The residue was separated by SFC (column: AD (250mm*30mm, 5um); mobile phase: [0.1%NH₃H₂O IPA]; B%: 45%-45%, min) afford the title compound 1 (5.64 mg, 10.73 umol, 31.57% yield, 92.9% purity, temporarily assigned) as white solid and the title compound 2 (7.07 mg, 13.82 umol, 40.68% yield, 95.5% purity, temporarily assigned)) as white solid.

### Example 102. (4-hydroxy-4-methyl-1-piperidyl)- [3-[2-[[(3S)-3-piperidyl] amino]-5-(trifluoromethyl) pyrimidin-4-yl]-1H-indol-6-yl] methanone (Compound 282).

### Step 1: Tert-butyl (3S)-3-[[4-[6-(4-hydroxy-4-methyl(4-hydroxy-4-methyl-piperidine-1H-carbonyl)-1H-indol3-yl]-5-(trifluoromethyl) pyrimidin-2-yl] amino] piperidine-1-carboxylate

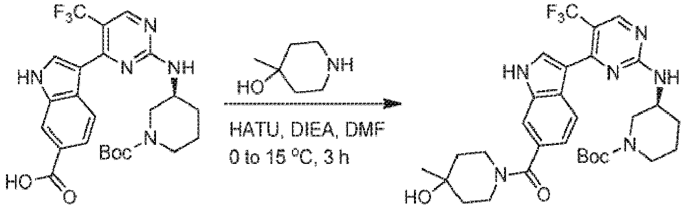

To a solution of 3-[2-[[(3S)-1-tert-butoxycarbonyl-3-piperidyl] amino]-5-(trifluoromethyl) pyrimidin-4-yl]-1H-indole-6-carboxylic acid (80 mg, 158.26 umol, 1 eq) in DMF (2 mL) was added HATU (72.21 mg, 189.92 umol, 1.2 eq) and DIEA (40.91 mg, 316.53 umol, 55.13 uL, 2 eq) at 0 °C. Then 4-methylpiperidin-4-ol (21.87 mg, 189.92 umol, 1.2 eq) was added to the mixture. The mixture was stirred at 15 °C for 3 h. The residue was poured into water (20 mL). The aqueous phase was extracted with ethyl acetate (20 mL x 3). The combined organic phase was washed with brine (20 mL x 2), dried with anhydrous Na₂SO₄, filtered and concentrated in vacuum. The residue was purified by silica gel chromatography DCM: MeOH = 100:2-100:3-100:4 to afford the title compound as yellow solid (Combined purification with another 20 mg scale batch).

### Step 2: (4-hydroxy-4-methyl-1-piperidyl)- [3-[2-[[(3S)-3-piperidyl] amino]-5-(trifluoromethyl) pyrimidin-4-yl]-1H-indol-6-yl] methanone

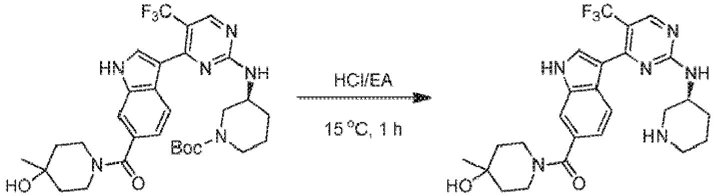

A solution of tert-butyl (3S)-3-[[4-[6-(4-hydroxy-4-methyl-piperidine-1-carbonyl) -1H-indol-3-yl]-5-(trifluoromethyl) pyrimidin-2-yl] amino] piperidine-1-carboxylate (0.04 g, 66.37 umol, 1 eq) in HCl/EtOAc (10 mL) was stirred at 15 °C for 1 h. The residue was purified by prep-HPLC (FA) to afford the title compound (12.86 mg, 23.44 umol, 35.32% yield, 100% purity, FA) as white solid.

### Example 103. [Intentionally Omitted]

### Example 104. 4-[6-[1-(difluoromethyl) pyrazol-3-yl]-1H-indol-3-yl]-N-[(3S)-3-piperidyl]-5-(trifluoromethyl) pyrimidin-2-amine (Compound 289).

### Step 1: 1-(difluoromethyl)-3-(4,4,5,5-tetramethyl-1,3,2-dioxaborolan-2-yl)pyrazole

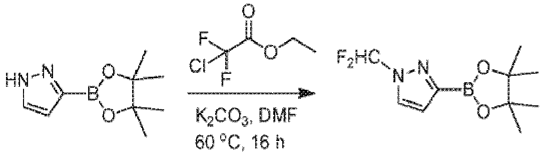

To a solution of 3-(4,4,5,5-tetramethyl-1,3,2-dioxaborolan-2-yl)-1H-pyrazole (0.5 g, 2.58 mmol, 1 eq) in DMF (5 mL) was added ethyl 2-chloro-2,2-difluoro-acetate (490.20 mg, 3.09 mmol, 392.16 uL, 1.2 eq) and K₂CO (712.26 mg, 5.15 mmol, 2 eq). The mixture was stirred at 60 °C for 16 h. The reaction mixture was poured into H₂O 10 mL, and extracted with EtOAc (10 mL x 3). The combined organic layers were washed with brine (10 mL x 2), dried over Na₂SO₄, filtered and concentrated under reduced pressure to afford the title compound (0.3 g, crude) as yellow solid, which was used into the next step without further purification. The structure was confirmed by ¹H NMR and HOSEY.

### Step 2: Tert-butyl (3S)-3-[[4-[6-[1-(difluoromethyl) pyrazol-3-yl]-1H-indol-3-yl]-5-(trifluoromethyl) pyrimidin-2-yl] amino] piperidine-1-carboxylate

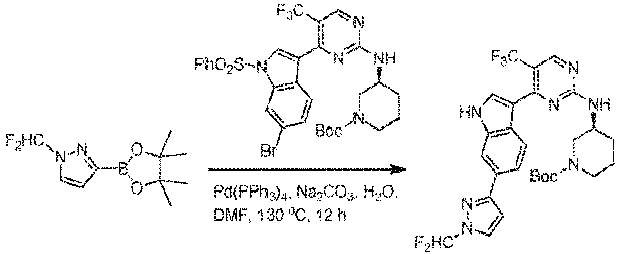

To a solution of tert-butyl (3S)-3-[[4-[1-(benzenesulfonyl)-6-bromo-indol-3-yl] -5-(trifluoromethyl)pyrimidin-2-yl]amino]piperidine-1-carboxylate (0.2 g, 293.89 umol, 1 eq) in DMF (1 mL) and H₂O (0.1 mL) was added 1-(difluoromethyl)-3-(4,4,5,5- tetramethyl-1,3,2-dioxaborolan-2-yl)pyrazole (143.44 mg, 587.77 umol, 2 eq), Pd(PPh₃)₄ (101.88 mg, 88.17 umol, 0.3 eq) and Na₂CO₃ (62.30 mg, 587.77 umol, 2 eq). The mixture was stirred under N₂ at 130 °C for 12 h. The mixture was poured into H₂O (10 mL), extracted with EtOAc (30 mL x 3). The combined organic layers were washed with brine (20 mL x 2), dried over Na₂SO₄, filtered and concentrated under reduced pressure to give the crude product. The residue was purified by column chromatography (SiO₂, PE/EtOAc = 10:1) to afford the title compound (0.1 g, crude) as yellow solid.

### Step 3: 4-[6-[1-(difluoromethyl) pyrazol-3-yl]-1H-indol-3-yl]-N-[(3S)-3-piperidyl]-5-(trifluoromethyl) pyrimidin-2-amine

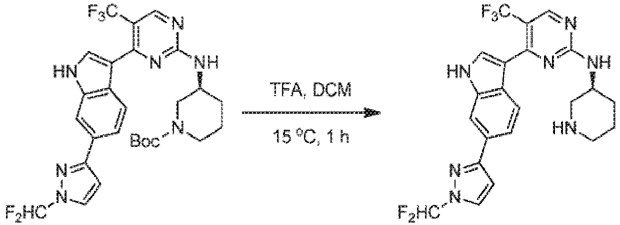

To a solution of tert-butyl (3S)-3-[[4-[6-[1-(difluoromethyl) pyrazol-3-yl]-1H-indol-3-yl]-5-(trifluoromethyl) pyrimidin-2-yl] amino] piperidine-1-carboxylate (0.1 g, 173.15 umol, 1 eq) in DCM (1 mL) was added TFA (592.31 mg, 5.19 mmol, 384.62 uL, 30.00 eq). The mixture was stirred at 15 °C for 1 h. The reaction mixture was concentrated under reduced pressure to give a residue. The residue was purified by prep-HPLC (FA condition) to afford the title compound (7.9 mg, 15.09 umol, 8.72% yield, 100% purity, FA) as white solid.

### Example 105. 4-[6-[1-(difluoromethyl) pyrazol-4-yl]-1H-indol-3-yl]-N-[(3S)-3-piperidyl]-5-(trifluoromethyl) pyrimidin-2-amine (Compound 290).

### Step 1: 1-(difluoromethyl)-4-(4,4,5,5-tetramethyl-1,3,2-dioxaborolan-2-yl)pyrazole

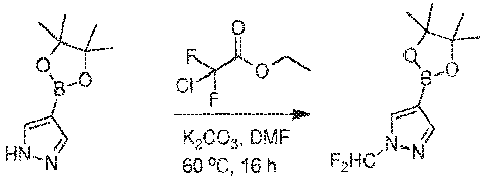

To a solution of 4-(4, 4, 5, 5-tetramethyl-1,3,2-dioxaborolan-2-yl)-1H-pyrazole (0.5 g, 2.58 mmol, 1 eq) in DMF (6 mL) was added K₂CO₃ (712.26 mg, 5.15 mmol, 2 eq) and ethyl 2-chloro-2,2-difluoro-acetate (490.21 mg, 3.09 mmol, 392.16 uL, 1.2 eq). The mixture was stirred at 60 °C for 16 h. The reaction mixture was poured into H₂O 10 mL, and extracted with EtOAc (10 mLx3). The combined organic layers were washed with brine (10 mLx2), dried over Na₂SO₄, filtered and concentrated under reduced pressure to afford the title compound (0.4 g, crude) as yellow oil, which was used into the next step without further purification.

### Step 2: Tert-butyl (3S)-3-[[4-[6-[1-(difluoromethyl) pyrazol-4-yl]-1H-indol-3-yl]-5-(trifluoromethyl) pyrimidin-2-yl] amino] piperidine-1-carboxylate

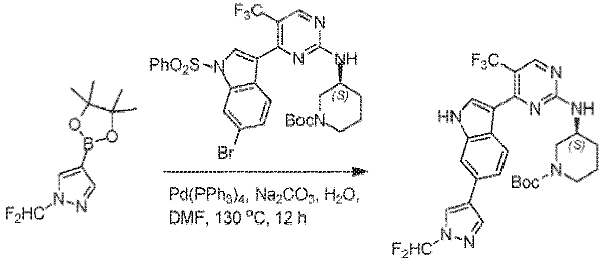

To a solution of tert-butyl (3S)-3-[[4-[1-(benzenesulfonyl)-6-bromo-indol-3-yl]-5-(trifluoromethyl)pyrimidin-2-yl]amino]piperidine-1-carboxylate (0.2 g, 293.89 umol, 1 eq) in DMF (1 mL) and H₂O (0.1 mL) was added 1-(difluoromethyl)-4-(4,4,5,5-tetramethyl -1,3,2-dioxaborolan-2-yl)pyrazole (143.45 mg, 587.78 umol, 2 eq), Pd(PPh₃)₄ (101.88 mg, 88.17 umol, 0.3 eq) and Na₂CO₃ (62.30 mg, 587.78 umol, 2 eq). The mixture was stirred under N₂ at 130 °C for 12 h. The mixture was poured into H₂O 10 mL, extracted with EtOAc (30 mLx3). The combined organic layers were washed with brine (20 mLx2), dried over Na₂SO₄, filtered and concentrated under reduced pressure to give the crude product. The residue was purified by column chromatography (SiO₂, PE/EtOAc=10:1) to afford the title compound (0.1 g, crude) as yellow solid.

### Step 3: 4-[6-[1-(difluoromethyl) pyrazol-4-yl]-1H-indol-3-yl]-N-[(3S)-3-piperidyl]-5-(trifluoromethyl) pyrimidin-2-amine

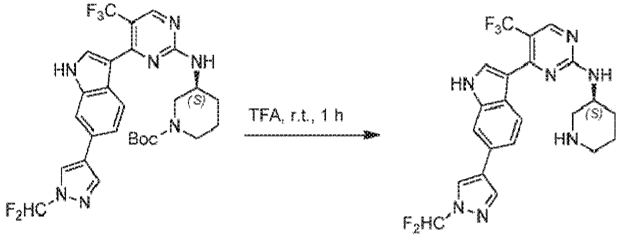

To a solution of tert-butyl (3S)-3-[[4-[6-[1-(difluoromethyl) pyrazol-4-yl]-1H-indol-3-yl]-5-(trifluoromethyl) pyrimidin-2-yl] amino] piperidine-1-carboxylate (0.1 g, 173.15 umol, 1 eq) in DCM (1 mL) was added TFA (770.00 mg, 6.75 mmol, 0.5 mL, 39.00 eq). The mixture was stirred at 15 °C for 1 h. The reaction mixture was concentrated under reduced pressure to give a residue. The residue was purified by prep-HPLC (HCl condition) to afford the title compound (19.4 mg, 37.75 umol, 100% purity, HCl) as white solid.

### Example 106. N-[(3S)-3-piperidyl]-4-[6-(1H-pyrazol-3-yl)-1H-indol-3-yl]-5-(trifluoromethyl) pyrimidin-2-amine (Compound 291).

### Step 1: trimethyl-[2-[[5-(4,4,5,5-tetramethyl-1,3,2-dioxaborolan-2-yl)pyrazol-1-yl]methoxy]ethyl]silane

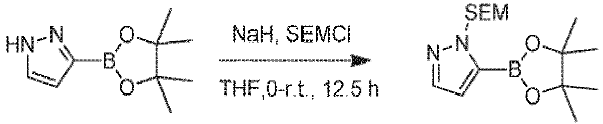

At 0°C, 3-(4,4,5,5-tetramethyl-1,3,2-dioxaborolan-2-yl)-1H-pyrazole (0.3 g, 1.55 mmol, 1 eq) was added to NaH (123.68 mg, 3.09 mmol, 60% purity, 2 eq) in THF (15 mL). After stirring at 15 °C for 30 min, the mixture was cooled to 0 °C and 2-(chloromethoxy)ethyl-trimethyl-silane (515.53 mg, 3.09 mmol, 547.27 uL, 2 eq) was added. The mixture was stirred at 15 °C for 12 h. The reaction mixture was quenched by addition H₂O 20 mL, and then extracted with EtOAc (20 mLx3). The combined organic layers were washed with brine (20 mLx2), dried over Na₂SO₄, filtered and concentrated under reduced pressure to afford the title compound (0.3 g, crude) as yellow oil, which was used for the next step without further purification.

### Step 2: Tert-butyl (3S)-3-[[5-(trifluoromethyl)-4-[6-[2-(2-trimethylsilylethoxymethyl)pyrazol-3- yl]-1H-indol-3-yl]pyrimidin-2-yl]amino]piperidine-1-carboxylate

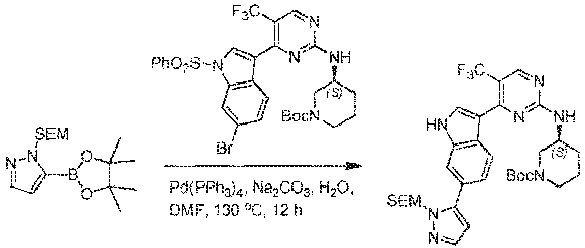

To a solution of tert-butyl (3S)-3-[[4-[1-(benzenesulfonyl)-6-bromo-indol-3-yl]-5-(trifluoromethyl)pyrimidin-2-yl]amino]piperidine-1-carboxylate (150 mg, 220.41 umol, 1 eq) in DMF (1 mL) and H₂O (0.1 mL) was added trimethyl-[2-[[5-(4,4,5,5-tetramethyl- 1,3,2-dioxaborolan-2-yl)pyrazol-1-yl]methoxy]ethyl]silane (142.96 mg, 440.82 umol, 2 eq), Pd(PPh₃)₄ (76.41 mg, 66.12 umol, 0.3 eq) and Na₂CO₃ (46.72 mg, 440.82 umol, 2 eq). The mixture was stirred under N₂ at 130 °C for 12 h. The mixture was poured into H₂O 10 mL, extracted with EtOAc (30 mLx3). The combined organic layers were washed with brine (20 mLx2), dried over Na₂SO₄, filtered and concentrated under reduced pressure to give the crude product. The residue was purified by column chromatography (SiO₂, PE/EtOAc=10:1) to afford the title compound (0.1 g, crude) as yellow solid. (The reaction was combined with another reaction in 50 mg scale for purification).

### Step 3: N-[(3S)-3-piperidyl]-4-[6-(1H-pyrazol-3-yl)-1H-indol-3-yl]-5-(trifluoromethyl) pyrimidin-2-amine

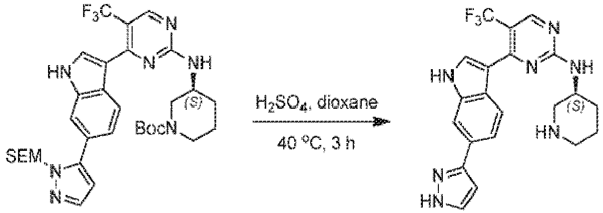

To a solution of tert-butyl (3S)-3-[[5-(trifluoromethyl)-4-[6-[2-(2-trimethylsilylethoxymethyl) pyrazol-3-yl]-1H-indol-3-yl] pyrimidin-2-yl] amino] piperidine-1-carboxylate (80 mg, 121.62 umol, 1 eq) in dioxane (0.5 mL) was added H₂SO₄ (119.28 mg, 1.22 mmol, 64.83 uL, 10 eq). The mixture was stirred at 40 °C for 3 h. The mixture was concentrated under reduced pressure to remove dioxane. The residue was diluted with ACN 1 mL. Then K₂CO₃ (0.2 g, powder) was added. The mixture was stirred at 15°C for another 1 h. Then the mixture was poured into H₂O (10 mL), and extracted with EtOAc (10 mLx2), the combined organic layers were washed with brine (10 mLx2), dried over Na₂SO₄, filtered and concentrated under reduced pressure to give a residue. The residue was purified by prep-HPLC (HCl condition) to afford the title compound (18.8 mg, HCl salt, 99% purity) as a white solid.

### Example 107. (3R)-1-[3-[2-[[(3S)-3-piperdyl] amino]-5-(trifluoromethyl) pyrimidin-4-yl]-1H-indole -6-carbonyl] pyrrolidine-3-carbonitrile (Compound 292).

### Step 1: Tert-butyl (3S)-3-[[4-[6-[(3R)-3-cyanopyrrolidine-1-carbonyl]-1H-indol-3-yl]-5-(trifluoromethyl) pyrimidin-2-yl] amino] piperidine-1-carboxylate

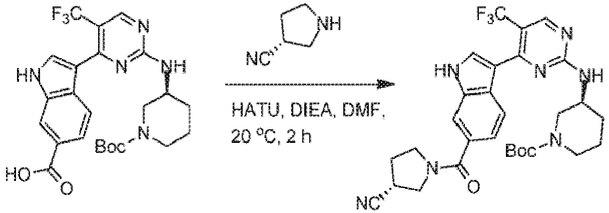

A mixture of 3-[2-[[(3S)-1-tert-butoxycarbonyl-3-piperidyl] amino]-5-(trifluoromethyl) pyrimidin-4-yl]-1H-indole-6-carboxylicacid (0.06 g, 118.70 umol, 1 eq), (3R)-pyrrolidine-3-carbonitrile (18.89 mg, 142.44 umol, 1.2 eq, HCl), HATU (45.13 mg, 118.70 umol, 1 eq), DIEA (46.02 mg, 356.09 umol, 62.02 uL, 3 eq) in DMF (1 mL) was stirred at 20°C for 2 h. The reaction mixture was poured into water 10 mL. The solid was formed and filtered to collect the cake to afford the title compound (50 mg, crude) as a yellow solid, which was used for next step directly.

### Step 2: (3R)-1-[3-[2-[[(3S)-3-piperidyl] amino]-5-(trifluoromethyl) pyrimidin-4-yl]-1H-indole -6-carbonyl] pyrrolidine-3-carbonitrile

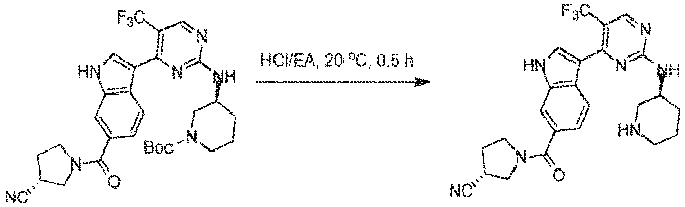

A mixture of tert-butyl (3S)-3-[[4-[6-[(3R)-3-cyanopyrrolidine-1-carbonyl] -1H-indol-3-yl] -5-(trifluoromethyl) pyrimidin-2-yl] amino] piperidine-1-carboxylate (0.05 g, 85.67 umol, 1 eq) in HCl/EtOAc (4 M, 642.56 uL, 30 eq) was stirred at 20 °C for 0.5 h. It was concentrated. The residue was purified by prep-HPLC (FA condition) to afford the title compound (10 mg, 18.70 umol, 99% purity, FA) as an off-white solid.

### Example 108. (3S)-1-[3-[2-[[(3S)-3-piperidyl] amino]-5-(trifluoromethyl) pyrimidin-4-yl]-1H-indole -6-carbonyl] pyrrolidine-3-carbonitrile (Compound 293).

### Step 1: Tert-butyl (3S)-3-[[4-[6-[(3S)-3-cyanopyrrolidine-1-carbonyl]-1H-indol-3-yl]-5-(trifluoromethyl) pyrimidin-2-yl] amino] piperidine-1-carboxylate

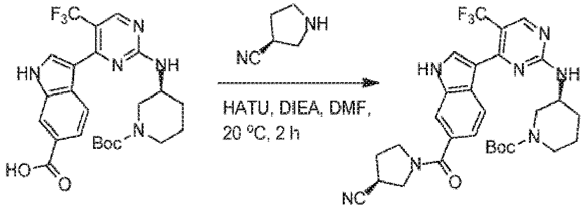

A mixture of 3-[2-[[(3S)-1-tert-butoxycarbonyl-3-piperidyl] amino]-5-(trifluoromethyl) pyrimidin-4-yl]-1H-indole-6-carboxylicacid (0.06 g, 118.70 umol, 1 eq), (3S)-pyrrolidine-3-carbonitrile (18.89 mg, 142.44 umol, 1.2 eq, HCl), HATU (45.13 mg, 118.70 umol, 1 eq), DIEA (46.02 mg, 356.09 umol, 62.02 uL, 3 eq) in DMF (1 mL) was stirred at 20°C for 2 h. The reaction mixture was poured into water 10 mL. The solid was formed and filtered to collect the cake to afford the title compound (60 mg, crude) as a yellow solid, which was used for next step directly.

### Step 2: (3S)-1-[3-[2-[[(3S)-3-piperidyl] amino]-5-(trifluoromethyl) pyrimidin-4-yl]-1H-indole -6-carbonyl] pyrrolidine-3-carbonitrile

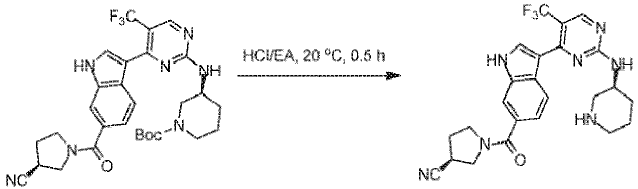

A mixture of tert-butyl (3S)-3-[[4-[6-[(3S)-3-cyanopyrrolidine-1-carbonyl] -1H-indol-3-yl] -5-(trifluoromethyl) pyrimidin-2-yl] amino] piperidine-1-carboxylate (0.06 g, 102.81 umol, 1 eq) in HCl/EtOAc (4 M, 771.07 uL, 30 eq) was stirred at 20°C for 0.5h. It was concentrated. The residue was purified by prep-HPLC (FA condition) to afford the title compound (8.2 mg, 15.49 umol, 100% purity, FA) as off-white solid.

### Example 109. 3-[5-chloro-2-[[(3S)-3-piperidyi] amino] pyrimidin-4-yl] -7-methylsulfonyl -1H-indole -6-carbonitrile (Compound 294).

### Step 1: 3-[5-chloro-2-[[(3S)-3-piperidyl] amino] pyrimidin-4-yl] -7-methylsulfonyl -1H-indole -6-carbonitrile

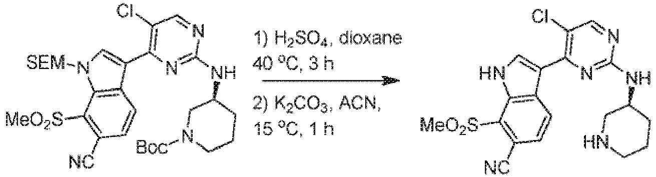

To a solution of tert-butyl (3S)-3-[[5-chloro-4-[6-cyano-7-methylsulfonyl-1- (2-trimethylsilylethoxymethyl) indol-3-yl] pyrimidin-2-yl] amino] piperidine-1-carboxylate (0.1 g, 151.22 umol, 1 eq) in dioxane (1 mL) was added H₂SO₄ (148.31 mg, 1.51 mmol, 80.61 uL, 10 eq). The mixture was stirred at 40 °C for 3 h. The reaction mixture was concentrated under reduced pressure to remove dioxane. The residue was diluted with ACN 2 mL. Then K₂CO₃ (0.2 g, powder) was added. The mixture was stirred at 15 °C for another 1 h. Then the mixture was poured into H₂O (10 mL), and extracted with EtOAc (10 mLx2), the combined organic layers were washed with brine (10 mLx2), dried over Na₂SO₄, filtered and concentrated under reduced pressure to give the crude product. The residue was purified by prep-HPLC (HCl condition) to afford the title compound (23 mg, 47.49 umol, 31.40% yield, 96.5% purity mmol, 98.79% yield) (HCl) as yellow solid.

### Example 110. [(3R)-3-hydroxy-3-(trifluoromethyl) pyrrolidin-1-yl]-[3-[2-[[(3S)-3-piperidyl]amino]-5-(trifluoromethyl) pyrimidin-4-yl]-1Hindol-6-yl] methanone (Compound 299) and [(3S)-3-hydroxy-3-(trifluoromethyl)pyrrolidin-1-yl]-[3-[2-[[(3S)-3-piperidyl]amino]-5-(trifluoromethyl)pyrimidin-4-yl]-1Hindol-6-yl]methanone (Compound 300).

### Step 1: Tert-butyl(3S)-3-[[4-[6-[(3R)-3-hydroxy-3-(trifluoromethyl)pyrrolidine-1-carbonyl]-1H-indol-3-yl]-5-(trifluoromethyl)pyrimidin-2-yl]amino]piperidine-1-carboxylatec (compound 1) & Tert-butyl(3S)-3-[[(E)-N'-[(E)-3,3,3-trifluoro-1-[6-[(3S)-3-hydroxy-3 (trifluoromethyl) pyrrolidine-1-carbonyl]-1H-indol-3-yl] prop-1-enyl] carbamimidoyl]amino]piperidine-1-carboxylat(compound 2)

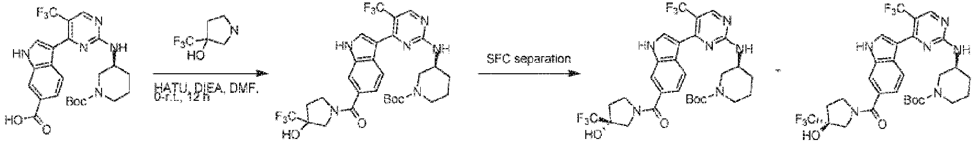

To a solution of 3-[2-[[(3S)-1-tert-butoxycarbonyl-3-piperidyl]amino]-5-(trifluoromethyl)pyrimidin-4-yl]-1H-indole-6-carboxylic acid (0.2 g, 395.66 umol, 1 eq) and 3-(trifluoromethyl)pyrrolidin-3-o1 (98.54 mg, 514.35 umol, 1.3 eq, HCl) in DMF(5 mL) was added HATU (180.53 mg, 474.79 umol, 1.2 eq) and DIEA (153.40 mg, 1.19 mmol, 206.74 uL, 3 eq) at 0°C. The mixture was stirred at 15°C for 12 h. The residue was poured into water (30 mL). The aqueous phase was extracted with EtOAc (30 mLx3). The combined organic phase was washed with brine (30 mLx2), dried with anhydrous Na₂SO₄, filtered and concentrated in vacuum. The residue was purified by prep-HPLC (TFA). The aqueous phase was adjusted pH to 9 with sat. NaHCO₃ and extracted with EtOAc (30 mLx3). The combined organic phase was dried with anhydrous Na₂SO₄, filtered and concentrated in vacuum to give 0.1 g product. Then the residue was separated by (SFC (column:AD(250mm*30mm,5um); mobile phase: [0.1%NH₃H₂O ETOH];B%:35%-35%,min) to afford the title compound 1 as (30 mg, 46.69 umol, 11.80% yield, peak 1:RT 2.48 min, 30 mg) as white solid and the title compound 2 (35 mg, 55.33 umol, 13.98% yield, peak 2, RT=2.78 min, 35 mg) as white solid (The reaction was combined with another reaction in 20 mg scale for purification).

### Step 2: [(3R)-3-hydroxy-3-(trifluoromethyl) pyrrolidin-1-yl]-[3-[2-[[(3S)-3-piperidyl]amino]-5-(trifluoromethyl) pyrimidire-4-yl]-1Hindol-6-yl] methanone

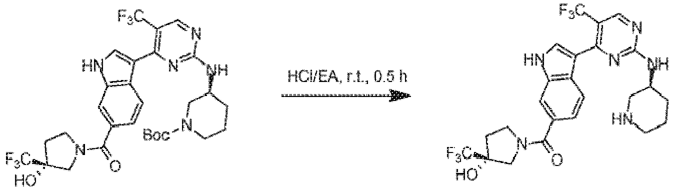

A mixture of tert-butyl (3S)-3-[[4-[6-[(3R)-3hydroxy-3-(trifluoromethyl) pyrrolidine-1-carbonyl]-1H-indol-3-yl]-5-(trifluoromethyl) pyrimidin-2-yl] amino] piperidine-1-carboxylate (30 mg, 46.69 umol, 1 eq) in HCl/EtOAc (0.5 mL) was stirred at 15 °C for 0.5 h. The mixture was concentrated in vacuum. The residue was purified by prep-HPLC (FA) to afford the title compound (11.1 mg, 18.86 umol, 40.40% yield, 100% purity, FA, temporarily assigned structure) as white solid.

### Step 3: [(3S)-3-hydroxy-3-(trifluoromethyl)pyrrolidin-1-yl]-[3-[2-[[(3S)-3-piperidyl]amino]-5-(trifluoromethyl)pyrimidin-4-yl]-1Hindol-6-yl]methanoine

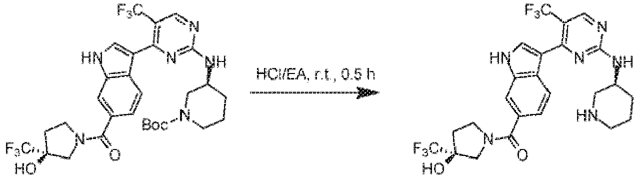

A mixture of tert-butyl (3S)-3-[[4-[6-[(3S)-3-hydroxy-3-(trifluoromethyl) pyrrolidine-1-carbonyl]-1H-indol-3-yl]-5-(trifluornmethyl) pyrimidin-2-yl] amino] piperidine-1-carboxylate (30 mg, 46.69 umol, 1 eq) in HCl/EtOAc (0.5 mL) was stirred at 15 °C for 0.5 h. The mixture was concentrated in vacuum. The residue was purified by prep-HPLC (FA) to afford the title compound (15.14 mg, 25.67 umol, 54.98% yield, 99.77% purity, FA, temporarily assigned structure) as white solid.

### Example 111. 3-[5-(difluoromethoxy)-2-[[(3S)-3-piperidyl] amino] pyrimidin-4-yl]-1H-indole-6-carbonitrile (Compound 301).

### Step 1: 3-(2-chloro-5-methoxy-pyrimidin-4-yl)-1H-indole-6-carbonitrile

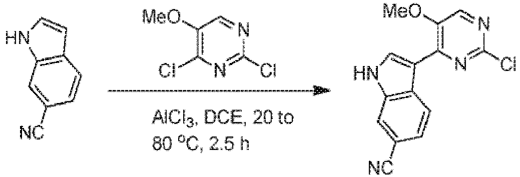

A flask was fitted with 2, 4-dichloro-5-methoxy-pyrimidine (3.78 g, 21.10 mmol, 1 eq) in DCE (15 mL). Then AlCl₃ (2.81 g, 21.10 mmol, 1.15 mL, 1 eq) was added by portion. Then the reaction mixture was stirred at 20 °C for 0.5 h. 1H-indole-6-carbonitrile (3 g, 21.10 mmol, 1 eq) was added. The reaction mixture continues to react at 80 °C for 2 h. The reaction mixture was poured into saturated Na₂CO₃ solution (200 mL) and some precipitate emerged. The reaction mixture was filtered and the cake was collected. The cake was recrystallized from MeOH (15 mL). The mixture was filtered and the cake was collected to afford two batches of the title compound. Batch 1 (2.3 g, 81% purity) was obtained as yellow solid. Batch 2 (1.2 g, 66% purity) was obtained as yellow solid.

### Step 2: 1-(benzenesulfonyl)-3-(2-chloro-5-hydroxy-pyrimidin-4-yl)indole-6-carbonitrile

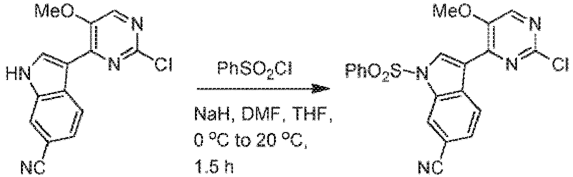

To a solution of 3-(2-chloro-5-methoxy-pyrimidin-4-yl)-1H-indole-6-carbonitrile (1.3 g, 4.57 mmol, 1 eq) in DMF (27 mL) and THF (3 mL) was added NaH (200.89 mg, 5.02 mmol, 60% purity, 1.1 eq) at 0 °C. Then the reaction mixture was stirred at 0 °C for 30 min before benzenesulfonyl chloride (887.13 mg, 5.02 mmol, 642.85 uL, 1.1 eq) was added. After that the reaction mixture was stirred at 20 °C for 1 h. The reaction mixture was poured into H2O (30 mL) and large amount of precipitate emerged. The reaction mixture was filtered and the cake was washed by MeOH (8 mL). The cake was collected and dried under vacuum to afford the title compound (1.3 g, 2.81 mmol, 61.52% yield, 91.8% purity) as grey solid.

### Step 3: 1-(benzenesulfonyl)-3-(2-chloro-5-hydroxy-pyrimidin-4-yl)indole-6-carbonitrile

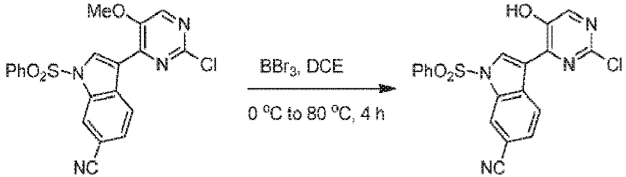

A flask was fitted with 1-(benzenesulfonyl)-3-(2-chloro-5-methoxy-pyrimidin-4-yl) indole-6-carbonitrile (100 mg, 235.37 umol, 1 eq) and DCE (10 mL). At 0 °C, BBr₃ (589.66 mg, 2.35 mmol, 226.79 uL, 10 eq) was added. Then the reaction mixture was heated to 80 °C and reacted for 4 h. The reaction mixture was poured into H₂O (50 mL) and DCM (50 mLx2) was used to extract the product. The organic layer was washed by brine (40 mL), dried over Na₂SO₄. filtered and evaporated to afford the title compound (250 mg, 480.73 umol, 68.08% yield, 79% purity) as brown solid.

### Step 4: (1-(benzenesulfonyl)-3-[2-chloro-5-(difluoromethoxypyrimidin-4-yl]indole-6-carbonitrile

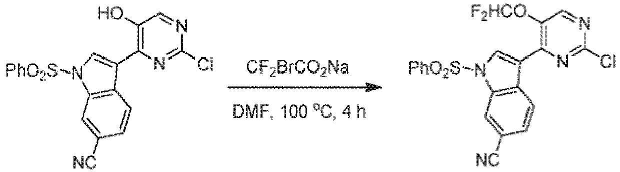

A solution of 1-(benzenesulfonyl)-3-(2-chloro-5-hydroxy-pyrimidin-4-yl) indole-6-carbonitrile (120 mg, 292.09 umol, 1 eq) and sodium chlorodifluoroacetate (44.53 mg, 292.09 umol, 1 eq) in DMF (8 mL). The mixture was heated to 100 °C and stirred for 4 hr under N₂. H₂O (20 mL) was poured into the mixture and EtOAc (40 mLx3) was used to extract the product. The organic extract was washed by brine (30 mL), dried over Na₂SO₄, filtered and evaporated to afford the crude product (300 mg). The crude product (200 mg) was purified by prep-TLC (PE: EtOAc =1:1) to afford the tide compound (90 mg, 42.5% purity) as light brown solid.

### Step 5: tert-butyl (3S)-3-[[4-(6-cyano-1H-indol-3-yl)-5-(difluoromethoxy)pyrimidin-2-yl]amino]piperidine-1-carboxylate

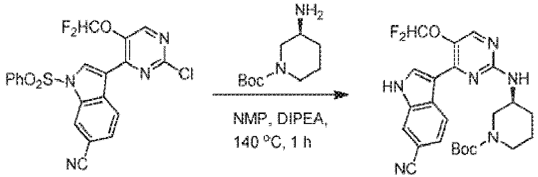

A flask was fitted with 1-(benzenesulfonyl)-3-[2-chloro-5-(difluoromethoxy) pyrimidin-4-yl]indole-6-carbonitrile (50 mg, 108.50 umol, 1 eq), tert-butyl (3S)-3-aminopiperidine-1-carboxylate (21.73 mg, 108.50 umol, 1 eq) and DIPEA (70.11 mg, 542.49 umol, 94.49 uL, 5 eq) in NMP (1 mL). The reaction mixture was heated to 140 °C for 1 h. The reaction mixture was poured into H₂O (20 mL) and EtOAc (35 mL) was used to extract the product. The organic layer was washed by brine (25 mL), dried over Na₂SO₄, filtered and evaporated to afford the title compound (60 mg, crude) as red solid. It will be used directly in next step.

### Step 6: 3-[5-(difluoromethoxy)-2-[[(3S)-3-piperidyl] amino] pyrimidin-4-yl]-1H-indole-6-carbonitrile

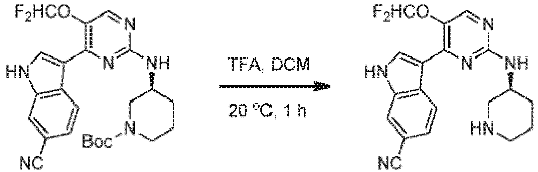

A flask was fitted with tert-butyl (3S)-3-[[4-(6-cyano-1H-indol-3-yl)-5-(difluoromethoxy) pyrimidin-2-ylj amino] piperidine-1-carboxylate (110 mg, 227.04 umol, 1 eq) in TFA (0.8 mL) and DCM (3 mL). The reaction mixture was stirred at 20 °C for 1 h. The reaction mixture was evaporated to afford the crude product The crude product was purified by acidic prep-HPLC to afford the title compound (2.92 mg, 6.94 umol, 3.06% yield, 100% purity, HCl) as yellow solid.

### Example 112. 1-[3-[2-[[(3S)-3-piperidyl] amino]-5-(trifluoromethyl) pyrimidin-4-yl]-1H-indole-6-carbonyl] pyrrolidin-3-one (Compound 302).

### Step 1: tert-butyl (3S)-3-[[4-[6-(3-oxopyrrolidine-1-carbonyl)-1H-indol-3-yl]-5-(trifluoromethyl)pyrimidin-2-yl]amino]piperidine-1-carboxylate

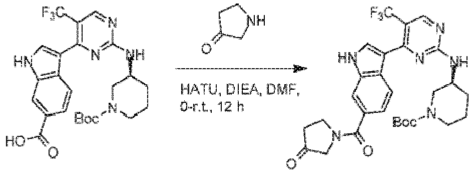

To a solution of 3-[2-[[(3S)-1-tert-butoxycarbonyl-3-piperidyl]amino]-5-(trifluoromethyl)pyrimidin-4-yl]-1H-indole-6-carboxylic acid (0.08 g, 158.26 umol, 1 eq) in DMF (5 mL) was added HATU (72.21 mg, 189.92 umol, 1.2 eq), DIEA (61.36 mg, 474.79 umol, 82.70 uL, 3 eq) and pyrrolidin-3-one (16.16 mg, 189.92 umol, 1.2 eq) at 0°C. The mixture was warmed to 20°C and stirred for 12 h. The reaction mixture was quenched by addition water 50 mL and extracted with EtOAc (15 mL x 3). The combined organic layers were washed with brine (15 mL x 3), dried over Na₂SO₄, filtered and concentrated under reduced pressure to give a residue. The residue was purified by column chromatography (SiO₂, DCM: MeOH = 20:1) to afford the title compound (45 mg) as a white solid. (The reaction was combined with another reaction in 20 mg scale for purification).

### Step 2: 1-[3-[2-[[(3S)-3-piperidyl] amino]-5-(trifluoromethyl) pyrimidin-4-yl]-1H-indole-6-carbonyl] pyrrolidin-3-one

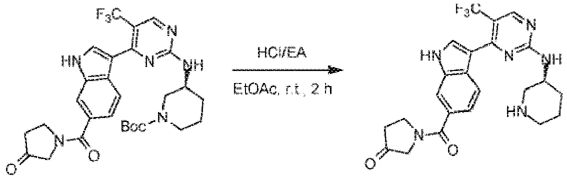

To a solution of tert-butyl (3S)-3-[[4-[6-(3-oxopyrrolidine-1-carbonyl)-1H-indol-3-yl]-5-(trifluoromethyl) pyrimidin-2-yl] amino] piperidine-1-carboxylate (0.04 g, 69.86 umol, 1 eq) in EtOAc (1 mL) was added HCl/EtOAc (4 M, 1 mL). The mixture was stirred at 20 °C for 2 h. The reaction mixture was concentrated under reduced pressure to give a residue. The residue was purified by prep-HPLC (HCl condition) to afford the title compound (5.88 mg, 10.40 umol, 14.88% yield, 90% purity, HCl) as a yellow solid.

### Example 113. 4-[6-(1-methylpyrazol-3-yl)-1H-indol-3-yl]-N-[(3S)-3-piperidyl]-5-(trifluoromethyl) pyrimidin-2-amine (Compound 303).

### Step 1: 1-methyl-3-(4, 4, 5, 5-tetramethyl-1, 3, 2-dioxaborolan-2-yl) pryrazole

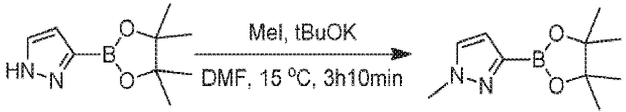

To a solution of 3-(4,4,5,5-tetramethyl-1,3,2-dioxaborolan-2-yl)-1H-pyrazole (0.5 g, 2.58 mmol, 1 *eq*) in DMF (5 mL) was added t-BuOK (1 M, 5.15 mL, 2 *eq*)*.* The mixture was stirred at 15 °C for 10 min. Then MeI (1.10 g, 7.73 mmol, 481.25 uL, 3 *eq*) was added. The resulting mixture was stirred at 15 °C for another 3 h. The reaction mixture was poured into H₂O (10 mL), and extracted with EtOAc (10 mLx3). The combined organic layers were washed with brine (10 mLx2), dried over Na₂SO₄, filtered and concentrated under reduced pressure to afford the title compound (0.3 g, crude) as yellow oil, which was used into the next step without further purification.

### Step 2: Tert-butyl (3S)-3-[[4-[6-(1-methylpyrazol-3-yl)-1H-indol-3-yl]-5-(trifluoromethyl) pyrimidin-2-yl] amino] piperidine-1-carboxylate

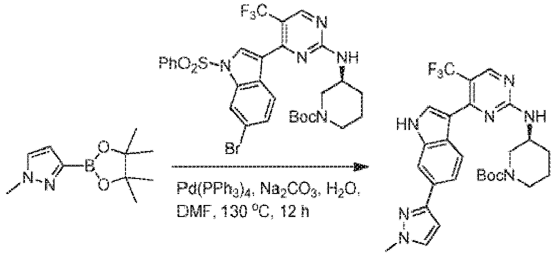

To a solution of tert-butyl (3S)-3-[[4-[1-(benzenesulfonyl)-6-bromo-indol-3-yl]-5-(trifluoromethyl)pyrimidin-2-yl]amino]piperidine-1-carboxylate (0.15 g, 220.41 umol, 1 *eq*) in DMF (1 mL) and H₂O (0.5 mL) was added 1-methyl-3-(4,4,5,5-tetramethyl-1,3,2-dioxaborolan-2-yl)pyrazole (91.72 mg, 440.83 umol, 2 *eq*), Na₂CO₃ (70.09 mg, 661.24 umol, 3 *eq*) and Pd(PPh₃)₄ (25.47 mg, 22.04 umol, 0.1 *eq*)*.* The mixture was stirred under N₂ at 130°C for 12 h. The reaction mixture was poured into H₂O (10 mL), and extracted with EtOAc (10 mLx3). The combined organic layers were washed with brine (10 mLx3), dried over Na₂SO₄, filtered and concentrated under reduced pressure to give a residue. The residue was purified by prep-TLC (PE: EtOAc= 1:1) to afford the title compound (0.06 g, crude) as a white solid.

### Step 3: 4-[6-(1-methylpyrazol-3-yl)-1H-indol-3-yl]-N-[(3S)-3-piperidyl]-5-(trifluoromethyl) pyrimidin-2-amine

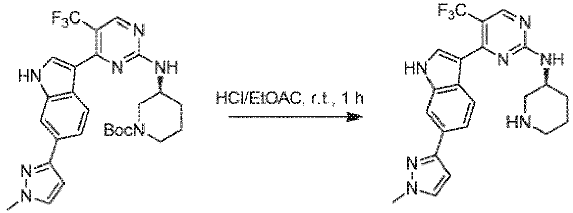

To a solution of tert-butyl (3S)-3-[[4-[6-(1-methylpyrazol-3-yl)-1H-indol-3-yl] -5-(trifluoromethyl) pyrimidin-2-yl] amino] piperidine-1-carboxylate (0.06 g, 110.79 umol, 1 *eq*) in EtOAc (1 mL) was added HCl/EtOAc (4 M, 1 mL, 36.10 *eq*). The mixture was stirred at 15 °C for 1 h. The reaction mixture was concentrated under reduced pressure to give a residue. The residue was purified by prep-HPLC (HCl condition) to afford the title compound (7.1 mg, 14.86 umol, 13.41% yield, 100% purity, HCl) as yellow solid.

### Example 114. 3-[2-[[(3S)-3-piperidyl] amino]-5-(trifluoromethyl) pyrimidin-4-yl]-1H-indole-6-Carboxylic acid (Compound 306).

### Step 1: Methyl 3-[2-chloro-5-(trifluoromethyl) pyrimidin-4-yl]-1H-indole-6-carboxylate

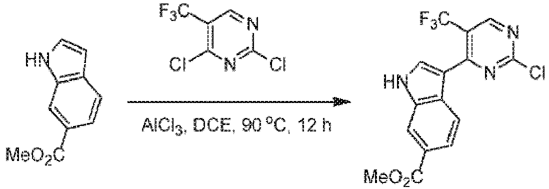

To a solution of 2, 4-dichloro-5-(trifluoromethyl) pyrimidine (12.39 g, 57.08 mmol, 2 eq) in DCE (120 mL) was added AlCl₃ (8 g, 60.00 mmol, 3.28 mL, 2.10 eq). The mixture was stirred at 90 °C for 0.5 h. Then methyl 1H-indole-6-carboxylate (5 g, 28.54 mmol, 1 eq) was added slowly at 90°C. The resulting mixture was stirred at 90 °C for 11.5 h. The mixture was poured into water (200 mL) and filtered, the filter cake was washed with EtOAc (100 mLx10). The aqueous phase was extracted with EtOAc (100 mLx3). The combined organic phase was dried with anhydrous Na₂SO₄, filtered and concentrated in vacuum. The residue was purified by silica gel chromatography (PE: EtOAc=5:1-3:1-1:1, contained 10% DCM) to give 6 g crude product. The crude product was dissolved in MeOH (100 mL) and stirred for 0.5 h, and then filtered and washed with MeOH (30 mL). The solid was purified by prep-HPLC (TPA) and concentrated. The residue was adjusted pH to 9 with sat. NaHCO₃ and extracted with EtOAc (100 mLx2). The combined organic phase was dried with anhydrous Na₂SO₄, filtered and concentrated in vacuum to afford the title compound as white solid.

### Step 2: Methyl 3-[2-[[(3S)-1-tert-butoxycarbonyl-3-piperidyl] amino]-5-(trifluoromethyl) Pyrimidin -4-yl]-1H-indole-6-carboxylate

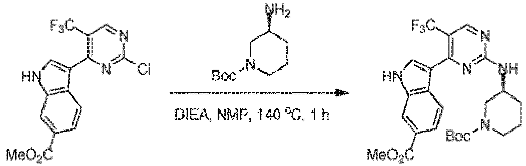

To a solution of methyl 3-[2-chloro-5-(trifluoromethyl) pyrimidin-4-yl]-1H-indole-6 -carboxylate (1.5 g, 4.22 mmol, 1 eq) and tert-butyl (3S)-3-aminopiperidine-1-carboxylate (1.01 g, 5.06 mmol, 1.2 eq) in NMP (20 mL) was added DIEA (2.73 g, 21.09 mmol, 3.67 mL, 5 eq). The mixture was stirred at 140 °C for 1 h. The residue was poured into water (20mL). The aqueous phase was extracted with EtOAc (30 mLx5). The combined organic phase was washed with brine (50 mLx2), dried with anhydrous Na₂SO₄, filtered and concentrated in vacuum. The residue was purified by MPLC (PE: EtOAc=3:1-2:1-1:1) to afford the title compound (1.43 g, 2.70 mmol, 63.97% yield, 98% purity) as white solid.

### Step 3: 3-[2-[1(3S)-1-tert-butoxycarbonyl-3-piperidyl] amino]-5-(trifluoromethyl) pyrimidin-4-yl]-1H-indole-6-carboxylic acid

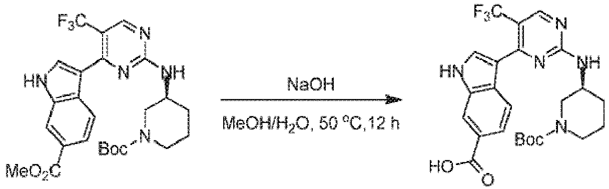

To a solution of methyl 3-[2-[[(3S)-1-tert-butoxycarbonyl-3-piperidyl] amino]-5 (trifluoromethyl) pyrimidin-4-yl]-1H-indole-6-carboxylate (1.43 g, 2.75 mmol, 1 eq) in MeOH (15 mL) /H₂O (5 mL) was added NaOH (550.47 mg, 13.76 mmol, 5 eq). The mixture was stirred at 50 °C for 12 h. The mixture was concentrated in vacuum to give a residue. The residue was adjusted to pH to 3 with HCl (1N) and extracted with EtOAc (100 mLx3). The combined organic phase was washed with brine (50 mL), dried with anhydrous Na₂SO₄, filtered and concentrated in vacuum to afford the title compound (1.3 g, crude) as yellow solid.

### Step 4: 3-[2-[[(3S)-3-piperidyl] amino]-5-(trifluoromethyl) pyrimidin-4-yl]-1H-indole-6-Carboxylic acid

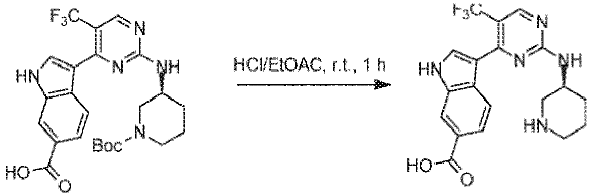

A solution of 3-[2-[[(3S)-1-tert-butoxycarbonyl-3-piperidyl] amino]-5-(trifluoromethyl) pyrimidin-4-yl]-1H-indole-6-carboxylicacid (50 mg, 98.91 umol, 1 eq) in HCl/EtOAc (10 mL) was stirred at 15 °C for 1 h. The mixture was concentrated in vacuum. The residue was purified by prep-HPLC (HCl condition) to afford the title compound (12.55 mg, 28.12 umol, 28.43% yield, 98.99% purity, HCl) as yellow solid.

### Example 115. [(4S)-3,3-difluoro-4-hydroxy-pyrrolidin-1-yl]-[3-[2-[[(3S)-3-piperidyl]amino]-5-(trifluoromethyl)pyrimidin-4-yl]-1H-indol-6-yl]methanone (Compound 308) and [(4R)-3, 3-difluoro-4-hydroxy-pyrrolidin-1-yl]- [3-[2-[[(3S)-3-piperidyl] amino]-5-(trifluoromethyl) pyrimidin-4-yl]-1H-indol-6-yl] methanone (Compound 349).

### Step 1: 4, 4-difluoropyrrolidin-3-ol

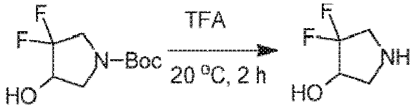

A solution of tert-butyl 3, 3-difluoro-4-hydroxy-pyrrolidine-1-carboxylate (0.3 g, 1.34 mmol, 1 *eq*) in TFA (3 mL) was stirred at 20°C for 2 h. The reaction mixture was concentrated under reduced pressure to afford the title compound (300 mg, TFA) as a white solid.

### Step 2: Tert-butyl (3S)-3-[[4-(6-chlorocarbonyl-1H-indol-3-yl)-5 (trifluoromethyl)pyrimidin-2-yl] amino] piperidine-1-carboxylate

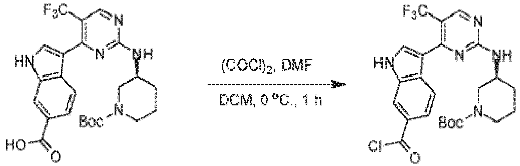

To a solution of 3-[2-[[(3S)-1-tert-butoxycarbonyl-3-piperidyl] amino]-5-(trifluoromethyl)pyrimidin-4-yl]-1H-indole-6-carboxylic acid (0.5 g, 989.14 umol, 1 *eq*) in DCM (5 mL) was added DMF (7.23 mg, 98.91 umol, 7.61 uL, 0.1 *eq*) and (COCl)₂ (150.66 mg, 1.19 mmol, 103.90 uL, 1.2 *eq*) at 0°C. The mixture was stirred at 0 °C for 1 h. The reaction mixture was concentrated under reduced pressure to afford the title compound (550 mg) as oil which was used in the next step directly

### Step 3: Tert-butyl (3S)-3-[[4-[6-(3, 3-difluoro-4-hydroxy-pyrrolidine-1-carbonyl)-1H-indol-3-yl]-5-(trifluoromethyl) pyrimidin-2-yl]amino]piperidine-1-carboxylate

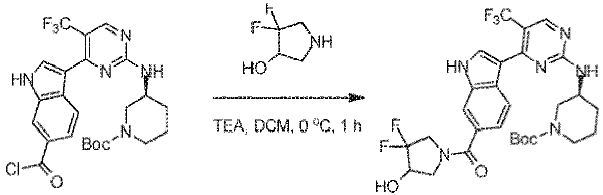

To a solution of tert-butyl (3S)-3-[[4-(6-chlorocarbonyl-1H-indol-3-yl)-5-(trifluoromethyl)pyrimidin-2-yl]amino]piperidine-1-carboxylate (0.5 g, 954.32 umol, 1 *eq*) in DCM (5 mL) was added4,4-difluoropyrrolidin-3-ol (0.281 g, 1.19 mmol, 1.24 *eq*, TFA) and TEA (349.69 mg, 3.46 mmol, 0.481 mL, 3.62 *eq*). The mixture was stirred at 0 °C for 1 h. The reaction mixture was diluted with water 50 mL and extracted with DCM (15 mLx3). The combined organic layers were washed with brine (15 mLx3), dried over Na₂SO₄, filtered and concentrated under reduced pressure to give a residue. The residue was purified by column chromatography (SiO₂, PE: EtOAc=1:1) to afford the title compound (0.3 g, 489.87 umol, 51.33% yield, 99.7% purity) as a white solid.

### Step 4: [(4S)-3,3-difluoro-4-hydroxy-pyrrolidin-1-yl]-[3-[2-[[(3S)-3-piperidyl]amino]-5-(trifluoromethyl)pyrimidin-4-yl]-1H-indol-6-yl]methanone & [(4R)-3, 3-difluoro-4-hydroxy-pyrrolidin-1-yl]- [3-[2-[[(3S)-3-piperidyl] amino]-5-(trifluoromethyl) pyrimidin-4-yl]-1H-indol-6-yl] methanone

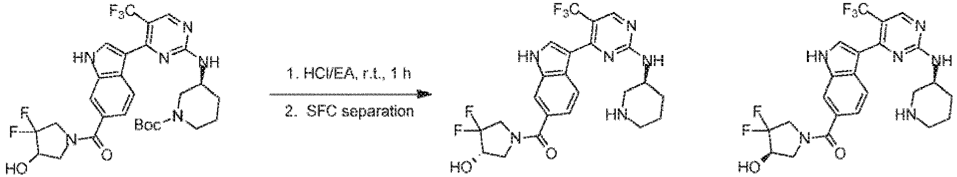

To a solution of tert-butyl (3S)-3-[[4-[6-(3, 3-difluoro-4-hydroxy-pyrrolidine-1-carbonyl)-1H-indol-3-yl]-5-(trifluoromethyl)pyrimidin-2-yl]amino]piperidine-1-carboxylate (0.12 g, 196.54 umol, 1 *eq*) in EtOAc (2 mL) was added HCl/EtOAc (4 M, 3 mL). The mixture was stirred at 20 °C for 1 h. The reaction mixture was concentrated under reduced pressure to afford the title compound 1 (33.5 mg, temporarily assigned structure) as a white solid and the title compound 2 (25.4 mg, temporarily assigned structure) as a white solid by SFC (Column: Chiralpak AD-H 250*30mm, i.d. 5u; Mobile phase: A for CO₂ and B for EtOH(0.1%NH₃H₂O).

### Example 116. [(3R)-3-ethyl-3-hydroxy-pyrrolidin-1-yl]-[3-[2-[[(3S)-3-piperidyl]amino]-5-(trifluoromethyl)pyrimidin-4-yl]-1H-indol-6-yl]methanone (Compound 309) and [(3S)-3-ethyl-3-hydroxy-pyrrolidin-1-yl]-[3-[2-[[(3S)-3-piperidyl]amino]-5-(trifluoromethyl)pyrimidin-4-yl]-1H-indol-6-yl]methanone (Compound 347).

### Step 1: 1-benzyl-3-ethyl-pyrrolidin-3-ol

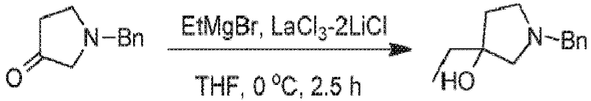

To a solution of LaCl_{3·2}LiCl (2.07 g, 6.28 mmol, 1.97 mL, 1.1 eq) in THF (10 mL) was added bromo(ethyl)magnesium (3 M, 2.85 mL, 1.5 eq) dropwise at 0°C. The mixture was stirred at 0 °C for 0.5 h. Then 1-benzylpyrrolidin-3-one (1 g, 5.71 mmol, 934.58 uL, 1 eq) was added to the above, and the mixture was stirred at 0 °C for 2 h. It was quenched with ice-NH₄Cl (50 mL), extracted with EtOAc (30 mLx5). The combined organic phases were washed dried with anhydrous Na₂SO₄, filtered and concentrated in vacuum. The residue was purified by silica gel chromatography (DCM: MeOH =100:1-50:1-40:1) to afford the title compound (0.72 g, 2.95 mmol, 51.62% yield, 84% purity) as brown oil.

### Step 2: 3-ethylpyrrolidin-3-ol

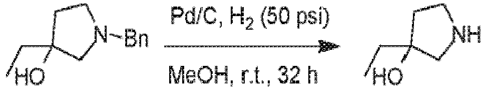

To a solution of 1-benzyl-3-ethyl-pyrrolidin-3-ol (0.25 g, 1.22 mmol, 1 eq) in MeOH (10 mL) was added Pd/C (10%, 50 mg) under N₂. The suspension was degassed under vacuum and purged with H₂ several times. The mixture was stirred under H₂ (50 psi) at 15 °C for 32 h. The reaction mixture was filtered and the filtrate was concentrated to afford the title compound (0.11 g, crude) as yellow oil and used directly in next step.

### Step 3: Tert-butyl (3S)-3-[[4-[6-[(3R)-3-ethyl-3-hydroxy-pyrrolidine-1-carbonyl]-1H-indol-3-yl]-5-(trifluoromethyl) pyrimidin-2-yl] amino] piperidine-1-carboxylate (compound 1) & Tert-butyl (3S)-3-[[4-[6-[(3S)-3-ethyl-3-hydroxy-pyrrolidine-1-carbonyl]-1H-indol-3-yl]-5-(trifluoromethyl) pyrimidin-2-yl] amino] piperidine-1-carboxylate(compound21)

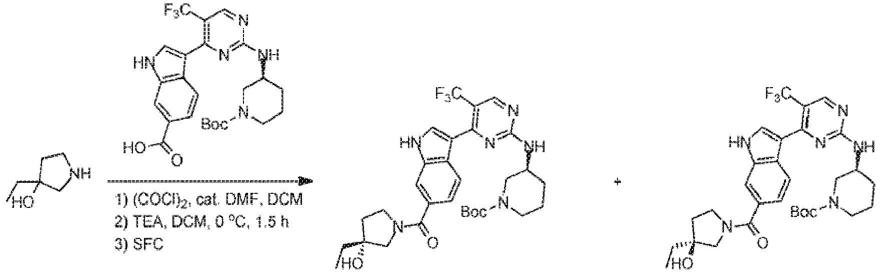

To a solution of 3-[2-[[(3S)-1-tert-butoxycarbonyl-3-piperidyl]amino]-5-(trifluoromethyl)pyrimidin-4-yl]-1H-indole-6-carboxylic acid (0.2 g, 395.66 umol, 1 eq) in DCM (2 mL) was added DMF (289.19 ug, 3.96 umol, 3.04e-1 uL, 0.01 eq) and (COCl)₂ (60.26 mg, 474.79 umol, 41.56 uL, 1.2 eq) at 0 °C under N₂. The mixture was stirred at 0°C for 0.5 h. Then the above mixture was added to a solution of 3-ethylpyrrolidin-3-ol (68.35 mg, 593.49 umol, 1.5 eq) and TEA (80.07 mg, 791.31 umol, 110.14 uL, 2 eq) in DCM (3mL), and the resulting mixture was stirred at 0 °C for another 1 h. The reaction mixture was poured into water (20 mL). The aqueous phase was extracted with DCM (20 mLx3). The combined organic phase was dried with anhydrous Na₂SO₄, filtered and concentrated in vacuum. The residue was purified by silica gel chromatography DCM: MeOH= (100:1-100:2-100:3), and then it was separated by SFC (column: AD (250mm*30mm,5um);mobile phase: [0.1%NH₃H₂O ETOH];B9b: 3596-359b,min) to afford the title compound 1 (35 mg, 58.08 umol, 14.68% yield, peak 1, temporarily assigned structure) as white solid and title compound 2 (40 mg, 66.37 umol, 16.78% yield, peak 2, temporarily assigned structure) as white solid.

### Step 4: [(3R)-3-ethyl-3-hydroxy-pyrrolidin-1-yl]-[3-[2-[[(3S)-3-piperidyl]amino]-5-(trifluoromethyl)pyrimidin-4-yl]-1H-indol-6-yl]methanone

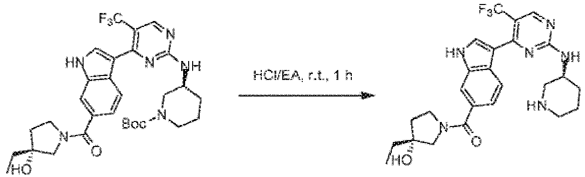

A solution of tert-butyl (3S)-3-[[4-[6-[(3R)-3-ethyl-3-hydroxy-pyrrolidine-1-carbonyl]- 1H-indol-3-yl]-5-(trifluoromethyl) pyrimidin-2-yl] amino] piperidine-1-carboxylate (30.00 mg, 49.78 umol, 1 eq) in HCl/EtOAc (5 mL) was stirred at 15 °C for 1 h. The mixture was concentrated in vacuum. The residue was washed with hexane (5 mL), filtered and the filter cake was collected to afford the title compound (8.83 mg, 98.59% purity, HCl) as yellow solid.

### [7U2] Step 5: [(3R)-3-ethyl-3-hydroxy-pyrrolidin-1-yl]-[3-[2-[[(3S)-3-piperidyl]amino]-5-(trifluoromethyl)pyrimidin-4-yl]-1H-indol-6-yl]methanone

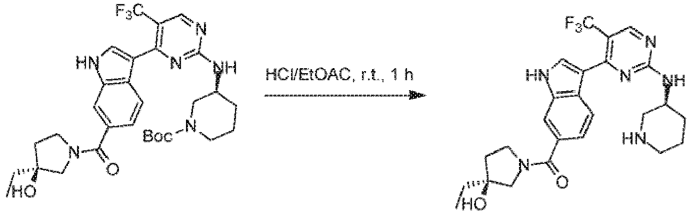

A solution of tert-butyl (3S)-3-[[4-[6-[(3S)-3-ethyl-3-hydroxy-pyrrolidine-1-carbonyl]-1H-indol-3-yl]-5-(trifluoromethyl) pyrimidin-2-yl] amino] piperidine-1-carboxylate (35.00 mg, 58.08 umol, 1 eq) in HCl/EtOAc (5 mL) was stirred at 15 °C for 1 h. The mixture was concentrated in vacuum. The residue was purified by prep-HPLC (FA) to afford the title compound (18.26 mg, 100% purity, FA) as white solid.

### Example 117. 4-(30, 30-dioxo-30thia-24-azatricyclododeca-, 2(12), 4(14), 11(15)-tetraen-14-yl)-N-[(3S)-3-piperidyl]-5-(trifluoromethyl) pyrimidin-2-amine (Compound 348).

### Step 1: Ethyl 3-(2,3-dihydro-1,4-benzothiazin-4-yl)-2-oxo-propanoate

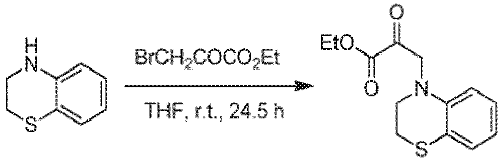

To a solution of 3, 4-dihydro-2H-1, 4-benzothiazine (10 g, 66.13 mmol, 1 *eq*) in THF (30 mL) was added ethyl 3-bromo-2-oxo-propanoate (12.90 g, 66.13 mmol, 8.27 mL, 1 *eq*) in small portion for 0.5 h. The mixture was stirred at 20 °C for 24 h. The reaction mixture was filtered and concentrated under reduced pressure to afford the title compound (14.5 g) which was used in the next step directly.

### Step 2: Ethyl-2,3-dihydro-[1,4]thiazino[2,3,4-hi]indole-6-carboxylate

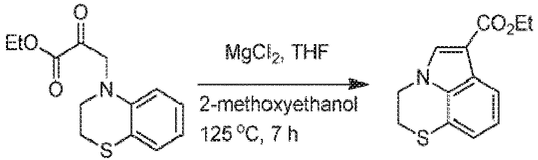

A solution of MgCl₂ (3.1 g, 32.56 mmol, 1.34 mL, 5.96e-1 *eq*) in 2-methoxyethanol (72.38 g, 951.12 mmol, 75 mL, 17.40 *eq*) was stirred at 125 °C for 30 min, then a solution of ethyl 3-(2,3-dihydro-1,4-benzothiazin-4-yl)-2-oxo-propanoate (14.5 g, 54.65 mmol, 1 *eq*) in 2-methoxyethanol (14.48 g, 190.22 mmol, 15 mL, 3.48 *eq*) and THF (5 mL) was added to the above solution at 125°C for 1 h, then the reaction mixture was stirred for 5.5 h. The reaction mixture was filtered and concentrated under reduced pressure to give a residue. The residue was purified by column chromatography (SiO₂, PE: EtOAc=2:1) to afford the title compound (2 g) as a brown oil.

### Step 3: 2,3-dihydro-[1,4]thiazino[2,3,4-hi]indole-6-carboxylic acid

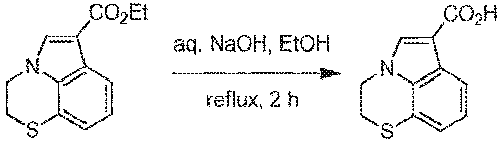

To a solution of ethyl 2,3-dihydro-[1,4]thiazino[2,3,4-hi]indole-6-carboxylate (2 g, 8.09 mmol, 1 eq) in H₂O (15 mL) and EtOH (22 mL) was added NaOH (800.00 mg, 20.00 mmol, 2.47 eq). The suspension was degassed and purged with N₂ for 3 times. The mixture was stirred at 80 °C for 2 h. To the reaction mixture was added DCM (10 mL) at 20 °C. The mixture was stirred for several minutes, and the organic layer was discarded. The pH of the water layer was adjusted to 6 with HCl (2 M), and the solid formed and was filtered to afford the title compound (1.1 g, 4.62 mmol, 57.07% yield, 92% purity) as a brown solid.

### Step 4: 2,3-dihydro-[1,4]thiazino[2,3,4-hi]indole

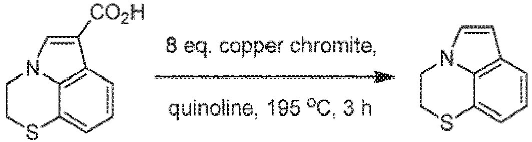

To a solution of 2,3-dihydro-[1,4]thiazino[2,3,4-hi]indole-6-carboxylic acid (1.1 g, 5.02 mmol, 1 *eq*) in quinoline (5 mL) was added copper chromite (880.00 mg, 3.80 mmol, 7.58e-1 *eq*). The suspension was degassed and purged with N₂ for 3 times. The mixture was stirred at 195 °C for 3 h. The reaction mixture was diluted with DCM (30 mL) and filtered. The filtrate was washed with HCl (2 M, 30 mL) twice and washed with aqueous of NaOH (2 M, 30 mL). The organic layer was washed with brine (30 mLx3), dried over Na₂SO₄, filtered and concentrated under reduced pressure to give a residue. The residue was purified by column chromatography (SiO₂, PE: EtOAc=10:1) to afford the title compound (500 mg) as a white solid.

### StepS: 6-(2-chloro-5-(trifluoromethyl)pyrimidin-4-yl)-2,3-dihydro-[1,4]thiazino[2,3,4-hi]indole

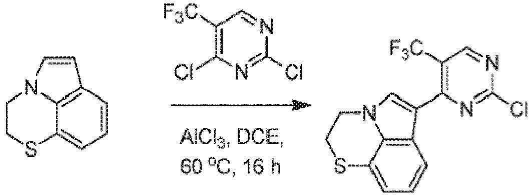

To a solution of 2,3-dihydro-[1,4]thiazino[2,3,4-hi]indole (0.5 g, 2.85 mmol, 1 *eq*) in DCE (5 mL) was added AlCl₃ (570.65 mg, 4.28 mmol, 233.87 uL, 1.5 *eq*) and 2, 4-dichloro-5-(trifluoromethyl) pyrimidine (619.05 mg, 2.85 mmol, 1 *eq*). The mixture was stirred at 60 °C for 16 h under N₂. The reaction mixture was quenched by water (40 mL) and then diluted with DCM (50 mL) and filtered. The filtrate was extracted with DCM (20 mLx3). The combined organic layers were washed with brine (20 mLx3), dried over Na₂SO₄, filtered and concentrated under reduced pressure to give a residue. The residue was purified by column chromatography (SiO₂, PE: EtOAc=20:1 to 0:1) to afford the title compound (500 mg) as a white solid.

### Step 6: 6-(2-chloro-5-(trifluoromethyl)pyrimidin-4-yl)-2,3-dihydro-[1,4]thiazino[2,3,4-hi]indole 1,1-dioxide

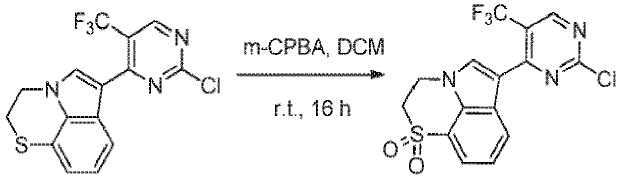

To a solution of 6-(2-chloro-5-(trifluoromethyl)pyrimidin-4-yl)-2,3-dihydro-[1,4]thiazino[2,3,4-hi]indole (0.4 g, 1.12 mmol, 1 *eq*) in DCM (5 mL) was added m-CPBA (456.52 mg, 2.25 mmol, 85% purity, 2 *eq*). The mixture was stirred at 20 °C for 16 h. The reaction mixture was quenched by addition saturated aqueous of Na₂SO₃ (100 mL) and NaHCO₃ (100 mL) for 0.5 h and extracted with DCM (25 mLx3). The combined organic layers were washed with brine (25 mLX3), dried over Na₂SO₄, filtered and concentrated under reduced pressure to give a residue. The residue was dissolved into MeOH (8 mL) and stirred for 0.5 h, then filtered to afford the title compound (380 mg) as a white solid.

### Step 7: (S)-tert-butyl 3-((4-(1,1-dioxido-2,3-dihydro-[1,4]thiazino[2,3,4-hi]indol-6-yl)-5-(trifluoromethyl)pyrimidin-2-yl)amino)piperidine-1-carboxylate

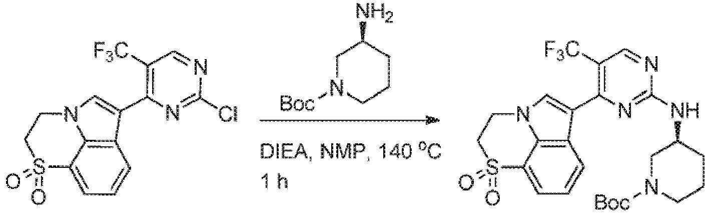

To a solution of 6-(2-chloro-5-(trifluoromethyl)pyrimidin-4-yl)-2,3-dihydro-[1,4]thiazino[2,3,4-hi]indole 1,1-dioxide (0.38 g, 979.98 umol, 1 eq) in NMP (4 mL) was added DIEA (189.98 mg, 1.47 mmol, 256.04 uL, 1.5 *eq*) and tert-butyl (3S)-3-aminopiperidine-1-carboxylate (196.27 mg, 979.98 umol, 1 *eq*). The mixture was stirred at 140 °C for 1 h under N₂. The reaction mixture was diluted with water (50 mL) and extracted with EtOAc (30 mLx3) and washed with water (30 mLx3). The organic layers were washed with brine (30 mL x 3), dried over Na₂SO₄ filtered and concentrated under reduced pressure to give a residue. The residue was purified by column chromatography (SiO₂, PE: EtOAc=1:1) to afford the title compound (400 mg) as a white solid

### Step 8: (S)-6-(2-(piperidin-3-ylamino)-5-(trifluoromethyl)pyrimidin-4-yl)-2,3-dihydro-[1,4]thiazino[2,3,4-hi]indole 1,1-dioxide

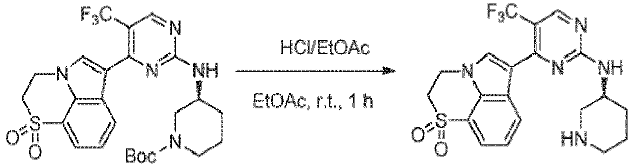

To a solution of (S)-tert-butyl 3-((4-(1,1-dioxido-2,3-dihydro-[1,4]thiazino[2,3,4-hi]indol-6-yl)-5-(trifiuotomethyl)pyrimidin-2-yl)amino)piperidine-1-carboxylate (0.2 g, 362.59 umol, 1 eq) in EtOAc (2 mL) was added HCl/EtOAc (4 M, 2.00 mL). The mixture was stirred at 20 °C for 1 h. The reaction mixture was filtered to collect the cake. It was washed with DCM again, and purified by prep-HPLC (HCl condition) to afford the title compound (0.034 g, 75.31 umol, 20.77% yield, 100% purity) as a white solid.

### Example 118. 4-(6-isopropylsulfonyl-1H-indol-3-yl)-N-[(3S)-3-piperidyl]-5-(trifluoromethyl) pyrimidin-2-amine (Compound 346).

### Step 1: 6-isopropylsulfanyl-1H-indole

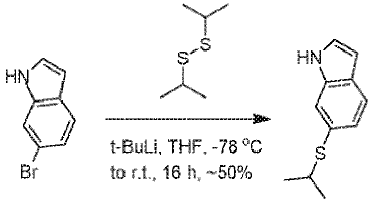

To a solution of 6-bromo-1H-indole (3 g, 15.30 mmol, 1 eq) in THF (100 mL) was added dropwise t-BuLi (1.3 M, 44.14 mL, 3.75 eq) at -78 °C. After addition, the mixture was stirred at this temperature for 1 h, and then 2-(isopropyldisulfanyl)propane (3.45 g, 22.95 mmol, 3.66 mL, 1.5 eq) was added dropwise at -78 °C. The resulting mixture was stirred at 20 °C for 15 h. The reaction mixture was quenched by addition NH₄Cl (300 mL) at 0 °C, and extracted with EtOAc (100 mLx3), dried over Na₂SO₄, filtered and concentrated under reduced pressure to give a residue. The residue was purified by column chromatography (SiO₂, PE: EtOAc =40:1 to 20:1) to give a residue (4.6 g). The residue was purified by reverse column (TFA condition). The pH of the eluent solution was adjusted to 8 with saturated aqueous NaHCO₃ and extracted with EtOAc (50 mLx3), dried over Na₂SO₄, filtered and concentrated under reduced pressure to afford the title compound (1.5 g) was obtained as a yellow solid.

### Step 2: 3-[2-chloro-5-(trifluoromethyl) pyrimidin-4-yl]-6-isopropylsulfanyl-1H-indole

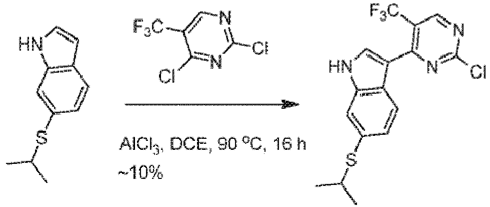

To a solution of 2, 4-dichloro-5-(trifluoromethyl) pyrimidine (816.67 mg, 3.76 mmol, 1.2 eq) in DCE (50 mL) was added dropwise AlCl₃ (543.70 mg, 4.08 mmol, 222.83 uL, 1.3 eq). After addition, the mixture was stirred for 30 min, and then 6-isopropylsulfanyl-1H-indole (0.6 g, 3.14 mmol, 1 eq) was added at 20 °C. The resulting mixture was stirred at 90 °C for 15.5 h. The reaction mixture was quenched with H₂O (150 mL) and filtered. The filtrate was extracted with EtOAc (60 mLx3), dried over Na₂SO₄, filtered and concentrated under reduced pressure to give a residue. The residue was purified by column chromatography (SiO₂, PE: EtOAc=1:0 to 30:1) to give a residue (800 mg). The residue was purified by prep-HPLC (TFA condition). The pH of the eluent solution was adjusted to 8 with saturated aqueous NaHCO₃ and extracted with EtOAc (60 mLx3), dried over Na₂SO₄, filtered and concentrated under reduced pressure to afford the title compound (340 mg) as a yellow solid.

### Step 3: 1-(benzenesulfonyl)-3-[2-chloro-5-(trifluoromethyl) pyrimidin-4-yl] -6-isopropyl sulfanyl -indole

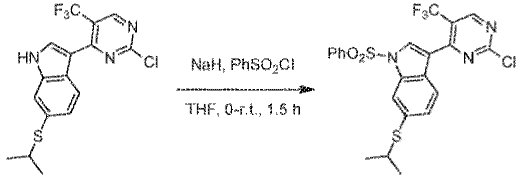

To a solution of 3-[2-chloro-5-(trifluoromethyl) pyrimidin-4-yl] -6-isopropylsulfanyl- 1H-indole (0.32 g, 860.66 umol, 1 eq) in THF (5 mL) was added batchwise NaH (51.64 mg, 1.29 mmol, 60% purity, 1.5 eq) at 0 °C. After addition, the mixture was stirred at this temperature for 30 min, and then benzenesulfonyl chloride (228.02 mg, 1.29 mmol, 165.23 uL, 1.5 eq) was added dropwise at 0 °C. The resulting mixture was stirred at 20°C for 1 h. The reaction mixture was quenched by addition H₂O (50 mL) at 0 °C, and extracted with EtOAc (30 mLx3), dried over Na₂SO₄, filtered and concentrated under reduced pressure to afford the title compound (0.49 g, crude) as a yellow oil. It was used to the next step directly without further purification.

### Step 4: 1-(benzenesulfonyl)-3-[2-chloro-5-(trifluoromethyl) pyrimidin-4-yl] - 6 - isopropylsulfonyl - indole

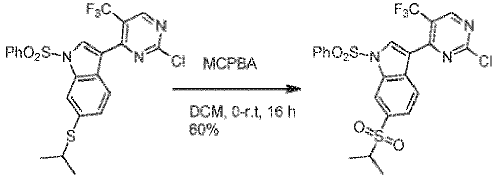

To a solution of 1-(benzenesulfonyl)-3-[2-chloro- 5-(trifluoromethyl)pyrimidin-4-yl] -6-isopropylsulfanyl-indole (0.44 g, 859.43 umol, 1 eq) in DCM (5 mL) was added MCPBA (383.87 mg, 1.89 mmol, 85% purity, 2.2 eq) at 0 °C. The mixture was stirred at 20 °C for 16 h. The reaction mixture was quenched by addition NaHCO₃ (30 mL) at 0 °C and stirred for 30 min, and extracted with EtOAc (20 mLx3), dried over Na₂SO₄, filtered and concentrated under reduced pressure to give a residue. The residue was washed with solvents (PE: EtOAc=5:1, 5 mL) and filtered to afford the title compound (0.3 g) as a yellow solid. (The reaction was combined with another reaction in 25 mg scale for purification).

### StepS: Tert-butyl (3S)-3-[[4-[1-(benzenesulfonyl) -6-isopropylsulfonyl-indol-3-yl]- 5-(trifluoromethyl) pyrimidin-2-yl] amino] piperidine-1-carboxylate

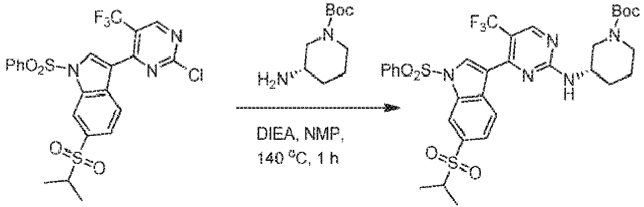

A mixture of 1-(benzenesulfonyl)-3-[2-chloro-5-(trifluoromethyl) pyrimidin-4-yl]-6-isopropylsulfonyl-indole (0.25 g, 459.59 umol, 1 eq), tert-butyl (3S)-3-aminopiperidine-1-carboxylate (110.45 mg, 551.51 umol, 1.2 eq), DIEA (178.19 mg, 1.38 mmol, 240.15 uL, 3 eq) in NMP (2 mL) was stirred at 140 °C for 1 h. The reaction mixture was diluted by addition H₂O (50 mL) at 0 °C, and extracted with EtOAc (30 mLx3), dried over Na₂SO₄, filtered and concentrated under reduced pressure to afford the title compound (0.6 g, crude) as a brown oil. It was used to next step directly without further purification.

### Step 6: Tert-butyl (3S)-3-[[4-(6-isopropylsulfonyl-1H-indol-3-yl) -5-(trifluoromethyl) pyrimidin-2-yl] amino] piperidine-1-carboxylate

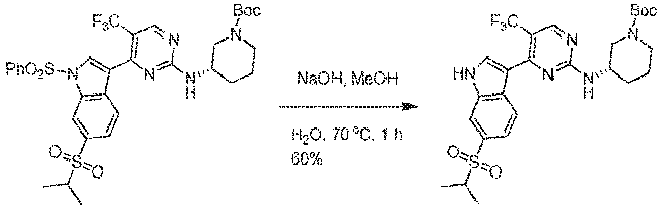

A mixture of tert-butyl (3S)-3-[[4-[1-(benzenesulfonyl)-6-isopropylsulfonyl-indol-3-yl]- 5-(trifluoromethyl)pyrimidin-2-yl]amino]piperidine-1-carboxylate (0.6 g, 847.72 umol, 1 eq) and NaOH (5 M in H₂O, 1.70 mL, 10 eq) in MeOH (6 mL) was stirred at 70 °C for 1 h. The reaction mixture was diluted by addition H₂O (200 mL) at 0 °C, and extracted with EtOAc (100 mLx3), dried over Na₂SO₄, filtered and concentrated under reduced pressure to afford the title compound (300 mg) as a yellow solid.

### Step 7: 4-(6-isopropylsulfonyl-1H-indol-3-yl)-N-[(3S)-3-piperidyl]-5-(trifluoromethyl) pyrimidin-2-amine

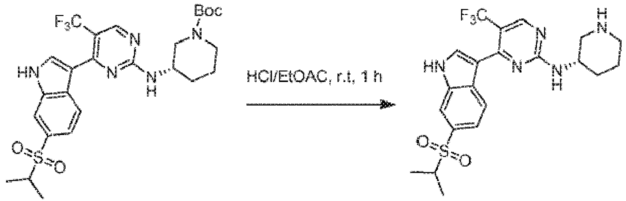

A mixture of tert-butyl (3S)-3-[[4-(6-isopropylsulfonyl-1H-indol-3-yl) -5-(trifluoromethyl) pyrimidin-2-yl] amino] piperidine-1-carboxylate (0.3 g, 528.52 umol, 1 eq) and HCl/EtOAc (4 M, 10 mL) was stirred at 20 °C for 1 h. The reaction mixture was filtered to collect cake. The cake was washed with n-hexane (5 mL), filtered and concentrated under reduced pressure to afford the title compound (158.8 mg, 311.95 umol, 59.02% yield, 99% purity, HCl) as a white solid.

### Example 119. 5-chloro-4-[7-(1, 1-dioxo-1, 4-thiazinan-4-yl) -1H-indol-3-yl]-N-[(3S)-3-piperidyl]pyrimidin-2-amine (Compound 357).

### Step 1: 1H-indole-6-sulfonyl chloride

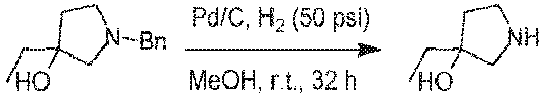

SO₂ was bubbled into THF (15 mL) at -78 °C for 30 minute to give an SO₂ solution (3.1 M in THF). To a solution of 6-bromo-1Hindole (5 g, 25.50 mmol, 1 eq) in (THF, 50 mL) was added NaH (1.02 g, 25.50 mmol, 60% purity, 1 eq) at 0 °C. After addition, the mixture was stirred at this temperature for 30 min. It was cooled to -78 °C and then t-BuLi (1.3 M, 39.24 mL, 2 eq) was added dropwise. The resulting mixture was stirred at -78 °C for 30 min, then SO₂ (3.1 M in THF, 15 mL) was added dropwise. The mixture was stirred at 15 °C for 12 h. To the resulting solution was added 75 mL of MBTE and 2 mL of AcOH (2.05 g, 34.18 mmol, 1.95 mL, 1.34 eq). It was stirred for 30 min at 0 °C, and filtered. The solid was then suspended in 75 mL of THF, cooled to 0 °C, and NCS (3.41 g, 25.50 mmol, 1.0 eq) was carefully added. The resulting suspension was stirred rapidly for 30 min. The reaction mixture was filtered and the filtrate was concentrated to afford the title compound (4 g, crude) as brown oil and used directly.

### Step 2: N, N-dimethyl-1H-indole-6-sulfonamide

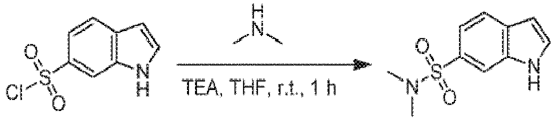

To a solution of 1H-indole-6-sulfonyl chloride (4.4 g, 20.40 mmol, 1 eq) in THF (20 mL) was added N-methylmethanamine (2M, 20.40 mL, 2 eq) at 15 °C. The mixture was stirred at 15 °C for 1 h. The residue was poured into water (30 mL). The aqueous phase was extracted with EtOAc (30 mLx3). The combined organic phase was washed with brine (50 mLx2), dried with anhydrous Na₂SO₄, filtered and concentrated in vacuum. The residue was purified by column (PE: EtOAc=5:1-4:1-3:1) to afford the title compound (2 g) as yellow solid.

### Step 3: 3-(2-chloro-5-(trifluoromethyl)pyrimidin-4-yl)-N,N-dimethyl-1H-indole-6-sulfonamide

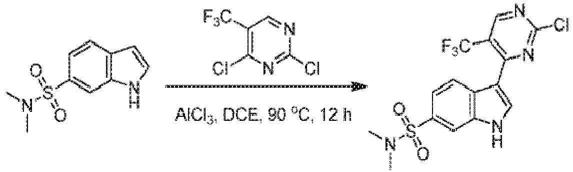

A solution of 2, 4-dichloro-5-(trifluoromethyl) pyrimidine (1.93 g, 8.92 mmol, 2 eq) and AlCl₃ (1.25 g, 9.36 mmol, 511.69 uL, 2.1 eq) in DCE (20 mL) was stirred at 90 °C for 0.5 h. Then N, N-dimethyl-1H-indole-6-sulfonamide (1 g, 4.46 mmol, 1 eq) was added to the above and the mixture was stirred at 90 °C for 11.5 h. The residue was poured into ice-water (150 mL) and stirred for 30 min, the mixture was filtered and the filter cake was collected. The filter cake was washed with MeOH (30 mL), filtered and the filter cake was collected afford the title compound (0.23 g, 82% purity, batch 1) as brown solid. The filtrate was concentrated in vacuum to afford the title compound (1 g crude, batch 2) as pink solid.

### Step 4: Tert-butyl (3S)-3-[[4-[6-(dimethylsulfamoyl)-1H-indol-3-yl]-5-(trifluoromethyl) pyrimidin-2-yl] amino] piperidine-1-carboxylate

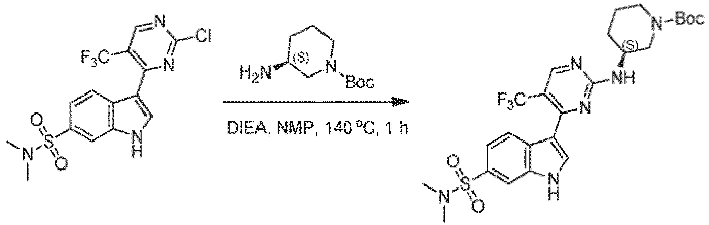

To a solution of 3-[2-chloro-5-(trifluoromethyl) pyrimidin-4-yl]-N, N-dimethyl-1H-indole-6-sulfonamide (0.1 g, 247.04 umol, 1eq), tert-butyl (3S)-3-aminopiperidine-1-carboxylate (59.37 mg, 296.45 umol, 1.2 eq) in NMP (2 mL) was added DIEA (159.64 mg, 1.24 mmol, 215.15 uL, 5 eq). The mixture was stirred at 140 °C for 1 h. The residue was poured into water (20 mL). The aqueous phase was extracted with EtOAc (30 mLx3). The combined Organic phase was washed with brine (30 mLx2), dried with anhydrous Na₂SO₄, filtered and concentrated in vacuum. The residue was purified by silica gel chromatography (PE: EtOAc=5:1-4:1-3:1) to afford the title compound (40 mg, 61.20 umol, 24.77% yield, 87% purity) as yellow oil.

### Step 5: N, N-dimethyl-3-[2-[[(3S)-3-piperidyl] amino]-5-(trifluoromethyl) pyrimidin-4-yl] -1H-indole-6-sulfonamide

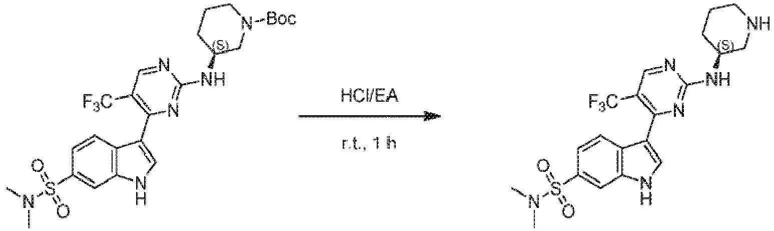

A solution of tert-butyl (3S)-3-[[4-[6-(dimethylsulfamoyl)-1H-indol-3-yl]-5-(trifluoromethyl) pyrimidin-2-yl] amino] piperidine-1-carboxylate (40 mg, 70.35 umol, 1 eq) in HCl/EtOAc (5 mL) was stirred at 15 °C for 1 h. The mixture was concentrated in vacuum. The residue was purified by prep-HPLC (FA condition) to afford the title compound (20.85 mg, 38.61umol, 54.88% yield, 95.27% purity, FA) as white solid.

### Example 120. 1-[3-[2-[[(3S)-3-piperidyl] amino]-5-(trifluoromethyl) pyrimidin-4-yl]-1H-indol-6-yl]ethanone (Compound 359).

### Step 1: Tert-butyl (3S)-3-[[4-[1-(benzenesulfonyl)-6-(1-ethoxyvinyl) indol-3-yl]-5-(trifluoromethyl) pyrimidin-2-yl] amino] piperidine-1-carboxylate

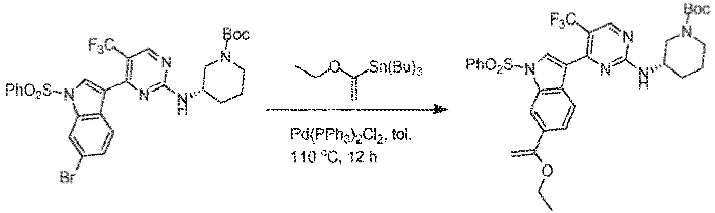

To a solution of tert-butyl (3S)-3-[[4-[1-(benzenesulfonyl)-6-bromo-indol-3-yl]- 5-(trifluoromethyl) pyrimidin-2-yl] amino] piperidine-1-carboxylate (0.5 g, 734.71 umol, 1 eq) and tributyl (1-ethoxyvinyl) stannane (398.01 mg, 1.10 mmol, 371.98 uL, 1.5 eq) in toluene (10 mL) was added Pd(PPh₃)₂Cl₂ (25.78 mg, 36.74 umol, 0.05 eq). The mixture was stirred at 110 °C under N₂ for 12 h. The residue was poured into water (20 mL). The aqueous phase was extracted with EtOAc (30 mLx3). The combined organic phase was dried with anhydrous Na₂SO₄, filtered and concentrated in vacuum. The residue was purified by MPLC (PE: EtOAc = 5:1-4:1-3:1) to afford the title compound (0.3 g) as yellow oil.

### Step 2: 1-[1-(benzenesulfonyl)-3-[2-1[(3S)-3-piperidyl] amino]-5-(trifluoromethyl) pyrimidin -4-yl] indol-6-yl] ethanone

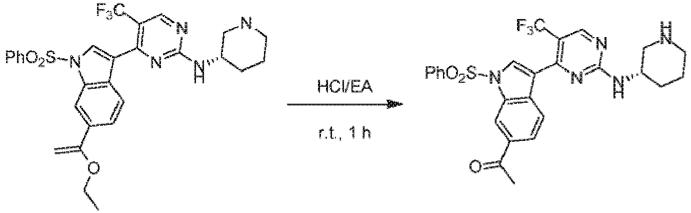

A solution of tert-butyl (3S)-3-[[4-[1-(benzenesulfonyl)-6-(1-ethoxyvinyl) indol-3-yl]-5-(trifluoromethyl) pyrimidin-2-yl] amino] piperidine-1-carboxylate (0.1 g, 148.87 umol, 1 eq) in HCl/EtOAc (5 mL) was stirred at 15 °C for 1 h. The mixture was concentrated in vacuum to afford the title compound (0.07 g, crude, HCl) as yellow oil.

### Step 3: 1-[3-[2-[[(3S)-3-piperidyl] amino]-5-(trifluoromethyl) pyrimidin-4-yl]-1H-indol-6-yl]ethanone

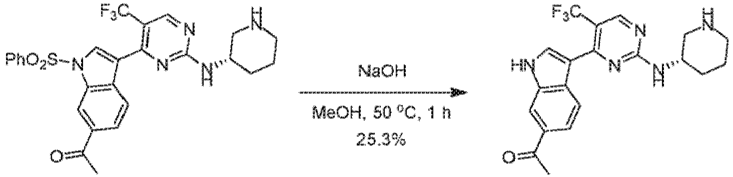

To a solution of 1-[1-(benzenesulfonyl)-3-[2-[[(3S)-3-piperidyl] amino]-5-(trifluoromethyl) pyrimidin-4-yl] indol-6-yl] ethanone (50 mg, 86.20 umol, 1 eq, HCl) in MeOH (3 mL) was added NaOH (5 M, 172.41 uL, 10 eq). The mixture was stirred at 50 °C for 1 h. The mixture was concentrated in vacuum. The residue was purified by prep-HPLC (FA) to afford the title compound (10.42 mg, 21.89 umol, 25.39%yield, 94.4% purity, FA) as white solid.

### Example 121. 5-chloro-4-[7-(1, 1-dioxo-1, 4-thiazinan-4-yl) -1H-indol-3-yl]-N-[(3S)-3-piperidyl] pyrimidin-2-amine (Compound 360).

### Step 1: 6-bromo-7-fluoro-1H-indole

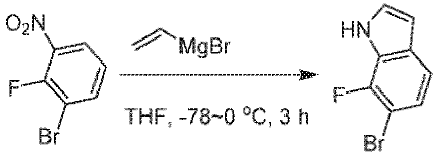

To a solution of 1-bromo-2-fluoro-3-nitro-benzene (10 g, 45.46 mmol, 1 eq) in THF (300 mL) was dropwise added bromo(vinyl)magnesium (1 M, 227.28 mL, 5 eq) at -78 °C over 1 h. The mixture was stirred at 0 °C for 2 h. The reaction mixture was poured into NH₄Cl (aq., 500 mL). The product was extracted with EtOAc (500 mLx3). The combined organic layers were washed with brine (500 mLx2), dried over Na₂SO₄, filtered and concentrated under reduced pressure to give a residue. The residue was purified by column chromatography (SiO₂, PE/EtOAc=20:1 to 5:1) to afford the title compound (2.8 g, crude) as brown solid.

### Step 2: 6-bromo-3-(2, 5-dichloropyrimidin-4-yl)-7-fluoro-1H-indole

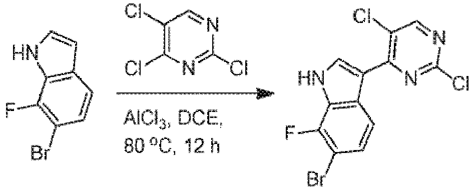

To a solution of 2, 4, 5-trichloropyrimidine (2.37 g, 12.90 mmol, 1.2 eq) in DCE (30 mL) was added AlCl₃ (1.86 g, 13.97 mmol, 763.42 uL, 1.3 eq) at 80 °C. The mixture was stirred at 80 °C for 0.5 h. Then 6-bromo-7-fluoro-1H-indole (2.3 g, 10.75 mmol, 1 eq) was added. The resulting mixture was stirred at 80 °C for another 12 h. The reaction mixture was quenched by addition H₂O 100 mL at 0 °C, and then filtered through the Celite. The cake was washed with EtOAc (100 mLx2). The filtrate was extracted with EtOAc (100 mLx3). The combined organic layers were washed with brine (100 mLx2), dried over Na₂SO₄, filtered and concentrated under reduced pressure to give a residue. The residue was purified by column chromatography (SiO₂, PE/EtOAc =50/1 to 511) to afford the title compound (2 g, crude) as brown solid (The reaction was combined with another reaction in 50 mg scale for purification).

### Step 3: 2-[[6-bromo-3-(2, 5-dichloropyrimidin-4-yl)-7-fluoro-indol-1-yl]methoxy] ethyl-trimethyl-silane

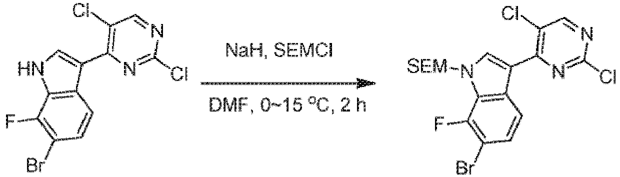

To a solution of 6-bromo-3-(2,5-dichloropyrimidin-4-yl)-7-fluoro-1H-indole (1.5 g, 4.16 mmol, 1 eq) in DMF (15 mL) was added NaH (249.29 mg, 6.23 mmol, 60% purity, 1.5 eq). The mixture was stirred at 0 °C for 0.5 h. Then the SEM-Cl (831.30 mg, 4.99 mmol, 882.49 uL, 1.2 eq) was added. The resulting mixture was stirred at 15 °C for another 3 h. The reaction mixture was quenched by addition H₂O 30 mL at 0 °C, and then extracted with EtOAc (30 mLx3). The combined organic layers were washed with brine (30 mLx2), dried over Na₂SO₄, filtered and concentrated under reduced pressure to give a residue. The residue was purified by column chromatography (SiO₂, PE: EtOAc =50:1 to 10:1) to afford the title compound (1.2 g, crude) as red solid.

### Step 4: Tert-butyl (3S)-3-[[4-[6-bromo-7-fluoro-1-(2-trimethylsilylethoxymethyl) indol-3-yl]-5- chloro-pyrimidin-2-yl] amino] piperidine-1-carboxylate

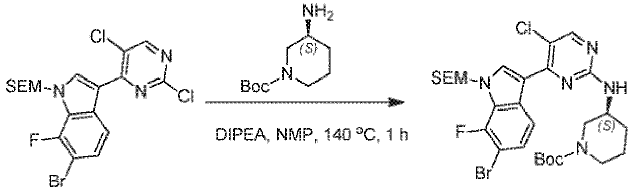

To a solution of 2-[[6-bromo-3-(2,5-dichloropyrimidin-4-yl)-7-fluoro-indol-1-yl]methoxy]ethyl-trimethyl-silane (1.2 g, 2.44 mmol, 1 eq) in NMP (10 mL) was added DIPEA (947.11 mg, 7.33 mmol, 1.28 mL, 3 eq) and tert-butyl (3S)-3-aminopiperidine-1-carboxylate (733.83 mg, 3.66 mmol, 1.5 eq). The mixture was stirred at 140 °C for 1 h. The reaction mixture was diluted with H₂O (20 mL) and extracted with EtOAc (20 mLx3). The combined organic layers were washed with brine (20 mLx2), dried over Na₂SO₄, filtered and concentrated under reduced pressure to give a residue. The residue was purified by column chromatography (SiO₂, PE: EtOAc =20:1 to 2:1) to afford the title compound (0.9 g, crude) as yellow solid.

### Step 5: Tert-butyl (3S)-3-[[5-chloro-4-[6-cyano-7-fluoro-1-(2-trimethylsilylethoxymethyl) indol-3-yl] pyrimidin-2-yl] amino] piperidine-1-carboxylate

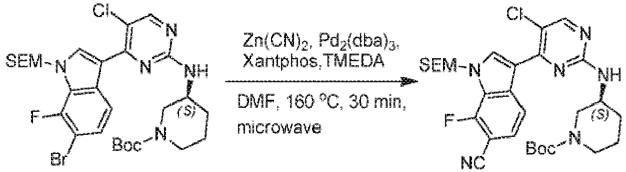

Tert-butyl (3S)-3-[[4-[6-bromo-7-fluoro-1-(2-trimethylsilylethoxymethyl)indol-3-yl]-5-chloro-pyrimidin-2-yl]amino]piperidine-1-carboxylate (0.3 g, 457.96 umol, 1 eq), Zn(CN)₂ (107.55 mg, 915.93 umol, 58.14 uL, 2 eq), Pd₂(dba)₃ (41.94 mg, 45.80 umol, 0.1 eq), Xantphos (79.50 mg, 137.39 umol, 0.3 eq) and N,N,N',N'-tetramethylethane-1,2-diamine (138.37 mg, 1.19 mmol, 179.70 uL, 2.6 eq) were taken up into a microwave tube in DMF (0.5 mL). The sealed tube was heated at 160 °C for 30 min under microwave. The combined mixture was poured into H₂O (20 mL), and extracted with EtOAc (20 mLx3). The combined organic layers were washed with brine (20 mLx2), dried over Na₂SO₄, filtered and concentrated under reduced pressure to give a residue. The residue was purified by column chromatography (SiO₂, PE: EtOAc =20:1 to 2:1) to afford the title compound (640 mg, crude) as a white solid (The reaction was combined with another reaction in 220 mg scale for purification).

### Step 6: Tert-butyl (3S)-3-[[4-[6-cyano-7-fluoro-1-(2-trimethylsilylethoxymethyl) indol-3-yl]-5- vinyl-pyrimidin-2-yl] amino] piperidine-1-carboxylate

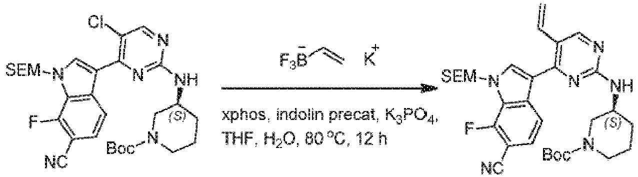

To a solution of tert-butyl (3S)-3-[[5-chloro-4-[6-cyano-7-fluoro-1-(2-trimethylsilylethoxymethyl)indol-3-yl]pyrimidin-2-yl]amino]piperidine-1-carboxylale (0.54 g, 898.22 umol, 1 eq) in THF (1 mL) and H₂O (0.2 mL) was added potassium trifluoro(vinyl)boranuide (601.58 mg, 4.49 mmol, 5 eq), [2-(2-aminoethyl)phenyl]-chloro-palladium;dicyclohexyl-[2-(2,4,6-triisopropylphenyl)phenyl]phosphane (66.36 mg, 89.82 umol, 0.1 eq) and K₃PO₄ (381.33 mg, 1.80 mmol, 2 eq). The mixture was stirred under N₂ at 80 °C for 12 h. The reaction mixture was poured into H₂O (10 mL), and extracted with EtOAc (10 mLx3). The combined organic layers were washed with brine (10 mLx2), dried over Na₂SO₄, filtered and concentrated under reduced pressure to give a residue. The residue was purified by column chromatography (SiO₂, PE: EtOAc =100: 1 to 5:1) to afford the title compound (200 mg, crude product) as yellow solid. (The reaction was combined with another reaction in 100 mg scale for purification).

### Step 7: Tert-butyl (3S)-3-[[4-[6-cyano-7-fluoro-1-(2-trimethylsilylethoxymethyl) indol-3-yl]-5- ethyl-pyrimidin-2-yl] amino] piperidine-1-carboxylate

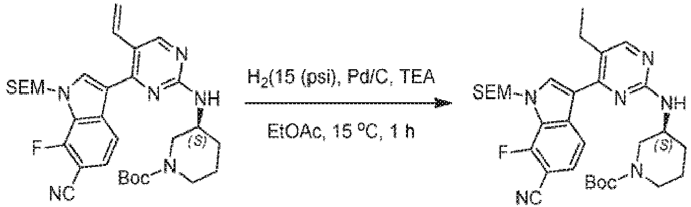

To a solution of tert-butyl (3S)-3-[[4-[6-cyano-7-fluoro-1-(2-trimethylsilylethoxymethyl) indol-3-yl]-5-yinyl-pyrimidin-2-yl]amino]piperidine-1-carboxylate (0.2 g, 337.39 umol, 1 eq) in MeOH (5 mL) was added Pd/C (0.1 g, 10% purity) and TEA (68.28 mg, 674.79 umol, 93.92 uL, 2 eq) under N₂. The suspension was degassed under vacuum and purged with H₂ several times. The mixture was stirred under H₂ (15 psi) at 15 °C for 1 h. The reaction mixture was filtered and the filter was concentrated to afford the title compound (0.15 g, crude) as yellow oil, which was used into the next step without further purification.

### Step 8: Tert-butyl (3S)-3-[[4-(6-cyano-7-fluoro-1H-indol-3-yl)-5-ethyl-pyrimidin-2-yl] amino] piperidine-1-carboxylate

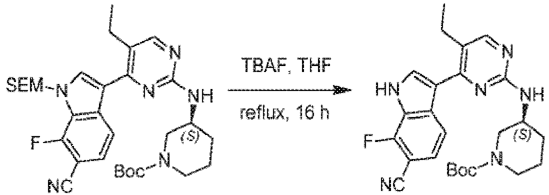

To a solution of tert-butyl (3S)-3-[[4-[6-cyano-7-fluoro-1-(2-trimethylsilylethoxymethyl) indol-3-yl]-5-ethyl-pyrimidin-2-yl] amino] piperidine-1-carboxylate (150 mg, 252.19 umol, 1 eq) in THF (3 mL) was added TBAF (659.38 mg, 2.52 mmol, 10 eq). The mixture was stirred at 80 °C for 16 h. The reaction mixture was concentrated under reduced pressure to give a residue. The residue was purified by column chromatography (SiO₂, PE: EtOAc=2:1 to 1:1) to afford the title compound (40 mg, 86.11 umol, 34.14% yield) as a white solid.

### Step 9: 3-15-ethyl-2-[[(3S)-3-piperidyl] amino] pyrimidin-4-yl]-7-fluoro-1H-indole-6-carbonitrile

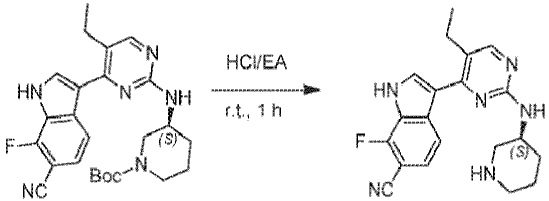

To a solution of tert-butyl (3S)-3-[[4-(6-cyano-7-fluoro-1H-indol-3-yl)-5-ethyl-pyrimidin-2-yl] amino] piperidine-1-carboxylate(0.05 g, 107.63 umol, 1 eq) in EtOAc (2 mL) was added HCl/EtOAc (4 M, 5.00 mL, 185.81 eq). The mixture was stirred at 20°C for 1 h and concentrated under reduced pressure to give a residue. The residue was purified by prep-HPLC (FA condition) to afford the title compound (6.9 mg, FA) was obtained as a white solid. (The reaction was combined with another reaction in 20 mg scale for purification).

### Example 122. 5-chloro-4-[7-(1,1-dioao-1,4-thazinan-4-yl)-1R-indol-3-yl]-N-[(3S)-3-piperidyl]pyrimidin-2-amine (Compound 361).

### Step 1: 3-(2-chloro-5-iodo-pyrimidin-4-yl)-1H-indole-6-carbonitrite

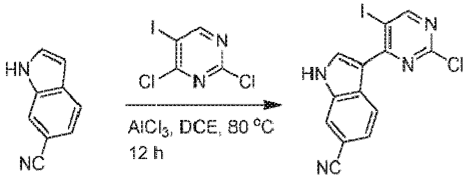

To a solution of 2, 4-dichloro-5-iodo-pyrimidine (4.35 g, 15.83 mmol, 1.5 eq) in DCE (30 mL) was added batchwise AlCl₃ (2.11 g, 15.83 mmol, 864.94 uL, 1.5 eq). After addition, the mixture was stirred at 80 °C for 0.5 h, and then 1H-indole-6-carbonitrile (1.5 g, 10.55 mmol, 1 eq) was added. The resulting mixture was stirred at 80 °C for 11.5 h. It was quenched by addition water 300 mL, then the solid was formed and filtered to collect the cake. The residue was purified by re-crystallization from MeOH (30 mL) to afford the title compound (4.3 g) as a yellow solid. (The reaction was combined with another reaction in 1 g scale for purification).

### Step 2: 3-(2-chloro-5-iodo-pyrimidin-4-yl)-1-(2-trimethylsilyleihoxymethyl)indole-6-carbonitrile

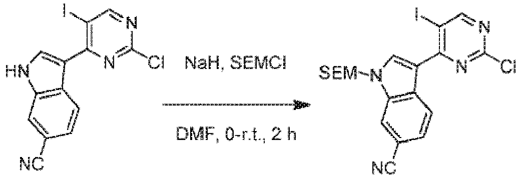

To a solution of 3-(2-chloro-5-iodo-pyrimidin-4-yl)-1H-indole-6-carbonitrile (3 g, 7.88 mmol, 1 eq) in DMF (6 mL) was added dropwise NaH (630.63 mg, 15.77 mmol, 60% purity, 2 eq) at 0°C. After addition, the mixture was stirred at this temperature for 0.5 h, and then 2-(chloromethoxy)ethyl-trimethyl-silane (1.97 g, 11.82 mmol, 2.09 mL, 1.5 eq) was added dropwise at 0 °C. The resulting mixture was stirred at 20°C for 1.5 h. It was quenched by addition water (100 mL), extracted with EtOAc (50 mLx3). The combined organic layers were washed with brine (200 mL), dried over Na₂SO₄, filtered and concentrated under reduced pressure to give a residue. The residue was purified by column chromatography (SiO₂, PE: EtOAc =20:1 to 3:1) to afford the title compound (0.78 g) as a yellow solid. (The reaction was combined with another reaction in 200 mg scale for purification).

### Step 3: Tert-butyl (3S)-3-[[4-[6-cyano-1-(2-trimethylsilylethoxymethyl)indol-3-yl]-5-iodo-pyrimidin-2-yl]amino]piperidine-carboxylate

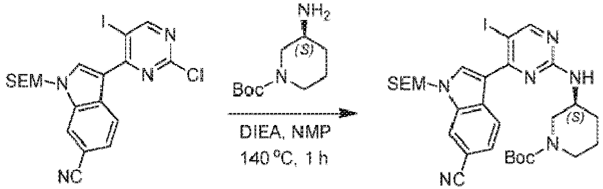

A mixture of 3-(2-chloro-5-iodo-pyrimidin-4-yl)-1 -(2-trimethylsilylethoxymethyl) Indole-6-carbonitrile (580 mg, 1.14 mmol, 1 eq), tert-butyl (3S)-3-aminopiperidine- 1-carboxylate (341.09 mg, 1.70 mmol, 1.5 eq), DIEA (733.70 mg, 5.68 mmol, 988.81 uL, 5 eq) in NMP (5 mL) was stirred at 140 °C for 1 h. The reaction mixture was poured into water 20 mL, and then extracted with EtOAc (10 mLx3). The combined organic layers were washed with brine (50 mL), dried over Na₂SO₄, filtered and concentrated under reduced pressure to give a residue. The residue was purified by column chromatography (SiO₂, PE: EtOAc=10:1 to 1:1) to afford the title compound (340 mg) as a brown solid.

### Step 4: Tert-butyl (3S)-3-[[5-acetylsulfanyl-4-[6-cyano-1-(2-trimethylsilylethoxymethyl) indol-3-yl] pyrimidin-2-yl] amino] piperidine-1-carboxylate

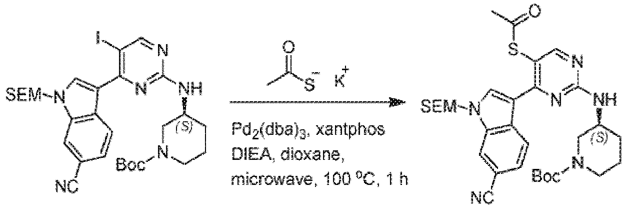

Tert-butyl (3S)-3-[[4-[6-cyano-1-(2-trimethylsilylethoxymethyl) indol-3-yl] -5- iodo-pyrimidin-2-yl] amino] piperidine-1- carboxylate (0.7 g, 1.04 mmol, 1 eq), Pd₂(dba)₃ (95.01 mg, 103.76 umol, 0.1 eq), Xantphos (120.07 mg, 207.52 umol, 0.2 eq), potassium ethanethioate (711.00 mg, 6.23 mmol, 6 eq) and DIEA (134.10 mg, 1.04 mmol, 180.72 uL, 1 eq) were taken up into a microwave tube in dioxane (10 mL). The sealed tube was heated at 100 °C for 1 h under microwave. The reaction mixture was poured into water 50 mL, and then extracted with EtOAc (20 mLx3). The combined organic layers were washed with brine (100 mL), dried over Na₂SO₄, filtered and concentrated under reduced pressure to give a residue. The residue was purified by column chromatography (SiO₂, PE: EtOAc=10:1 to 2:1) to afford the title compound (400 mg, 552.30 umol, 53.23% yield, 86% purity) as a brown solid.

### Step 5: Tert-butyl (3S)-3-[[4-[6-cyurso-1-(2-trimethylsilylethoxyrnethyl)indal-3-yl]-5-sulfanyl-pyrimidin-2-yl]amino]piperidine-1-carboxylate

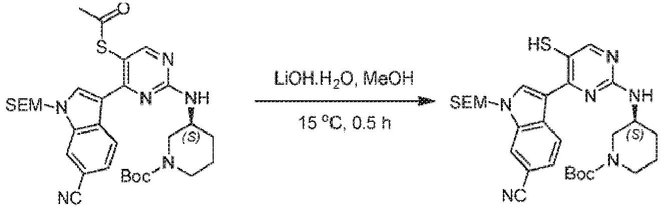

To a solution of tert-butyl (3S)-3-[[5-acetylsulfanyl- 4-[6-cyano-1-(2-trimethylsilylethoxymethyl) indol-3-yl]pyrimidin-2-yl]amino]piperidme-1-carboxylate (0.25 g, 401.38 umol, 1 eq) in MeOH (0.5 mL) was added LiOH.H₂O (5 M, 160.55 uL, 2eq). The mixture was stirred at 15°C for 0.5 h. The mixture was concentrated to afford the title compound (350 mg, crude) as a brown oil which was used in the next step directly.

### Step 6: Tert-butyl (3S)-3-[[4-[6-cyano-1-(2-trmethylsilylethoxymethyl) indol-3-yl]-5-(difluoromethylsulfanyl) pyrimidin-2-yl] amino] piperidine-1--carboxylate

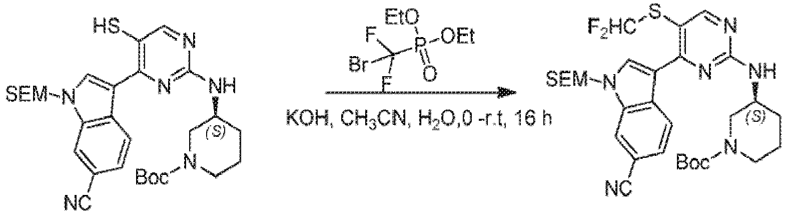

To a solution of tert-butyl (3S)-3-[[4-[6-cyano-1-(2-trimethylsilylethoxymethyl) indol-3-yl]-5-sulfanyl-pyrimidin-2-yl] amino] piperidine-1-carboxylate (0.3 g, 516.52 umol, 1 eq) in MeCN (0.5 mL) and H₂O (0.5 mL) was added KOH (579.63mg, 10.33 mmol, 20 eq) and 1-[[bromo(difluoro)methyl]-ethoxy-phosphoryl]oxyethane (137.91 mg, 516.52 umol, 1 eq) at 0°C. The mixture was stirred at 20 °C for 16 h. The mixture was concentrated and diluted with H₂O (20 mL), extracted with EtOAc (10 mLx3), dried over Na₂SO₄ and concentrated. The residue was purified by column chromatography (SiO₂, PE: EtOAc=5:1 to 1:1) to afford the title compound (0.1 g, 26% purity) as a white solid which was used in the next step without further purification.

### Step 7: 3-[5-(difluoromethylsulfanyl)-2-[[(3S)-3-piperidyl]amino]pyrimidin-4-yl]-1H-indole-6-carbonitrile

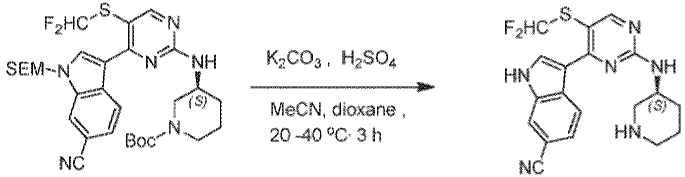

To a solution of tert-butyl (3S)-3-[[4-[6-cyano-1-(2-trimethylsilylethoxymethyl) indol-3-yl]-5-(difluoromethylsulfanyl) pyrimidin-2-yl] amino] piperidine-1-carboxylate (0.1 g, 158.52 umol, 1 eq) in dioxane (1 mL) was added H₂SO₄ (77.74 mg, 792.62 umol, 42.25 uL, 5 eq). The mixture was stirred at 40 °C for 2 h. The mixture was adjusted pH to 9 by Sat. NaOH, and extracted with DCM (10 mLx3), dried over Na₂SO₄, and concentrated. The residue was diluted with MeCN (1 mL), then the K₂CO₃ (43.82mg, 317.05 umol, 2 eq) was added. The solution was stirred at 20°C for 1 h. The mixture was concentrated. The residue was diluted with MeCN (10 mL) and filtered. The filtrate was concentrated. The residue was purified by prep-HPLC (FA condition) to afford the title compound (3.9 mg, 8.39umol, 5.29% yield, 96% purity, FA) as a white solid.

### Example 123. 3-[5-ethyl-2-[[(3S)-3-piperidyl] amino] pyrimidin-4-yl]-1H-indole-7-sulfonic add (Compound 362) and 3-[5-ethyl-2-[[(3S)-3-piperidyl] amino] pyrimidin-4-yl]-1H-indole-7-sulfinic acid (Compound 363).

### Step 1: a -bromo- 3-(2-chloro-5-iodo-pyrimidin-4-yl) -1H-indole

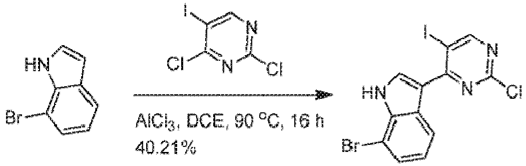

To a solution of 2, 4-dichloro-5-iodo-pyrimidine (3.37 g, 12.24 mmol, 1.2 eq) in DCE (40 mL) was added AlCl₃ (2.04 g, 15.30 mmol, 836.25 uL, 1.5 eq). After addition, the mixture was stirred at this temperature for 30 min, and then 7-bromo-1H-indole (2 g, 10.20 mmol, 1 eq) was added at 20 °C. The resulting mixture was stirred at 90 °C for 15.5 h. The reaction mixture was quenched with H₂O (150 mL) and filtered. The filtrate was extracted with EtOAc (60 mLx3), dried over Na₂SO₄, filtered and concentrated under reduced pressure to give a residue as a brown solid. The residue was washed with MeOH (50 mL) and filtered to afford the title compound (1.8 g, 4.10 mmol, 40.21% yield, 99% purity) as a brown solid.

### Step 2: 2-[[7-bromo-3-(2-chloro-5-iodo-pyrimidin-4-yl) indol-1-yl] methoxy] ethyl-trimethyl -silane

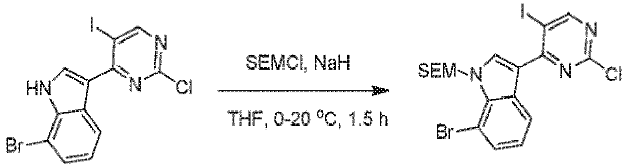

To a solution of 7-bromo-3-(2-chloro-5-iodo-pyrimidin-4-yl)-1H-indole (1.77 g, 4.07 mmol, 1 eq) in THF (20 mL) was added batchwise NaH (244.44 mg, 6.11 mmol, 60% purity, 1.5 eq) at 0 °C for 0.5 h. After addition, the mixture was stirred at this temperature for 30 min, and then 2-(chloromethoxy) ethyl-trimethyl-silane (1.02 g, 6.11 mmol, 1.08 mL, 1.5 eq) was added dropwise at 0 °C. The resulting mixture was stirred at 20 °C for 1 h. The reaction mixture was quenched with H₂O (100 mL) and filtered. The filtrate was extracted with EtOAc (60 mLx3), dried over Na₂SO₄, filtered and concentrated under reduced pressure to give a residue as a brown solid. The residue was purified by column chromatography (SiO₂, PE/EtOAc=20:1 to 10:1) to afford the title compound (1.65 g) as a brown oil.

### Step 3: Tert-butyl (3S)-3-[[4-[7-bromo-1-(2-trimethylsilylethoxymethyl) indol -3-yl]-5-iodo - pyrimidin- 2-yl] amino] piperidine-1-carboxylate

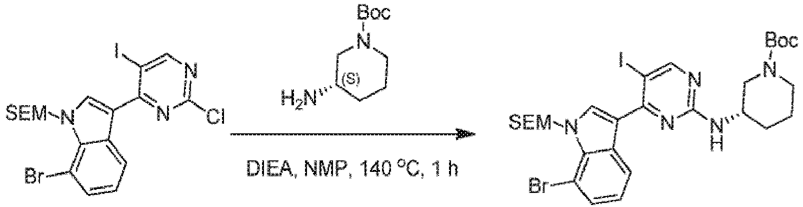

A mixture of 2-[[7-bromo-3-(2-chloro-5-iodo-pyrimidin-4-yl)indol-1-yl]methoxy]ethyl-trimethyl-silane (1.65 g, 2.92 mmol, 1 eq), tert-butyl (3S)-3-aminopiperidine-1-carboxylate (702.21 mg, 3.51 mmol, 1.2 eq), DIEA (1.13 g, 8.77 mmol, 1.53 mL, 3 eq) in NMP (16 mL) was stirred at 140 °C for 1 h. The reaction mixture was quenched with H₂O (150 mL) and filtered. The filtrate was extracted with EtOAc (60 mLx3), dried over Na₂SO₄, filtered and concentrated under reduced pressure to give a residue as a brown solid. The residue was purified by column chromatography (SiO₂, PE/EtOAc=10:1 to 3:1) to afford the title compound (1.3 g) as a yellow solid.

### Step 4: Tert-butyl (3S)-3-[[4-[7-bromo-1-(2-trimethylsilylethoxymethyl)indol-3-yl]-5-vinyl-pyrimidin-2-yl]amino piperidine-1-carboxylate

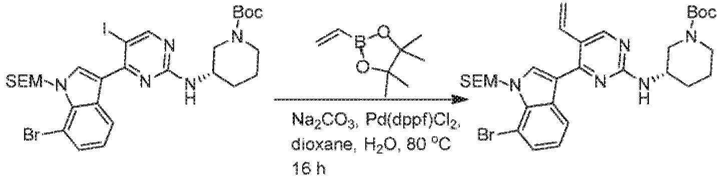

A mixture of tert-butyl (3S)-3-[[4-[7bromo-1-(2-trimethylsilylethoxymethyl)indol-3-yl] -5-iodo-pyrimidin-2-yl]amino]piperidine-1-carboxylate (1.2 g, 1.65 mmol, 1 eq), 4,4,5,5-tetramethyl-2-vinyl-1,3,2-dioxaborolane (253.68 mg, 1.65 mmol, 279.39 uL, 1 eq), Na₂CO₂ (349.16 mg, 3.29 mmol, 2 eq), Pd(dppf)Cl₂ (120.52 mg, 164.71 umol, 0.1 eq) in dioxane (20 mL) and H₂O (2 mL) was degassed and purged with N₂ for 3 times, and then the mixture was stirred at 80 °C for 16 h under N₂ atmosphere. The reaction mixture was quenched with H₂O (100 mL) and filtered. The filtrate was extracted with EtOAc (50 mLx3), dried over Na₂SO₄, filtered and concentrated under reduced pressure to give a residue. The residue was purified by column chromatography (SiO₂, PE/EtOAc=5:1 to 3:1) to afford the title compound (600 mg) as a yellow solid.

### Step 5: Tert-butyl (3S)-3-[[4-[7-bromo-1-(2-trimethylsilylethoxymethyl)indol-3-yl]-5-vinyl-pyrimidin-2-yl]amino]piperidine-1-carboxylate

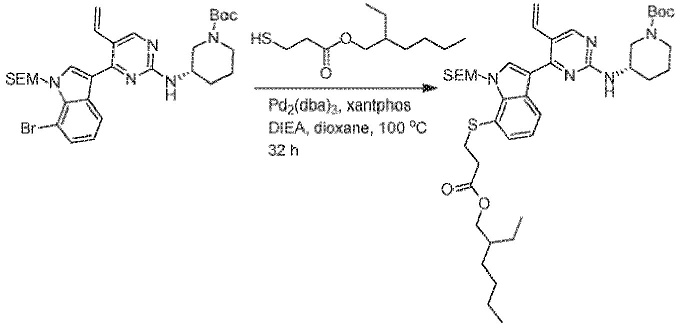

A mixture of tert-butyl (3S)-3-[[4-[7-bromo-1-(2-trimethylsilylethoxymethyl)indol-3-yl]- 5-vinyl-pyrimidin-2-yl]amino]piperidine-1-carboxylate (0.35 g, 556.73 umol, 1 eq), 2-ethylpentyl 3-sulfanylpropanoate (568.78 mg, 2.78 mmol, 5 eq), Pd₂(dba)₃ (50.98 mg, 55.67 umol, 0.1 eq), Xantphos (64.43 mg, 111.35 umol, 0.2 eq) and DIEA (143.90 mg, 1.11 mmol, 193.94 uL, 2 eq) in dioxane (3 mL) was degassed and purged with N₂ for 3 times, and then the mixture was stirred at 100 °C for 32 h under N₂ atmosphere. The reaction mixture was quenched with H₂O (20 mL) and filtered. The filtrate was extracted with EtOAc (10 mLx3), dried over Na₂SO₄, filtered and concentrated under reduced pressure to give a residue. The residue was purified by column chromatography (SiO₂, PE/EtOAc=10:1 to 5:1) to afford the title compound (0.14 g, 146.19 umol, 26.26% yield, 80% purity) as a yellow oil. (The reaction was combined with another reaction in 20 mg scale for purification).

### Step 6: Tert-butyl (3S)-3-[[4-[7-[3-(2-ethylhexoxy)-3-oxo-propyl] sulfanyl- 1- (2-trimethylsilylethoxymethyl) indol-3-yl]-5-vinyl-pyrimidin-2-yl] amino] piperidine-1- carboxylate

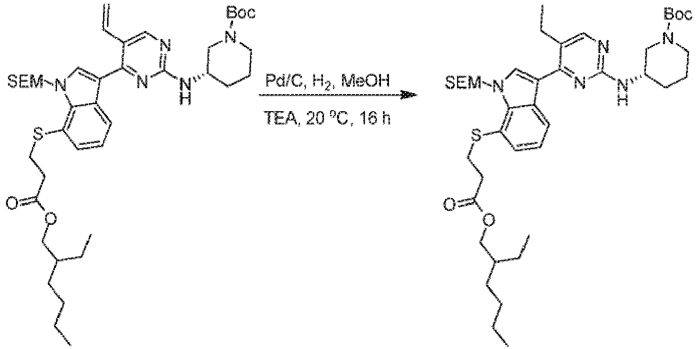

To a solution of tert-butyl (3S)-3-[[4-[7-[3-(2-ethylhexoxy)-3-oxo-propyl]sulfanyl-1-(2-trimethylsilylethoxymethyl)indol-3-yl]-5-vinyl-pyrimidin-2-yl]amino]piperidine-1-carboxylate (0.14 g, 182.74 umol, 1 eq) in MeOH (3 mL) was added Pd/C (10%,wet, 300 mg), TEA (36.98 mg, 365.48 umol, 50.87 uL, 2 eq) under N₂. The suspension was degassed under vacuum and purged with H₂ several times. The mixture was stirred under H₂ (15 psi) at 20 °C for 16 h. The reaction mixture was filtered and the filtrated was concentrated to afford the title compound (0.11 g, crude) as a yellow solid.

### Step 7: 3-[2-[[(3S)-1-tert-butoxycarbonyl-3-piperidyl]amino]-5-ethyl-pyrimidin-4-yl]-1-(2-trimethylsilylethoxymethyl)indole-7-sulfinic acid and 3-[2-[[(3S)-1-tert-butoxycarbonyl-3-piperidyljamino]-5-ethyl-pyrimidin-4-yl]-1-(2-trimethylsilylethoxymethyl)indole-7-sulfonic acid

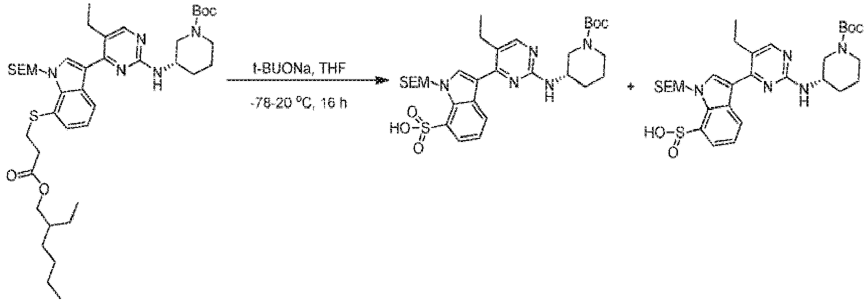

To a solution of tert-butyl (3S)-3-[[5-ethyl-4-[7-[3-(2-ethylhexoxy)-3 -oxo-propyl]sulfanyl-1-(2-trimethylsilylethoxymethyl)indol-3-yl]pyrimidin-2-yl]amino]piperidine-1-carboxylate (0.11 g, 143.20 umol, 1 eq) in THF (2 mL) was added t-BuONa (28.09 mg, 286.41 umol, 98% purity, 2 eq) at -78 °C. The mixture was stirred at 20 °C for 16 h. The reaction mixture was diluted with H₂O (20 mL) and extracted with EtOAc (10 mLx3), dried over Na₂SO₄, filtered and concentrated under reduced pressure to afford the title compound (0.1 g, crude) as a yellow solid. It was used next step directly without further purification.

### Step 8: 3-[5-ethyl-2-[[(3S)-3-piperidyl] amino] pyrimidin-4-yl]-1H-indole-7-sulfonic acid (compound 1) and 3-[5-ethyl-2-[[(3S)-3-piperidyl] amino] pyrimidin-4-yl]-1H-indole-7-sulfinic acid (compound 2)

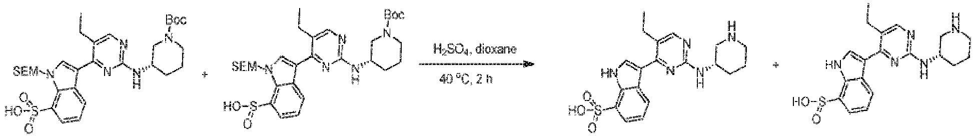

A mixture of 3-[2-[[(3S)-1-tert-butoxycarbonyl-3-piperidyl] amino]-5-ethyl-pyrimidin-4-yl]-I-(2-trimethylsilylethoxymethyl)indole-7-sulfinic acid; 3-[2-[[(3S)-1-tert-butoxycarbonyl-3-piperidyl]amino]-5-ethyl-pyrimidin-4-yl]-1-(2-trimethylsilylethoxymethyl)indole-7-sulfonic acid (100 mg, 158.26 umol, 1 eq) and H₂SO₄ (155.22 mg, 1.58 mmol, 84.36 uL, 10 eq) in dioxane (2 mL) was stirred at 40 °C for 2 h. The mixture was adjusted pH to 8 with Saturated aqueous NaOH and extracted with EtOAc (10 mLx3), the combined organic was washed with brine (30 mLx3), dried over Na₂SO₄, filtered and concentrated under reduced pressure to give a residue. The residue was purified by prep-HPLC (FA condition) to afford the title compound 1 (4.45 mg, FA) as a yellow solid and the title compound 2 (2.35 mg, FA) as a yellow solid.

### Example 124. N-[(3S)-1-methyl-3-piperidyl]-4-(6-methylsulfonyl-1H-indol-3-yl)-5-(trifluoromethyl)pyrimidin-2-amine (Compound 364).

### Step 1: Tert-butyl (3S)-3-[[4-(6-methylsulfonyl-1H-indol-3-yl)-5-(trifluoromethyl) pyrimidin-2-yl] amino] piperidine-1-carboxylate

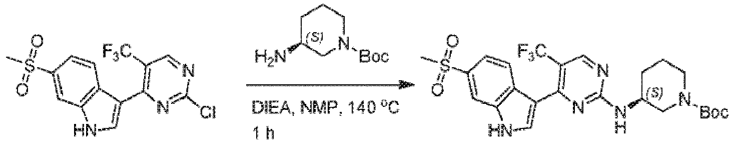

A solution of 3-[2-chloro-5-(trifluoromethyl)pyrimidin-4-yl]-6-methylsulfonyl-1H-indole (300 mg, 798.40 umol, 1 eq), tert butyl (3S)-3-aminopiperidine-1-carboxylate (159.90 mg, 798.40 umol, 1 eq), DIPEA (515.94 mg, 3.99 mmol, 695.33 uL, 5 eq) and NMP (4 mL) was heated to 140 °C for 1 h. The reaction mixture was poured into H₂O (20 mL). EtOAc (30 mLx3) was used to extract the product. The organic layer was washed by brine (30 mL), dried over Na₂SO₄, filtered and evaporated to afford the crude product The crude product was purified by MPLC (SiO₂, PE/EtOAc=3/1-1/1) to afford the title compound (160 mg, 247.60 umol, 31.01% yield, 83.5% purity) as light brown solid.

### Step 2: 4-(6-methylsulfonyl-1H-indol-3-yl)-N-[(3S)-3-piperidyl]-5-(trifluoromethyl)pyrimidin-2-amine

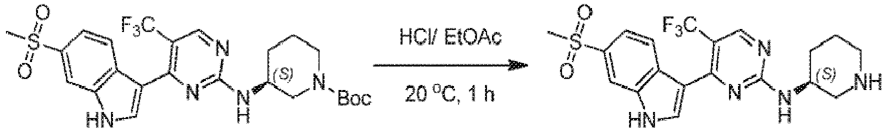

A solution of tert-butyl (3S)-3-[[4-(6-methylsulfonyl-1H-indol-3-yl)-5-(trifluoromethyl)pyrimidin-2-yl] amino] piperidine-1-carboxylate (160 mg, 296.53 umol, 1 eq) in HCl/EtOAc (15 mL) was stirred at 20 °C for 1 h. The reaction mixture was evaporated to afford a residue. Then saturated Na₂CO₃ solution (40 mL) was added and EtOAc (20 mLx2) was used to extract the product. The organic layer was washed by brine (30 mL), dried over Na₂SO₄, filtered and evaporated to afford the title compound (120 mg, 177.49 umol, 59.86% yield, 65% purity) as light yellow solid. It will be used directly in next step without any further purification.

### Step 3: N-[(3S)-1-methyl-3-piperidyl]-4-(6-methylsulfonyl-1H-indol-3-yl)-5-(trifluoromethyl)pyrimidin-2-amine

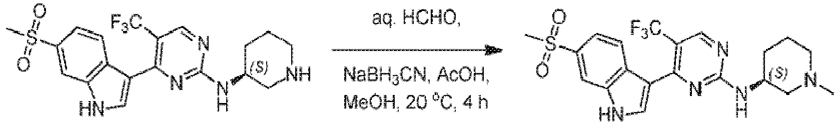

To a mixture of 4-(6-methylsulfonyl-1H-indol-3-yl)-N-[(3S)-3-piperidyl]-5-(trifluoromethyl)pyrimidin-2-amine (40 mg, 91.02 umol, 1 eq) and HCHO (7.39 mg, 91.02 umol, 6.78 uL, 1 eq) in MeOH (4 mL), was added NaBH₃CN (8.58 mg, 136.53 umol, 1.5 eq) and HOAc (8.20 mg, 136.53 umol, 7.81 uL, 1.5 eq). The mixture was stirred at 20 °C and stirred for 4 hr. The reaction solution was purified by acidic prep-HPLC (HCl condition) to afford the title compound (10.92 mg, 22.29 umol, 24.49% yield, 100% purity, HCl) as yellow solid.

### Example 125.4-[6-(fluoromethylsulfonyl)-1H-indol-3-yl]-N-[(3S)-3-piperidyl]-5-(trifluoromethyl)pyrimidin-2-amine (Compound 365).

### Step 1: Tert-butyl (3S)-3-[[4-[6-acetylsulfanyl-1-(benzenesulfonyl)indol-3-yl]-5-(trifluoromethyl)pyrimidin-2-yl]amino]piperidine-1-carboxylate

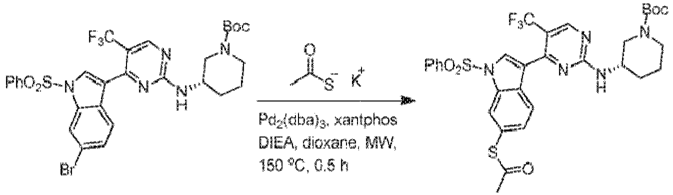

Tert-butyl (3S)-3-[[4-[1-(benzenesulfonyl)-6-bromo-indol-3-yl]-5-(trifluoromethyl)pyrimidin- 2-yl]amino]piperidine-1-carboxylate (250 mg, 367.36 umol, 1 eq), K⁺S(O)CH₃⁻ (62.93 mg, 551.04 umol, 1.5 eq), Pd₂(dba)₃ (33.64 mg, 36.74 umol, 0.1 eq), Xantphos (42.51 mg, 73.47 umol, 0.2 eq) and DIEA (94.95 mg, 734.72 umol, 127.97 uL, 2 eq) were taken up into a microwave tube in dioxane (10 mL). The sealed tube was heated at 150 °C for 0.5 h under microwave. The reaction mixture was diluted with water (100 mL) and extracted with EtOAc (50 mL x 5). The combined organic layers were washed with brine (50 mL x 2), dried over Na₂SO₄, filtered and concentrated under reduced pressure to give a residue. The residue was purified by column chromatography (SiO₂, PE/EtOAc = 1011 to 511) to afford the title compound (100 mg crude) as a light yellow solid.

### Step 2: Tert-butyl (3S)-3-[[4-[1-(benzenesulfonyl)-6-sulfanyl-indol-3-yl]-5-(trifluoromethyl) pyrimidin-2-yl]amino]piperidine-1-carboxylate

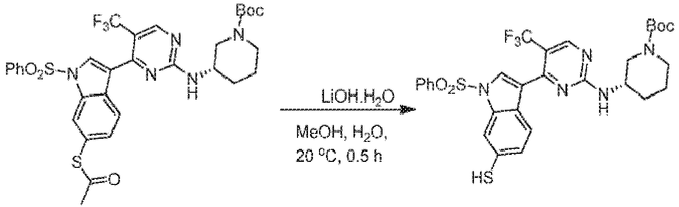

LiOH.H₂O (18.63 mg, 443.96 umol, 3 eq) was added to a solution of tert-butyl(3S)-3-[[4- [6-acetylsulfanyl-1-(benzenesulfonyl)indol-3-yl]-5-(trifluoromethyl)pyrimidin-2-yl]amino] *piperidine-1-carboxylate* (100 mg, 147.99 umol, 1 eq) in MeOH (2 mL) and water (0.5 mL). The mixture was stirred at 20 °C for 0.5 h under nitrogen. The reaction mixture was evaporated. The aqueous residue was neutralized with 0.5 M HCl to pH = 6. The mixture was diluted with H₂O (10 mL) and solid was formed. The mixture was filtered and the cake was collected to afford the title compound. (40 mg, 27.77 umol, 18.77% yield, 44% purity) as light yellow solid. It was used into the next step without further purification.

### Step 3: Tert-butyl (3S)-3-[[4-[1-(benzenesulfonyl)-6-(fluoromethylsulfanyl)indol-3-yl]-5-(trifluor omethyl)pyrimidin-2-yl]amino]piperidine-1-carboxylate

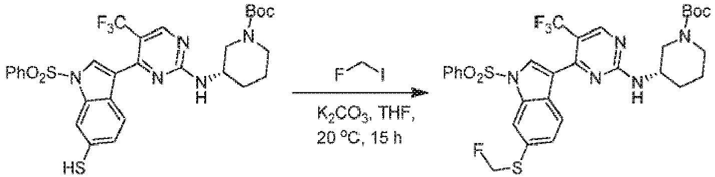

To a solution of tert-butyl (3S)-3-[[4-[1-(benzenesulfonyl)-6-sulfanyl-indol-3-yl]-5-(trifluoro methyl)pyrimidin-2-yl]amino]piperidine-1-carboxylate (40 mg, 63.12 umol, 1 eq) in THF (1.5 mL) was added K₂CO₃ (13.09 mg, 94.68 umol, 1.5 eq) and fluoro(iodo)methane (12.11 mg, 75.75 umol, 1.02 uL, 1.2 eq). The mixture was stirred at 20 °C for 15 h. The reaction mixture was diluted with H₂O (20 mL) and extracted with EtOAc (50 mL x 5). The combined organic layers were washed with brine (30 mL x 2), dried over Na₂SO₄, filtered and concentrated under reduced pressure to give a crude product (100 mg). The residue (90 mg) was further purified by column chromatography (SiO₂, PE/EtOAc = 511 to 1/1) to afford the title compound (30 mg crude) as yellow solid. (Note: Combined purification with another reaction in 5 mg scale)

### Step 4: Tert-butyl (3S)-3-[[4-[1-(benzenesulfonyl)-6-(fluoromethylsulfonyl)indol-3-yl]-5-(trifluoromethyl)pyrimidin-2-yl]amino]piperidine-1-carboxylate

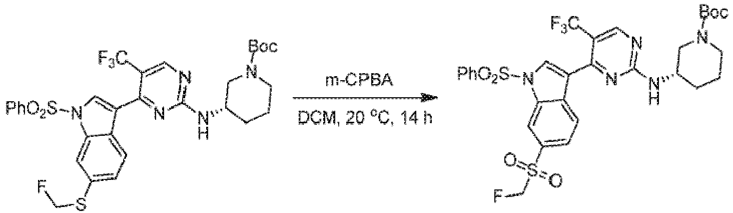

To a solution of tert-butyl (3S)-3-[[4-[1-(benzenesulfonyl)-6-(fluoromethylsulfanyl)indol -3-yl]-5-(trifluoromethyl)pyrimidin-2-yl]amino]piperidine-1-carboxylate (30 mg, 45.06 umol, 1 eq) in DCM (2 mL) was added m-CPBA (20.13 mg, 99.14 umol, 85% purity, 2.2 eq). The mixture was stirred at 20 °C for 14 hr. The reaction mixture was quenched by addition a mixture solution of saturated NaHCO₃ (10 mL) and Na₂SO₃ (10 mL) and extracted with EtOAc (20 mL x 3). The combined organic layers were washed with brine (20 mL x 2), dried over Na₂SO₄, filtered and concentrated under reduced pressure to afford the title compound (25 mg, crude) as yellow solid. It was used into the next step without further purification.

### Step 5: tert-butyl (3S)-3-[[4-[6-(fluoromethylsulfonyl)-1H-indol-3-yl]-5-(trifluoromethyl) pyrimidin-2-yl)amino)piperidine-1-carboxylate

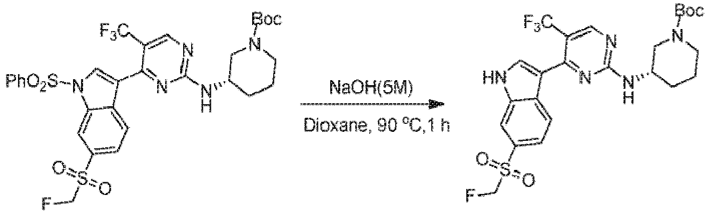

To a solution of tert-butyl (3S)-3-[[4-[1-(benzenesulfonyl)-6-(fluoromethylsulfonyl)indol3-yl]-5-(trifluoromethyl)pyrimidin-2-yl]amino]piperidine-1-carboxylate (25 mg, 35.83 umol, 1 eq) in dioxane (2 mL) was added NaOH (5 M, 71.66 uL, 10 eq). The mixture was stirred at 90 °C for 1 h. The residue was diluted with H₂O (20 mL) and extracted with EtOAc (20 mL x 3). The combined organic layers were washed with brine (20 mL x 2), dried over Na₂SO₄, filtered and concentrated under reduced pressure to give a residue. The residue was purified by prep-TLC (SiO₂, PE: EtOAc = 1:1) to afford the title compound (10 mg, 17.94 umol, 50.06% yield) as a white solid.

### Step 6: 4-[6-(fluoromethylsulfonyl)-1H-indol-3-yl]-N-[(3S)-3-piperidyl]-5-(trifluoromethyl)pyrimidin-2-amine

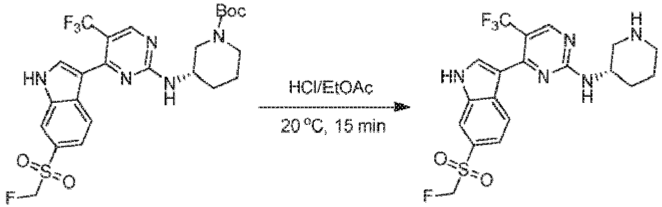

A solution of tert-butyl (3S)-3-[[4-[6-{fluoromethylsulfonyl)-1H-indol-3-yl]-5-(trifluoro methyl)pyrimidin-2-yl]amino]piperidine-1-carboxylate (10 mg, 17.94 umol, 1 eq) in HCl/EtOAc (5 mL) was stirred at 20°C for 0.25 h. the mixture was concentrated to give the residue. The residue was purified by acidic prep-HPLC (HCl condition) to afford the title compound (1.2 mg, 2.43 umol, 13.55% yield, HCl) as a white solid.

### Example 126. 3-methyl-1-[[3-[2-[[(3S)-3-piperidyl]amino]-5-(trifluoroinethyl)pyrimidln-4-yl]-1H-indol-6-yl]sulfonyl]pyrrolidin-3-ol (Compound 196).

### Step 1: 1H-indole-6-sulfonyl chloride

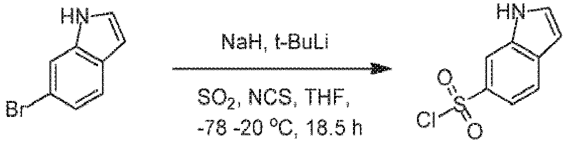

SO₂ was bubbled into THF (15 mL) at -78 °C for 30 minutes to give a solution (3.1 M, 19 % purity) for use. To a solution of 6-bromo-1H-indole (5 g, 25.50 mmol, 1 eq) in THF (50 mL) was added batchwise NaH (1.02 g, 25.50 mmol, 60% purity, 1 eq) at 0 °C. After addition, the mixture was stirred at this temperature for 30 min, the mixture was cooled to -78 °C and then t-BuLi (1.3 M, 39.24 mL, 2 eq) was added dropwise. The resulting mixture was stirred at -78 °C for 30 min, then SO₂ solution (1.63 g, 25.50 mmol, 1 eq) was added dropwise, the mixture was stirred at 20 °C for 16 h. (To the resulting solid was added 75 mL of MBTE and 2 mL of glacial acetic acid. The mixture was stirred for 30 min at 0 °C, and filtered). The solids were then suspended in 75 mLof THF, chilled to 0 °C, and NCS (3.41 g, 25.50 mmol, 1 eq) was carefully added. The resulting suspension was stirred rapidly for 30 min. The reaction was stopped. The reaction mixture filtered and concentrated under reduced pressure to afford the title compound (3 g) as a black oil. The residue was used to the next step directly.

### Step 2: 1-(1H-indol-6-ylsulfonyl)-3-methyl-pyrrolidin-3-ol

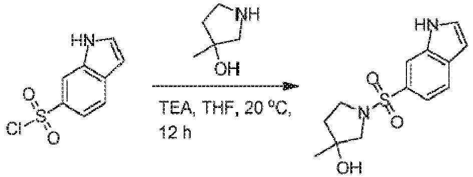

To a solution of 1H-indole-6-sulfonyl chloride (3 g, 13.91 mmol, 1 eq) in THF (25 mL) was added 3-methylpyrrolidin-3-ol (2.30 g, 16.69 mmol, 1.2 eq, HCl) and TEA (4.22 g, 41.73 mmol, 5.81 mL, 3 eq). The suspension was degassed and purged with N₂ for 3 times. The mixture was stirred at 20 °C for 12 h. The reaction mixture was diluted with water 300 mL and extracted with EtOAc (50 mLx3). The combined organic layers were washed with brine (50 mLx3), dried over Na₂SO₄, filtered and concentrated under reduced pressure to give a residue. The residue was purified by column chromatography (SiO₂, PE: EtOAc=2:1) to afford the title compound (1.2 g, 3.68 mmol, 26.46% yield, 86% purity) as a brown oil.

### Step 3: 1-[(3-bromo-1H-indol-6-yl)sulfonyl]-3-methyl-pyrrolidin-3-ol

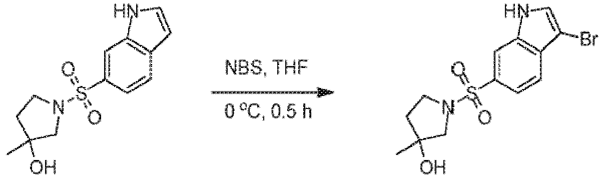

To a solution of 1-(1H-indol-6-ylsulfonyl)-3-methyl-pyrrolidin-3-ol (1.2 g, 4.28 mmol, 1 eq) in DCM (10 mL) was added NBS (761.86 mg, 4.28 mmol, 1 eq) in small portion at 0°C. The mixture was stirred at 0 °C for 0.5 h. The reaction mixture was diluted with water 30 mL and extracted with DCM (20 mLx3). The combined organic layers were washed with brine (20 mLx3), dried over Na₂SO₄, filtered and concentrated under reduced pressure to afford the title compound (800 mg) as a brown solid.

### Step 4: 1-[3-bromo-1-(2-trimethylsilylethoxymethyl)indol-6-yl]sulfonyl-3-methylpyrrolidin-3-ol

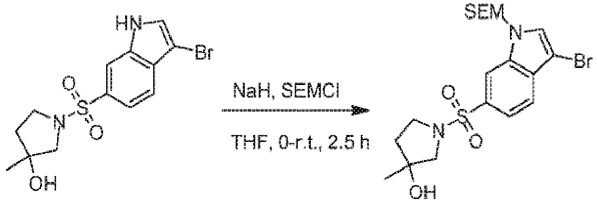

To a solution of 1-[(3-bromo-1H-indol-6-yl)sulfonyl]-3-methyl-pyrrolidin-3-ol (1.1 g, 3.06 mmol, 1 eq) in THF (5 mL) was added NaH (183.70 mg, 4.59 mmol, 60% purity, 1.5 eq) for 0.5 h at 0°C, then SEM-Cl (765.76 mg, 4.59 mmol, 812.90 uL, 1.5 eq) was added to the solution at 0°C. The mixture was stirred at 20°C for 2.5 h and then diluted with water 50 mL and extracted with EtOAc (20 mLx3). The combined organic layers were washed with brine (20 mLx3), dried over Na₂SO₄, filtered and concentrated under reduced pressure to give a residue. The residue was purified by column chromatography (SiO₂, DCM: MeOH=20:1) to afford the title compound (800 mg) as a white solid. (The reaction was combined with another reaction in 100 mg scale for purification).

### Step 5: 3-methyl-1-[3-(4,4,5,5-tetramethyl-1,3,2-dioxaborolan-2-yl)-1-(2-trimethylsilylethoxymethyl)indol-6-yl]sulfonylpyrrolidin-3-ol

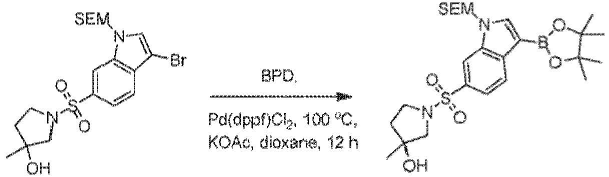

To a solution of 1-[3-bromo-1-(2-trimethylsilylethoxymethyl)indol-6-yl]sulfonyl-3-methylpyrrolidin-3-ol (0.76 g, 1.55 mmol, 1 eq) in dioxane (10 mL) was added4,4,5,5-tetramethyl-2-(4,4,5,5-tetramethyl-1,3,2-dioxaborolan-2-yl)-1,3,2-dioxaborolane (591.40 mg, 2.33 mmol, 1.5 eq), KOAc (304.75 mg, 3.11 mmol, 2 eq) and Pd(dppf)Cl₂ (113.61 mg, 155.26 umol, 0.1 eq). The suspension was degassed and purged with N₂ for 3 times. The mixture was stirred at 100 °C for 12 h. The reaction mixture was diluted with water 100 mL and extracted with EtOAc (50 mLx3). The combined organic layers were washed with brine (50 mLx3), dried over Na₂SO₄, filtered and concentrated under reduced pressure to afford the title compound (770 mg) as brown oil.

### Step 6: tert-butyl (3S)-3-[[4-[6-(3-hydroxy-3-methyl -pyrrolidin-1-yl)sulfonyl-1-(2-trimethylsilylethoxymethyl)indol-3-yl]-5-(trifluoromethyl) pyrimidin-2-yl]amino]piperidine-1-carboxylate

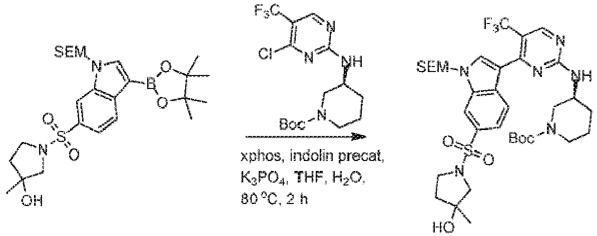

To a solution of 3-methyl-1-[3-(4,4,5,5-tetramethyl-1,3,2-dioxaborolan-2-yl)-1-(2 - trimethylsilylethoxymethyl)indol-6-yl]sulfonyl-pyrrolidin-3-ol (0.77 g, 861.04 umol, 1 eq) in THF (10 mL) and H₂O (2 mL) was added tert-butyl (3S)-3-[[4-chloro-5-(trifluoromethyl) pyrimidin-2-yl] amino] piperidine-1-carboxylate (327.88 mg, 861.04 umol, 1 eq), ditert-butyl(cyclopentyl)phosphane;dichloropalladium;iron (56.12 mg, 86.10 umol, 0.1 eq) and K₃PO₄ (365.54 mg, 1.72 mmol, 2 eq). The suspension was degassed and purged with N₂ for 3 times. The mixture was stirred at 80 °C for 2 h. The reaction mixture was diluted with water 100 mL and extracted with EtOAc (50 mLx3). The combined organic layers were washed with brine (50 mL x 3), dried over Na₂SO₄, filtered and concentrated under reduced pressure to give a residue. The residue was purified by column chromatography (SiO₂, PE: EtOAc=2:1) to afford the title compound (300 mg) as a colorless oil.

### Step 7: tert-butyl (3S)-3-[[4-[6-(3-hydroxy-3-methyl-pyrrolidin-1-yl)sulfonyl-1H-indol-3-yl] -5-(trifluoromethyl)pyrimidin-2-yl]amino]piperidine-1-carboxylate

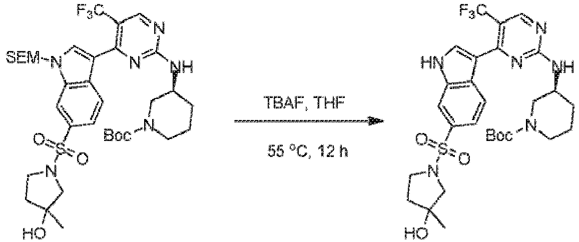

To a solution of tert-butyl (3S)-3-[[4-[6-(3-hydroxy-3-methyl-pyrrolidin-1-yl)sulfonyl-1-(2-trimethylsilylethoxymethyl)indol-3-yl]-5-(trifluoromethyl)pyrimidin-2-yl]amino]piperidine-1-carboxylate (0.27 g, 357.65 umol, 1 eq) in THF (2 mL) was added TBAF (1 M, 2.15 mL, 6 eq). The mixture was stirred at 55 °C for 12 h. The reaction mixture was diluted with water (20 mLx3) and extracted with EtOAc (10 mLx3). The combined organic layers were washed with brine (10 mLx3), dried over Na₂SO₄, filtered and concentrated under reduced pressure to give a residue. The residue was purified by Cleanert PWCX-SPE for two times to afford the title compound (80 mg) as a brown solid.

### Step 8: 3-methyl-1-[[3-[2-[[(3S)-3-piperidyl]amino]-5-(trifluoromethyl)pyrimidin-4-yl]-1H-indol-6-yl]sulfonyl]pyrrolidin-3-ol

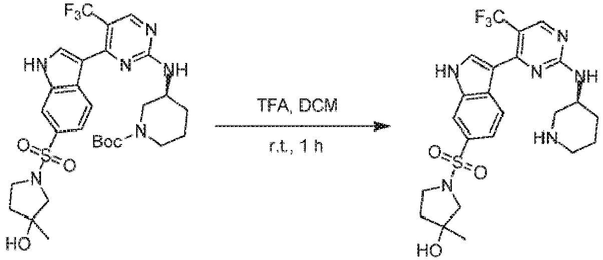

To a solution of tert-butyl (3S)-3-[[4-[6-(3-hydroxy-3-methyl-pyrrolidin-1-yl) sulfonyl- 1H-indol-3-yl]-5-(trifluoromethyl) pyrimidin-2-yl] amino] piperidine-1-carboxylate (0.07 g, 112.06 umol, 1 eq) in DCM (3 mL) was added TFA (5.39 g, 47.27 mmol, 3.50 mL, 421.84 eq). The mixture was stirred at 20°C for 1 h. The reaction mixture was concentrated under reduced pressure to give a residue. The residue was purified by prep-HPLC (HCl condition) to afford the title compound (0.01491 g, 26.02 umol, 23.22% yield, 97.9% purity, HCl) as a white solid.

### Example 127. 7-methylsulfonyl-3-[2-[[(3S)-3-piperidyl] amino]-5-propyl-pyrimidin-4-yl] -1H-indole -6-carbonitrile (Compound 367).

### Step 1: Tert-butyl (3S)-3-[[5-allyl-4-[6-cyano-7-methylsulfonyl-1-(2-trimethylsilylethoxymethyl) indol-3-yl] pyrimidin-2-yl] amino] piperidine-1-carboxylate

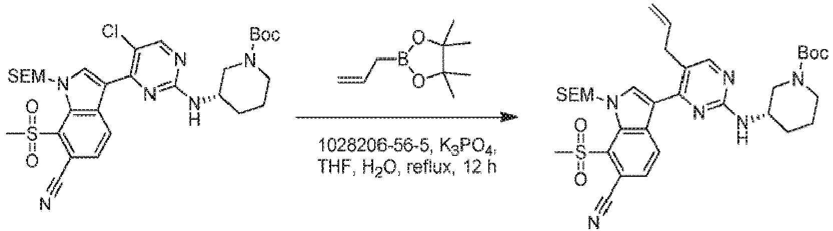

To a solution of tert-butyl (3S)-3-[[5-chloro-4-[6-cyano-7-methylsulfonyl-1- (2-trimethylsilylethoxymethyl)indol-3-yl]pyrimidin-2-yl]amino]piperidine-1-carboxylate (0.3 g, 453.66 umol, 1.00 eq) in THF (3 mL) and H₂O (0.6 mL) was added 2-allyl-4,4,5,5-tetramethyl-1,3,2-dioxaborolane (381.17 mg, 2.27 mmol, 5 eq), K₃PO₄ (192.60 mg, 907.32 umol, 2.00 eq) and [2-(2-aminoethyl)phenyl]-chloro- palladium;dicyclohexyl-[2-(2,4,6-triisopropylphenyl)phenyl]phosphane (33.51 mg, 45.37 umol, 0.1 eq) under N₂. The mixture was stirred under N₂ at 80 °C for 12 h. The reaction mixture was concentrated under reduced pressure to give a residue. The residue was diluted with H₂O 30 mL and extracted with EtOAc (30 mLx2). The combined organic layers were washed with brine (30 mLx2), dried over Na₂SO₄, filtered and concentrated under reduced pressure to give a residue. The mixture was purified by column chromatography (SiO₂, PE/EtOAc=10/1 to 3:1) to afford the title compound (150 mg, crude) as yellow solid.

### Step 2: Tert-butyl (3S)-3-[[4-[6-cyano-7-methylsulfonyl-1-(2-trimethylsilylethoxymethyl) indol-3-yl]-5-propyl-pyrimidin-2-yl] amino] piperidine-1-carboxylate

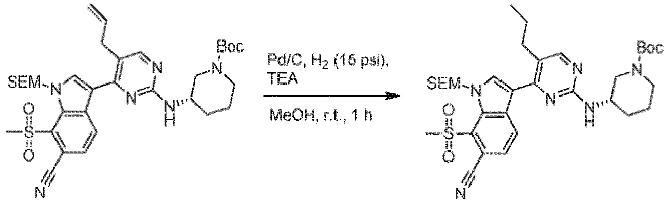

To a solution of tert-butyl (3S)-3-[[5-allyl-4-[6-cyano-7-methylsulfonyl-1-(2-trimethylsilylethoxymethyl)indol-3-yl]pyrimidin-2-yl]amino]piperidine-1-carboxylate (0.15 g, 224.92 umol, 1 eq) in MeOH (5 mL) was added Pd/C (0.1 g, 10%) and TEA (45.52 mg, 449.84 umol, 62.61 uL, 2 eq) under N₂. The suspension was degassed under vacuum and purged with H₂ several times. The mixture was stirred under H₂ (15 psi) at 20 °C for 1 h. The reaction mixture was filtered and the filter was concentrated to afford the title compound (0.1 g, crude) as yellow solid, which was used into the next step without further purification.

### Step 3: 7-methylsulfonyl-3-[2-[[(3S)-3-piperidyl]amino]-5-propyl-pyrimidin-4-yl]-1H-indole-6-carbonitrile

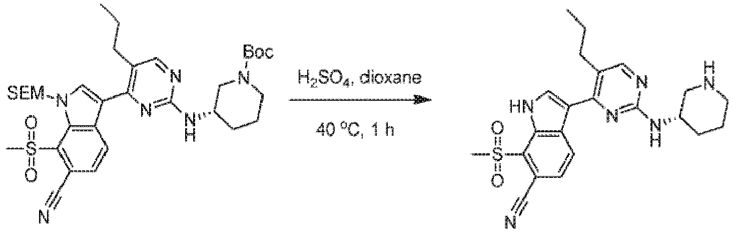

To a solution of tert-butyl (3S)-3-[[4-[6-cyano-7-methylsulfonyl-1-(2-trimethylsilylethoxymethyl)indol-3-yl]-5-propyl-pyrimidin-2-yl]amino]piperidine-1-caiboxylate (0.08 g, 119.60 umol, 1 eq) in dioxane (0.5 mL) was added H₂SO₄ (119.69 mg, 1.20 mmol, 65.05 uL, 98% purity, 10 eq). The mixture was stirred at 40 °C for 1 h. The reaction mixture was concentrated under reduced pressure to give a residue. The residue was purified by prep-HPLC (HCl condition) to afford the title compound (24.7 mg, 50.34 umol, 96.8% purity, HCl) as a white solid.

### Example 12$. 3-[2-[[(3S, 5S)-5-fluoro-3-piperidyl]amino]-5-(trifluoromethyl) pyrimidin-4-yl]-1H-Indole-6-carbonitrile (Compound 368).

### Step 1: 3-[2-chloro-5-(trifluoromethyl) pyrimidin-4-yl]-1H-indole-6-carbonitrile

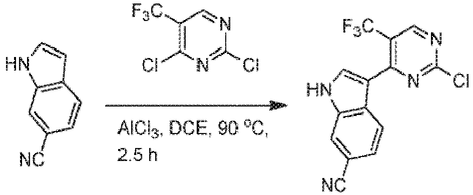

To a solution of 2, 4-dichloro-5-(trifluoromethyl) pyrimidine (30.53 g, 140.69 mmol, 2 eq) in DCE (250 mL) was added AlCl₃(19.70 g, 147.72 mmol, 8.07 mL, 2.1 eq). The mixture was stirred at 90 °C for 0.5 h. Then 1H-indole-6-carbonitrile (10 g, 70.34 mmol, 1 eq) was added and the resulting solution was stirred at 90°C for 2 h. The residue was dissolved in MeOH (100 mL) and poured into ice-water (1000 mL) and stirred for 5 min. The solids were formed and filtered to collect the cake. The cake was washed with MeOH (500 mL), filtered and concentrated in vacuum. The residue was purified by silica gel chromatography (PE: EtOAc = 1:1) to afford the title compound (10 g, 26.34 mmol, 37.45% yield, 85% purity) as yellow solid.

### Step 2: 3-[2-chloro-5-(trifluoromethyl) pyrimidin-4-yl]-1-(2-trimethylsilylethoxymethyl)indole-6-cαrbonitrile

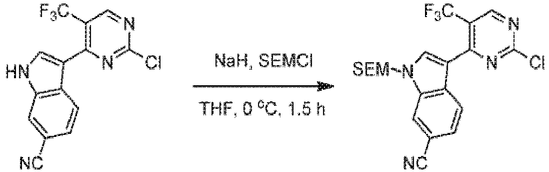

To a solution of 3-[2-chloro-5-(trifluoromethyl) pyrimidin-4-yl]-1H-indole-6-carbonitrile (1 g, 3.10 mmol, 1 eq) in THF (20 mL) was added NaH (185.93 mg, 4.65 mmol, 60% purity, 1.5 eq) at 0°C. The mixture was stirred at 0°C for 0.5 h. Then SEMCI (775.03 mg, 4.65 mmol, 822.75 uL, 1.5 eq) was added and the mixture was stirred at 0°C for 1 h. The residue was poured into ice-water (50 mL). The aqueous phase was extracted with EtOAc (50 mLx3). The combined organic phase was washed with brine (50 mLx2), dried with anhydrous Na₂SO₄, filtered and concentrated in vacuum. The residue was purified by silica gel chromatography PE: EtOAc = 20:1-10:1-8:1 to afford the title compound (0.7 g, 1.53 mmol, 49.37% yield, 99% purity) as white solid.

### Step 3: Allyl (3S, 5R)-3-[[4-[6-cyano-1-(2-trimethylsilylethoxymethyl) indol-3-yl]-5-(trifluoromethyl) pyrimidin-2-yl] amino]-5-hydroxy- piperidine-1-carboxylate

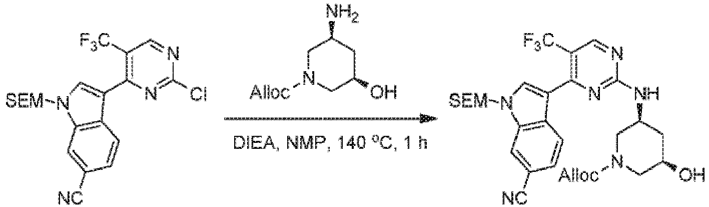

To a solution of 3-[2-chloro-5-(trifluoromethyl) pyrimidin-4-yl]-1-(2-trimethylsilylethoxymethyl) indole-6-carbonitrile (0.2 g,441.57 umol, 1 eq) and allyl (3S,5R)-3-amino-5-hydroxy-piperidine-1-carboxylate (0.2 g, 636.42 umol, 1.44 eq, TFA) in NMP (5 mL) was added DIEA (285.34 mg, 2.21 mmol, 384.56 uL, 5 eq). The mixture was stirred at 140 °C for 1 h. The residue was poured into water (20 mL). The aqueous phase was extracted with EtOAc (30 mLx3). The combined organic phase was washed with brine (50 mLx2), dried with anhydrous Na₂SO₄, filtered and concentrated in vacuum. The residue was purified by silica gel chromatography (PE: EtOAc = 5:1-3:1-1:1) to afford the title compound (0.15 g, 243.23 umol, 55.08% yield) as yellow solid.

### Step 4: Allyl(3S,5S)-3-[[4-[6-cyano-1-(2-trimethylsilylethoxymethyl)indol-3-yl]-5-(trifluoromethyl)pyrimidin-2-yl]amino]-5-fluoropiperidine-1-carboxylate

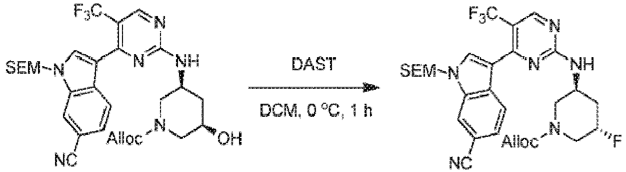

To a solution of allyl (3S, 5R)-3-[[4-[6-cyano-I-(2-trimethylsilylethoxymethyl) indol-3-yl]-5-(1,1-difluoroethyl)pyrimidin-2-yl]amino]-5-hydroxy-piperidine-1-carboxylate (80 mg, 130.56 umol, 1 eq) in DCM (3 mL) was added DAST (31.57 mg, 195.84 umol, 25.87 uL, 1.5 eq) at 0 °C. The mixture was stirred at 0 °C for 1 h. The residue was poured into ice-water (5 mL). The aqueous phase was extracted with EtOAc (10 mLx3). The combined organic phase was dried with anhydrous Na₂SO₄, filtered and concentrated in vacuum. The residue was purified by silica gel chromatography (PE: EtOAc = 10:1-5:1-3:1) to afford the title compound (30 mg, 37.82 umol, 28.97% yield, 78% purity) as yellow solid.

### Step 5: 3-[2-[[(3S, 5S)-5-fluoro-3-piperidyl]amino]-5-(trifluoromethyl)pyrimidin-4-yl]-1-(2-trimethylsilylethoxymethyl)indole-6- carbonitrile

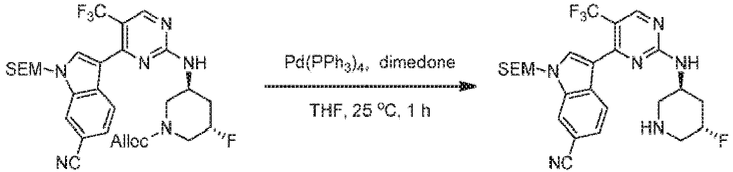

To a solution of allyl (3S, 5S)-3-[[4-[6-cyano-1-(2-trimethylsilylethoxymethyl) indol-3-yl]-5-(trifluoromethyl) pyrimidin-2-yl] amino]-5-fluoro-piperidine-1-carboxylate (55 mg, 88.90 umol, 1 eq) in THF (5 mL) was added Pd (PPh₃)₄ (10.27 mg, 8.89 umol, 0.1 eq) and DIMEDONE (37.38 mg, 266.69 umol, 3 eq). The mixture was stirred at 25 °C for 1 h. The residue was poured into HCl (10 mL, 1M). The aqueous phase was extracted with EtOAc (15 mLx3). The combined organic phase was washed with sat. NaHCO₃ (10 mL). Then the combined organic phase was dried with anhydrous Na₂SO₄, filtered and concentrated in vacuum to afford the title compound (50 mg, crude) as yellow oil and used directly in next step.

### Step 6: 3-[2-[[(3S, 5S)-5-fluoro-3-piperidyl]amino]-5-(trifluoromethyl)pyrimidin-4-yl]-1H-Indole-6-carbonitrile

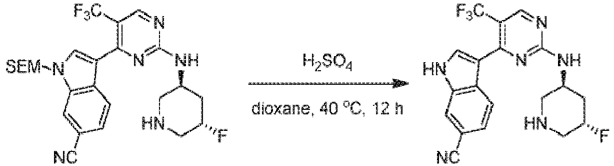

To a solution of 3-[2-[[(3S, 5S)-5-fluoro-3-piperidyl]amino]-5-(trifluoromethyl)pyrimidin-4-yl]-1-(2-trimethylsilylethoxymethyl)indole-6-carbonitrile (50 mg, 93.52 umol, 1 eq) in dioxane (5 mL) was added H₂SO₄ (183.45 mg, 1.87 mmol, 99.70 uL, 20 eq). The mixture was stirred at 40 °C for 12 h. The mixture was extracted with EtOAc (10 mLx3). The combined organic phase was discarded. The aqueous phase was adjusted to pH to 9 with sat. NaOH and extracted with EtOAc (10 mLx3). The residue was purified by prep-HPLC (FA) to afford the title compound (6.8 mg, 14.65 umol, 97% purity, FA) as white solid.

### Example 129. N-[(3S)-1-ethyl-5,5-dimethyl-3-piperidyl]-4-(6-methylsulfonyl-1H-indol-3-yl)-5-(trifluoromethyl)pyrimidin-2-amine (Compound 369).

### Step 1: N-[(3S)-1-ethyl-5,5-dimethyl-3-piperidyl]-4-(6-methylsulfonyl-1H-indol-3-yl)-5-(trifluoromethyl)pyrimidin-2-amine

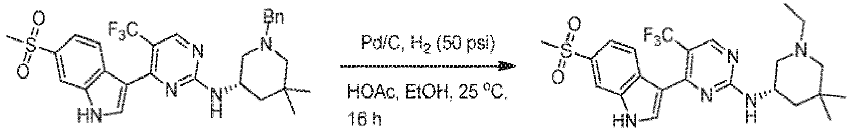

To a solution of N-[(3S)-1-benzyl-5,5-dimethyl-3-piperidyl]-4-(6-methylsulfonyl-1H-indol-3-yl)-5-(trifluoromethyl)pyrimidin-2-amine (40 mg, 71.73 umol, 1 eq) in EtOH (15 mL) was added Pd/C (15 mg, 10% purity) and HOAc (8.62 mg, 143.46 umol, 8.21 uL, 2 eq) under N₂. The suspension was degassed under vacuum and purged with H₂ several times. The mixture was stirred under H₂ (50 psi) at 25 °C for 16 hr. The reaction mixture was filtered through celite and the filtrate was evaporated to afford the crude product. The crude product was purified by acidic prep-HPLC purification (HCl condition) to afford the title compound (10.88 mg, 18.85 umol, 26.28% yield, 92.18% purity, HCl) as yellow solid.

### Example 130.3-[2-[[(3S, 5R)-5-hydroxy-3-piperidyl]amino]-5-(trifluoromethyl)pyrimidin-4-yl]-1H-indole-6-carbonitrile (Compound 370).

### Step 1: Tert-butyl N-[(5R)-1-benzyl-5-hydroxy-3-piperidyl] carbamate

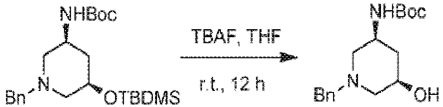

To a solution of tert-butyl N-[(5R)-1-benzyl-5-[tert-butyl(dimethyl)silyl]oxy-3-piperidyl]carbamate (0.5 g, 1.19 mmol, 1 eq) in THF (5 mL) was added TBAF (1 M, 1.43 mL, 1.2 eq). The mixture was stirred at 20 °C for 12 h. The reaction mixture was concentrated under reduced pressure to give a residue. The residue was purified by column chromatography (SiO₂, PE/EtOAc=10/1-2/1) to afford the title compound (0.3 g, 979.12 umol, 82.38% yield) as a white solid.

### Step 2: 4-[3-(2,5-dichloropyrimidin -4-yl)-1H-indol-7-yl]thiomorpholine

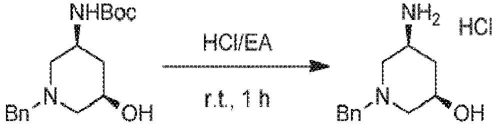

To a solution of tert-butyl N-[(SR)-1-benzyl-5-hydroxy-3-piperidyl] carbamate (0.3 g, 979.11. umol, 1 eq) in EtOAc (1 mL) was added HCl/EtOAc (4 M, 5 mL, 20.43 eq). The mixture was stirred at 20 °C for 1 h. The reaction mixture was concentrated under reduced pressure to afford the title compound (0.25 g, crude, HCl) as white solid, which was used into the next step without further purification.

### Step 3: 3-[2-[[(3S, 5R)-1-benzyl-5-hydroxy-3-piperidyl]amino]-5-(trifluoromelhyl)pyrimidin-4-yl]-1H-indole-6-carbonitrile

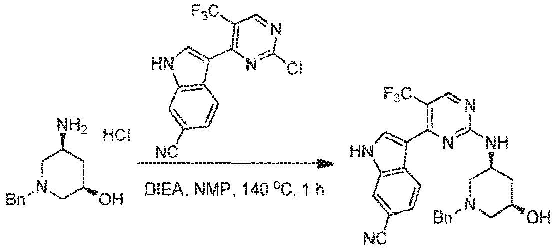

To a solution of 3-[2-chloro-5-(trifluoromethyl) pyrimidin-4-yl]-1H-indole-6-carbonitrile (73.11 mg, 226.58 umol, 1.1 eq) in NMP (0.5 mL) was added DIPEA (53.24 mg, 411.96 umol, 71.75 uL, 2 eq) and (3R, 5S)-5-amino-1-benzyl-piperidin-3-ol (0.05 g, 205.98 umol, 1 eq, HCl). The mixture was stirred at 140 °C for 1 h. The reaction mixture was poured into H₂O 10 mL, while brown solid formed. The solid was filtered and washed with H₂O (1 mLx2). The solid was dissolved into EtOAc (10 mL), and washed with brine (10 mLx2), dried over Na₂SO₄, filtered and concentrated under reduced pressure to give a residue. The residue was purified by column chromatography (SiO₂, PE /EtOAc=5/1 to 1/1) to afford the title compound (0.05 g, 69.04 umol, 33.52% yield, 68% purity) as yellow solid.

### Step 4: 3-[2-[[(3S, 5R)-5-hydroxy-3-piperidyl]amino]-5-(trifluoromelhyl)pyrimidin-4-yl]-1H-indole-6-carbonitrile

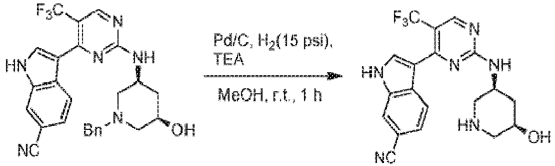

To a solution of 3-[2-[[(3S, 5R)-l-benzyl-5-hydroxy-3-piperidyl]amino]-5-(trifluoromethyl)pyrimidin-4-yl]-1H-indole-6-carbonitrile (50.00 mg, 101.52 umol, 1 eq) in MeOH (5 mL) was added Pd/C (0.05 g, 10% purity) and TEA (20.55 mg, 203.05 umol, 28.26 uL, 2 eq) under N₂. The suspension was degassed under vacuum and purged with H₂ several times. The mixture was stirred under H₂ (15 psi) at 20°C for 1 h. The reaction mixture was filtered and the filter was concentrated. The residue was purified by prep-HPLC (HCl condition) to afford the title compound (1.6 mg, 3.57 umol, 3.52% yield, 97.9% purity, HCl) as yellow solid.

### Example 131. 4-[6-(difluoromethylsulfanyl)-1H-indol-3-yl]-N-[(3S)-3-piperidyl]-5-(trifluoromethyl) pyrimidin-2-amine (Compound 374).

### Step 1: 4-[6-(difluoromethylsulfanyl)-1H-indol-3-yl]-N-[(3S)-3-piperidyl]-5-(trifluoromethyl) pyrimidin-2-amine

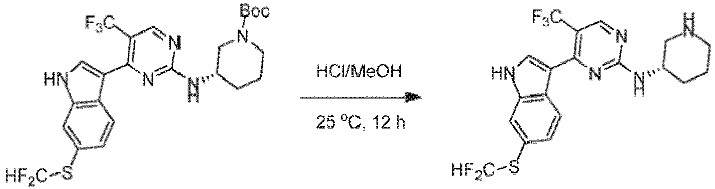

To solution of tert-butyl (3S)-3-[[4-[6-(difluoromethylsulfanyl)-1H-indol-3-yl]-5 -(trifluoromethyl)pyrimidin-2-yl]amino]piperidine-1-carboxylate (15 mg, 27.60 umol, 1 eq) in HCl/MeOH (5 mL) was stirred at 25 °C for 12 h. The residue was concentrated in vacuum. The residue was purified by prep-HPLC (FA) to afford the title compound (5.6 mg, 11.39 umol, 41.27% yield, 99.55% purity, FA) as white solid.

### Example 132. 4-[6-(difluoromethylsulfonyl)-1H-indol-3-yl]-N-[(3S)-3-piperidyl]-5-(trifluoromethyl)pyrimidin-2-amine (Compound 375).

### Step 1: (3S)-3-[[4-[1-(benzenesulfonyl)-6-sulfanyl-indol-3-yl]-5-(trifluoromethyl)pyrimidin-2-yl] amino] piperidine-1-carboxylate

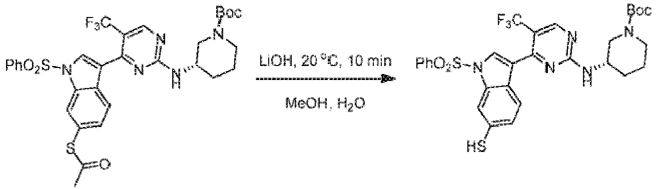

To a solution of tert-butyl (3S)-3-[[4-[6-acetylsulfanyl-1-(benzenesulfonyl) indol-3-yl]-5-(trifluoromethyl) pyrimidin-2-yl] amino] piperidine-1-carboxylate (0.3 g, 443.96 umol, 1 eq) in MeOH (10 mL)/H₂O (1 mL) was added LiOH.H₂O (55.89 mg, 1.33 mmol, 3 eq). The mixture was stirred at 20 °C for 10 min. The residue was adjusted pH to 3 with HCl (2M), filtered and the filter cake was collected. The filter cake was dissolved in DCM (50 mL), dried with anhydrous Na₂SO₄, filtered and concentrated in vacuum to afford the title compound (0.2 g, crude) as yellow solid and used directly in next step.

### Step 2: (3S)-3-[[4-[6-(difluoromethylsulfanyl)-1H-indol-3-yl]-5-(trifluoromethyl)pyrimidin-2-yl]amino]piperidine-1-carboxylate

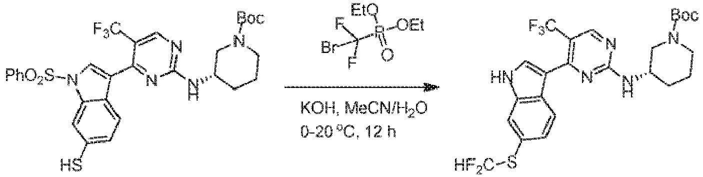

To a solution of tert-butyl (3S)-3-[[4-[1-(benzenesulfonyl)-6-sulfanyl-indol-3-yl]-5-(trifluoromethyl)pyrimidin-2-yl]amino]piperidine-1-carboxylate (0.2 g, 315.60 umol, 1 eq) and 1-[[bromo (difluoro) methyl]-ethoxy phosphoryl] oxyethane (421.34 mg, 1.58 mmol, 5 eq) in H₂O (5 mL)/MeCN (5 mL) was added KOH (354.17 mg, 6.31 mmol, 20 eq) at 0 °C. The mixture was stirred at 20°C for 12 h. The mixture was concentrated in vacuum to remove MeOH, the residue was extracted with ethyl acetate (20 mLx3). The combined organic phase was dried with anhydrous Na₂SO₄, filtered and concentrated in vacuum. The residue was purified by silica gel chromatography (PE: EtOAc = 10:1-5:1-3:1) to afford the title compound (0.08 g, 117.74 umol, 37.31% yield, 80% purity) as yellow solid.

### Step 3: Tert-butyl 3-[2-[[(3S)-1-tert-butoxycarbonyl-3-piperidyl] amino]-5-(trifluoromethyl) pyrimidin-4-yl]-6-(difluoromethylsulfanyl) indole-1-carboxylate

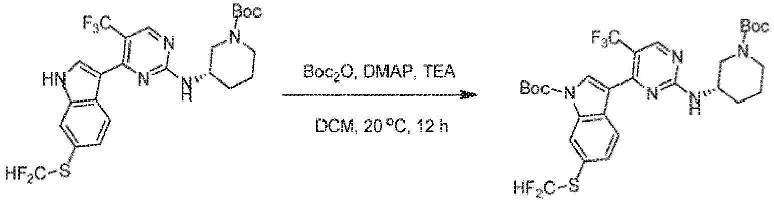

To a solution of tert-butyl (3S)-3-[[4-[6-(difluoromethylsulfanyl)-1H-indol-3-yl]-5-(trifluoromethyl) pyrimidin-2-yl] amino] piperidine-1-carboxylate (0.08 g, 147.18 umol, 1 eq) in DCM (5 mL) was added DMAP (17.98 mg, 147.18 umol, 1eq), TEA (14.89 mg, 147.18 umol, 20.49 uL, 1 eq) and Boc₂O (35.33 mg, 161.90 umol, 37.19 uL, 1.1 eq). The mixture was stirred at 20 °C for 12 h. The residue was poured into water (10 mL). The aqueous phase was extracted with DCM (10 mLx3). The combined organic phase was dried with anhydrous Na₂SO₄, filtered and concentrated in vacuum. The residue was purified by silica gel chromatography (PE: EtOAc = 10:1-5:1) to afford the title compound (0.05 g, 69.91 umol, 47.50% yield, 90% purity) as yellow solid.

### Step 4: Tert-butyl 3-[2-[[(3S)-1-tert-butoxycarbonyl-3-piperidyl]amino]-5-(trifluoromethyl)pyrimidin-4-yl]-6-(difluoromethylsulfonyl)indole-1-carboxylate

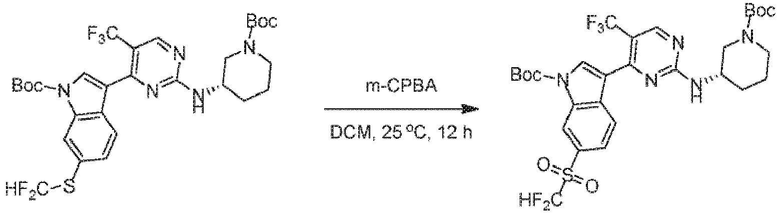

To a solution of tert-butyl 3-[2-[[(3S)-1-tert butoaycarbonγl-3-piperidyl] amino]-5-(trifluoromethyl) pyrimidin-4-yl]-6-(difluoromethylsulfanyl) indole-1-carhoxylate (45 mg, 69.91 umol, 1 eq) in DCM (5 mL) was added m-CPBA (31.23 mg, 153.81 umol, 85% purity, 2.2 eq). The mixture was stirred at 25 °C for 12 h. The residue was poured into sat. NaHCO₃ (5 mL) and stirred for 10 min. The aqueous phase was extracted with EtOAc (10 mLx3). The combined organic phase was dried with anhydrous Na₂SO₄, filtered and concentrated in vacuum. The residue was purified by silica gel chromatography PE: EtOAc = 10:1-5:1-3:1 to afford the title compound (15 mg, 19.98 umol, 28.58% yield, 90% purity) as yellow solid.

### Step 5: 4-[6-(difluoromethylsulfonyl)-1H-indol-3-yl]-N-[(3S)-3-piperidyl]-5-(trifluoroniethyl)pyrimidin-2-amine

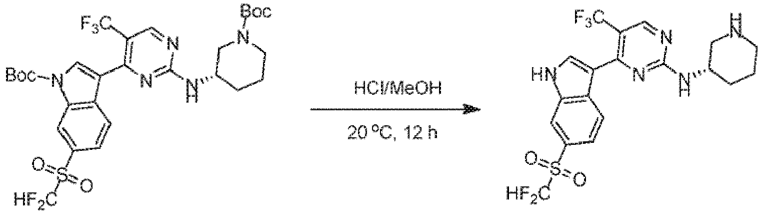

A solution of tert-butyl 6-(difluoromethylsulfonyl)-3-[2-[[(3S)-3-piperidyl]amino]-5-(trifluoromethyl) pyrimidin-4-yl]indole-1-carboxylate (15 mg, 26.06 umol, 1 eq) in HCl/MeOH (3 mL) was stirred at 20 °C for 12 h. The mixture was concentrated in vacuum. The residue was purified by prep-HPLC (FA) to afford the title compound (4.3 mg, 8.20umol, 31.47% yield, 99.46% purity, FA) as white solid.

### Example 133. N-[(3S)-5, 5-dimethyl-3-piperidyl]-4-(6-methylsulfonyl-1H-indol-3-yl)-5-(trifluoromethyl) pyrimidin-2-amine (Compound 376).

### Step 1: Methyl (2S)-5-oxopyrrolidine-2-carboxylate

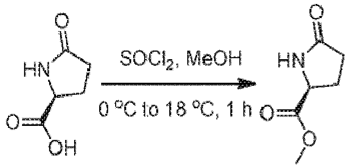

To a solution of (2S)-5-oxopyrrolidine-2-carboxylic acid (117 g, 906.18 mmol, 1 eq) in MeOH (500 mL) was added SOCl₂ (215.62 g, 1.81 mol, 131.47 mL, 2 eq) at 0 °C. The mixture was stirred at 18 °C for 1 hr. The reaction mixture was concentrated. The residue was diluted with EtOAc (1000 mL) and TEA (150 mL). The solid was formed and filtered. The filtrate was evaporated to afford the title compound (147 g, crude) as light yellow oil. It will be used directly in next step without any further purification.

### Step 2: (S)-l-tert-butyl 2-methyl 5-oxopyrrolidine-l,2-dicarboxylate

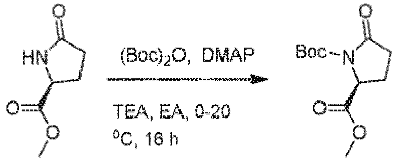

To a solution of methyl (2S)-5-oxopyrrolidine-2-carboxylate (147 g, 1.03 mol, 1 eq), DMAP (15.06 g, 123.24 mmol, 0.12 eq) and TEA (259.80 g, 2.57 mol, 357.35 mL, 2.5 eq) in EtOAc (500 mL) was added dropwise tert-butoxycarbonyl tert-butyl carbonate (291.37 g, 1.34 mol, 306.71 mL, 1.3 eq) at 0 °C. The mixture was stirred at 20 °C for 16 h. The reaction mixture was washed with HCl (0.5 M, 1000 mL), sat. NaHCO₃ (1000 mL), brine (1500 mL), dried over Na₂SO₄, filtered and concentrated under reduced pressure to give a residue. The residue was purified by re-crystallization from MTBE (250 mL). The reaction mixture was filtered and evaporated to afford the title compound (2 batches obtained; Batch 1: 108 g, 100% HPLC purity; Batch 2: 53 g, 90% ¹H NMR purity) as white solid.

### Step 3: (S)-1-tert-butyl 2-methyl 4,4-dimethyl-5-oxopyrrolidine-1,2-dicarboxylate

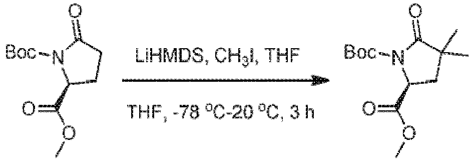

To a solution of (S)-l-tert-butyl 2-methyl 5-oxopyrnolidine-1, 2-dicarboxylate (20 g, 82.22 mmol, 1 eq) in THF (500 mL) was added dropwise LiHMDS (1 M, 172.66 mL, 2.1 eq) at -78 °C under N₂ atmosphere. After addition, the mixture was stirred at this temperature for 0.5 h, and then CH₃I (35.01 g, 246.65 mmol, 15.36 mL, 3 eq) was added dropwise at -78 °C under N₂ atmosphere. The resulting mixture was stirred at 20 °C for 2.5 h. The reaction mixture was diluted with saturated aqueous NH₄Cl (1000 mL) and extracted with EtOAc (300 mL x 3). The combined organic layers were washed with brine (500 mL), dried over Na₂SO₄, filtered and concentrated under reduced pressure to give a residue. The residue was purified by MPLC (SiO₂, PE: EtOAc = 4: 1-3:1) to afford the title compound (8 g, 25.95 mmol, 31.56% yield, 88% purity) as light yellow solid.

### Step 4: tert-butyl N-[(1S)-4-hydroxy-1-(hydroxymethyl)-3,3-dimethyl-butyl]carbamate

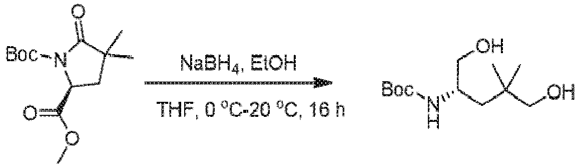

To a solution of (S)-1-tert-butyl 2-methyl 4, 4-dimethyl-5-oxopyrrolidine-1,2-dicarboxylate (4.3 g, 15.85 mmol, 1 eq) in THF (35 mL) was added NaBH₄ (1.80 g, 47.55 mmol, 3 eq) by portions at 0 °C under N₂. After addition, EtOH (8.25 g, 179.09 mmol, 10.47 mL, 11.3 eq) was added dropwise at 0 °C. The resulting mixture was stirred at 20 °C for 16 h. The reaction mixture was poured into saturated aqueous NH₄Cl (250 mL) and extracted with EtOAc (100 mL x 3). The combined organic layers were washed with brine (250 mL), dried over Na₂SO₄, filtered and concentrated under reduced pressure to afford the title compound (3.67 g, crude) as colorless oil. It was used directly in next step without any further purification

### Step 5: [(2S)-2-(tert-butoxycarbonylamino)-4,4-dimethyl-5-methylsulfonyloxy-pentyl] methanesulfonate

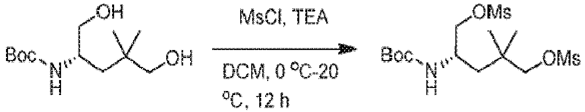

To a solution of tert-butyl N-[(1S)-4-hydroxy-1-(hydroxymethyl)-3,3-dimethyl-butyl]carbamate (3.67 g, 14.84 mmol, 1 eq) and TEA (6.01 g, 59.35 mmol, 8.26 mL, 4 eq) in EtOAc (25 mL) was added dropwise methanesulfonyl chloride (5.10 g, 44.52 mmol, 3.45 mL, 3 eq) at 0 °C, the resulting mixture was stirred at 20 °C for 12 h. The reaction mixture was poured into H₂O (200 mL). EtOAc (50 mL x 3) was used to extract the product. The organic layer was washed by brine (30 mL), dried over Na₂SO₄, filtered and evaporated to afford the title compound (6.06 g crude) as colorless oil. It was used directly in next step without any further purification.

### Step 6: Tert-butyl N-[(3S)-1-benzyl-5,5-dimethyl-3-piperidyl] carbamate

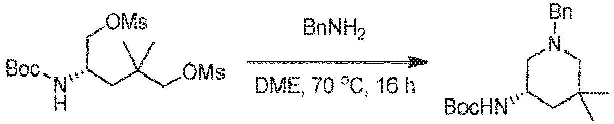

A flask was fitted with [(2S)-2-(tert-butoxycarbonylamino)-4, 4-dimethyl-5-methylsulfonyloxy-pentyl] methanesulfonate (6.06 g, 15.02 mmol, 1 eq), phenylmethanamine (5.15 g, 48.06 mmol, 5.24 mL, 3.2 eq) and DME (50 mL). The reaction mixture was heated to 70 °C for 16 hr. The reaction mixture was poured into H₂O (40 mL). DCM (40 mL x 3) was used to extract the product The organic layer was washed by brine (30 mL), dried over Na₂SO₄, filtered and evaporated to afford the crude product. The crude product was purified by twice MPLC (SiO₂, PE: EtOAc=20:1-10:1) to afford the title compound (580 mg, 1.49 mmol, 9.91% yield, 81.7% purity) as colorless oil.

### Step 7: (3S)-1-benzyl-5, 5-dimethyl-piperidin-3-amine

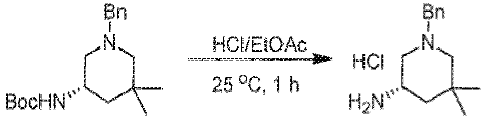

A flask was fitted with tert-butyl N-[(3S)-1-benzyl-5, 5-dimethyl-3-piperidyl] carbamate (300 mg, 942.05 umol, 1 eq) in HCl/EtOAc (15 mL). The mixture was stirred at 25 °C for 1 hr. Some white precipitate was formed. The mixture was filtered and the cake was washed by EtOAc (5 mL). The cake was collected and dried over vacuum to afford the title compound (220 mg, 738.23 umol, 78.36% yield, 85.5% purity, HCl) as white solid. It will be used directly in next step.

### Step 8: 6-methylsulfonyl-1H-indole

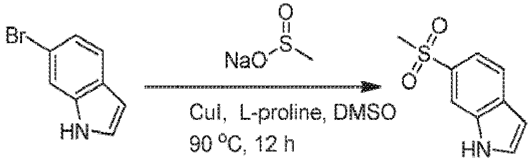

A mixture of 6-bromo-1H-indole (5 g, 25.50 mmol, 1 eq), sodium methanesulfinate (3.38 g, 33.16 mmol, 1.3 eq), CuI (97.15 mg, 510.09 umol, 0.02 eq) and L-Proline (117.45 mg, 1.02 mmol, 0.04 eq) in DMSO (10 mL) was degassed and purged with N₂ for 3 times, and then the mixture was stirred at 90 °C for 12 hr under N₂ atmosphere. The reaction mixture was poured into H₂O (300 mL) and then EtOAc (150 mL x 3) was used to extract the product. The combined organic layer was washed by brine (100 mL), dried over Na₂SO₄, filtered and evaporated to afford the crude product. The crude product was purified by MPLC (SiO₂, PE: EtOAc =3:1-1:1) to afford the title compound (4.17 g, 61 % purity) as dark yellow solid (combined purification with another 2 g scale reaction).

### Step 9: 3-[2-chloro-5-(trifluoromethyl)pyrimidin-4-yl]-6-methylsulfonyl-1H-indole

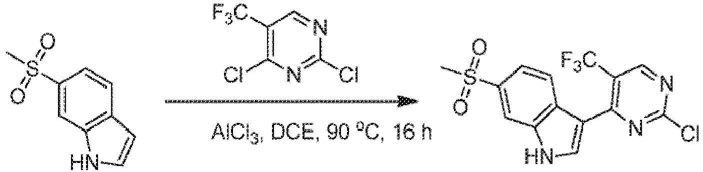

To a solution of 2, 4-dichloro-5-(trifluoromethyl)pyrimidine (5.00 g, 23.05 mmol, 1.5 eq) in DCE (20 mL) was added AlCl₃ (3.28 g, 24.59 mmol, 1.34 mL, 1.6 eq). After addition, the mixture was stirred at 90 °C for 30 min, and then 6-methylsulfonyl-1H-indole (3 g, 15.37 mmol, 1 eq) was added at 90 °C. The resulting mixture was stirred at 90 °C for 15.5 h. The reaction mixture was poured into saturated NaHCOa solution (200 mL). Then EtOAc (200 mL x 3) was added to extract the product. Then organic layers were washed by brine (300 mL), dried over Na₂SO₄, filtered and evaporated to afford the crude product The crude product was purified by acidic prep-HPLC (TFA condition). The separated solution was adjusted to pH=8 by saturated NaHCO₃ solution. EtOAc (150 mL x 3) was used to extract the product. The organic layer was dried over Na₂SO₄, filtered and evaporated to afford the title compound (1.8 g, 4.44 mmol, 28.90% yield, 92.7% purity) as dark yellow solid.

### Step 10: N-[(3S)-1-benzyl-5,5-dimethyl-3-piperidyl]-4-(6-methylsulfonyl-1H-indol-3-yl)-5-(trifluoromethyl)pyrimidin-2-amine

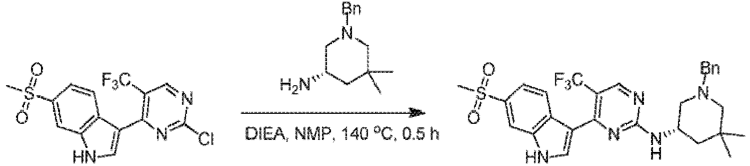

A flask was fitted with 3-[2-chloro-5-(trifluoromethyl)pyrimidin-4-yl]-6-methylsulfonyl-1H-indole (200 mg, 532.26 umol, 1 eq), (3S)-1-benzyl-5,5-dimethyl-piperidin-3-amine (116.21 mg, 456.10 umol, 8.57e-1 eq, HCl) and DIPEA (412.75 mg, 3.19 mmol, 556.27 uL, 6 eq) in NMP (3 mL). The reaction mixture was heated to 140 °C for 30 min. The combined reaction mixture was poured into H₂O (40 mL). EtOAc (25 mL x 3) was used to extract the product. The organic layer was washed by brine (30 mL), dried over Na₂SO₄, filtered and evaporated to afford the crude product. The crude product was purified by MPLC (SiO₂, PE: EtOAc =3:1-1:1) to afford the title compound (2 batches; batch 1: 80 mg, 74.4% purity; batch 2: 20 mg crude, 85% purity) as light yellow solid (combined purification with another 20 mg scale reaction).

### Step 11: tert-butyl (5S)-3,3-dimethyl-5-[[4-(6-methylsulfonyl-1H-indol-3-yl)-5-(trifluoromethyl)pyrimidin-1-yl]amino]piperidine-1-carboxylate

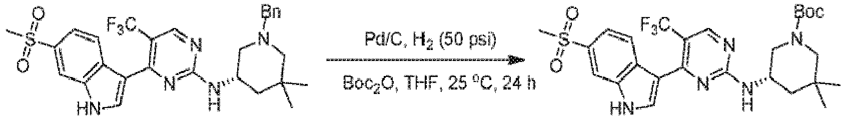

To a solution of N-[(3S)-1-benzyl-5,5-dimethyl-3-piperidyl]-4-(6-methylsulfonyl-1H-indol-3-yl)-5-(trifluoromethyl)pyrimidin-2-amine (55 mg, 98.63 umol, 1 eq) and Boc₂O (43.05 mg, 197.26 umol, 45.32 uL, 2 eq) in THF (20 mL) was added Pd/C (15 mg, 10% purity) under N₂. The suspension was degassed under vacuum and purged with H₂ several times. The mixture was stirred under H₂ (50 psi) at 25 °C for 24 hr. The combined mixture was filtered through celite and the cake was washed by THF (20 mL). The filtrate was evaporated to afford title compound (60 mg, 70% purity) as light yellow solid (combined purification with another 10 mg scale reaction). It was used directly in next step without any further purification

### [$15] Step 12: N-[(3S)-5,5-dimethyl-3-piperidyl]-4-(6-methylsulfonyl-1H-indol-3-yl)-5-(trifluoromethyl)pyrimidin-2-amine

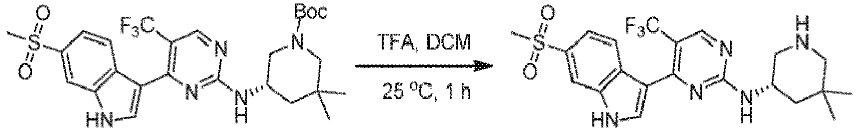

To a solution of tert-butyl (5S)-3,3-dimethyl-5-[[4-(6-methylsulfonyl-1H-indol-3-yl)-5-(trifluoromethyl) pyrimidin-2-yl] amino] *piperidine-1-carboxylate* (60 mg, 105.70 umol, 1 eq) in DCM (4 mL) was added TFA (2 mL). Then the reaction mixture was stirred at 25 °C for 1 hr. The reaction mixture was evaporated to afford the crude product. The crude product was purified by neutral prep-HPLC purification to afford the title compound (21.5 mg, 45.33 umol, 42.88% yield, 98.57% purity) as white solid.

### Example 134. 4-[6-(difluoromethylsulfinyl)-1H-indol-3-yl]-N-[(3S)-3-piperidyl]-5-(trifluoromethyl) pyrimidin-2-amine (Compound 380).

### Step 1: 4-[6-(difluoromethylsulfinyl)-1H-indol-3-yl]-N-[(3S)-3-piperidyl]-5-(trifluoromethyl) pyrimidin-2-amine

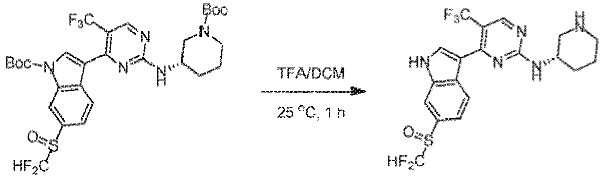

To a solution of tert-butyl 3-[2-[[(3S)-1-tert-butoxycarbonyl-3-piperidyl]amino]-5-(trifluoromethyl)pyrimidin-4-yl]-6-(difluoromethylsulfinyl) indole-1-carboxylate (15 mg, 22.74 umol, 1 eq) in TFA (0.5 mL) and DCM (1 mL) was stirred at 25 °C for 1 h. The mixture was concentrated in vacuum. The residue was purified by prep-HPLC (FA) to afford the title compound (3.5 mg, 6.84 umol, 30.07% yield, 98.75% purity, FA) as white solid.

### Example 135. N-[(3S)-1-cyclopropyl-3-piperidyl]-4-(6-methylsulfonyl-1H-indol-3-yl)-5-(trifluoromethyl) pyrimidin-2-amine (Compound 381).

### [$19] Step 1: Tert-butyl (3S)-3-(benzyloxycarbonylamino)piperidine-1-carboxylate

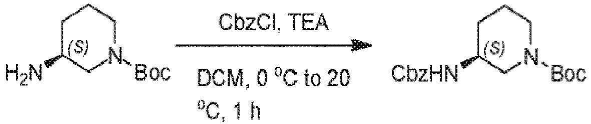

To a solution of tert-butyl (3S)-3-aminopiperidine-1-carboxylate (1 g, 4.99 mmol, 1 eq), TEA (1.52 g, 14.98 mmol, 2.08 mL, 3 eq) in DCM (10 mL) was added benzyl carbonochloridate (1.28 g, 7.49 mmol, 1.06 mL, 1.5 eq) at 0 °C. The mixture was stirred at 20 °C for 1 hr. The reaction mixture was diluted by addition H₂O (100 mL) extracted with EtOAc (50 mL x 3), dried over Na₂SO₄, filtered and concentrated under reduced pressure to give a residue. The residue was purified by column chromatography (SiO₂, PE: EtOAc = 10:1 to 3:1) to afford the title compound (1 g, 2.09 mmol, 41.92% yield, 70% purity) as a colorless oil

### Step 2: Benzyl N-[(3S)-3-piperidyl]carbamate

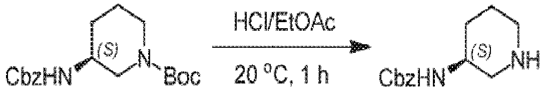

A mixture of tert-butyl (3S)-3-(benzyloxycarbonylamino)piperidine-1-carboxylate (1 g, 2.99 mmol, 1 eq) and HCl/EtOAc (4 M, 10 mL) was stirred at 20 °C for 1 hr. The reaction mixture was concentrated to afford the product (0.85 g, crude, HCl). The product (500 mg, 1.85 mmol, 1 eq, HCl) was dissolved in saturated Na₂CO₃ solution (100 mL) and EtOAc (50 mL x 3) was used to extract the product. The organic layer was dried over Na₂SO₄, filtered and evaporated to afford the title compound (400 mg, 1.71 mmol, 92.45% yield) as white solid. It will be used directly in next step without any further purification.

### Step 3: Benzyl N-[(3S)-1-cyclopropyl-3-piperidyl]carbamate

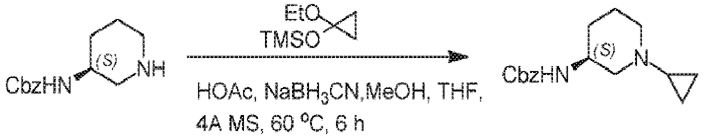

To a mixture of benzyl N-[(3S)-3-piperidyl]carbamate (300 mg, 1.28 mmol, 1 eq) and (1-ethoxycyclopropoxy)-trimethyl-silane (446.39 mg, 2.56 mmol, 514.87 uL, 2 eq) in THF (8 mL) and MeOH (8 mL) were added HOAc (384.47 mg, 6.40 mmol, 366.16 uL, 5 eq), NaBH₃CN (120.70 mg, 1.92 mmol, 1.5 eq) and 4A MS (0.5 g). The reaction mixture was stirred at 60 °C for 6 hr. The reaction mixture was poured into H₂O (20 mL). EtOAc (30 mL x 3) was used to extract the product The organic layer was washed by brine (30 mL), dried over Na₂SO₄, filtered and evaporated to afford the crude product. The crude product was purified by MPLC (SiO₂, PE: EtOAc = 5: 1-1:1) to afford the title compound (300 mg, 702.77 umol, 54.88% yield, 64.27% purity) as white solid.

### Step 4: tert-butyl N-[(3S)-1-cyclopropyl-3-piperidyl]carbamate

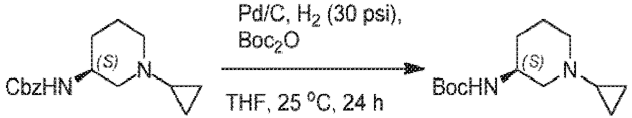

To a solution of benzyl N-[(3S)-1-cyclopropyl-3-piperidyl]carbamate (180 mg, 656.08 umol, 1 eq) and Boc₂O (286.37 mg, 1.31 mmol, 301.45 uL, 2 eq) in THF (15 mL) was added Pd/C (40 mg, 10% purity) under N₂. The suspension was degassed under vacuum and purged with H₂ several times. The mixture was stirred under H₂ (30 psi) at 25 °C for 24 hr. The mixture was filtered through celite and the cake was washed by THF (10 mL) and MeOH (10 mL). The filtrate was evaporated to afford the title compound (400 mg crude) as red yellow solid. It was used directly in next step without any further purification (Combined purification with another 30 mg scale reaction).

### Step 5: (3S)-1-cyclopropylpiperidin-3-amine

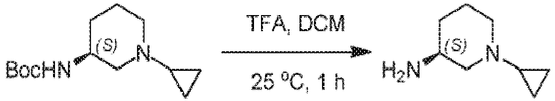

A flask was fitted with tert-butyl N-[(3S)-1-cyclopropyl-3-piperidyl]carbamate (200 mg, 249.65 umol, 1 eq) in DCM (2 mL). Then TFA (1 mL) was added. After that the reaction mixture was stirred at 25 °C for 1 hr. The reaction mixture was evaporated to afford the title compound (240 mg crude, TFA) as red oil. It will be used directly in next step without any further purification.

### Step 6: N-[(3S)-1-cyclopropyl-3-piperidyl]-4-(6-methylsulfonyl-1H-indol-3-yl)-5-(trifluoromethyl)pyrimidin-2-amine

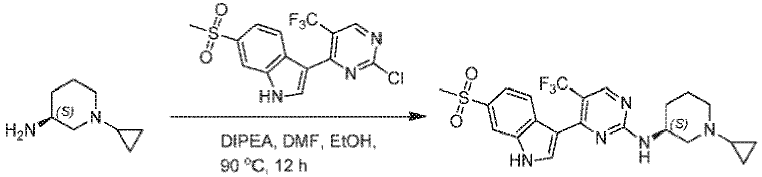

A flask was fitted with 3-[2-chloro-5-(trifluoromethyl)pyrimidin-4-yl]-6-methylsulfonyl-1H-indole (50 mg, 133.07 umol, 1 eq), (3S)-1-cyclopropylpiperidin-3-amine (120 mg, 471.98 umol, 3.55 eq, TFA) and DIPEA (137.58 mg, 1.06 mmol, 185.42 uL, 8 eq) in EtOH (2 mL) and DMF (2 mL). The reaction mixture was heated to 90 °C and reacted for 12 hr. The reaction mixture was poured into H₂O (20 mL). EtOAc (43 mL x 3) was used to extract the product. The organic layer was washed by brine (30 mL), dried over Na₂SO₄, filtered and evaporated to afford the crude product The crude product was purified by acidic prep-HPLC (HCl condition) to afford the title compound (10.35 mg, 19.95 umol, 7.50% yield, 99.46% purity, HCl) as yellow solid.

### Example 136. 3-[5-chloro-2-[[(3S)-5, 5-dimethyl-3-piperidyl]amino]pyrimidin-4-yl]-7-methylsulfonyl-1H-indole-6-carbonitrile (Compound 382).

### Step 1: 3-[2-[[(3S)-1-benzyl-5,5-dimethyl-3-piperidyl]amino]-5-chloro-pyrimidin-4-yl]-7-methylsulfonyl-1-(2-trimethylsilylethoxymethyl)indole-6-carbonitrile

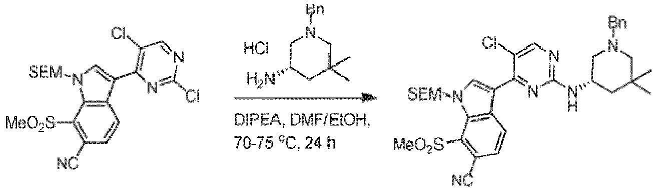

To a solution of 3-(2,5-dichloropyrimidin-4-yl)-7-methylsulfonyl-1-(2-trimethylsilylethoxy methyl)indole-6-carbonitrile (244.05 mg, 490.58 umol, 1 eq) in DMF (2 mL) and EtOH (2 mL) was added DIPEA (380.43 mg, 2.94 mmol, 512.71 uL, 6 eq) and (3S)-1-benzyl-5,5-dimethyl-piperidin-3-amine (0.15 g, 588.70 umol, 1.2 eq, HCl). The mixture was stirred at 70°C for 12 h under N₂. The mixture was stirred at 75°C for 12 h under N₂. The reaction mixture was diluted with water 200 mL and extracted with EtOAc (50 mL x 3). The combined organic layers were washed with brine (50 mL x 3), dried over Na₂SO₄, filtered and concentrated under reduced pressure to give a residue. The residue was purified by column chromatography (SiO₂, PE: EtOAc=10: 1) to afford the title compound (0.1 g, 104.51 umol, 21.30% yield, 71% purity) as a white solid.

### Step 2: 3-[2-[[(3S)-1-benzyl-5,5-dimethyl-3-piperidyl]amino]-5-chloro-pyrimidin-4-yl]-7-methylsulfonyl-1H-indole-6-carbonitrile

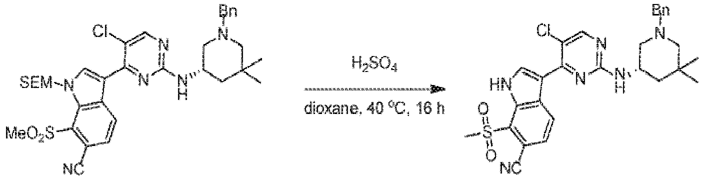

To a solution of 3-[2-[[(3S)-1-benzyl-5, 5-dimethyl-3-piperidyl]amino]-5-chloro-pyrimidin-4-yl]-7-methylsulfonyl-1-(2-trimethylsilylethoxymethyl)indole-6-carbonitrile (0.08 g, 117.76 umol, 1 eq) in dioxane (3 mL) was added H₂SO₄ (117.85 mg, 1.18 mmol, 64.05 uL, 98% purity, 10 eq). The mixture was stirred at 40 °C for 16 h. The reaction mixture was diluted with saturated aqueous of Na₂CO₃ to adjust pH to 8 and extracted with EtOAc (lOmL x 3). The combined organic layers were washed with brine (10 mL x 3), dried over Na₂SO₄, filtered and concentrated under reduced pressure to give a residue. The residue was purified by column chromatography (SiO₂, PE: EtOAc=3: 1) to afford the title compound (60 mg) as a yellow oil. (The reaction was combined with another reaction in 5 mg scale for purification)

### Step 3: Tert-butyl(5S)-5-[[5-chloro-4-(6-cyano-7-methylsulfonyl-1H-indol-3-yl)pyrimidin-2-yl]amino]-3,3-dimethylpiperidine-1-carboxylate

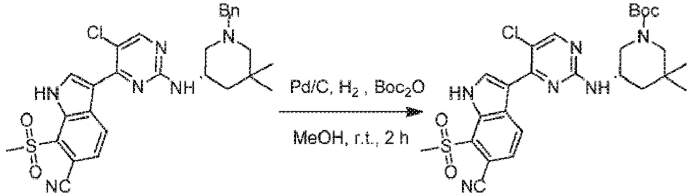

To a solution of 3-[2-[[(3S)-1-benzyl-5,5-dimethyl-3-piperidyl]amino]-5-chloro-pyrimidin-4-yl]-7-methylsulfonyl-1H-indole-6-carbonitrile (0.03 g, 54.64 umol, 1 eq) in MeOH (1 mL) was added Pd/C (0.03 g, 10% purity) and Boc₂O (11.92 mg, 54.64 umol, 12.55 uL, 1 eq). The mixture was stirred at 20 °C for 2 h under H₂ (15 Psi). The reaction mixture was filtered and concentrated under reduced pressure to afford the title compound (50 mg) as a yellow oil. (The reaction was combined with another reaction in 10 mg scale for purification)

### Step 4: 3-[5-chloro-2-[[(3S)-5,5-dimethyl-3-piperidyl]amino]pyrimidin-4-yl]-7-methylsulfonyl-1H-indole-6-carbonitrile

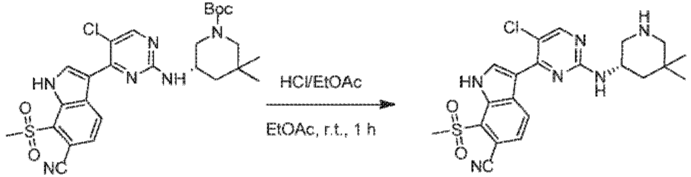

To a solution of tert-butyl (5S)-5-[[5-chloro-4-(6-cyano-7-methylsulfonyl-1H-indol-3-yl) pyrimidin-2-yl]amino]-3,3-dimethylpiperidine-1-carboxylate (0.04 g, 71.55 umol, 1 eq) in EtOAc (2 mL) was added HCl/EtOAc (4 M, 2 mL, 111.82 eq). The mixture was stirred at 20 °C for 1 h. The reaction mixture was concentrated under reduced pressure to give a residue. The residue was purified by prep-HPLC (HCl condition) to afford the title compound (6.90 mg, 13.31 umol, 18.61% yield, 95.58% purity, HCl) as a white solid. (The reaction was combined with another reaction in 10 mg scale for purification).

### Example 137. 3-[3-[2-[[(3S)-3-piperidyl]amino]-5-(trifluoromethyl)pyrimidin-4-yl]-1H-indol-6-yl]pyridin-2-ol (Compound 310)

### Step 1: Tert-butyl (3S)-3-[[4-[1-(benzenesulfonyl)-6-(4,4,5,5-tetramethyl-1,3,2-dioxaborolan-2-yl)indol-3-yl]-5-(trifluoromethyl)pyrimidin-2-yl]amino]piperidine-1-carboxylate

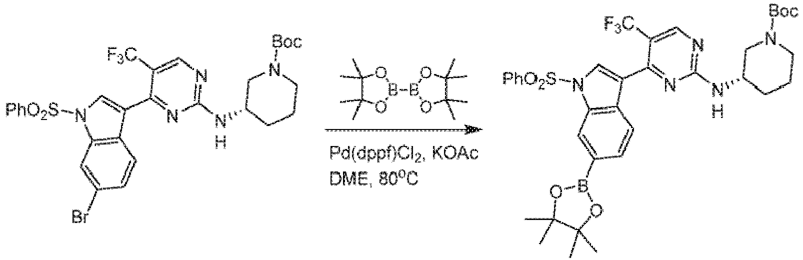

To the solution of tert-butyl (3S)-3-[[4-[1-(benzenesulfonyl)-6-bromo-indol-3-yl]-5-(trifluoromethyl)pyrimidin-2-yl]amino]piperidine-1-carboxylate (5.00 g, 7.35 mmol, 1 eq) in DME was added 4,4,5,5-tetramethyl-2-(4,4,5,5-tetramethyl-1,3,2-dioxaborolan-2-yl)-1,3,2-dioxaborolane (3.73 g, 14.7 mmol, 2 eq), KOAc (2.16 g, 22.0 mmol, 3 eq) and Pd(dppf)Cl₂ (806 mg, 1.10 mmol, 0.15 eq) . The reaction mixture was degassed and purged with N₂ 3 times, then stirred at 80°C for 16 hr under N₂ atmosphere. LC-MS and TLC indicated complete reaction. The reaction mixture was poured into water 100 mL, and then extracted with EtOAc (3X50 mL), dried over Na₂SO₄, filtered and concentrated under reduced pressure to give a residue.

The residue was purified by column chromatography (SiO2, Petroleum ether/Ethyl acetate=10/l to 1:1) to afford the title compound (6.03 g, crude) as a yellow gum.

### Step 2: tert-butyl (3S)-3-[[4-[1-(benzenesulfonyl)-6-(2-hydroxy-3-pyridyl)indol-3-yl]-5-(trifluoromethyl)pyrimidin-2-yl]amino]piperidine-1-carboxylate

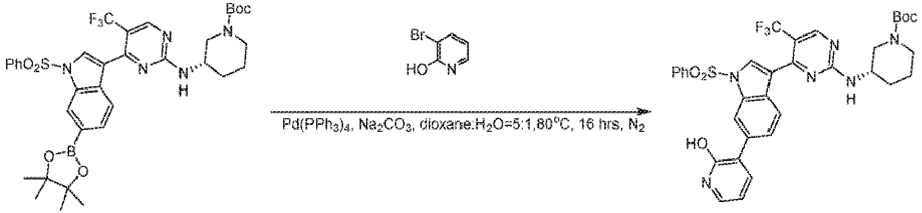

4-[1-(benzenesulfonyl)-6-(4,4,5,5-tetramethyl-1,3,2-dioxaborolan-2-yl)indol-3-yl]-5-(trifluoromethyl)pyrimidin-2-amine (0.3 g, 551 umol, 1 eq) , 3-bromopyridin-2-ol (105 mg, 606 umol, 1.1 eq) ,Pd(PPh3)₄ (63.6 mg, 55.1 umol, 0.1 eq) Na₂CO₃ (116 mg, 1.10 mmol, 2 eq) in dioxane (2.5 mL) and H₂O (0.5 mL) was stirred at 80°C for 16 hours under N₂. LC-MS and TLC indicated completion of the reaction. The mixture was concentrated and the residue was purified by prep-TLC (EA/MeOH=10/1) to afford the title compound (0.3 g, 431 umol, 78.3% yield) obtained as a yellow solid.

### Step 3: tert-butyl-(3S)-3-[[4-[6-(1-hydroxy-3-pyridyl)-1H-indol-3-yl]-5-(trifluoromethyl)pyrimidin-1-yl]amino]piperidine-1-carboxylate

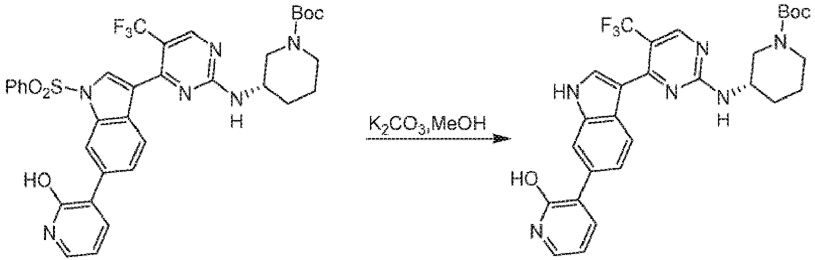

To a mixture of tert-butyl (3S)-3-[[4-[1-(benzenesulfonyl)-6-(2-hydroxy-3-pyridyl)indol-3-yl]-5-(trifluoromethyl)pyrimidin-2-yl]amino]piperidine-1-carboxylate (0.3 g, 481 umol, 1 eq) in MeOH (2 mL) was added K₂CO₃ (119 mg, 863 umol, 2 eq). The mixture was stirred at 80°C for 16 hours, until LC-MS and TLC indicated completion of the reaction. The mixture was concentrated and the mixture was purified by Prep-TLC (EtOAc). The title compound (0.15 g, 270 umol, 62.6% yield) was obtained as yellow solid.

### [$35] Step 4: 3-[3-[2-[[(3S)-3-piperidyl]amino]-5-(trifluoromethyl)pyrimidin-4-yl]-1H-indol-6-yl]pyridin-2-ol (Compound 310)

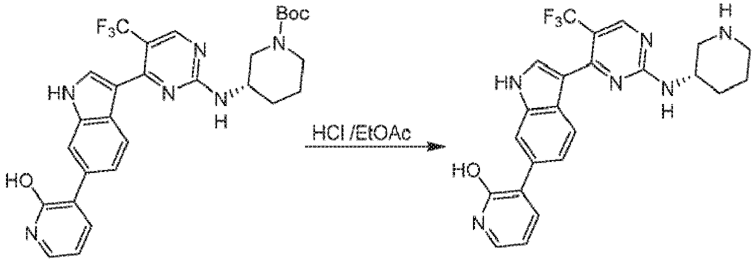

tert-butyl (3S)-3-[[4-[6-(2-hydroxy-3-pyridyl)-1H-indol-3-yl]-5-(trifluoromethyl)pyrimidin-2-yljamino]piperidino-l-carboxylate (0.1 g, 180 umol, 1 eq) was dissolved in DCM (1 mL) .Then HCl/EtOAc (4 M, 90.6 uL, 10 eq) was added The mixture was shaken at 25°C for 2 hours. TLC indicated completion of the reaction. The mixture was concentrated to afford the title compound (0.03 g, 59.8 umol, 33.2% yield, 98% purity, HCl) as a yellow solid without further purification.

### Example 138. 4-methyl-2-oxo-5-[3-[2-[[(3S)-3-piperidyl]amino]-5-(trifluoromethyl)pyrimidin-4-yl]-1H-indol-6-yl]-1H-pyridine-3-carbonitrile (Compound 311)

### Step 1: tert-butyl (3S)-3-[[4-[1-(benzenesulfonyl)-6-(5-cyano-4-methyl-6-oxo-1H-pyridin-3-yl)indol-3-yl]-5-(trifluoromethyl)pyrimidin-2-yl]amino]piperidine-1-carboxylate

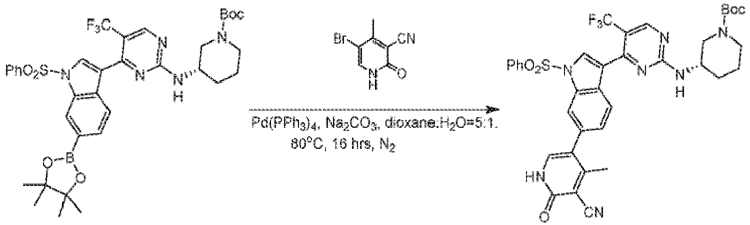

A mixture of tert-butyl (3S)-3-[[4-[1-(benzenesulfonyl)-6-(4,4,5,5-tetramethyl-1,3,2-dioxaborolan-2-yl)indol-3-yl]-5-(trifluoromethyl)pyrimidin-2-yl]amino]piperidine-1-carboxylate (0.2 g, 274 umol, 1 eq), 5-bromo-4-methyl-2-oxo-3,4-dihydro-1H-pyridine-3-carbonitrile (70.9 mg, 329 umol, 1.2 eq), Na₂CO₃ (58.2 mg, 549 umol, 2 eq), Pd(PPh₃)₄ (158 mg, 137 umol, 0.5 eq) in dioxane (10 mL) and H₂O (2 mL) was degassed and purged with N₂ for 3 times, and then the mixture was stirred at 80°C for 12 hr under N₂ atmosphere. LCMS and TLC showed the reaction was complete. The reaction mixture was filtered and concentrated under reduced pressure. The residue was purified by prep-TLC (SiO₂, Ethyl acetate/Methanol= 30/1) to afford the title compound (0.17 g, 231 umol, 84.2% yield) as a yellow solid.

### Step 2: tert-butyl (3S)-3-[[4-[6-(5-cyano-4-methyl-6-oxo-1H-pyridin-3-yl)-1H-indol-3-yl]-5-(trifluoromethyl)pyrimidin-2-yl]amino]piperidine-1-carboxylate

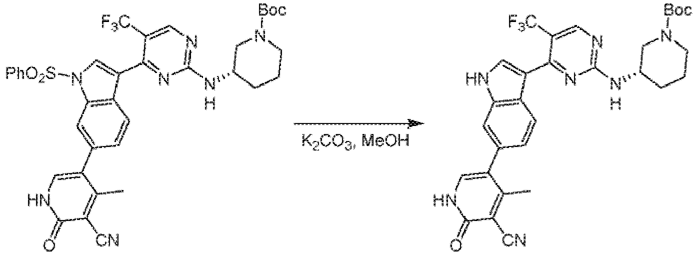

To a solution of tert-butyl (3S)-3-[[4-[1-(benzenesulfonyl)-6-(5-cyano-4-methyl-6-oxo-1H-pyridin-3-yl)indol-3-yl]-5-(trifluoromethyl)pyrimidin-2-yl]amino]piperidine-1-carboxylate (0.1 g, 136.28 umol, 1 eq) in MeOH (3 mL) was added K₂CO₃ (37.6 mg, 272 umol, 2 eq) and H₂O (1 mL), DCE (1 mL). The mixture was stirred at 45°C for 12 hours LCMS showed the reaction was complete. The reaction was concentrated under reduced pressure to give a residue. The residue was purified by prep-HPLC (HCl condition) to afford the title compound (0.06 g, 101 umol, 74.1% yield) as a yellow solid.

### Step 3: 4-methyl-2-oxo-5-[3-[2-[[(3S)-3-piperidyl]amino]-5-(trifluoromethyl)pyrimidin-4-yl]-1H-indol-6-yl]-1H-pyridine-3-carbonitrile (Compound 311)

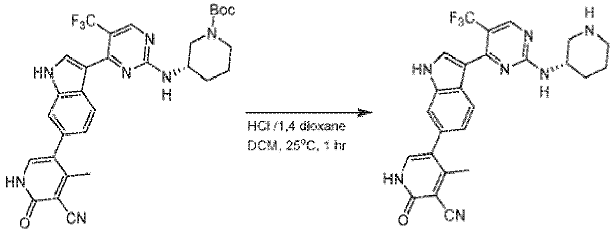

To a solution of tert-butyl (3S)-3-[[4-[6-(5-cyano-4-methyl-6-oxo-1H-pyridin-3-yl)-1H-indol-3-yl]-5-(trifluoromethyl)pyrimidin-2-yl]amino]piperidine-l-cafboxylate (0.05 g, 84.2 umol, 1 eq) in DCM (2 mL) was added dioxane/HCl (2 mL) at 0°C. The mixture was stirred at 25°C for 1 hour, until TLC showed the reaction was complete. The reaction was filtered and concentrated under reduced pressure to afford the title compound (35 mg, 66.0 umol, 78.4% yield, HCl) as a white solid.

### Example 139, 5-[3-[2-[[(3S)-3-piperidyl]amino]-5-(trifluoromethyl)pyrimidin-4-yl]-1H-indol-6-yl]-1H-pyrimidine-2,4-dione (Compound 333)

### Step 1: tert-butyl (3S)-3-[[4-[1-(benzenesulfonyl)-6-(2,4-dioxo-1H pyrimidin-5-yl)indol-3-yl]-5-(trifluoromethyl)pyrimidin-2-yl]amino]piperidine-1-carboxylate

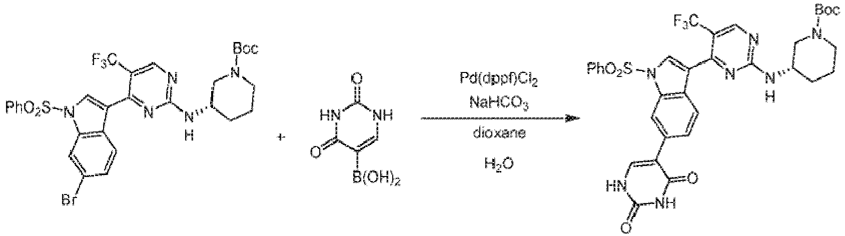

To a solution of tert-butyl (3S)-3-[[4-[1-(benzenesulfonyl)-6-bromo-indol-3-yl]-5-(trifluoromethyl) pyrimidin-2-yl]amino]piperidine-1-carboxylate (200 mg, 293 umol, 1 *eq)* in dioxane (8 mL) and H₂O (1.6 mL) was added NaHCOa (49.3 mg, 587 umol, 22.8 uL, 2 *eq)* and (2,4-dioxo-1H-pyrimidin-5-yl)boronic acid (55 mg, 352 umol, 1.2 *eq).* Then Pd(dppf)Cl₂ (215 mg, 293 umol, 1 *eq)* was added into the reaction mixture under N2. The reaction mixture was stirred at 100°C for 16 hours. LCMS indicated completion of the reaction. The mixture was extracted with DCM (30mL) and water (20mL*3). The combined organic layers were washed with brine (20 mL). The organic layers were dried over Na₂SO₄ and then filtered and concentrated under reduced pressure to give a residue. The residue was purified by prep-TLC (SiO2, Ethyl acetate:Methanol = **10:1)** to afford the title compound (150 mg, 210 umol, 71.7% yield) as light brown solid.

### Step 2: tert-butyl (3S)-3-[[4-[6-(2,4-dioxo-1H-pyrimidin-5-yl)-1H-indol-3-yl]-5-(trifluoromethyl)pyrimidin-2-yl]amino]piperidine-1-carboxylate

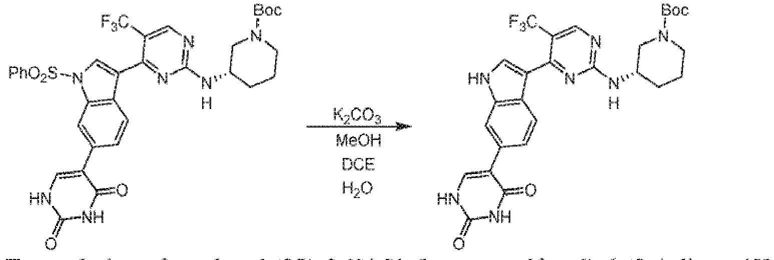

To a solution of tert-butyl (3S)-3-[[4-[1-(benzenesulfonyl)-6-(2,4-dioxo-1H-pyrimidin-5-yl)indol-3-yl]-5-(trifluoromethyl)pyrimidin-2-yl]amino]piperidine-1-carboxylate (140 mg, 196 umol, 1 *eq)* in MeOH (3 mL), DCE (1 mL) and H₂O (1 mL) was added K₂CO₃ (54.3 mg, 393 umol, 2 *eq).* The reaction mixture was stirred at 45°C for 16 hours. LCMS indicated completion of the reaction. The mixture was extracted with EtOAc (30mL) and water (20mL*3). The combined organic layers were washed with brine (20 mL). The organic layers were dried over Na₂SO₄ and then filtered and concentrated under reduced pressure to give a residue. The residue was purified by prep-TLC (SiO₂, Ethyl acetate:Methanol = 10:1) to afford the title compound (40 mg, 69.9 umol, 35.5% yield) was obtained as off-white solid.

### Step 3: 5-[3-[2-[[(3S)-3-piperidyl]amino]-5-(trifluoromethyl)pyrimidin-4-yl]-1H-indol-6-yl]-1H-pyrimidine-2,4-dione (Compound 333)

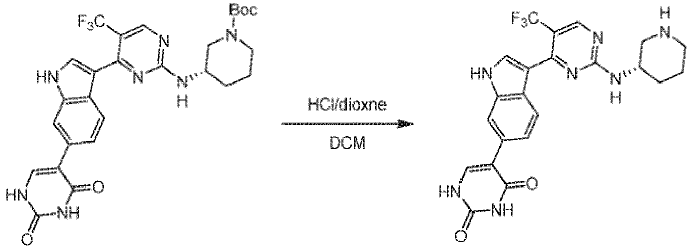

To a solution of tert-butyl (3S)-3-[[4-[6-(2,4-dioxo-1H-pyrimidin-5-yl)-1H-indol-3-yl]-5-(trifluoromethyl)pyrimidin-2-yl]amino]piperidine-l-carboxylate (40 mg, 69.99 umol, 1 *eq)* in DCM (2 mL) was added HCl/dioxane (4 M, 17.50 uL, 1 *eq).* The reaction mixture was stirred at 25°C for 2 hours. LCMS indicated completion of the reaction. The solid was filtered and the filter cake was washed with DCM (5 mL) and dried to afford the title compound (33.5mg, 65.9 umol, 94.2% yield, HCl) as yellow solid.

### Example 140. tert-butyl (3S)-3-[[4-[6-[3-(pyrrolidine-1-carbonyl)phenyl]-1H-indol-3-yl]-5-(trifluoromethyl)pyrimidin-2-yl]amino]piperidine-1-carboxylate (Compound 340)

### Step 1: Tert-butyl (3S)-3-[[4-[1-(benzenesulfonyl)-6-[3-(pyrrolidine-1-carbonyl)phenyl]indol-3-yl]-5-(trifluoromethyl)pyrimidin-1-yl]amino]piperidine-1-carboxylate

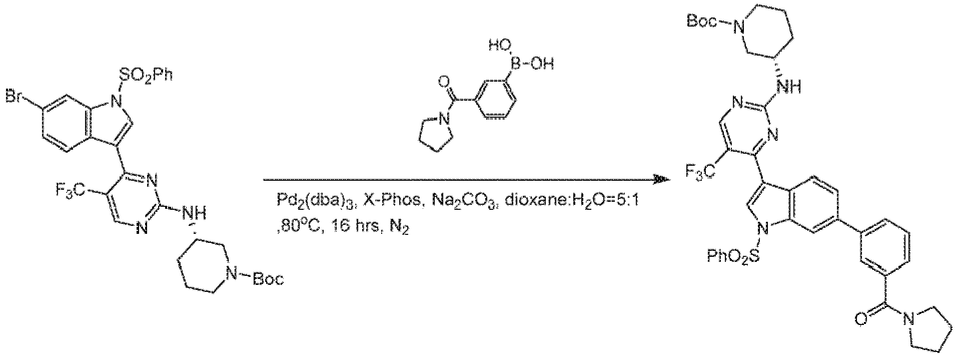

[3-(pyrrolidine-1-carbonyl)phenyl]boronic acid (115 mg, 529 umol, 1.2 eq), tert-butyl (3S)-3-[[4-[1-(benzenesulfonyl)-6-bromo-indol-3-yl]-5-(trifluoromethyl)pyrimidin-2-yl]amino]piperidine-1-carboxylate (0.3 g, 440 umol, 1 eq) ,Pd₂(dba)₃ (40.3 mg, 44.0 umol, 0.1 eq) X-PHOS (21.7 mg, 44.0 umol, 0.1 eq) Na₂CO₃ (93.4 mg, 881 umol, 2 eq) in dioxane (5 mL) and H₂O (1 mL) was stirred at 80°C for 16 hrs under N₂. LC-MS and TLC indicated complete reaction. The mixture was concentrated and the residue was purified by prep-TLC (EtOAc/MeOH=10/1) to afford the title compound (0.3 g, 387 umol, 87.8% yield) as a yellow solid.

### Step 2: tert-butyl (3S)-3-[[4-[6-[3-(pyrrolidine-1-carbonyl)phenyl]-1H-indol-3-yl]-5-(trifluoromethyl)pyrimidin-2-yl]amino]piperidine-1-carboxylate

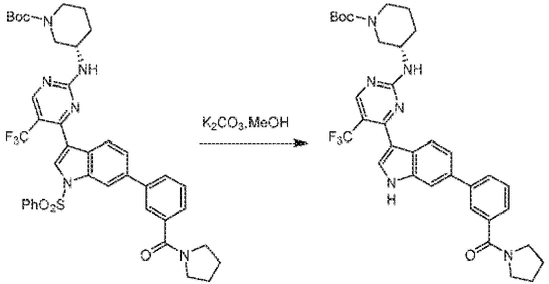

A mixture of tert-butyl (3S)-3-[[4-[1-(benzenesulfonyl)-6-[3-(pyrrolidine-1-carbonyl)phenyl]indol-3-yl]-5-(trifluoromethyl)pyrimidin-2-yl]amino]piperidine-1-carboxylate (0.3 g, 387 umol, 1 eq), K₂CO₃ (107 mg, 774 umol, 2 eq) in MeOH (5 mL) was stirred at 45°C for 16 hr. LC-MS and TLC indicated complete reaction. The mixture was concentrated and purified by preparative TLC (EtOAc) to afford the title compound (0.07 g, 110 umol, 28.4% yield) as a yellow solid.

### Step 3: [3-[3-[2-[[(3S)-3-piperidyl]amino]-5-(trifluoromethyl)pyrimidin-4-yl]-1H-indol-6-yl]phenyl]-pyrrolidin-1-yl-methanone

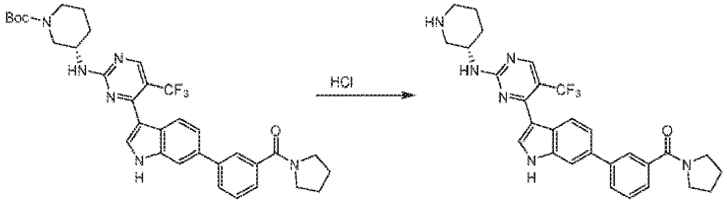

Tert-butyl (3S)-3-[[4-[6-[3-(pyrrolidine-1-carbonyl)phenyl]-1H-indol-3-yl]-5-(trifluoromethyl) pyrimidin-2-yl]amino]piperidine-1-carboxylate (0.1 g, 157 umol, 1 eq) was dissolved in DCM (1 mL). HCl/EtOAc (4 M, 393 uL, 10 eq) was added and the mixture was shaken at 25°C for 2 hr. TLC indicated complete reaction. The mixture was concentrated to afford the title compound (0.07 g, 126 umol, 80.3% yield, 96.7% purity) as yellow solid.

### Example 141. 4-[6-(3-phenoxyphenyl)-1H-indol-3-yl]-N-[(3S)-3-piperidyl]-5 (trifluoromethyl) pyrimidin-2-amine (Compound 341)

### Step 1: Tert-butyl (3S)-3-[[4-[1-(benzenesulfonyl)-6-(3-phenoxyphenyl)indol-3-yl]-5-(trifluoromethyl)pyrimidin-1-yl]amino]piperidine-1-carboxylate

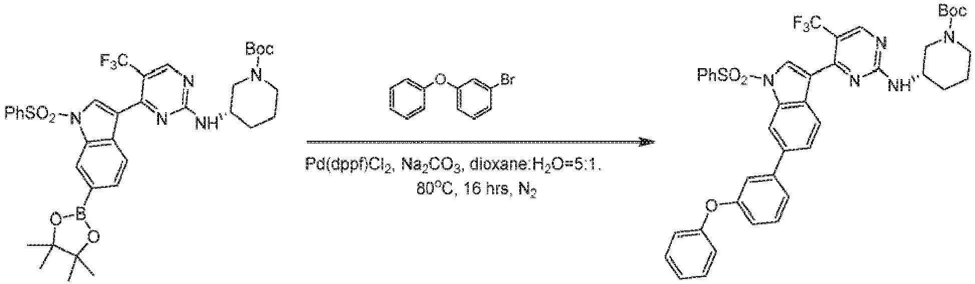

A mixture of tert-butyl (3S)-3-[[4-[1-(benzenesulfonyl)-6-bromo-indol-3-yl]-5 (trifluoromethyl)pyrimidin-2-yl]amino]piperidine-1-carboxylate (0.2 g, 293 umol, 1 eq), (3-phenoxyphenyl)boronic acid (75.4 mg, 352 umol, 81.9 uL, 1.2 eq), Na₂CO₃ (62.30 mg, 587 umol, 2 eq), Pd(dppf)Cl₂ (107 mg, 146 umol, 0.5 eq) in dioxane (10 mL) and H₂O (2 mL) was degassed and purged with N₂ 3 times, and then the mixture was stirred at 80°C for 12 hr under N₂ atmosphere. LCMS and TLC showed the reaction was complete. The reaction mixture was filtered and concentrated under reduced pressure to give a residue. The residue was purified by preparative TLC (SiO₂, Ethyl acetate:Methanol= 30:1) to afford the title compound (0.17 g, 220 umol, 75.1% yield) as a yellow solid.

### Step 2: (3S)-3-[[4-[6-(3-phenoxyphenyl)-1H-indol-3-yl]-5-(trifluoromethyl)pyrimidin-2-yl]amino]piperidine-1-carboxylate

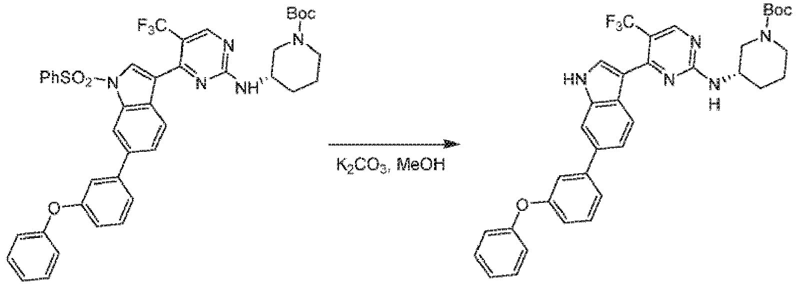

To a solution of tert-butyl (3S)-3-[[4-[1-(benzenesulfonyl)-6-(3-phenoxyphenyl)indol-3-yl]-5-(trifluoromelhyl)pyrimidin-2-yl]amino]piperidine-1-carboxylate (0.16 g, 207 umol, 1 eq) in MeOH (3 mL) was added K₂CO₃ (57.4 mg, 415 umol, 2 eq) and H₂O (1 mL), DCE (1 mL).The mixture was stirred at 80°C for 12 hr. TLC showed the reaction was complete. The reaction mixture was filtered and concentrated under reduced pressure to give a residue. The residue was purified by preparative TLC (SiO2, Ethyl acetate: Methanol= 30:1) to afford the title compound (0.12 g, 190 umol, 91.6% yield) as a yellow solid.

### Step 3: 4-[6-(3-phenoxyphenyl)-1H-indol-3-yl]-N-[(3S)-3-piperidyl]-5 (trifluoromethyl) pyrimidin-2-amine

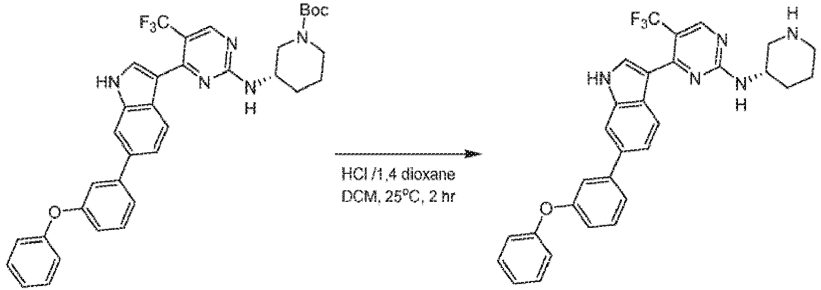

To a solution of tert-butyl (3S)-3-[[4-[6-(3-phenoxyphenyl)-1H-indol-3-yl]-5-(trifluoromethyl) pyrimidin-2-yl]amino]piperidine-1-carboxylate (0.12 g, 190 umol, 1 eq) in DCM (2 mL) was added dioxane/HCl (4 M, 2 mL, 41.9 eq) at 0°C. The mixture was stirred at 25 °C for 1 hour. LCMS and TLC showed the reaction was complete. The reaction mixture was filtered and concentrated under reduced pressure to afford the title compound (0.070 g, 132 umol, 69.3%).

**Table of selected compounds, their synthesis, NMR and Mass Spectrometry data.**

| Compound # | Done as | ¹H NMR | Calcd. Mass | Found Mass (MH⁺) |
|---|---|---|---|---|
| 111 | Example 1 | ¹H NMR (500 MHz, DMSO) δ 12.09 (br s, 1H), 8.70 (br s, 1H), 8.57 (s, 1H), 8.33 (s, 1H), 8.29 (s, 1H), 7.51 (dd, *J* = 10.7, 7.2 Hz, 1H), 7.56 - 7.28 (br m, 1H), 4.05 - 3.93 (m, 1H), 3.21 (d, *J* = 11.5 Hz, 1H), 3.00 (d, *J* = 12.3 Hz, 1H), 2.66 (t, *J* = 10.4 Hz, 2H), 2.12 - 1.95 (br m, 1H), 1.84 -1.75 (m, 1H), 1.65 - 1.43 (m, 2H). | 363.79 | 364.20 |
| 131 | Starting with (S)-N-(5-chloro-4-(l-(phenylsulfonyl)-1H-indol-3-yl)pyrimidin-2-yl)quinuclidin-3-amine (Example 1 from 3-(2,5-dichloropyrimidin-4-yl)-l-(phenylsulfonyl)-1H-indole and (S)-(-)-3-aminoquinuclidine dihydrochloride) following Example 2. | ¹H NMR (500 MHz, DMSO) δ 11.98 (brs, 1H), 8.44 - 8.32 (m, 4H), 7.53 - 7.48 (m. 1H), 7.25 - 7.15 (m, 2H), 5.12 - 5.04 (m, 0.5H), 4.92 - 4.85 (m, 0.5H), 4.13 - 4.02 (m, 1H), 3.82 (dd, *J* = 13.8, 4.4 Hz, 1H), 3.46 - 3.32 (m, 2H), 3.10 - 3.00 (m, 2H), 2.94 - 2.82 (m, 2H), 2.03 - 1.94 (m, 1H), 1.68 -1.54 (m, 1H), 1.46 - 1.29 (m, 1H). | 353.85 | 354.07 |
| 133 | Starting with 3-(5-bromo-2-chloropyrimidin-4-yl)-1-(phenylsulfonyl)-1H-indole (Example 3) following Example 1 and 2. | ¹H NMR (500 MHz, DMSO) δ 11.84 (brs, 1H), 8.51 (d, *J* = 2.8 Hz, 1H), 8.47 (brs, 1H), 8.37 (s, 1H), 8.33 (brs, 1H), 7.49 (d, *J* = 8.0 Hz, 1H), 7.27 - 7.13 (m, 3H), 3.96 (brs, 1H), 3.19 (dd, *J* = 11.9, 3.2 Hz, 1H), 2.95 (d, *J* = 12.5 Hz, 1H), 2.66 - 2.58 (m, 2H), 2.00 (brs, 1H), 1.79 -1.72 (m, 1H), 1.61 - 1.42 (m, 2H). | 372.26 | 374.02 |
| 134 | Starting from 3-(2,5-dichloropyrimidin-4-yl)-l-(phenylsulfonyl)-1H-indole and (R)-(-)-3-aminoquinuclidine dihydrochloride) following Example 2 and 1. | ¹H NMR (500 MHz, DMSO) δ 11.93 (brs, 1H), 8.43 - 8.32 (m, 4H), 7.52 - 7.49 (m, 1H), 7.25 - 7.15 (m, 2H), 5.13 - 5.06 (m, 0.5H), 4.92 - 4.85 (m, 0.5H), 4.17 - 4.08 (m, 1H), 3.81 (dd, *J* = 13.9, 4.3 Hz, 1H), 3.43 - 3.34 (m, 2H), 3.11 - 3.02 (m, 2H), 2.99 - 2.83 (m. 2H), 2.03 - 1.94 (m, 1H), 1.70 - 1.54 (m, 1H), 1.46 - 1.30 (m, 1H). | 353.85 | 354.05 |
| 135 | Starting with (S)-tert-butyl 3-((5-cyclopropyl4-(1-(pheaylsulfonyl)-1H-indol-3-yl)pyrimidin-2-yl)amino)piperidine-1-carboxylate (Example 4) following Example 1 and 2. | ¹H NMR (500 MHz, DMSO) δ 11.70 (brs, 1H), 8.65 (brs, 1H), 8.35 (brs, 1H), 8.30 (d, *J* = 2.9 Hz, 1H), 8.06 (s, 1H), 7.46 (d, *J* = 8.0 Hz, 1H), 7.22 - 7.10 (m, 2H), 6.81 (brs, 1H), 4.05 (brs, 1H), 3.23 (d, *J* = 11.5 Hz, 1H), 3.00 (d, *J* = 11.3 Hz, 1H), 2.71 - 2.61 (m, 2H), 2.07 -1.91 (m, 2H), 1.77 (brs, 1H), 1.65 - 1.46 (m, 2H), 0.99 - 0.90 (m, 2H), 0.64 - 0.55 (m, 2H). | 333.43 | 334.10 |
| 136 | Starting with (S)-tert-butyl 3-((5-chloro-4-(1-(phenylsulfonyl)-1H-indol-3-yl)pyrimidin-2-yl)amino)piperidine-1-carboxylate (Example 4) following Example 5 and 2. | ¹H NMR (500 MHz, DMSO) δ 11.83 (brs, 1H), 8.57 (brs, 1H), 8.46 (d, *J* = 3.0 Hz, 1H), 8.25 (s, 1H), 7.49 (d, *J* = 8.0 Hz, 1H), 7.25 -7.18 (m, 1H), 7.14 (t, *J* = 7.6 Hz, 1H), 7.08 (brs, 1H), 3.98 (brs, 1H), 2.95 (brs, 1H), 2.64 (brs, 1H), 2.20 (s, 3H), 1.99 -1.84 (m, 3H), 1.76 -1.67 (m, 1H), 1.57 (brs, 1H), 1.36 -1.26 (m, 1H). | 341.84 | 342.09 |
| 144 | Starting with 2,4,5-trichloropyrimidine and 7-fluoroindole following Example 1 and Example 6. | ¹H NMR (500 MHz, DMSO) δ 12.13 (s, 1H), 8.32 (s, 1H), 8.17 (s, 1H), 8.12 (d, *J* = 7.8 Hz, 1H), 7.84 (s, 1H), 7.10 - 6.98 (m, 2H), 6.76 (d, *J* = 7.9 Hz, 1H), 4.01 - 3.89 (m, 1H), 3.17 (dd, *J* = 11.8, 3.2 Hz, 1H), 2.92 (d, *J* = 12.3 Hz, 1H), 2.69 (q, *J* = 7.4 Hz, 2H), 2.58 (dd, *J* = 20.0, 8.7 Hz, 2H), 2.00 - 1.89 (m, 1H), 1.77 - 1.61 (m, 1H), 1.57 -1.43 (m. 2H), 1.14 (t, *J* = 7.5 Hz. 3H). | 339.41 | 340.05 |
| 145 | Starting with (S)-5-chloro-4-(1-(phenylsulfonyl)-1H-indol-3-yl)-N-(piperidin-3-yl)pyrimidin-2-amine (Example 5) and isobutyraldehylde following Example 5 and 2. | ¹H NMR (500 MHz, DMSO) δ 11.83 (s, 1H), 8.55 (s, 1H), 8.46 (d, *J* = 3.0 Hz, 1H), 8.25 (s, 1H), 7.49 (d, *J* = 7.9 Hz, 1H), 7.24 - 7.17 (m, 1H), 7.13 (t, *J* = 7.4 Hz, 1H), 7.03 (s, 1H), 4.05 (s, 1H), 2.95 (s, 1H), 2.75 - 2.66 (m, 1H), 2.11 - 2.04 (m, 2H), 1.93 (s, 3H), 1.82 - 1.65 (m, 2H). 1.61 - 1.49 (m, 1H), 1.40 - 1.32 (m, 1H), 0.87 - 0.85 (m, 6H). | 383.92 | 384.13 |
| 147 | Starting with 5-iodo-2,4-dichloropyrimidine and indole following Example 3, land 2. | ¹H NMR (500 MHz, DMSO) δ 11.94 (brs, 1H), 8.52 (s, 1H), 8.46 (bra, 2H), 8.27 (brs, 1H), 7.47 (d, *J* = 8.0 Hz, 1H), 7.20 - 7.15 (m, 1H), 7.14 - 7.10 (m, 2H), 3.86 (brs, 1H), 3.09 (dd, *J* = 11.6. 3.0 Hz, 1H), 2.84 (d, *J* = 11.9 Hz, 1H), 2.47 (brs, 1H), 2.38 (brs, 1H), 1.95 (brs, 1H), 1.72 - 1.62 (m, 1H), 1.54 - 1.42 (m, 2H). | 419.26 | 419.94 |
| 148 | Starting with (S)-5-chloro-4-(1-(phenylsulfonyl)-1H-indol-3-yl)-N-(piperidin-3-yl)pyrimidin-2-amine (Example 5) following Example 7 and 2. | ¹H NMR (500 MHz, DMSO) δ 11.77 (s, 1H), 8.50 (s, 1H), 8.40 (s, 1H), 8.19 (d, *J* = 15.0 Hz. 2H), 7.42 (d, *J* = 8.1 Hz, 1H), 7.16 - 7.13 (m, 1H), 7.08 - 7.05 (m, 1H), 6.97 (s, 1H), 3.91 (s, 1H), 2.70-2.64 (m, 2H), 2.06 (t, *J* = 10.3 Hz, 1H), 2.02-1.97 (m, 1H), 1.83 (s, 1H), 1.65-1.63 (m, 1H), 1.44 (s, 1H), 1.31-1.25 (m, 1H), 0.92-0.89 (m, 6H). | 369.89 | 370.14 |
| 149 | Starting with (S)-5-chloro-4-(1-(phenylsulfonyl)-1H-indol-3-yl)-N-(piperidin-3-yl)pyrimidin-2-amine (Example 5) following Example 8 and 2. | ¹H NMR (500 MHz, DMSO) δ 11.83 (s, 1H), 8.55 (s, 1H), 8.47 (d, *J* = 2.9 Hz, 1H), 8.25 (s, 1H), 7.49 (d, *J* = 8.0 Hz, 1H), 7.23 - 7.19 (m, 1H), 7.16 - 7.13 (m, 1H), 7.06 (s, 1H), 3.97 (s, 1H), 3.44 (t, *J* = 5.9 Hz, 4H), 3.19 (s, 3H), 2.77 (d, *J=* 11.0 Hz, 1H), 2.52 (s, 1H), 2.01 - 1.97 (m, 2H), 1.91 (s, 1H), 1.72 - 1.68 (m, 1H), 1.54 (s, 1H), 1.37 - 1.30 (m, 1H). | 385.89 | 386.10 |
| 157 | Starting with 3-(2-chloro-5-iodopyrimidin-4-yl)-1-(phenylsulfonyl)-1H-indole and (S)-*tert*-butyl 3-aminopiperidine-1-carboxylate following Example 4, 9, 1 and 2. | ¹H NMR (500 MHz, DMSO) δ 12.02 (d, *J* = 21.5 Hz, 1H), 8.71 (d, *J* = 7.7 Hz, 1H), 8.62 (d, *J* = 34.2 Hz, 1H), 8.52 - 8.47 (m, 1H), 8.29 (s, 1H), 8.05 (dd, *J* = 15.3, 7.9 Hz, 1H), 7.53 (m, 1H), 7.26 - 7.17 (m, 2H), 4.04 (d, *J* = 41.0 Hz, 1H), 2.93 (d, *J* = 11.4 Hz, 1H), 2.66 - 2.54 (m, 2H), 2.09 - 2.07 (m, 1H), 1.95 (s, 1H), 1.74 (d, *J* = 13.0 Hz, 1H), 1.58 - 1.49 (m, 2H).* rotamers confirmed by VT ¹H NMR (500 MHz, DMSO, 100C) δ 8.60 - 8.52 (m, 2H), 8.47 (s, 1H), 8.17 (s, 1H), 7.52 (d, *J =* 7.9 Hz, 2H), 7.22 (dt, *J =* 14.9, 7.1 Hz, 2H), 4.03 (s, 1H), 2.88 - 2.83 (m, 2H), 2.65 - 2.56 (m, 2H), 2.05 -1.98 (m, 1H), 1.76 -1.68 (m, 1H), 1.63 - 1.49 (m, 2H). | 318.38 | 318.95 |
| 158 | Starting from 3-(5-bromo-2-chloropyrimidin-4-yl)-1-(phenylsulfonyl)-1H-indole (Example 2) following Example 1, 2, 4 and 10. | ¹M NMR (500 MHz, D₂O) δ 8.40 (s, 1H), 7.92 (d, *J* = 8.0 Hz, 1H), 7.80 (s, 1H), 7.64 (d, *J* = 8.2 Hz, 1H), 7.38 - 7.34 (m, 1H), 7.30 - 7.25 (m, 1H), 4.28 - 4.23 (m, 1H), 3.66 (q, *J* = 10.9 Hz, 2H), 3.57 (dd, *J* = 12.6, 3.7 Hz, 1H), 3.36 (dt, *J* = 12.9, 4.6 Hz, 1H), 3.14 - 3.05 (m. 2H), 2.24 - 2.16 (m, 1H), 2.14 - 2.06 (m, 1H), 1.94 -1.83 (m, 1H), 1.81 - 1.71 (m, 1H). ¹⁹F NMR (471 MHz, DMSO) δ -63.89 (s). | 375.39 | 376.26 |
| 159 | Starting from 3-(2,5-dichloropyrimidin-4-yl)-1-(phenylsulfonyl-1H-indole and (S)-5-aminopiperidin-2-one (Tetrahedron Letters, 1995, 36, 8205-8) following Example 1 and 2. | ¹H NMR (500 MHz, DMSO) δ 11.86 (s, 1H), 8.59 (br s, 1H), 8.48 (d, *J* = 2.7 Hz, 1H), 8.30 (d, *J* = 2.2 Hz, 1H), 7.49 (d, *J* = 8.0 Hz, 1H), 7.44 (s, 1H), 7.38 (d, *J* = 7.1 Hz, 1H), 7.21 (t, *J* = 7.5 Hz, 1H), 7.15 (t, *J* = 7.5 Hz, 1H), 4.17 (br s, 1H), 3.47 - 3.40 (m, 1H), 3.19 - 3.11 (m, 1H), 2.37 (dt, *J* = 10.7, 5.6 Hz, 1H), 2.33 - 2.22 (m, 1H), 2.12 - 2.00 (m, 1H), 1.91 -1.81 (m, 1H). | 341.79 | 341.90 |
| 162 | Starting with (S)-tert-butyl 3-((4-(7-(pyridin-4-yl)-1H-indol-3-yl)-5-(trifluoromethyl)pyrimidin-2-yl)amino)piperidine-1-carboxylate (Example 11) following Example 1. | ¹H NMR (500 MHz, DMSO) δ 11.82 (brs, 1H), 8.72 (d, *J* = 5.5 Hz, 2H), 8.64 - 8.56 (m, 1H), 8.36 - 8.28 (m, 2H), 7.88 (t, *J* = 9.1 Hz, 1H), 7.79 (d, *J* = 11.4 Hz, 1H), 7.68 (dd, *J* = 4.4, 1.6 Hz, 2H), 7.36 - 7.28 (m, 2H), 4.05 (brs, 1H), 3.18 (brs, 1H), 2.98 - 2.88 (m, 1H), 2.67 - 2.55 (m, 2H), 2.05 - 1.93 (m, 1H), 1.74 (bis, 1H), 1.61 - 1.47 (m, 2H). | 438.45 | 439.02 |
| 163 | Starting with (S)-tert-butyl 3-((4-(7-bromo-1H-indol-3-yl)-5-(trifluoromethyl)pyrimidin-2-yl)amino)piperidine-l-carboxylate (Example 11) following Example 1. | ¹H NMR (500 MHz, DMSO) δ 12.01 (s, 1H), 8.60 (d, *J* = 21.2 Hz, 1H), 8.41 - 8.16 (m, 2H), 7.88 (t, *J* = 8.6 Hz, I H.), 7.80 (s, 1H), 7.45 (d, *J* = 7.5 Hz, 1H), 7.13-7.09 (m, 1H), 4.01 (s, 1H), 3.15 (m, 2H), 2.94 - 2.88 (m, 1H), 2.59 - 2.55 (m, 1H), 1.99 - 1.94 (m, 1H), 1.72 (s, 1H), 1.53 -1.49 (m, 2H). | 440.26 | 441.92 |
| 164 | Starting with (S)-tert-butyl 3-((4-(7-(1-methyl-1H-pyrazol-4-yl)-1H-indol-3-yl)-5-(trifluoromethyl)pyrimidin-2-yl)amino)piperidine-1-carboxylate (Example 12) following Example 1. | ¹H NMR (500 MHz, DMSO) δ 11.41 (d, *J* = 10.7 Hz, 1H), 8.51 (d, *J* = 22.9 Hz, 1H), 8.26 - 8.19 (m, 1H), 8.14 (s, 1H), 8.08 (d, *J* = 7.8 Hz, 1H), 7.85 (s, 1H), 7.75 - 7.65 (m, 2H), 7.24 (d, *J* = 7.1 Hz, 1H), 7.11 (t, *J* = 7.7 Hz, 1H), 3.99 - 3.90 (m, 1H), 3.88 (s, 3H), 3.08 - 3.00 (m, 2H), 2.83 - 2.75 (m, 1H), 1.97 -1.83 (m, 2H), 1.65 -1.58 (m, 1H), 1.50-1.35 (m, 2H). | 441.45 | 442.01 |
| 168 | Starting with (S)-*tert*-butyl 3-((4-(7-(diethoxyphosphoryl)-1H-indol-3-yl)-5-(trifluoromethyl)pyrimidin-2-yl)amino)piperidine-1-carboxylate (Example 13) following Example 1. | Rotamer 1: ¹H NMR (500 MHz, DMSO) δ 11.33 (brs, 1H), 8.68 (d, *J* = 8.1 Hz, 1H), 8.61 (s, 1H), 8.37 (s, 1H), 7.89 (s, 1H), 7.82 (d, *J* = 8.1 Hz, 1H), 7.58 - 7.51 (m, 1H), 7.34 - 7.27 (m, 1H), 4.15 - 4.00 (m, 4H). 3.92 (bra, 1H), 3.11 - 3.06 (m, 1H), 2.83 (brs, 1H), 2.48 - 2.44 (m, 2H), 2.03 - 1.91 (m, 1H), 1.70 -1.64 (m, 1H), 1.55 - 1.40 (m, 2H), 1.28 -1.22 (m, 6H). | 497.45 | 498.13 |
| | | Rotamer 2: ¹H NMR (500 MHz, DMSO) δ 11.33 (brs, 1H), 8.56 (s, 1H), 8.52 (d, *J* = 7.7 Hz, 1H), 8.37 (s, 1H), 7.89 (s, 1H), 7.82 (d, *J* = 8.1 Hz, 1H), 7.58 - 7.51 (m, 1H), 7.34 - 7.27 (m, 1H), 4.15 - 4.00 (m, 4H), 3.92 (brs, 1H), 3.11 - 3.06 (m, 1H), 2.83 (brs, 1H), 2.48 - 2.44 (m, 2H), 2.03 - 1.91 (m, 1H), 1.70-1.64 (m, 1H), 1.55 - 1.40 (m, 2H), 1.28 - 1.22 (m, 6H). | | |
| 170 | Starting with (S)-tert-butyl 3-((4-(7-bromo-1H-indol-3-yi)-5-(trifluoromethyl)pyrimidin-2-yl)amino)piperidine-1-carboxylate (Example 11) and (1-(tert-butoxycarbonyl)-1H-pyrazol-4-yl)boronic acid following Example 11 and 1. | ¹H NMR (500 MHz, DMSO) δ 11.49 (d, *J* = 7.9 Hz, 1H), 8.59 (d, *J* = 21.3 Hz, 1H), 8.31 - 8.29 (m, 2H), 8.15 - 8.14 (m, 2H), 7.86 - 7.82 (m, 1H), 7.78 (s, 1H), 7.36 (d, *J* = 7.2 Hz, 1H), 7.20 - 7.17 (m, 1H), 4.11-3.96 (m, 1H), 3.20-3.14 (m, 2H), 2.94 - 2.90 (m, 1H), 2.63 - 2.57 (m, 2H), 2.03 - 1.95 (m, 1H), 1.73 (s, 1H), 1.55 - 1.41 (m, 2H). | 427.43 | 428.07 |
| 171 | Starting with (S)-tert-butyl 3-((4-(7-bromo-1H-indol-3-yl)-5-(trifluoromethyl)pyrimidin-2-yl)amino)piperidine-1-carboxylate (Example 11) and 1,3-dimthyl-4-(4,4,5,5-tetramethyl-1,3,2-dioxaborolan-2-yl)-1H-pyrazole following Example 11 and 1. | ¹H NMR (500 MHz, DMSO) δ 11.46 (s, 1H), 8.58 (d, *J =* 20.1 Hz, 1H), 8.36 - 8.16 (m, 2H), 7.89 (s, 1H), 7.83 (d, *J =* 7.9 Hz, 1H), 7.73 (d, *J* = 5.5 Hz, 1H), 7.23-7.17 (m, 1H), 7.09 (d, *J* = 7.1 Hz. 1H), 4.12-3.97 (m, 1H), 3.86 (s, 3H), 3.22-3.13 (m, 1H), 2.96 - 2.91 (m. 1H), 2.63 - 2.60 (m. 2H), 2.18 (s, 3H), 2.04 - 1.95 (m, 1H), 1.74 (s, 1H), 1.54 - 1.52 (m, 2H). | 455.48 | 456.18 |
| 172 | Starting from (S)-*tert*-butyl 3-((4-(7-(4-methyl-1H-imidazol-1-yl)-1H-indol-3-yl)-5-(trifluoromethyl)pyrimidin-2-yl)amino)piperidine-l-carboxylate (Example 14) following Example 1 | ¹H NMR (500 MHz, DMSO) δ 11.83 (brs, 1H), 8.64 - 8.58 (m, 1H), 8.47 - 8.42 (m, 0.5H), 8.30 (s, 1H), 8.23 (d, *J* = 6.5 Hz, 0.5H), 7.95 (d, *J* = 1.3 Hz, 1H), 7.94 - 7.90 (m, 1H), 7.76 (d, *J* = 14.9 Hz, 1H), 7.34 - 7.31 (m, 1H), 7.30 - 7.21 (m, 2H), 4.06 (brs, 1H), 3.24 - 3.13 (m. 1H), 3.00 - 2.88 (m, 1H), 2.68 - 2.55 (m, 2H), 2.24 (s, 3H), 2.04 - 1.92 (m, 1H), 1.80-1.70 (m, 1H), 1.60 -1.46 (m, 2H). | 441.45 | 442.08 |
| 175 | Starting from 3-methyl-4-(4,4,5,5-tetramethyl-1,3,2-dioxaborolan-2-yl)-1-((2-(trimethylsilyl)ethoxy)meth yl)-1H-pyrazole and (S)-tert-butyl 3-((4-(7-bromo-1H-indol-3-yl)-5-(trifluoromethyl)pyrimidin-2-yl)amino)piperidine-l-carboxylate (Example 11) following Example 11 and 1. | ¹H NMR (500 MHz, DMSO) δ 11.42 (brs, 1H), 8.63 - 8.55 (m, 1H), 8.45 - 8.30 (m, 1H), 8.37 (d, *J* = 7.8 Hz, 1H), 8.17 (d, *J* = 7.9 Hz, 1H), 7.87 (brs, 1H), 7.78 - 7.70 (m, 2H), 7.24 - 7.16 (m, 1H), 7.10 (d, *J* = 6.7 Hz, 1H), 4.11 (brs, 1H), 3.23 (brs, 1H), 2.98 (brs, 1H), 2.75 - 259 (m, 2H), 2.26 (s, 3H), 2.10- 1.95 (m, 1H), 1.77 (brs, 1H), 1.64 - 1.47 (m, 2H). | 441.45 | 442.19 |
| 176 | Starting from (S)-*tert*-butyl 3-((4-(7-(3-methyl-2-oxoimidazolidin-1-yl)-1H-indol-3-yl)-5-(trifluoromethyl)pyrimidin-2-yl)amino)pipendine-1-carboxylate (Example 15) following Example 1. | ¹H NMR (500 MHz, DMSO) shows 2 rotamers. Rotamer 1: ¹H NMR (500 MHz, DMSO) δ 11.47 (brs, 1H), 8.59 (s, 1H), 8.35 (brs, 1H), 8.24 (d, *J* = 7.8 Hz, 1H), 7.82 - 7.76 (m, 2H), 7.17 - 7.10 (m, 1H), 7.03 - 6.98 (m, 1H), 4.00 (brs, 1H), 3.90 - 3.85 (m, 2H), 3.57 - 3.51 (m, 2H), 3.18 - 3.10 (m, 1H), 2.93 - 2.85 (m, 1H), 2.83 (s, 3H), 2.63 -2.53 (m, 2H), 2.03 - 1.91 (m, 1H), 1.71 (brs, 1H), 1.59 -1.43 (m, 2H). | 459.47 | 460.21 |
| | | Rotamer 2: ¹H NMR (500 MHz, DMSO) δ 11.47 (brs, 1H), 8.54 (s, 1H), 8.35 (bra, 1H), 8.06 (d, *J* = 7.8 Hz, 1H), 7.82 - 7.76 (m, 2H), 7.17 - 7.10 (m, 1H), 7.03 - 6.98 (m, 1H), 4.00 (brs, 1H), 3.90 - 3.85 (m, 2H), 3.57 - 3.51 (m, 2H), 3.18 - 3.10 (m, 1H), 2.93 - 2.85 (m, 1H), 2.83 (s, 3H), 2.63 - 2.53 (m, 2H), 2.03 -1.91 (m, 1H), 1.71 (brs, 1H), 1.59 - 1.43 (m, 2H). | | |
| 179 | Starting from (S)-tert-butyl 3-((4-(7-(ethoxy(methyl)phosphoryl) -1H-indol-3-yl)-5-(trifluoromethyl)pyrimidin-2-yl)amino)piperidine-1-carboxylate (Example 16) following Example 1. | ¹H NMR (500 MHz, CDCl₃) δ 11.43 (brs, 1H), 8.70 - 8.44 (m, 2H), 8.32 (s, 1H), 7.95 - 7.86 (m, 2H), 7.55 (dd, *J* = 13.2, 7.3 Hz, 1H), 7.31 (dd, *J* = 15.0. 7.2 Hz, 1H), 4.10 - 3.94 (m, 2H), 3.83 - 3.75 (m, 1H), 3.21 - 3.13 (m, 1H), 2.93 (t, *J* = 12.4 Hz, 1H), 2.65 - 256 (m, 2H), 2.05 - 1.93 (m, 1H), 1.80 - 1.69 (m, 4H), 1.60 - 1.46 (m, 2H), 1.21 (q, *J* = 6.9 Hz, 3H). ³¹P NMR (203 MHz, DMSO) δ 42.60 (s). | 467.42 | 468.17 |
| 180 | Starting from (S)-*tert*-butyl 3-((4-(7-(pyridazin-4-yl)-1H-indol-3-yl)-5-(trifluoromethyl)pyrimidin-2-yl)amino)piperidine-l-carboxylate (Example 17) following Example 1. | ¹H NMR (500 MHz, DMSO) δ 12.02 (brs, 1H), 9.56 (dd, *J* = 2.4, 1.2 Hz, 1H), 9.36 (d, *J* = 4.9 Hz, 1H), 8.63 - 8.60 (m, 1H), 8.56 (d, *J* = 7.9 Hz, 0.5H), 8.35 (d, *J* = 7.9 Hz, 0.5H). 8.31 (brs, 1H), 7.99 (dd, *J* = 5.3,2.5 Hz, 1H), 7.94 - 7.90 (m, 1H), 7.82 (d, *J* = 14.1 Hz, 1H), 7.47 - 7.42 (m, 1H), 7.37 -7.31 (m, 1H), 4.06 (brs, 1H), 3.19 (brs, 1H), 3.0 - 2.90 (m, 1H), 2.71 - 2.56 (m, 2H), 2.06 - (m, 1H), 1.75 (brs, 1H), 1.61 - 1.46 (m, 2H). | 439.44 | 440.20 |
| 184 | Starting from 5,6-difluoroindole and 4-chloro-2-(methylthio)-5-(trifluoromethyl)pyrimidine 4-chloro-2-(methylthio)-5-(trifluoromethyl)pyrimidine (U.S. Pat. AppL Publ., 20130017194, 17 Jan 2013) following Example 18 and 1. | ¹H NMR (500 MHz, DMSO) δ 11.98 (bra, 1H), 8.59 - 8.52 (m, 1H), 8.46 (dd, *J* = 12.1, 8.6 Hz, 0.5H; rotamer 1), 8.32 (brs, 1H), 8.16 (dd, *J* = 11.7, 8.5 Hz, 0.5H; rotamer 2), 7.96 - 7.92 (m, 1H), 7.92 -7.89 (m, 0.5H; rotamer 1), 7.83 (d, *J* = 8.4 Hz, 0.5H; rotamer 2), 7.57 - 7.47 (m, 1H), 3.95 (brs, 1H), 3.14 - 3.07 (m, 1H), 2.93 - 2.82 (m, 1H), 2.58 - 2.50 (m, 2H), 2.07 - 1.93 (m, 1H), 1.74 - 1.67 (m, 1H), 1.56 - 1.44 (m, 2H). | 397.35 | 398.88 |
| 185 | Starting with (S)-tert-butyl 3-((4-(7-bromo-1H-indol-3-yi)-5-(trifluoromethyl)pyrimidin-2-yl)amino)piperidine-1-carboxylate (Example 11) and 4,4,5,5-tetramethyl-2-(1,4-dioxaspiro[4.5]dec-7-en-8-yl)-1,3,2-dioxaborolane following Example 1, 11 and 19. | ¹H NMR (500 MHz, DMSO) δ 11.82 - 11.75 (m, 1H), 8.57 (s, 0.5H), 8.52 (s, 0.5H), 8.37 (s, 1H), 8.21 (d, *J* = 7.6 Hz, 0.5H), 8.06 (d, *J* = 7.8 Hz, 0.5H), 7.75 (brs, 1H), 7.72 (t, *J* = 9.6 Hz, 1H), 7.11 - 7.03 (m, 2H), 4.60 (brs, 1H), 3.96 (brs, 1H), 3.60 - 3.50 (m, 3H), 3.08 (brs, 1H), 3.04 - 2.94 (m, 1H), 2.89 - 2.80 (m, 1H), 2.01 - 1.83 (m, 5H), 1.72 - 1.54 (m, 3H), 1.53 - 1.37 (m, 4H). | 459.51 | 460.98 |
| 189 | Starting with (S)-tert-butyl 3-((5-methoxy-4-(1-(phenylsulfonyl)-1H-indol-3-yl)pyrimidin-2-yl)amino)piperidine-l-carboxylate (Example 20) following Example 1 and 2. | ¹H NMR (500 MHz, DMSO) δ 11.69 (s, 1H), 8.78 (d, *J* = 7.8 Hz, 1H), 8.36 (s. 1H), 8.30 - 8.28 (m, 1H), 8.07 (s, 1H), 7.46 (d, *J* = 7.9 Hz. 1H), 7.16 (dt, *J* = 23.0. 7.2 Hz, 2H), 6.52 (d, *J* = 7.8 Hz, 1H), 3.97 - 3.90 (m, 1H), 3.89 (s, 3H), 2.94 (d. *J* = 12.4 Hz, 1H), 2.60 (dd, *J* = 21.1, 10.7 Hz. 2H), 2.04 - 1.97 (m, 1H), 1.78 -1.71 (m, 1H), 1.61 - 1.43 (m, 3H). | 323.39 | 323.39 |
| 190 | Starting with (S)-N-(6,6-dimethylpiperidin-3-yl)-4-(1-(phenylsulfonyl)-1H-indol-3-yl)-5-(trifluoromethyl)pyrimidin-2-amine (Example 21) following Example 2. | ¹H NMR (500 MHz, DMSO) δ 11.82 (br s, 1H), 8.54 (d, *J* = 19.2 Hz, 1H), 8.40 (s, 2H), 7.82 (s, 1H), 7.72 (d, *J* = 8.1 Hz, 1H), 7.49 (t, *J* = 9.0 Hz, 1H), 7.20 (t, *J* = 7.5 Hz, 1H), 7.14 (t, *J* = 7.4 Hz, 1H), 4.00 - 3.87 (m, 1H), 2.95 (dd, *J* = 12.6, 4.1 Hz,1H), 2.74 (dd, *J* = 22.9, 13.0 Hz, 1H), 1.93 - 1.75 (m, 1H), 1.75 - 1.60 (m, 1H), 1.60 - 1.52 (m, 1H), 1.44 - 1.34 (m, 1H), 1.18 - 1.01 (m, 6H). | 389.42 | 390.2 |
| 191 | Starting with (S)-tert-butyl 3-((4-(7-bromo-1H-indol-3-yl)-5-(trifluoromethyl)pyrimidin-2-yl)amino)piperidine-l-carboxylate (Example 11) following Example 23 and 1. | ¹H NMR (500 MHz, DMSO) δ 11.67 (d, *J* = 11.0 Hz, 1H). 8.56 (d, *J* = 22.1 Hz, 1H), 8.27 (s, 1H), 8.13 (dd, *J* = 71.2,7.8 Hz, 1H), 7.76-7.75 (m, 2H), 7.08 (t, *J* = 7.2 Hz, 1H), 7.03-7.01 (m, 1H), 4.32 (s, 1H), 4.00 (s, 1H), 3.16-3.14 (m, 2H), 2.93-2.87 (m, 3H), 2.55 (m, 1H), 2.00-1.93 (m, 1H), 1.79 - 1.74 (m, 2H), 1.70 (s, 1H), 1.55-1.48 (m, 2H), 1.21 (s, 6H). | 447.5 | 448.31 |
| 192 | Starting with (S)-tert-butyl 3-((4-(7-(4-oxocycliohexyl)-1H-indol-3-yl)-5-(trifluoromethyl)pyrimidin-2-yl)amino)piperidine-1-carboxylate (Example 19) following Example 22 and 1 for the final step upon which Compound 192 and Compound 193 were separated. | ¹H NMR (500 MHz, DMSO) δ 11.79 (brs, 1H), 8.56 (s, 0.5H), 8.52 (s, 0.5H), 8.44 (brs, 1H), 8.21 (s, 0.5H), 8.07 (s, 0.5H), 7.74 (brs, 1H), 7.71 - 7.63 (m, 1H), 7.11 - 7.07 (m, 2H), 4.40 (brs, 1H), 3.94 (brs, 1H), 3.04 (brs, 2H), 2.79 (brs, 1H), 1.85 - 1.78 (m, 2H), 1.72 - 1.59 (m, 8H), 1.50 - 1.35 (m, 4H), 1.24 (s, 3H). | 473.53 | 474.31 |
| 193 | Starting with (S)-tert-butyl 3-((4-(7-(4-oxocyclohexyl)-1H-indol-3-yl)-5-(trifluoromethyl)pyrimidin-2-yl)amino)piperidine-1-carboxylate (Example 19) following Example 22 and 1 for the final step upon which Compound 192 and Compound 193 were separated. | ¹H NMR (500 MHz, DMSO) δ 11.74 (brs, 1H), 8.57 (s, U.5H), 8.52 (s, 0.5H), 8.43 (brs, 1H), 8.20 (d, *J* = 7.2 Hz 0.5H), 8.06 (d, *J* = 7.5 Hz, 0.5H), 7.74 (brs, 1H), 7.71 - 7.63 (m, 1H), 7.11 - 7.04 (m, 2H), 4.10 (brs, 1H), 3.93 (brs, 1H), 3.08 - 3.04 (m, 1H), 2.97 (brs, 1H), 2.81 (brs, 1H), 2.00 - 1.85 (m, 4H), 1.72 -1.60 (m, 5H), 1.55 - 1.40 (m, 5H), 1.23 (s, 3H). | 473.53 | 474.31 |
| 194 | Starting with 3-(2-chloro-5-iodapyrimidin-4-yl)-1-(phenylsulfonyl)-1H-iodole and (S)-*tert*-butyl 3-aminopiperidine-1-carboxylate following Example 5 and 24 (A). | ¹H NMR (500 MHz, DMSO) δ 11.80 (brs, 1H), 8.78 (d, *J* = 2.6 Hz, 1H), 8.57 (brs, 1H), 8.33 (brs, 2H), 7.48 (d, *J* = 7.9 Hz, 1H), 7.40 (brs, 1H), 7.24 -7.12 (m, 2H), 4.56 (s, 1H), 4.00 (brs, 1H), 3.21 (d, *J* = 10.6 Hz, 1H), 2.96 (d, *J* = 12.1 Hz. 1H), 2.71 - 2.57 (m, 2H), 2.13 - 1.92 (m, 1H), 1.82 - 1.72 (m, 1H), 1.54 (brs, 2H). | 317.39 | 318.20 |
| 195 | Starting with 3-(2-chloro-5-iodopyrimidin-4-yl)-1-(phenylsulfonyl)-1H-indole and (S)-*tert*-butyl 3-aminopiperidine-1-carboxylate following Example 5 and 24 (B). | ¹H NMR (500 MHz, DMSO) δ 8.85 - 8.55 (m, 2H), 8.34 (brs, 2H), 7.60 - 7.40 (m, 2H), 7.30 - 7.18 (m, 2H), 4.51 (s, 1H), 3.97 (brs, 1H), 3.89 (s, 3H), 3.19 (brs, 1H), 2.93 (d, *J* = 11.0 Hz, 1H), 2.61 - 2.53 (m, 2H), 2.10 - 1.90 (m, 1H), 1.73 (brs, 1H), 1.52 (brs, 2H). | 331.41 | 332.23 |
| 196 | Example 40 | ¹H NMR (400 MHz, CDCl₃) δ 8.39 (s, 1H), 8.31 (br d, *J* = 7.72 Hz, 1 H), 8.24 (s, 1 H), 7.15 (t, *J* = 7.83 Hz, 1 H), 7.00 (d, *J =* 7.06 Hz, 1 H), 4.25 (br s, 1 H), 3.48-3.59 (m, 5 H), 3.34-3.41 (m, 4 H), 3.28 (br s, 1 H), 2.97-3.11 (m, 2H), 219 (br d, *J* = 13.23 Hz, 1 H), 2.08 (br dd, *J =* 9.26, 5.51 Hz, 1 H), 1.82-1.93 (m, 1 H), 1.69-1.79 (m, 1 H) | 460.14 | 461.1 |
| 196 | Example 126 | 1H NMR (400 MHz, MeOD) δ 8.66 (s, 2H), 8.22 (br. s., 1H), 8.04 (br.s., 1H), 7.76 (br.s., 1H), 4.63 - 4.30 (m, 1H), 3.68 - 3.60 (m, 1H), 3.46 (br.d, J = 5.6, 8.4 Hz, 2H), 3.28 (br.d., 7 = 10.5 Hz, 2H), 3.22 - 3.12 (m. 2H), 3.08 (br.d., J = 10.9 Hz, 1H), 2.27 (br.s.. 1H), 2.19 - 2.09 (m, 1H), 1.94 (br.d., 1 = 11.4 Hz, 1H), 1.88 - 1.74 (m, 3H), 1.25 (s, 3H) | 524.18 | 525.10 |
| 197 | Example 41 | ¹H NMR (400 MHz, MeOD,) δ 8.58 (br s, 1 H), 8.35 (br s, 1 H), 8.10 (br s, 1 H), 7.85 (br s, 1 H), 4.30 (br s, 1 H), 3.51 (br s, 1 H), 3.28 (br s, 1 H), 2.88-3.05 (m, 2 H), 2.18 (br s, 1 H), 2.05 (br d, J = 14.77 Hz, 1 H), 1.67-1.91 (m, 2 H) | 404.14 | 405.2 |
| 198 | Starting with (S)-tert-butyl 3-((4-(7-(1,4-dioxaspiro[4.5]dec-7-en-8-yl)-1H-indol-3-yl)-5-(trifluoromethyl)pyrimidin-2-yl)amino)piperidine-1-carboxylate and following Example 19. | ¹H NMR (500 MHz, DMSO) δ 11.51 - 11.44 (m, 1H), 8.58 (s, 0.5H; rotamer 1), 8.53 (s, 0.5H; rotamer 2), 8.41 (brs, 2H), 8.27 (d, *J* = 8.0 Hz, 0.5H; rotamer 1), 8.12 (d, *J* = 7.7 Hz, 0.5H; rotamer 2), 7.77 - 7.71 (m, 2H), 7.14 - 7.08 (m, 1H), 7.06 (s, 0.5H; rotamer 1), 7.5 (s, 0.5H; rotamer 2), 5.89 (brs, 1H), 3.97 (brs, 1H), 3.88 (brs, 1H), 3.60 - 3.48 (m, 4H), 3.10 (brs, 1H), 2.84 (brs, 1H), 2.17 - 2.10 (m, 1H), 2.04 -1.89 (m, 2H), 1.73 - 1.63 (m, 2H), 1.55 -1.40 (m, 3H). | 457.49 | 458.27 |
| 199 | Example 42 | ¹H NMR (400 MHz, MeOD) δ 8.59 (s, 1 H) 8.49 (s. 1 H) 8.34 (br, 1 H) 8.12 - 8.02 (m, 2 H) 7.70 (br, J = 7.72 Hz, 1 H) 4.34 (br, 1 H) 3.55 (br, J = 9.92 Hz, 1 H) 3.14 (s, 3 H) 3.28 (s, 1 H) 3.05- 2.93 (m, 2 H) 2.22 - 2.01 (m, 2 H) 1.91 - 1.69 (m, 2 H) | 439.13 | 440.2 |
| 200 | Example 43 | ¹H NMR (400 MHz, MeOH) δ 8.59 (s, 1 H) 8.49 (br, 1 H) 8.36 (s, 1 H) 8.02- 7.97 (m, 2 H) 7.61 (br, *J* = 8.16 Hz, 1 H) 4.32 (br, 1 H) 3.54 (br, *J* = 10.14 Hz, 1 H) 3.28 - 3.25 (m, 1 H) 3.03- 2.94 (m, 2 H) 2.51 (s, 3 H) 2.24 - 2.02 (m, 2 H) 1.90 -1.70 (m, 2 H) | 454.14 | 455.2 |
| 201 | Example 44 | ¹H NMR (400 MHz, MeOH) δ 8.52 (br., 2 H) 8.43 - 8.40 (m, 1 H) 8.23 - 8.18 (m, 1 H) 7.44 (dd, *J=* 6.95,1.21 Hz, 1 H) 7.19 (m, 2H) 4.25 (br., 1 H) 3.61 (br., *J* = 12.02, 4.08 Hz, 1 H) 3.28 - 3.23 (m, 1 H) 2.75 - 2.48 (m, 2 H) 2.27 (br, *J* = 11.25 Hz, 1 H) 2.07- 1.95 (m, 1 H) 1.29 (q, *J* = 12.13 Hz, 1 H) 1.04 (d, *J* = 6.62 Hz, 3 H). | 341.14 | 342.2 |
| 202 | Example 45 | ¹H NMR (400 MHz, CDCl₃) δ8.57 (s, 1 H) 8.37 (br. s, 2H) 7.99 - 7.87 (m, 2 H) 7.71 (br. d, *J*=8.82 Hz, 1 H) 7.19 (s, 2 H) 4.35 (br. s, 1 H) 3.56 (br. d, *J*=9.48 Hz, 1 H) 3.38 - 3.30 (m, 1 H) 3.10 - 2.92 (m, 2 H) 2.26 - 2.01 (m, 2 H) 1.93 - 1.68 (m, 2 H) | 427.17 | 428.2 |
| 203 | Example 46 | ¹H NMR (400 MHz, CDCl₃) 88.70 (s, 1H), 8.37-8.30 (m, 1H), 8.28 (s, 1H), 7.55 (br d, *J* = 5.95 Hz, 1H), 7.38-7.25 (m, 2H), 4.86-4.78 (m, 1H), 4.33 (br s, 1H), 3.67 (br d, *J* = 13.23 Hz, 1H), 3.50 (br d, J = 13.23 Hz, 1H), 3.32 (br d, J = 1.98 Hz, 2H), 2.36-2.26 (m, 1H), 2.23-2.12 (m. 1H) | 377.15 | 378.1 |
| 204 | Example 47 | ¹H NMR (400 MHz, MeOD) δ 8.57 (s, 1 H), 8.44 - 8.25 (m, 2 H), 7.92 (br. s, 1 H) 7.67 (br. d, *J* = 7.06 Hz, 1 H) 7.35 (br. t, *J* = 6.95 Hz, 1 H), 4.50 (hr. s, 0.5 H) 4.36 (br. s, 1.5 H) 3.69 - 3.86 (m, 2 H) 3.49 - 3.65 (m, 2 H) 3.30 - 3.46 (m, 2 H) 2.90 - 3.12 (m, 2 H) 1.62 - 2.28 (m, 6 H) | 474.2 | 475.3 |
| 205 | Example 48 | ¹H NMR (400 MHz, MeOD) δ 8.57 (s, 1 H), 8.46 (m, 2 H), 7.92 (br. s, 1 H) 7.67 (br. d, J = 7.06 Hz, 1 H) 7.35 (br. t, 1 = 6.95 Hz, 1 H), 4.50 (br. s, 0.5 H) 4.36 (br. s, 1.5 H) 3.69 - 3.86 (m, 2 H) 3.49 - 3.65 (m, 2 H) 3.30 - 3.46 (m, 2 H) 2.90 - 3.12 (m, 2 H) 1.62 - 2.28 (m, 6 H) | 474.2 | 475.2 |
| 206 | Starting from 3-(5-bromo-2-chloropyrimidin-4-yl)-l-(phenylsulfonyl)-1H-indole following*Example 26 and then Example 1 and 2. | ¹H NMR (500 MHz, DMSO) δ 11.64 (brs, 1H), 8.35 (brs, 1H), 8.26 (d, *J* = 7.6 Hz, 1H), 8.18 (s, 1H), 7.82 (d, *J* = 2.6 Hz, 1H), 7.46 (d, *J* = 8.0 Hz. 1H), 7.17 (t, *J* = 7.5 Hz, 1H), 7.10 (t, *J* = 7.1 Hz, 1H), 6.86 (d, *J* = 7.8 Hz, 1H), 4.66 (t, *J* = 6.4 Hz, 1H), 4.56 (t, *J* = 6.4 Hz, 1H), 3.99 (brs, 1H), 3.18 (d, *J* = 11.8 Hz, 1H), 3.11 (t, *J* = 6.4 Hz, 1H), 3.06 (t, *J* = 6.4 Hz, 1H), 2.94 (d, *J* = 12.2 Hz, 1H), 2.65 - 2.56 (m, 2H), 1.97 (brs, 1H), 1.79 - 1.72 (m, 1H), 1.60 - 1.46 (m, 2H). ¹⁹F NMR (471 MHz, DMSO) δ -214.28 (s). | 339.41 | 340.21 |
| 207 | Starting with (S)-*tert*-butyl 3-((4-(7-(4-oxocyclohexyl)-1H-indol-3-yl)-5-(trifluoromethyl)pyrimidin-2-yl)amino)piperidine-1-carboxylate and following Example 27 and then Example 1 for the final step. | ¹H NMR (500 MHz, DMSO) δ 11.80 - 11.75 (m, 1H), 8.58 (s, 1H; rotamer 1), 8.53 (s, 1H; rotamer 2), 8.29 (s, 1H), 8.21 (d, *J* = 7.3 Hz, 1H; rotamer 1), 8.05 (d, *J* = 7.4 Hz, 1H; rotamer 2), 7.75 (brs, 2H), 7.14 - 7.05 (m, 2H), 4.39 (brs, 1H), 4.04 - 3.94 (m, 2H), 3.16 - 3.10 (m, 2H), 3.07 - 3.00 (m, 1H), 2.92 - 2.83 (m, 1H), 2.04 - 1.87 (m, 3H), 1.84 -1.78 (m, 2H), 1.75 -1.56 (m, 5H), 1.54 - 1.45 (m, 2H), 1.42 - 1.31 (m, 1H). | 459.51 | 460.32 |
| 208 | Starting with (S)-tert-butyl 3-((5-iodo-4-(1-(jphenylsulfonyl)-1H-indol-3-yl)pyrimidin-2-yl)amino)piperidine-1-carboxylate and tributyl(1-ethoxyvinyl)stannane following Example 26 (Step 1), and then Example 1 and 2. | ¹H NMR (500 MHz, DMSO) δ 11.85 -11.70 (m, 1H), 8.51 (brs, 1H), 8.37 (brs, 1H), 8.09 (brs, 1H; rotamer 1), 7.99 (brs, 1H; rotamer 2), 7.79 - 7.72 (m, 1H), 7.69 (d, *J* = 7.9 Hz, 1H), 7.50 - 7.44 (m, 1H), 7.18 (t, *J* = 7.5 Hz, 1H), 7.13 (brs, 1H), 4.09 (brs, 1H), 3.26 - 3.18 (m, 1H), 3.02 - 2.95 (m, 1H), 2.72 - 2.60 (m, 2H), 2.24 (s. 3H), 2.05 - 1.91 (m, 1H), 1.82 - 1.74 (m, 1H), 1.64 - 1.49 (m, 2H). | 335.4 | 336.28 |
| 209 | Starting with 4,4-dimethylpiperidine-2,6-dione and following Example 28 (compound **A**). | ¹H NMR (500 MHz, DMSO) δ 11.85 - 11.75 (m, 1H), 8.57 - 8.52 (m. 1H), 8.39 (d, *J* = 8.1 Hz, 0.5H; rotamer 1), 8.35 (br s, 1H), 8.2.5 (d, *J* = 7.7 Hz, 0.5H; rotamer 2), 7.81 (br s, 1H), 7.55-7.45 (m, 2H), 7.20 (t, *J* = 7.4 Hz, 1H), 7.15 (t, *J* = 7.4 Hz, 1H), 4.10 (br s, 1H), 2.95-2.84 (m, 1H), 2.80-2.68 (m, 3H), 1.56-1.46 (m, 1H), 1.45-1.33 (m, 1H), 0.97 (br s, 6H). | 389.42 | 390.25 |
| 210 | Starting with 4,4-dimethylpiperidine-2,6-dione and following Example 28 (compound **B**). | ¹H NMR (500 MHz, DMSO) δ 11.83 (br s, 1H), 8.57 - 8.52 (m, 1H), 8.39 (d, *J* = 8.2 Hz, 0.5H; rotamer 1), 8.36 (br s, 1H), 8.25 (d, *J* = 8.0 Hz, 0.5H; rotamer 2), 7.81 (br s, 1H), 7.55-7.45 (m, 2H), 7.20 (t, *J* = 7.5 Hz. 1H), 7.16 (t, *J* = 7.3 Hz, 1H), 4.12 (br s, 1H), 2.95-2.84 (m, 1H), 2.80-2.68 (m, 3H), 1.56-1.46 (m, 1H), 1.45-1.33 (m, 1H), 0.97 (br s, 6H). | 389.42 | 390.27 |
| 211 | Starting with (S)-tert-butyl (1-benzyl-6-oxopiperidin-3-yl)carbamate following Example 29 (compound **A**) and then Example 2 for the final step. | ¹H NMR (500 MHz, DMSO) δ 12.86 (br s, 1H), 8.56 (s, 1H), 8.47 -8.33 (m,1H), 8.31 -8.21 (m, 1H), 7.81 (s, 1H), 7.50 (d, *J* = 8.0 Hz, 1H), 7.33 (d, *J* = 7.4 Hz, 1H), 7.23 -7.18 (m, 1H), 7.15 (t, *J* = 7.1 Hz, 1H), 4.13 - 4.03 (m, 1H), 3.05 - 2.98 (m, 1H), 2.85 (dd, *J* = 12.7, 2.9 Hz, 1H), 2.44 - 2.38 (m, 1H), 2.00 - 1.93 (m, 1H), 1.71 - 1.61 (m, 1H), 1.55 - 1.48 (m, 1H), 1.46 - 1.39 (m, 1H), 1.39-1.31 (m, 2H), 0.91. (t, *J* = 7.5 Hz, 3H).* VT NMR @50 °C | 389.42 | 390.3 |
| 212 | Starting with (S)-4-azaspiro[2.5]octan-6-amine (Example 30)*and then following Example 21 and 2. | ¹H NMR (500 MHz, DMSO) δ 11.76 (m, 1H), 8.50 (s, 0.5H), 8.47 (s, 0.5H), 8.37 - 8.26 (m. 2H), 7.76 (s, 1H), 7.72 (t, *J* = 7.7 Hz, 1H), 7.42 (dd, *J* = 11.6,8.2 Hz, 1H), 7.13 (dd, *J* = 14.5,6.7 Hz, 1H), 7.10 - 7.05 (m, 1H), 3.90 (d, *J* = 32.2 Hz, 1H), 2.91 (d, *J* = 12.5 Hz, 1H), 2.60 - 2.48 (m, 1H), 1.99 -1.84 (m, 1H), 1.71 -1.56 (m, 2H), 1.26 (dd, *J* = 28.3, 11.9 Hz, 1H), 0.41 (dd, *J* = 12.5, 6.2 Hz, 1H), 0.31 (q, *J* = 6.6 Hz, 2H), 0.24 (q, *J* = 6.4 Hz, 1H). Two rotamers. | 387.4 | 388.27 |
| 213 | Example 49 | ¹H NMR (400 MHz, MeOD) δ 8.35 (s, 1 H), 8.29 (s, 1 H), 7.79 (br. d, J=8.60 Hz, 1 H), 7.65 (br. s, 1 H), 6.72 (br. s, 1 H), 6.63 (br. d, J=8.60 Hz, 1 H), 4.02 (br. s, 1 H), 3.30 (br. d, J=13.45 Hz, 1 H), 3.11 (br. s, 4 H), 2.91 - 2.78 (m, 2 H), 1.86 (br. s, 5 H) , 1.72 - 1.42 (m, 3 H) | 430.21 | 431.1 |
| 214 | Example 50 | ¹H NMR (400 MHz, MeOD) δ 8.56 (s, 1H), 8.52 (br s, 1H), 8.00 (s, 1H), 7.86 (s, 1H), 7.82 (s, 1H), 7.64 (s, 1H), 7.42 (br d, J = 8.16 Hz, 1H), 4.37 (br s, 1H), 4.13 (s, 2H), 3.56 (br d, 7 = 9.26 Hz, 1H), 3.30-3.26 (m, 1H), 3.09-2.96 (m, 2H), 2.20 (br s, 1H), 2.13-2.02 (m, 1H), 1.94-1.84 (m, 1H), 1.82-1.70 (m, 1H), 1.22 (s, 6H) | 499.23 | 500.3 |
| 215 | Starting with isoquinolin-4-amine following Example 31 (compound **A**) and then Example 28 for the final deprotections. | ¹H NMR (500 MHz, DMSO) δ 11.80 (br s, 1H), 8.64 (s, 0.5H; rotamer 1), 8.57 (s, 0.5H; rotamer 2), 8.46 (d, *J* = 8.0 Hz, 0.5H; rotamer 1), 8.23 (br s, 1H), 8.16-8.13 (m, 1H), 8.06 (d, *J* = 9.0 Hz, 0.5H; rotamer 2), 7.86 (d, *J* = 16.5 Hz, 1H), 7.48 (t, *J* = 8.5 Hz, 1H), 7.31 (d, *J* = 7.5 Hz, 1H), 7.21-7.14 (m, 3H), 7.11-7.03 (m, 2H), 5.37-5.29 (m, 1H), 3.97-3.85 (m, 2H), 3.22-3.16 (m, 1H), 3.05-2.99 (m, 1H). | 409.41 | 410.31 |
| 216 | Starting with isoquinolin-4-amine following Example 31 (compound **B**) and then Example 28 for the final deprotections. | ¹H NMR (500 MHz, DMSO) δ 11.81 (br s, 1H), 8.64 (s. 0.5H; rotamer 1), 8.57 (s, 0.5H; rotamer 2), 8.46 (d, *J* = 7.9 Hz, 0.5H; rotamer 1), 8.26 (br s, 1H), 8.16-8.13 (m, 1H), 8.07 (d, *J* = 9.0 Hz, 0.5H; rotamer 2), 7.86 (d, *J* = 16.9 Hz, 1H), 7.48 (t, *J* = 8.1 Hz, 1H), 7.31 (d, *J* = 7.1 Hz, 1H), 7.21-7.14 (m, 3H), 7.11-7.03 (m, 2H), 5.37-5.29 (m, 1H), 3.97-3.85 (m, 2H), 3.22-3.16 (m, 1H), 3.05-2.99 (m, 1H). | 409.41 | 410.3 |
| 217 | Starting with (S)-*tert*-butyl (1-benzyl-6-oxopiperidin-3-yl)carbamate following Example 29 (compound **B**) and then Example 2 for the final step. | ¹H NMR (500 MHz, DMSO) δ 11.74 (m, 1H), 8.49 (s, 0.5H), 8.44 (s, 0.5 H), 8.36-8.20 (m, 2H), 7.76 (s, 1H), 7.64 (d, *J* = 7.1 Hz, 1H), 7.46 - 7.37 (m, 1H), 7.18 -7.11 (m, 1H), 7.06 (t, *J* = 7.5 Hz, 1H), 3.90 - 3.74 (m. 1H), 3.10 - 3.00 (m, 2H), 2.25 -2.17 (m, 1H), 2.11 -1.98 (m, 0.5H), 2.00-1.87 (m, 0.5H), 1.69 - 1.62 (m, 1H), 1.38 - 1.20 (m, 2H), 1.08 - 0.94 (m, 2H), 0.80 (dd, *J* = 15.6, 8.3 Hz, 3H). Two rotamers. | 389.42 | 390.33 |
| 218 | Starting with (S)-tert-butyl 3-((5-chloro-4-(1-(phenylsulfonyl)-1H-indol-3-yl)pyrimidin-2-yl)amino)piperidine-1-carboxylate (Example 4) following Example 6. 1 and 2. | ¹H NMR (500 MHz, DMSO) δ 11.70 (bra, 1H), 8.39 (s, 1H), 8.34 (bm, 2H), 7.72 (d, *J* = 2.5 Hz, 1H), 7.47 (d, *J* = 8.0 Hz, 1H), 7.21 - 7.16 (m, 1H), 7.12 (t, *J* = 7.5 Hz, 1H), 7.08 (d, *J* = 7.9 Hz, 1H), 6.83 (dd, *J* = 17.5, 10.9 Hz, 1H), 5.62 (dd, *J* = 17.5, 1.3 Hz, 1H), 5.18 (dd, *J* = 10.9, 1.5 Hz, 1H), 4.04 (brs, 1H), 3.21 (d, *J=* 12.0 Hz, 1H), 2.97 (d, *J* = 12.6 Hz, 1H), 2.69 - 2.60 (m, 2H), 1.99 (brs, 1H), 1.81 - 1.73 (m, 1H), 1.62 - 1.47 (m, 2H). | 319.4 | 320.24 |
| 219 | Example 51 | ¹H NMR (400 MHz, MeOH-d4) δ 8.58 (s, 1H), 8.53 (br., 1H), 8.40 (br., 1H), 7.88 (s, 1H), 7.58 (s. 1H), 7.27 (d, 7 = 8.38 Hz, 1H), 4.53 (s, 2H), 4.34 (br., 1H), 3.56 (br. d, J = 12.13 Hz, 1H), 3.28 (br., 1H),3.06 - 2.96 (m, 2H), 2.89 (s, 3H), 2.29-2.05 (m. 2H), 1.93-1.71 (m, 2H) | 453.14 | 454.2 |
| 220 (tentatively assigned to Fl); and 221 (tentatively assigned to P2) | Example 52 | P1:1H NMR (400 MHz, METHANOL-d4) 88.61 (s, 2H), 8.52 - 8.51 (m, 1H), 7.96 (br., s, 0.1H), 7.70 (br., d, J=8.4 Hz, 1H), 7.39 (br., t, J=7.3 Hz, 1H), 4.61 (br., s, 1H), 4.36 (br., s, 1H), 3.94 - 3.72 (m, 2H), 3.71 - 3.56 (m. 2H), 3.45 (br., d, J=11.4 Hz, 1H), 3.10 - 2.93 (m, 2H), 2.21 (br., s, 1H), 2.12 - 1.78 (m, 5H), 1.52 -1.32 (m, 3H); P2:1H NMR (400 MHz, DMSO-d6) 11.83 (br., s, 1H), 8.54 (s, 1H), 8.28 (br., d, J=8.2 Hz, 1H), 7.87 (s, 1H), 7.64 (s, 1H), 7.52 (br., d, J=7.7 Hz, 1H). 7.29 (br., d, J=8.2 Hz, 1H), 4.60 (br., s, 1H), 4.07 (br., d, J=3.3 Hz, 1H), 3.68 (br., dd, J=9.2, 18.9 Hz, 1H), 3.52 (br., s, 1H), 3.43 - 3.31 (m. 2H), 2.90 (br., d, J=12.6 Hz, 1H), 2.72 - 2.55 (m, 2H), 2.02 - 1.67 (m, 4H), 1.60 - 1.45 (m, 2H), 1.40 - 1.22 (m, 3H) | 488.21 | P1: 489.2; P2: 489.3 |
| 222 | Example 53 | 1H NMR (400 MHz, MeOD) δ 8.58 (br s, 1H), 8.53 (br s, 1H), 8.28 (s, 1H), 7.81 (s, 1H), 7.45 (br d, *J* = 7.72 Hz, 1H), 7.27-7.13 (m, 2H), 4.21 (br s, 2H), 3.36 (br d, *J* = 10.58 Hz, 1H), 3.10 (d, *J* = 11.03 Hz, 1H), 2.82 (br s, 1H), 2.14 (br d, *J* = 13.67 Hz, 2H), 1.84 (br t, *J* = 9.48 Hz, 1H), 1.65 (br t, *J* = 8.82 Hz, 1H) | 373.3 | 374.2 |
| 223 | Example 54 | ¹H NMR (400 MHz, MeOD) δ 8.87 (br s, 1H), 8.61-8.48 (m, 3H), 8.16 (d, *J* = 8.16 Hz, 1H), 7.91 (s, 1H), 7.76 (a, 1H), 7.57-7.48 (m, 3H), 4.37 (br s, 1H), 3.57 (br d, *J* = 11.47 Hz, 1H), 3.36-3.32 (m, 1H), 3.07-2.95 (m, 2H), 2.23 (br s, 1H), 2.12-2.06 (m, 1H), 1.92-1.73 (m, 2H). | 438.4 | 439.2 |
| 224 | Example 55 | ¹H NMR (400 MHz, MeOD) δ 8.59 (s, 4H), 7.93 (s, 1H), 7.88 (s, 1H), 7.79 (br d, *J* = 4.85 Hz, 3H), 7.60 (br d, *J* = 8.60 Hz, 1H), 4.38 (br s, 1H), 3.58 (br d, *J* = 11.47 Hz, 1H), 3.35-3.32 (m, 1H), 3.08-2.97 (m, 2H), 2.23 (br s, 1H), 2.14-2.06 (m, 1H), 1.93-1.76 (m, 2H) | 438.18 | 439.2 |
| 225 | Example 56 | ¹H NMR (400 MHz, MeOD) δ ppm 8.55 (a, 2 H), 8.29 (br., s, 1 H), 7.81 (s. 1 H), 7.59 (s, 1 H), 7.45 - 7.35 (m, 2 H), 7.18 (d, *J* = 1.76 Hz, 1 H), 4.32 (br., s, 1 H), 3.84 (s, 3 H), 3.52 (br., d, *J* =10.80 Hz, 1 H), 3.28 - 3.19 (m, 1 H), 3.03 - 2.90 (m, 2 H), 2.20 (br., s, 1 H), 2.03 (br., s, 1 H), 1.88 - 1.68 (m, 2 H) | 465.19 | 466.3 |
| 226 | Example 57 | ¹H NMR (400 MHz, MeOD) δ 8.61-8.49 (m, 2H), 8.34 (br s, 1H), 7.84 (s, 1H), 7.71 (s, 1H), 7.49 (s, 1H), 7.27 (br d, *J* = 8.38 Hz, 1H), 4.35 (br s, 1H), 3.88 (s, 3H), 3.54 (br d, *J* = 12.57 Hz, 1H), 3.27 (br s, 1H). 3.07-2.94 (m, 2H), 2.40 (s, 3H), 2.22 (br s, 1H), 2.05 (br d, *J* = 3.09 Hz, 1H), 1.92-1.71 (m, 2H) | 455.2 | 456.3 |
| 227 | **Example 58** | ¹H NMR (MeOD, 400 MHz) δ 8.56 (d, *J* = 6.39 Hz, 2H), 8.33 (br s, 1H), 7.85 (s, 1H), 7.57 (s, 1H), 7.45 (s, 1H), 7.24 (br d, *J* = 8.16 Hz, 1H), 4.33 (br s, 1H), 3.87 (s, 3H), 3.53 (br d, *J* = 11.25 Hz, 1H), 3.28-3.19 (m, 1H). 3.05-2.90 (m, 2H), 2.46 (s, 3H), 2.20 (br s, 1H), 2.10-2.00 (m, 1 H), 1.92-1.68 (m. 2H). | 455.2 | 456.3 |
| 228 | Starting with (2R,5S)-tert-butyl 5-amino-2-(pyrrolidine-1-carbonyl)piperidine-1-carboxylate (Example 32) and 3-(2-(niethykulfonyl)-5-(trifluoromethyl)pyrimidin-4-yl)-1-(phenylsulfonyl)-1H-indole (Example 21) and following Examples 21, 1 and 2. | Rotamer 1:¹H NMR (500 MHz, DMSO) δ 11.86 (s, 1H), 8.52 (s, 1H), 8.46 (s, 1H), 8.31 (d, *J* = 8.0 Hz, 1H), 7.84 (s, 1H), 7.76 (d, *J* = 7.7 Hz, 1H), 7.51 (d, *J* = 8.0 Hz, 1H), 7.21 (dd, *J* = 14.7, 7.2 Hz, 1H), 7.13 (t, J= 7.5 Hz, 1H), 3.74 - 3.93 (m, 2H), 3.61 - 3.56 (m, 2H), 3.51 - 3.18 (m, 5H), 2.17 (d, *J* = 11.5 Hz, 1H), 1.87 (dt, *J* = 13.1, 6.6 Hz, 2H), 1.82 - 1.73 (m, 2H), 1.56 - 1.49 (m, 1H), 1.43 -1.37 (m, 1H).**Rotamer 2:¹H NMR (500 MHz, DMSO) δ 11.80 (s, 1H), 8.57 (s, 1H), 8.46 (s, 1H), 8.39 (d, *J* = 8.0 Hz, 1H), 7.82 (s, 1H), 7.73 (d, *J* = 8.0 Hz, 1H), 7.47 (d, *J* = 7.9 Hz, 1H),7.21 (dd, *J* = 14.7,7.2 Hz, 1H), 7.13 (t, *J* = 7.5 Hz, 1H), 3.74 - 3.93 (m, 2H), 3.61 - 3.56 (m, 2H), 3.51 - 3.18 (m, 5H), 2.13 - 2.06 (m, 1H), 1.87 (dt, *J* = 13.1, 6.6 Hz, 2H), 1.82 - 1.73 (m, 2H), 1.56 - 1.49 (m, 1H), 1.43 - 1.37 (m, 1H). | 458.48 | 459.3 |
| 229 | Example 59 | ¹H NMR (MeOD, 400 MHz)δ 8.56 (s, 2H), 8.34 (br s, 1H), 7.83 (s, 1H), 7.70 (s, 1H), 7.50 (s, 1H), 7.29 (br d, *J* = 8.38 Hz, 1H), 4.34 (br s, 1H), 3.55 (br d, *J* = 11.47 Hz, 1H), 3.29-3.22 (m, 1H), 3.10-2.91 (m, 2H). 2.46 (s, 3H), 2.22 (br s. 1H), 2.05 (br s, 1H), 1.91-1.68 (m, 2H). | 441.19 | 442.2 |
| 230 | Example 60 | ¹H NMR (400 MHz, METHANOL-d4) δ ppm 9.60 (s, 1H), 9.18 (d, *J* = 5.5 Hz, 1H), 8.60 (s, 1H), 8.52 (br. s, 1H), 8.06 (dd., *J* =2.4, 5.5 Hz, 1H), 7.98 (br. d, *J* =8.8 Hz, 2H), 7.66 (br. d, *J* =8.2 Hz, 1H), 4.37 (br. s, 1H), 3.57 (br. d, *J* = 11.7 Hz, 1H), 3.27 - 3.24 (m, 1H), 3.10 - 2.95 (m, 2H), 2.29 - 2.03 (m, 2H), 1.96 - 1.72 (m, 2H) | 439.17 | 440.2 |
| 231 | Example 61 | ¹H NMR (400 MHz, MeOH-d4) δ 8.50 (dd, *J* = 2.65,9.48 Hz, 1H), 8.34 (br., 1H), 8.28 (s, 1H), 8.18 (s, 1H), 8.01 (s, 1H), 4.76-4.66 (m, 1H), 3.61 (br., *J* = 3.31, 12.35 Hz, 1H), 3.42 (br., *J* = 12.79 Hz, 1H), 3.14-2.99 (m, 2H), 2.53 (q, *J* = 7.50 Hz, 2H), 2.42-2.28 (m, 1H), 2.17-1.93 (m, 2H), 1.81-1.69 (m, 1H), 1.26 (t, *J* = 7.39 Hz, 3H) | 340.18 | 341.2 |
| 232 | Example 62 | ¹H NMR (MeOD, 400 MHz) δ 8.84 (br s, 1H), 8.65 (s, 1H), 8.52 (br s, 1H), 8.16 (br s, 1H), 7.73 (br d, *J* = 8.60 Hz, 1H), 4.36-4.25 (m, 1H), 3.62-3.47 (m, 1H), 3.43 (s, 3H), 3.28- 3.20 (m, 1H), 3.02-2.91 (m, 2H), 2.18 (br d, *J* = 12.57 Hz, 1H), 2.05 (br s, 1H), 1.90-1.68 (m, 2H) | 464.12 | 465.2 |
| 233 | Example 63 | ¹H NMR (400 MHz, MeOH-d₄) δ 8.56 (br., 1H), 8.53-8.48 (m, 0.47H), 8.20 (s, 1H), 7.81 (br., 1H), 7.62 (br., 1H), 7.40 (br., 2H), 7.20 (br., 1H), 4.37 (br., 1H), 3.55 (br., 1H), 3.41-3.33 (m, 1H), 3.02 (br., 2H), 2.28-2.03 (m, 2H), 1.94-1.73 (m, 2H). | 451.17 | 452.3 |
| 234 | Starting with (S)-tert-butyl 3-((5-iodo-4-(l-(phenylsulfonyl)-1H-indol-3-yl)pyrimidio-2-yl)amino)piperidine-l-carboxylate and tributyl(1-ethoxyvinyl)stannane following Example 26 (Step 1) and 33, and then Examples 1 and 2 for the final steps. | ¹H NMR (500 MHz, DMSO) δ 11.55 (brs, 1H), 8.39 (s, 1H), 8.27 (d, *J* = 8.0 Hz, 1H), 7.89 (d, *J* = 7.1 Hz, 1H), 7.45 (d, *J* = 8.0 Hz, 1H), 7.18 - 7.13 (m, 1H), 7.12 - 7.06 (m, 1H), 6.67 (d, *J* = 8.2 Hz, 1H), 5.09 (d, *J* = 4.1 Hz, 1H), 4.91 (brs, 1H), 3.85 (brs, 1H), 3.09 - 3.01 (m, 1H), 2.79 - 2.73 (m, 1H), 2.47 - 2.38 (m, 2H), 1.92 (brs, 1H), 1.68 - 1.58 (m, 1H), 1.48 -1.35 (m, 5H). | 337.42 | 338.39 |
| 235 | Example 64 | ¹H NMR (400 MHz, MeOD) 88.57 (s, 1H), 8.54 (s, 1H), 8.09 (s, 1H), 7.92 (s, 1H), 7.82 (br d, *J* = 8.16 Hz, 1H), 4.33 (br s, 1H), 3.57-3.49 (m, 1H), 3.26 (br d, *J* = 12.35 Hz, 1H), 3.04-2.91 (m, 2H), 2.50 (s, 3H), 2.21 (br s, 1H), 2.10-2.02 (m, 1H), 1.91-1.82 (m, 1H), 1.81-1.69 (m, 1H) | 442.44 | 443.2 |
| 236 | Example 65 | ¹H NMR (400 MHz, CDCl₃) 88.52 (br s, 1H), 8.33 (br s, 1H), 7.84 (s, 1H), 7.45 (br d, *J* = 7.72 Hz, 1H), 7.25-7.14 (m, 2H), 4.40 (br s, 1H), 3.24 (br d, *J* = 13.01 Hz, 1H), 3.18-3.08 (m, 1H), 3.00-2.86 (m, 1H), 2.66 (br s, 2H), 2.16-1.98 (m, 1H) | 397.35 | 398 |
| 237 | Starting with (S)-*tert*-butyl (1-benzyl-6-oxopiperidin-3-yl)carbamate and isopropylmagnesium bromide and following Example 29 (as compound A) and then Example 2 for the final step. | ¹H NMR (500 MHz, DMSO) δ 11.80 (d, *J* = 20.3 Hz, 1H), 8.53 (d, *J* = 24.7 Hz, 1H), 8.42 - 8.28 (m, 2H), 7.83 (d, *J* = 4.2 Hz, 1H), 7.71 (d, *J* = 8.1 Hz, 1H), 7.48 (dd, *J* = 15.2, 8.1 Hz, 1H), 7.20 (dd, *J* = 12.7, 6.4 Hz, 1H), 7.15 - 7.10 (m, 1H). 3.87 (d, *J* = 41.0 Hz, 1H). 3.19 - 3.14 (m, 2H), 2.19 - 2.12 (m. 1H), 2.09 (d, *J* = 11.0 Hz, 0.5H), 2.00 (d, *J =* 11.0 Hz, 0.5H), 1.66 (dd, *J* = 11.8. 1.3 Hz, 1H), 1.55 - 1.51 (m, 1H), 1.45 - 1.34 (m, 1H), 1.18 -1.11 (m, 1H), 0.92 - 0.84 (m, 6H). | 403.44 | 404.2 |
| 238 | Starting with Peak 1 after chiral separation of (3S)-tert-butyl 3-((5-(1-hydroxyethyl)-4-(1 - (phenylsulfonyl)-1H-indol-3-yl)pyrimidin-2-yl)amino)pipmidine-1-carboxylate (Example 33) and following Examples 1 and 2 for the final steps. Note: stereochemistry tentatively assigned. | ¹H NMR (500 MHz, DMSO) δ 11.55 (brs, 1H), 8.40 (s, 1H), 8.27 (d, *J=* 7.9 Hz, 1H), 7.88 (d, *J* = 2.5 Hz, 1H), 7.45 (d, *J* = 8.0 Hz, 1H), 7.20 - 7.13 (m, 1H), 7.12 - 7.06 (m, 1H), 6.69 (d, *J* = 8.2 Hz, 1H), 5.10 (d, *J* = 3.6 Hz, 1H), 4.97 - 4.85 (m, 1H), 3.87 (bra, 1H), 3.06 (dd, *J* = 11.5, 3.0 Hz, 1H), 2.80 - 2.75 (m, 1H), 2.47 - 2.38 (m, 2H), 1.92 (brs, 1H), 1.68 -1.58 (m, 1H), 1.51 - 1.38 (m, 5H). | 337.42 | 338.33 |
| 239 | Starting with Peak 2 after chiral separation of (3S)-*tert*-butyl 3-((5-(1-hydroxyethyl)-4-(1-(phenylsulfonyl)-1H-indol-3-yl)pyrimidin-2-yl)amino)piperidine-l-carboxylate (Example 33) and following Examples 1 and 2 for the final steps. Note: stereochemistry tentatively assigned. | ¹H NMR (500 MHz, DMSO) δ 11.55 (brs, 1H), 8.39 (s, 1H), 8.27 (d, *J* = 7.9 Hz, 1H), 7.90 (d, *J* = 2.7 Hz, 1H), 7.45 (d, *J* = 8.0 Hz, 1H), 7.20 - 7.13 (m, 1H), 7.12 - 7.06 (m, 1H), 6.67 (d, *J* = 8.2 Hz, 1H), 5.09 (d, *J* = 4.8 Hz, 1 H), 4.95 - 4.87 (m, 1 H), 3.86 (brs, 1H), 3.08 - 3.02 (m, 1 H), 2.80 - 2.75 (m, 1H), 2.47 - 2.38 (m, 2H), 1.92 (brs, 1H), 1.68 -1.58 (m, 1H), 1.51- 1.38 (m, 5H). | 337.42 | 338.34 |
| 240 | Starting from (S)-*tert*-butyl (1-benzyl-6-oxopiperidin-3-yl)carbamate and following Example 34, 21 and 2. | ¹H NMR (500 MHz, DMSO) δ 11.79 (brs, 0.5H; rotamer 1), 11.76 (brs, 0.5H; retainer 2), 8.55 (s, 0.5H; rotamer 1), 8.51 (s, 0.5H; rotamer 2), 8.46 - 8.34 (m, 1H). 8.24 (d, *J* = 8.0 Hz, 1H), 7.82 (s, 1H), 7.71 - 7.67 (m, 1H), 7.51 - 7.46 (m, 1H), 7.20 (t, *J* = 7.5 Hz, 1H), 7.14 (t, *J* = 7.0 Hz, 1H), 3.96 -3.86 (m, 1H), 2.91-2.88 (m, 1H), 2.71-2.65 (m, 1H), 2.54-2.52 (m, 1H), 1.89-1.59 (m, 4H), 1.57-1.71 (m. 9H). | 429.48 | 430.42 |
| 241 | Starting from (S)-*tert*-butyl (1-benzyl-6-oxopiperidin-3-yl)carbamate and following Example 34, 21 and 2. | Mixture of diastereomers and retainers: ¹H NMR (500 MHz, DMSO) δ 11.82 (br s, 1H), 11.80 (br s. 1H), 8.60-8.50 (m, 2H), 8.46-8.33 (m, 5H), 8.28-8.19 (m, 2H), 7.82 (s, 2H), 7.74-7.65-7.44 (m, 5H), 7.27-7.10 (m, 6H), 4.11-3.98 (m, 1H), 3.96-3.83 (m, 1H), 3.60 (t, *J* = 6.3 Hz, 3H), 3.12 (dd, *J* = 12.0 Hz, 3.5 Hz, 1H), 2.99 (d, *J* = 13.0 Hz, 110, 2.83 (dd, *J* = 13.0 Hz, 2.5 Hz, 1H), 2.52-2.51 (m, 1H), 2.47-2.40 (m, 1H), 2.30 (s, 1H), 2.01-1.88 (m, 2H), 1.77-1.74 (m, 2H), 1.73-1.63 (m, 2H), 1.53-1.44 (m, 1H), 1.44-1.19 (m, 18H), 1.13-1.08 (m, 1H), 0.87 (t, *J* = 6.9 Hz, 6H). | 431.5 | 432.4 |
| 242 | Example 66 | ¹H NMR (400 MHz, CDCl₃) δ8.52 (s, 2H), 8.17 (br s, 1H), 7.75 (s, 1H), 7.27 (s, 1H), 7.05 (br d, *J* = 8.16 Hz, 1H), 4.31 (br s, 1H), 3.52-3.49 (br d, *J* = 11.47 Hz, 1H), 3.24 (br s, 1H), 2.98-2.93 (m, 2H), 2.90-2.76 (m, 2H), 2.03 (br s, 1H), 1.82 (br d, *J* = 13.89 Hz, 1H), 1.81-1.78 (m, 3H), 1.74-1.72 (br d, *J* = 11.03 Hz, 1H), 1.26 (s, 6H) | 447.5 | 448.2 |
| 243 | Example 67 | ¹H NMR (400 MHz, MeOH-d4) δ 8.53 (br., 2H), 8.40-8.17 (m, 1H), 7.81 (s, 1H), 7.45 (d, *J* = 7.89 Hz, 1H), 7.26-7.13 (m, 2H), 4.68-4.37 (m, 1H), 3.50 (br., 1H), 2.97 (br., *J* = 13.15 Hz, 1H), 2.70 (br., *J* = 12.28 Hz, 2H), 1.97 (br., 1 H), 1.51 (br., 1H), 1.20 (s, 3H), 1.08 (br., 3H) | 389.18 | 390.2 |
| 244 | Starting with (2R,5S)-*tert-*butyl 5-amino-2-((S)-3-hydroxypyrrolidine-1-carbonyl)piperidine-l-carboxylate (Example 35) and following Examples 21, 1 and 2. | ¹H NMR (500 MHz, DMSO) δ 11.88 - 11.75 (m, 1H), 8.59 - 8.49 (m. 1H), 8.43 - 8.19 (m, 2H), 7.83 (d, *J* = 10.2 Hz, 1H), 7.78 - 7.70 (m, 1H). 7.49 (dd, *J* = 18.4, $.2 Hz, 1H), 7.20 (dd, *J* = 14.3, 7.1 Hz, 1H), 7.13 (t, *J* = 7.1 Hz, 1H), 4.33 - 4.21 (m, 1H), 3.94 - 3.78 (m, 1H), 3.78 - 3.59 (m, 2H), 3.26 -3.11 (m, 4H), 2.17 (t, *J* = 12.2 Hz, 1H), 2.13 - 2.04 (m, 1H), 1.97 - 1.88 (m, 1H), 1.87 - 1.77 (m, 2H), 1.76 -1.70 (m, 1H), 1.61 - 1.48 (m, 1H), 1.46 - 1.34 (m, 1H). In mix with *cis*-isomer, but *trans-* is major. | 474.48 | 475.43 |
| 245 | Example 68 | ¹H NMR (MeOD, 400 MHz) δ 8.58-8.53 (m, 2H), 8.08 (s, 1H), 7.89 (s, 1H), 7.83 (br. d, *J* = 8.77 Hz, 1H), 4.33 (br. s, 1H), 3.89 (s, 3H), 3.51 (br. d, *J* = 10.09 Hz, 1H), 3.25 (br. d, *J* = 12.28 Hz, 1H), 3.01-2.91 (m, 2H), 2.52 (s, 3H), 2.20 (br. s, 1H), 2.05 (br. d, *J* = 14.47 Hz, 1H), 1.89-1.81 (m, 1H), 1.79-1.71 (m, 1H) | 456.2 | 457.3 |
| 246 | Starting with 3-(4,4,5,5-tetramethyl-1,3,2-dioxaborolan-2-yl)-1-((2-(trimethylsilyl)ethoxy)meth yl)-1H-indole-6-carbonitrile (Example 36) and 2,4,5-trichloropyrimidine* following Examples 11, 4, 6, 23 and 1. | ¹H NMR (500 MHz, DMSO) δ 12.19 (br s, 1H), 8.50 (s, 1H), 8.35 (s, 1H), 8.19 (s, 1H), 8.12 (s, 1H), 7.97 (d, *J* = 0.9 Hz, 1H), 7.43 (dd, *J* = 8.4,1.5 Hz, 1H), 6.89 (d, *J* = 7.8 Hz, 1H), 4.06 - 3.96 (m, 1H), 3.22 (dd, *J* = 12.1, 2.7 Hz, 1H), 3.02 - 2.93 (m, 1H), 2.70 (q, *J* = 7.5 Hz, 2H), 2.67 - 2.59 (m, 2H), 2.01 -1.93 (m, 1H), 1.83 -1.73 (m, 1H), 1.56 (ddd, *J* = 31.3, 16.6, 10.2 Hz, 2H), 1.15 (t, *J* = 7.5 Hz, 3H). | 346.43 | 347.4 |
| 247 | Starting from (3S)-tert-butyl 3-((5-(1-hydroxyethyl)-4-(1-(phenylsulfonyl)-1H-indol-3-yl)pyrimidin-2-yl)amino)piperidine-l-carboxylate (Example 33) following Examples 37 and 2. | ¹H NMR (500 MHz, DMSO) δ 11.62 (s, 1H), 8.37 (brs, 1H), 8.32 (s, 1H), 8.22 (d, *J* = 7.3 Hz, 1H), 7.70 (s, 1H), 7.46 (d, *J* = 8.0 Hz, 1H), 7.17 (t, *J* = 7.1 Hz, 1H), 7.10 (t, *J* = 7.5 Hz, 1H), 6.96 (d, *J* = 7.8 Hz, 1H), 4.68 - 4.60 (m, 1H), 4.01 (brs, 1H), 3.25 - 3.15 (m, 1H), 3.08 (d, *J* = 2.5 Hz, 3H), 2.94 (brs, 1H), 2.63 (brs, 2H), 1.97 (brs, 1H), 1.75 (brs, 1H), 1.60 - 1.48 (m, 2H), 1.44 (d, *J* = 6.4 Hz, 3H). | 351.45 | 352.31 |
| 248 | Example 69 | ¹H NMR (400 MHz, MeOH-d4) δ = 8.59 (s, 1H), 8.47 (br., 2H), 8.14 (br., 1H), 7.93 (br., 1H), 7.60 (br., *J* = 16.1 Hz, 2H), 7.38 - 7.29 (m, 1H), 7.18 (br., 1H), 4.37 (br., 1H), 3.57 (br. d, *J* = 10.4 Hz, 1Σi), 3.37 - 3.30 (m, 1H), 3.10 - 2.97 (m, 2H), 2.27 -2.02 (m, 2H), 1.96 -1.69 (m, 2H) | 427.17 | 428.2 |
| 249 | Starting 1-(1H-indol-6-yl)-1H-imidazole-4-carbonitrile following Example 69 | ¹H NMR (400 MHz, MeOH-d4) δ 8.63-8.41(br. s, 2H), 8.40(s, 1H), 8.29 (s, 1H), 7.98 (s, 1H), 7.72 (s, 1H), 7.4 1-7.39 (d, *J* = 8 Hz, 1H), 4.38 (m, 1H), 3.58 (m, 1H), 3.26-3.25 (m, 1H), 3.06 - 3.00 (m, 2H), 2.22 - 2.10 (m, 2H), 1.91 - 1.76 (m, 2H), | 452.17 | 453.17 |
| 250 | Starting from (2R,5S)-5-azido-1-(*tert-*butoxycarbonyl)piperidine-2-carboxylic acid (Example 35) and (R)-pyrrolidin-3-ol following Examples 35, 21, 1 and 2.* | ¹H NMR (500 MHz, DMSO) δ 11.99 - 11.92 (m, 1H), 11.89 - 11.74 (m, 1H), 8.62 - 8.48 (m, 2H), 8.44 - 8.26 (m, 1H), 7.89 - 7.81 (m, 1H), 7.81 - 7.71 (m, 1H), 7.56 - 7.43 (m, 1H), 7.24 - 7.17 (m, 1H), 7.16 - 7.09 (m, 1H), 4.34 - 4.20 (m, 1H), 3.93 - 3.16 (m, 1H), 3.69 - 3.59 (m, 2H), 3.25 - 3.13 (m, 2H), 2.46 - 2.42 (m, 1H), 2.21 - 2.15 (m, 1H), 2.14 - 2.05 (m, 1H), 1.98 - 1.89 (m, 1H), 1.89 - 1.77 (m, 2H), 1.77 - 1.68 (m, 1H), 1.60 - 1.47 (m, 1H), 1.44 - 1.34 (m, 1H). Two rotamers. In mix with *cis*-isomer, but *trans-* is major. | 474.48 | 475.4 |
| 251 | Example 70 | ¹H NMR (400 MHz, MeOD) δ 8.98 (br., 1H), 8.62 (s, 1H), 8.53 (br., 1H), 8.20 (br., 1H), 7.65 (br., *J* = 8.2 Hz, 1H), 4.32 (br., 1H), 3.66 - 3.47 (m, 1H), 3.30 (br., 1H), 3.07 - 2.92 (m, 2H), 2.25 - 1.99 (m, 2H), 1.92 - 1.66 (m, 2H) | 387.14 | 388.2 |
| 252 | Starting from 1-(tert-butoxycarbonyl)-5-(methoxycarbonyl)piperidin e-3-carboxylic acid (US5817678A) and following Example 38 (compound **A**), 1 and 2. Note: stereochemistry tentatively assigned. | Rotamer 1: ¹H NMR (500 MHz, DMSO) δ 11.77 (brs, 1H), 8.56 (s, 1H), 8.40 (d, *J* = 8.0 Hz, 1H), 8.19 (s, 1H), 7.82 (s, 1H), 7.80-7.73 (m, 2H), 7.49 (dd, *J* = 13.0 Hz, 8.0 Hz, 1H), 7.23-7.17 (m, 1H), 7.13 (t, *J* = 7.5 Hz, 1H), 4.03-3.89 (m, 1H), 3.14-3.06 (m, 2H), 2.94 (d, *J* = 11.0 Hz, 1H), 2.63 (s, 1H), 2.54 (t, *J* = 4.0 Hz, 3H), 2.42 (d, *J* = 11.5 Hz, 1H), 2.07-2.00 (m. 1H), 1.66-1.57 (m, 1H).*Rotamer 2: ¹H NMR (500 MHz, DMSO) δ 11.75 (brs, 1H), 8.52 (s, 1H), 8.23 (d, *J =* 7.0 Hz, 1Σi), 8.19 (s, 1H), 7.82 (s, 1H), 7.80-7.73 (m, 2H), 7.47 (dd, *J* = 13.0 Hz, 8.0 Hz. 1H), 7.23-7.17 (m, 1Σi), 7.13 (t, *J* = 7.5 Hz, 1H), 4.03-3.89 (m, 1H), 3.14-3.06 (m, 2H), 2.94 (d, *J* = 11.0 Hz, 1H), 2.63 (s, 1H), 2.42 (d, *J* = 11.5 Hz. 1H), 2.39-2.33 (m, 3H), 2.07-2.00 (m, 1H), 1.66-1.57 (m, 1H). | 418.42 | 419.3 |
| 253 | Starting from 1-(tert-butoxycarbonyl)-5-(metboxycarbonyl)piperidin e-3-carboxylic acid (US5817678A) and following Example 38 (compound B), 1 and 2. Note: stereochemistry tentatively assigned. | Rotamer 1: ¹H NMR (500 MHz, DMSO) δ 11.78 (brs, 1H), 8.55 (s, 1H), 8.40 (d, *J* = 8.0 Hz, 1H), 8.10 (brs, 1H), 7.83 (s, 1H), 7.79-7.74 (m, 2H), 7.49 (d, *J* = 7.5 Hz, 1H), 7.23-7.16 (m, 1H), 7.13 (t, *J* = 7.5 Hz, 1H), 4.01-3.86 (m, 1H), 3.09 (m, 1H), 2.94-2.89 (m, 1H), 2.54 (t, *J* = 4.0 Hz, 3H), 2.43-2.30 (m, 3H), 2.03 (m, 1H), 1.65-1.57 (m, 1H).*Rotamer 2: ¹H NMR (500 MHz, DMSO) δ 11.75 (brs. 1H), 8.51 (s, 1H), 8.23 (d, *J* = 7.5 Hz, 1H), 7.83 (s, 1H), 7.79-7.74 (m, 2H), 7.47 (d*, J* = 8.5 Hz, 1H), 7.23-7.16 (m, 1H), 7.13 (t, *J* = 7.5 Hz, 1H), 6.58 (brs, 1H), 4.01-3.86 (m, 1H), 3.09 (t, *J* = 9.5 Hz, 1H), 2.94-2.89 (m, 1H), 2.54 (t, *J* = 4.0 Hz, 3H), 2.43-2.30 (m, 3H), 2.03 (m, 1H), 1.65-1.57 (m, 1H). | 418.42 | 419.3 |
| 254 (tentatively assigned to P1) and 259 (tentatively assigned to P2) | Example 71 | ¹H NMR (400 MHz, MeOD) (Pl): δ 8.56 (br s, 2H), 7.98 (s, 1H), 7.80 (d, J = 7.40 Hz, 1H), 7.50-7.40 (br d, 1 = 4.00 Hz, 1H), 4.17 (br s, 1H), 3.94 (br s, 1H), 3.28 (br s, 1H), 3.24 (s, 3H), 3.12 (br d, J = 9.66 Hz, 1H), 2.42 (br s, 2H), 2.35-2.26 (m, 1H), 2.20 (q, J = 7.61 Hz, 2H), 1.54-1.40 (m, 1H), 1.13 (t, J = 7.65 Hz, 3ED; (P2): δ 8.70-8.56 (m, 2H), 7.98 (s, 1H), 7.81-7.79 (d, J = 7.40 Hz, 1H), 7.50-7.40 (br d, J = 4.00 Hz, 1H), 4.17 (br s, 1H), 3.94 (br s, 1H), 3.28 (br s, 1H), 3.24 (s, 3H), 3.13-3.11 (br d, 1 = 9.66 Hz, 1H), 2.42 (br s, 2H), 2-33-2.27 (m, 1H), 2.21-2.18 (q, J = 7.61 Hz, 2H), 1.51-1.42 (m, 1H), 1.30 (s, 1H), 1.15-1.11 (t, J = 7.65 Hz, 3H) | 510.17 | 511.2 |
| 256 | Starting from 1-(tert-butoxycarbonyl)-5-(methoxycarbonyl)piperidin e-3-carboxyEc acid (US5817678A) and following Example 38 (compound **D**), 1 and 2. Note: stereochemistry tentatively assigned. | Rotamer 1: ¹H NMR (500 MHz, DMSO) δ 11.78 (brs, 1H), 8.59 (s, 1H), 8.41 (d, *J* = 7.5 Hz, 1H), 8.30-8.18 (m, 1H),7.83 (d, *J* = 2.5 Hz, 1H), 7.83-7.79 (m, 1H), 7.72-7.65 (m, 1H), 7.51-7.46 (m, 1H), 7.21 (t, *J* = 7.0 Hz, 1H), 7.15 (t, *J* = 6.5 Hz, 1H), 4.18 (brs, 1H), 2.97-2.82 (m, 3H), 2.79-2.70 (m, 1H), 2.58-2.53 (m, 2H), 2.52-2.50 (m, 2H), 2.04-1.94 (m, 1H), 1.90-1.82 (m, 1H).*Rotamer 2: ¹H NMR (500 MHz, DMSO) δ 11.81 (brs, 1H), 8.56 (s, 1H), 8.30-8.18 (m, 1H), 8.22 (d, *J* = 8.0 Hz, 1H), 7.83 (d, *J* = 2.5 Hz, 1H), 7.83-7.79 (m, 1H), 7.72-7.65 (m, 1H), 7.51-7.46 (m, 1H), 7.21 (t, *J* = 7.0 Hz, 1H), 7.15 (t, *J* = 6.5 Hz, 1H), 4.11 (brs, 1H), 2.97-2.82 (m, 3H), 2.79-2.70 (m, 1H), 2.58-2.53 (m, 2H), 2.52-2.50 (m, 2H), 2.04-1.94 (m, 1H), 1.90-1.82 (m, 1H). | 418.42 | 419.3 |
| 257 | Starting with (S)-tert-butyl (1-benzyl-6-oxopiperidin-3-yl)carbamate and isopropylmagnesium bromide following Example 29 and then Example 2 for the final step**.** | ¹H NMR (500 MHz, DMSO) δ 11.86 (d, *J* = 23.3 Hz, 1H), 8.53 - 8.46 (m, 1H), 8.34 - 8.14 (m, 2H), 7.76 (s, 1H), 7.64 (d, *J* = 8.1 Hz, 0.5H), 7.48 (d, *J* = 7.3 Hz, 0.5H), 7.42 (t, *J* = 8.9 Hz, 1H), 7.13 (t, *J* = 7.2 Hz, 1H), 7.09 - 7.02 (m, 1H), 3.94 (d, *J* = 35.6 Hz, 0.5H), 3.80 (d, *J* = 44.9 Hz, 0.5H), 3.14 - 3.08 (m, 1H), 2.96 (d, *J* = 12.7 Hz, 0.5H), 2.76 (d, *J* = 11.4 Hz, 0.5H), 2.11 (d, *J* = 30.2 Hz, 1H), 1.92 (s, 1H), 1.63 - 1.43 (m, 2H), 1.21 -1.03 (m, 2H), 0.85 - 0.79 (m, 6H). *Rac* at the C-6 position. | 403.44 | 404.4 |
| 258 | Example 72 | ¹H NMR (400 MHz, MeOD) δ 8.79 (br., 1H), 8.64 (s, 1H), 8.50 (br., 1H), 8.12 (br., 1H), 7.64 (br., *J* = 8.6 Hz, 1H), 4.34 - 4.23 (m, 1H), 3.59 - 3.45 (m, 1H), 3.28 - 3.23 (m. 1H), 2.95 (br., 2H), 2.22 - 1.99 (m, 2H), 1.90 - 1.66 (m, 2H) | 411.14 | 412.2 |
| 260 | Starting with (S)-tert-butyl (1-benzyl-6-oxopiperidin-3-yl)carbamate and isopropylmagnesium bromide following Example 29 (as compound **B**) and then Example 2 for the final step. | ¹H NMR (500 MHz, DMSO) δ 11.85 (d, *J* = 25.0 Hz, 1H), 8.57 (d, *J* = 24.8 Hz, 1H), 8.27 (dd, *J* = 48.2, 7.8 Hz, 2H), 7.83 (s, 1 H), 7.52 (dd, *J* = 31.0, 7.0 Hz, 2H), 7.20 (t, *J* = 7.5 Hz, 1H), 7.14 (t, *J* = 7.2 Hz, 1H), 4.02 (d, *J* = 35.3 Hz, 1H), 3.03 (d, *J* = 13.9 Hz, 1H), 2.83 (d, *J* = 8.6 Hz, 1H), 2.22 (s, 1H), 2.00 (d, *J* = 8.2 Hz, 1H), 1.70 - 1.51 (m, 2H), 1.51 - 1.30 (m, 2H), 0.90 (dd, *J* = 13.5, 6.1 Hz, 6H). | 403.44 | 404.3 |
| 261 | Example 73 | ¹H NMR (400 MHz, CDCl₃) δ 9.5 1-9.44 (m, 2H), 8.64 (s, 1H), 8.47 (br., 1H), 8.08-7.98 (m, 2H), 7.66 (br., *J* = 9.65 Hz, 1H), 4.34 (br., 1H), 3.57 (br., 1H), 3.39-3.32 (m, 1H), 3.01 (br., 2H), 2.24-2.04 (m, 2H), 1.78 (br., 2H) | 464.17 | 465.1 |
| 262 | Example 74 | ¹H NMR (400 MHz, MeOH-d₄) δ 8.96 (br. d, *J*=7.9 Hz, 1H), 8.57 (dd., *J* = 1.5, 4.6 Hz, 2H), 8.32 (s, 1H), 7.48 - 7.30 (m, 1H), 4.36 - 4.21 (m, 1H), 3.57 (br. dd., *J*=3.5, 12.3 Hz, 1H), 3.30 (br. s, 1H), 3.16 - 2.97 (m, 4H), 2.29 - 2.03 (m, 2H). 1.99 - 1.71 (m, 2H), 1.24 (t. *J*=7.4 Hz, 3H). | 323.4 | 324.1 |
| 263 | Example 75 | ¹H NMR (400 MHz, MeOD) δ 8.76 - 8.68 (m, 1H), 8.57 - 8.47 (m, 1H), 8.27 (s, 1H), 8.10 (s, 1H), 7.70 (d, *J*= 8.3 Hz, 1H), 4.26 - 4.15 (m, 1H), 3.49 (br.s, 1H), 3.42 (s, 3H), 3.27 - 3.21 (m, 1H), 2.99 (br.d, *J* = 10.1 Hz, 2H), 2.75 (br.d, *J* = 7.5 Hz, 2H), 2.21 - 2.12 (m, 1H), 2.10 - 1.99 (m, 1H), 1.90 - 1.67 (m, 2H), 1.21 (br.t, *J* =7.5 Hz, 3H) | 424.17 | 425.3 |
| 264 | Example 76 | ¹H NMR (400 MHz MeOH-d4) δ = 8.73 - 8.56 (m, 1H), 8.49 (br. s, 1H), 7.97 (br. s, 1H), 7.81 (br. d, *J* = 7.1 Hz, 1H), 7.40(br. t, *J* = 7.7 Hz, 1H), 6.83 - 6.30 (m, 1H), 6.10 (br. d, *J* = 11.0 Hz, 1H), 5.66 (br. d, *J* = 12.8 Hz, 1H), 4.65 - 4.35 (m, 2H), 4.12 (br. s, 1H), 3.94 - 3.82 (m, 1H), 3.79 (br. s, 1H), 3.58 - 3.40 (m, 2H), 3.22 (s, 7H), 2.99 - 2.4.1 (m, 2H), 2.13 - 1.73 (m, 4H), 1.21 (br s, 2H) | 606.22 | 607.2 |
| 265 | Example 77 | ¹H NMR (400 MHz, MeOH-d4) δ 8.78 (br. s, 1H), 8.58 - 8.30 (m, 2H), 8.17 (br. s, 1H), 8.05- 7.75 (m, 2H), 7.56 - 7.34 (m, 1H), 7.20 (br. s, 2H), 6.71 (br. s, 1H), 4.33 - 4.06 (m, 1H), 3.41 (br. s, 4H), 2.70 - 2.41 (m, 1H), 2.34 - 2.03 (m, 3H), 1.91 - 1.46 (m, 4H). | 555.17 | 556.2 |
| 256 | Starting with *cis* 1-*tert*-butyl 3-mediyl 5-(((benzyloxy)carbonyl)amin o)piperidine-1,3-dicarboxylate (from Example 38) following Example 39, and then Examples 1 and 2 for the final steps. | ¹H NMR (500 MHz, DMSO) δ 11.79 (d, *J* = 23.2 Hz, 1H), 8.59 (s, 0.5H), 8.53 (d, *J* = 6.6 Hz, 1H), 8.41 (d, *J* = 7.4 Hz, 1H), 8.34 (d, *J* = 7.5 Hz, 0.5H), 8.25 (s, 1H), 7.92 (d, *J* = 8.0 Hz, 1H), 7.84 (s, 1H), 7.71 (t, *J* = 8.3 Hz, 1H), 7.51 (d, *J* = 7.6 Hz, 0.5H), 7.47 (d, *J* = 8.0 Hz, 0.5H), 7.36 - 7.28 (m, 1H), 7.24 - 7.16 (m, 1.5H), 7.13 (t, *J* = 7.4 Hz, 0.5H), 6.97 (t, *J* = 6.6 Hz, 0.5H), 6.86 (t, *J* = 6.7 Hz, 0.5H), 4.18 (d, *J* = 3.3 Hz, 1H), 3.49-3.22 (m, 4H), 2.71 - 2.64 (m, 1H), 2.47 - 2.37 (m, 1H), 2.11 -1.92 (m, 1H), **Cis*-Racemic | 478.47 | 479.2 |
| 267 | Starting with *cis*-1-*tert*-butyl 3-methyl 5-((4-(l-(phenylsulfonyl)-1H-indol-3-yl)-5-(trifluoromethyl)pyrimidin-2-yl)amino)piperidine-l,3-dicarboxylate (from Example 39), following Example 84 and then Examples 1 and 2 for the final steps. | ¹H NMR (500 MHz, DMSO) δ 11.79 (d, *J* = 23.2 Hz, 1H), 8.59 (s, 0.5H), 8.53 (d, *J* = 6.6 Hz, 1H), 8.41 (d, *J* = 7.4 Hz, 1H), 8.34 (d, *J* = 7.5 Hz, 0.5H), 8.25 (s, 1H), 7.92 (d, *J* = 8.0 Hz, 1H), 7.84 (s, 1H), 7.71 (t, *J* = 8.3 Hz, 1H), 7.51 (d, *J* = 7.6 Hz, 0.5H), 7.47 (d, *J* = 8.0 Hz, 0.5H), 7.36 - 7.28 (m, 1H), 7.24 - 7.16 (m, 1.5H), 7.13 (t, *J* = 7.4 Hz*,* 0.5H), 6.97 (t, *J* = 6.6 Hz, 0.5H), 6.86 (t, *J* = 6.7 Hz, 0.5H), 4.18 (d, *J* = 3.3 Hz, 1H), 3.49-3.22 (m, 4H), 2.71 - 2.64 (m, 1H), 2.47 - 2.37 (m, 1H), 2.11 - 1.92 (m, 1H), *Mixture of rotamers | 478.47 | 479.2 |
| 268 | Starting with *cis*-1-*tert*-butyl 3-methyl 5-((4-(1-(phenylsulfonyl)-1H-indol-3-yl)-5-(trifluoromethyl)pyrimidin-2-yl)amino)piperidine-l,3-dicarboxylate (from Example 39), following Example 84 and then Examples 1 and 2 for the final steps. | ¹H NMR (500 MHz, DMSO) δ 11.79 (d, *J* = 23.2 Hz, 1H), 8.59 (s, 0.5H), 8.53 (d, *J* = 6.6 Hz, 1H), 8.41 (d, *J* = 7.4 Hz, 1H), 8.34 (d, *J* = 7.5 Hz, 0.5H), 8.25 (s, 1H), 7.92 (d, *J =* 8.0 Hz. 1H), 7.84 (s, 1H), 7.71 (t, *J* = 8.3 Hz, 1H), 7.51 (d, *J* = 7.6 Hz, 0.5H), 7.47 (d, *J* = 8.0 Hz, 0.5H), 7.36 - 7.28 (m, 1H), 7.24 - 7.16 (m, 1.5H), 7.13 (t, *J* = 7.4 Hz, 0.5H), 6.97 (t, *J* = 6.6 Hz, 0.5H), 6.86 (t, *J* = 6.7 Hz, 0.5H), 4.18 (d, *J =* 3.3 Hz, 1H), 3.49-3.22 (m, 4H), 2.71 - 2.64 (m, 1H), 2.47 - 2.37 (m, 1H), 2.11 - 1.92 (m, 1H), *Mixture of rotamers | 478.47 | 479.2 |
| 269 | Example 80 | ¹H NMR: DMSO 400MHz δ 8.72 (d, *J* = 7.6 Hz, 1H), 8.19 (s, 1H), 7.87 (s, 1H), 7.71 (d, *J* = 7.2 Hz, 1H), 7.35 (t, *J* = 7.6 Hz, 1H), 6.70 (d, *J* = 7.2 Hz, 1H), 3.81 (br. s., 1H), 3.33 (br. s., 3H), 3.06 (br. s., 1H), 2.64-2.84 (m, 5H), 1.93 (br. s., 1H), 1.65 (br. s., 1H), 1.44 (d, *J* = 8.0 Hz, 2H), 1.17 (t, *J* = 7.6 Hz, 3H). | 399.17 | 400.2 |
| 270 | Example 79 | ¹H NMR: 400 MHz CDCl₃ δ 8.41 (d, *J* = 9.6 Hz, 1H), 8.27 (s, 1H), 8.22 (s, 1H), 7.75 (s, 1H), 5.34 (br. s, m), 4.04 (br. s, m), 3.29-3.34 (m, 1H), 2.95 (m, 1H), 2.67-2.76 (m, 3H), 2.09 (m, 1H), 1.61-1.72 (m, 5H), 1.26 (t, *J* = 7.6 Hz, 3H). | 340.18 | 341.1 |
| 271 | Starting with 6-bromoindole and 2,4-dichloro-5-ethylpyrimidine, following Examples 3 and 85 and then Example 1 for the final step. | ¹H NMR (500 MHz, DMSO) δ 12.32 (brs, 1H), 8.55 (d, *J* = 8.5 Hz, 1H), 8.41 (brs, 1H), 8.18 (s, 1H), 8.15 (s, 1H), 8.03 (d, *J* = 1.4 Hz, 1H), 7.62 (d, *J* = 8.5 Hz, 1H), 6.80 (d, *J* = 7.8 Hz, 1H), 3.96 (brs, 1H), 3.20 (s, 3H), 3.17 (d, *J* = 12.9 Hz, 1H). 2.92 (d, *J* = 12.1 Hz, 1H), 2.71 (q, *J* = 7.4 Hz, 2H), 2.62 - 2.53 (m, 2H), 1.99 - 1.93 (m, 1H), 1.77 - 1.71 (m, 1H), 1.58 - 1.45 (m, 2H), 1.15 (t, *J* = 7.5 Hz, 3H). | 399.51 | 400.2 |
| 272 | Example 99 | 1H NMR (400 MHz, MeOD) δ 8.90 (s, 1H), 8.72 - 8.63 (m, 1H), 8.41 (s, 1H), 7.88 (s, 1H), 7.48 - 7.42 (m, 1H), 4.31 - 4.20 (m, 1H), 3.52 (br.dd, 1 = 3.3,12.3 Hz, 1H), 3.29 - 3.23 (m, 1H), 3.05 - 2.93 (m. 2H), 2.29 (s, 3H), 2.24 - 2.15 (m, 1H), 2.11 - 2.01 (m, 1H), 1.92 - 1.68 (m, 2H) | 364.15 | 365.1 |
| 273 | Starting with 1H-pyrrolo[2,3-b]pyridine-6-carbonitrile (WO2011128455) and following Examples 86, 10, 11 and then Example 1 for the final step. | ¹H NMR (500 MHz, DMSO) δ 9.04 (d, *J* = 8.5Hz, 0.6H), 8.71 (d, *J* = 8.5Hz, 0.4H), 8.63 (d, *J* = 14.0Hz, 1H), 8.33 (d, *J* = 10.0Hz, 1H), 8.30 (br s, 1H), 8.04 (dd, *J* = 33.0Hz, 6.5Hz, 1H), 7.84 (m, 1H), 4.05-3.99 (m, 1H), 3.97 (s. 3H), 3.23-3.13 (m, 1H), 2.98-2.89 (m ,1H), 2.65-2.54 (m, 2H), 2.01-1.92 (m 1H), 1.80-1.67 (m, 1H), 1.58-1.47 (m, 2H). *Mixture of rotamers | 401.39 | 402.2 |
| 274 | Starting with (*S*)-*tert*-butyl 3-((4-(6-bromo-l-(phenylsulfonyl)-1H-indol-3-yl)-5-(trifluoromethyl)pyrimidin-2-yl)amino)piperidine-1-carboxylate and sodium ethanesulfinate, following Example 85, and then Example 1 for the final step. | ¹H NMR (500 MHz, DMSO) δ 12.30 (s, 1H), 8.61 (d, *J* = 23.2 Hz, 1H), 8.48 (dd, *J* = 58.6, 8.5 Hz, 1H), 8.28 (s, 1H), 8.12 (d, *J* = 7.1 Hz, 1H), 8.03 (d, *J* = 9.5 Hz. 1H), 7.90 (d, *J* = 7.9 Hz, 1H), 7.60 (dd, *J* = 25.2, 8.5 Hz, 1H), 4.02 (s, 1H), 3.32 -3.28 (m, 2H), 3.16 (d, *J* = 9.1 Hz, 2H), 2.91 (d, *J* = 11.9 Hz, 1H), 2.60 - 2.54 (m, 1H), 2.05 - 1.92 (m, 1H), 1.72 (s, 1H), 1.55 - 1.47 (m, 2H), 1.16 - 1.07 (m, 3H). | 453.48 | 454.1 |
| 275 | Example 81 | ¹H NMR: 400 MHz MeOD δ 8.67 (d, *J* = 8.4 Hz, 1H), 8.53 (br. s., 1H), 8.30 (s, 1H), 8.06 (s, 1H), 7.48 (d, *J* = 8.4 Hz, 1H), 4.22-4.27 (m, 1H), 3.50-3.55 (m, 1H), 2.95-3.05 (m, 4H), 2.17-2.21 (m, 1H), 2.05-2.17 (m, 1H) 1.72-1.76 (m, 2H) 1.19 (t, *J* = 7.2 Hz, 3H) | 347.19 | 348.2 |
| 276 | Example 82 | ¹H NMR: (400 MHz, CDCl₃): δ 9.01 (d, *J* = 7.2 Hz, 1H), 8.49 (s, 1H), 7.61 (d, *J* = 8.4 Hz, 1H), 4.49 (s, 1H), 4.01-4.05 (m, 1H), 3.43 (d, *J* = 13.6 Hz, 1H), 3.20 (s, 3H), 2.18 (s, 1H), 2.05 (s, 1H), 1.91 (s, 1H), 1.27-1.31 (m, 8H). | 362.2 | 363.2 |
| 277 | Example 83 | ¹H NMR: (400 MHz, CDCl₃): δ 8.99 (d, *J* = 8.0 Hz, 1H), 8.29 (s, 1H), 7.53 (d, *J* = 8.0 Hz, 1H), 5.40 (s, 1H), 4.15 (s, 1H), 3.30 (d, *J* = 11.2 Hz, 1H), 2.91-3.01 (m, 4H), 1.93-2.05(m, 2H), 1.70 (s, 3H), 1.20-1.26 (m, 5H). | 348.18 | 349.2 |
| 278 | Example 100 | 1H NMR (400 MHz, MeOD) δ 8.61 - 8.28 (m, 2H), 7.93 (s, 1H), 7.66 (s, 1H), 7.37 (br. s, 1H), 4.24 - 3.98 (m, 1H), 3.73 - 3.50 (m, 4H), 3.25 (br.dd, 1 = 3.2, 12.2 Hz, 1H), 2.95 (br. d, J = 12.5 Hz, 1H), 2.67 - 2.54 (m, 2H), 2.15 (br. s, 1H), 2.02 (q, J = 6.8 Hz, 2H), 1.96 - 1.87 (m, 2H), 1.85 - 1.75 (m, 1H), 1.69 - 1.53 (m, 2H) | 458.20 | 459.2 |
| 279 | Starting with 7-bromo-1-methyl-1H-indole, following Examples 9, 86, 10, 11 and then Example 1 for the final step. | ¹H NMR (500 MHz, DMSO) δ 8.67 (d, *J* = 8.0Hz, 0.5H), 8.62 (d, *J* = 18.0Hz, 1H), 8.46 (d, *J* = 8.0Hz, 0.5H), 8.30 (br s, 1H), 7.98-7.93 (m, 2H), 7.76 (d, *J* = 7.5Hz*,* 1H), 7.35-7.31 (m, 1H), 4.20 (s, 3H), 4.05-3.94 (m, 2H), 3.22-3.14 (m, 1H). 3.00-2.88 (m, 1H), 2.64-2.54 (m ,1H), 2.03-1.92 (m, 1H), 1.80-1.69 (m, 1H), 1.58-1.45 (m, 2H). *Mixture of rotamers | 400.40 | 401.1 |
| 280 | Example 101 | 1H NMR (400 MHz, MeOD) δ 8.59 - 8.28 (m. 2H), 7.94 (s, 1H), 7.67 (s, 1H), 7.37 (br. s, 1H), 4.23 - 3.95 (m. 2H), 3.77 - 3.66 (m, 3H), 3.62 - 3.52 (m, 1H). 3.41 - 3.37 (m, 1H), 3.26 (s, 2H), 2.99 (br. d, J = 10.8 Hz, 1H), 2.67 (br. s, 2H), 2.25 - 1.74 (m, 5H), 1.72 - 1.52 (m, 2H) | 488.21 | 489.20 |
| 281 | Example 101 | 1H NMR (400 MHz, MeOD) δ 8.64 - 8.22 (m, 2H), 7.94 (s, 1H), 7.72 - 7.58 (m, 1H), 7.36 (br. s, 1H), 4.27 - 3.96 (m, 2H), 3.77 - 3.65 (m, 3H), 3.61 - 3.51 (m, 1H), 3.42 - 3.35 (m, 2H), 3.25 (s, 1H), 3.07 (br. d, J = 7.5 Hz, 1H), 2.75 (br. s, 2H), 2.25 - 2.04 (m, 3H), 2.03 - 1.84 (m, 2H), 1.78 - 1.59 (m, 2H) | 488.21 | 489.20 |
| 282 | Example 102 | 1H NMR (400 MHz, MeOD) δ 8.80 (s, 1H), 8.51 (br., 1H), 8.47 (br., 1H), 8.39-8.35 (m, 1H), 7.60-7.54 (m, 1H), 7.38 (dquin, J = 1.32, 7.44 Hz, 2H), 4.32-4.25 (m, 1H), 3.55 (dd, J = 3.53,12.13 Hz, 1H), 3.34-3.32 (m, 0.5H), 3.29-3.28 (m, 0.4H), 3.09-2.99 (m, 2H), 2.25-2.05 (m, 2H), 1.92-1.70 (m, 2H) | 328.11 | 329.1 |
| 283 | Starting with 1H-pyrrolo[2,3-b]pyridine-6-carbonitrile (WO2011128455), following Examples 87, 86, 10, 11 and then Example 1 for the final step. | ¹H NMR (500 MHz, DMSO) δ 8.96 (d, J = 8.0Hz, 0.5H), 8.69 (s, 1H), 8.66 (d, *J* = 6.5Hz, 0.5H), 8.41 (d, *J* = 7.5Hz, 1H), 8.33 (br s, 1H), 8.13 (dd, *J* = 34.5Hz. 8.0Hz, 1H), 7.91-7.88 (m, 1H), 5.76 (s, 2H), 4.10-4.01 (m, 1H), 3.27 (d, *J* = 7.5Hz, 3H), 3.23-3.17 (m, 1H), 2.99-2.94 (m, 1H), 2.65-2.61 (m, 2H), 2.01-1.93 (m, 1H), 1.82-1.71 (m, 1H), 1.60-1.49 (m, 2H). *Mixture of retainers | 431.41 | 432.2 |
| 284 | Starting with (*S*)-*tert*-butyl 3-((4-(6-bromo-1-(phenylsulfonyl)-1H-indol-3-yl)-5-(trifluoromethyl)pyrimidin-2-yl)amino)piperidine-1-carboxylate, following Examples 1, 88, 2, and then Example 9 for the cyanation step. | ¹H NMR (500 MHz, DMSO) δ 12.27 (brs, 1H), 8.63 (s, 0.5H; rotamer 1), 8.57 (s, 0.5H; rotamer 2), 8.49 (d, *J* = 8.4 Hz, 0.5H; rotamer 1), 8.33 (d, *J* = 8.3 Hz, 0.5H; rotamer 2), 8.07 (s, 1H), 8.01 (d, *J* = 15.0 Hz, 1H), 7.89 - 7.78 (m, 1Σi). 7.46 (d, *J* = 8.4 Hz, 0.5H; rotamer 1), 7.42 (d, *J* = 8.3 Hz, 0.5H; rotamer 2), 4.02 (brs, 1H), 3.06 - 2.95 (m, 1H), 2.75 (brs, 1H), 2.42 - 2.31 (m, 2H), 1.97 - 1.86 (m, 3H), 1.75 -1.67 (m, 1H), 1.58 - 1.46 (m, 1H), 1.42 - 1.31 (m, 1H), 1.00 (t, *J* = 6.2 Hz, 3H). | 414.43 | 415.1 |
| 285 | Starting with 3-(2-(methylsulfonyl)-5-(trifluoromethyl)pyrimidin-4-yl)-1-(phenylsulfonyl)-1H-indole (from Example 21) and (3*S*,4*S*)-*tert*-butyl 3-amino-4-methylpiperidine-1-carboxylate, following Example 21 and then Examples 2 and 1 for the final steps. | ¹H NMR (500 MHz, DMSO) δ 1 1.80 (brs, 1H), 8.59 - 8.55 (m, 1H), 8.40 (d, *J* = 8.0 Hz, 0.5H; rotamer 1), 8.32 (brs, 1H), 8.20 (d, *J* = 8.0 Hz, 0.5H; rotamer 2), 7.83 (s, 1H), 7.60 - 7.53 (m, 1H), 7.49 (t, *J* = 8.4 Hz, 1H), 7.23 - 7.19 (m, 1H), 7.18 - 7.13 (m, 1H), 4.32 (brs, 1H), 3.07 - 2.93 (m, 2H), 2.86 (t, *J* = 12.5 Hz, 1H), 2.70 - 2.60 (m, 1H), 2.01 (brs, 1H), 1.65 - 1.45 (m, 2H), 0.90 (bra, 3H). | 375.39 | 376.0 |
| 286 | Starting with (*S*)-*tert*-butyl 3-((4-(7-bramo-1H-indol-3-yl)-5-(trifluoromethyl)pyrimidin-2-yl)amino)piperidine-1-carboxylate (from Example 11) and sodium ethanesulfinate, following Example 85, and then Example 1 for the final step. | ¹H NMR (500 MHz, DMSO) δ 8.76 (d, *J* = 8.0 Hz, 0.5H), 8.63 (d, *J* = 19.5 Hz, 1H), 8.57 (d, *J* = 8.0 Hz, 0.5H), 8.35 (s, 1H), 7.99 (dd, *J* = 15.1, 7.9 Hz, 1H), 7.88 (d, *J* = 6.1 Hz, 1H), 7.73 - 7.67 (m, 1H), 7.41 (dd, *J* = 16.7, 8.2 Hz, 1H), 4.06 (s, 1H), 3.40 (q, *J =* 7.0 Hz, 2H), 3.19 (t, *J* = 10.3 Hz, 1H), 2.95 (t, *J* = 13.5 Hz, 1H), 2.70 - 2.55 (m, 2H), 2.01 - 1.97 (m, 1H), 1.77 -1.74 (m, 1H), 1.57 - 1.51 (m, 2H), 1.14 (dd, *J* = 17.3, 7.4 Hz, 3H). *Mixture of rotamers | 453.48 | 454.2 |
| 287 | Example 78 | ¹H NMR: 400 MHz DMSO δ 8.32 (t, *J* = 10.8 Hz, 2H), 8.21 (s, 1H), 7.74 (d, *J* = 8.0 Hz, 1H), 6.99 (s, 1H), 3.98 (s, 1H), 3.25 (s, 1H), 2.97 (s, 1H), 2.62-2.77 (m, 5H), 1.98 (s, 1H), 1.76 (s, 1H), 1.53 (s, 2H), 1.17 (t, *J* = 7.4 Hz, 3H). | 347.19 | 348.1 |
| 289 | Example 104 | 1H NMR (400 MHz, MeOD) δ 8.55 (s, 1H), 8.51 - 8.19 (m, 1H), 8.08 (d, J = 2.7 Hz, 1H), 7.97 (s, 1H), 7.89 (s, 1H), 7.74 (br.d, J = 7.7 Hz, 1H), 7.69 - 7.35 (m, 1H), 6.93 (d, J = 2.7 Hz, 1H), 4.40 - 4.16 (m, 1H), 3.47 (br.d, J = 11.5 Hz, 1H), 3.24 - 3.11 (m, 1H), 2.88 (br.d, J = 9.9 Hz, 2H), 2.31 - 2.10 (m, 1H), 2.06 - 1.94 (m, 1H), 1.88 - 1.63 (m, 2H) | 477.17 | 478.3 |
| 290 | Example 105 | 1H NMR (400 MHz, CDCl3) δ 8.62 (s, 1H), 8.47 (s, 2H), 8.16 (s, 2H), 7.83 - 7.37 (m, 3H), 4.70 - 4.27 (m, 1H), 3.64 (dd, J = 3.5, 12.4 Hz, 1H), 3.36 (br.s, 1H), 3.11 (br.d, J = 9.9 Hz, 2H), 2.33 (br.s, 1H), 2.16 (br.d, J = 14.2 Hz, 1H), 1.85 (br.d, J = 10.1 Hz, 2H) | 477.43 | 478.2 |
| 291 | Example 106 | 1H NMR (400 MHz, CDCl3) δ 8.66 (s, 2H), 8.35 - 7.98 (m, 3H), 7.91 - 7.62 (m, 1H), 7.19 (d, J = 2.4 Hz, 1H), 4.73 - 4.26 (m, 1H), 3.64 (br.dd, J = 3.4, 12.2 Hz, 1H), 3.37 (br.s, 1Σi). 3.29 - 2.97 (m, 2H), 2.44 - 2.22 (m, 1H). 2.20 - 2.08 (m, 1H), 2.06 - 1.92 (m, 1H), 1.85 (br.d, J = 10.5 Hz, 1H) | 427.43 | 428.1 |
| 292 | Example 107 | 1H NMR (400 MHz, CDCl3) δ 8.59 (s, 1H), 8.55 - 8.46 (m, 1H), 7.95 (br., 1H), 7.70 (s, 1H), 7.41 - 7.33 (m, 1H), 4.39 - 4.25 (m, 1H), 4.02 - 3.63 (m, 5H), 3.61 - 3.43 (m, 2H), 3.41 - 3.31 (m, 1H), 3.01 (br., J = 10.5 Hz, 2H), 2.06 (br., 3H), 1.94 - 1.67 (m, 2H) | 483.49 | 484.3 |
| 293 | Example 108 | 1H NMR (400 MHz, CDCl3) δ 8.59 (s, 1H), 8.55 - 8.46 (m, 1H), 7.98 - 7.92 (m, 1H), 7.73 - 7.65 (m, 1H), 7.41 - 7.34 (m, 1H), 4.41 - 4.26 (m, 1H), 4.02 - 3.45 (m, 6H), 3.42 - 3.32 (m, 1H), 3.08 - 2.90 (m, 2H), 2.49 - 2.01 (m, 4H), 1.93 - 1.69 (m, 2H) | 483.49 | 484.3 |
| 294 | Example 109 | 1H NMR (400 MHz, MeOD) δ 9.17 - 8.98 (m, 1H), 8.82 (s, 1H), 8.40 (s, 1H), 7.83 - 7.75 (m, 1H), 4.38 - 4.23 (m, 1H), 3.64 - 3.53 (m, 1H), 3.44 (s, 3H), 3.38 - 3.34 (m, 1H), 3.16 - 3.00 (m, 2H), 2.29 - 2.06 (m, 2H), 1.80 (br.s., 2H) | 430.10 | 431.0 |
| 295 | Starting with 7-bromo indole, following Examples 9, 87, 86, 10, 11 and then Example 1 as the final step. | ¹H NMR (500 MHz, DMSO) δ 8.66 (d, J = 20.5Hz, 1H), 8.55 (d, *J* = 8.0Hz, 0.5H), 8.41 (d, J = 8.0Hz, 0.5H), 8.32 (br a, 1H), 8.09 (s, 1H), 8.04 (t, J = 7.5Hz, 1H), 7.80 (d, *J* = 7.0Hz, 1H), 7.38 (app q, *J*= 7.0Hz, 1H), 5.86 (s, 2H), 4.02 (s, 1H), 3.24 (s, 1.5H). 3.21 (s, 1.5H), 3.19-3.12 (m, 1H), 3.00-2.87 (m, 1H), 2.65-2.56 (m, 2H), 2.01-1.93 (m, 1H), 1.80-1.68 (m, 1H), 1.59-1.44 (m, 2H). *Mixture of rotamers | 430.43 | 431.2 |
| 296 | Starting with 7-(methylthio)-1H-indole (WO2009008992), following Examples 86, 36, 11, 21 and then Example 1 for the final step. | ¹M NMR (500 MHz, DMSO) δ 8.64 (d, *J* = 20.6 Hz, 1H), 8.59 (d, *J* = 7.9 Hz, 0.5H), 8.44 (d, *J* = 7.9 Hz, 0.5H), 8.30 (s.1H), 7.95 (d, *J* = 7.9 Hz, 1H), 7.91 (d, *J* = 7.6 Hz, 1H), 7.88 (d, *J* = 5.1 Hz, 1H), 7.36 (t, *J* = 7.8 Hz, 1H), 4.28 (a, 3H), 3.95 (d, J= 20.9 Hz, 1H), 3.52 (s, 3H), 3.13 (dd, *J* = 21.1, 11.8 Hz, 1H), 2.88 (dd, *J* = 27.3, 12.3 Hz, 1H), 2.59 - 2.51 (m, 2H), 1.94 (br s, 1H), 1.70 (br s, 1H), 1.57 - 1.38 (m, 2H). *Mixture of rotamers | 453.48 | 454.1 |
| 297 | Starting with 1H-pyrrolo[2,3-b]pyridine-6-carboxylic acid, following Examples 86, 89, 3, 10, 11 and then Examples 2 and 1 for the final steps. | ¹H NMR (500 MHz, DMSO) δ 11.75 (br s, 1H), 8.95 (d, *J =* 8.2 Hz, 0.5H; rotamer 1), 8.71 (d, *J* = 8.2 Hz, 0.5H; rotamer 2), 8.62 (d, *J* = 16.9 Hz, 1H), 8.47 (d, *J=* 4.8 Hz, 1H), 8.37 (br s, 1H), 8.12 - 8.02 (m, 1H), 7.99 (dd, *J* = 14.2,8.2 Hz, 1H), 7.91 (d, *J* = 8.2 Hz, 1H), 4.18-4.04 (m, 1H), 3.29-3.19 (m, 1H), 3.01 (d, *J* = 11.6 Hz, 1H), 2.87 (d, *J* = 4.8 Hz, 3H; retainer 1 + rotamer 2), 2.75-2.59 (s, 2H), 2.08 - 1.91 (m, 1H), 1.86-1.74 (m, 1H), 1.66 - 1.44 (m, 2H). | 419.40 | 420.1 |
| 298 | Starting with (*S*)-4-(6-bromo-1-(phenylsulfonyl)-1H-indol-3-yl)-N-(1-ethylpiperidin-3-yl)-5-(trifluoromethyl)pyrimidin-2-amine (Example 88) and following Example 85. | ¹M NMR (500 MHz, DMSO) δ 12.27 (bra, 1H), 8.65 (s, 0.5H; rotamer 1), 8.58 (s, 0.5H; rotamer 2), 8.52 (d, *J* = 8.7 Hz, 0.5H; rotamer 2), 8.41 (d, *J* = 8.6 Hz, 0.5H; rotamer 1), 8.13 - 8.04 (m, 2H), 7.85 - 7.79 (m, 1H), 7.6 (t, *J* = 7.5 Hz, 1H), 4.05 (brs, 1H), 3.21 (d, *J=* 7.3 Hz, 2H), 3.08 - 3.03 (m, 1H), 3.01 - 2.95 (m. 1H), 2.79 - 2.73 (m, 1H), 2.44 - 2.30 (m, 2H), 1.90 (bis. 3H), 1.76 - 1.68 (m, 1H), 1.57 -1.47 (m, 1H), 1.42 - 1.31 (m, 1H), 1.00 (t, *J* = 7.2 Hz, 3H). | 467.51 | 468.1 |
| 299 | Example 110 | 1H NMR (400 MHz, MeOD) δ 8.59 (s, 1H), 8.53 (s, 1H), 8.30 (br. s, 1H), 7.96 (br. s, 1H), 7.70 (br. s, 1H), 7.38 (br. s, 1H), 4.34 (br. s, 1H), 3.99 - 3.85 (m, 2H), 3.82 - 3.69 (m, 1H), 3.57 (br. d, J = 10.0 Hz, 1H), 3.30 - 3.23 (m, 2H), 3.09 - 2.94 (m, 2H), 2.45 - 2.01 (m, 4H), 1.94 - 1.67 (m, 2H) | 542.19 | 543.10 |
| 300 | Example 110 | 1H NMR (400 MHz, MeOD)) δ 8.59 (s, 1 H), 8.53 (s, 1 H), 8.48 - 8.16 (m, 1 H), 7.96 (s, 1 H), 7.70 (hr. s, 1 H), 7.38 (br. s, 1 H), 4.34 (br. s, 1 H), 3.99 - 3.87 (m, 2 H), 3.82 - 3.71 (m, 1 H), 3.56 (br. t, J = 9.5 Hz, 1 H), 3.30 (br. s, 2 H), 3.09 - 2.94 (m, 2 H), 2.44 - 2.01 (m, 4 H), 1.93 - 1.69 (m, 2 H) | 542.19 | 543.10 |
| 301 | Example 111 | 1H NMR (400 MHz, MeOD) δ 8.82 - 8.68 (m, 2H), 8.22 (s, 1H), 7.95 (s, 1H), 7.65 - 7.58 (m, 1H), 7.16 - 6.76 (m, 1H), 4.49 - 4.39 (m, 1H), 3.66 - 3.59 (m, 1H), 3.40 - 3.33 (m, 1H), 3.23 - 3.14 (m, 1H), 3.14 - 3.04 (m, 1H), 2.34 - 2.25 (m, 1H), 2.18 - 2.09 (m, 1H), 2.03 - 1.93 (m, 1Σi), 1.86 - 1.75 (m, 1H) | 384.15 | 385.10 |
| 302 | Example 112 | 1H NMR (400 MHz, MeOD) δ 8.63 (s, 1H), 8.42 (br., 1H), 8.32 - 8.09 (m, 1H), 7.73 (br., 1Σi), 7.51 (br., 1H), 4.69 - 4.26 (m, 1H), 4.69 - 4.26 (m, 1H), 3.74 - 3.69 (m, 2H), 3.67 - 3.59 (m, 3H), 3.36 (br. d, J = 13.2 Hz, 1H), 3.27 - 2.98 (m, 2H), 2.31 (br., 1H), 2.20 (br. t, J = 7.2 Hz, 1H), 2.12 (hr. t, J = 7.2 Hz, 2H), 1.95 (br. d, J = 12.3 Hz, 1H), 1.83 (br. d, J = 10.5 Hz, 1H), | 472.18 | 473.20 |
| 303 | Example 113 | 1H NMR (400 MHz, MeOD) δ 8.60 (s, 1H), 8.57 - 8.28 (m, 1H), 8.15 - 8.01 (m, 1H), 7.93 (s, 1H), 7.90 - 7.54 (m, 2H), 6.82 (br.s, 1H), 4.64 - 4.23 (m, 1H), 4.04 (s, 3H), 3.62 (br.d., J = 13.2 Hz, 1H), 3.35 (br.d., J = 13.6 Hz, 1H), 3.07 (br.s, 2H), 2.11 (br.s, 2H), 1.82 (br.d, J = 10.5 Hz, 2H) | 441.19 | 442.10 |
| 304 | Starting with 7-bromo indole and following Examples 9, 87,86, 10, 11 and Example 90. | ¹H NMR (500 MHz, DMSO) δ 8.85 (br s, 0.5H), 8.65 (br s, 0.5H), 8.33-8.20 (m, 3H), 8.03 (s, 1H), 7.77 (d, *J* = 7.0Hz, 1H), 7.47 (s, 1H), 7.37 (t, *J* = 8.0Hz, 1H), 5.81 (s, 2H), 4.03 (s, 1H), 3.23 (s, 3H), 3.20 (s, 1H), 2.97 (d, J = 10.5Hz, 1H), 2.70 (d, *J* = 4.5Hz, 3H), 2.67-2.57 (m, 2H), 2.04-1.90 (m, 1H), 1.83-1.71 (m, 1H), 1.65-1.46 (m, 2H). *Mixture of rotamers | 462.47 | 420.3 |
| 305 | Starting with (*S*)-*tert*-butyl 3-((5-iodo-4-(1-(phenylsulfonyl)-1H-indol-3-yl)pyrimidin-2-yl)amino)piperidine-1-carboxylate (from Example 9), following Example 91, and then Example 1 for the final step. | ¹H NMR (500 MHz, CD₃CN) δ 9.91 (s, 1H), 8.74 - 8.35 (m, 2H), 8.28 (s, 1H), 7.51 (d, *J* = 7.7 Hz, 1H), 7.34 - 7.08 (m, 2H), 6.87 - 6.48 (m, 1H), 4.43 - 4.07 (m, 1H), 3.28 (dd, *J* = 12.4, 3.7 Hz, 1H), 3.08 - 2.93 (m, 1H), 2.93 - 2.81 (m, 2H), 1.80 - 1.60 (m, 2H), 1.42 - 1.17 (m, 2H), 0.93 - 0.76 (m, 1H). | 393.43 | 394.1 |
| 306 | Example 114 | 1H NMR (400 MHz, DMSO-d6) δ = 12.50 - 12.26 (m, 1H), 9.45 (br. s, 1H), 9.33 - 9.05 (m, 1H), 8.63 (br. s, 1H), 8.53 - 8.24 (m, 1H), 8.15 (br. s, 2H), 8.04 (br. s, 1H), 7.89 - 7.67 (m, 1H), 4.33 (br. d, J = 14.3 Hz, 1H), 3.37 (br. d, J = 10.8 Hz, 1H), 3.16 (br. d, J= 12.0 Hz, 1H), 2.99 - 2.76 (m, 2H), 2.19 - 1.97 (m, 1H), 1.96 - 1.69 (m, 2H), 1.66 - 1.46 (m, 1H), | 405.14 | 406.20 |
| 307 | Starting with 7 bromo indole and following Examples 9, 86, 10, 11 and 90. | ¹H NMR (D₂O, 500MHz): δ(ppm) = 8.41 (s, 1H), 8.38 (br s, 1H), 8.13 (dd, *J* = 8.0 Hz, 1.0 Hz, 1H), 7.71 (s, 1H), 7.46 (dd, *J* = 7.0 Hz, 1.0 Hz, 1H), 7.33 (dd, *J* = 8.0 Hz, 7.5Hz, 1Σi), 4.37-4.30 (m, 1H), 3.91 (s, 3H), 3.60 (dd, *J* = 11.5 Hz, 3.0 Hz, 1H), 3.39 (t, *J* = 4.0 Hz, 1H), 3.14-3.07 (m, 2H), 2.77 (s, 3H), 2.24-2.18 (m, 1H), 2.15-2.09 (m, 1H), 1.94-1.86 (m, 1H), 1.82-1.75 (m, 1H). *Formic acid salt | 478.47 | 408.2 |
| 308 (tentatively assigned to P1) and 349 (tentatively assigned to P2) | Example 115 | 1H NMR (P1) (400MHz, MeOD) δ 8.62 - 8.29 (m, 2H), 7.98 (s,1H), 7.72 (s, 1H), 7.40 (br., 1H), 4.42 -4.16 (m, 2H), 4.11 - 3.81 (m, 3H), 3.77 - 3.56 (m, 1H), 3.42 - 3.35 (m, 1H), 3.09 (br., J = 8.8 Hz, 1H), 2.77 (br. d, J = 10.4 Hz, 2H), 2.17 (br. s, 1H), 1.98 -1.87 (m, 1H), 1.81 - 1.58 (m, 2H) 1H NMR(P2) (400MHz, MeOD) δ 8.53 - 8.19 (m, 2H), 7.86 (s, 1H), 7.60 (s, 1H), 7.33 - 7.22 (m, 1H), 4.29 - 4.02 (m, 2H), 3.98 - 3.72 (m, 3H), 3.66 - 3.46 (m, 1H), 3.25 (br, s, 1H), 2.92 (br., J = 12.6 Hz, 1H), 2.72 - 2.51 (m, 1H), 2.72 - 2.47 (m, 1H), 2.05 (br. s, 1H), 1.75 (br. s, 1H), 1.66 - 1.41 (m, 2H) | 510.18 | 511.20 |
| 309 | Example 116 | 1H NMR: (400 MHz, MeOD) δ 8.64 (br. s, 1H), 8.45 (br. s. 1H), 8.36 - 8.04 (m, 1H), 7.76 (br. s, 1H), 7.65 - 7.37 (m, 1H), 4.66 (br. s, 0.6H), 4.37 (br. s, 0.2H), 3.97 - 3.71 (m, 2H), 3.70 - 3.48 (m, 3H), 3.44 - 3.32 (m, 2H), 3.09 (br. d, J = 12.8 Hz,1H), 2.33 (br. s, 1H), 2.13 (br. d, J = 13.8 Hz, 1H), 2.04 -1.82 (m, 4H), 1.81 - 1.70 (m, 1H), 1.65 (q, J = 7.2 Hz, 1M), 1.06 - 0.88(m, 3H). | 502.23 | 503.20 |
| 310 | Done as Example 137 | 1H NMR (400MHz, METHANOL-d4): δ = 8.60 (s, 1H), 8.33 (br s, 1H), 7.97 (br s, 1H), 7.90 - 7.76 (m, 2H), 7.64 - 7.41 (m, 2H), 6.57 (t, J=6.8 Hz, 1H), 3.72 - 3.67 (m, 1H), 3.65 - 3.57 (m, 1H), 3.77 - 3.54 (m, 1H), 3.20 - 3.01 (m, 2H), 2.37 - 2.07 (m, 2H). 1.98 - 1.75 (m, 2H) | 454.2 | 455.0 |
| 311 | Done as Example 138 | 1H NMR (400MHz, METHANOL-d4): δ ppm 8.61 (s, 1 H) 8.43 (br s, 1 H) 8.03 (s, 1 H) 7.46 (br s, 1 H) 7.28 (br s, 1 H) 3.52 - 3.71 (m, 2 H) 3.31 - 3.43 (m, 2 H) 3.09 (br d, J=10.52 Hz, 2 H) 2.43 - 2.59 (m, 1 H) 2.37 (s, 2 H) 2.03 - 2.31 (m, 1 H) 1.72 - 2.01 (m, 1 H) | 493.2 | 494.1 |
| 312 | Starting with 4-bromobenzo[d]thiazol-2-amine and following procedure for Example 141 | 1H NMR (400MHz, METHANOL-d4): δ = 8.548-8.652 (m, 3H), 8.197 (br s, 1H), 7.846-7.866 (d,J=8 Hz, 1H), 7.719 (s, 1H), 7.511-7.549 (m, 2H), 3.658-3.687 (dd,J=11.6 Hz, 1H), 3.371-3.405 (dd,J=13.6 Hz, 1H), 3.083-3.329 (m, 2H), 2.310 (s, 1H), 2.119 (s, 2H), 1.856 ppm (s, 1H) | 509.2 | 510.1 |
| 313 | Starting with 5-bromo-1H-benzo[d]imidazole and following procedure for Example 141 | 1H NMR (400MHz, D2O): δ = 8.97-9.11 (m, 1H), 8.31 (br. s, 1H), 7.68-8.06 (m, 5H), 7.48 (brs, 1H), 7.27 (br s, 1H), 4.19 (br s, 1H), 3.46 (br s, 1H), 3.29 (br d, J=12.6 Hz, 1H), 2.95-3.09 (m, 2H), 1.95-2.20 (m, 2H), 1.58-1.89 ppm (m, 2H) | 477.2 | 478.2 |
| 314 | Starting with (3-carbamoylphenyl)boronic acid and following procedure for Example 140 | 1H NMR (400MHz, D2O): δ = 8.10 (br s, 1H), 7.22-7.98 (m, 6H), 6.79-7.13 (m, 2H), 3.94 (brs, 1H), 3.29 (br t, J=13.3 Hz, 2H), 2.83-3.04 (m, 2H), 1.38-1.99 ppm (m, 4H) | 480.2 | 481.2 |
| 315 | Starting with 5-bromo-2-hydroxybenzonitrile and following procedure for Example 141 | 1H NMR (400MHz, METHANOL-d4): δ = 8.60 (s, 1H), 8.43 (br s, 1H), 8.30 - 8.10 (m, 1H), 7.90 - 7.78 (m, 2H), 7.55 - 7.54 (m, 1H), 7.75 - 7.51 (m, 1H), 7.07 (d, J=8.6 Hz, 1H), 3.69 - 3.55 (m, 3H), 3.34 (s, 1H), 3.11 (br d, J=11.2 Hz, 1H), 2.35 (br s, 1H), 2.14 (br d, J=15.0 Hz, 1H), 2.21 - 2.08 (m, 1H), 2.05 - 1.17 (m, 2H) | 478.2 | 479.2 |
| 316 | Starting with (6-bromopyridin-2-yl)methanol and following procedure for Example 141 | 1H NMR (400MHz, D2O): δ = 8.33-8.55 (m, 2H), 7.93-8.23 (m, 3H), 7.69-7.92 (m, 2H), 7.49 (br d, J=8.1 Hz, 1H), 4.97 (s, 2H), 4.22 (br s, 1H), 3.24-3.34 (m, 2H), 3.02 (br d, J=10.4 Hz, 2H), 1.95-2.17 (m, 2H), 1.62-1.89 ppm (m, 2H) | 468.2 | 469.2 |
| 317 | Starting with (E)-styrylboronic acid and following procedure for Example 140 | 1H NMR (400MHz, DMSO-d6): δ = 11.84 (br s, 1H), 8.62 - 8.48 (m, 1H), 8.41 - 8.24 (m, 1H), 7.86 (br s, 1H), 7.76 - 7.62 (m, 4H), 7.56 - 7.45 (m, 1H), 7.44 - 7.33 (m, 4H), 7.31 - 7.18 (m, 1H), 4.04 - 3.86 (m, 1H), 3.10 (br d, J=9.3 Hz, 1H), 2.83 (br d, 1=12.2 Hz, 1H), 1.99 (br d, J=19.6 Hz, 1H). 1.74 - 1.40 (m, 4H) | 463.2 | 464.2 |
| 318 | Starting with (5-(hydroxymethyl)pyridin-3-yl)boronic acid and following procedure for Example 140 | 1H NMR (400MHz, D2O): δ = 8.91 (br s, 1H), 8.73 (br s, 1H), 8.45-8.63 (m, 2H), 8.00-8.24 (m, 2H), 7.83 (br s, 1H), 7.49 (br s, 1H), 4.86 (br s, 2H), 4.27 (br s, 1H), 3.50 (br d, J=13.0 Hz, 1H), 3.30 (br d, J=12.3 Hz, 1H), 2.94-3.13 (m, 2H), 1.97-2.21 (m, 2H), 1.62-1.90 ppm (m, 2H) | 468.2 | 469.2 |
| 319 | Starting with 3-bromo-6-(trifluoromethyl)-1H-pyrrolo[3,2-c]pyridine and following procedure for Example 141 | 1H NMR (400MHz, DMSO-d6): δ = 9.411 (s, 1H), 8.605 (s, 1H), 8.478 (m, 1H), 8.285 (s, 1H), 8.146 (s, 1H), 7.997 (s, 1H), 7.855 (s, 1H), 7.646 (m, 1H), 3.681-3.657 (m, 1H), 3.643-3.541 (m, 2H), 3.327 (m, 1H), 3.079-3.029 (m, 1Σi), 2.256 (m, 1H), 2.135-2.099 (m, 1Σi), 1.949 (m, 1H), 1.847-1.772 (m, l H) | 545.2 | 546.2 |
| 320 | Starting with 3-bromo-1-methyl-6-(trifluoromethyl)-1H-pyrrolo[3,2-c]pyridine and following procedure for Example 141 | 1H NMR (400MHz, DMSO-d6): δ = 9.316 (s, 1H), 8.596 (s, 1H), 8.434 (m, 1H), 8.301 (s, 1H), 8.011 (s, 1H), 7.949 (s, 1H), 7.815 (s, 1H), 7.605 (m, 1H), 4.089 (s, 3H), 3.714-3.690 (m, 1H), 3.598-3.575 (m, 2H), 3.354 (m, 1H), 3.073-3.048 (m, 1H), 2.231 (m, 1H), 2.092 (m, 1H), 1.936 (m, 1H), 1.816-1.789 (m, 1H) | 559.2 | 560.2 |
| 321 | Starting with (1-methyl-6-oxo-1,6-dihydropyridin-3-yl)boronic acid and following procedure for Example 139 | 1H NMR (400MHz, DMSO-d6): δ = 8.596 (s, 1H), 8.395-8.296 (m, 1H), 8.130-8.049 (m, 3H),7.676 (s, 1H), 7.496-7.430 (m, 1H), 6.794-6.771 (d, J=9.2 Hz, 1H), 3.743 (s, 3H), 3.686-3.675 (m, 1H), 3.663-3.546 (m, 2H), 3.365-3.332 (m, 1H), 3.097-3.066 (m, 1H), 2.271-2.228 (m, 1H), 2.107 (m, 1H), 1.952 (m, 1H), 1.861-1.804 (m, 1H) | 468.2 | 469.0 |
| 322 | Starting with 8-bromoquinazolin-2-amine and following procedure for Example 141 | 1H NMR (400MHz, D2O): δ = 8.209-7.740 (m, 3H), 8.732 (br s, 1H), 8.603 - 8.537 (d, J=26.4Hz,2H,), 8.196 -8.176 (m, 2H),8.053 (br s, 1H),7.838 (br s, 1H), 7.491 (br s, 1H),4.273 (br s, 1H), 3.525 -3.489 (d, 1H), 3.321 -3.288 (d, 1H), 3.082 2.997 (m, 2H),2.125 - 1.815 (m, 2H), 1.752 -1.701 (m, 2H) | 504.2 | 505.2 |
| 323 | Starting with (3-bromopyridin-2-yl)methanol and following procedure for Example 141 | 1H NMR (400MHz, D2O): δ = 8.62 - 8.24 (m, 3H), 8.19 - 7.72 (m, 3H), 7.50 - 7.35 (m, 1H), 7.19 - 6.98 (m, 1H), 4.82 (br d, J=14.8 Hz, 1H), 4.19 (br s, 1H), 3.46 - 3.12 (m, 3H), 2.92 (br s, 2H), 1.95 (br s, 2H), 1.65 (br s, 2H) | 468.2 | 469.2 |
| 324 | Starting with (3-(2-hydroxyethyl)phenyl)boroni c acid and following procedure for Example 140 | 1H NMR (400MHz, D2O): δ = 8.209-7.740 (m, 3H),7.426 -7.183((m, 5H), 1.055 (br s, 1H),1.819 (br s, 1H), 3.301 -3.299 (t, 2H), 2.29.3 -2.800(m, 4H), 1.921-1.518 (m, 4H) | 481.2 | 482.2 |
| 325 | Starting with 7-bromoisoquinoline and following procedure for Example 141 | 1H NMR (400MHz, D2O): δ = 9.296-9.247 (d, J=19.6Hz,2H),8.251-7.892((m, 8H), 7.362 -7.290 (m, 1H),7.194-7.146 (m, 1Σi), 4.128 (s, 1H), 3.493-3.464(d, J=11.6Hz,1H), 3.346-3.283 (t, J=25.2Hz, 2H), 2.070-2.038 (m, 2H),1.844-1.816 (m, 1H), 1.685 (br s, 1H), | 488.2 | 489.1 |
| 326 | Starting with 3-bromobenzenesulfonamide and following procedure for Example 141 | 1H NMR (400MHz, D2O): δ = 8.312 (s, 1H),7.942-7.691 ((m, 4H), 7.527 -7.489 (m, 2H),7.422-7.141 (m, 1H), 4.075 (s, 1H), 3.398-3.257(m,2H), 2.981 (t, 3H), 1.981-1.955 (d, J=26Hz, 2H),1.738-1.562 (m,2H) | 516.2 | 517.4 |
| 327 | Starting with (2-ethoxyphenyl)boronic acid and following procedure for Example 140 | 1H NMR (400MHz, METHANOL-d4): δ = 8.58 (s, 1H), 8.37 (br s, 1H), 8.10 (br s, 1H), 7.69 (br s, 1H), 7.54 - 7.24 (m, 1H), 7.11 - 6.97 (m, 1H), 4.13 - 3.93 (m, 1H), 3.60 (br d, J=12.6 Hz, 1H), 3.37 - 3.31 (m, 2H), 3.07 (br s, 1H), 2.38 - 2.05 (m, 1H), 2.00 -1.74 (m, 1H), 1.32 (t, J=6.9 Hz, 3H) | 481.2 | 482.2 |
| 328 | Starting with 2-bromopyrazine and following procedure for Example 141 | 1H NMR (400MHz, D2O): δ=8.671-8.592(m,1H),8.394-8.235(m,3H),8.000(br s,1H),7.692-7.217(m,3H).4.099(br s,1H),3.460-3.310(m,2H),3.049-2.995(m,2H),1.991-1.609(m,4H) | 439.2 | 440.1 |
| 329 | Starting with (2H-indazol-6-yl)boronic acid and following procedure for Example 140 | 1H NMR (400 MHz, DMSD-d6) δ 11.93 (br s, 1H), 8.81-9.09 (m, 2H), 8.61 (br s, 1H), 8.49 (br s, 1H), 8.30 (br s, 1H), 8.08 (s, 1H), 8.02 (br d, J=7.72 Hz, 1H), 7.90 (br s, 1H), 7.84 (d, J=8.38 Hz, 1H), 7.79 (br s, 1H), 7.74 (s, 1H), 7.39-7.61 (m, 2H), 4.23-4.43 (m, 1H), 3.40 (br d, J=9.92 Hz, 1H), 3.17 (br s, 1H), 2.86 (br d, J=9.48 Hz, 2H), 1.98-2.18 (m, 1H), 1.90 (br s, 1H), 1.75 (br s, 1H), 1.59 (br s, 1H), 1.49-1.69 (m, 1H), 1.47-1.84 (m, 1H) | 477.2 | 478.1 |
| 330 | Starting with (3,5-dimeihylisoxazol-4-yl)boronic acid and following procedure for Example 140 | 1H NMR (400MHz, METHANOL-d4)δ=8.440-8.316(m,2H),7.788(s,1H),7.305(s,1H),7.049(s,1H), 4.086(br s,1H),2.979(m,1H),2.684-2.633(m,2H),2.345(s,3H),2.189(m,1H),2.087(ni,l H),1.801(m,1H),1571(m,3H) | 456.2 | 457.1 |
| 331 | Starting with 4-bromobenzenesulfonamide and following procedure for Example 141 | 1H NMR (400MHz, DMSO-d6): δ = 12.30 - 12.54 (m, 1 H) 10.07 (s, 1 H) 9.39 - 9.78 (m, 2 H) 8.57 - 8.75 (m, 3 H) 8.39 - 8.51 (m, 1 H) 8.19 - 8.32 (m, 2 H) 8.08 - 8.16 (m, 1 H) 8.01 (br s, 1 H) 7.68 (br s, 1 H) 7.24 - 7.54 (m, 1 H) 4.25 - 4.58 (m, 1 H) 3.39 (br s, 1 H) 3.15 (br s, 1 H) 2.81 - 3.02 (m, 2 H) 1.96 - 2.21 (m, 1 H) 1.73 - 1.94 (m, 2 H) 1.64 (br d, J=11.25 Hz, 1 H) | 516.2 | 517.1 |
| 332 | Starting with 2-bromothiazole and following procedure for Example 141 | 1H NMR (400MHz, D2O): δ=8.357-8.333(m,1H),7.998-7.558(m,5H),7.277(s,1H),4.1 19(br s,1H),3.489-3.331(m,2H),3.076-3.023(m,2H),2.053-2.026(m,2H),1.834-1.646(m,1H),2.081(m,2H) | 444.1 | 445.1 |
| 333 | Done as Example 139 | 1H NMR (400MHz, DMSO-d6) δ = 11.95 (br d, J=10.8 Hz, 1H), 11.28 - 11.09 (m, 2H), 9.34 - 8.99 (m, 2H), 8.61 (br s, 1H), 8.44 - 8.14 (m, 1H), 8.05 (br s, 1H), 7.86 (s, 1H), 7.76 (br d. J=5.5 Hz, 1H), 7.61 (d, J=5.7 Hz, 1H), 7.64 - 7.58 (m, 1H), 7.39 - 7.26 (m, 1H), 4.42 - 4.21 (m, 1H), 3.45 - 3.34 (m, 1H), 3.17 (br s, 1H), 3.03 - 2.81 (m, 2H), 2.17 - 1.87 (m, 2H), 1.84 - 1.52 (m, 2H) | 471.2 | 472.2 |
| 334 | Starting with 7 -bromo-3.4-dihydroquinolin-2(1H)-one and following procedure for Example 141 | 1H NMR (400MHz, DMSO-d6) Shift = 11.95 (br s, 1H), 10.15 (s, 1H), 9.13 - 8.95 (m, 1H), 9.08 - 8.91 (m, 1H), 8.68 - 8.17 (m, 2H). 8.07 - 7.78 (m, 2H), 7.63 (br s, 1H), 7.48 - 7.06 (m, 4H), 4.27 (br s, 1H), 4.05 (br s, 2H), 3.38 (br d, J=9.7 Hz, 1H), 3.16 (br s, 1H), 2.99 - 2.78 (m, 4H), 2.18 - 1.87 (m, 2H), 1.80 - 1.50 (m, 2H) | 506.2 | 507.2 |
| 335 | Starting with (5-fluoropyridin-3-yl)boronic acid and following procedure for Example 1.40 | 1H NMR (400MHz, D2O): δ=8.512(s,1H),8.335-8.305(d,J=12Hz,2H),8.026-8.003(d,J=9.2Hz,1H),7.925-7.799(m,2H),7.282(s, 1H),7.108-7.089(d,J=7.2Hz,1H),4.091(br s, 1H),3.434-3.409(m,1H),3.301-3.269(M,1H),3.017-2.959(m,2H),1.993(hr s,2H)1.779-1.743(m,1H),1.614-1.590(m,1H) | 456.2 | 457.2 |
| 336 | Starting with (4-methylpyridin-3-yl)boronic acid and following procedure for Example 140 | 1H NMR (400MHz, METHANOL-d4): δ = 8.68 (br s, 1H), 8.62 (br d, J=5.9 Hz, 1H), 8.54 (s, 1H), 8.45 (br s, 1H), 7.98 (br d, J=5.9 Hz, 1H), 7.54 (br s, 1H), 7.24 (br s, 1H), 3.60 - 3.47 (m, 1H), 3.27 (br s, 2H), 2.97 (br s, 1H), 2.57 (s, 3H), 2.25 - 1.96 (m, 1H), 1.92 - 1.66 (m, 2H) | 452.2 | 453.2 |
| 337 | Starting with (1-methyl-1H-indazol-6-yl)boronic acid and following procedure for Example 140 | 1H NMR (400MHz, D2O): δ = 7.95 - 7.63 (m, 1H), 7.66 (br s, 1H), 7.31 (br s, 2H), 6.92 (br s, 4H), 4.00 (br s, 1H), 3.65 (br s, 3H), 3.27 (br s, 2H), 3.01 - 2.39 (m, 3H), 1.85 - 1.42 (m, 4H) | 491.2 | 492.2 |
| 338 | Starting with N-(3-(4,4,5,5-tetramethyl-1,3,2-dioxaborolan-2-yl)benzyl)acetamide and following procedure for Example 140 | 1H NMR (400MHz, METHANOL-d4): δ = 8.63 (s, 1H), 8.48 (br s, 1H), 8.25 (br s, 1H), 7.83 - 758 (m, 2H), 7.45 (br t, J=7.6 Hz, 1H), 7.30 (br d, J=6.8 Hz, 1H), 4.47 (s, 2H), 3.69 - 3.61 (m, 1H), 3.39 (br d, J=14.7 Hz, 3H), 3.22 - 3.05 (m. 1H), 2.46 - 2.12 (m, 2H), 2.07 - 1.80 (m, 5H) | 508.2 | 509.2 |
| 339 | Starting with 4-bromoquinoline and following procedure for Example 141. | 1H NMR (400MHz, DMSO-d6): δ = 12-33 (br a, 1H), 9.29 (br s, 1H), 9.41 - 9.19 (m, 1H), 8.68 (br s, 1H), 8.47 (br d, J=8.2 Hz, 1H), 8.38 - 8.23 (m, 1H), 8.15 (br d, J=7.2 Hz, 2H), 8.05 (br d, J=17.6 Hz, 2H), 7.96 - 7.81 (m, 2H), 7.60 - 7.39 (m, 1H), 4.49 - 4.23 (m, 1H), 3.24 - 2.80 (m, 4H), 2.13 - 2.03 (m, 1H), 1.91 (br s, 1H), 1.63 (br s, 1H), 1.78 - 1.52 (m, 1H). | 488.2 | 489.0 |
| 340 | Done as Example 140 | 1H NMR (400MHz, METHANOL-d4): δ = 8.62 (s. 1H), 8.43 (br s, 1H), 8.07 (br s, 1H), 7.88 - 7.78 (m, 3H), 7.69 - 7.48 (m. 3H), 3.71 - 3.53 (m, 6H), 3.24 - 3.02 (m, 2H), 2.31 (br s, 1H), 2.15 (br d, J=15.2 Hz, 1H), 2.09 - 1.81 (m, 7H) | 534.2 | 535.0 |
| 341 | Done as Example 141 | 1H NMR (400MHz, METHANOL-d4): δ ppm 8.56 (s, 1 H) 8.38 (br s, 1 H) 8.13 (br s, 1 H) 7.57 - 7.86 (m, 2 H) 7.28 - 7.46 (m, 6 H) 6.87 - 7.16 (m, 4 H) 3.60 (br t, J=5.26 Hz, 1 H) 3.31 - 3.39 (m, 2 H) 2.99 - 3.27 (m, 2 H) 1.74 - 2.34 (m, 1 H) | 529.2 | 530.2 |
| 342 | Starting with 6-bromo-2,3-dihydrobenzo[b][1,4]dioxin e and following procedure for Example 141 | 1H NMR (400MHz, METHANOL-d4): δ = 8.60 (s, 1H), 8.38 (br s, 1H), 8.03 (br s, 1H), 7.67 (br s, 1H), 7.51 (br s, 1H), 7.14-7.25 (m, 2H), 6.93 (d, J=8.9 Hz, 1H), 4.31 (a, 4H), 3.61-3.68 (m, 1H), 3.60-3.69 (m, 1H), 3.38 (br s, 2H), 3.02-3.25 (m, 2H), 2.09-2.43 (m. 2H), 1.75-2.01 ppm (m, 2H) | 495.2 | 496.0 |
| 343 | Starting with 6-bromobenzo[d]thiazole and following procedure for Example 141 | 1H NMR (400MHz, METHANOL-d4): δ = 9.36-9.42 (m, 1H), 8.63 (s, 1H), 8.36-8.55 (m, 2H), 8.02-8.26 (m, 2H), 7.83-7.96 (m, 2H), 7.71 (br s, 1H), 4.60 (br s, 1H), 3.60-3.71 (m, 1H), 3.36-3.42 (m, 1H), 3.03-3.27 (m, 2H), 2.11-2.43 (m. 2H), 1.80-2.04 ppm (m, 2H) | 494.2 | 495.0 |
| 344 | Starting with isoquinolin-5-ylboronic acid and following procedure for Example 140 | 1H NMR (400MHz, DMSO-d6): δ = 12.30 - 12.54 (m, 1 H) 10.07 (s, 1 H) 9.39 - 9.78 (m, 2 H) 8.57 - 8.75 (m, 3 H) 8.39 - 8.51 (m, 1 H) 8.19 - 8.32 (m, 2 H) 8.08 - 8.16 (m, 1 H) 8.01 (br a, 1 H) 7.68 (hr s, 1 H) 7.24 - 7.54 (m, 1 H) 4.25 - 4.58 (m, 1 H) 3.39 (br s, 1 H) 3.15 (br s, 1 H) 2.81 - 3.02 (m, 2 H) 1.96 - 2.21 (m, 1 H) 1.73 - 1.94 (m, 2 H) 1.64 (br d, J=11.25 Hz, 1 H) | 488.2 | 489.1 |
| 345 | Starting with 1-bromo-3-fluorobenzene and following procedure for Example 141 | 1H NMR (400MHz, DMSO-d6): δ = 12.12 (br s, 1H), 8.64 - 8.26 (m, 2H), 8.00 - 7.85 (m, 2H), 7.82 - 7.62 (m, 2H), 7.58 - 7.39 (m, 4H), 7.15 (dt, J=2.0, 7.9 Hz, 1H), 4.20 (br t, J=6.6 Hz, 1H), 3.15 (s, 1H), 3.01 (br s, 1H), 2.85 - 2.67 (m, 2H), 2.12 - 1.92 (m, 1H), 1.80 (br s, 1H), 1.73 - 1.47 (m, 2H), 1.42 - 1.17 (m, 1H), 0.94 - 0.80 (m, 1H) | 455.2 | 456.1 |
| 346 | Example 118 | 1H NMR (400 MHz, MeOD) δ 8.68 (br., 1H), 8.52 (br., 1H), 8.33 (br., 1H), 8.09 (br., 1H), 7.90-7.65 (m, 1H), 4.70-4.30 (m, 1H), 3.65 (br dd, J = 3.61, 12.29 Hz, 1H), 3.44-3.35 (m. 2H), 3.27-3.04 (m, 2H), 2.30 (br., 1H), 2.20-2.10 (m, 1H), 2.03-1.79 (m, 2H), 1.32-1.27 (m, 6H) | 467.16 | 468.10 |
| 347 | Example 116 | 1H NMR: (400 MHz, MeOD) δ 8.59 (s, 1H), 8.57 - 8.11 (m, 2H), 7.94 (s, 1H), 7.68 (d, J = 7.6 Hz, 1H), 7.37 (br. t, J = 7.2 Hz, 1H), 4.35 (br. s, 1H), 3.93 - 3.82 (m, 1H), 3.80 - 3.71 (m, 0.55H), 3.68 - 3.50 (m, 3H), 3.41 (br. d, J = 11.1 Hz, 0.41H),3.30 - 3.25 (m, 1H), 3.08 - 2.94 (m, 2H), 2.20 (br. s, 1H), 2.13 - 2.04 (m, 1H), 2.02 - 1.93 (m, 1H), 1.92 - 1.71 (m, 4H), 1.64 (q, J = 7.4 Hz, 1H), 1.09 - 0.90 (m, 3H). | 502.23 | 503.20 |
| 348 | Example 117 | 1H NMR (400MHz, MeOD) δ 8.85 - 8.46 (m, 2H), 8.31 - 8.08 (m,1Σi), 7.78 (br., 1H), 7.65 - 7.39 (m, 1H), 4.97 (br., 2H), 4.65 - 4.27 (m, 1Σi), 3.83 (br., 2H), 3.62 (br., J = 10.8 Hz, 1H), 3.37 (br., J = 13.0 Hz, 1H), 3.28 - 3.01 (m, 2H), 2.28 (br., 1H), 2.18 - 2.07 (m, 1H), 2.01 - 1.77 (m, 2H) | 451.13 | 452.10 |
| 350 | Starting with 3-iodo-1H-indole-6-carbonitrile (from Example 36), following Examples 3, 36, 11, 21 and then Example 92 for the final step. | ¹H NMR (500 MHz, MeOD) δ 8.61 (s, 1H), 8.54 (s, 2H), 8.04 (s, 1H), 7.45 (d, *J* = 7.6 Hz, 1Σi), 4.28 (s, 1H), 3.45 (s, 1Σi), 3.17 (s, 1H), 2.09 (s, 1H), 1.91 (d, *J =* 12.2 Hz, 2H), 1.77 (s, 1H), 1.40 (d, *J =* 12.4 Hz, 6H). | 414.43 | 415.1 |
| 351 | Starting from 3-iodo-1H-indole-6-caibonitrile (from Example 36), following Examples 3, 36, 11 and 21 to give 3-(2-[methylsulfonyl)-5-(trifluoromethyl)pyrimidin-4-yl)-1-(phenylsulfonyl)-1H-iodole-6-carbonitrile and then following Examples 93 , 1 and 2. | ¹H NMR (500 MHz, DMSO) δ 12.26 (br s, 1H), 8.62 (d, *J =* 11.0 Hz, 1H), 8.50 (d, *J* = 8.3 Hz, 0.5H; rotamer 1), 8.32 (d, *J* = 8.4 Hz, 0.5H; rotamer 2), 8.18 (s, 1H), 8.14 - 8.04 (m, 2H), 8.02 (d, *J* = 6.2 Hz, 1H), 7.49 (dd, *J* = 8.4, 1.5 Hz, 1H), 4.77 - 4.50 (m, 1H), 4.24 - 4.07 (m, 1H), 3.18 - 3.06 (m, 2H), 3.03 - 2.92 (m, 1H), 2.47 - 2.41 (m, 1H), 2.16 - 2.05 (m, 1H), 1.66 - 1.54 (m, 1H). | 404.36 | 405.2 |
| 353 | Starting with (*S*)-*tert*-butyl 3-((4-(7-bromo-1H-indol-3-yl)-5-(trifluoromethyl)pyrimidin-2-yl)amino)piperidine-1-carboxylate (Example 11) and following Example 94. | ¹H NMR (500 MHz, DMSO) δ 8.63 (d, *J* = 7.5 Hz, 0.5H; rotamer 1), 8.59 (d, *J* = 16.5 Hz, 1H), 8.41 (d, *J* = 7.5 Hz. 0.5H; rotamer 2), 8.35 (brs, 1H), 7.99 - 7.89 (m, 2H), 7.53 - 7.47 (m, 1H), 7.33 - 7.23 (m, 1H), 4.12 (bra, 1H), 3.28 - 3.18 (m, 1H), 3.05 - 2.94 (m, 1H), 2.75 - 2.63 (m, 2H), 2.06 - 1.94 (m, 1H), 1.82 (s, 3H), 1.80 (s, 3H), 1.80 - 1.74 (brs, 1H), 1.65 - 1.45 (m, 2H). ³¹P NMR (203 MHz, DMSO) δ 37.59 (s). | 437.40 | 438.2 |
| 354 | Starting with (*S*)-*tert*-butyl 3-((4-(6-bromo-1-(phenylsutfonyl)-1H-indol 3-yl)-5-(trifluoromethyl)pyrimidin-2-yl)amino)piperidine-1-carboxylate, following Examples 2 and 94. | ¹H NMR (500 MHz, DMSO) δ 12.22 (brs, 1H), 8.62 (s, 0.5H; rotamer 1), 8.58 (s, 0.5H; rotamer 2), 8.50 - 8.27 (m, 2H), 8.00 - 7.87 (m, 3H), 7.49 (dt, *J* = 18.3,9.3 Hz, 1H), 4.16 (brs, 1H), 3.24 (brs, 1H), 3.02 (brs, 1H), 2.79 - 2.65 (m, 2H), 2.08 - 1.94 (m, 1H), 1.80 (brs, 1H), 1.69 (s, 3H), 1.67 (s, 3H), 1.64 - 1.49 (m, 2H). ³¹P NMR (203 MHz, DMSO) δ 33.16 (s). | 437.40 | 438.2 |
| 355 | Starting with 3-(2-(methylsulfonyl)-5-(trifluoromethyl)pyrimidin-4-yl)-1-(phenylsulfonyl)-1H-indole-6-carbonitrile and (3*R*,4*R*)-tert-butyl 3-amino-4-hydroxypiperidine-1-carboxylate, following Example 93, and then Examples 1 and 2 for the final steps. | ¹H NMR (500 MHz, DMSO) δ 8.60 (d, *J* = 120 Hz, 1H), 8.56 (d. *J* = 8.3 Hz, 0.5H), 8.44 (d, *J* = 8.3 Hz, 0.5H), 8.33 (s, 1H), 8.07 (d, J = 19.1 Hz, 1H), 8.02 (s, 1H), 7.92 (d, *J* = 7.8 Hz, 0.5H), 7.81 (d, *J* = 8.2 Hz, 0.5H), 7.47 (dd, *J* = 8.4, 1.2 Hz, 1H), 3.96 (d, *J =* 13.5 Hz, 2H), 3.68 - 3.55 (m, 2H), 3.24 - 3.12 (m, 1H), 3.02 (s, 1H), 2.71 - 2.59 (m, 1H), 2.59 - 2.52 (m, 1H), 1.95 (d, *J* = 12.2 Hz, 1H), 1.49 (d, *J* = 9.9 Hz, 1H). | 402.39 | 403.2 |
| 356 | Same as in Example 80 2-(isopropyldisulfanyl) propane instead of (methyldisulfanyl) methane in Step 1 | 1H NMR (400 MHz, MeOD) δ 8.58 (d, J = 7.94 Hz, 1H), 8.45 (br., 1H), 8.25 (s, 1H), 7.85 (s, 1H), 7.69 (d, J = 7.50 Hz, 1H), 7.37 (t, J = 7.83 Hz, 1H), 4.34-4.19 (m, 1H), 3.53 (dd, J = 3.53,12.13 Hz, 1H), 3.41 (quin, J = 6.78 Hz, 1H), 3.27 (br., J = 4.30 Hz, 1H), 3.08-2.97 (m, 2H), 2.76 (q, J = 7.50 Hz, 2H), 2.23-2.05 (m, 2H), 1.92-1.72 (m, 2H), 1.29 (d, J = 6.84 Hz, 6H), 1.20 (t, J = 7.50 Hz, 3H) | 427.56 | 428.10 |
| 357 | Example 119 | 1H NMR (400 MHz, MeOD) δ 8.74 - 8.52 (m, 2H), 8.37 (br. s, 0.5 H), 8.06 (br. s, 1H), 7.95 (s, 1H), 7.63 - 7.53 (m, 1H), 4.34 (br. s, 1H), 3.56 (br. d, J = 10.6 Hz, 1H), 3.30 - 3.23 (m, 1H), 3.12 - 2.90 (m, 2H), 2.70 (s, 6H), 2.19 (br. s, 1H), 2.12-1.95 (m, 1H), 1.92 - 1.64 (m, 2H) | 468.16 | 469.10 |
| 358 | Starting from (2*R*,5*S*)-1-*tert*-butyl 2-methyl 5-azidopiperidine-1,2-dicarboxylate (from Example 35) and following Examples 39, 95, and 1 for the final step. | ¹H NMR (500 MHz, DMSO) δ 12.02 (brs, 1H), 8.63 (s, 0.5H; rotamer 1), 8.59 (s, 0.5H; rotamer 2), 8.51 (d, *J* = 8.4 Hz, 0.5H; rotamer 1), 8.41 (brs, 1H), 8.33 (d, *J* = 8.4 Hz, 0.5H; rotamer 2), 8.07 (d, *J* = 3.8 Hz, 1H), 8.02 (d, *J* = 7.9 Hz, 1H), 7.95 - 7.88 (m, 1H), 7.47 (dd, *J* = 8.4, 1.5 Hz, 1H), 3.98 - 3.92 (m, 2H), 3.24 - 3.18 (m, 1H), 2.62 - 2.54 (m, 1H), 2.34 (s, 3H), 2.16 - 2.08 (m, 2H), 1.75 - 1.56 (m, 2H). | 468.43 | 469.2 |
| 359 | Example 120 | 1H NMR: (400MHz, MeOD) δ = 8.58 (s, 1H), 8.55 (br. s, 1H), 8.37 (br. s, 1H), 8.17 (a, 1H), 8.01 (s, 1H), 7.85 (br. d, J = 8.2 Hz, 1H), 4.31 (br. s, 1H), 3.50 (br. d, J = 11.7 Hz, 1H), 3.23 (br. d, J = 12.7 Hz, 1H), 3.01 - 2.88 (m, 2H), 2.68 (s, 3H), 2.19 (br. s, 1H), 1.89 - 1.66 (m, 3H). | 403.16 | 404.10 |
| 360 | **Example** 121 | 1H NMR (400 MHz, MeOD) δ 8.43 (br.s., 1H), 8.28 (s, 1H), 8.13 (d, J = 8.3 Hz, 1H), 8.03 (s, 1H), 7.35 (dd, J = 5.6, 8.4 Hz, 1H), 4.32 - 4.19 (m, 1H), 3.59 - 3.49 (m, 1H), 3.32 - 3.31 (m, 1H), 3.11 - 299 (m, 2H), 2.77 (q, J = 7.5 Hz, 2H), 2.23 - 2.04 (m, 2H), 1.95 - 1.71 (m, 2H), 121 (t, J = 7.5 Hz, 3H) | 364.18 | 365.10 |
| 361 | Example 122 | 1H NMR (400 MHz, MeOD) δ 8.74 (br., 1H), 8.53 (b, 1H), 8.43 (s, 1H), 7.87 (s, 1H), 7.45 (br., 7 = 8.3 Hz, 1H), 7.10 - 6.75 (m, 1H), 4.32 (br., 1H), 3.55 (br., J = 15.3 Hz, 1H), 3.29 - 3.25 (m, 1H), 3.09 - 2.93 (m, 2H), 2.26 - 1.99 (m, 2H), 1.90 - 1.66 (m, 2H) | 400.13 | 401.10 |
| 362 | Example 122 | 1H NMR (400 MHz, CDC13) δ 8.00 (d, J = 7.94 Hz, 1H), 7.72 (a, 2H), 7.52 (d, J = 7.28 Hz, 1H), 7.12 (t, J = 7.83 Hz, 1H), 3.91 (br., 1H), 3.31 (br., 1H), 3.19 (br d, J = 13.23 Hz, 1H), 2.96-2.81 (m, 2H), 2.29 (br., J = 2.87, 7.50 Hz, 2H), 1.98-1.85 (m, 2H), 1.68 (br., J = 11.25 Hz, 1H), 1.56-1.41 (m, 1H), 0.96 (t, J = 7.39 Hz, 3H) | 408.48 | 402.1 |
| 363 | Example 123 | 1H NMR (400 MHz, CDC13) δ 8.20-8.19 (s, 2H), 7.83-7.77 (m, 1H), 7.40 (d, J = 7.02 Hz, 1H), 7.19 (t, J = 7.67 Hz, 1H), 4.16 (br., 1H), 3.39 (br., J = 10.52 Hz, 1Σi), 3.22 (br., J = 13.16 Hz, 1H), 2.87-2.73 (m, 3H), 2.58 (br., 1H), 2.10-1.96 (m, 2H), 1.80 (s, 1H), 1.56 (br., 1H), 1.19 (t, J = 7.45 Hz, 3H) | 385.48 | 386.1 |
| 364 | Example 124 | 1H NMR (400 MHz, MeOD) δ 8.74 - 8.61 (m, 1H), 8.59 - 8.40 (m, 1H), 8.37 - 8.18 (m, 1H), 8.15 - 8.04 (m, 1H), 8.01 - 7.69 (m, 1H), 4.81 - 4.26 (m, 1H), 3.90 - 3.77 (m, 1H), 3.62 - 3.49 (m, 1H), 3.20-2.80 (m, 8H), 2.37 - 2.21 (m, 1H), 2.18 - 1.93 (m, 2H), 1.80-1.60 (m, 1H) | 453.14 | 454.10 |
| 365 | Example 125 | 1H NMR (400 MHz, MeOD) δ ppm 8.64 (s, 2 H), 8.15 (s, 2 H), 7.75 - 7.70 (m, 1 H), 5.44 (s,l H), 5.32 (s, 1 H), 4.38 (br.s, 1H), 3.60 - 3.57 (m, 1 H), 3.06 - 3.00 (m, 2 H), 2.01 (br.s, 1H), 1.97 - 1.87 (m, 1 H), 1.83 - 1.77 (m, 2 H), 1.32 -1.28 (m, 1 H) | 457.12 | 458.1 |
| 367 | Example 127 | 1H NMR (400 MHz, CDC13) δ8.90 - 8.79 (m, 1H), 8.40 (s, 1H), 8.23 (s, 1H), 7.90 - 7.83 (m, 1H), 4.56 - 4.42 (m, 1H), 3.67 - 3.59 (m, 1H), 3.43 (s, 3H), 3.41 - 3.34 (m, 1H), 3.23 - 3.15 (m, 1H), 3.14 - 3.05 (m, 1Σi), 2.89 - 2.82 (m, 2H), 2.33 - 2.24 (m, 1H), 2.18 - 2.08 (m, 1H), 1.99 (s, 1H), 1.84 (br.s., 1H), 1.77 - 1.66 (m, 2H), 1.06 (t, J = 7.2 Hz, 3H) | 438.55 | 439.2 |
| 368 | Example 128 | 1H NMR (400 MHz, CDCl3) δ8.69 - 8.56 (m, 1H), 8.55 - 8.22 (m, 2H), 8.05 (s, 1H), 7.89 (s, 1H), 7.45 (br. d, J = 7.3 Hz, 1H), 4.62 (br. s, 1H), 4.34 (br. s, 1H), 3.23 (br. d. J = 12.0 Hz, 2H), 3.10 - 2.93 (m, 2H), 2.48 - 2.32 (m, 1H), 2.14 - 1.97 (m, 1H) | 404.36 | 405.1. |
| 370 | Example 130 | 1H NMR (400 MHz, CDC13) δ8.66 (s, 1H), 8.32 (br.s., 1H), 8.18 - 8.03 (m, 1H), 7.95 - 7.86 (m, 1H), 7.56 - 7.37 (m, 1H), 4.68 - 4.49 (m, 1H), 4.29 - 4.11 (m, 1H), 3.49 - 3.34 (m, 3H), 3.13 (br.d., J = 1.8 Hz, 1H), 2.28 (td, J = 3.6,13.9 Hz, 1H), 2.14 - 1.90 (m, 1H) | 402.37 | 403.1 |
| 371 | Starting with *N*,*N*-dimethyl-1H-indole-7-sulfonamide (from Example 96), following Examples 36 and 11 and then Examples 23 and 1 for the final steps. | ¹H NMR (500 MHz, DMSO) δ 11.67 (s, 1H), 8.75 - 8.52 (m, 2H), 8.33 (s, 1H), 7.96 (d, *J* = 8.0 Hz, 1H), 7.86 (s, 1H), 7.59 (t, J = 7.3 Hz, 1H), 7.39 (dt, *J* = 15.7, 7.9 Hz, 1H), 4.05 (s, 1H), 3.18 (s, 1H), 2.94 (s, 1H), 2.68 (d, *J* = 11.8 Hz, 6H), 2.64 - 2.54 (m, 2H), 1.99 (d, *J* = 24.5 Hz, 1H), 1.75 (s, 1H), 1.53 (s, 2H). | 468.50 | 469.1 |
| 372 | Starting from 3-(2-(methylsulfonyl)-5-(trifluorometbyl)pyrimidin-4-yl)-1-(phenylsulfonyl)-1H-indole and following Examples 97 (compound A), 1, and 2. Note: stereochemistry tentatively assigned. | ¹H NMR (500 MHz, DMSO) δ 11.79 (brs, 1H), 8.55 (s, 0.5H; rotamer 1), 8.53 (s, 0.5H; rotamer 2), 8.39 (d, *J* = 7.9 Hz, 0.5H; rotamer 1), 8.30 (brs, 1H), 8.19 (d, *J* = 7.9 Hz, 0.5H; rotamer 2), 7.86 - 7.71 (m, 2H), 7.49 (t, *J* = 8.7 Hz, 1H), 7.22 - 7.18 (m, 1H), 7.18 - 7.12 (m, 1H), 3.82 - 3.72 (m, 1H), 3.17 - 3.11 (m, 1H), 3.03 - 2.91 (m, 1H), 2.60 - 2.53 (m, 1H), 2.47 - 2.38 (m, 1H), 1.79 - 1.51 (m, 2H), 1.33 - 1.17 (m, 1H), 0.99 - 0.92 (m, 3H). | 375.39 | 376.3 |
| 373 | Starting from 3-(2-(methylsulfonyl)-5-(trifluoromethyl)pyrimidin-4-yl)-1-(phenylsulfonyl)-1H-indole and following Examples 97 (compound **B**), 1, and 2. Note: stereochemistry tentatively assigned. | ¹H NMR (500 MHz, DMSO) δ 11.80 (brs, 1H), 8.56 (s, 0.5H; rotamer 1), 8.53 (s, 0.5H; rotamer 2), 8.39 (d, *J* = 7.9 Hz, 0.5H; rotamer 1), 8.30 (brs, 1H), 8.19 (d, *J* = 7.8 Hz, 0.5H; rotamer 2), 7.86 - 7.72 (m, 2H), 7.49 (t, *J* = 8.6 Hz. 1H), 7.23 - 7.18 (m, 1H), 7.18 - 7.12 (m, 1H), 3.84 - 3.74 (m, 1H), 3.18 - 3.12 (m, 1H), 3.05 - 2.93 (m, 1H), 2.62 - 2.52 (m, 1H), 2.49 - 2.40 (m, 1H), 1.80 - 1.61 (m, 2H), 1.34 -1.19 (m, 1H), 0.99 - 0.93 (m, 3H). | 375.39 | 376.3 |
| 374 | Example 131 | 1H NMR (400 MHz, MeOD) δ 8.58 (s, 1H), 8.54 (br. s, 1H), 8.49 - 8.15 (m, 1H), 7.92 (s, 1H), 7.75 (s, 1H), 7.39 (br. d, J = 8.3 Hz, 1H), 7.22 - 6.86 (m, 1H), 4.31 (hr. s, 1H), 3.58 - 3.44 (m, 1H), 3.25 (br. d, J = 13.6 Hz, 1H), 3.02 - 2.88 (m, 2H), 2.27 - 2.14 (m, 1H), 2.09 - 1.99 (m, 1H), 1.92 - 1.66 (m, 2H). | 443.12 | 444.1 |
| 375 | Example 132 | 1H NMR (400 MHz, CDCl3) δ 8.63 (s, 1H), 8.54 (br. s, 1H), 8.41 (br. s, 1H), 8.16 (br. s, 2H), 7.74 (br. s, 1H), 6.91 - 6.53 (m, 1H), 4.34 (br. s, 1H), 3.64 - 3.43 (m, 1H), 3.30 - 3.24 (m, 1H), 3.09 - 2.87 (m, 2H), 2.19 (br. s, 1H), 2.08 (br. d, J = 14.7 Hz, 1H), 1.92 - 1.64 (m, 2H) | 475.44 | 476.0 |
| 376 | Example 133 | 1H NMR (400 MHz, MeOD) 8 7.67 - 7.61 (m, 2H), 7.53 (br d, J=1.8 Hz, 3H), 4.58 - 4.35 (m, 2H), 3.85 - 3.67(m, 2H), 3.16 - 2.83 (m, 3H), 2.00 - 1.91 (m, 1H), 1.61 - 1.49 (m, 1H), 1.19 (s, 3H), 1.10 (s, 3H) | 218.18 | 219.2 |
| 377 | Starting with 7-methylene-5-tosyl-5-azaspiro[3.5]nonane (J. Org. Chem. 2003, 68(11), 4286) and following Example 98 (compound **A**) | ¹H NMR (500 MHz, DMSO) δ 12.26 (brs, 1H), 8.62 (s, 0.5H; rotamer 1), 8.57 (s, 0.5H; rotamer 2), 8.51 (d, *J* = 8.3 Hz, 0.5H; retainer 1), 8.32 (d, *J* = 8.3 Hz, 0.5H; rotamer 2), 8.24 (s, 1H), 8.07 (d, *J* = 4.1 Hz, 1H), 8.02 (s, 0.5H; rotamer 1), 8.00 (s, 0.5H; rotamer 2), 7.80 (d, *J* = 8.1 Hz, 0.5H; rotamer 1), 7.75 (d, *J =* 8.4 Hz, 0.5H; rotamer 2), 7.47 (t, *J* = 1.5 Hz, 1H), 3.90 (brs, 1H), 2.97 - 2.91 (m, 1H), 2.59 - 2.53 (m, 1H), 2.00 - 1.91 (m, 1H), 1.89 -1.79 (m, 5H), 1.77 -1.δ6 (m, 2H), 1.61 - 1.49 (m, 1H), 1.47 -1.39 (m, 1H). | 426.44 | 427.3 |
| 378 | Starting with 7-methylene-5-tosyl-5-azaspiro[3.5]nonane (*J*. *Org. Chem.* **2003**, 68(11), 4286) and following Example 98 (compound **B**). | ¹H NMR (500 MHz, DMSO) δ 12.30 (brs, 1H), 8.62 (s, 0.5H; rotamer 1), 8.57 (s, 0.5H; rotamer 2), 8.51 (d, *J =* 8.4 Hz, 0.5H; rotamer 1), 8.32 (d, *J* = 8.4 Hz, 0.5H; rotamer 2), 8.23 (s, 1H), 8.07 (d, *J* = 4.7 Hz, 1H), 8.02 (s, 0.5H; rotamer 1), 8.00 (s, 0.5H; rotamer 2), 7.81 (d, *J* = 8.1 Hz, 0.5H; rotamer 1), 7.76 (d, *J =* 8.1 Hz, 0.5H; rotamer 2), 7.47 (t, *J =* 7.6 Hz, 1H), 3.91 (bra, 1H), 2.97 - 2.91 (m, 1H), 2.59 - 2.52 (m, 1H), 1.99 -1.91 (m, 1H), 1.91 -1.79 (m, 5H), 1.77 -1.67 (m, 2H), 1.61 - 1.50 (m, 1H), 1.49 - 1.40 (m, 1H). | 426.44 | 427.3 |
| 379 | Starting with (S)-tert-butyl 3-((4-(6-bromo-1-(phenylsulfonyl)-1H-indol-3-yl)-5-(trifluoromethyl)pyrimidin-2-yl)amino)piperidine-1-carboxylate and diethylphosphine oxide following Examples 94 and 2. | ¹H NMR (500 MHz, DMSO) δ 12.09 (brs, 1H), 8.61 (s, 0.5H; rotamer 1), 8.57 (s, 0.5H; rotamer 2), 8.47 - 8.22 (m, 2H), 7.97 (d, *J* = 9.0 Hz, 1H), 7.91 - 7.83 (m, 2H), 7.47 - 7.35 (m, 1H), 4.02 (brs, 1H), 3.18 (brs, 1H), 2.95 (brs, 1H), 2.61 (brs, 1H), 2.04 - 1.84 (m, 5H), 1.75 (brs, 1H), 1.53 (brs, 2H), 1.02 - 0.88 (m, 6H). | 465.45 | 466.2 |
| 380 | Example 134 | 1H NMR (400 MHz, MeOD) δ 8.71 - 8.63 (m, 0.3H), 8.61 (s, 1H), 8.55 - 8.32 (m, 1H), 8.08 - 8.01 (m, 1H), 7.96 (s, 1H), 7.58 - 7.47 (m, 1H), 6.56 (s, 1H), 4.43 - 4.24 (m, 1H), 3.62 - 3.49 (m, 1H), 3.29 - 3.22 (m, 1H), 3.07 - 2.90 (m, 2H), 2.27 - 2.00 (m, 2H), 1.93 - 1.68 (m, 2H) | 459.12 | 460.1 |
| 381 | Example 135 | 1H NMR (400 MHz, MeOD) δ ppm 8.73 - 8.36 (m, 2H), 8.28 - 8.06 (m, 2H), 7.94 - 7.68 (m, 1H), 4.67 - 4.24 (m, 1H), 4.05 - 3.56 (m, 2H), 3.28 - 3.01 (m, 5H), 2.97 - 2.86 (m, 1H), 2.37 - 1.85 (m, 3H), 1.84-1.61 (m, 1H), 1.23- 0.85 (m, 4H) | 479.16 | 480.1 |
| 382 | Example 136 | 1H NMR (400MHz, MeOD)δ 8.94 (br s, 1H), 8.74 (br d, J = 17.4 Hz, 1H), 8.31 (br d, J = 4.2 Hz, 1H), 7.66 (br d, J = 8.2 Hz, 1H), 355 (br s, 1H), 3.34 (s, 3H), 3.20 (br s, 1H), 3.02 (br d, J = 12.7 Hz, 1H), 2.75 (br dd, J = 5.8, 12.3 Hz, 2H), 2.00 - 1.85 (m, 1H), 1.57 - 1.42 (m, 1H), 1.16 (br d, J = 4.8 Hz, 3H), 1.03 (s, 3H) | 458.13 | 459.10 |
| 396 | Example 129 | 1H NMR (400 MHz, MeOD) δ 8.69-8.40 (m, 2H). 8.24 - 8.12 (m, 1H), 8.12-8.05 (m, 1H), 7.85-7.65 (m, 1H), 3.78 - 3.75 (m, 1H), 3.31 - 3.27 (m, 4H), 3.13 (s, 3H), 3.00 - 2.65 (m, 2H), 2.06 - 1.93 (m, 1H), 1.72 -1.51 (m, 1H), 1.43-1.33 (m, 3H), 127 (br s, 3H), 1.11-1.04 (br s, 3H) | 495.19 | 494.1 |

**Example 142. Inhibition of CDK Kinase Activity.** Compounds of the invention were assayed for inhibition of CDK7, CDK9, CDK12, and CDK2 activity at Biortus Biosciences (Jiangyin, Jiangsu Province, P.R. of China) using kinase assays for each CDK developed with a Caliper/LabChip EZ Reader (Perkin Elmer, Waltham, MA). These assays measure the amount of phosphorylated peptide substrate produced as a fraction of the total peptide following an incubation period at 27 °C with the following components: test compounds (variable concentrations from 10 µM down to 0.508 nM in a series of 3-fold serial dilutions), active CDK kinase protein (with the indicated Cyclin, listed below for each CDK), ATP (2 mM), and substrate peptide (listed below) in the following buffer: 2-(N-morpholino)ethanesulfonate (MES buffer, 20 mM), pH 6.75, 0.01% (v/v) Tween 20 detergent, 0.05 mg/mL bovine serum albumin (BSA).

Specifically, the CDK7 inhibition assay used CDK7/Cyclin H/MAT1 complex (6 nM) and "S-FAM-CDK7tide" peptide substrate (2 µM, synthesized fluorophore-labeled peptide with the following sequence: 5-FAM-YSPTSPSYSPTSPSYSPTSPSKKKK, where "5-FAM" means 5-carboxyfluorescein) with 6 mM MgCl₂ in the buffer composition listed above. Furthermore, the CDK9 inhibition assay used CDK9/Cyclin T1 complex (8 nM) and "S-FAM-CDK9tide" peptide substrate (2 µM, synthesized fluorophore-labeled peptide with the following sequence: 5-FAM-GSRTPMY-NH₂ where 5-FAM is defined above and NH₂ signifies a C-terminal amide) with 10 mM MgCl₂ in the buffer composition listed above. The CDK12 inhibition assay used CDK12 (aa686-1082)/Cyclin K complex (50nM) and "5-FAM-CDK9tide" (2µM) as definied above, with 2mM MgCl₂ in the buffer composition above. Additionally, the CDK2 inhibition assay used CDK2/Cyclin El complex (0.5 nM) and "5-FAM-CDK7tide" (2 µM) as defined above, with 2 mM MgCl₂ in the buffer composition listed above.

The incubation period at 27 °C for each CDK inhibition assay was chosen such that the fraction of phosphorylated peptide product produced in each assay, relative to the total peptide concentration, was approximately 20% (±5%) for the uninhibited kinase (35 min. for CDK7, 35 min. for CDK2, 3 hr. for CDK12, 15 min. for CDK9). In cases where the compound titrations were tested and resulted in inhibition of peptide product formation, these data were fit to produce best-fit IC₅₀ values. The results of these assays are shown below in Table 1 where "A" represents a calculated IC₅₀ of less than 20 nM; "B" represents a calculated IC₅₀ of between 20 nM and less than 200 nM; "C" represents a calculated IC₅₀ of between 200 nM and less than 5 µM; "D" represents a calculated IC₅₀ of greater than or equal to 5 µM, and "NT" represents that the specified compound was not tested in the specified assay.

**Table 1. Inhibitory Activity of Selected Compounds of the Invention Against CDK2, CDK7. CDK9. and CDK12.**

| **Compound** | **CDK2** | **CDK7** | **CDK9** | **CDK 12** |
|---|---|---|---|---|
| 100 | C | A | C | C |
| 101 | C | C | C | C |
| 102 | C | A | B | B |
| 103 | C | A | C | B |
| 104 | C | B | C | B |
| 105 | D | B | D | C |
| 106 | D | B | C | C |
| 107 | C | B | C | B |
| 108 | C | B | C | C |
| 109 | C | A | C | B |
| 110 | C | B | C | C |
| 111 | C | A | B | B |
| 112 | C | A | C | B |
| 113 | D | B | D | C |
| 114 | C | B | C | C |
| 115 | D | C | D | D |
| 116 | D | D | D | D |
| 117 | C | B | C | C |
| 118 | C | A | C | B |
| 119 | D | B | C | C |
| 120 | D | c | D | C |
| 121 | C | B | C | C |
| 122 | D | C | C | D |
| 123 | D | B | C | D |
| 124 | C | A | C | B |
| 125 | D | B | D | D |
| 126 | C | B | C | C |
| 127 | C | C | C | D |
| 128 | D | D | D | D |
| 129 | NT | B | NT | C |
| 130 | NT | D | NT | D |
| 131 | D | D | D | D |
| 132 | D | C | C | D |
| 133 | B | A | C | B |
| 134 | D | D | D | D |
| 135 | D | B | D | D |
| 136 | C | A | C | C |
| 137 | C | A | C | C |
| 138 | C | B | C | C |
| 139 | D | B | D | D |
| 140 | D | C | D | D |
| 141 | C | A | C | C |
| 142 | C | A | C | C |
| 143 | C | A | C | C |
| 144 | C | A | C | C |
| 145 | D | B | D | C |
| 146 | D | D | D | D |
| 147 | B | A | C | C |
| 148 | D | B | C | C |
| 149 | C | B | C | C |
| 150 | C | B | C | C |
| 151 | D | C | D | D |
| 152 | C | A | C | C |
| 153 | B | A | C | B |
| 154 | D | B | C | C |
| 155 | C | B | C | C |
| 156 | C | A | C | C |
| 157 | C | A | C | C |
| 158 | D | C | D | C |
| 159 | B | B | C | C |
| 160 | C | A | C | C |
| 161 | C | A | C | C |
| 162 | NT | B | C | C |
| 163 | NT | B | C | C |
| 164 | NT | B | C | C |
| 165 | C | A | C | C |
| 166 | B | A | C | C |
| 167 | C | A | C | B |
| 168 | D | C | D | D |
| 169 | D | B | D | C |
| 110 | C | A | C | B |
| 171 | C | A | C | C |
| 172 | C | A | C | C |
| 173 | C | A | C | C |
| 174 | C | A | C | C |
| 175 | C | A | B | C |
| 176 | C | A | C | C |
| 177 | D | B | D | C |
| 178 | D | B | D | C |
| 179 | C | A | C | C |
| 180 | B | A | C | B |
| 181 | C | A | C | C |
| 182 | D | B | C | C |
| 183 | C | B | C | D |
| 184 | B | A | B | B |
| 185 | B | A | C | C |
| 186 | C | A | C | C |
| 187 | D | B | D | C |
| 188 | C | A | C | C |
| 189 | D | C | D | D |
| 190 | C | A | C | C |
| 191 | C | A | C | C |
| 192 | C | B | C | C |
| 193 | C | B | C | C |
| 194 | C | A | C | C |
| 195 | D | C | D | D |
| 196 | C | A | C | C |
| 197 | C | A | C | C |
| 198 | C | A | C | C |
| 199 | C | A | C | C |
| 200 | C | A | C | C |
| 201 | C | A | C | B |
| 202 | C | A | C | D |
| 203 | C | A | C | C |
| 204 | D | A | D | C |
| 205 | D | A | D | C |
| 206 | D | B | C | C |
| 207 | C | A | C | C |
| 208 | D | B | D | C |
| 209 | D | C | D | D |
| 210 | D | C | D | D |
| 211 | C | B | C | C |
| 212 | C | A | C | C |
| 213 | D | B | D | C |
| 214 | C | B | C | C |
| 215 | D | B | D | D |
| 216 | C | C | D | D |
| 217 | C | A | C | C |
| 218 | C | A | C | C |
| 219 | C | A | C | C |
| 220 | D | A | D | C |
| 221 | D | A | D | D |
| 222 | C | B | C | C |
| 223 | C | A | C | C |
| 224 | C | A | C | C |
| 225 | C | A | C | C |
| 226 | C | A | C | C |
| 227 | C | A | C | C |
| 228 | C | A | C | C |
| 229 | C | A | C | C |
| 230 | C | A | C | C |
| 231 | D | D | D | D |
| 232 | C | A | C | C |
| 233 | C | A | C | C |
| 234 | D | C | D | D |
| 235 | C | A | C | C |
| 236 | C | A | C | C |
| 237 | C | B | D | C |
| 238 | D | C | D | D |
| 239 | D | C | D | D |
| 240 | C | B | C | C |
| 241 | C | B | C | C |
| 242 | D | B | C | C |
| 243 | C | A | C | B |
| 244 | C | B | D | C |
| 245 | C | A | D | C |
| 246 | D | A | D | D |
| 247 | D | C | D | D |
| 248 | C | A | C | C |
| 249 | D | A | D | C |
| 250 | C | A | D | C |
| 251 | C | A | C | C |
| 252 | C | B | D | D |
| 253 | B | A | C | B |
| 254 | D | C | D | D |
| 255 | C | A | C | C |
| 256 | C | B | D | C |
| 257 | D | B | D | D |
| 258 | C | A | C | C |
| 259 | C | A | C | C |
| 260 | D | C | D | C |
| 261 | C | B | C | C |
| 262 | C | A | C | C |
| 263 | D | A | D | D |
| 264 | D | B | D | C |
| 265 | C | C | C | C |
| 266 | C | B | C | C |
| 267 | C | B | C | C |
| 268 | D | C | D | C |
| 269 | C | A | C | C |
| 270 | C | A | C | C |
| 271 | D | B | D | D |
| 272 | C | A | C | C |
| 273 | D | C | D | D |
| 274 | C | A | C | C |
| 275 | C | A | C | C |
| 276 | D | B | D | D |
| 277 | C | A | C | C |
| 278 | C | B | D | C |
| 219 | D | B | D | D |
| 280 | D | B | D | C |
| 281 | C | A | C | C |
| 282 | C | A | D | C |
| 283 | D | C | D | D |
| 2β4 | C | A | C | C |
| 285 | D | C | D | D |
| 286 | C | A | C | C |
| 287 | D | A | D | D |
| 289 | C | B | C | C |
| 290 | C | B | C | C |
| 291 | C | A | C | C |
| 292 | C | A | C | C |
| 293 | C | A | C | C |
| 294 | C | A | C | C |
| 295 | D | B | D | D |
| 296 | D | B | D | D |
| 297 | C | A | C | C |
| 298 | C | A | D | C |
| 299 | C | A | C | C |
| 300 | C | A | C | C |
| 301 | D | B | D | D |
| 302 | C | B | C | C |
| 303 | C | A | C | C |
| 304 | D | D | D | D |
| 305 | C | A | C | C |
| 306 | c | B | D | C |
| 307 | D | D | D | D |
| 308 | C | A | C | C |
| 309 | D | B | D | C |
| 310 | C | A | D | C |
| 311 | C | A | C | C |
| 312 | NT | B | NT | C |
| 313 | C | A | C | C |
| 314 | C | A | C | C |
| 315 | NT | B | NT | C |
| 316 | C | A | D | C |
| 317 | NT | B | NT | C |
| 318 | C | A | C | C |
| 319 | NT | B | NT | C |
| 320 | NT | B | NT | C |
| 321 | D | A | D | C |
| 322 | NT | A | NT | C |
| 323 | C | A | D | C |
| 324 | NT | B | NT | C |
| 325 | NT | B | NT | C |
| 32δ | NT | B | NT | C |
| 327 | NT | B | NT | C |
| 328 | C | A | C | C |
| 329 | NT | B | NT | C |
| 330 | C | A | C | C |
| 331 | NT | B | NT | C |
| 332 | C | A | C | C |
| 333 | D | A | D | C |
| 334 | C | B | C | C |
| 335 | C | B | C | C |
| 336 | C | A | C | C |
| 337 | NT | B | NT | C |
| 338 | C | B | C | C |
| 339 | D | B | D | C |
| 340 | C | B | C | C |
| 341 | D | C | D | D |
| 342 | D | B | D | C |
| 343 | C | B | C | C |
| 344 | C | B | D | C |
| 345 | D | C | D | D |
| 346 | C | B | C | C |
| 347 | D | B | D | C |
| 348 | D | B | D | D |
| 349 | C | A | C | C |
| 350 | D | B | D | C |
| 351 | C | A | C | C |
| 352 | D | D | D | D |
| 353 | C | A | D | C |
| 354 | D | B | D | C |
| 355 | D | B | D | C |
| 356 | D | B | D | D |
| 357 | C | A | C | C |
| 358 | D | B | D | D |
| 359 | C | A | C | C |
| 360 | D | B | D | D |
| 361 | D | B | C | C |
| 362 | D | B | D | D |
| 363 | D | B | D | D |
| 364 | D | B | D | D |
| 365 | C | A | C | C |
| 366 | C | B | C | C |
| 367 | D | B | D | D |
| 368 | D | B | D | C |
| 369 | D | B | D | D |
| 370 | D | B | D | C |
| 371 | C | A | C | C |
| 372 | C | B | C | C |
| 313 | D | B | D | D |
| 374 | C | A | C | B |
| 375 | C | A | C | C |
| 376 | C | B | D | C |
| 377 | D | A | C | C |
| 378 | D | B | D | D |
| 379 | D | B | D | D |
| 380 | C | A | C | C |
| 381 | D | B | D | C |
| 382 | C | A | C | C |

### Example 143. Inhibition of Cell Proliferation.

***A673 Cells.*** A673 cells are a cell line derived from human muscle Ewing's sarcoma. Representative compounds of the invention were tested at different concentrations (from 4 µM to 126.4 pM; 0.5 log serial dilutions) for their ability to inhibit the proliferation of A673 cells. Known CDK inhibitors dinaciclib or N-((1S,3R)-3-((5-chloro-4-(1H-indol-3-yl)pyrimidin-2-yl)amino)cyclohexyl)-5-((E)-4-(dimethylamino)but-2-enamido)picolinamide and triptolide were used as positive controls. Cells were grown in Dulbecco's Modified Eagle's Medium, + 10% PBS +lmM Sodium Pyruvate. The cells were cultured at 37 °C in a humidified chamber in the presence of 5% CO₂. Proliferation assays were conducted over a 72 hour time period. CyQUANT^{®} (Life Technologies, Chicago, IL USA) was used to assess the anti-proliferative effects of the compounds following manufacturer's directions and utilizing the reagents supplied with the CyQUANT^{®} kit. The results of the assay are shown below in Table 1 where "A" represents a calculated IC₅₀ of less than 20 nM; "B" represents a calculated IC₅₀ of between 20 nM and less than 200 nM; "C" represents a calculated IC₅₀ of 200 nM and less than 5 µM; "D" represents a calculated IC₅₀ of greater than 5 µM.

***HCC70 cells.*** HCC70 cells are a cell line derived from human triple negative breast cancer. Representative compounds of the invention were tested at different concentrations (from 4 µM to126.4 pM; 0.5 log serial dilutions) for their ability to inhibit the proliferation of HCC70 cells. Known CDK inhibitors dinaciclib or N-((1S,3R)-3-((5-chloro-4-(1H-indol-3-yl) pyrimidin-2-yl)amino)cyclohexyl)-5-((E)-4-{dimethylamino)but-2-enamido)picolinamide and triptolide were used as positive controls. Cells were grown in ATCC-formulated RPMI-1640 Medium (ATCC 30-2001) + 10% PBS. The cells were cultured at 37 °C in a humidified chamber in the presence of 5% CO₂. Proliferation assays were conducted over a 72 hour time period. CyQUANT^{®} Direct Cell Proliferation Assay (Life Technologies, Chicago, IL USA) was used to assess the anti-proliferative effects of the compounds following manufacturer's directions and utilizing the reagents supplied with the CyQUANT^{®} Direct Cell kit. The results of the assay are shown below in Table 2 where "A" represents a calculated IC₅₀ of less than 100 nM; "B" represents a calculated IC₅₀ of between 100 nM and less than 1000 nM; "C" represents a calculated IC₅₀ of between 1000 nM and less than 5 µM; "D" represents a calculated IC₅₀ of greater than 5 µM.

**Table 2. Inhibition of Proliferation of A673 Cells and HCC70 Cells by Compounds of the Invention.**

| **Compound** | **A673 Cells** | **HCC70 Cells** |
|---|---|---|
| 100 | A | NT |
| 101 | B | NT |
| 102 | A | NT |
| 103 | B | A |
| 104 | B | B |
| 105 | B | NT |
| 106 | B | NT |
| 107 | A | B |
| 108 | C | NT |
| 109 | B | B |
| 110 | B | NT |
| 111 | B | NT |
| 112 | A | A |
| 113 | C | NT |
| 114 | B | NT |
| 115 | C | NT |
| 116 | C | NT |
| 117 | B | NT |
| 118 | B | A |
| 119 | B | NT |
| 120 | NT | NT |
| 121 | B | NT |
| 122 | C | NT |
| 123 | C | NT |
| 124 | A | NT |
| 125 | C | NT |
| 126 | NT | NT |
| 127 | C | NT |
| 128 | NT | NT |
| 129 | B | NT |
| 130 | C | NT |
| 131 | C | NT |
| 132 | C | NT |
| 133 | A | NT |
| 134 | C | NT |
| 135 | C | NT |
| 136 | B | NT |
| 137 | B | NT |
| 138 | C | NT |
| 139 | C | C |
| 140 | C | NT |
| 141 | A | A |
| 142 | A | NT |
| 143 | A | NT |
| 144 | B | B |
| 145 | C | NT |
| 146 | B | NT |
| 147 | A | NT |
| 148 | B | NT |
| 149 | B | NT |
| 150 | B | NT |
| 151 | B | NT |
| 152 | A | NT |
| 153 | A | NT |
| 154 | C | NT |
| 155 | A | NT |
| 156 | A | NT |
| 157 | A | NT |
| 158 | B | NT |
| 159 | C | C |
| 160 | A | NT |
| 161 | A | A |
| 162 | A | NT |
| 163 | B | NT |
| 164 | A | NT |
| 165 | B | B |
| 166 | A | A |
| 167 | A | NT |
| 168 | C | NT |
| 169 | C | NT |
| 170 | A | A |
| 171 | A | NT |
| 112 | A | NT |
| 173 | | B |
| 174 | B | NT |
| 175 | A | C |
| 176 | A | B |
| 177 | C | NT |
| 178 | C | NT |
| 179 | A | NT |
| 180 | A | A |
| 181 | A | NT |
| 182 | B | NT |
| 183 | C | NT |
| 184 | A | NT |
| 185 | A | A |
| 186 | B | NT |
| 187 | B | NT |
| 188 | B | NT |
| 189 | C | NT |
| 190 | A | B |
| 191 | A | NT |
| 192 | NT | NT |
| 193 | NT | NT |
| 194 | A | NT |
| 195 | B | NT |
| 196 | B | NT |
| 197 | A | NT |
| 198 | A | NT |
| 199 | A | B |
| 200 | B | NT |
| 201 | A | NT |
| 202 | B | NT |
| 203 | A | NT |
| 204 | C | NT |
| 205 | C | NT |
| 206 | C | NT |
| 207 | A | NT |
| 208 | B | NT |
| 209 | B | NT |
| 210 | C | NT |
| 211 | B | NT |
| 212 | A | NT |
| 213 | B | NT |
| 214 | B | NT |
| 215 | C | NT |
| 216 | C | NT |
| 217 | B | NT |
| 218 | B | NT |
| 219 | B | NT |
| 220 | NT | NT |
| 221 | C | B |
| 212 | B | NT |
| 223 | B | NT |
| 224 | B | NT |
| 225 | B | NT |
| 226 | A | NT |
| 227 | B | NT |
| 228 | B | NT |
| 229 | A | NT |
| 230 | B | NT |
| 231 | C | NT |
| 232 | A | A |
| 233 | B | NT |
| 234 | A | NT |
| 235 | C | NT |
| 236 | B | NT |
| 237 | NT | NT |
| 238 | C | NT |
| 239 | C | NT |
| 240 | NT | NT |
| 241 | B | NT |
| 242 | B | NT |
| 243 | A | NT |
| 244 | C | NT |
| 245 | B | NT |
| 246 | C | B |
| 247 | C | NT |
| 248 | NT | NT |
| 249 | B | NT |
| 200 | C | C |
| 251 | A | A |
| 252 | C | NT |
| 253 | B | NT |
| 254 | NT | NT |
| 255 | B | NT |
| 256 | C | NT |
| 257 | C | NT |
| 258 | B | NT |
| 259 | B | NT |
| 260 | C | NT |
| 261 | A | A |
| 262 | B | NT |
| 263 | B | B |
| 264 | A | B |
| 265 | B | NT |
| 266 | B | NT |
| 267 | A | A |
| 268 | C | NT |
| 269 | NT | B |
| 270 | NT | NT |
| 271 | C | NT |
| 272 | B | NT |
| 273 | C | C |
| 274 | B | NT |
| 275 | B | NT |
| 276 | C | NT |
| 277 | B | B |
| 278 | B | B |
| 279 | C | C |
| 280 | NT | B |
| 281 | NT | B |
| 282 | NT | B |
| 283 | C | C |
| 284 | B | B |
| 285 | A | A |
| 286 | C | C |
| 287 | B | B |
| 289 | B | B |
| 290 | B | B |
| 291 | B | B |
| 292 | B | B |
| 293 | NT | B |
| 294 | NT | A |
| 295 | NT | C |
| 296 | NT | C |
| 297 | NT | A |
| 298 | NT | B |
| 299 | NT | C |
| 300 | NT | B |
| 301 | NT | C |
| 302 | NT | B |
| 303 | NT | B |
| 304 | NT | C |
| 305 | NT | C |
| 306 | NT | B |
| 307 | NT | C |
| 308 | NT | B |
| 309 | NT | C |
| 310 | NT | B |
| 311 | NT | C |
| 312 | NT | B |
| 313 | NT | B |
| 314 | NT | B |
| 315 | NT | C |
| 316 | NT | B |
| 317 | NT | C |
| 318 | NT | B |
| 319 | NT | C |
| 320 | NT | C |
| 321 | NT | B |
| 322 | NT | B |
| 323 | NT | B |
| 324 | NT | B |
| 325 | NT | B |
| 326 | NT | B |
| 327 | NT | B |
| 328 | NT | A |
| 329 | NT | B |
| 330 | NT | A |
| 331 | NT | B |
| 332 | NT | A |
| 333 | NT | C |
| 334 | NT | B |
| 335 | NT | B |
| 336 | NT | A |
| 337 | NT | B |
| 338 | NT | B |
| 339 | NT | C |
| 340 | NT | C |
| 341 | NT | C |
| 342 | NT | C |
| 343 | NT | B |
| 344 | NT | B |
| 345 | NT | C |
| 346 | NT | B |
| 347 | NT | B |
| 348 | NT | B |
| 349 | NT | B |
| 350 | NT | B |
| 351 | NT | A |
| 352 | NT | C |
| 353 | NT | A |
| 354 | NT | C |
| 355 | NT | B |
| 356 | NT | B |
| 357 | NT | C |
| 358 | NT | B |
| 359 | NT | A |
| 360 | NT | C |
| 361 | NT | B |
| 362 | NT | NT |
| 363 | NT | NT |
| 364 | NT | C |
| 365 | NT | B |
| 366 | NT | C |
| 367 | NT | C |
| 368 | NT | C |
| 369 | NT | B |
| 370 | NT | B |
| 371 | NT | A |
| 372 | NT | B |
| 373 | NT | C |
| 374 | NT | A |
| 375 | NT | B |
| 376 | NT | B |
| 377 | NT | B |
| 378 | NT | C |
| 379 | NT | C |
| 380 | NT | A |
| 381 | NT | C |
| 382 | NT | A |

### Example 144. Comparison of Compounds of the Invention with Structural Analogs

Representative compounds of the invention were directly compared against those with a similar structural scaffold that were previously described in co-pending PCT publication WO2015154039 using the enzymatic and cellular assays outlined Examples 1 and 2 above. Table 3 below summarizes the resulting data, where "A" represents a calculated IC₅₀ of less than 20 nM; "B" represents a calculated IC₅₀ of between 20 nM and less than 200 nM; "C" represents a calculated IC₅₀ of 200 nM and less than 5 µM; "D" represents a calculated IC₅₀ of greater than 5µM.

As shown in Table 3, representative compounds of formula (I) had a lower IC₅₀ value against CDK7 and in A2137 cells compared with similar compounds bearing a cyclohexyl ring in the place of the piperidinyl ring. These data suggest that compounds of formula (I) are highly specific for CDK7.

**Table 3. Comparison of Compounds of the Invention with Structurally Related Analogs**

| **Structure** | **CDK2** | **CDK7** | **CDK9** | **A673 Cells** |
|---|---|---|---|---|
| 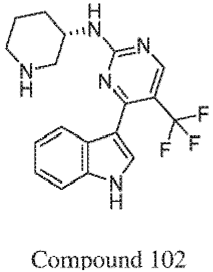 | C | A | B | A |
| 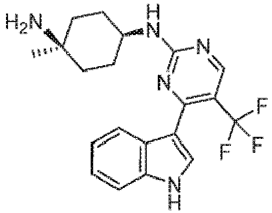 | C | C | D | B |
| 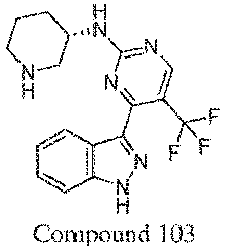 | C | A | c | A |
| 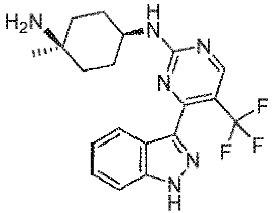 | C | C | C | B |

### Equivalents and Scope

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims are introduced into another claim. For example, any claim that is dependent on another claim can be modified to include one or more limitations found in any other claim that is dependent on the same base claim. Where elements are presented as lists, *e.g.,* in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

The disclosure also comprises the following clauses, which may be claimed:
1. A compound of formula I: 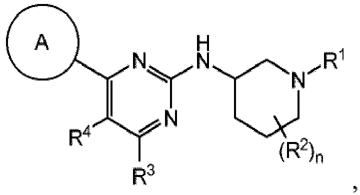 or a pharmaceutically acceptable salt thereof wherein
   ring A is a bicyclic 6,5-ring system selected from: 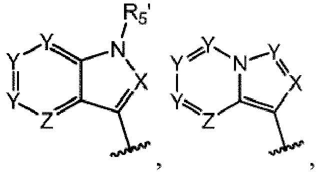 and 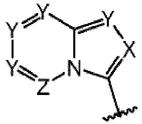 and comprises no more than four ring nitrogen atoms;
   X is selected from N and C(R⁶);
   each Y is independently selected from N and C(R⁷);
   Z is selected from N and C(R⁸);
   R¹ is selected from hydrogen, -C₁-C₆ alkyl, -O-(C₁-C₆-alkylene)-O-(C₁-C₄-alkyl), -(C₀-C₆ alkylene)-carbocyclyl, -C(O)-O-(C₁-C₆ alkylene), -(C₁-C₆ alkylene)-heterocyclyl, -(C₁-C₆ alkylene)-heteroaryl, -(C₁-C₆ alkylene)-N(R^{1'})₂, -(C₁-C₆ alkylene)-NR^{1'}-S(O)₂-(C₁-C₄ alkyl), -(C₁-C₆ alkylene)-NR^{1'}-SO₂-N(R^{1'})₂, -(C₁-C₆ alkylene)-S(O)₂-(C₁-C₄ alkyl), and -(C₁-C₆ alkylene)-S(O)₂-N(R^{1'})₂, wherein any carbocyclyl, heterocyclyl or heteroaryl portion of R¹ is optionally substituted, and wherein any alkyl or alkylene portion of R¹ is optionally substituted with one or more independently selected monovalent substituents;
   each R^{1'} is independently selected from hydrogen and optionally substituted C₁-C₆ alkyl, or
   two R^{1'} are optionally taken together with the nitrogen atom to which they are bound to form a 4-6 membered, optionally substituted heterocyclyl or heteroaryl ring comprising up to 2 additional heteroatoms selected from N, O, and S, wherein:
   each R², if present, is independently selected from =O, halo, -OH, -CN, -C₁-C₆ alkyl, -(C₀-C₆ alkylene)-carbocyclyl, -(C₀-C₆ alkylene)-heterocyclyl, -(C₀-C₆ alkylene)-heteroaryl, - (C₀-C₆ alkylene)-aryl, -(C₀-C₆ alkylene)-C(O)-heterocyclyl, -(C₀-C₆ alkylene)-C(O)-heteroaryl, -O-(C₁-C₆-alkyl), -O-(C₁-C₆-alkylene)-O-(C₁-C₄₋alkyl), -O-(C₁-C₄-alkylene)-carbocyclyl, -O-(C₁-C₆-alkylene)-heterocyclyl, -O-(C₁-C₆-alkylene)-heteroaryl, -O-(C₁-C₆-alkylene)-aryl, -NH-C(O)-C₁-C₄ alkyl, and -C(O)-NH-(unsubstituted C₁-C₄ alkyl), or
   R¹ and any R² are taken together with the atoms to which they are bound to form an optionally substituted heterocyclyl or heteroaryl ring fused, spirofused or bridged to the piperidine ring, or
   two R² are taken together with the atom or atoms to which they are bound and any intervening ring atoms to form an optionally substituted aryl, carbocyclyl, heterocyclyl or heteroaryl ring fused, spirofused or bridged to the piperidine ring,
   wherein any carbocyclyl, heterocyclyl, or heteroaryl portion of R², any ring formed by taking R¹ together with R², or any ring formed by taking two R² together is optionally substituted, and wherein any alkyl or alkylene portion of R² is optionally substituted with one or more independently selected monovalent substituents unless otherwise specified;
   R³ is selected from hydrogen, halo, -CN, optionally substituted -C₁-C₆ alkyl, or optionally substituted carbocyclyl;
   R⁴ is selected from halo, -CN, -C₁-C₆ alkyl, -C₂-C₆ alkenyl, C₂-C₆ alkynyl, -O-C₁-C₆ alkyl, -S-C₁-C₆ alkyl, and carbocyclyl, wherein any alkyl, alkenyl, alkynyl, or carbocyclyl portion of R⁴ is optionally substituted;
   each R⁵ is independently selected from halo, -OH, -C₁-C₆ alkyl, -CN, -(C₀-C₆ alkylene)-C(O)OH, -(C₀-C₆ alkylene)-C(O)-(C₁-C₄ alkyl), -(C₀-C₆ alkylene)-C(O)-N(R^{1'})₂, - (C₀-C₆ alkylene)-S(O)₂-(C₁-C₄ alkyl), -(C₀-C₆ alkylene)-S(O)₂-N(R^{1'})₂, -(C₀-C₆ alkylene)-P(O)-O-(C₁-C₄ alkyl)₂, -(C₀-C₆ alkylene)-P(O)-(C₁-C₄ alkyl)(O-C₁-C₄ alkyl), -(C₀-C₆ alkylene)-P(O)(C₁-C₄ alkyl)₂, -(C₀-C₆ alkylene)-carbocyclyl, -(C₀-C₆ alkylene)-heterocyclyl, - (C₀-C₆ alkylene)-heteroaryl, -(C₀-C₆ alkylene)-C(O)-heterocyclyl, -(C₀-C₆ alkylene)-C(O)-heteroaryl, -O-(C₁-C₆-alkyl), -O-(C₁-C₆-alkylene)-O-(C₁-C₄-alkyl), -O-(C₀-C₆-alkylene)-carbocyclyl, -O-(C₁-C₆-alkylene)-heterocyclyl, -O-(C₁-C₆-alkylene)-heteroaryl, phenyl, -(C₂-C₄ alkenylene)-phenyl, -S(O)-(C₁-C₄ alkyl), -S-(C₁-C₄ alkyl), -S(O)-OH, and - S(O)₂-OH, wherein any alkyl, alkylene, alkenylene, carbocyclyl, heterocyclyl, phenyl, and heteroaryl portion of R³ is optionally substituted; or
   two vicinal R⁵ are taken together with the ring atoms to which they are bound to form an optionally substituted carbocyclyl or optionally substituted heterocyclyl, wherein each carbocyclyl or heterocyclyl is fused to ring A;
   R^{5'} is selected from hydrogen, -CN, -C₁-C₆ alkyl, -(C₀-C₆ alkylene)-S(O)₂₋N(R^{1'})₂, - (C₀-C₆ alkylene)-carbocyclyl, -(C₀-C₆ alkylene)-C(O)-N(R^{1'})₂, -(C₀-C₆ alkylene)-aryl, -(C₀-C₆ alkylene)-heterocyclyl, -(C₀-C₆ alkylene)-heteroaryl, -(C₀-C₆ alkylene)-S(O)₂-(C₁-C₄ alkyl), - (C₁-C₆ alkylene)-O-(C₁-C₃ alkylene)-C(O)-N(R^{1'})₂, -(C₁-C₆ alkylene)-O-(C₁-C₄ alkylene)-P(O)(C₁-C₄ alkyl)₂, -(C₁-C₆ alkylene)-O-(C₁-C₄ alkylene)-P(O)(C₁-C₄ alkyl)-O-(C₁-C₄ alkyl), -(C₁-C₆ alkylene)-O-(C₁-C₄ alkylene)-P(O)-(O-C₁-C₄ alkyl)₂, -(C₁-C₆ alkylene)-O-(C₁-C₄ alkylene)-S(O)₂-(C₁-C₄ alkyl), -(C₁-C₆ alkylene)-O-(C₁-C₄ alkylene)-S(O)₂-N(R^{1'})₂, -(C₁-C₆ alkylene)-O-(C₁-C₄ alkyl), -(C₁-C₆ alkylene)-O-carbocyclyl, -(C₁-C₆alkylene)-O-heteroaryl, - (C₁-C₆ alkylene)-O-heterocyclyl, -(C₁-C₆ alkylene)-P(O)(C₁-C₄ alkyl)₂, -(C₁-C₆ alkylene)-P(O)(C₁-C₄ alkyl)-O-(C₁-C₄ alkyl), -(C₁-C₆ alkylene)-P(O)-(O-C₁-C₄ alkyl)₂, -(C₁-C₆ alkylene)-C(O)-(C₁-C₄ alkyl), and -(C₁-C₆ alkylene)-C(O)OH, wherein any alkyl, alkylene, carbocyclyl, heterocyclyl and heteroaryl portion of R^{5'} is optionally substituted;
   R^{5'} and any R⁵ are taken together with the ring atoms to which they are bound to form an optionally substituted heterocyclyl, wherein each heterocyclyl is fused to ring A;
   R⁶ is selected from hydrogen, -CN, -CH₃, -CH₂F, -CHF₂ and -CF₃;
   each R⁷ is independently selected from hydrogen and R⁵;
   R⁸ is selected from hydrogen and fluoro; and
   n is 0, 1, 2, 3, or 4, wherein the compound is other than 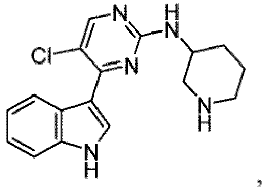 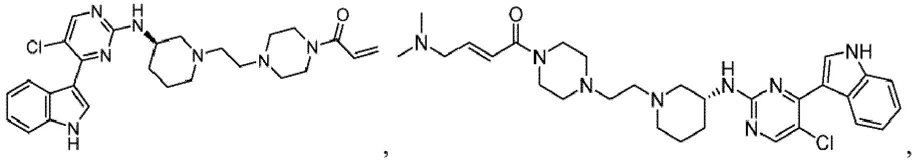 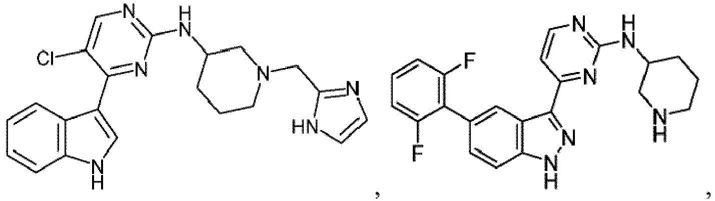 or a stereoisomer or pharmaceutical salt of any of the foregoing.
2. The compound of clause 1, wherein ring A is selected from: 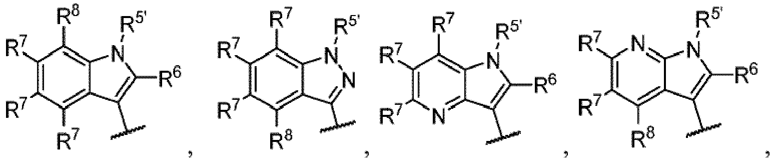 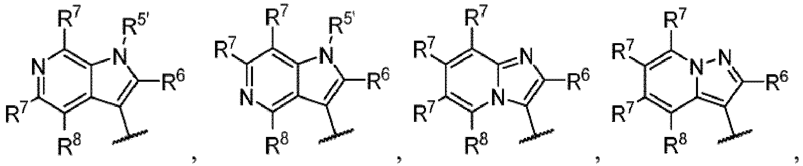 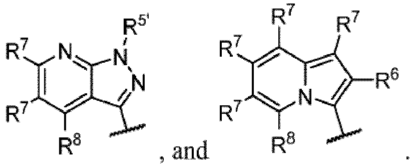
3. The compound of clause 2, wherein ring A is selected from 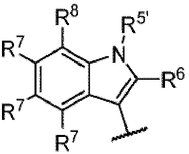 and 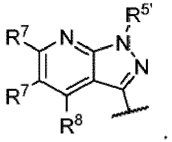
4. The compound of any of clauses 1-3, wherein R¹ is selected from hydrogen, cyclopropyl, -CH₃, -CH₂CH₃, -CH₂CH₂OCH₃, -CH(CH₃)₂, or -CH₂CH(CH₃)₂, or wherein R¹ is taken together with one R² and the ring atoms to which each are bound to form a ring which, taken together with the ring to which R¹ and R² are bound, is 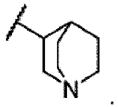
5. The compound of clause 4, wherein R¹ is hydrogen.
6. The compound of any one of clauses 1-5, wherein each R², if present, is independently selected from halo, =O, -OH, -C₁-C₄ alkyl, phenyl, heteroaryl, -C(O)-heterocyclyl, -NH-C(O)-C₁-C₄ alkyl, and -C(O)-NH-(unsubstituted C₁-C₄ alkyl), wherein any heteroaryl or heterocyclyl portion of R² is optionally substituted and any C₁-C₄ alkyl or phenyl portion of R² is optionally substituted with one or more independently selected monovalent substituents unless otherwise specified, and either
   a. two R² bound to different ring atoms are optionally taken together with the atoms to which they are bound and any intervening ring atoms to form an optionally substituted aryl or cycloalkyl, fused or bridged to the piperidine ring; or
   b. two R² bound to the same ring atom are optionally taken together with the atom to which they are bound to form an optionally substituted cycloalkyl spirofused to the piperidine ring.
7. The compound of clause 6, wherein n is 0, 1, 2 or 3, and each R², if present, is independently selected from fluoro, =O, -CH₃, -CH₂CH₃, -OH, -CH(CH₃)₂, -C(O)NHCH₃, - NHC(O)CH₂CH₃, 3-methyl-1,2,4-oxadiazol-5-yl, 1,2,4-triazolo[4,3-a]pyridin-3-yl, 8-methylsulfonyl-l,2,4-triazolo[4,3-a]pyridin-3-yl, pyrrolidin-1-ylcarbonyl, 3-hydroxypyrrolidin-1-ylcarbonyl, and unsubstituted phenyl, or
   two R² on different atoms are taken together with the atoms to which they are bound and any intervening ring atoms to form a ring which, taken together with the piperdine ring to which both R² are bound, is 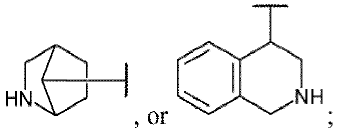 or
   two R² bound to the same ring atom are taken together with the atom to which they are bound to form a ring which, taken together with the piperdine ring to which both R² are bound, is: 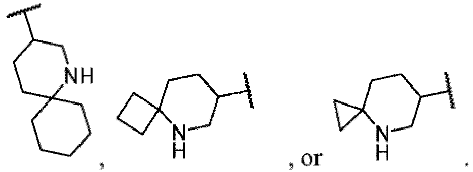
8. The compound of clause 7, wherein n is 0.
9. The compound of any one of clauses 1-8, wherein R³ is hydrogen.
10. The compound of any one of clauses 1-9, wherein R⁴ is selected from hydrogen, halo, -CN, optionally substituted C₁-C₄ alkyl, optionally substituted C₂-C₄ alkynyl, optionally substituted -O-C₁-C₄ alkyl, optionally substituted C₃-G₅ cycloalkyl, and -C(O)-optionally substituted C₁-C₄ alkyl.
11. The compound of clause 10, wherein R⁴ is selected from chloro, fluoro, bromo, iodo, cyclopropyl, -CN, -CF₃, -CH₂CF₃, -CH₂CH₂F, -CH₃, -CH₂CH₃, -CH₂CH₂CH₃, -CH(CH₃)₂. - CH₂CH(CH₃)₂, -OCH₃, -CH(OH)CH₃, -CH=CH₂, -C(O)CH₃, -OCHF₂, S-CH₃, -S-CHF₂, -S-CF₃, and -C≡CH.
12. The compound of clause 11, wherein R⁴ is selected from -CF₃, -CH₂CH₃, and - CH₂CH₂F
13. The compound of any one of clauses 1-12, wherein each R⁷ is independently selected from hydrogen, halo, -C₁-C₆ alkyl, -CN, -C(O)OH, -C(O)-(C₁-C₄ alkyl), -C(O)-N(R^{1'})₂, - S(O)₂-(C₁-C₄ alkyl), -CH₂-S(O)₂-(C₁-C₄ alkyl), -S(O)₂-N(R^{1'})₂, -P(OXC₁-C₄ alkyl)-O-C₁-C₄ alkyl, -P(O)(O-(C₁-C₄ alkyl))₂, carbocyclyl, heterocyclyl, heteroaryl, -C(O)-heterocyclyl, - (C₁-C₄ alkylene)-S(O)₂-(C₁-C₄ alkyl), -O-(C₀-C₆-alkylene)-carbocyclyl, phenyl, -(C₂-C₄ alkenylene)-phenyl, -S(O)-(C₁-C₄ alkyl), -S-(C₁-C₄ alkyl), -S(O)-OH, and -S(O)₂-OH, wherein any alkyl, alkylene, alkenylene, carbocyclyl, phenyl, heterocyclyl or heteroaryl is optionally substituted.
14. The compound of clause 13, wherein R⁷ is selected from hydrogen, fluoro, chloro, bromo, -CN, -CH₃, -CH₂CH₂C(CH₃)₂OH, -C(O)-CH₃, -C(O)OH, -C(O)-NH-CH₃, - P(=OXOCH₂CH₃)₂, -P(=OXOCH₂CH₃)CH₃, -S(O)₂CH₃, -P(O)-(CH₃)₂, -P(O)-(CH₂CH₃)₂, - S(O)₂N(CH₃)₂, -S(O)₂CH(CH₃)₂, -S(O)₂CH₂F, -S(O)₂CHF₂, -SCHF₂, -S(O)CHF₂, -S(O)OH, - S(O)₂OH, -S(O)₂NHCH₃, -(CH₂)₄CH₃, -CH₂S(O)₂CH₃, -S(O)₂-CH₂CH₃, 1H-pyrazol-4-yl, 1-methylpyrazol-4-yl, l,3-dimethyl-pyrazol-4-yl, 5-methyl-1H-pyrazol-4-yl, 1-methyl-2-oxoimidazolidin-3-yl, 4-methylimidazol-1-yl, morpholin-4-yl, pyridin-4yl, pyridazin-4-yl, 4-hydroxycyclohexyl, 4-hydroxy-4-methylcyclohexyl, 5-methyl-1,2,4-triazol-3-yl, 5-methyl, l,2,4-oxadiazol-3-yl, 1,3-dimethylpyridazin-4-yl, 1,5-dimethylpyridazin-4-yl, 3-methyl-1H-pyridazin-4-yl, 1-(2-methyl-2-hydroxypropyl)pyridazin-4-yl, imidazol-1-yl, 1-methyl-5-cyanopyrrol-3-yl, 5-cyano-1H-pyrrol-3-yl, and pyridazin-4-yl, 1H-pyrazol-3-yl, 1-ditluoromethyl-pyrazol-3-yl, 1-difluoromethyl-pyrazol-4-yl, 1-methylpyrazol-3-yl, 3-methyl-1H-pyrazol-4-yl, 3-methyl-3-hydroxypyrrolidin-1-ylcarbonyl, 3-hydroxypyrrolidin-1-ylcarbonyl, 4-hydroxycyclohexyl, 4-hydroxycyclohex-1-enyl, 1,1-dioxothiomorpholin-4-yl, 4-cyano-1H-imidazol-1-yl, 2,3-dimethyl-l,2,4-triazol-5-yl, l,5-dimethyl-pyrazol-4-yl, pyridin-3-yl, 1-(2-methyl-2-hydroxypropan-1-yl)pyrazol-4yl, pyrrolidin-1yl, pyrrolidin-1-ylcarbonyl, 1H-pyrazol-2-yl, 3-hydroxy-3-trifluoromethylpyrrolidin-1-ylcarbonyl, 3-methoxypyrrolidin-1-ylcarbonyl, 3-cyanopyrrolidin-1-ylcarbonyl, 4-hydroxy-4-methylpiperindin-1-ylcarbonyl, 3-oxopyrrolidin-1-ylcarbonyl, 3-(pyrrolidin-1-ylcarbonyl)phenyl, 3-phenoxyphenyl, thiazol-2-yl, pyrazin-2-yl, 2,4-dioxo-1H,3H-pyrimidin-5-yl, 3-methyl-3-hydroxypyrrolidin-1-ylsulfonyl, 5-flluorapyridin-3-yl, 2-hydroxpyridin-3-yl, 3,3-difluoro-4-hydroxy, 3,5-dimethyloxazol-4-yl, 3-fluorophenyl, 4-methylpyridin-3-yl, 2-hydroxymethylpyridin-3-yl, 6-hydroxymethylpyridin-2-yl, 5-hydroxymethylpyridin-3-yl, 1-methyl-6-oxopyridin-3-yl, 4-aminosulfonylphenyl, 3-aminosulfonylphenyl, 3-hydroxy-3-ethylpyrrolidin-1-ylcarbonyl, 3-cyano-4-hydroxyphenyl, benzo[d]thiazol-6-yl, 2H-indazol-6-yl, 1H-benzoimidazol-5-yl, 2-oxo-3-cyano-4-methylpyridin-5yl, 2-aminobenzo[d]thiazol-2-yl, 3-aminocarbonylphenyl, 6-trifluoromethyl-1H-pyrrolo[3,2-c]pyridin-3-yl, 2-aminoquinazolin-8-yl, styryl, 1methyl-1H-iadazol-6-yl, 2,3-dihydrobenzo[b][1,4]dioxin-7-yl, 2-ethoxyphenyl, 3-(2-hydroxyethyl)phenyl, 3-(methylcarbonylaminomethyl)phenyl, 1-methyl-6-trifluoromethyl-1H-pyrrolo[3,2-c]pyridin-3-yl, quinolin-4-yl, isoquinolin-5-yl, isoquinolin-7-yl, and 2-oxo-3,4-dihydroquinolin-7-yl.
15. The compound of clause 14, wherein R⁷ is selected from hydrogen, fluoro, -CN, - S(O)₂CH₃, -S(O)₂NHCH₃, 3-hydroxy-pyrrolidin-1-ylcarbonyl, 3-hydroxy-3-methylpyrrolidin-1-ylcarbonyl, and 1H-imidazol-2-yl.
16. The compound of any one of clauses 1-15, wherein R^{5'} is selected from hydrogen, C₁-C₄ alkyl, -(C₀-C₃ alkylene)-aryl and -(C₁-C₃ alkylene)-O-(C₁-C₄ alkyl).
17. The compound of clause 16, wherein R^{5'} is selected from hydrogen, methyl, isopropyl, -CH₂-O-CH₃, -(CH₂)₂-O-CH₃, and phenyl.
18. The compound of any one of clauses 1-17, wherein R⁶ is selected from hydrogen and methyl.
19. The compound of clause 18, wherein R⁶ is hydrogen.
20. The compound of clause 1, wherein the compound is a compound of formula (II): 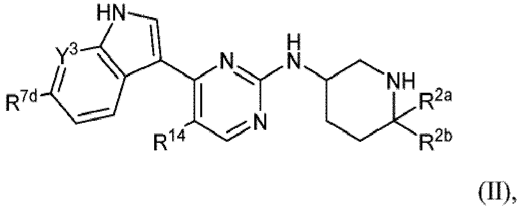 or a pharmaceutically acceptable salt thereof, wherein:
   Y³ is selected from N and C(R^{7e});
   each of R^{2a} and R^{2b} is independently selected from hydrogen and C₁-C₃ alkyl; or
   R^{2a} and R^{2b} are taken together to form a cycloalkyl or a heterocycle spirofused to the piperidine ring, wherein said cycloalkyl or heterocycle is optionally substituted with one or more independently selected C₁-C₄ alkyl or C₁-C₄ haloalkyl;
   R^{7d} is selected from hydrogen, -C(O)-(C₁-C₄ alkyl), -CN, and heteroaryl optionally substituted with one or more independently selected C₁-C₄ alkyl or C₁-C₄ haloalkyl;
   R^{7e}, if present, is selected from hydrogen, halo, -S(O)₂-(C₁-C₄ alkyl), -P(OXC₁-C₄ alkyl)2, -C(O)NH-(C₁-C₄ alkyl), -C(O)N(C₁-C₄ alkyl)₂, -S(O)₂NH-(C₁-C₄ alkyl), -S(O)₂N-(C₁-C₄ alkyl)₂, and heteroaryl optionally substituted with one or more independently selected C₁-C₄ alkyl or C₁-C₄ haloalkyl; and
   R¹⁴ is selected from C₁-C₃ alkyl and C₁-C₃ haloalkyl.
21. The compound of clause 1, wherein the compound is a compound of formula (III): 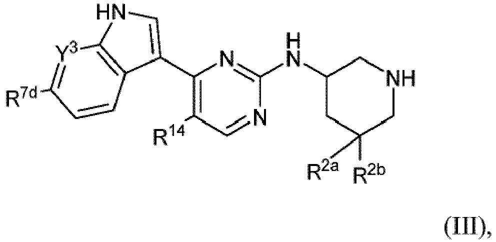 or a pharmaceutically acceptable salt thereof, wherein:
   Y³ is selected from N and C(R^{7e});
   each of R^{2a} and R^{2b} is independently selected from hydrogen and C₁-C₃ alkyl; or
   R^{2a} and R^{2b} are taken together to form a cycloalkyl or a heterocycle spirofused to the piperidine ring, wherein said cycloalkyl or heterocycle is optionally substituted with one or more independently selected C₁-C₄ alkyl or C₁-C₄ haloalkyl;
   R^{7d} is selected from hydrogen, -C(O)-(C₁-C₄ alkyl), -CN, and heteroaryl optionally substituted with one or more independently selected C₁-C₄ alkyl or C₁-C₄ haloalkyl;
   R^{7e}, if present, is selected from hydrogen, halo, -S(O)₂-(C₁-C₄ alkyl), -P(OXC₁-C₄ alkyl)₂, -C(O)NH-(C₁-C₄ alkyl), -C(O)N(C₁-C₄ alkyl)₂, -S(O)₂NH-(C₁-C₄ alkyl), -S(O)₂N-(C₁-C₄ alkyl)₂, and heteroaryl optionally substituted with one or more independently selected C₁-C₄ alkyl or C₁-C₄ haloalkyl; and
   R¹⁴ is selected from C₁-C₃ alkyl and C₁-C₃ haloalkyl.
22. The compound of clause 20 or 21, or a pharmaceutically acceptable salt thereof, wherein:
   R^{2a} is selected from hydrogen and -CH₃;
   R^{2b} is selected from hydrogen, -CH₃, -CH₂CH₃, and -CH(CH₃)₂; or
   R^{2a} and R^{2b} are taken together to from oxetan-3-yl;
   R^{7d} is selected from hydrogen, -C(O)CH₃, -CN, pyridin-3-yl, pyridin-4-yl, 1-methyl-5-cyanopyrrol-3-yl, 1-methylpyrazol-4-yl, 1-methylpyrazol-3-yl, 1H-pyrazol-4-yl, 1H-pyrazol-3-yl, 1H-imidazol-2-yl, 1,3-dimethylpyrazol-4-yl, 1,5-dimethylpyrazol-4-yl, 1,5-di.methyl-1,2,4-triazol-3 yl, imidazol-1-yl, l-difluoromethylpyrazol-3-yl, 1-difluoromethylpyrazol-4-yl and thiazol-2yl;
   R^{7e}, if present, is selected from hydrogen, fluoro, chloro, bromo, -CN, -P(O)(CH3)₂, - S(O)₂CH(CH₃)₂, -S(O)₂CH₂CH₃, -S(O)₂N(CH₃)₂, -C(O)NHCH₃, pyridin-4-yl, pyridazin-4yl, 5-methyl-1H-pyrazol-4-yl, 1-methylpyrazol-4yl, 4-methyl-1H-imidazol-1-yl, 1H-benzo[d]imidazol-5-yl, 6-(trifluoromethyl)-1H-pyrrolo[3,2-c]pyridin-3-yl, 1-methyl-6-(trifluoromethyl)-1H-pyrrolo[3,2-c]pyridin-3-yl, isoquinolin-7-yl, isoquinolin-5yl, pyrazin-2-yl, 2H-indazol-6-yl, 3,5-dimethylisoxazol-4-yl, thiazol-2-yl, 4-methylpyridin-3-yl, 1-methylindazol-6-yl, quinolin-4-yl, benzo[d]thiazol-6-yl, and 1,3-dimethylpyrazol-4-yl; and
   R¹⁴ is selected from -CH₃, -CF₃, -CH₂CH₃, -CH₂CF₃, -CH₂CH₂F, and -CH(CH₃)₂.
23. The compound of clause 21, or a pharmaceutically acceptable salt thereof wherein:
   R^{2a} is selected from hydrogen and -CH₃;
   R^{2b} is selected from hydrogen, and -CH₃;
   R^{7d} is selected from hydrogen, -CN, pyrazin-2yl, thiazol-2-yl, and 3,5-dimethylisoxazol-4-yl;
   R^{7e}, if present, is selected from hydrogen, fluoro, -C(O)NHCH₃, -P(O)(CH₃)₂, - S(O)₂CH₃, -S(O)₂N(CH₃)₂, 1,3-dimethylpyrazol-4-yl, and pyridazin-4-yl; and
   R¹⁴ is selected from -CH₂CH₃, and -CF₃.
24. A pharmaceutical composition comprising a compound of any one of clauses 1-23 and a pharmaceutically acceptable excipient.
25. A composition for use in treating a subject suffering from a disease or condition associated with aberrant activity of a serine/threonine kinase comprising a composition of clause 24 or a compound of any one of clauses 1-23.
26. The composition of clause 25, wherein the subject is suffering from a disease or condition associated with aberrant activity of CDK7.
27. The composition of clause 25 or 26, wherein the disease or condition is selected from cancer, benign neoplasm, angiogenesis, inflammatory disease, autoinflammatory disease, autoimmune disease, or an infectious disease.
28. The composition of any one of clauses 25-27, wherein the subject is a mammal.
29. The composition of any one of clauses 25-28, wherein the disease is cancer.
30. The composition of clause 29, wherein the cancer is selected from a blood cancer, melanoma, a bone cancer, a breast cancer, a brain cancer, or a lung cancer.
31. The composition of clause 30, wherein the cancer is a blood cancer selected from chronic lymphocytic leukemia (CLL), acute lymphoblastic leukemia (ALL), T-cell acute lymphoblastic leukemia (T-ALL), chronic myelogenous leukemia (CML), acute myelogenous leukemia (AML), lymphoma, and multiple myeloma.
32. The composition of clause 30, wherein the disease is a bone cancer selected from osteosarcoma and Ewing's sarcoma.
33. The composition of clause 30, wherein the disease is triple-negative breast cancer (TNBC).
34. The composition of clause 30, wherein the disease is neuroblastoma.
35. The composition of clause 30, wherein the disease is small cell lung cancer (SCLC).
36. The composition of any one of clauses 25-36, wherein the composition comprises one or more additional agents independently selected from anti-proliferative agents, anti-cancer agents, immunosuppressant agents, and pain-relieving agents.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims. Those of ordinary skill in the art will appreciate that various changes and modifications to this description may be made without departing from the spirit or scope of the present invention, as defined in the following claims.

## Claims

1. A compound of formula I: 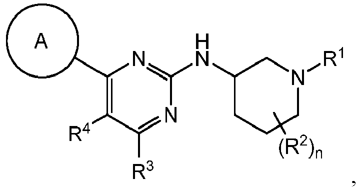 or a pharmaceutically acceptable salt thereof, wherein
ring A is a bicyclic 6,5-ring system selected from: 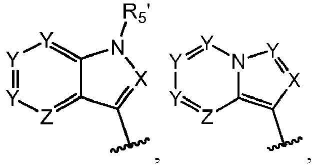 and 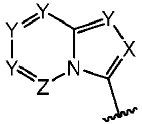 and comprises no more than four ring nitrogen atoms;
X is selected from N and C(R⁶);
each Y is independently selected from N and C(R⁷);
Z is selected from N and C(R⁸);
R¹ is selected from hydrogen, -C₁-C₆ alkyl, -O-(C₁-C₆-alkylene)-O-(C₁-C₄-alkyl), -(C₀-C₆ alkylene)-carbocyclyl, -C(O)-O-(C₁-C₆ alkylene), -(C₁-C₆ alkylene)-heterocyclyl, -(C₁-C₆ alkylene)-heteroaryl, -(C₁-C₆ alkylene)-N(R^{1'})₂, -(C₁-C₆ alkylene)-NR^{1'}-S(O)₂-(C₁-C₄ alkyl), -(C₁-C₆ alkylene)-NR^{1'}-SO₂.N(R^{1'})₂, -(C₁-C₆ alkylene)-S(O)₂-(C₁-C₄ alkyl), and -(C₁-C₆ alkylene)-S(O)₂-N(R^{1'})₂, wherein any carbocyclyl, heterocyclyl or heteroaryl portion of R¹ is optionally substituted, and wherein any alkyl or alkylene portion of R¹ is optionally substituted with one or more independently selected monovalent substituents;
each R^{1'} is independently selected from hydrogen and optionally substituted C₁-C₆ alkyl, or
two R^{1'} are optionally taken together with the nitrogen atom to which they are bound to form a 4-6 membered, optionally substituted heterocyclyl or heteroaryl ring comprising up to 2 additional heteroatoms selected from N, O, and S, wherein:
each R², if present, is independently selected from =O, halo, -OH, -CN, -C₁-C₆ alkyl, - (C₀-C₆ alkylene)-carbocyclyl, -(C₀-C₆ alkylene)-heterocyclyl, -(C₀-C₆ alkylene)-heteroaryl, -(C₀-C₆ alkylene)-aryl, -(C₀-C₆ alkylene)-C(O)-heterocyclyl, -(C₀-C₆ alkylene)-C(O)-heteroaryl, -O-(C₁-C₆-alkyl), -O-(C₁-C₆-alkylene)-O-(C₁-C₄-alkyl), -O-(C₁-C₄-alkylene)-carbocyclyl, -O-(C₁-C₆-alkylene)-heterocyclyl, -O-(C₁-C₆-alkylene)-heteroaryl, -O-(C₁-C₆-alkylene)-aryl, -NH-C(O)-C₁-C₄ alkyl, and -C(O)-NH-(unsubstituted C₁-C₄ alkyl), or
R¹ and any R² are taken together with the atoms to which they are bound to form an optionally substituted heterocyclyl or heteroaryl ring fused, spirofused or bridged to the piperidine ring, or
two R² are taken together with the atom or atoms to which they are bound and any intervening ring atoms to form an optionally substituted aryl, carbocyclyl, heterocyclyl or heteroaryl ring fused, spirofused or bridged to the piperidine ring,
wherein any carbocyclyl, heterocyclyl, or heteroaryl portion of R², any ring formed by taking R¹ together with R², or any ring formed by taking two R² together is optionally substituted, and wherein any alkyl or alkylene portion of R² is optionally substituted with one or more independently selected monovalent substituents unless otherwise specified;
R³ is selected from hydrogen, halo, -CN, optionally substituted -C₁-C₆ alkyl, or optionally substituted carbocyclyl;
R⁴ is selected from halo, -CN, -C₁-C₆ alkyl, -C₂-C₆ alkenyl, C₂-C₆ alkynyl, -O-C₁-C₆ alkyl, -S-C₁-C₆ alkyl, and carbocyclyl, wherein any alkyl, alkenyl, alkynyl, or carbocyclyl portion f R⁴ is optionally substituted;
each R⁵ is independently selected from halo, -OH, -C₁-C₆ alkyl, -CN, -(C₀-C₆ alkylene)-C(O)OH, -(C₀-C₆ alkylene)-C(O)-(C₁-C₄ alkyl), -(C₀-C₆ alkylene)-C(O)-N(R^{1'})₂, -(C₀-C₆ alkylene)-S(O)₂-(C₁-C₄ alkyl), -(C₀-C₆ alkylene)-S(O)₂₋N(R^{1'})₂, -(C₀-C₆ alkylene)-P(O)-O-(C₁-C₄ alkyl)₂, -(C₀-C₆ alkylene)-P(O)-(C₁-C₄ alkyl)(O-C₁-C₄ alkyl), -(C₀-C₆ alkylene)-P(O)(C₁-C₄ alkyl)₂, -(C₀-C₆ alkylene)-carbocyclyl, -(C₀-C₆ alkylene)-heterocyclyl, -(C₀-C₆ alkylene)-heteroaryl, -(C₀-C₆ alkylene)-C(O)-heterocyclyl, -(C₀-C₆ alkylene)-C(O)-heteroaryl, -O-(C₁-C₆-alkyl), -O-(C₁-C₆-alkylene)-O-(C₁-C₄-alkyl), -O-(C₀-C₆-alkylene)-carbocyclyl, -O-(C₁-C₆-alkylene)-heterocyclyl, -O-(C₁-C₆-alkylene)-heteroaryl, phenyl, -(C₂-C₄ alkenylene)-phenyl, - S(O)-(C₁-C₄ alkyl), -S-(C₁-C₄ alkyl), -S(O)-OH, and -S(O)₂-OH, wherein any alkyl, alkylene, alkenylene, carbocyclyl, heterocyclyl, phenyl, and heteroaryl portion of R⁵ is optionally substituted; or
two vicinal R⁵ are taken together with the ring atoms to which they are bound to form an optionally substituted carbocyclyl or optionally substituted heterocyclyl, wherein each carbocyclyl or heterocyclyl is fused to ring A;
R^{5'} is selected from hydrogen, -CN, -C₁-C₆ alkyl, -(C₀-C₆ alkylene)-S(O)₂₋N(R^{1'})₂, - (C₀-C₆ alkylene)-carbocyclyl, -(C₀-C₆ alkylene)-C(O)-N(R^{1'})₂, -(C₀-C₆ alkylene)-aryl, -(C₀-C₆ alkylene)-heterocyclyl, -(C₀-C₆ alkylene)-heteroaryl, -(C₀-C₆ alkylene)-S(O)₂-(C₁-C₄ alkyl), -(C₁-C₆ alkylene)-O-(C₁-C₃ alkylene)-C(O)-N(R^{1'})₂, -(C₁-C₆ alkylene)-O-(C₁-C₄ alkylene)-P(O)(C₁-C₄ alkyl)₂, -(C₁-C₆ alkylene)-O-(C₁-C₄ alkylene)-P(O)(C₁-C₄ alkyl)-O-(C₁-C₄ alkyl), -(C₁-C₆ alkylene)-O-(C₁-C₄alkylene)-P(O)-(O-C₁-C₄alkyl)₂, -(C₁-C₆alkylene)-O-(C₁-C₄alkylene)-S(O)₂-(C₁-C₄ alkyl), -(C₁-C₆ alkylene)-O-(C₁-C₄ alkylene)-S(O)₂₋N(R^{1'})₂, -(C₁-C₆ alkylene)-O-(C₁-C₄ alkyl), - (C₁-C₆ alkylene)-O-carbocyclyl, -(C₁-C₆ alkylene)-O-heteroaryl, -(C₁-C₆ alkylene)-O-heterocyclyl, -(C₁-C₆ alkylene)-P(O)(C₁-C₄ alkyl)₂, -(C₁-C₆ alkylene)-P(O)(C₁-C₄ alkyl)-O-(C₁-C₄ alkyl), -(C₁-C₆ alkylene)-P(O)-(O-C₁-C₄ alkyl)₂, -(C₁-C₆ alkylene)-C(O)-(C₁-C₄ alkyl), and -(C₁-C₆ alkylene)-C(O)OH, wherein any alkyl, alkylene, carbocyclyl, heterocyclyl and heteroaryl portion of R^{5'} is optionally substituted;
R^{5'} and any R⁵ are taken together with the ring atoms to which they are bound to form an optionally substituted heterocyclyl, wherein each heterocyclyl is fused to ring A;
R⁶ is selected from hydrogen, -CN, -CH₃, -CH₂F, -CHF₂ and -CF₃;
each R⁷ is independently selected from hydrogen and R⁵;
R⁸ is selected from hydrogen and fluoro; and
n is 0, 1, 2, 3, or 4, wherein the compound is other than 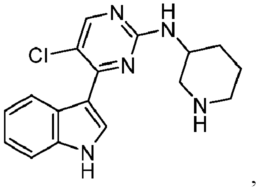 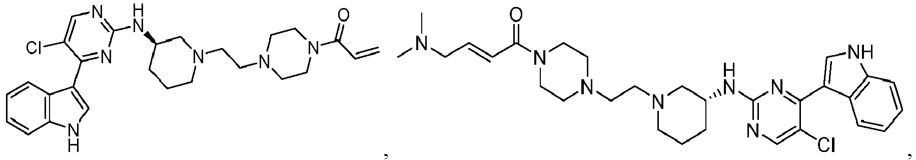 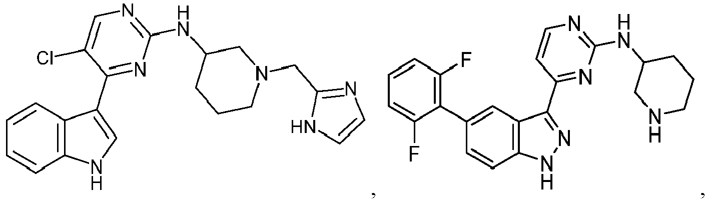 or a stereoisomer or pharmaceutical salt of any of the foregoing.

2. The compound of claim 1, or the pharmaceutically acceptable salt, solvate, hydrate, tautomer, stereoisomer, or isotopically labeled derivative thereof, wherein
ring A is 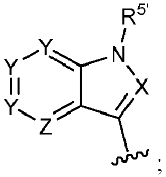
X is N;
any heterocyclyl or heteroaryl portion of R¹ is optionally substituted with one or more substituents independently selected from halo, C₁-C₄ alkyl, C₃-C₆ cycloalkyl, -OH, =0, -CN, - C(O)N(R^{1'})₂, S(O)₂-(C₁-C₄ alkyl), and -S(O)₂-N(R^{1'})₂, and wherein any cycloalkyl, alkyl or alkylene portion of R¹ is optionally substituted with one or more substituents independently selected from fluorine, -OH, and -CN; and
the compound is other than 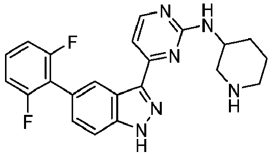 or a pharmaceutically acceptable salt or stereoisomer thereof.

3. The compound of claim 1 or 2, wherein the compound conforms to formula I or is a pharmaceutically acceptable salt thereof.

4. The compound of any one of claims 1-3, or the pharmaceutically acceptable salt, solvate, hydrate, tautomer, stereoisomer, or isotopically labeled derivative thereof,
wherein ring A is

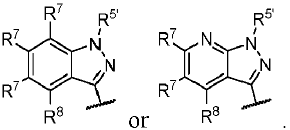

5. The compound of any one of claims 1-4, or the pharmaceutically acceptable salt, solvate, hydrate, tautomer, stereoisomer, or isotopically labeled derivative thereof, wherein R¹ is hydrogen, cyclopropyl, -CH₃, -CH₂CH₃, -CH₂CH₂OCH₃, -CH(CH₃)₂, or -CH₂CH(CH₃)₂, or wherein R¹ is taken together with one R² and the ring atoms to which each are bound to form a ring which, taken together with the ring to which R¹ and R² are bound, is 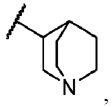 preferably wherein R¹ is hydrogen.

6. The compound of any one of claims 1-5, or the pharmaceutically acceptable salt, solvate, hydrate, tautomer, stereoisomer, or isotopically labeled derivative thereof, wherein each R², if present, is independently selected from halo, =0, -OH, -C₁-C₄ alkyl, phenyl, heteroaryl, -C(0)-heterocyclyl, -NH-C(O)-C₁-C₄ alkyl, and -C(O)-NH-(unsubstituted C₁-C₄ alkyl), wherein any heteroaryl or heterocyclyl portion of R² is optionally substituted and any C₁-C₄ alkyl or phenyl portion of R² is optionally substituted with one or more independently selected monovalent substituents, or
two R² are taken together with the atom or atoms to which they are bound and any intervening ring atoms to form an optionally substituted aryl cycloalkyl, heterocyclyl or heteroaryl ring fused, spirofused or bridged to the piperidine ring,
preferably wherein n is 0, 1, 2 or 3, and each R², if present, is independently selected from fluoro, =O, -CH₃, -CH₂CH₃, -OH, -CH(CH₃)₂, -C(O)NHCH₃, -NHC(O)CH₂CH₃, 3-methyl-1,2,4-oxadiazol-5-yl, 1,2,4-triazolo[4,3-a]393uinolin-3-yl, 8-methylsulfonyl-1,2,4-triazolo[4,3-a]393uinolin-3-yl, 393uinoline393e-l-ylcarbonyl, 3-hydroxypyrrolidin-1-ylcarbonyl, and unsubstituted phenyl, or
two R² on different atoms are taken together with the atoms to which they are bound and any intervening ring atoms to form a ring which, taken together with the piperidine ring to which both R² are bound, is 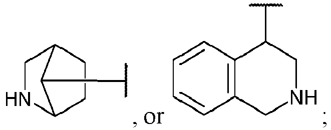 or
two R² bound to the same ring atom are taken together with the atom to which they are bound to form a ring which, taken together with the piperidine ring to which both R² are bound, is: 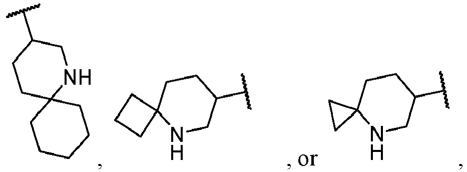
more preferably wherein n is 0.

7. The compound of any one of claims 1-6, or the pharmaceutically acceptable salt, solvate, hydrate, tautomer, stereoisomer, or isotopically labeled derivative thereof, wherein n is 2.

8. The compound of any one of claims 1-7, or the pharmaceutically acceptable salt, solvate, hydrate, tautomer, stereoisomer, or isotopically labeled derivative thereof, wherein R³ is hydrogen.

9. The compound of any one of claims 1-8, or the pharmaceutically acceptable salt, solvate, hydrate, tautomer, stereoisomer, or isotopically labeled derivative thereof, wherein R⁴ is halo, -CN, optionally substituted C₁-C₄ alkyl, optionally substituted C₂-C₄ alkynyl, optionally substituted -O-C₁-C₄ alkyl, optionally substituted C₃-C₆ cycloalkyl, or -C(O)-optionally substituted C₁-C₄ alkyl,
preferably wherein R⁴ is chloro, fluoro, bromo, iodo, cyclopropyl, -CN, -CF₃, -CH₂CF₃, - CH₂CH₂F, -CH₃, -CH₂CH₃, -CH₂CH₂CH₃, -CH(CH₃)₂, -CH₂CH(CH₃)₂, -OCH₃, -CH(OH)CH₃, -CH=CH₂, -C(O)CH₃, -OCHF₂, S-CH₃, -S-CHF₂, -S-CF₃, or -C≡CH,
or more preferably wherein R⁴ is -CF₃, -CH₂CH₃, or -CH₂CH₂F.

10. The compound of any one of claims 1-9, or the pharmaceutically acceptable salt, solvate, hydrate, tautomer, stereoisomer, or isotopically labeled derivative thereof, wherein each R⁷ is, independently, hydrogen, halo, -C₁-C₆ alkyl, -CN, -C(O)OH, -C(O)-(C₁-C₄ alkyl), - C(O)-N(R^{1'})₂, -S(O)₂-(C₁-C₄ alkyl), -CH₂-S(O)₂-(C₁-C₄ alkyl), -S(O)₂-N(R^{1'})₂, -P(O)(C₁-C₄ alkyl)-O-C₁-C₄ alkyl, -P(O)(O-(C₁-C₄ alkyl))₂, carbocyclyl, heterocyclyl, heteroaryl, -C(O)-heterocyclyl, -(C₁-C₄ alkylene)-S(O)₂-(C₁-C₄ alkyl), -O-(C₀-C₆-alkylene)-carbocyclyl, phenyl, -(C₂-C₄ alkenylene)-phenyl, -S(O)-(C₁-C₄ alkyl), -S-(C₁-C₄ alkyl), -S(O)-OH, or -S(O)₂-OH, wherein any alkyl, alkylene, alkenylene, carbocyclyl, phenyl, heterocyclyl or heteroaryl is optionally substituted,
preferably wherein each R⁷ is hydrogen, fluoro, chloro, bromo, -CN, -CH₃, - CH₂CH₂C(CH₃)₂OH, -C(O)-CH₃, -C(O)OH, -C(O)-NH-CH₃, -P(=O)(OCH₂CH₃)₂, - P(=O)(OCH₂CH₃)CH₃, -S(O)₂CH₃, -P(O)-(CH₃)₂, -P(O)-(CH₂CH₃)₂, -S(O)₂N(CH₃)₂, - S(O)₂CH(CH₃)₂, -S(O)₂CH₂F, -S(O)₂CHF₂, -SCHF₂, -S(O)CHF₂, -S(O)OH, -S(O)₂OH, - S(O)₂NHCH₃, -(CH₂)₄CH₃, -CH₂S(O)₂CH₃, -S(O)₂-CH₂CH₃, 1H-pyrazol-4-yl, 1-methylpyrazol-4-yl, 1,3-dimethyl-pyrazol-4-yl, 5-methyl-1H-pyrazol-4-yl, 1-methyl-2-oxoimidazolidin-3-yl, 4-methylimidazol-1-yl, morpholin-4-yl, 394uinolin-4-yl, 394uinoline394-4-yl, 4-hydroxycyclohexyl, 4-hydroxy-4-methylcyclohexyl, 5-methyl-1,2,4-triazol-3-yl, 5-methyl, 1,2,4-oxadiazol-3-yl, 1,3-dimethylpyridazin-4-yl, 1,5-dimethylpyridazin-4-yl, 3-methyl-1H-pyridazin-4-yl, 1-(2-methyl-2-hydroxypropyl)394uinoline394-4-yl, 394uinoline-l-yl, 1-methyl-5-cyanopyrrol-3-yl, 5-cyano-1H-pyrrol-3-yl, and 394uinoline394-4-yl, 1H-pyrazol-3-yl, 1-difluoromethyl-pyrazol-3-yl, 1-difluoromethyl-pyrazol-4-yl, 1-methylpyrazol-3-yl, 3-methyl-1H-pyrazol-4-yl, 3-methyl-3-hydroxypyrrolidin-1-ylcarbonyl, 3-hydroxypyrrolidin-1-ylcarbonyl, 4-hydroxycyclohexyl, 4-hydroxycyclohex-l-enyl, l,l-dioxothiomorpholin-4-yl, 4-cyano-1H-imidazol-l-yl, 2,3-dimethyl-1,2,4-triazol-5-yl, 1,5-dimethyl-pyrazol-4-yl, 394uinolin-3-yl, 1-(2-methyl-2-hydroxypropan-1-yl)pyrazol-4-yl, 394uinoline394e-1-yl, 3 94uinoline3 94e-1 -ylcarbonyl, 1H-pyrazol-2-yl, 3-hydroxy-3-trifluoromethylpyrrolidin-1-ylcarbonyl, 3 -methoxypyrrolidin-1 - ylcarbonyl, 3-cyanopyrrolidin-1-ylcarbonyl, 4-hydroxy-4-methylpiperidin-1-ylcarbonyl, 3-oxopyrrolidin-1-ylcarbonyl, 3-(394uinoline394e-1-ylcarbonyl)phenyl, 3-phenoxyphenyl, thiazol-2-yl, pyrazin-2-yl, 2,4-dioxo-1H,3H-pyrimidin-5-yl, 3-methyl-3-hydroxypyrrolidin-1-ylsulfonyl, 5-flluoropyridin-3-yl, 2-hydroxpyridin-3-yl, 3,3-difluoro-4-hydroxy, 3,5-dimethyloxazol-4-yl, 3-fluorophenyl, 4-methylpyridin-3-yl, 2-hydroxymethylpyridin-3-yl, 6-hydroxymethylpyridin-2-yl, 5-hydroxymethylpyridin-3-yl, 1-methyl-6-oxopyridin-3-yl, 4-aminosulfonylphenyl, 3-aminosulfonylphenyl, 3-hydroxy-3-ethylpyrrolidin-1-ylcarbonyl, 3-cyano-4-hydroxyphenyl, benzo[*d*]thiazol-6-yl, 2H-indazol-6-yl, 1H-benzoimidazol-5-yl, 2-oxo-3-cyano-4-methylpyridin-5-yl, 2-aminobenzo[*d*]thiazol-2-yl, 3-aminocarbonylphenyl, 6-trifluoromethyl-1H-pyrrolo[3,2-c]394uinolin-3-yl, 2-aminoquinazolin-8-yl, styryl, 1-methyl-1H-indazol-6-yl, 2,3-dihydrobenzo[b][l,4]dioxin-7-yl, 2-ethoxyphenyl, 3-(2-hydroxyethyl)phenyl, 3-(methylcarbonylaminomethyl)phenyl, 1-methyl-6-trifluoromethyl-1H-pyrrolo[3,2-c]394uinolin-3-yl, 395uinoline-4-yl, isoquinolin-5-yl, isoquinolin-7-yl, or 2-oxo-3,4-dihydroquinolin-7-yl,
more preferably wherein each R⁷ is hydrogen, fluoro, -CN, -S(O)₂CH₃, -S(O)₂NHCH₃, 3-hydroxy-pyrrolidin-1-ylcarbonyl, 3-hydroxy-3-methyl-pyrrolidin-1-ylcarbonyl, or 1H-imidazol-2-yl.

11. The compound of any one of claims 1-10, or the pharmaceutically acceptable salt, solvate, hydrate, tautomer, stereoisomer, or isotopically labeled derivative thereof, wherein R^{5'} is selected from hydrogen, C₁-C₄ alkyl, -(C₀-C₃ alkylene)-aryl and -(C₁-C₃ alkylene)-O-(C₁-C₄ alkyl),
preferably wherein R^{5'} is hydrogen, methyl, isopropyl, -CH₂-O-CH₃, -(CH₂)₂-O-CH₃, or phenyl.

12. The compound of any one of claims 1-3, or the pharmaceutically acceptable salt, solvate, hydrate, tautomer, stereoisomer, or isotopically labeled derivative thereof, wherein the compound is selected from:

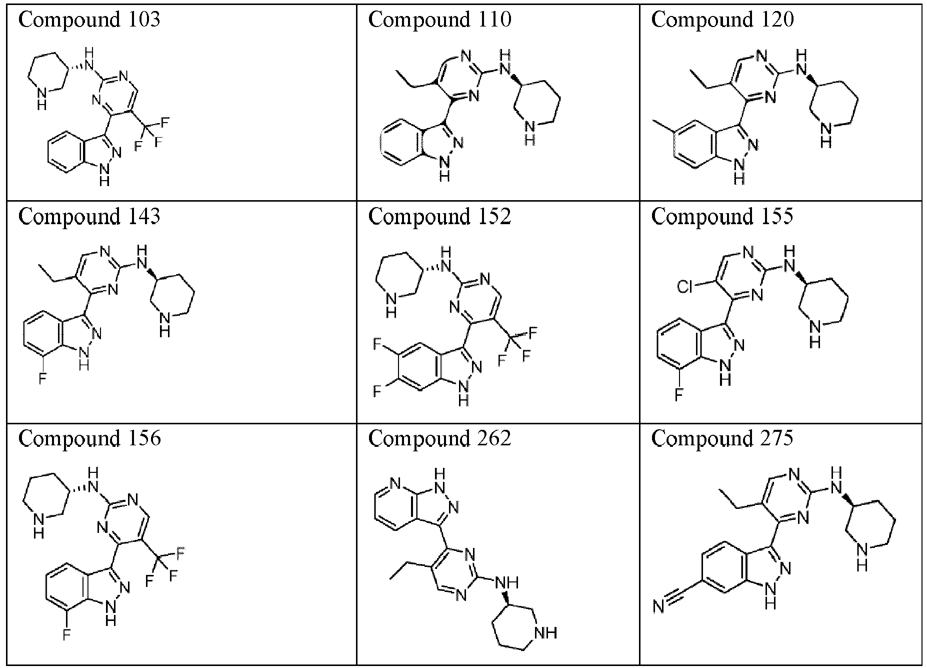

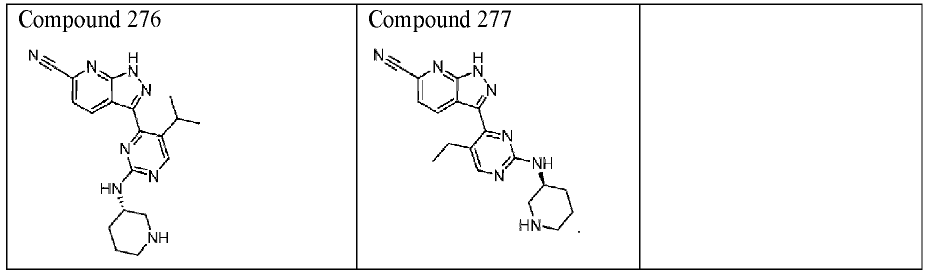

13. A pharmaceutical composition comprising (a) the compound of any one of claims 1-12, or the pharmaceutically acceptable salt, solvate, hydrate, tautomer, stereoisomer, or isotopically labeled derivative thereof, and (b) a pharmaceutically acceptable excipient.

14. The compound of any one of claims 1-12, or the pharmaceutically acceptable salt, solvate, hydrate, tautomer, stereoisomer, or isotopically labeled derivative thereof, or the pharmaceutical composition of claim 13 for use in treating a subject suffering from a disease or condition associated with aberrant activity of CDK7,
wherein the disease or condition is a cancer, a benign neoplasm, an inflammatory disease, an autoimmune disease, or an infectious disease,
preferably wherein the disease is a cancer,
more preferably wherein the cancer is a blood cancer, a melanoma, a bone cancer, a breast cancer, a brain cancer, a pancreatic cancer, or a lung cancer.

15. The compound or pharmaceutical composition for use according to claim 14, wherein the cancer is:
(a) a blood cancer selected from chronic lymphocytic leukemia (CLL), acute lymphoblastic leukemia (ALL), T-cell acute lymphoblastic leukemia (T-ALL), chronic myelogenous leukemia (CML), acute myelogenous leukemia (AML), a lymphoma, and multiple myeloma;
(b) a bone cancer selected from osteosarcoma and Ewing's sarcoma;
(c) triple-negative breast cancer (TNBC);
(d) neuroblastoma;
(e) pancreatic cancer; or
(f) small cell lung cancer (SCLC).

16. The compound or pharmaceutical composition for use according to claim 14 or claim 15, wherein the use further comprises the use of one or more additional agents independently selected from anti-proliferative agents, anti-cancer agents, immunosuppressant agents, and pain-relieving agents.
